# EUROPEAN PATENT APPLICATION

(11) **EP 1 666 594 A2**
(43) Date of publication of application: **07.06.2006**
(21) Application number: 05024035.7
(22) Date of filing: 01.06.2001
(51) Int. Cl.: C12N 15/12, C12N 15/62, C12N 15/63, C12N 5/10, C12N 1/19, C12N 1/21, C07K 14/47, C07K 16/18, C12Q 1/68, G01N 33/574

(54) **Polypeptide, nucleic acid encoding it, and their use for the diagnosis of cancer**

(30) Priority: 02.06.2000 WO PCT/US00/15264; 05.06.2000 US 209832 P; 20.06.2000 US 212901 P; 22.06.2000 US 213807 P; 20.07.2000 US 219556 P; 25.07.2000 US 220585 P; 25.07.2000 US 220605 P; 25.07.2000 US 220607 P; 25.07.2000 US 220624 P; 25.07.2000 US 220638 P; 25.07.2000 US 220664 P; 25.07.2000 US 220666 P; 26.07.2000 US 220893 P; 28.07.2000 WO PCT/US00/20710; 01.08.2000 US 222425 P; 22.08.2000 US 227133 P; 23.08.2000 WO PCT/US00/23522; 24.08.2000 WO PCT/US00/23328; 15.09.2000 WO PCT/US00/60000; 10.11.2000 WO PCT/US00/30873; 28.11.2000 US 253646 P; 01.12.2000 WO PCT/US00/32678; 20.12.2000 US 747259; 20.12.2000 WO PCT/US00/34956; 28.02.2001 WO PCT/US00/06520; 01.03.2001 WO PCT/US01/06666; 22.03.2001 US 816744; 10.05.2001 US 854208; 10.05.2001 US 854280; 25.05.2001 WO PCT/US00/17092
(62) Divisional of application: 04005726.7
(71) Applicant: GENENTECH, INC., South San Francisco, CA 94080-4990 (US)
(72) Inventor: Baker, Kevin P., Darnestown, MD 20878 (US); Desnoyers, Luc, San Francisco, CA 94133 (US); Gerritsen, Mary E., San Mateo, CA 94402 (US); Goddard, Audrey, San Francisco, CA 94402 (US); Godowski, Paul J., Burlingame, CA 94010 (US); Grimaldi, Christopher J., San Francisco, CA 64122 (US); Gurney, Austin L., San Francisco, CA 94114 (US); Smith, Victoria, Burlingame, CA 94010 (US); Stephan, Jean-Philippe F., San Carlos, CA 94070 (US); Watanabe, Colin K., Moraga, CA 94556 (US); Wood, William I., Hillsborough, CA 94010 (US)
(74) Representative: Denison, Christopher Marcus

(57) **Abstract**

The present invention is directed to novel polypeptides and to nucleic acid molecules encoding those polypeptides. Also provided herein are vectors and host cells comprising those nucleic acid sequences, chimeric polypeptide molecules comprising the polypeptides of the present invention fused to heterologous polypeptide sequences, antibodies which bind to the polypeptides of the present invention and to methods for producing the polypeptides of the present invention.

## Description

### FIELD OF THE INVENTION

The present invention relates generally to the identification and isolation of novel DNA and to the recombinant production of novel polypeptides.

### BACKGROUND OF THE INVENTION

Extracellular proteins play important roles in, among other things, the formation, differentiation and maintenance of multicellular organisms. The fate of many individual cells, e.g., proliferation, migration, differentiation, or interaction with other cells, is typically governed by information received from other cells and/or the immediate environment. This information is often transmitted by secreted polypeptides (for instance, mitogenic factors, survival factors, cytotoxic factors, differentiation factors, neuropeptides, and hormones) which are, in turn, received and interpreted by diverse cell receptors or membrane-bound proteins. These secreted polypeptides or signaling molecules normally pass through the cellular secretory pathway to reach their site of action in the extracellular environment.

Secreted proteins have various industrial applications, including as pharmaceuticals, diagnostics, biosensors and bioreactors. Most protein drugs available at present, such as thrombolytic agents, interferons, interleukins, erythropoietins, colony stimulating factors, and various other cytokines, are secretory proteins. Their receptors, which are membrane proteins, also have potential as therapeutic or diagnostic agents. Efforts are being undertaken by both industry and academia to identify new, native secreted proteins. Many efforts are focused on the screening of mammalian recombinant DNA libraries to identify the coding sequences for novel secreted proteins. Examples of screening methods and techniques are described in the literature [see, for example, Klein et al., Proc. Natl. Acad. Sci. 93:7108-7113 (1996); U.S. Patent No. 5,536,637)].

Membrane-bound proteins and receptors can play important roles in, among other things, the formation, differentiation and maintenance of multicellular organisms. The fate of many individual cells, e.g., proliferation, migration, differentiation, or interaction with other cells, is typically governed by information received from other cells and/or the immediate environment. This information is often transmitted by secreted polypeptides (for instance, mitogenic factors, survival factors, cytotoxic factors, differentiation factors, neuropeptides, and hormones) which are, in turn, received and interpreted by diverse cell receptors or membrane-bound proteins. Such membrane-bound proteins and cell receptors include, but are not limited to, cytokine receptors, receptor kinases, receptor phosphatases, receptors involved in cell-cell interactions, and cellular adhesin molecules like selectins and integrins. For instance, transduction of signals that regulate cell growth and differentiation is regulated in part by phosphorylation of various cellular proteins. Protein tyrosine kinases, enzymes that catalyze that process, can also act as growth factor receptors. Examples include fibroblast growth factor receptor and nerve growth factor receptor.

Membrane-bound proteins and receptor molecules have various industrial applications, including as pharmaceutical and diagnostic agents. Receptor immunoadhesins, for instance, can be employed as therapeutic agents to block receptor-ligand interactions. The membrane-bound proteins can also be employed for screening of potential peptide or small molecule inhibitors of the relevant receptor/ligand interaction.

Efforts are being undertaken by both industry and academia to identify new, native receptor or membrane-bound proteins. Many efforts are focused on the screening of mammalian recombinant DNA libraries to identify the coding sequences for novel receptor or membrane-bound proteins.

### SUMMARY OF THE INVENTION

In one embodiment, the invention provides an isolated nucleic acid molecule comprising a nucleotide sequence that encodes a PRO polypeptide.

In one aspect, the isolated nucleic acid molecule comprises a nucleotide sequence having at least about 80 % nucleic acid sequence identity, alternatively at least about 81 % nucleic acid sequence identity, alternatively at least about 82 % nucleic acid sequence identity, alternatively at least about 83 % nucleic acid sequence identity, alternatively at least about 84% nucleic acid sequence identity, alternatively at least about 85% nucleic acid sequence identity, alternatively at least about 86% nucleic acid sequence identity, alternatively at least about 87% nucleic acid sequence identity, alternatively at least about 88 % nucleic acid sequence identity, alternatively at least about 89% nucleic acid sequence identity, alternatively at least about 90% nucleic acid sequence identity, alternatively at least about 91 % nucleic acid sequence identity, alternatively at least about 92% nucleic acid sequence identity, alternatively at least about 93 % nucleic acid sequence identity, alternatively at least about 94% nucleic acid sequence identity, alternatively at least about 95 % nucleic acid sequence identity, alternatively at least about 96% nucleic acid sequence identity, alternatively at least about 97% nucleic acid sequence identity, alternatively at least about 98% nucleic acid sequence identity and alternatively at least about 99% nucleic acid sequence identity to (a) a DNA molecule encoding a PRO polypeptide having a full-length amino acid sequence as disclosed herein, an amino acid sequence lacking the signal peptide as disclosed herein, an extracellular domain of a transmembrane protein, with or without the signal peptide, as disclosed herein or any other specifically defined fragment of the full-length amino acid sequence as disclosed herein, or (b) the complement of the DNA molecule of (a).

In other aspects, the isolated nucleic acid molecule comprises a nucleotide sequence having at least about 80 % nucleic acid sequence identity, alternatively at least about 81 % nucleic acid sequence identity, alternatively at least about 82 % nucleic acid sequence identity, alternatively at least about 83 % nucleic acid sequence identity, alternatively at least about 84% nucleic acid sequence identity, alternatively at least about 85% nucleic acid sequence identity, alternatively at least about 86 % nucleic acid sequence identity, alternatively at least about 87 % nucleic acid sequence identity, alternatively at least about 88 % nucleic acid sequence identity, alternatively at least about 89% nucleic acid sequence identity, alternatively at least about 90% nucleic acid sequence identity, alternatively at least about 91 % nucleic acid sequence identity, alternatively at least about 92% nucleic acid sequence identity, alternatively at least about 93 % nucleic acid sequence identity, alternatively at least about 94 % nucleic acid sequence identity, alternatively at least about 95 % nucleic acid sequence identity, alternatively at Least about 96% nucleic acid sequence identity, alternatively at least about 97% nucleic acid sequence identity, alternatively at least about 98% nucleic acid sequence identity and alternatively at least about 99% nucleic acid sequence identity to (a) a DNA molecule comprising the coding sequence of a full-length PRO polypeptide cDNA as disclosed herein, the coding sequence of a PRO polypeptide lacking the signal peptide as disclosed herein, the coding sequence of an extracellular domain of a transmembrane PRO polypeptide, with or without the signal peptide, as disclosed herein or the coding sequence of any other specifically defined fragment of the full-length amino acid sequence as disclosed herein, or (b) the complement of the DNA molecule of (a).

In a further aspect, the invention concerns an isolated nucleic acid molecule comprising a nucleotide sequence having at least about 80 % nucleic acid sequence identity, alternatively at least about 81 % nucleic acid sequence identity, alternatively at least about 82 % nucleic acid sequence identity, alternatively at least about 83 % nucleic acid sequence identity, alternatively at least about 84 % nucleic acid sequence identity, alternatively at least about 85% nucleic acid sequence identity, alternatively at least about 86% nucleic acid sequence identity, alternatively at least about 87% nucleic acid sequence identity, alternatively at least about 88% nucleic acid sequence identity, alternatively at least about 89% nucleic acid sequence identity, alternatively at least about 90 % nucleic acid sequence identity, alternatively at least about 91 % nucleic acid sequence identity, alternatively at least about 92% nucleic acid sequence identity, alternatively at least about 93% nucleic acid sequence identity, alternatively at least about 94% nucleic acid sequence identity, alternatively at least about 95% nucleic acid sequence identity, alternatively at least about 96 % nucleic acid sequence identity, alternatively at least about 97 % nucleic acid sequence identity, alternatively at least about 98% nucleic acid sequence identity and alternatively at least about 99 % nucleic acid sequence identity to (a) a DNA molecule that encodes the same mature polypeptide encoded by any of the human protein cDNAs deposited with the ATCC as disclosed herein, or (b) the complement of the DNA molecule of (a).

Another aspect the invention provides an isolated nucleic acid molecule comprising a nucleotide sequence encoding a PRO polypeptide which is either transmembrane domain-deleted or transmembrane domain-inactivated, or is complementary to such encoding nucleotide sequence, wherein the transmembrane domain(s) of such polypeptide are disclosed herein. Therefore, soluble extracellular domains of the herein described PRO polypeptides are contemplated.

Another embodiment is directed to fragments of a PRO polypeptide coding sequence, or the complement thereof, that may find use as, for example, hybridization probes, for encoding fragments of a PRO polypeptide that may optionally encode a polypeptide comprising a binding site for an anti-PRO antibody or as antisense oligonucleotide probes. Such nucleic acid fragments are usually at least about 10 nucleotides in length, alternatively at least about 15 nucleotides in length, alternatively at least about 20 nucleotides in length, alternatively at least about 30 nucleotides in length, alternatively at least about 40 nucleotides in length, alternatively at least about 50 nucleotides in length, alternatively at least about 60 nucleotides in length, alternatively at least about 70 nucleotides in length, alternatively at least about 80 nucleotides in length, alternatively at least about 90 nucleotides in length, alternatively at least about 100 nucleotides in length, alternatively at least about 110 nucleotides in length, alternatively at least about 120 nucleotides in length, alternatively at least about 130 nucleotides in length, alternatively at least about 140 nucleotides in length, alternatively at least about 150 nucleotides in length, alternatively at least about 160 nucleotides in length, alternatively at least about 170 nucleotides in length, alternatively at least about 180 nucleotides in length, alternatively at least about 190 nucleotides in length, alternatively at least about 200 nucleotides in length, alternatively at least about 250 nucleotides in length, alternatively at least about 300 nucleotides in length, alternatively at least about 350 nucleotides in length, alternatively at least about 400 nucleotides in length, alternatively at least about 450 nucleotides in length, alternatively at least about 500 nucleotides in length, alternatively at least about 600 nucleotides in length, alternatively at least about 700 nucleotides in length, alternatively at least about 800 nucleotides in length, alternatively at least about 900 nucleotides in length and alternatively at least about 1000 nucleotides in length, wherein in this context the term "about" means the referenced nucleotide sequence length plus or minus 10% of that referenced length. It is noted that novel fragments of a PRO polypeptide-encoding nucleotide sequence may be determined in a routine manner by aligning the PRO polypeptide-encoding nucleotide sequence with other known nucleotide sequences using any of a number of well known sequence alignment programs and determining which PRO polypeptide-encoding nucleotide sequence fragment(s) are novel. All of such PRO polypeptide-encoding nucleotide sequences are contemplated herein. Also contemplated are the PRO polypeptide fragments encoded by these nucleotide molecule fragments, preferably those PRO polypeptide fragments that comprise a binding site for an anti-PRO antibody.

In another embodiment, the invention provides isolated PRO polypeptide encoded by any of the isolated nucleic acid sequences hereinabove identified.

In a certain aspect, the invention concerns an isolated PRO polypeptide, comprising an amino acid sequence having at least about 80% amino acid sequence identity, alternatively at least about 81% amino acid sequence identity, alternatively at least about 82 % amino acid sequence identity, alternatively at least about 83 % amino acid sequence identity, alternatively at least about 84 % amino acid sequence identity, alternatively at least about 85% amino acid sequence identity, alternatively at least about 86% amino acid sequence identity, alternatively at least about 87% amino acid sequence identity, alternatively at least about 88 % amino acid sequence identity, alternatively at least about 89% amino acid sequence identity, alternatively at least about 90% amino acid sequence identity, alternatively at least about 91 % amino acid sequence identity, alternatively at least about 92% amino acid sequence identity, alternatively at least about 93% amino acid sequence identity, alternatively at least about 94% amino acid sequence identity, alternatively at least about 95% amino acid sequence identity, alternatively at least about 96 % amino acid sequence identity, alternatively at least about 97 % amino acid sequence identity, alternatively at least about 98% amino acid sequence identity and alternatively at least about 99% amino acid sequence identity to a PRO polypeptide having a full-length amino acid sequence as disclosed herein, an amino acid sequence lacking the signal peptide as disclosed herein, an extracellular domain of a transmembrane protein, with or without the signal peptide, as disclosed herein or any other specifically defined fragment of the full-length amino acid sequence as disclosed herein.

In a further aspect, the invention concerns an isolated PRO polypeptide comprising an amino acid sequence having at least about 80% amino acid sequence identity, alternatively at least about 81% amino acid sequence identity, alternatively at least about 82% amino acid sequence identity, alternatively at least about 83 % amino acid sequence identity, alternatively at least about 84% amino acid sequence identity, alternatively at least about 85% amino acid sequence identity, alternatively at least about 86% amino acid sequence identity, alternatively at least about 87% amino acid sequence identity, alternatively at least about 88% amino acid sequence identity, alternatively at least about 89% amino acid sequence identity, alternatively at least about 90% amino acid sequence identity, alternatively at least about 91 % amino acid sequence identity, alternatively at least about 92% amino acid sequence identity, alternatively at least about 93% amino acid sequence identity, alternatively at least about 94% amino acid sequence identity, alternatively at least about 95% amino acid sequence identity, alternatively at least about 96% amino acid sequence identity, alternatively at least about 97% amino acid sequence identity, alternatively at least about 98% amino acid sequence identity and alternatively at least about 99% amino acid sequence identity to an amino acid sequence encoded by any of the human protein cDNAs deposited with the ATCC as disclosed herein.

In a specific aspect, the invention provides an isolated PRO polypeptide without the N-terminal signal sequence and/or the initiating methionine and is encoded by a nucleotide sequence that encodes such an amino acid sequence as hereinbefore described. Processes for producing the same are also herein described, wherein those processes comprise culturing a host cell comprising a vector which comprises the appropriate encoding nucleic acid molecule under conditions suitable for expression of the PRO polypeptide and recovering the PRO polypeptide from the cell culture.

Another aspect the invention provides an isolated PRO polypeptide which is either transmembrane domain-deleted or transmembrane domain-inactivated. Processes for producing the same are also herein described, wherein those processes comprise culturing a host cell comprising a vector which comprises the appropriate encoding nucleic acid molecule under conditions suitable for expression of the PRO polypeptide and recovering the PRO polypeptide from the cell culture.

In yet another embodiment, the invention concerns agonists and antagonists of a native PRO polypeptide as defined herein. In a particular embodiment, the agonist or antagonist is an anti-PRO antibody or a small molecule.

In a further embodiment, the invention concerns a method of identifying agonists or antagonists to a PRO polypeptide which comprise contacting the PRO polypeptide with a candidate molecule and monitoring a biological activity mediated by said PRO polypeptide. Preferably, the PRO polypeptide is a native PRO polypeptide.

In a still further embodiment, the invention concerns a composition of matter comprising a PRO polypeptide, or an agonist or antagonist of a PRO polypeptide as herein described, or an anti-PRO antibody, in combination with a carrier. Optionally, the carrier is a pharmaceutically acceptable carrier.

Another embodiment of the present invention is directed to the use of a PRO polypeptide, or an agonist or antagonist thereof as hereinbefore described, or an anti-PRO antibody, for the preparation of a medicament useful in the treatment of a condition which is responsive to the PRO polypeptide, an agonist or antagonist thereof or an anti-PRO antibody.

In other embodiments of the present invention, the invention provides vectors comprising DNA encoding any of the herein described polypeptides. Host cell comprising any such vector are also provided. By way of example, the host cells may be CHO cells, E. *coli,* or yeast. A process for producing any of the herein described polypeptides is further provided and comprises culturing host cells under conditions suitable for expression of the desired polypeptide and recovering the desired polypeptide from the cell culture.

In other embodiments, the invention provides chimeric molecules comprising any of the herein described polypeptides fused to a heterologous polypeptide or amino acid sequence. Example of such chimeric molecules comprise any of the herein described polypeptides fused to an epitope tag sequence or a Fc region of an immunoglobulin.

In another embodiment, the invention provides an antibody which binds, preferably specifically, to any of the above or below described polypeptides. Optionally, the antibody is a monoclonal antibody, humanized antibody, antibody fragment or single-chain antibody.

In yet other embodiments, the invention provides oligonucleotide probes which may be useful for isolating genomic and cDNA nucleotide sequences, measuring or detecting expression of an associated gene or as antisense probes, wherein those probes may be derived from any of the above or below described nucleotide sequences. Preferred probe lengths are described above.

In yet other embodiments, the present invention is directed to methods of using the PRO polypeptides of the present invention for a variety of uses based upon the functional biological assay data presented in the Examples below.

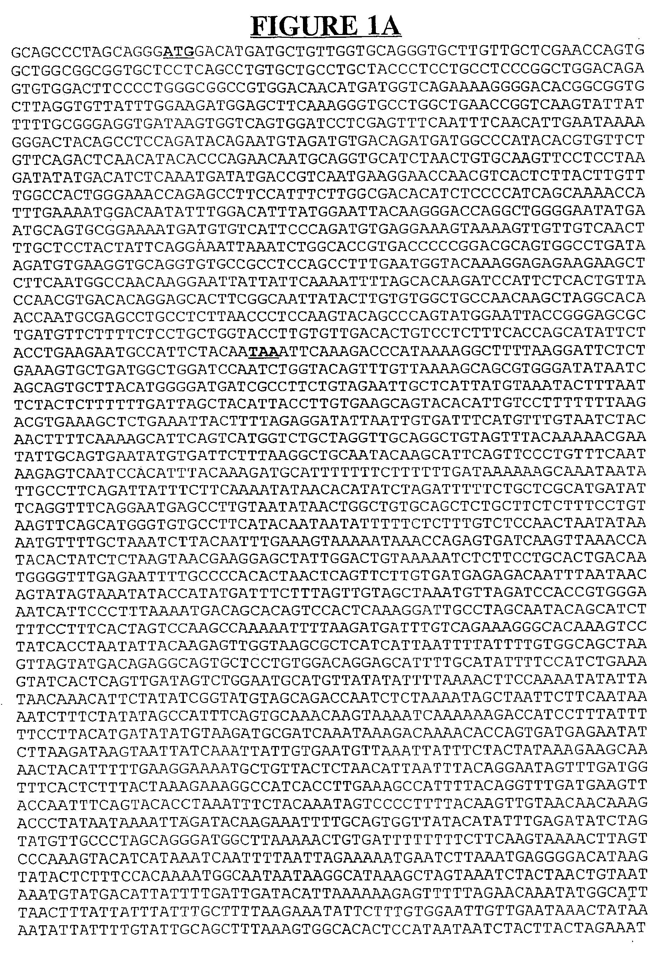

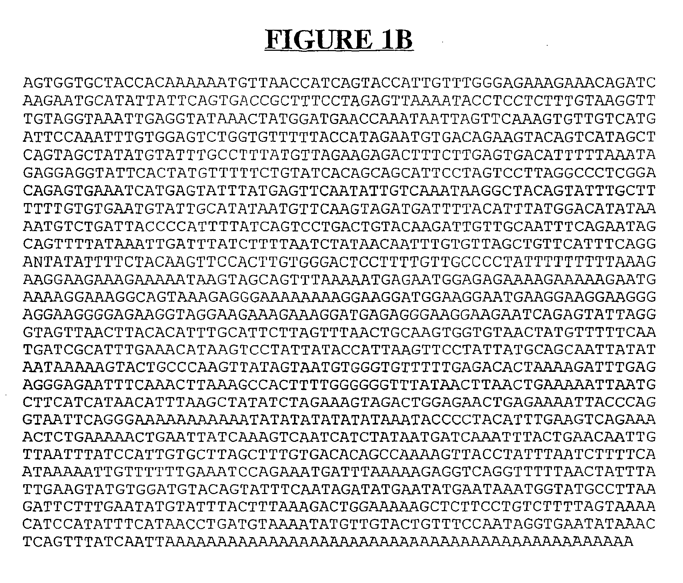

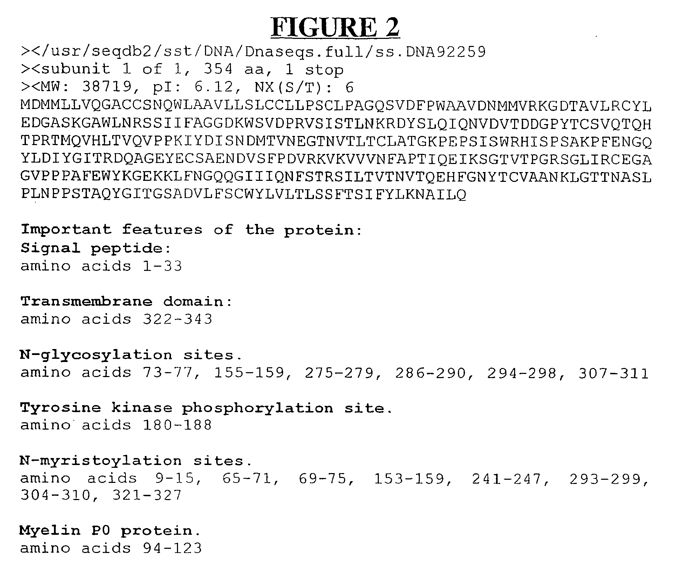

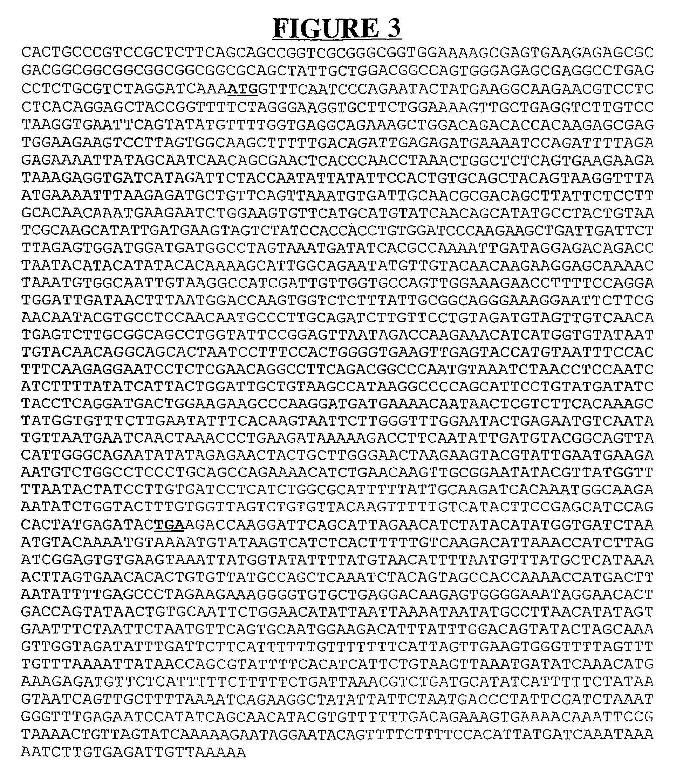

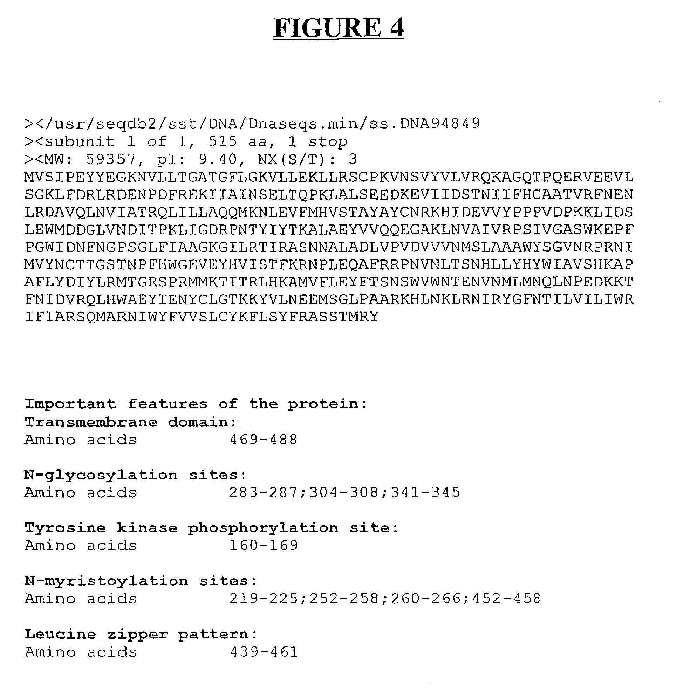

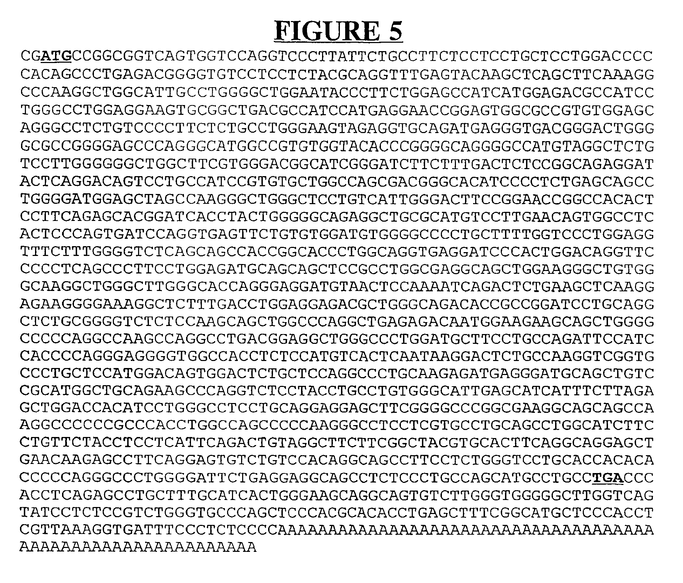

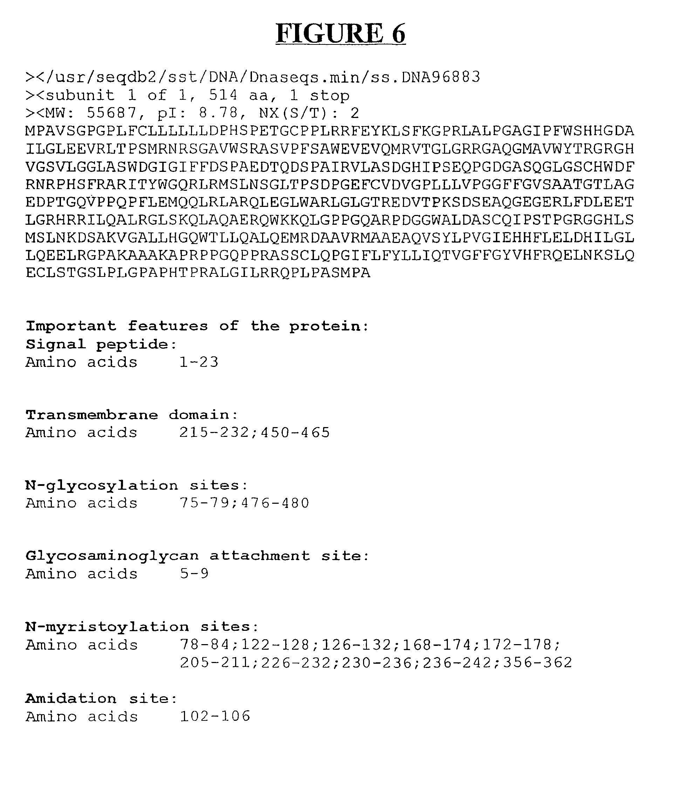

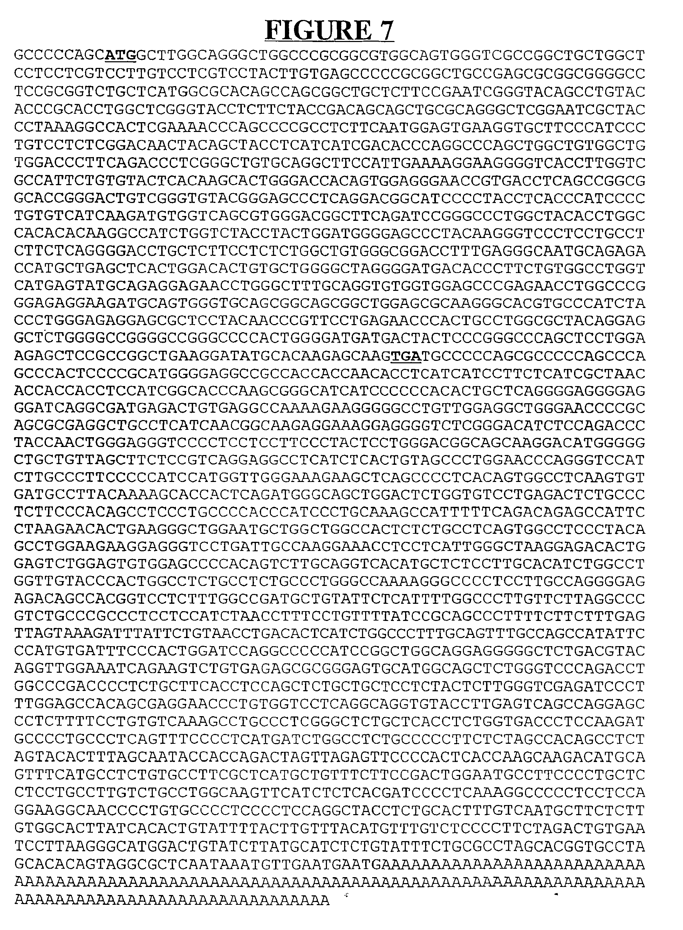

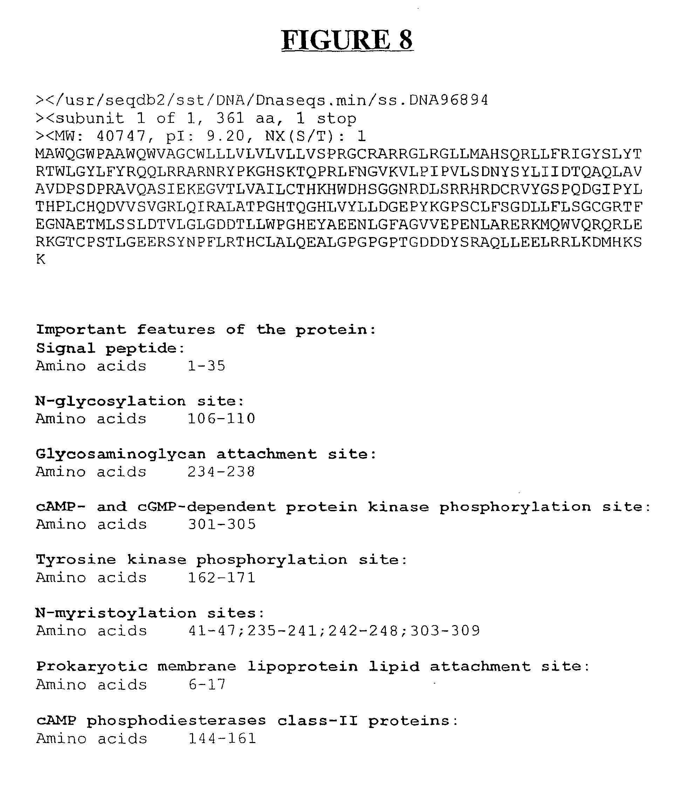

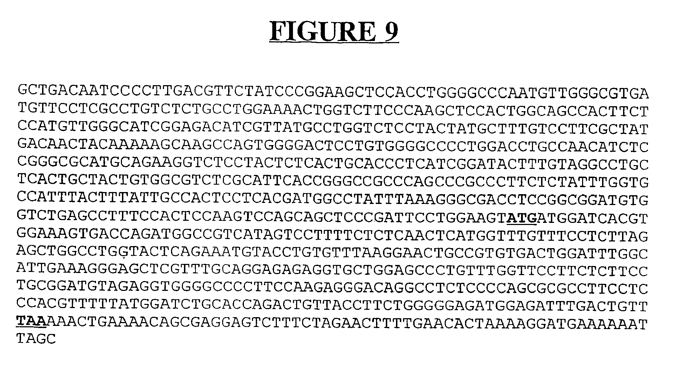

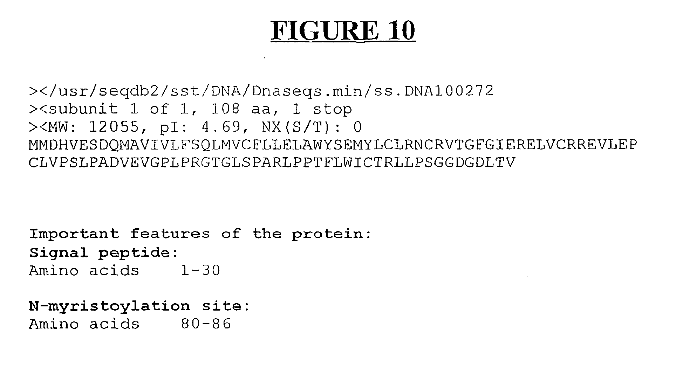

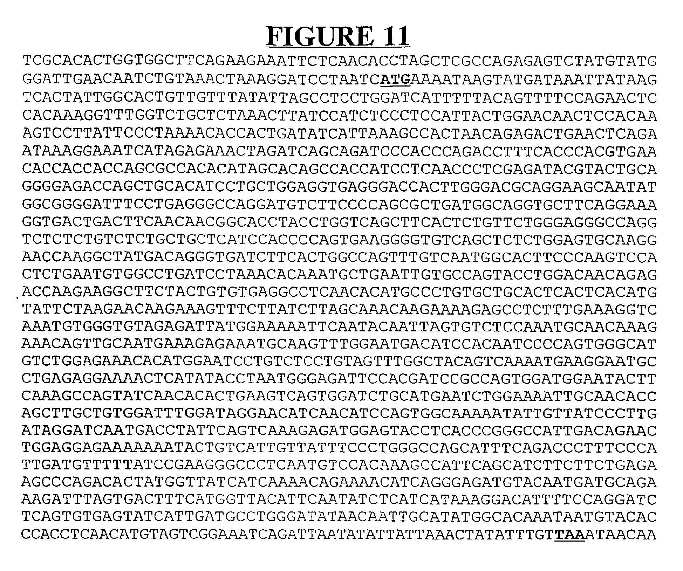

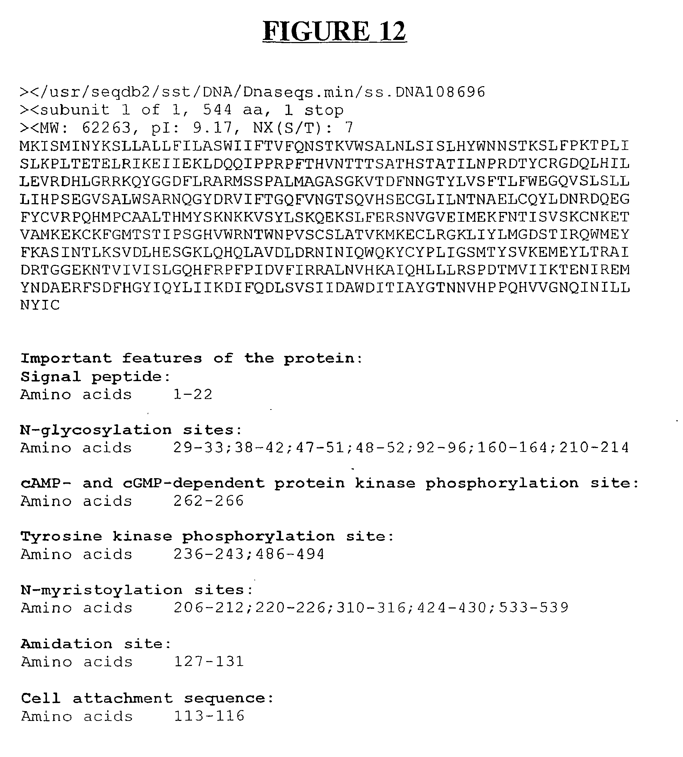

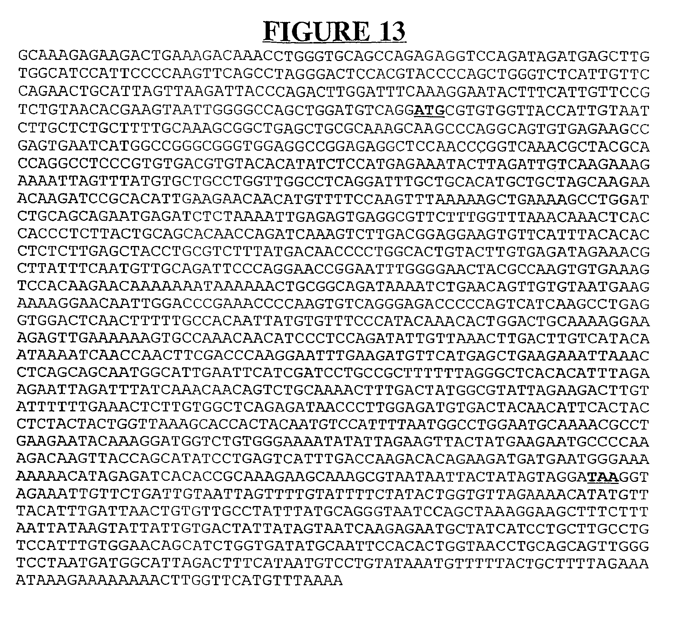

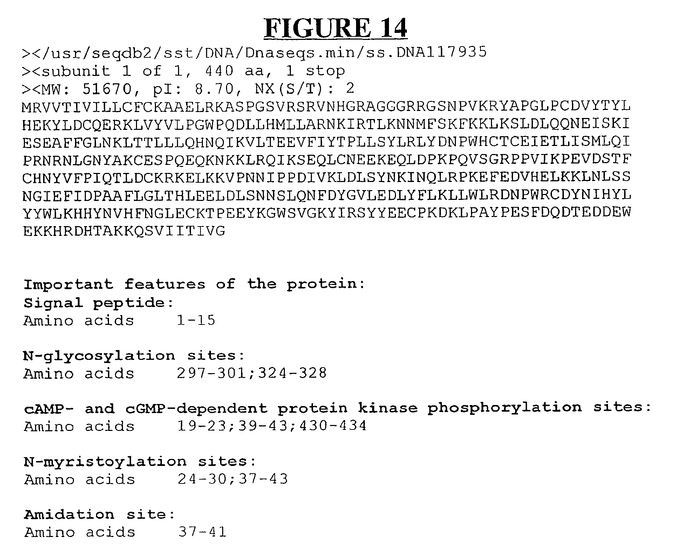

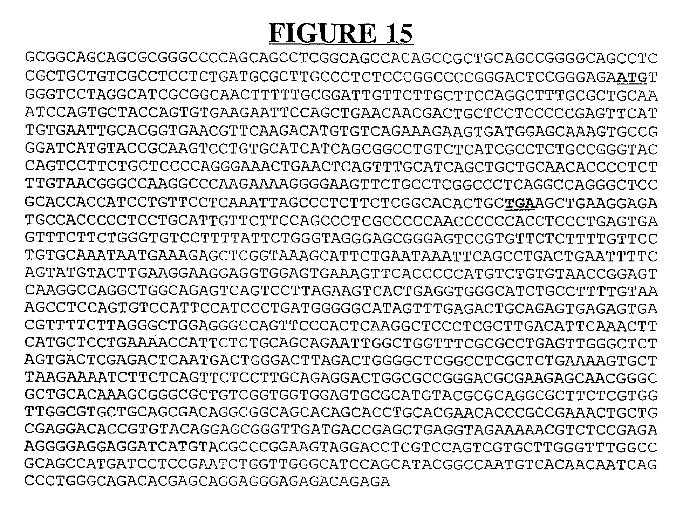

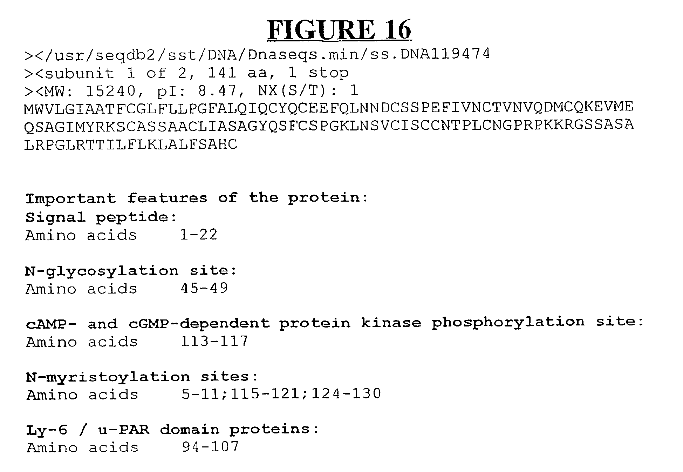

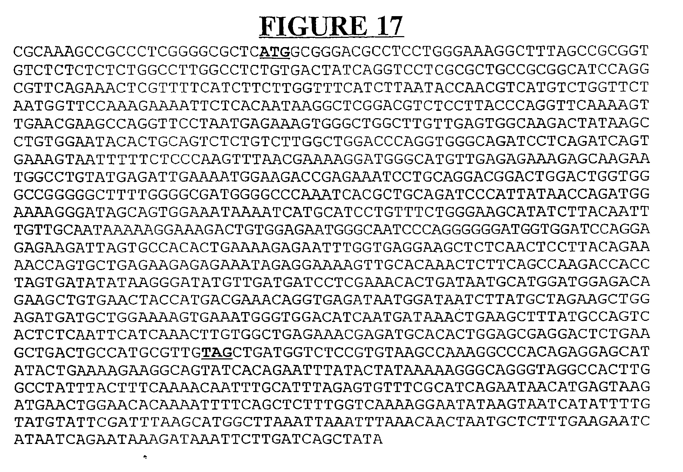

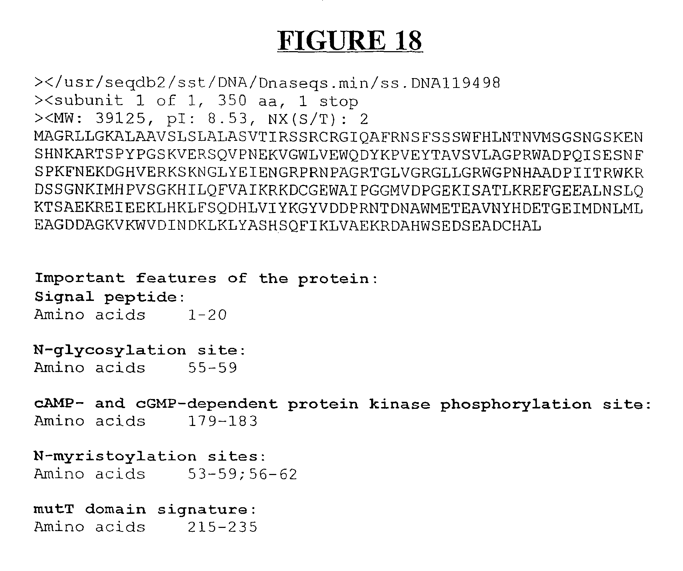

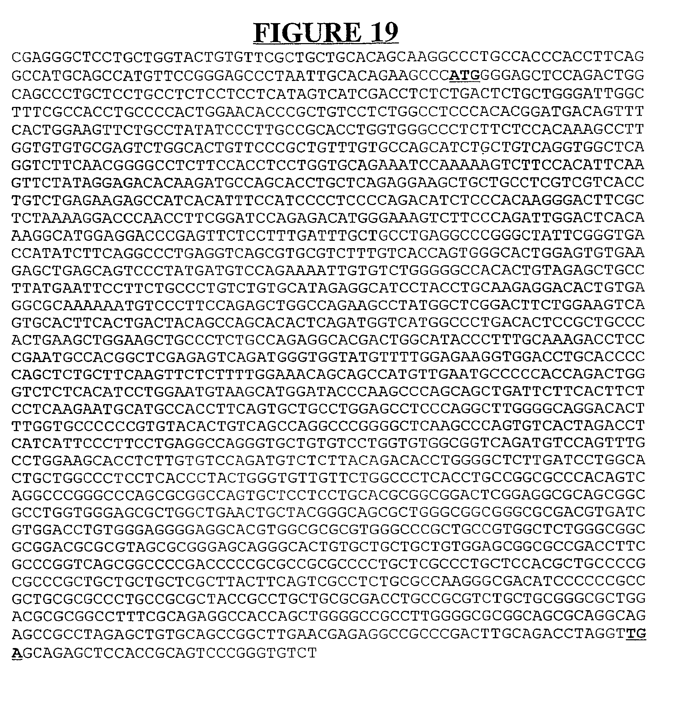

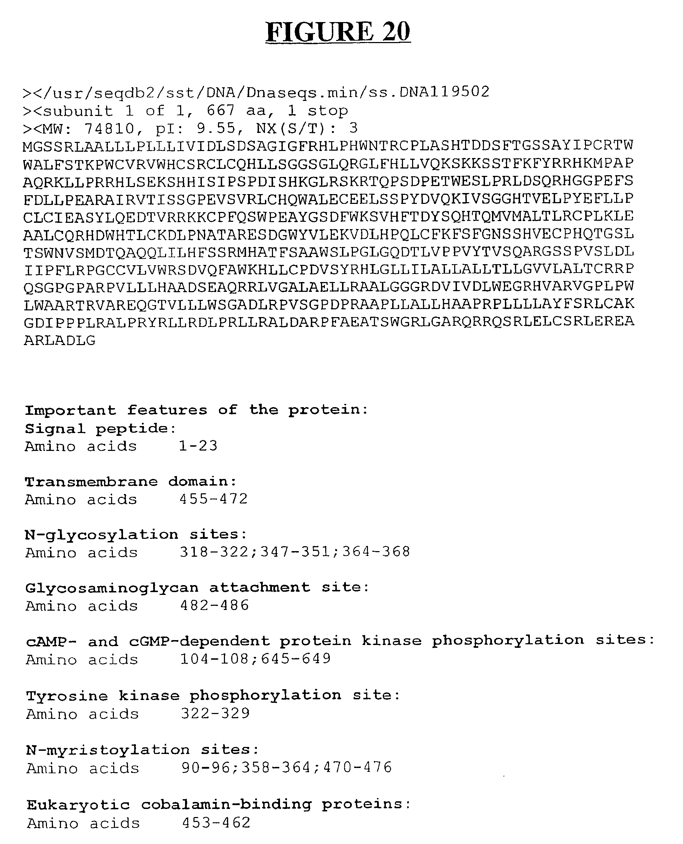

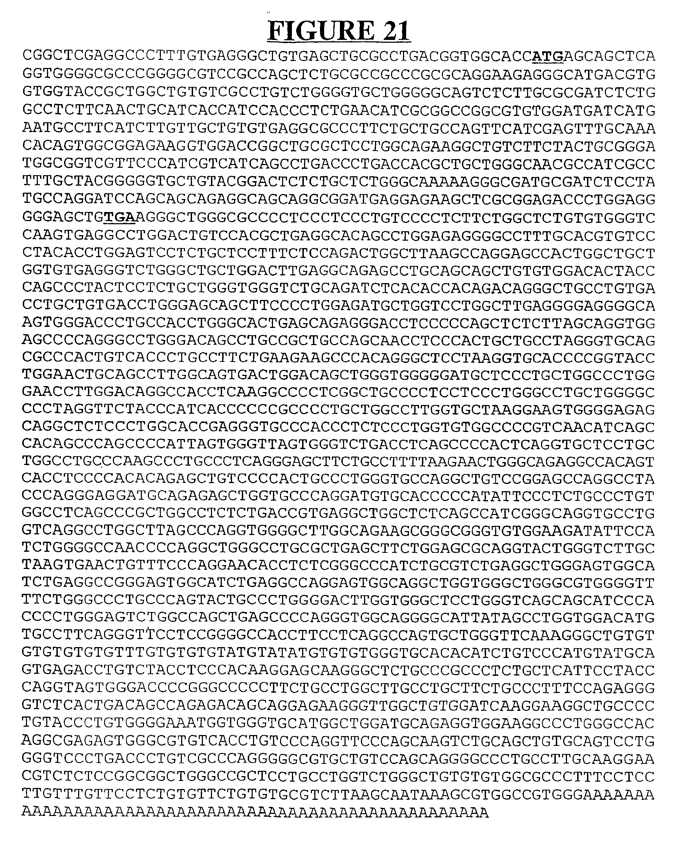

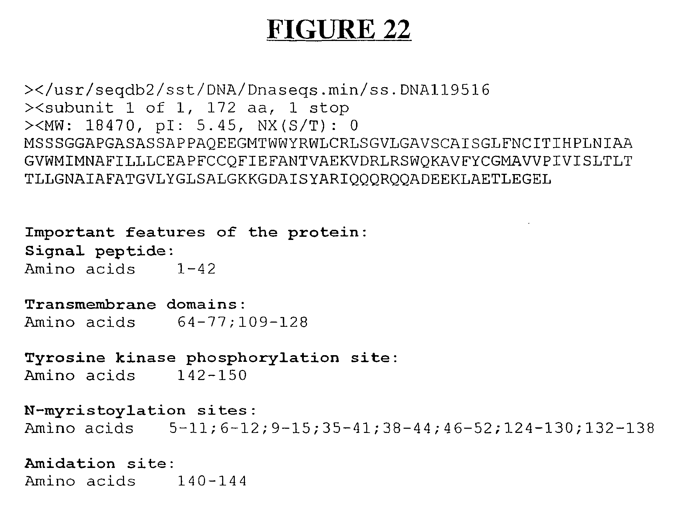

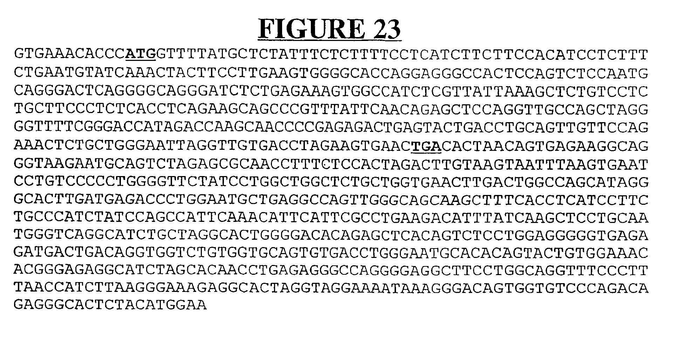

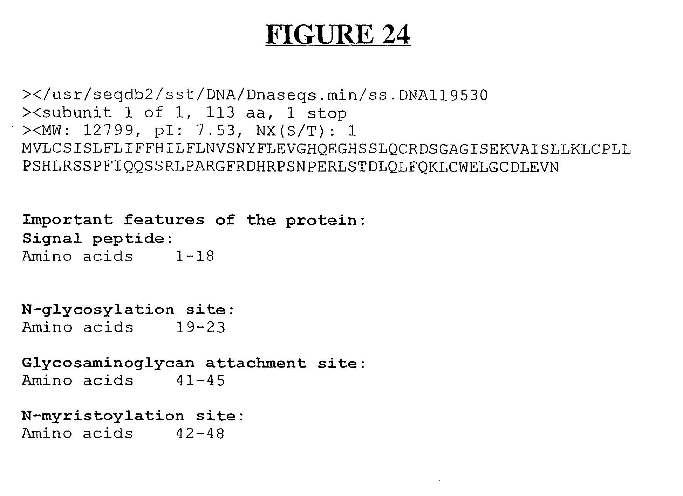

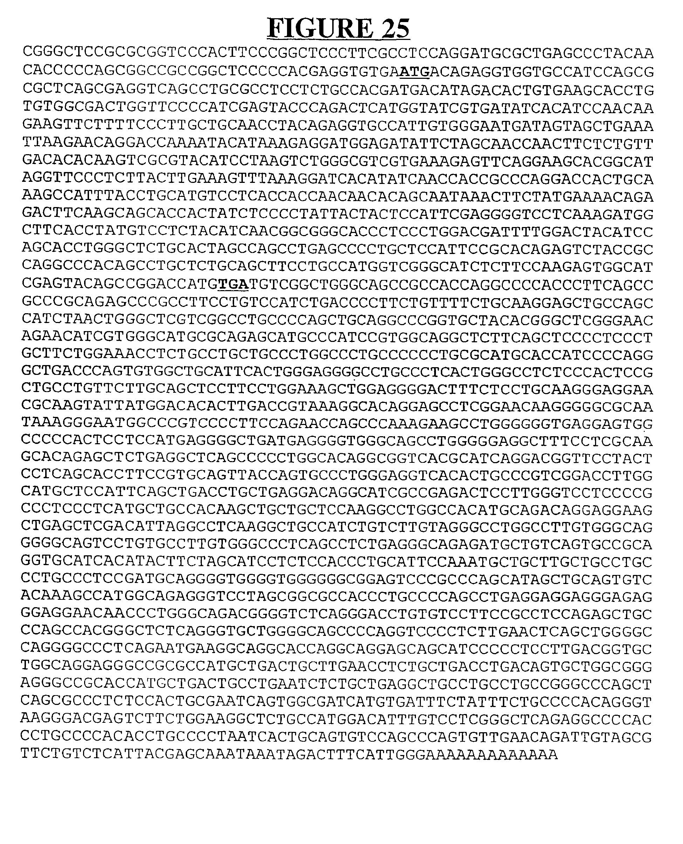

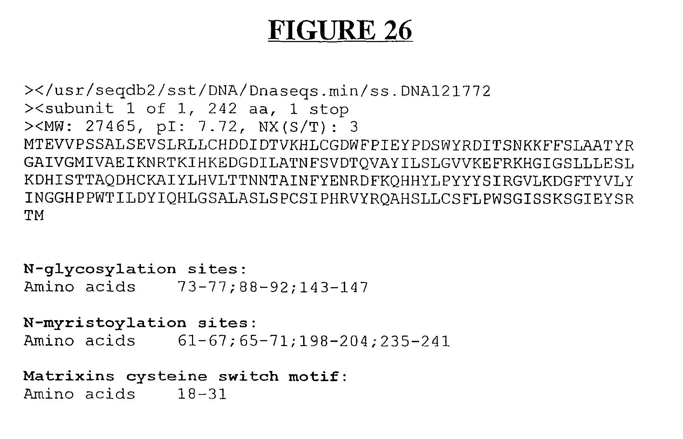

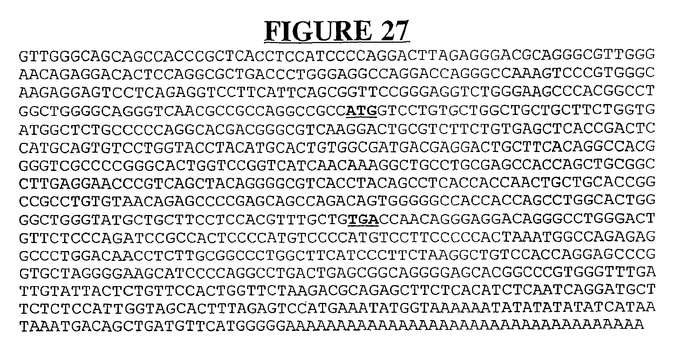

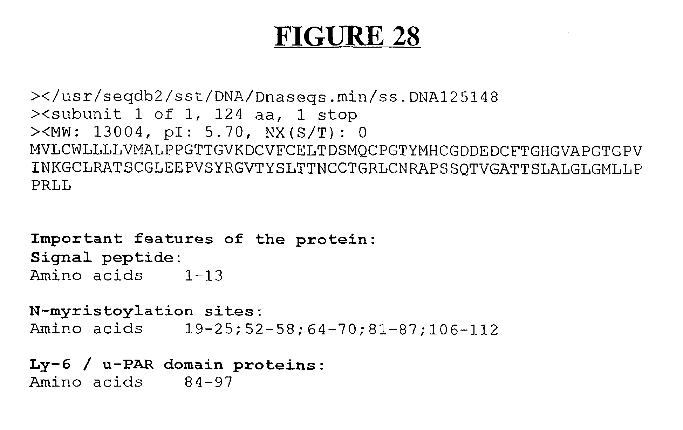

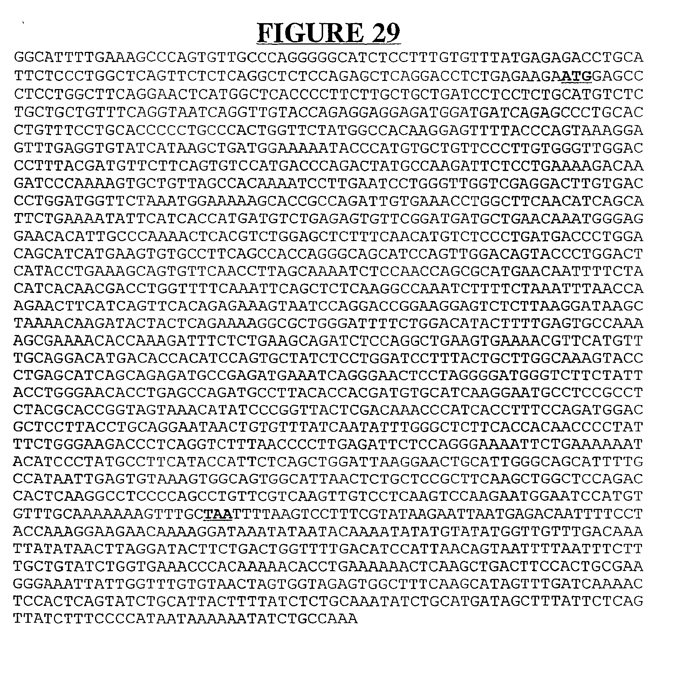

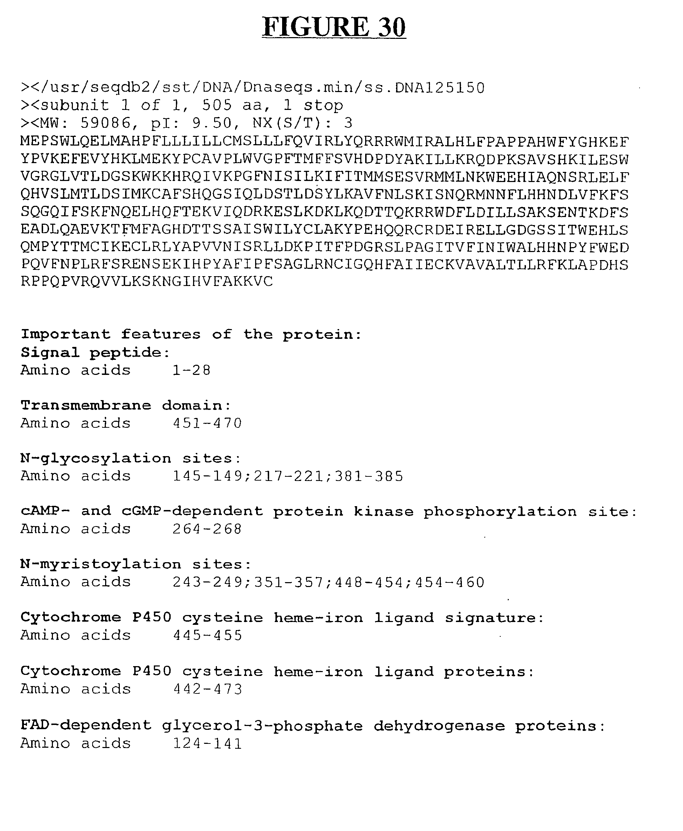

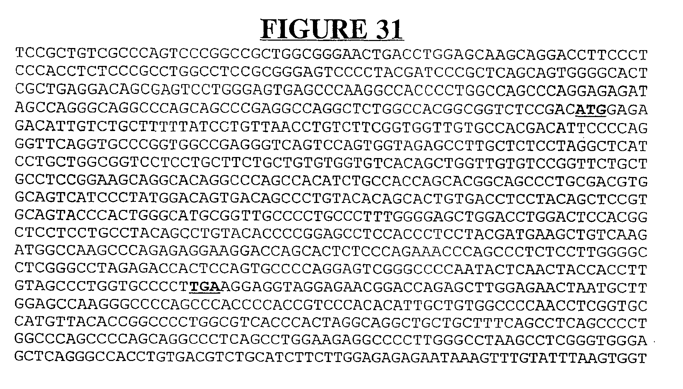

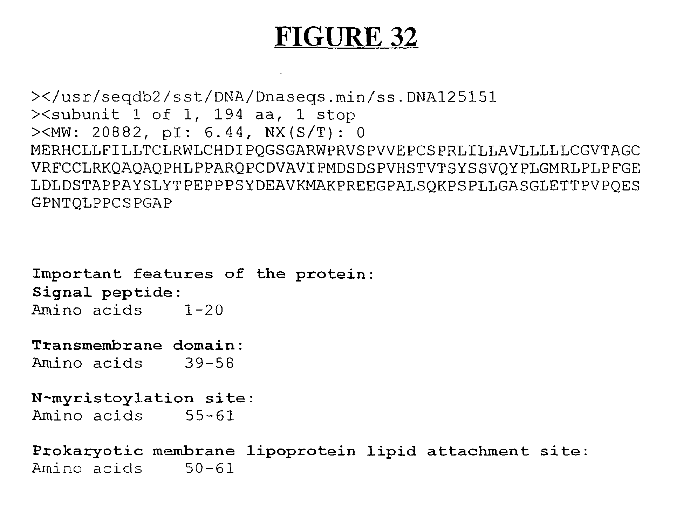

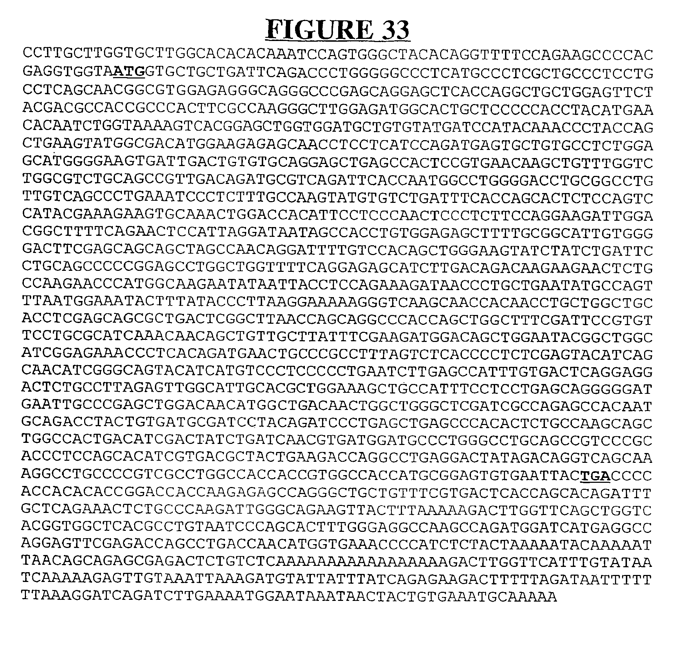

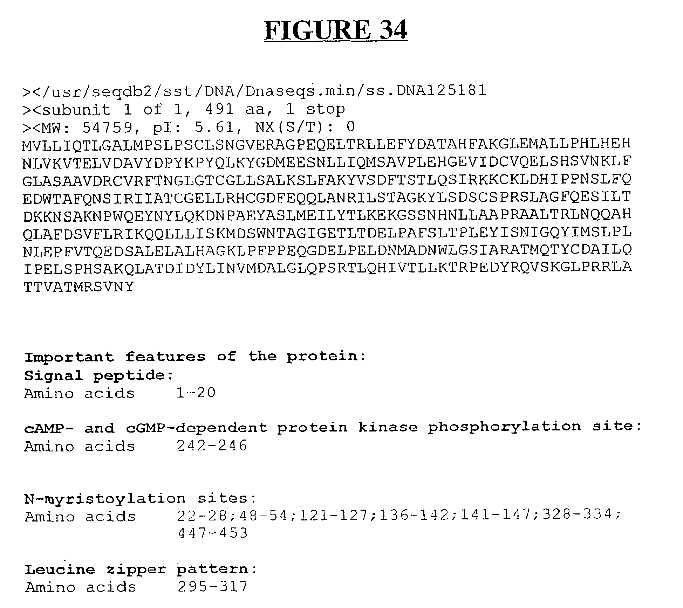

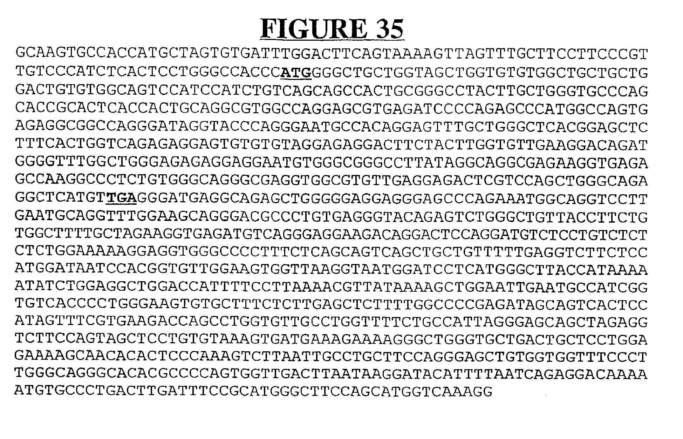

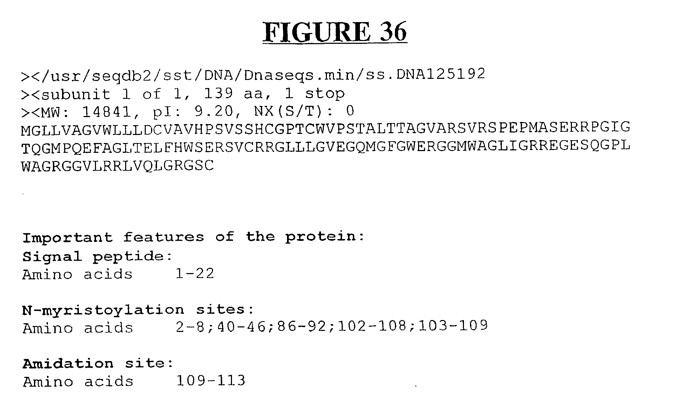

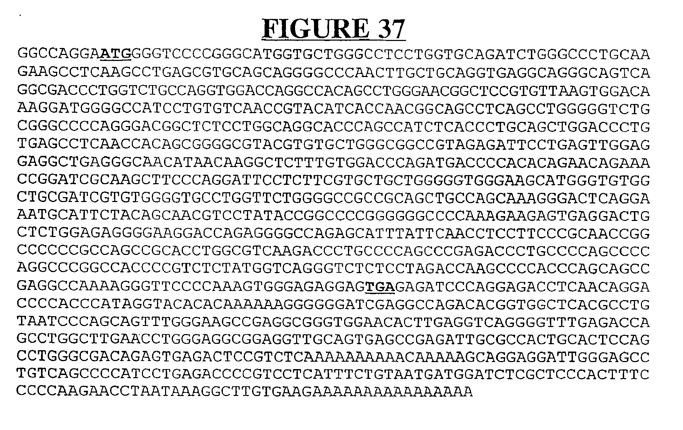

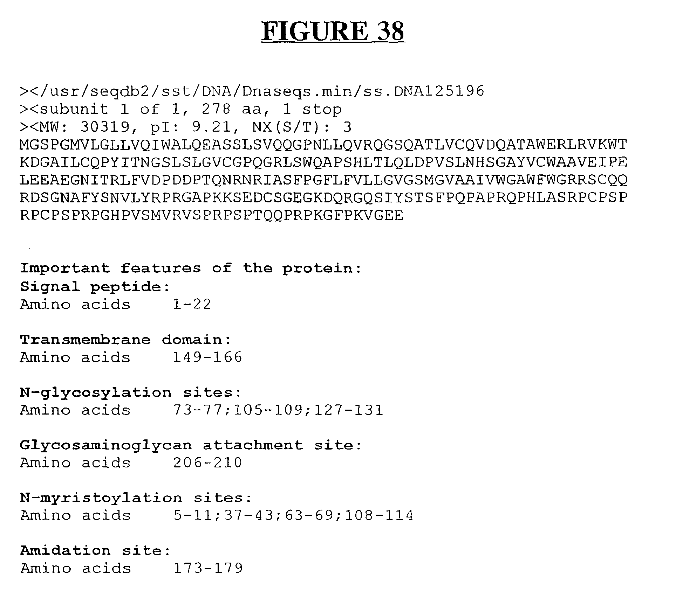

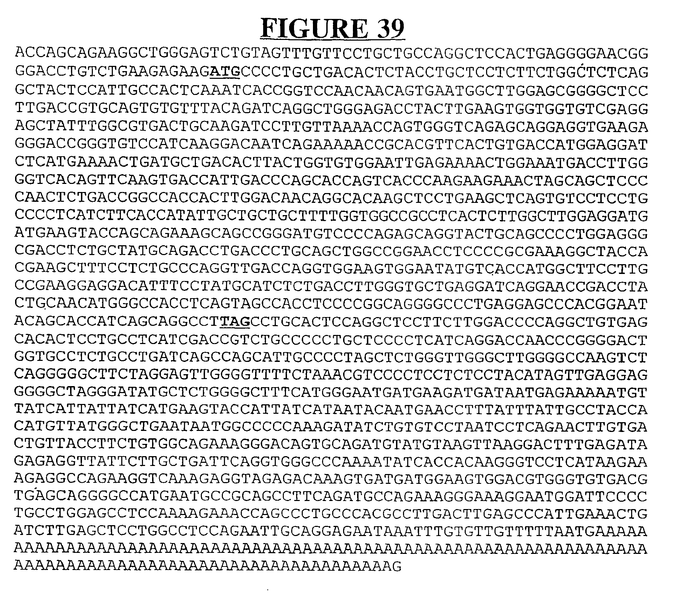

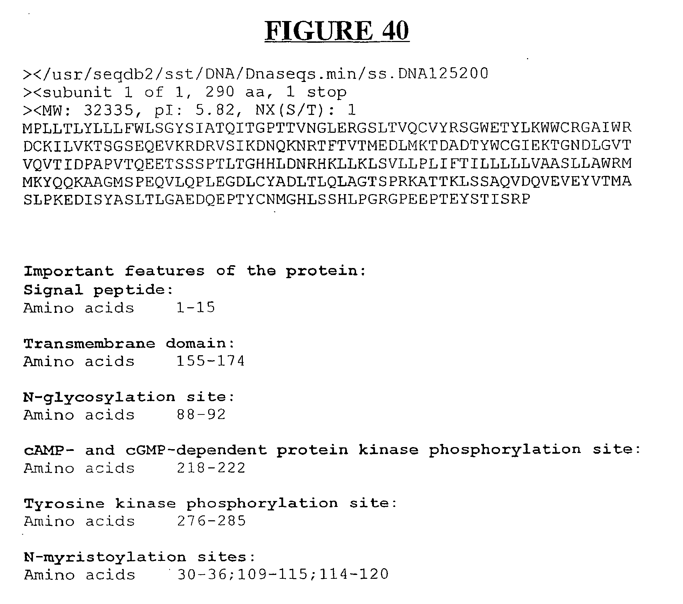

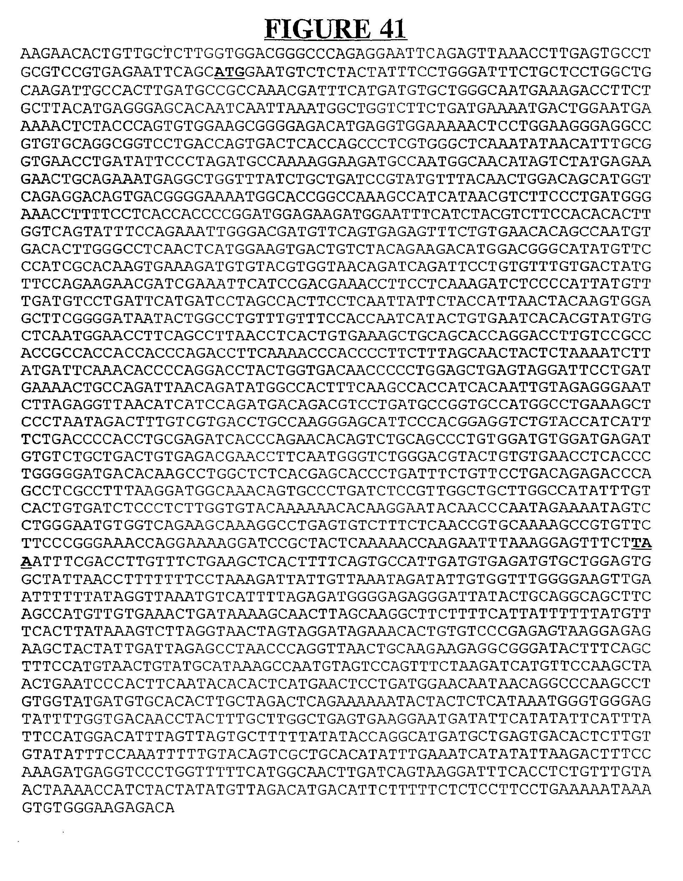

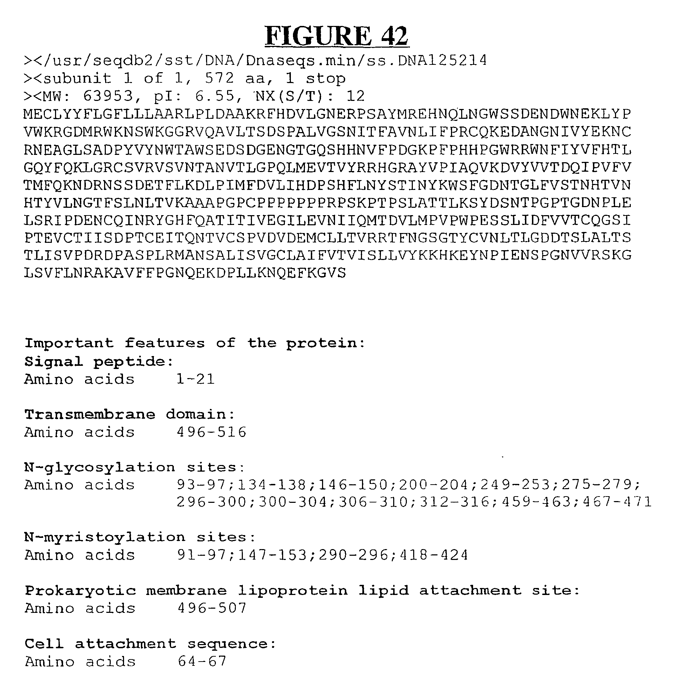

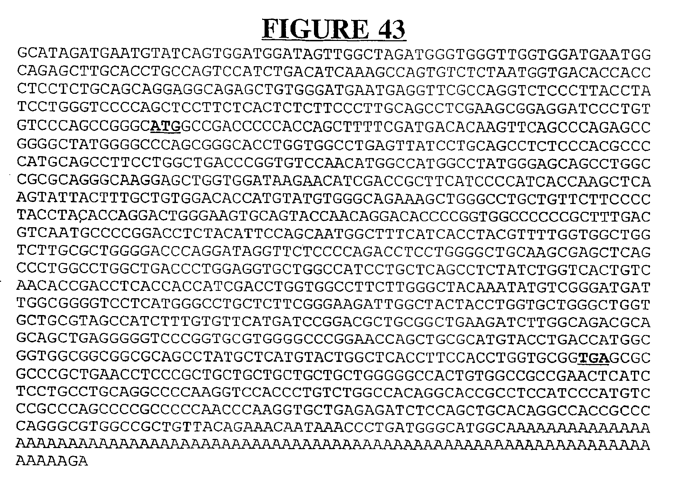

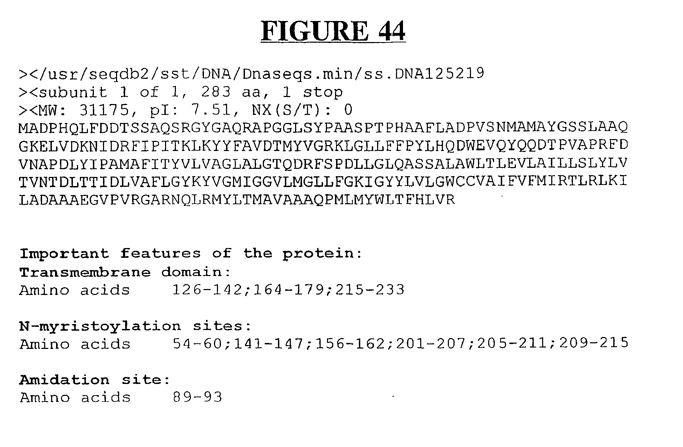

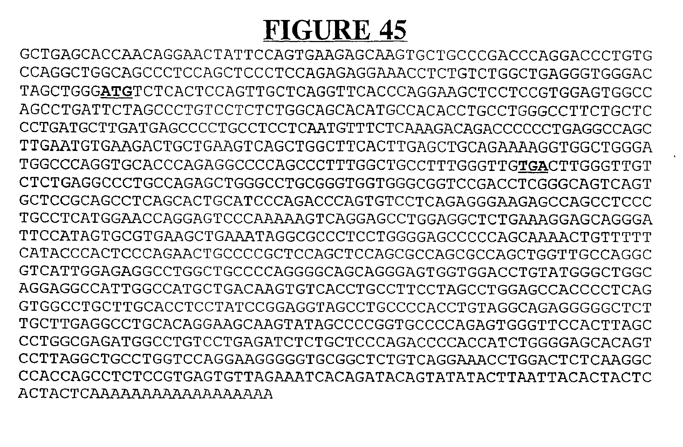

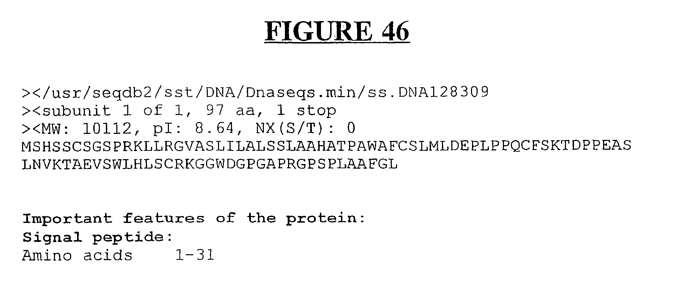

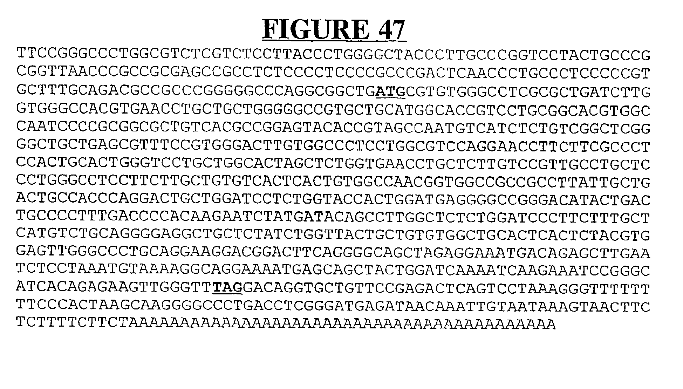

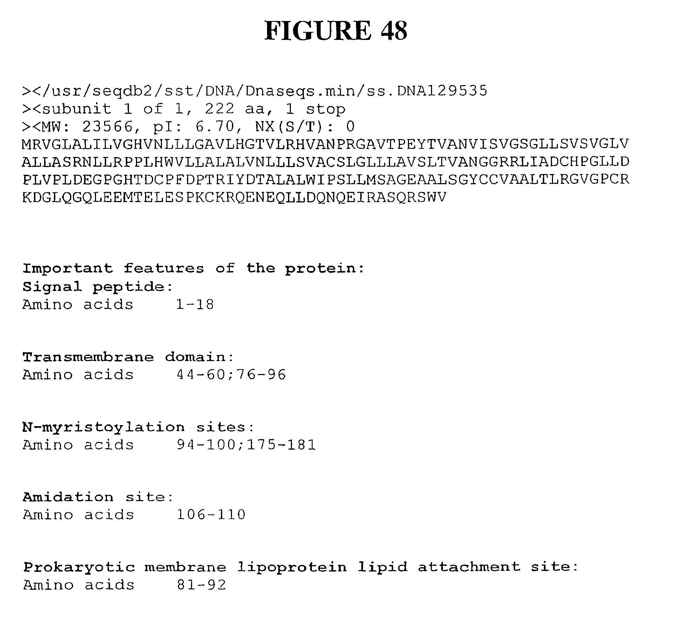

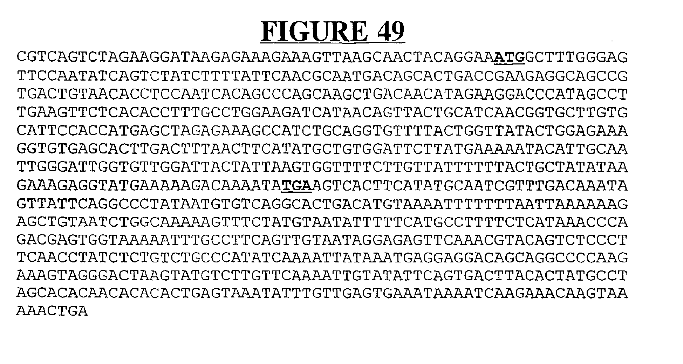

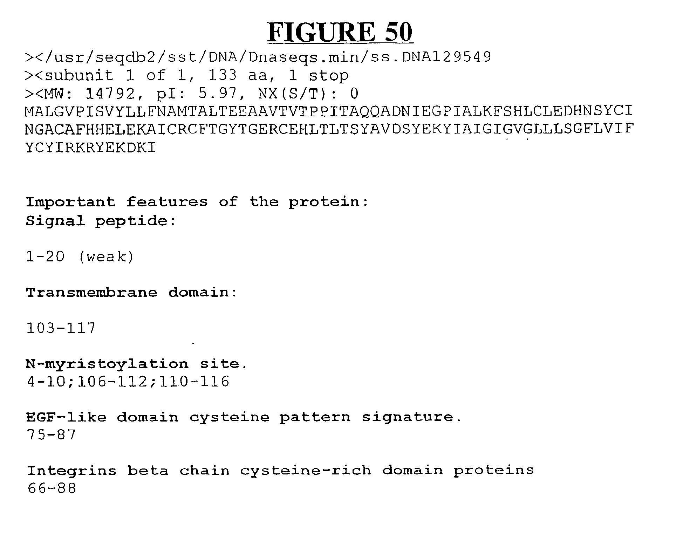

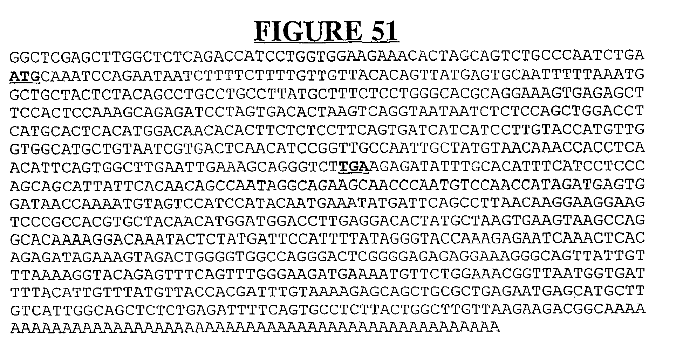

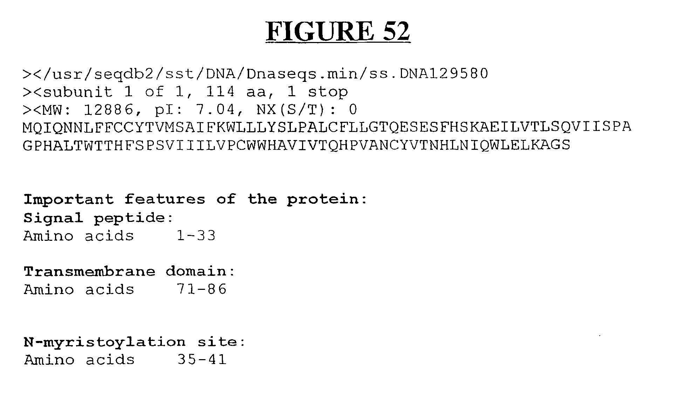

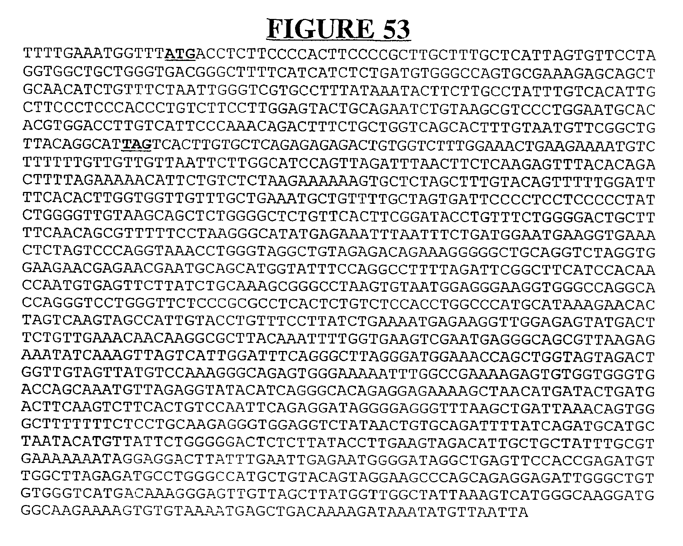

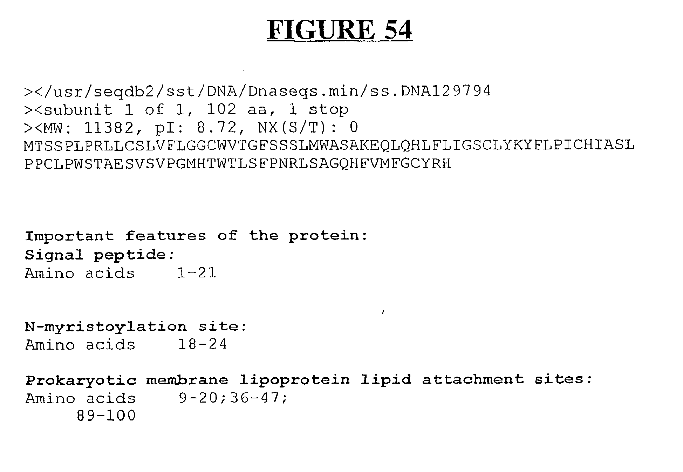

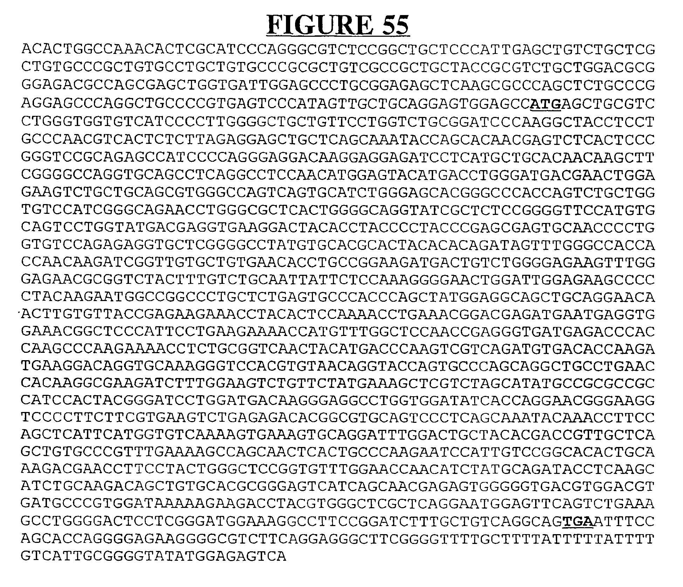

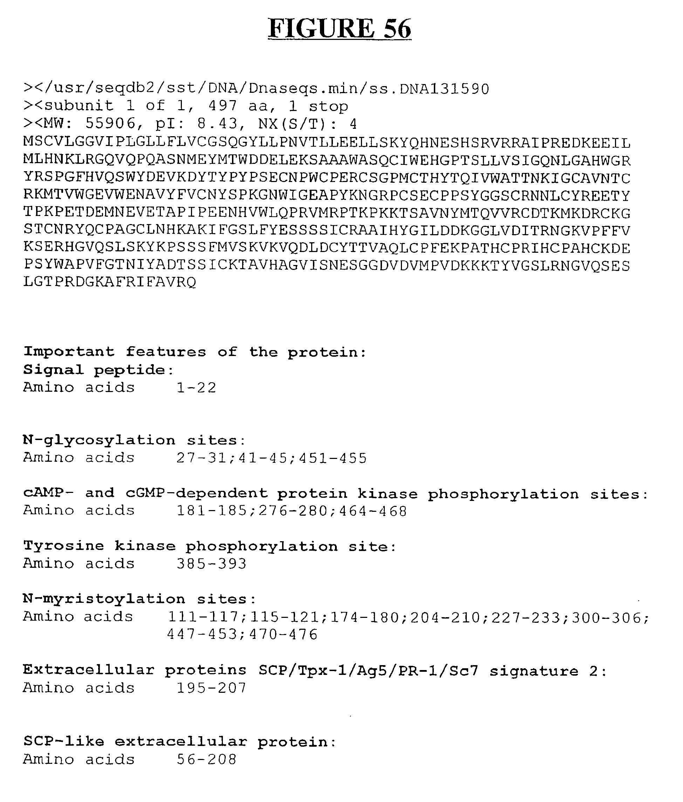

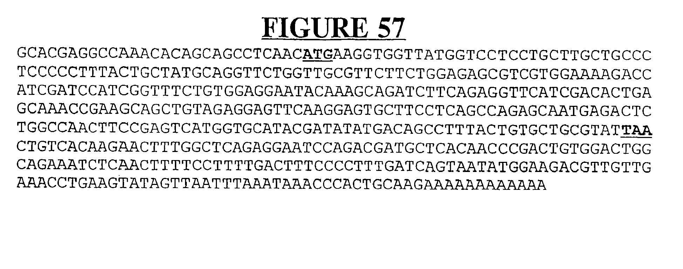

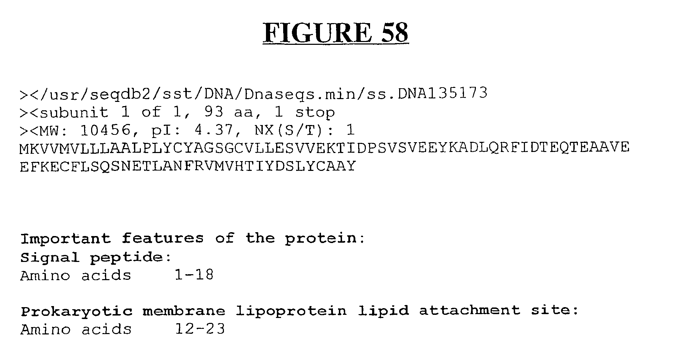

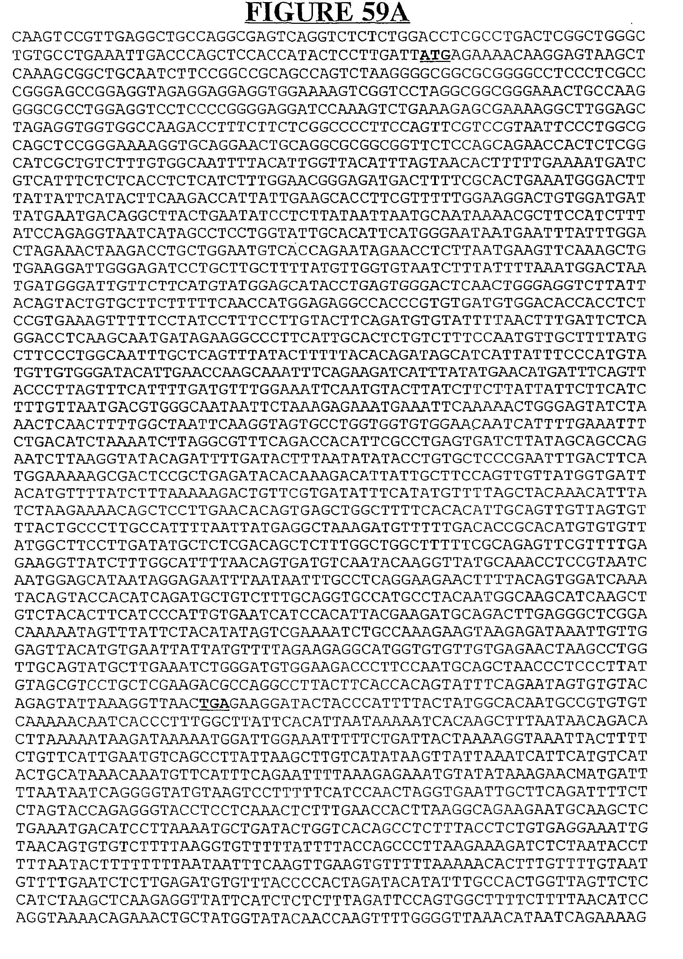

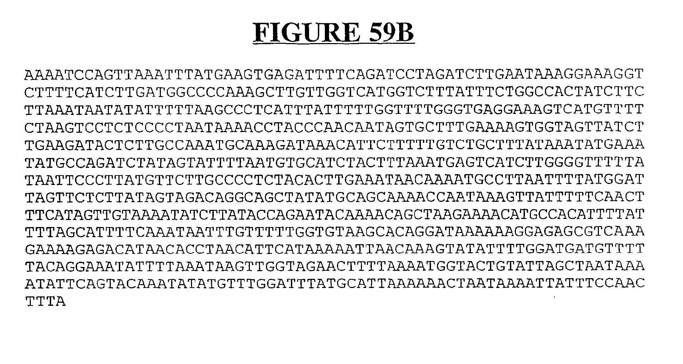

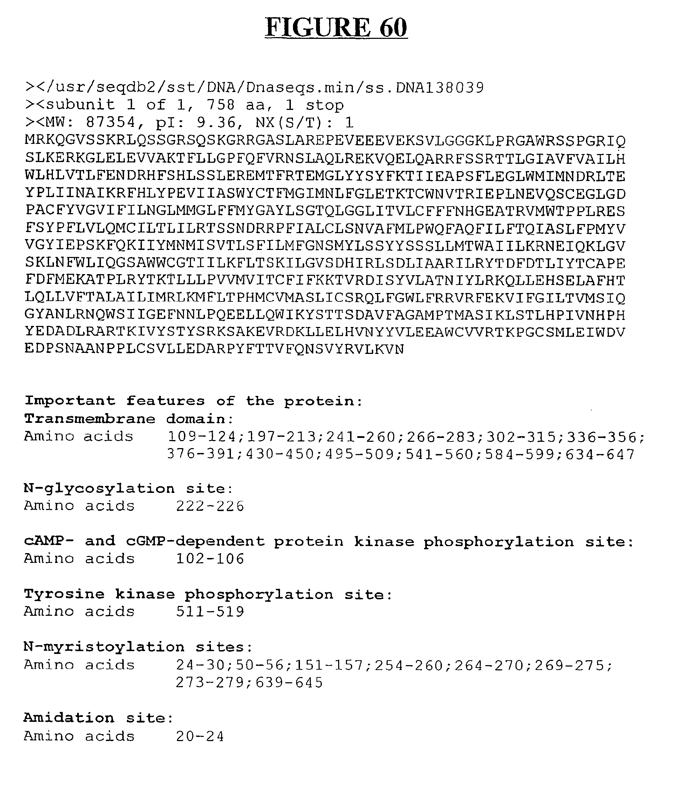

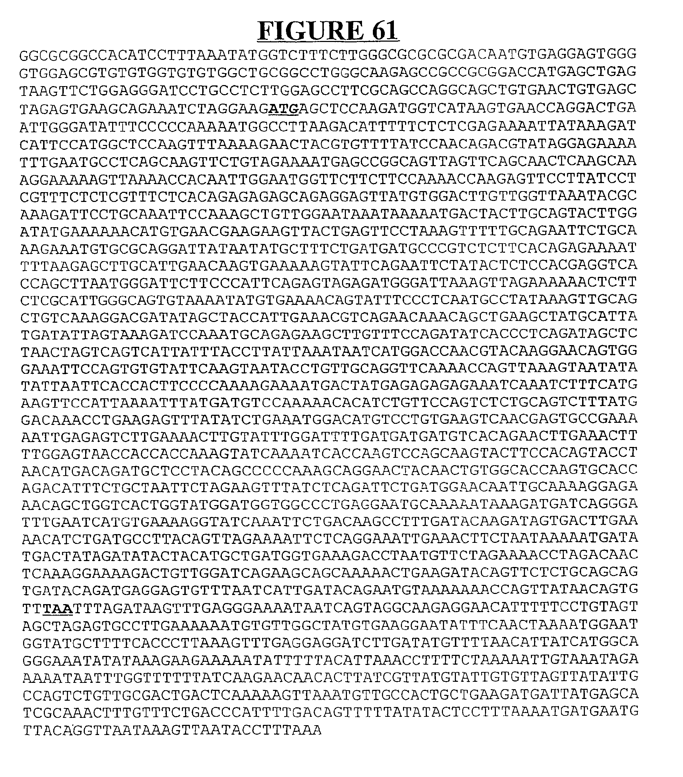

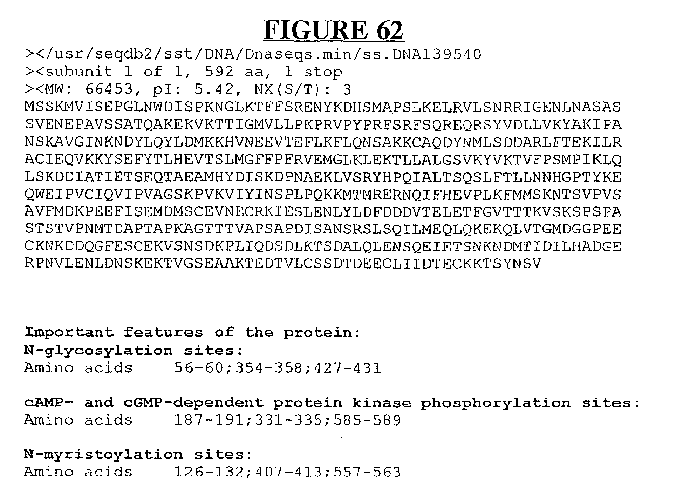

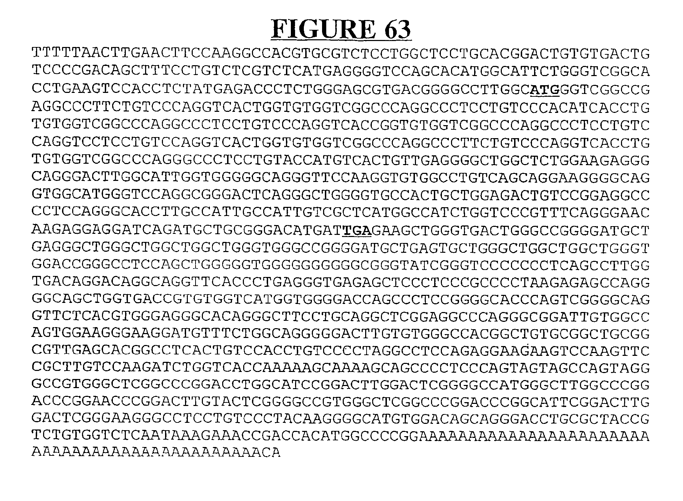

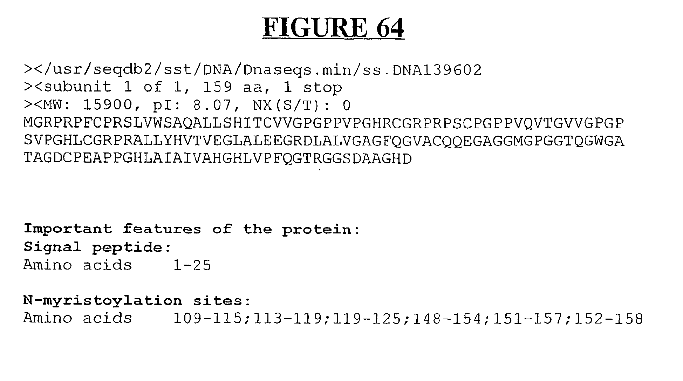

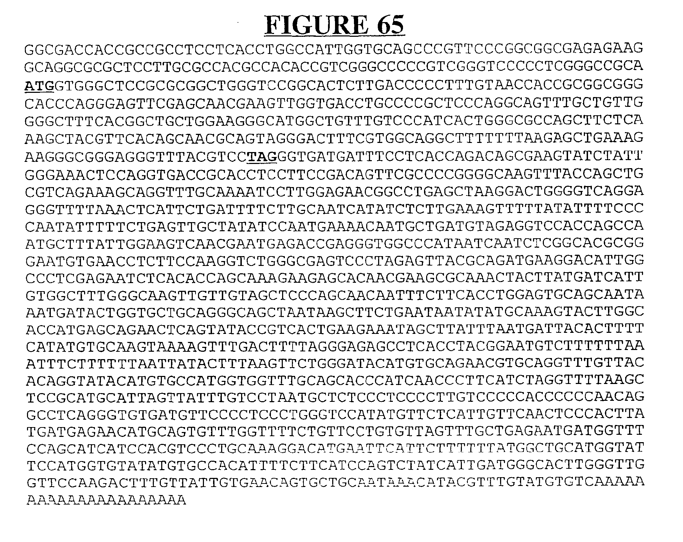

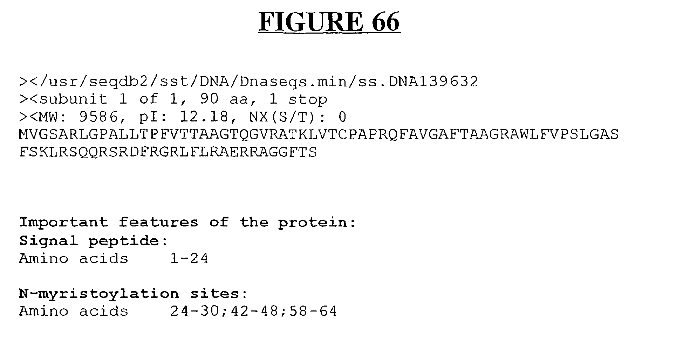

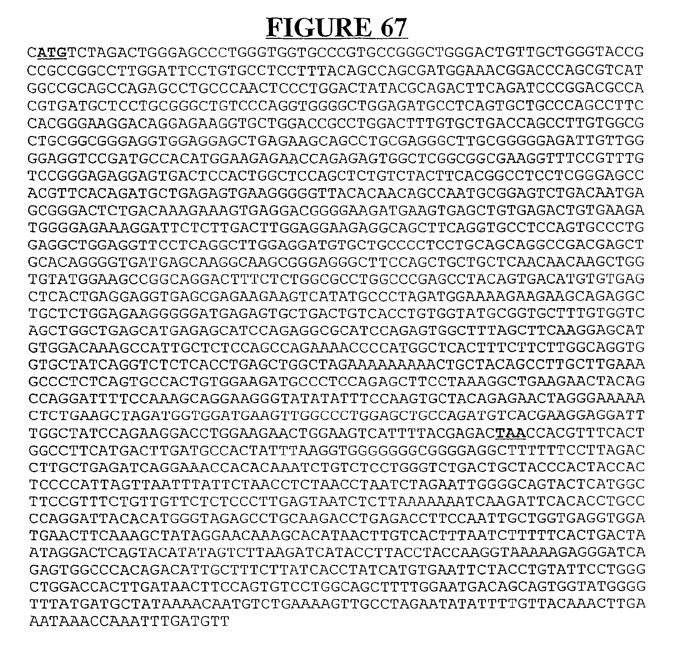

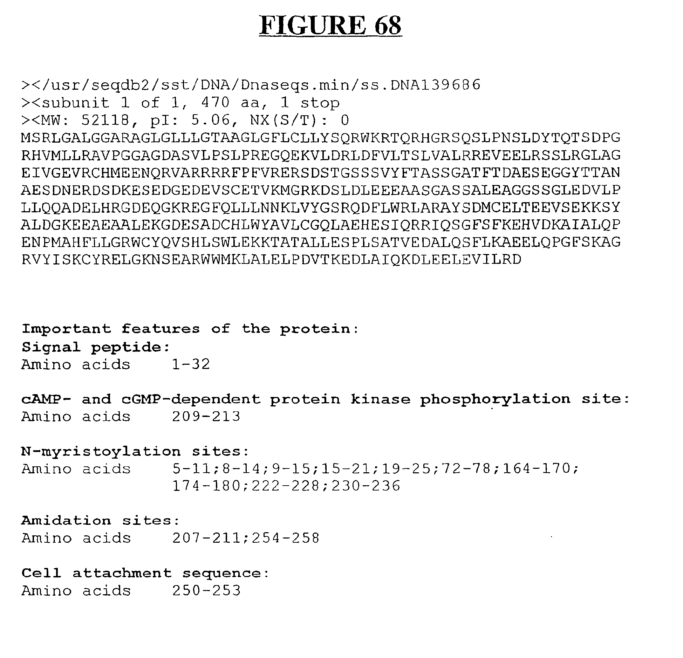

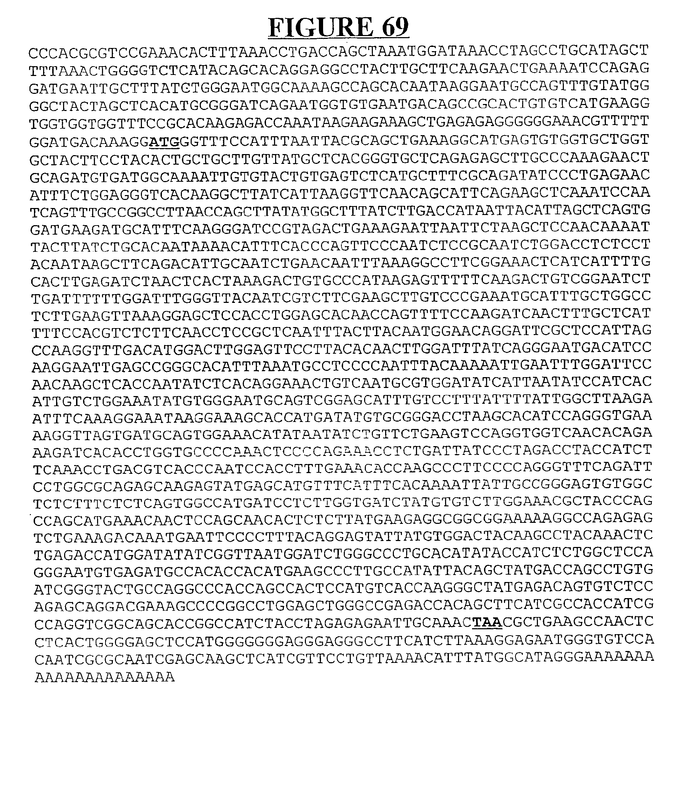

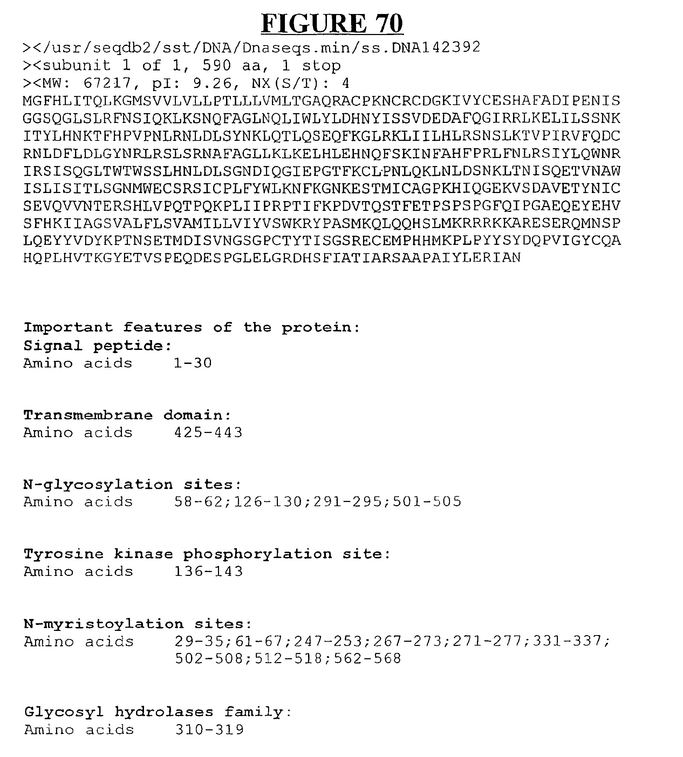

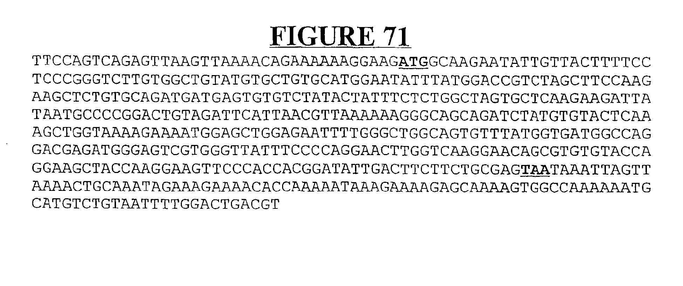

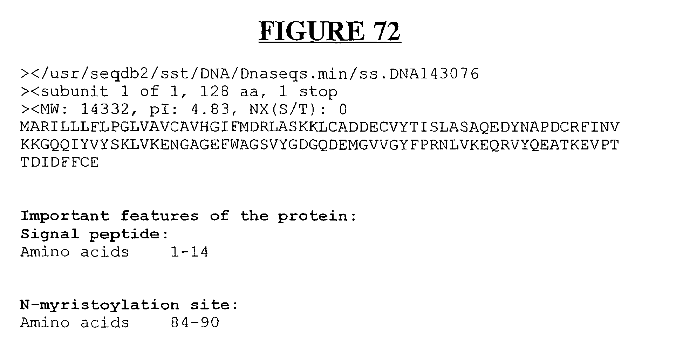

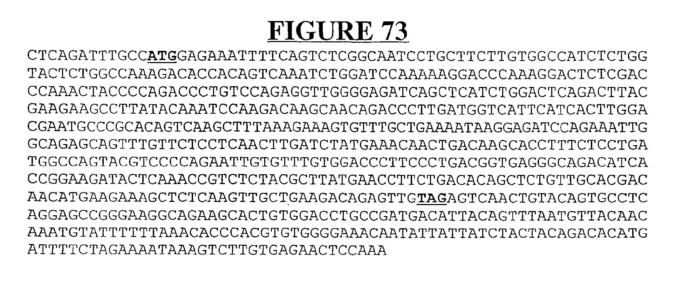

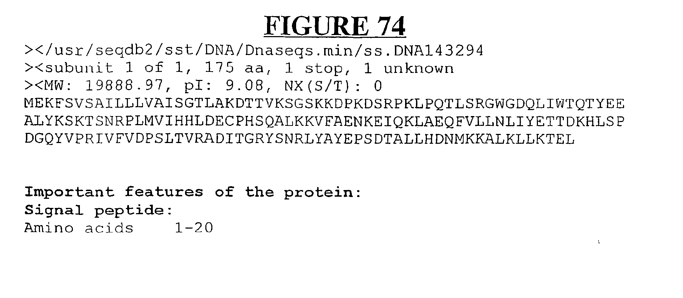

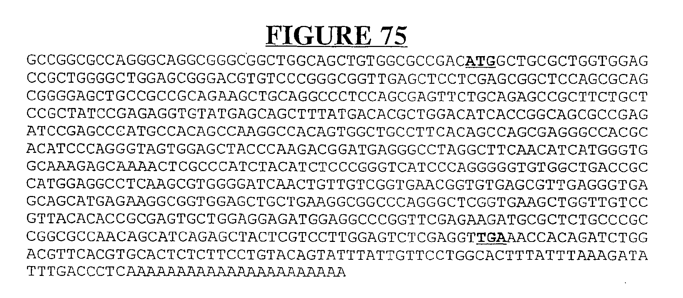

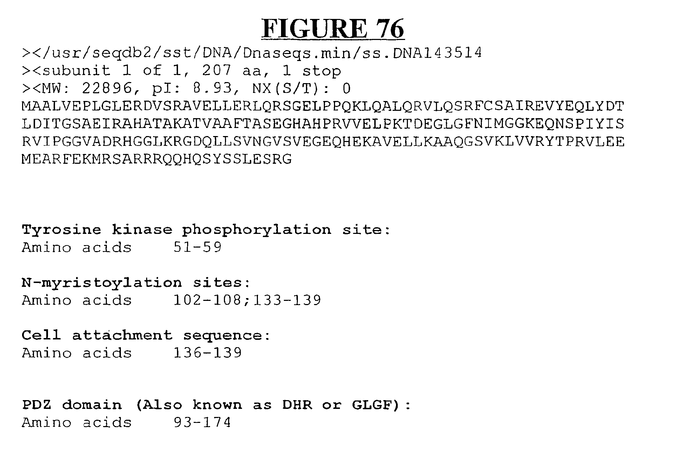

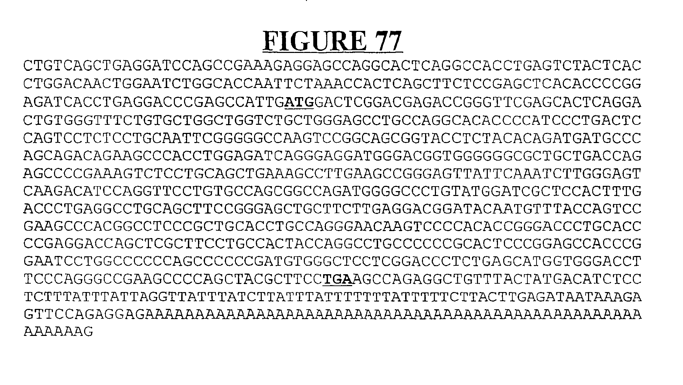

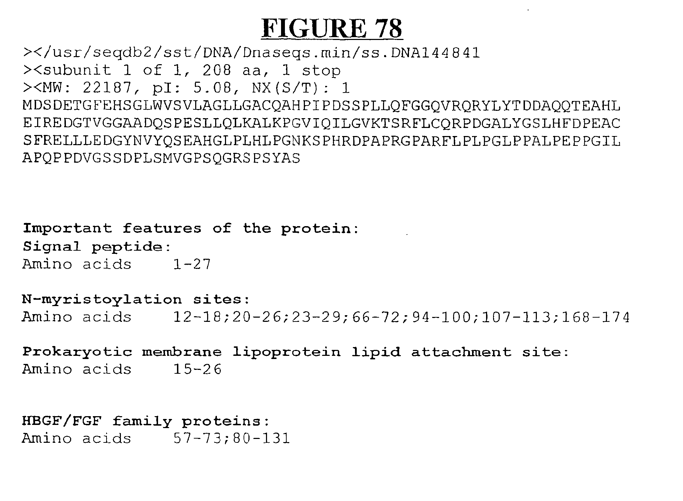

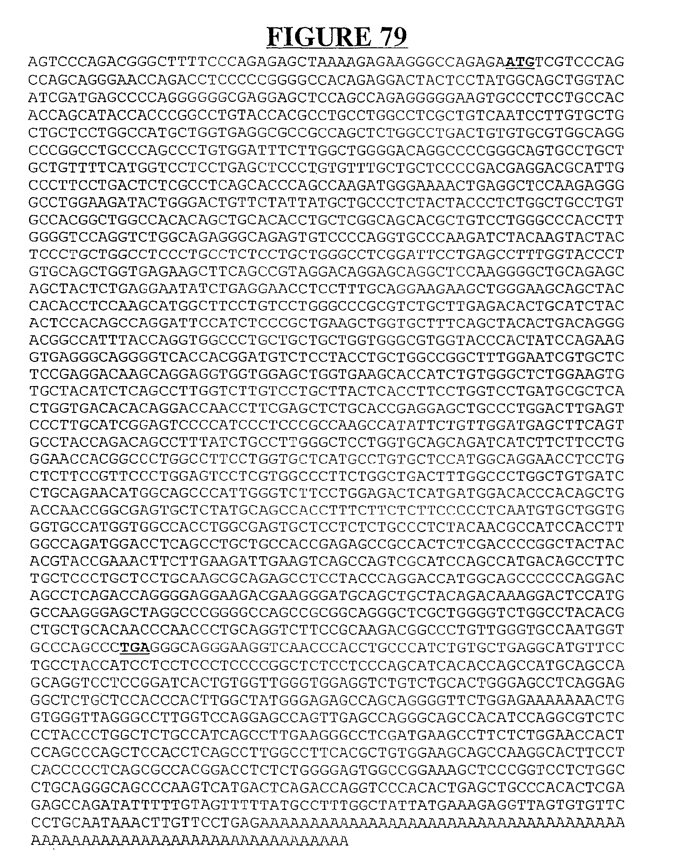

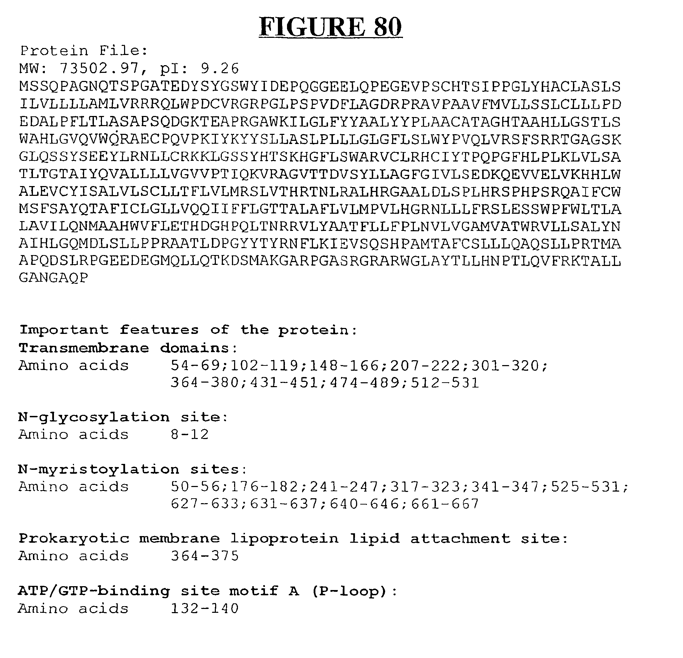

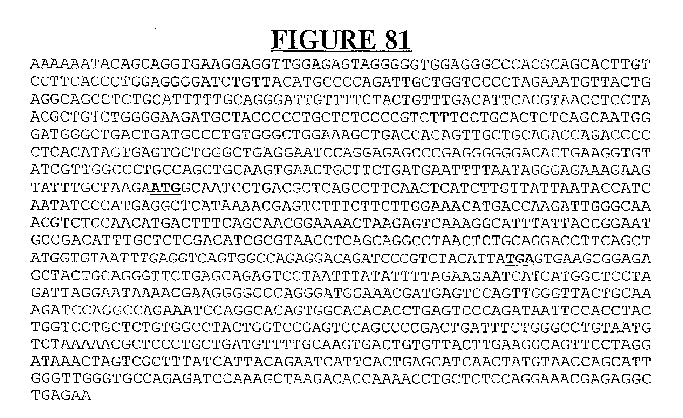

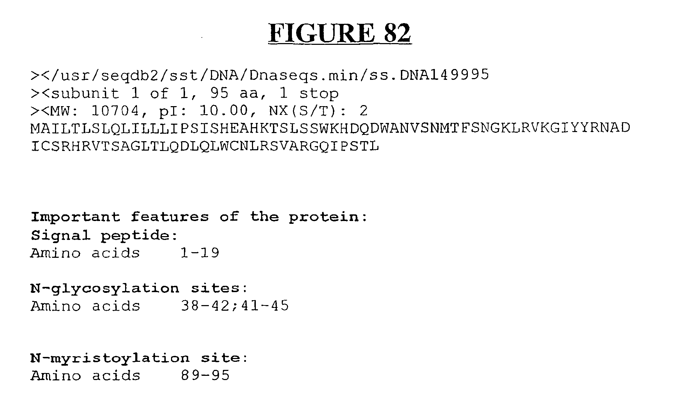

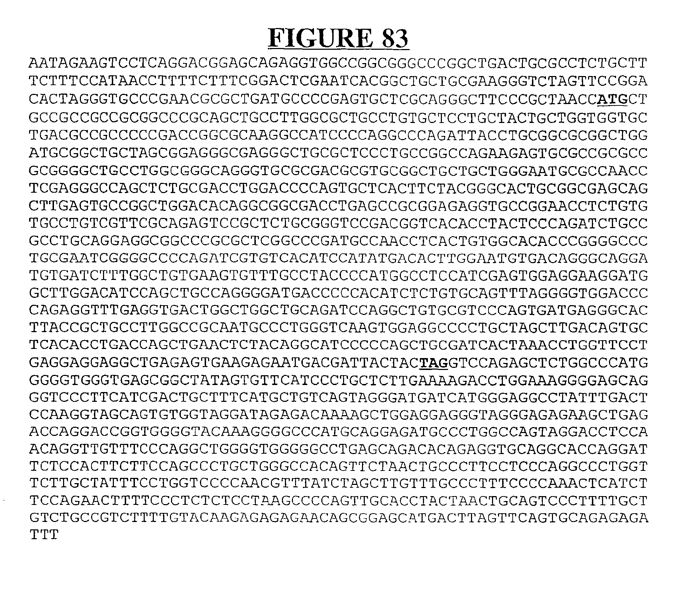

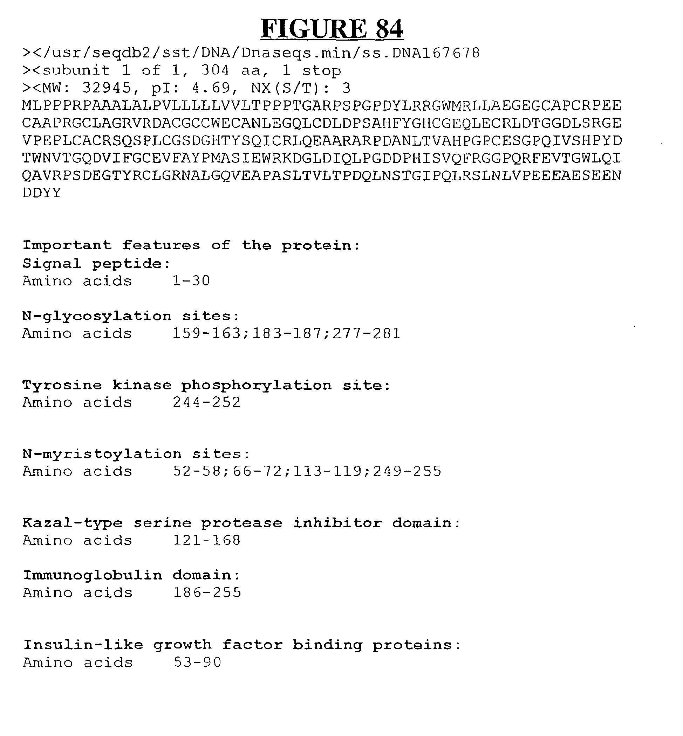

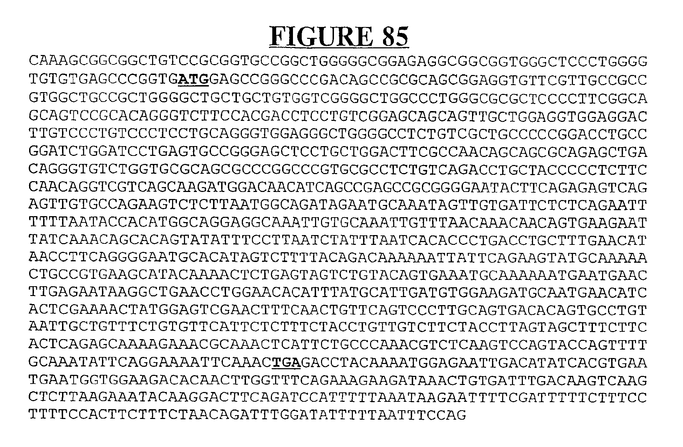

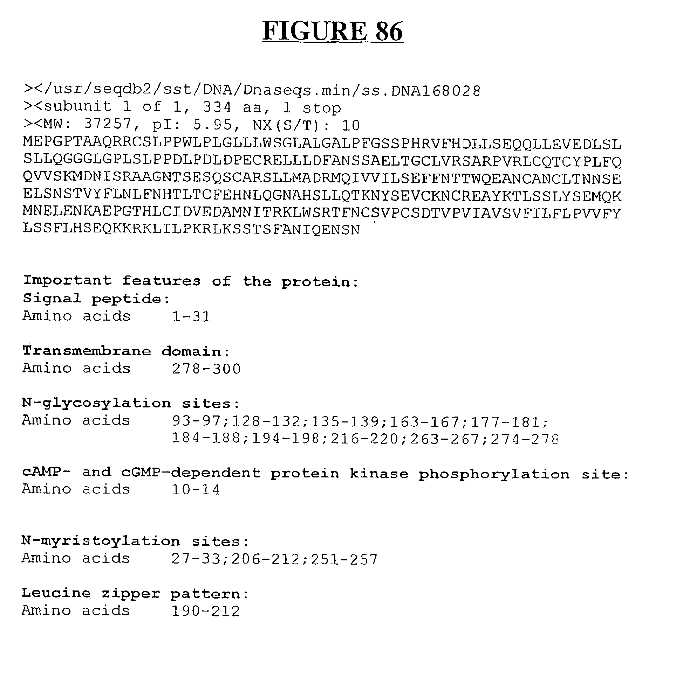

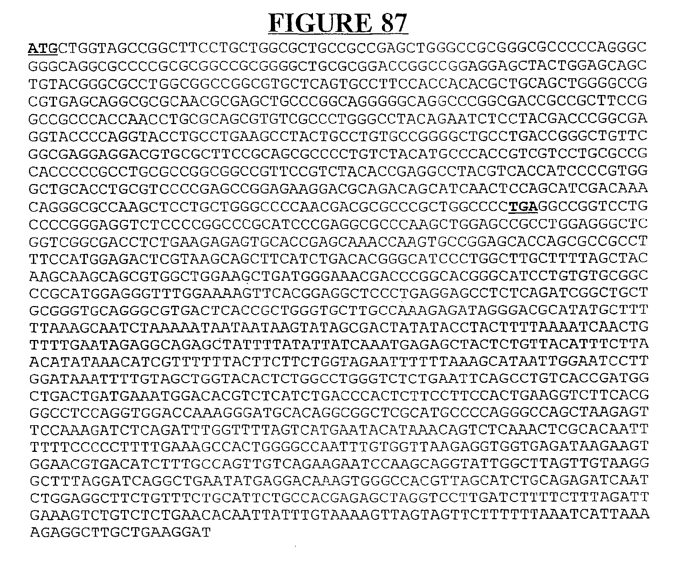

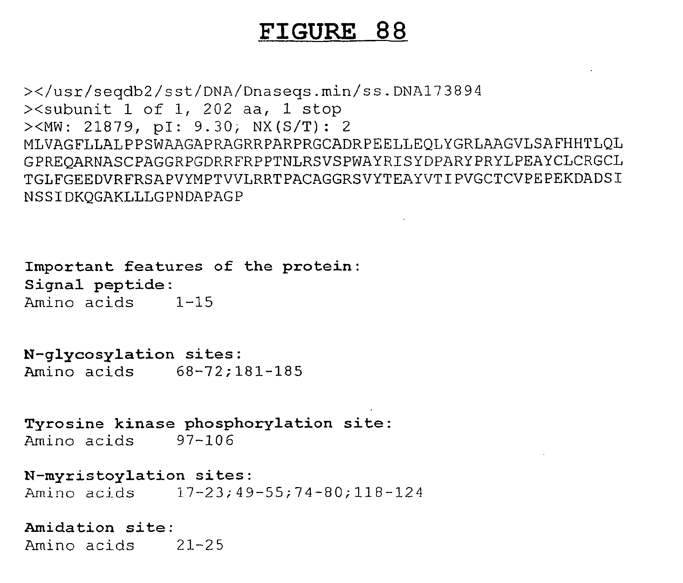

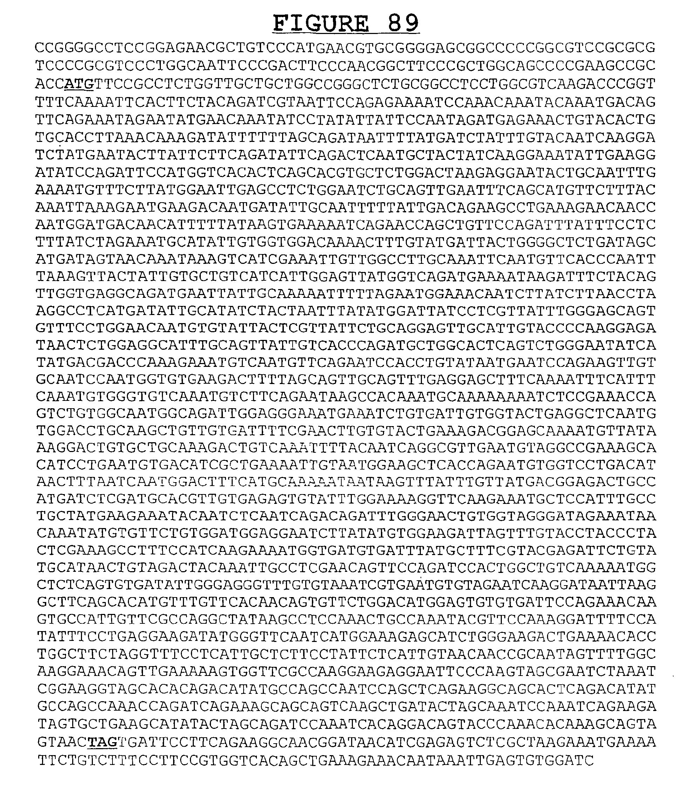

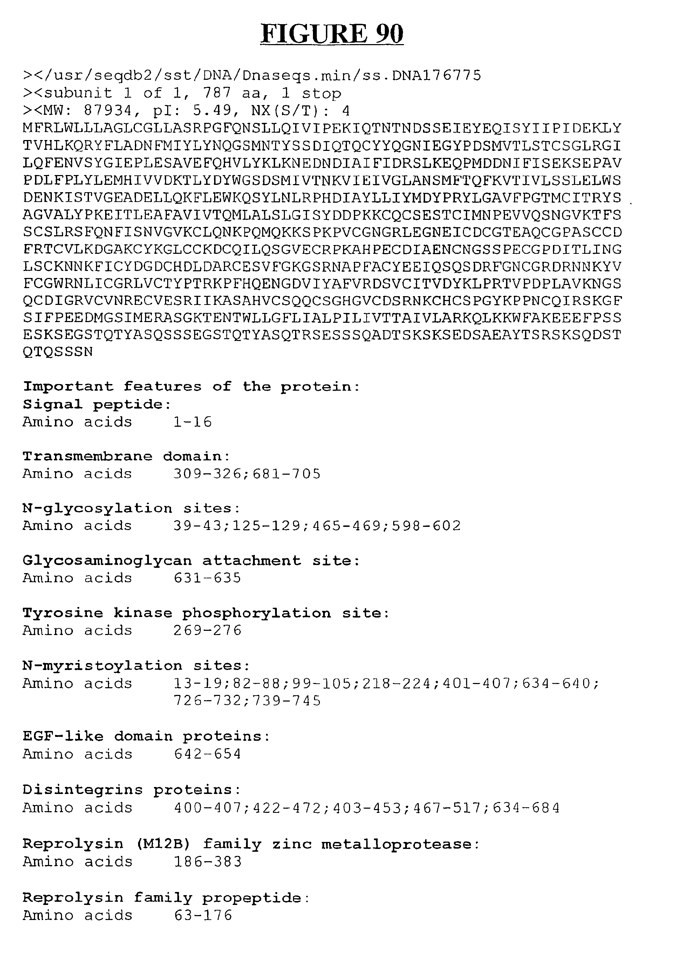

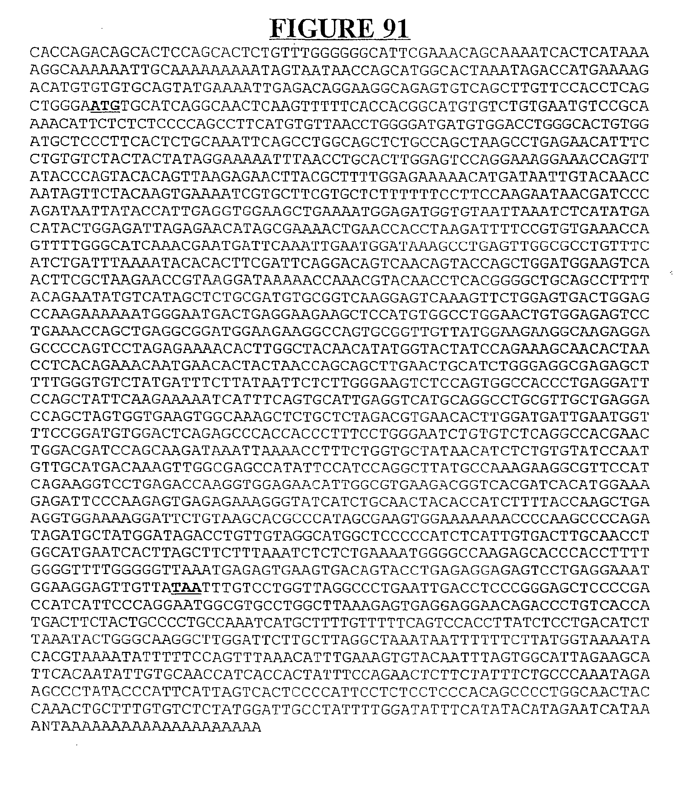

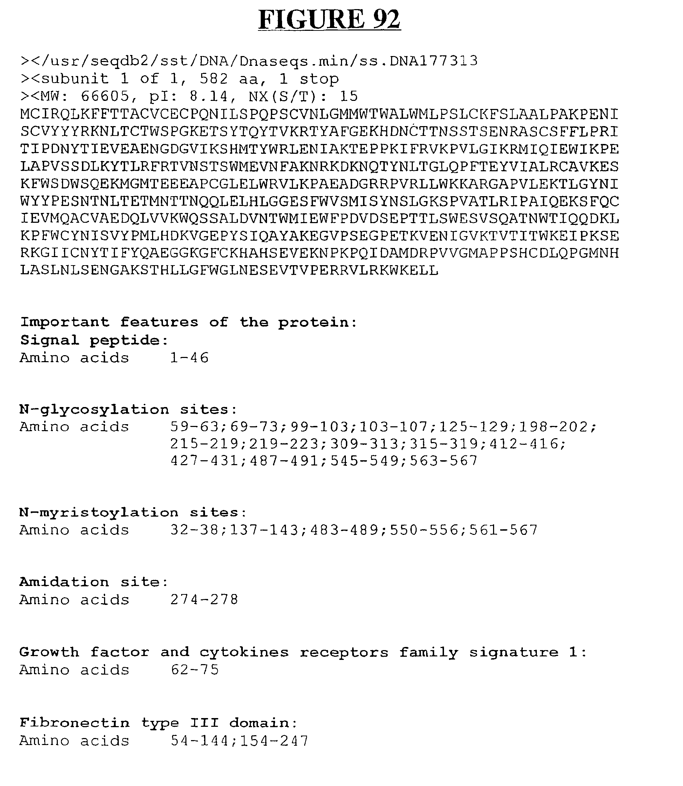

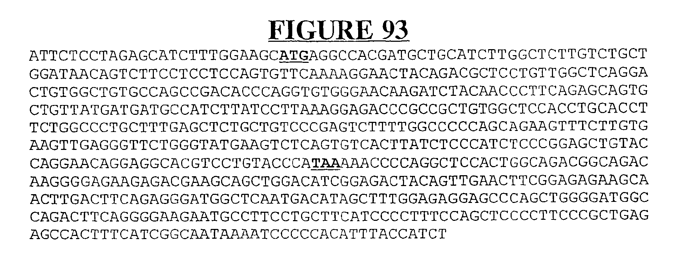

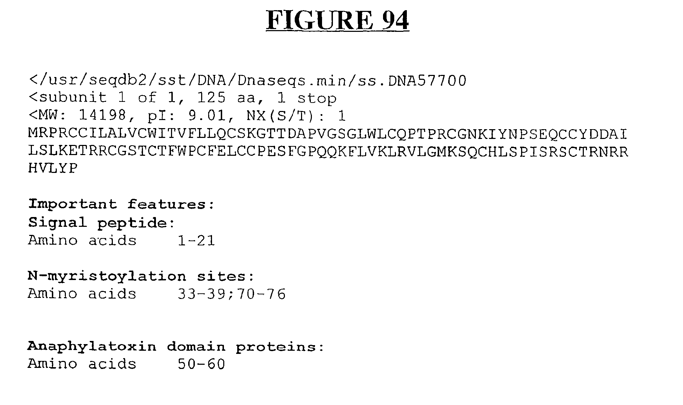

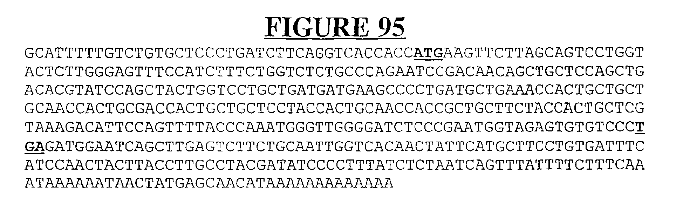

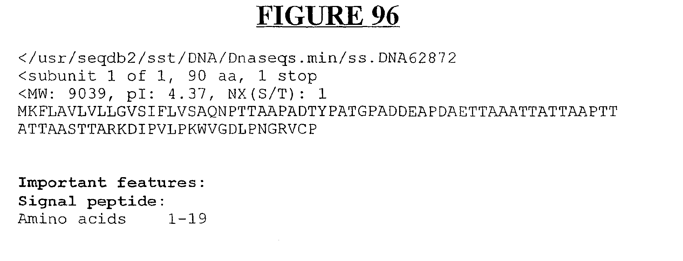

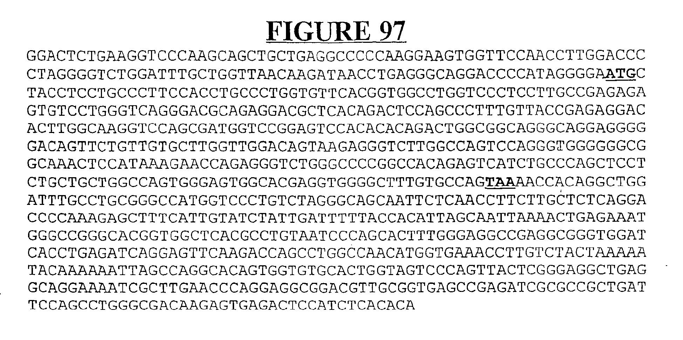

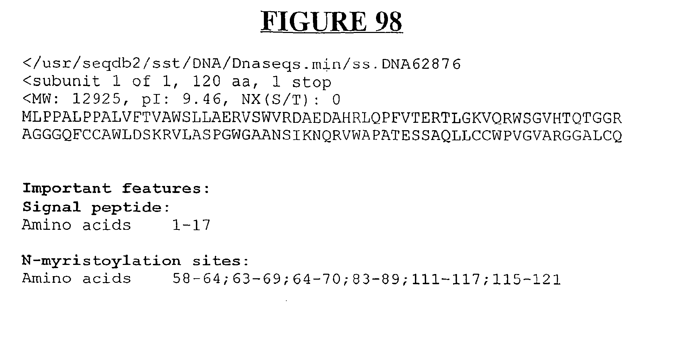

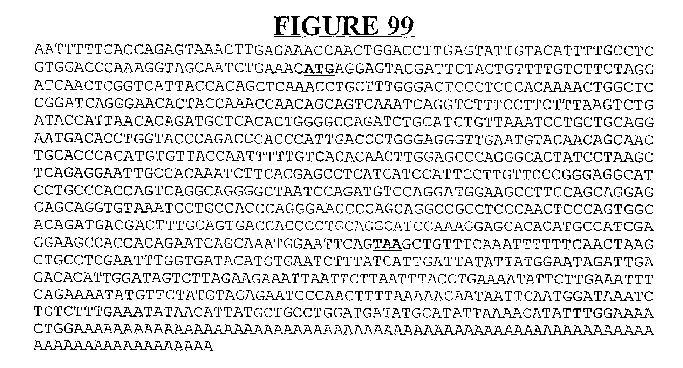

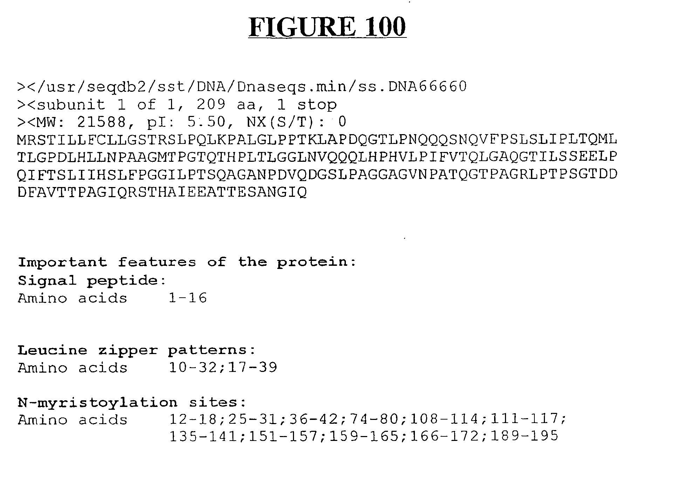

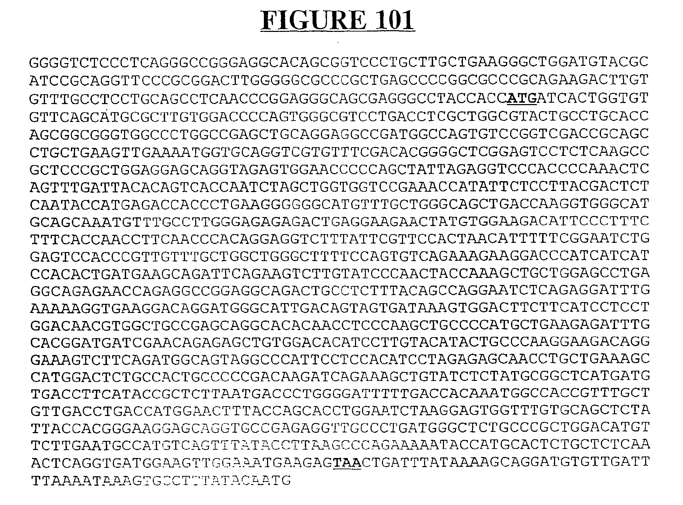

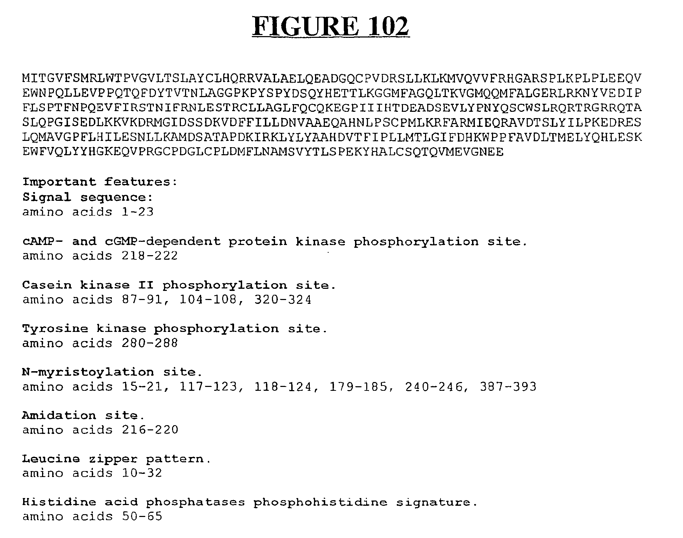

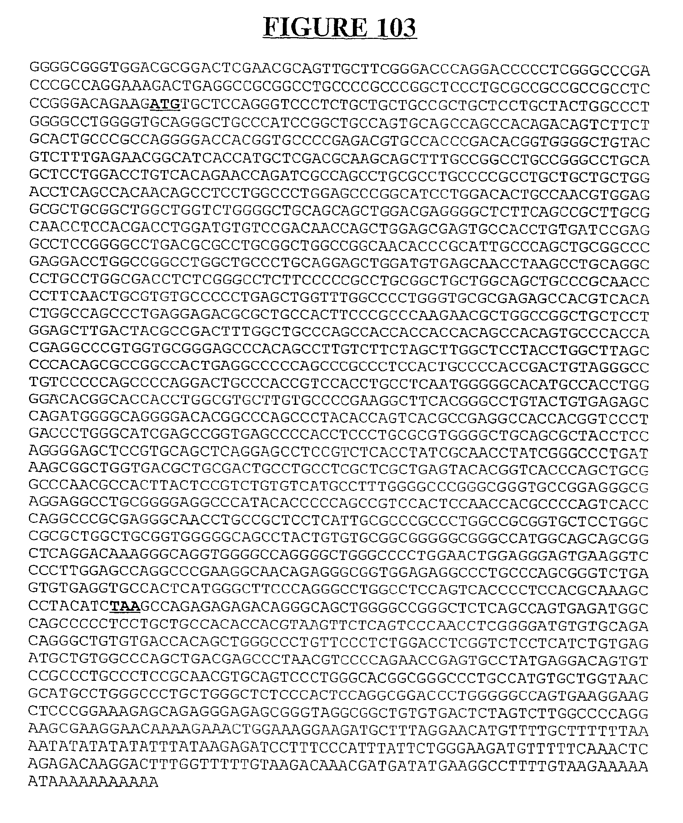

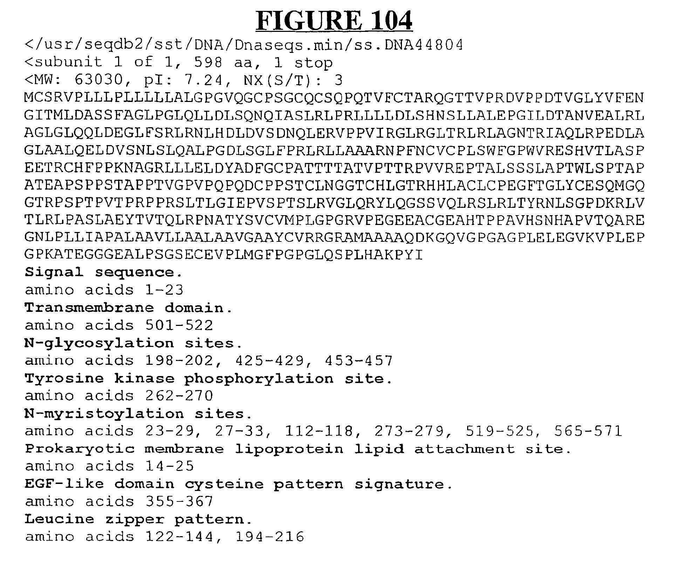

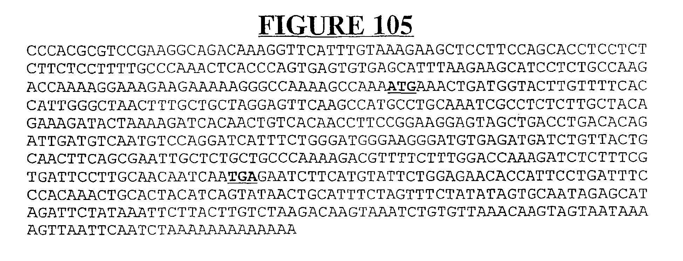

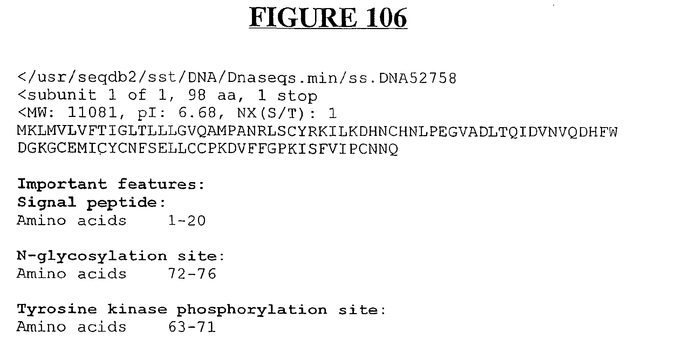

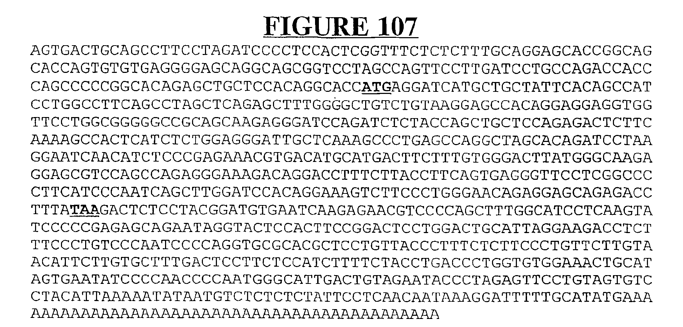

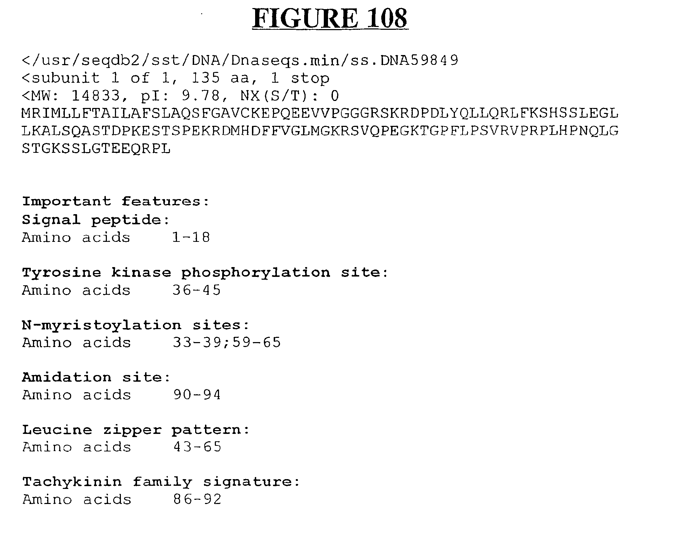

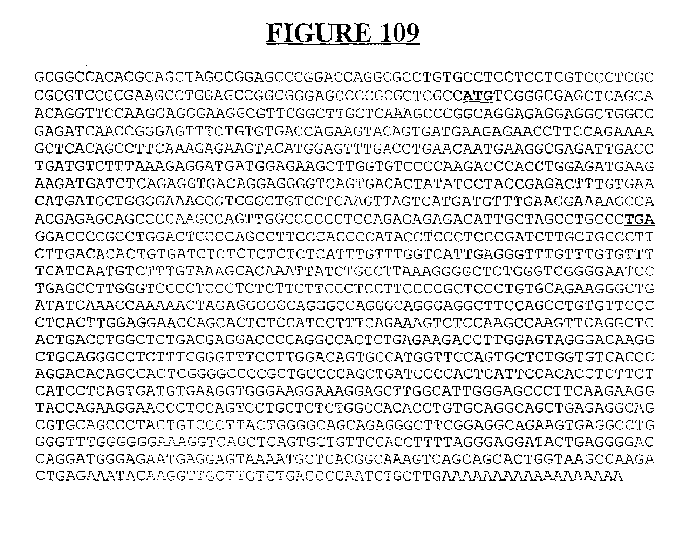

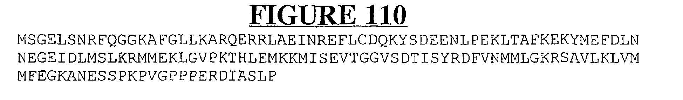

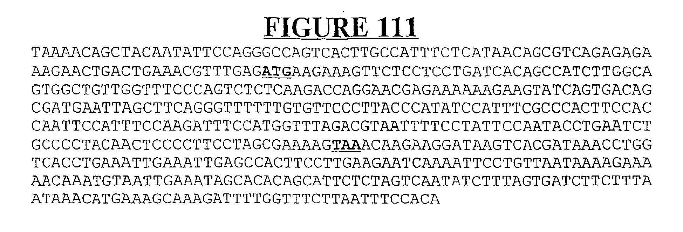

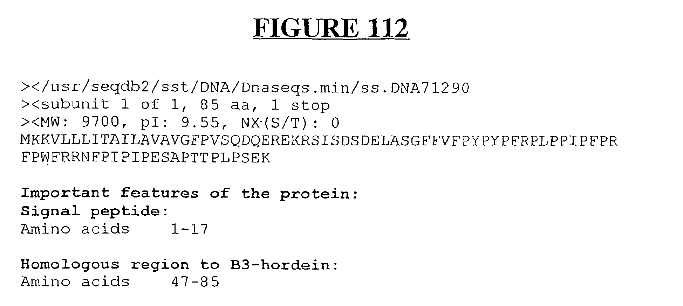

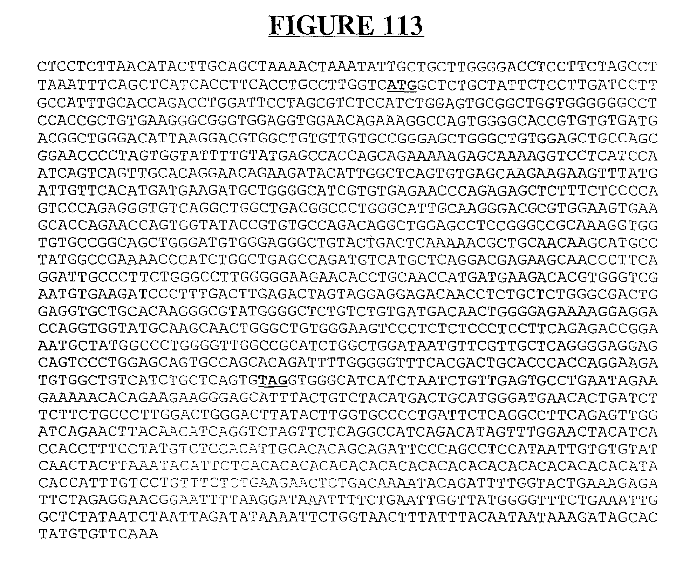

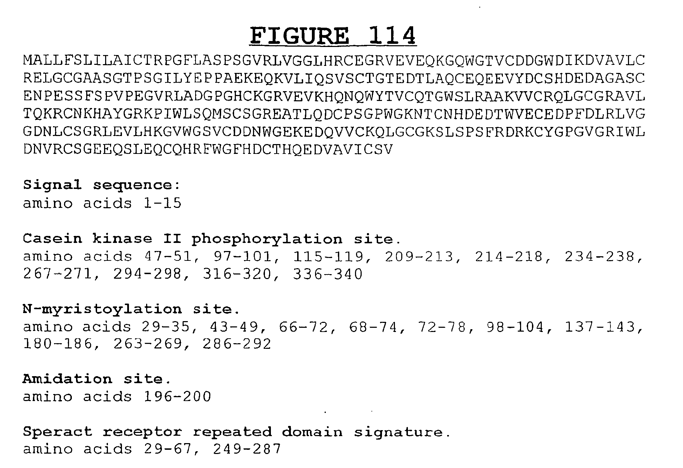

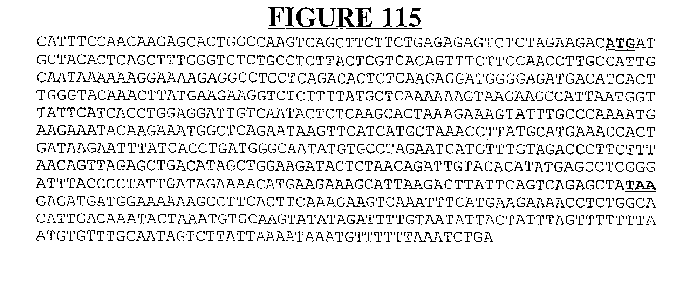

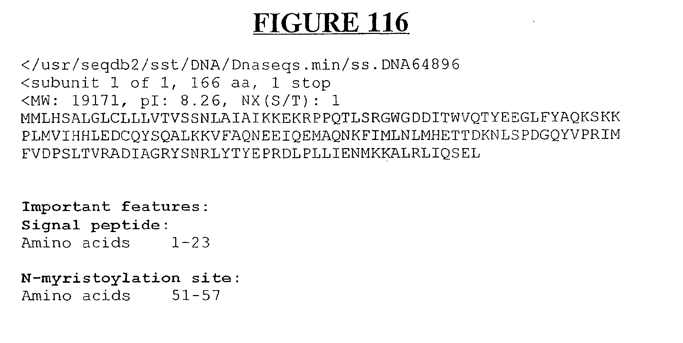

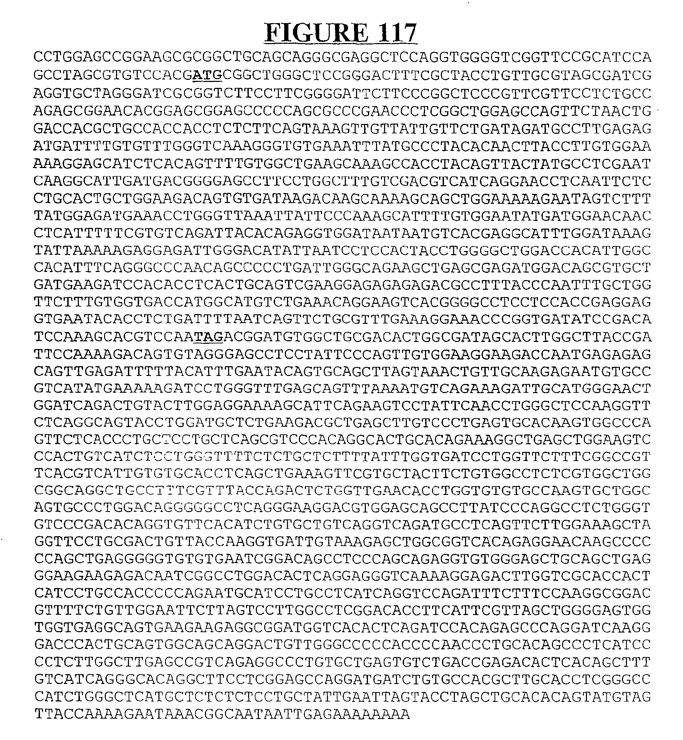

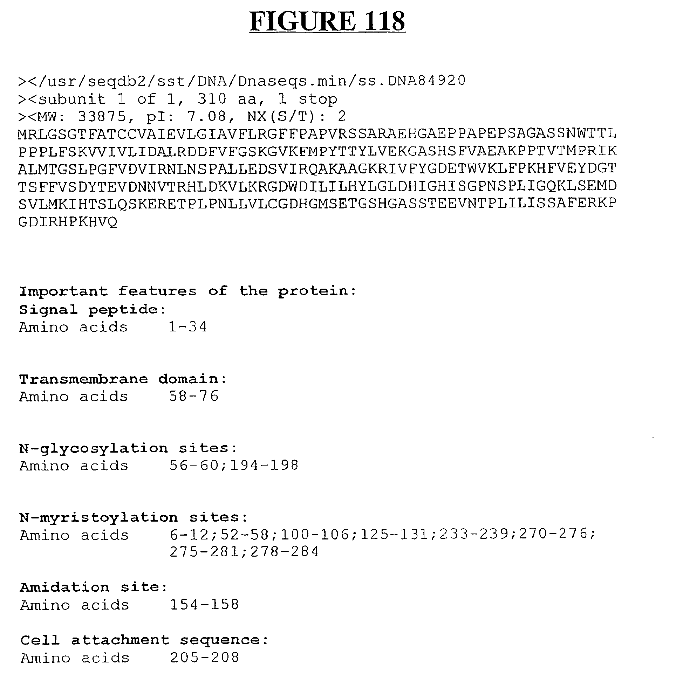

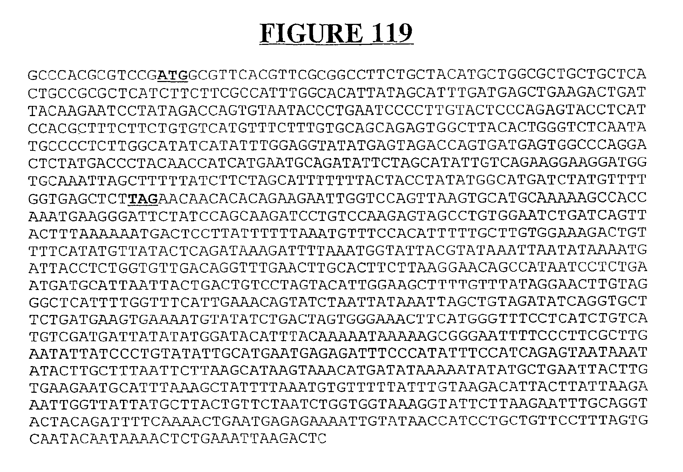

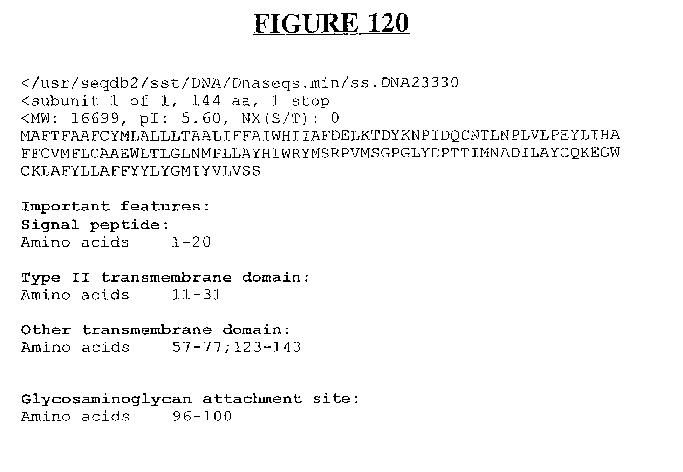

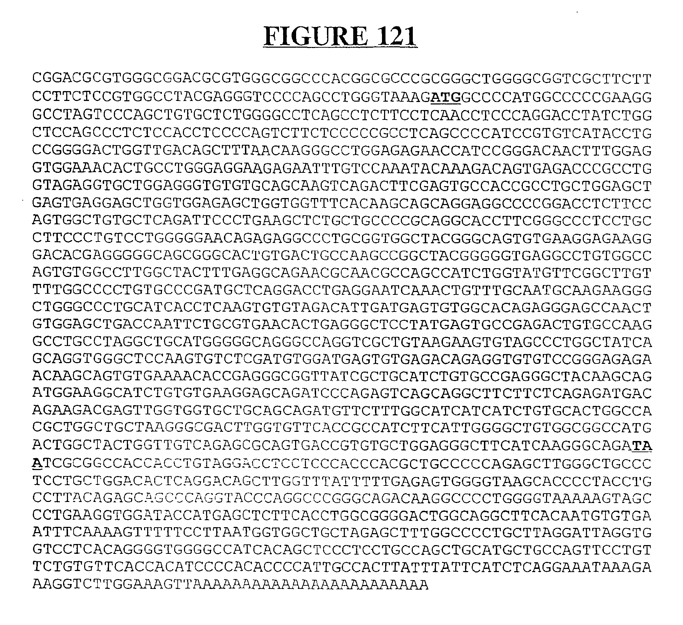

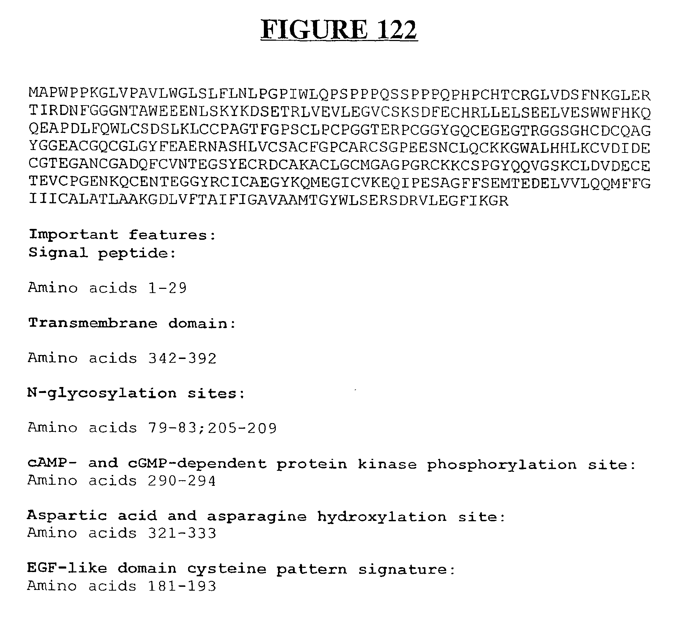

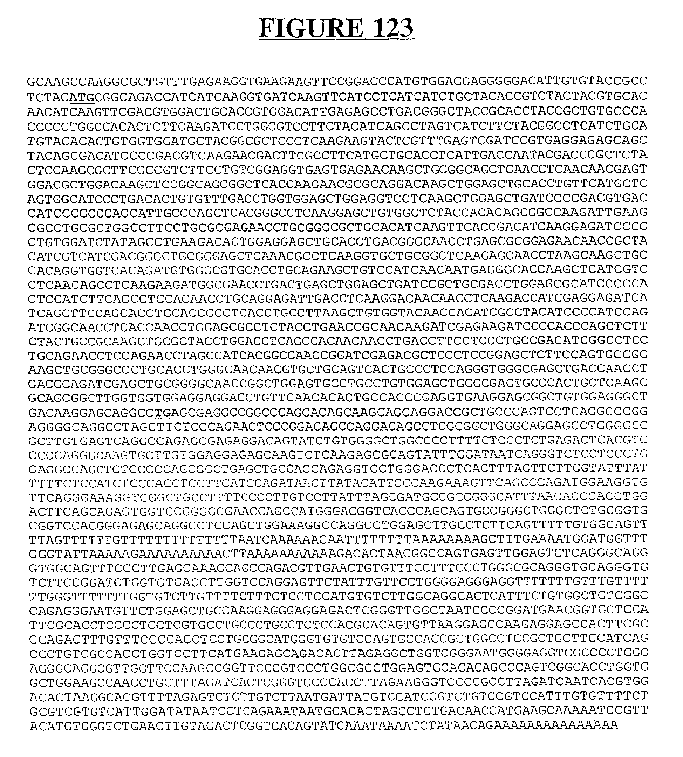

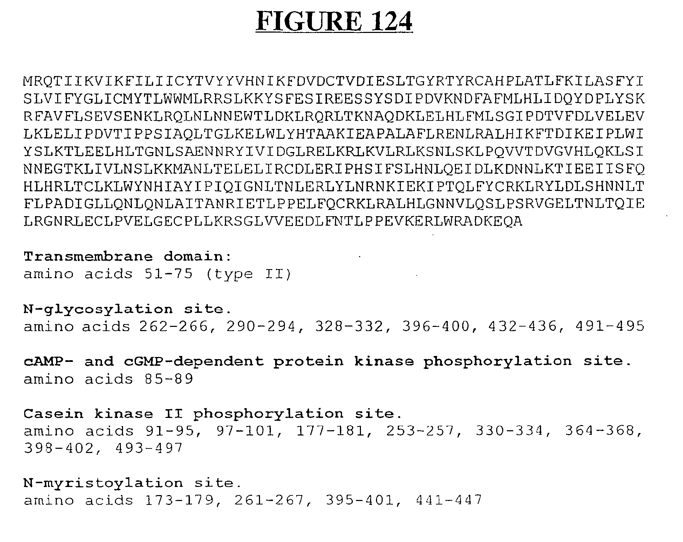

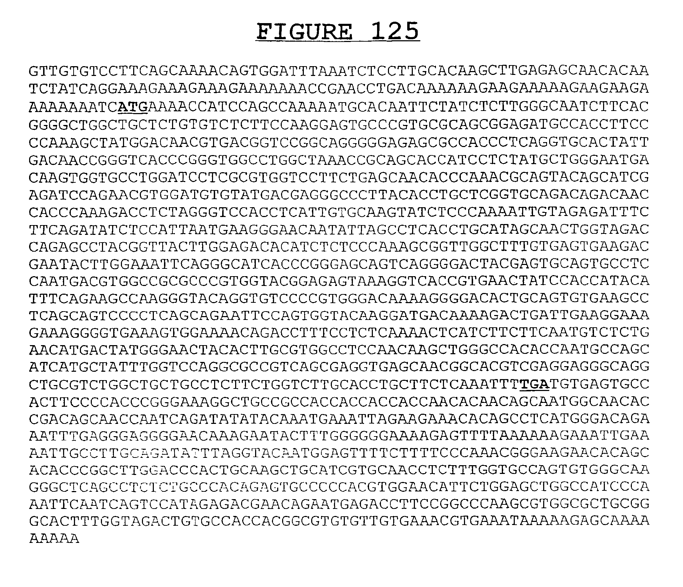

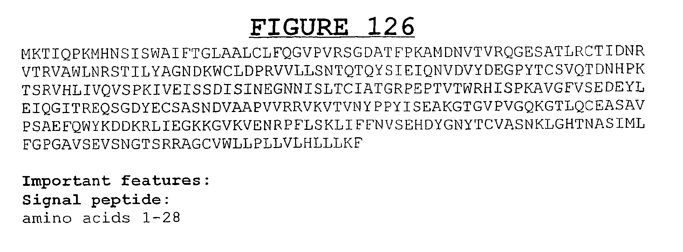

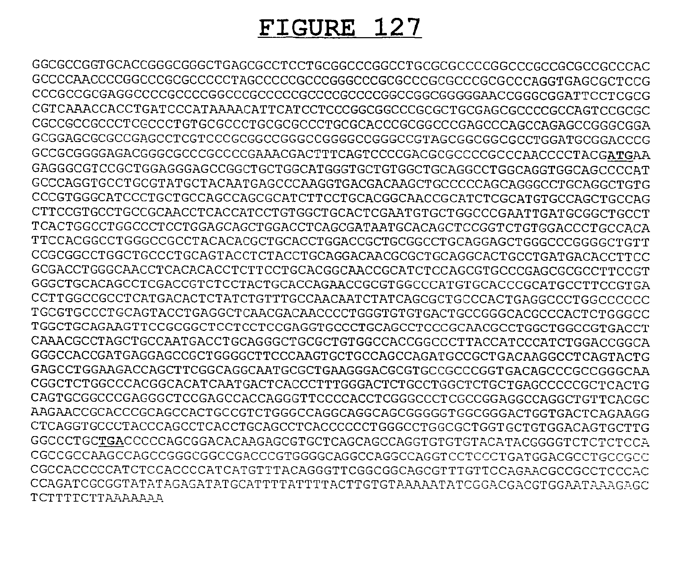

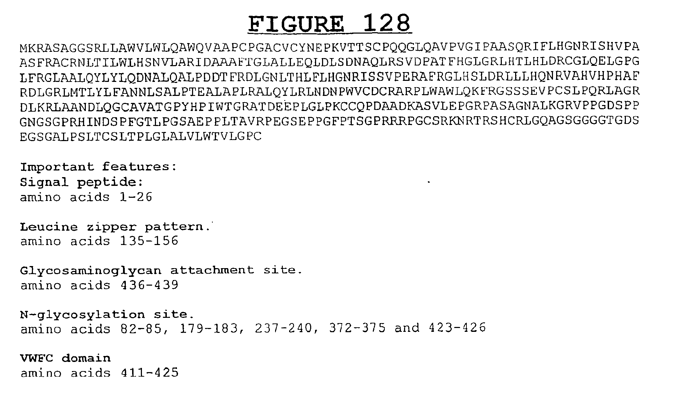

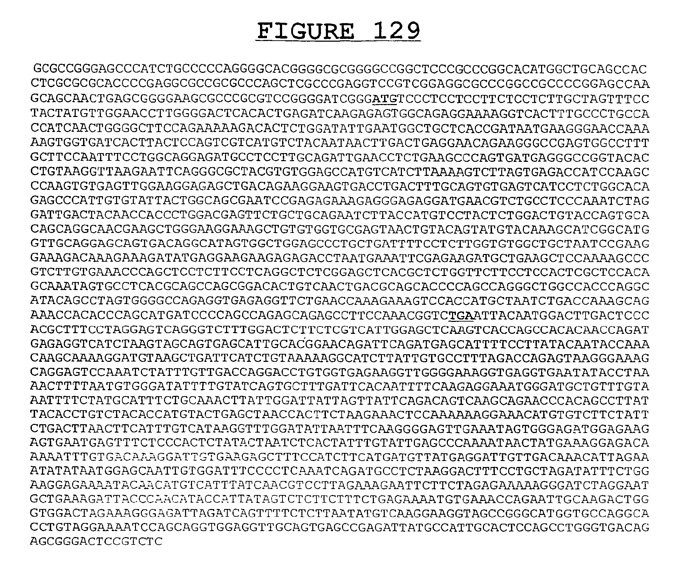

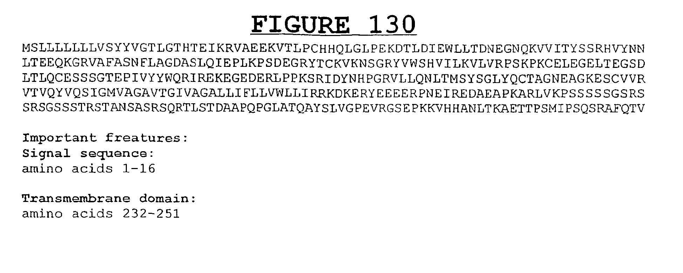

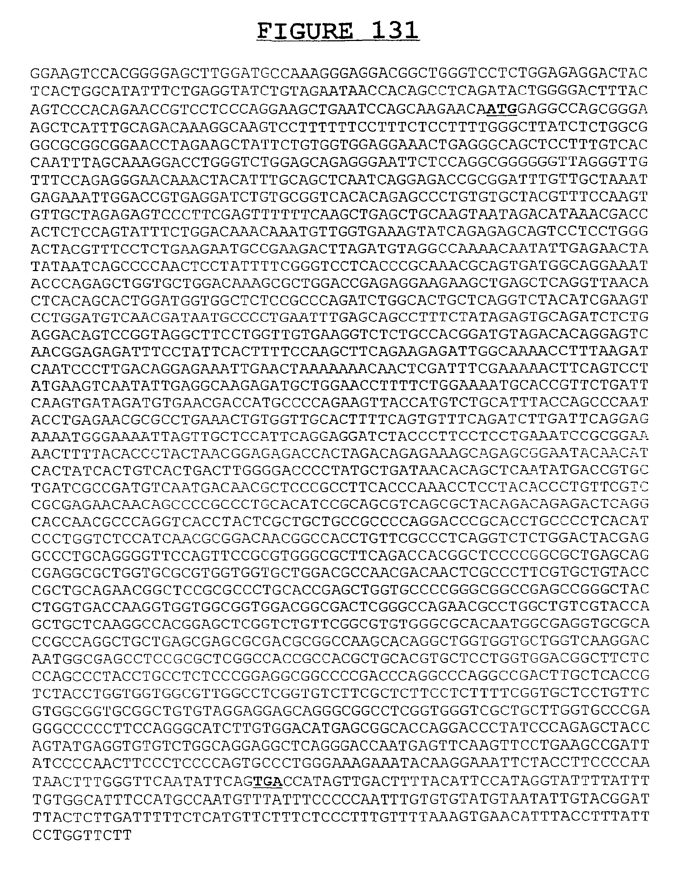

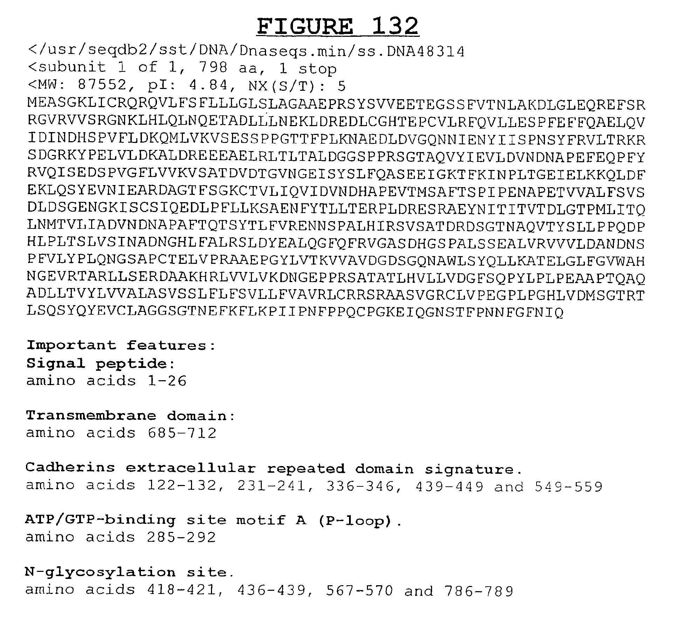

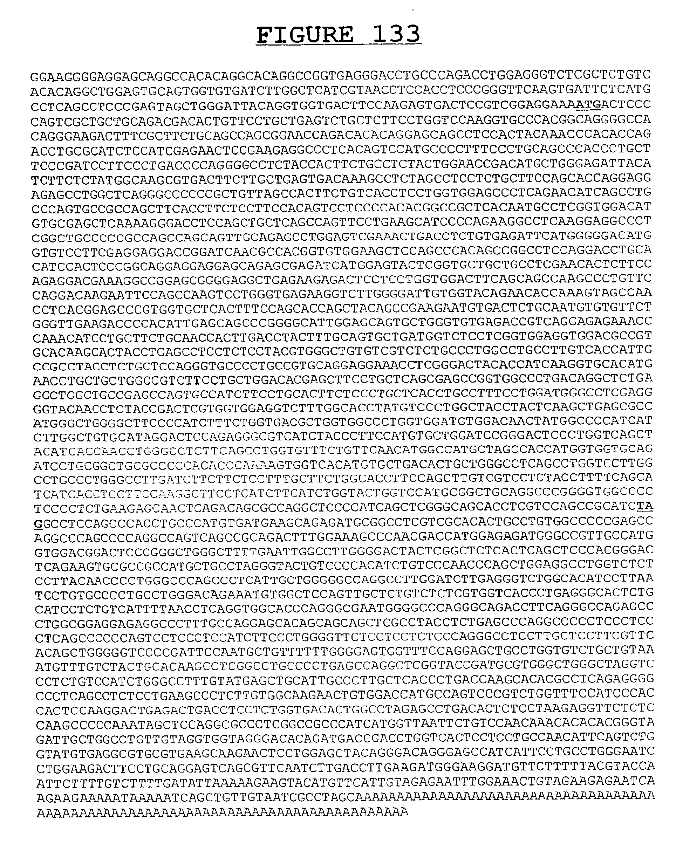

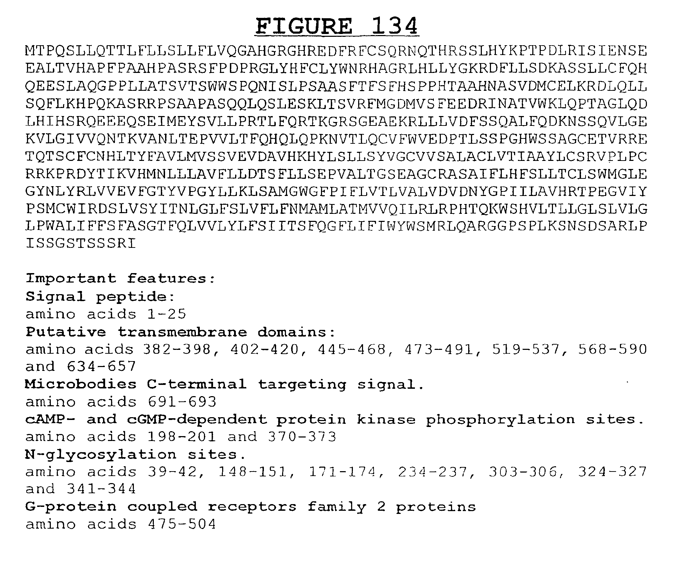

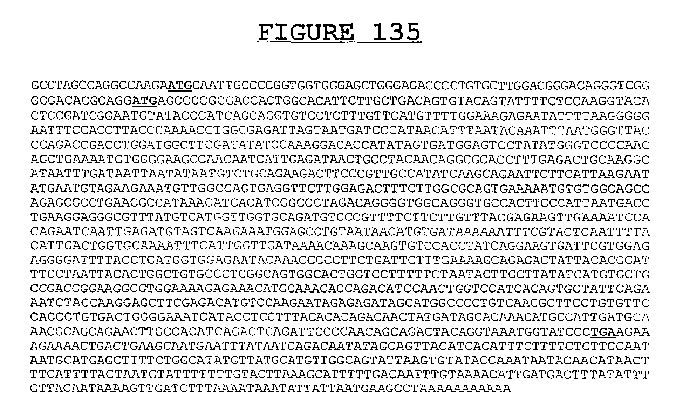

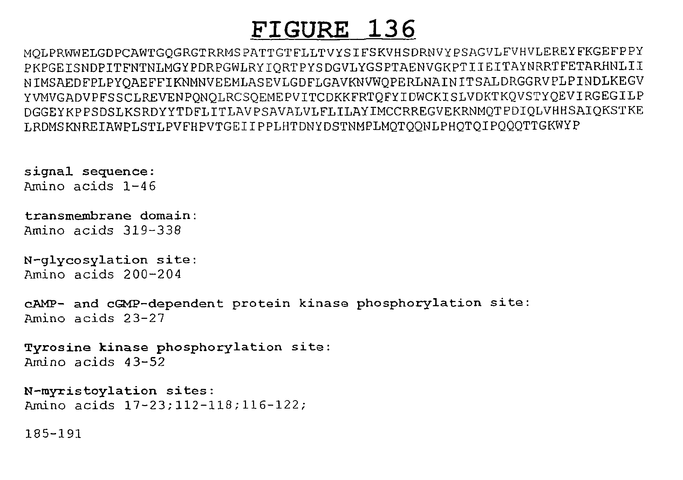

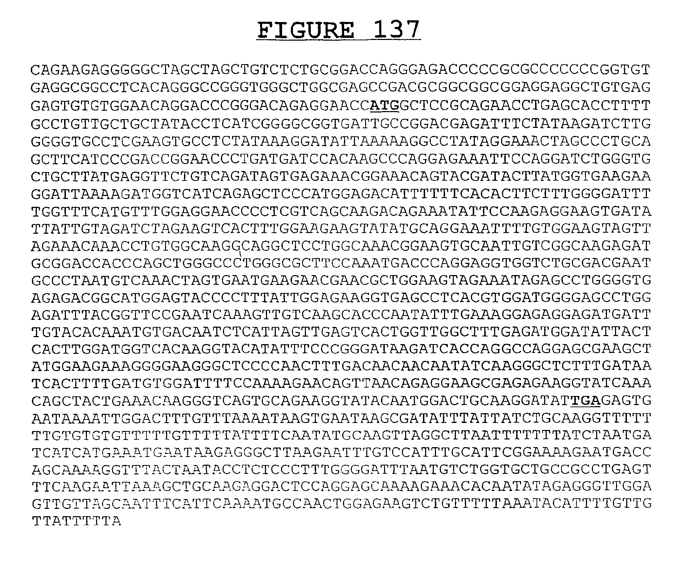

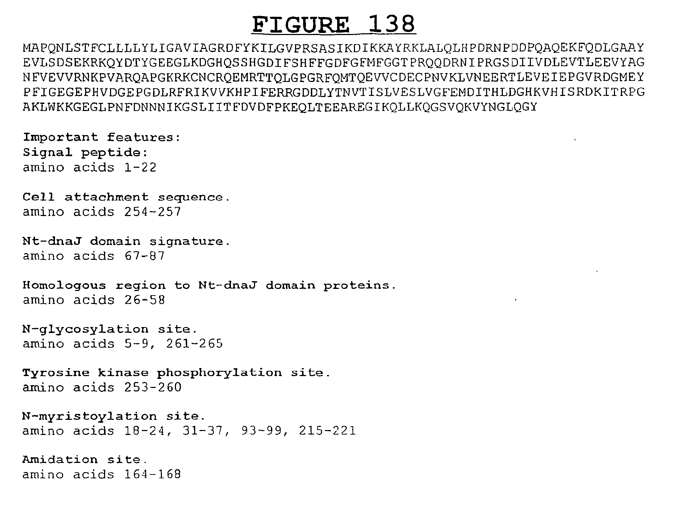

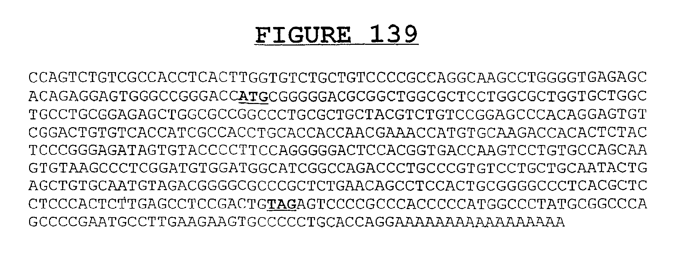

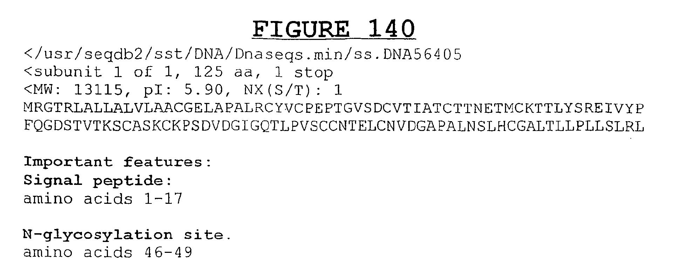

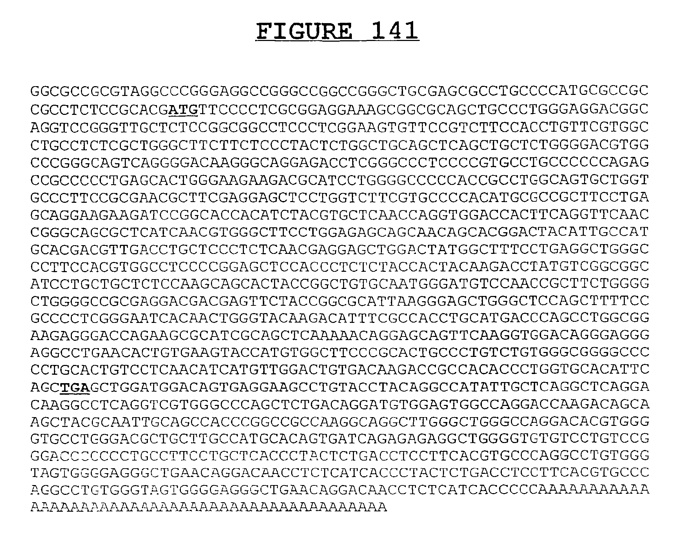

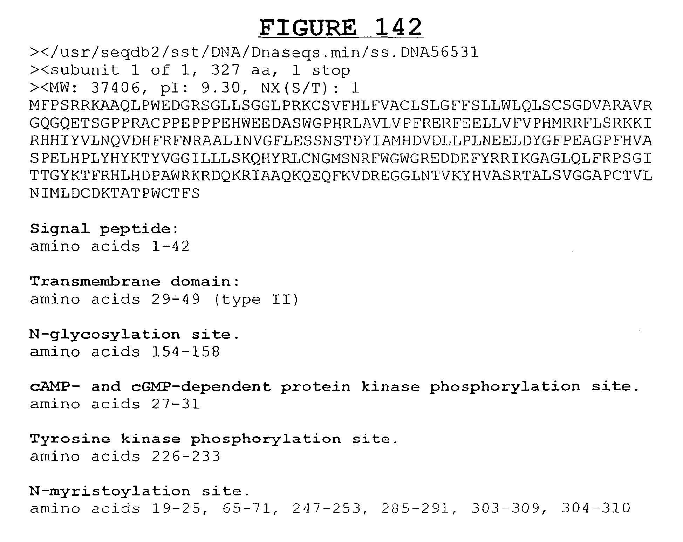

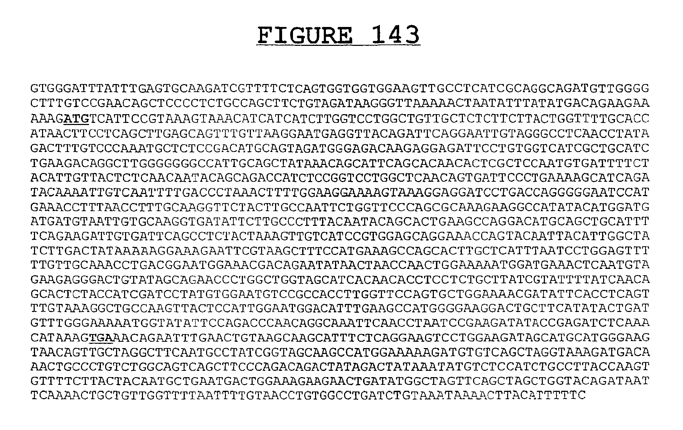

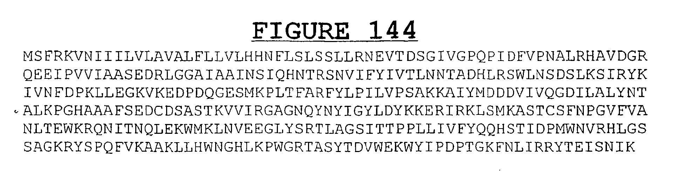

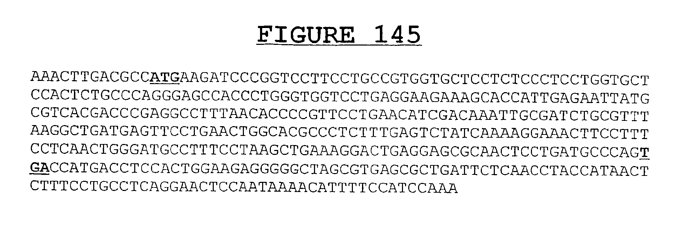

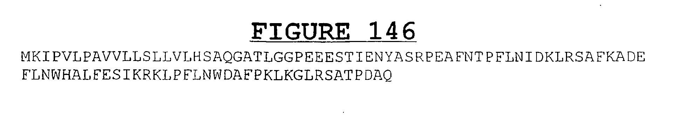

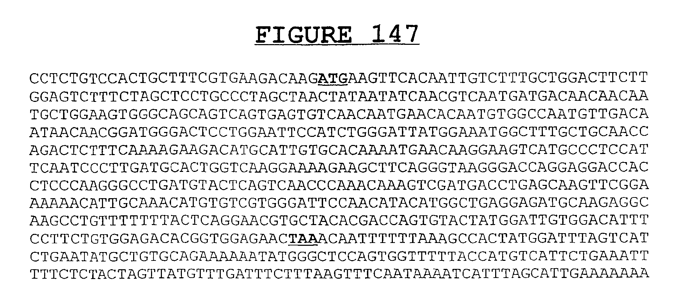

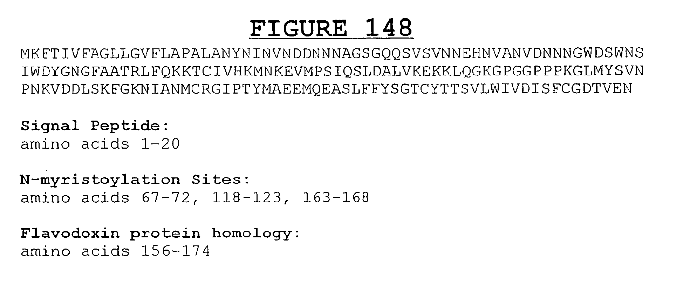

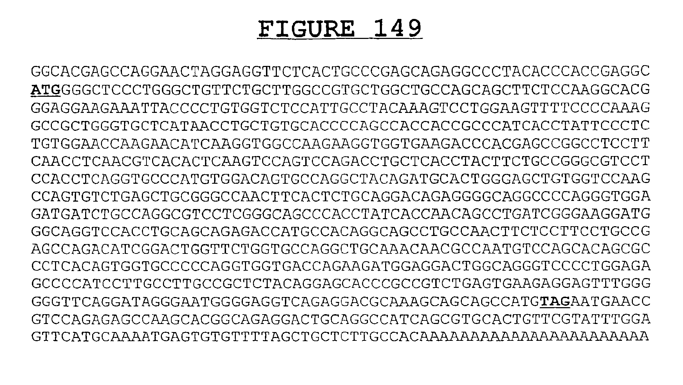

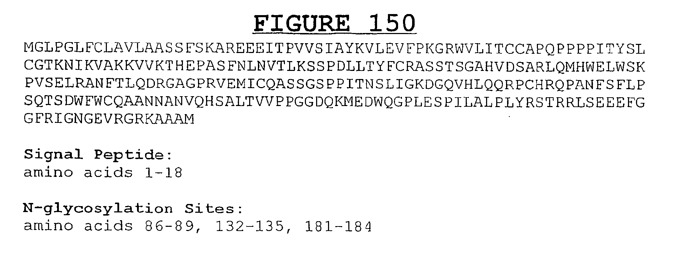

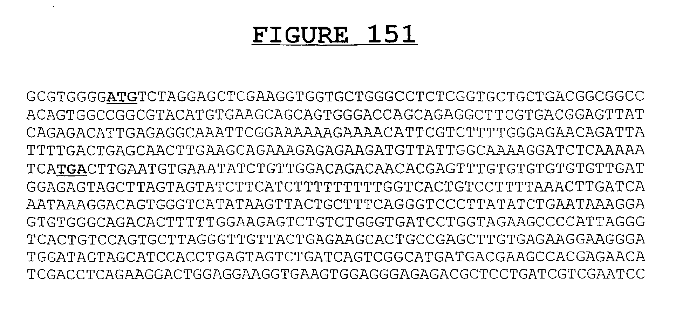

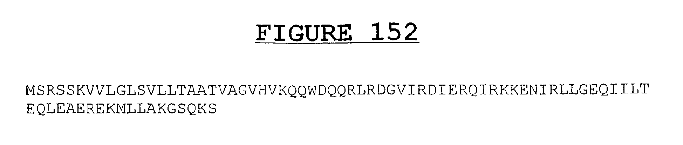

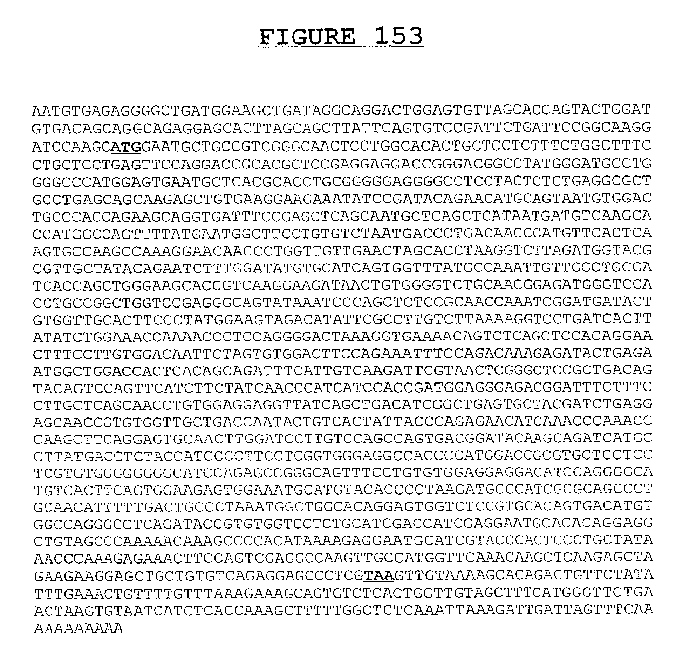

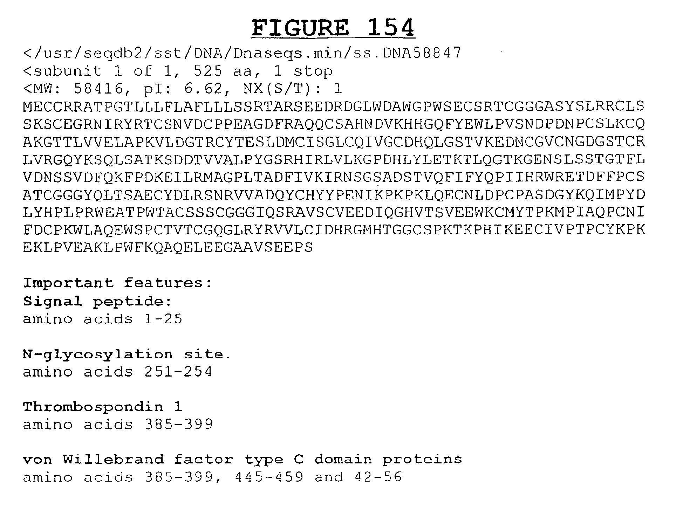

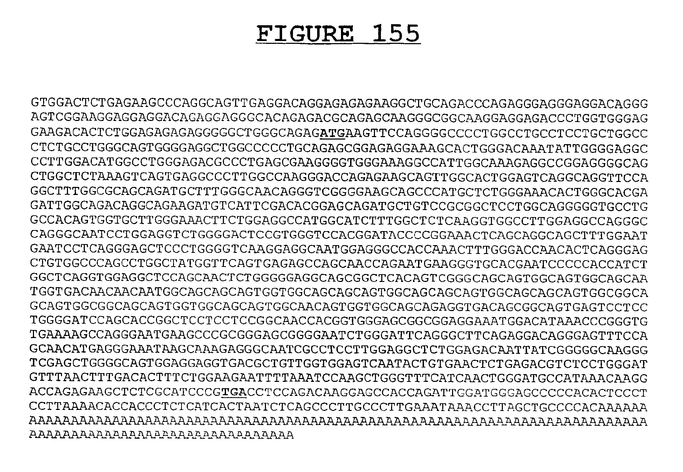

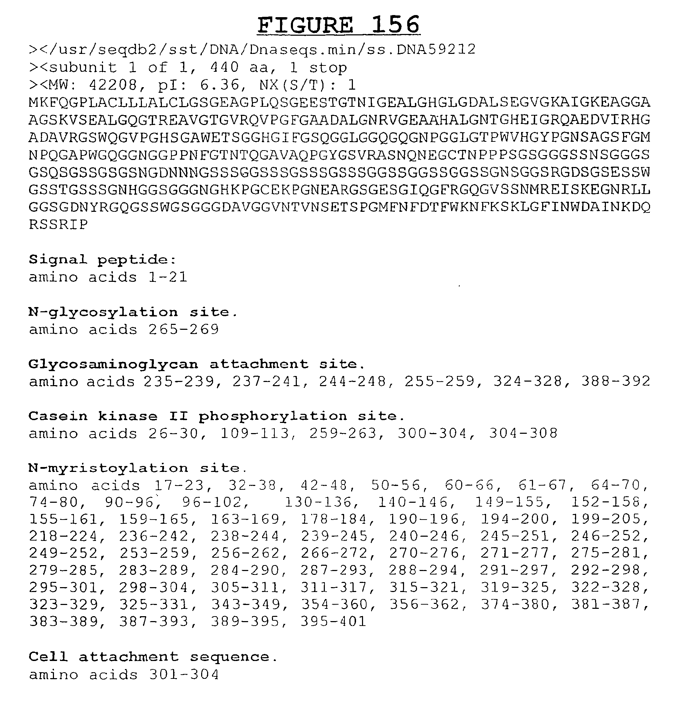

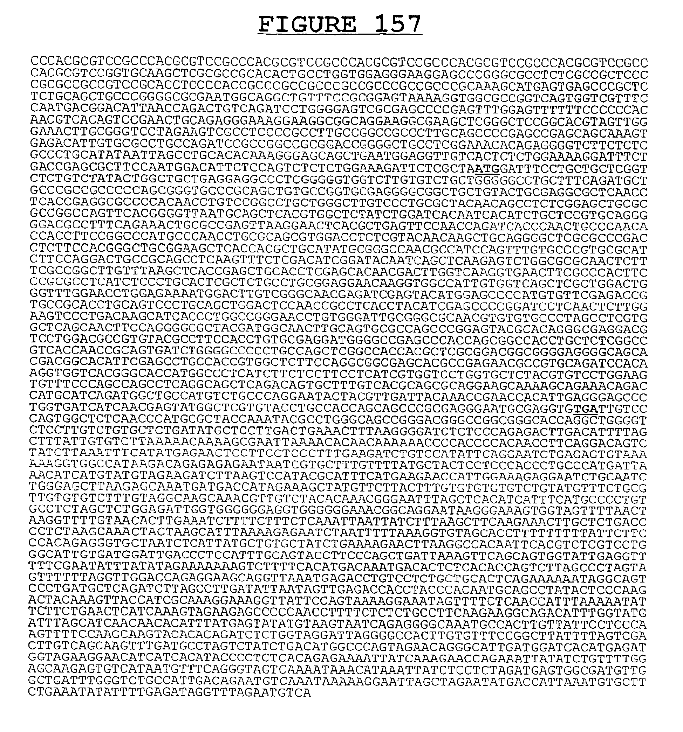

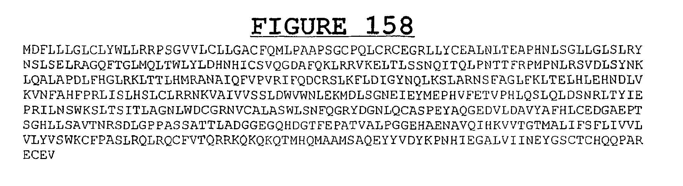

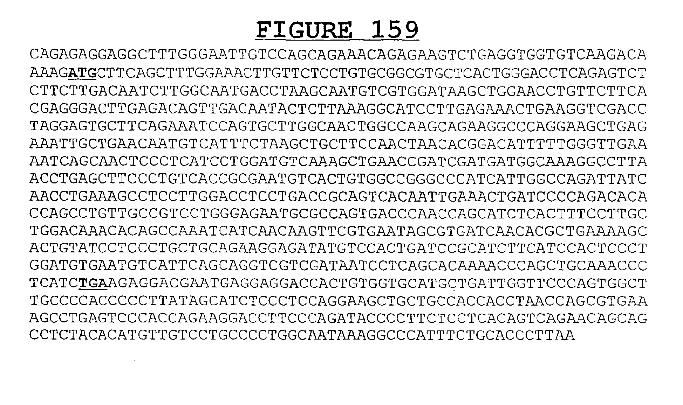

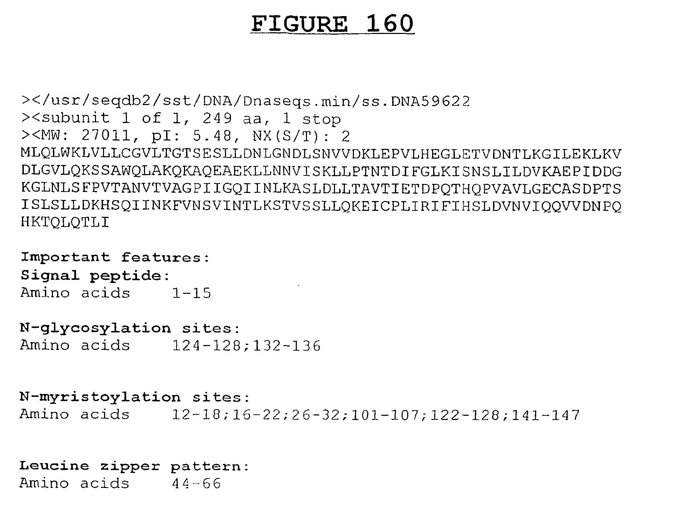

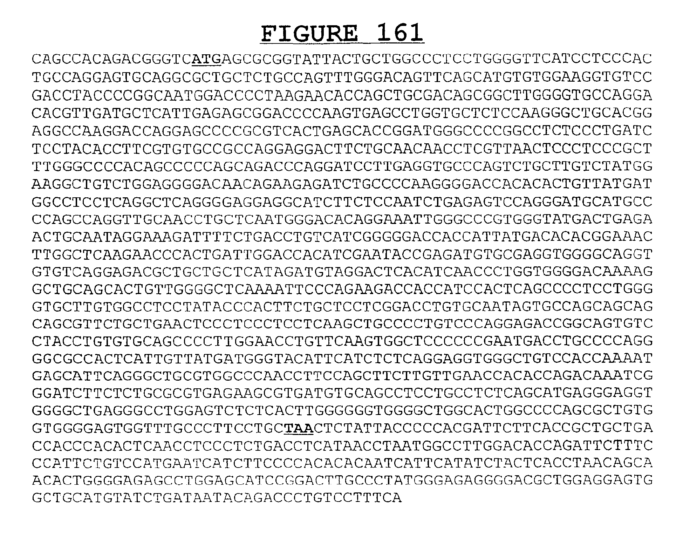

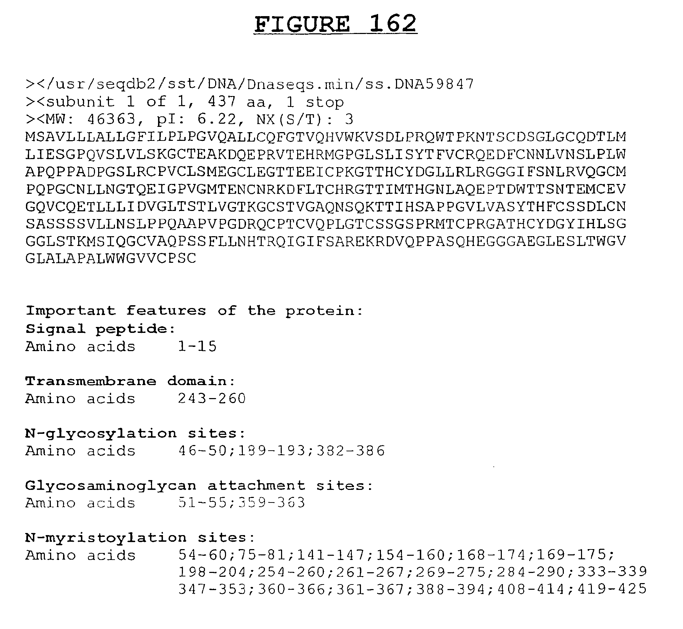

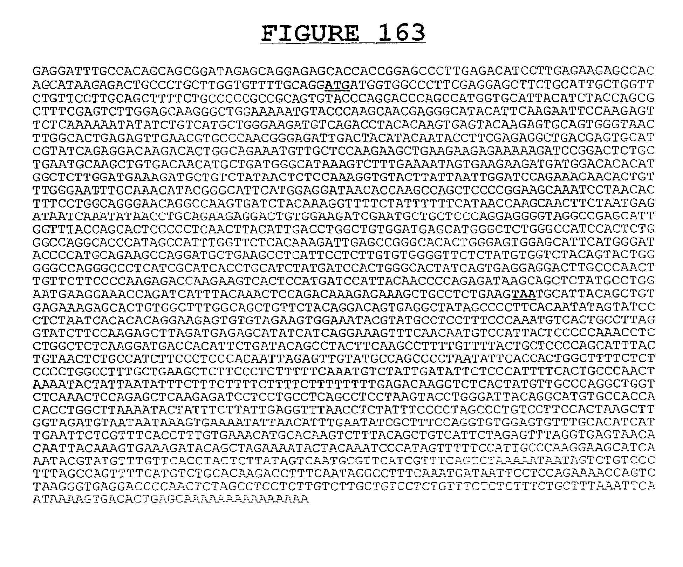

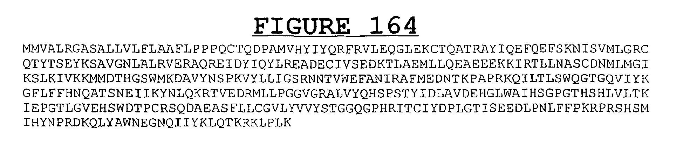

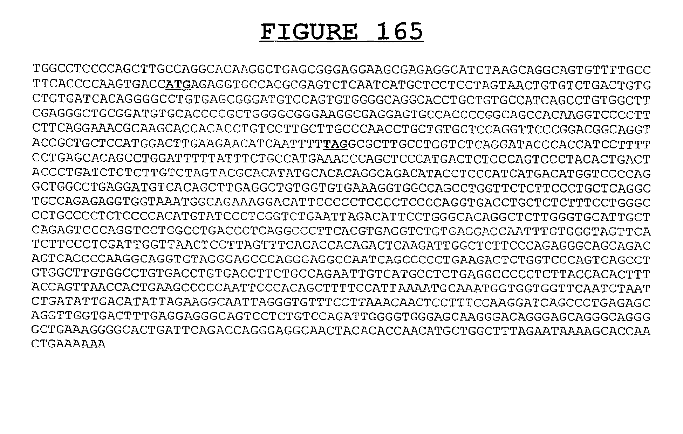

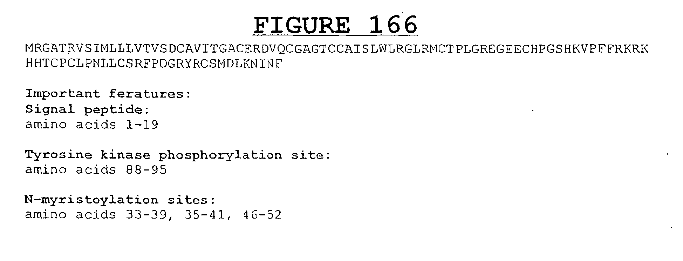

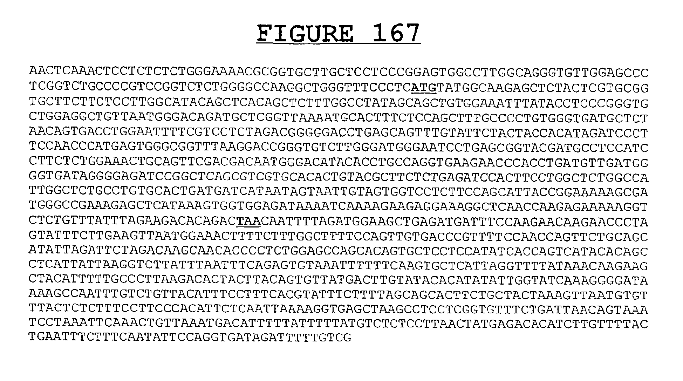

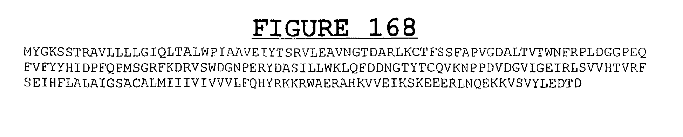

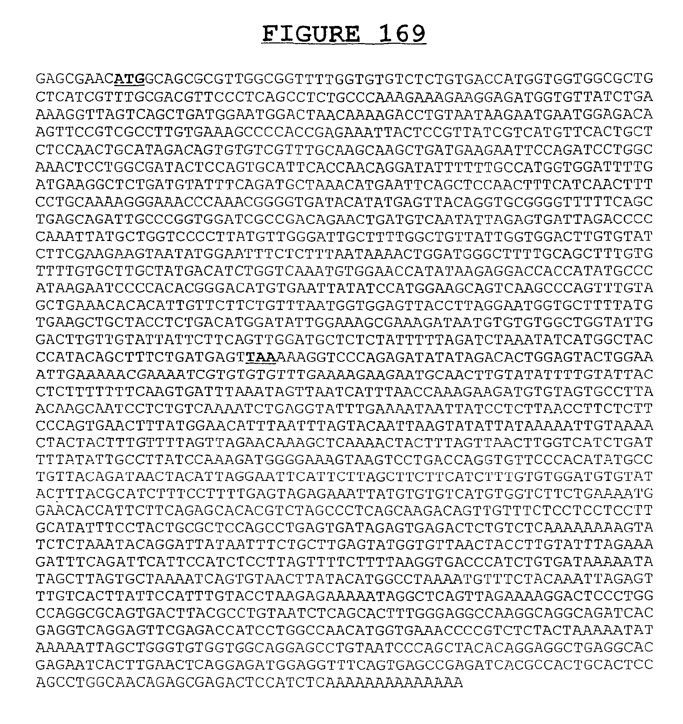

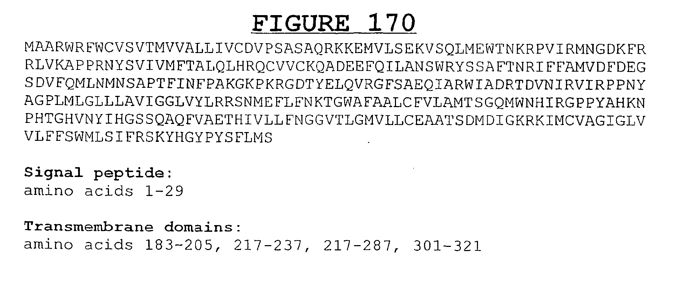

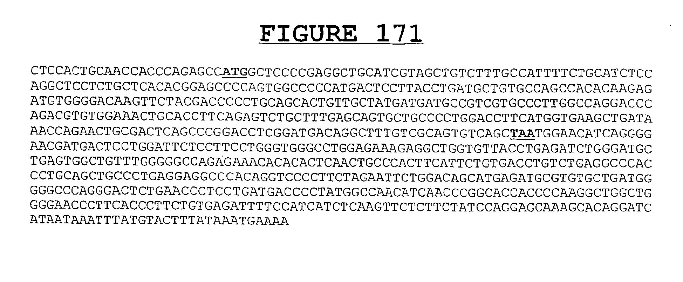

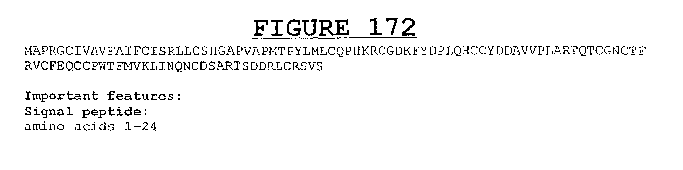

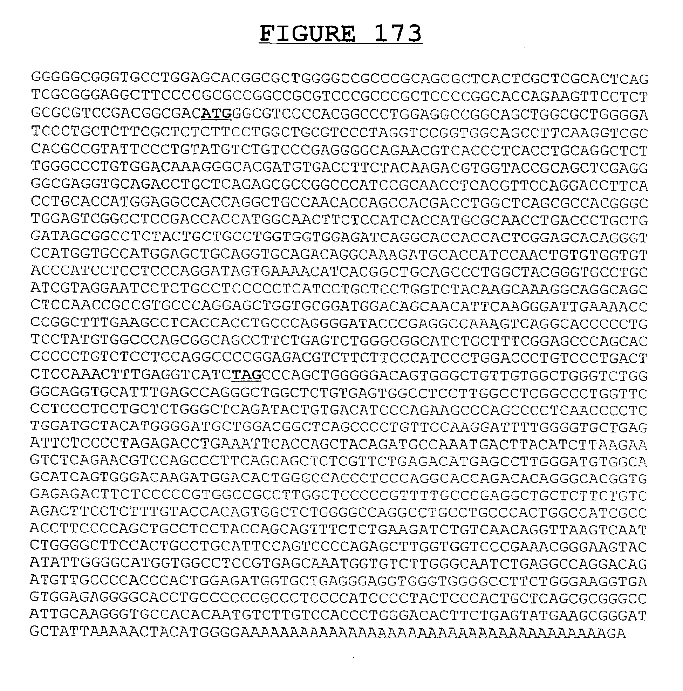

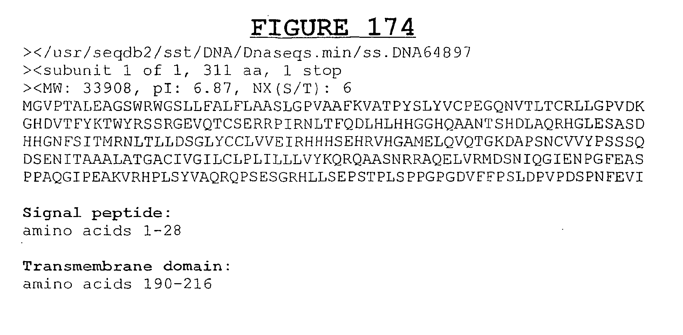

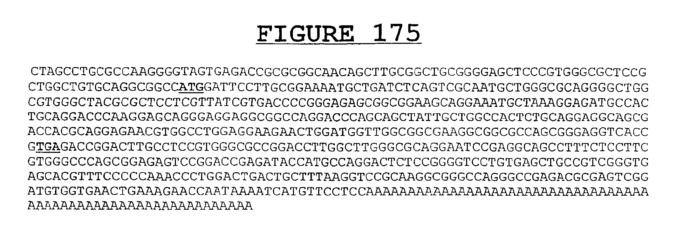

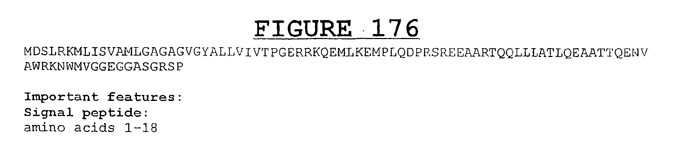

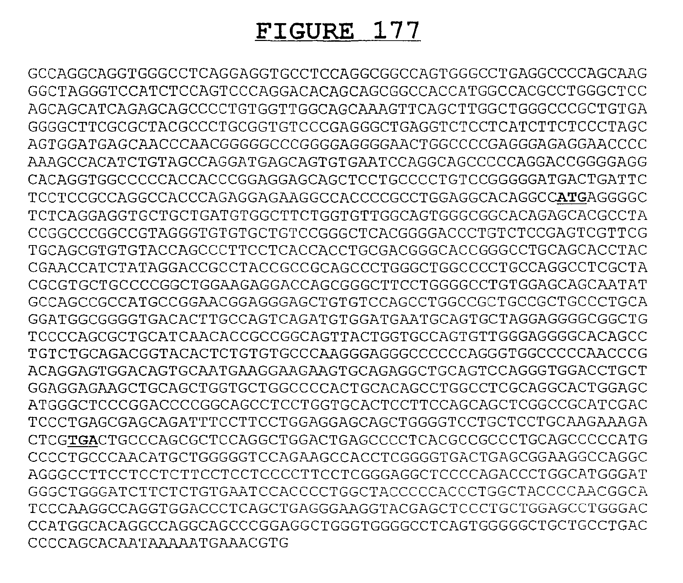

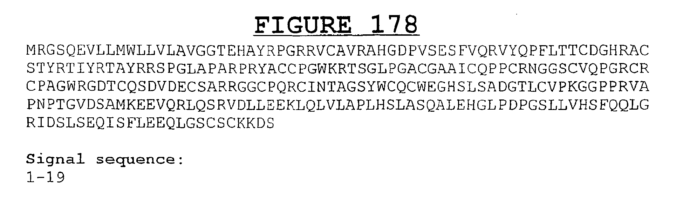

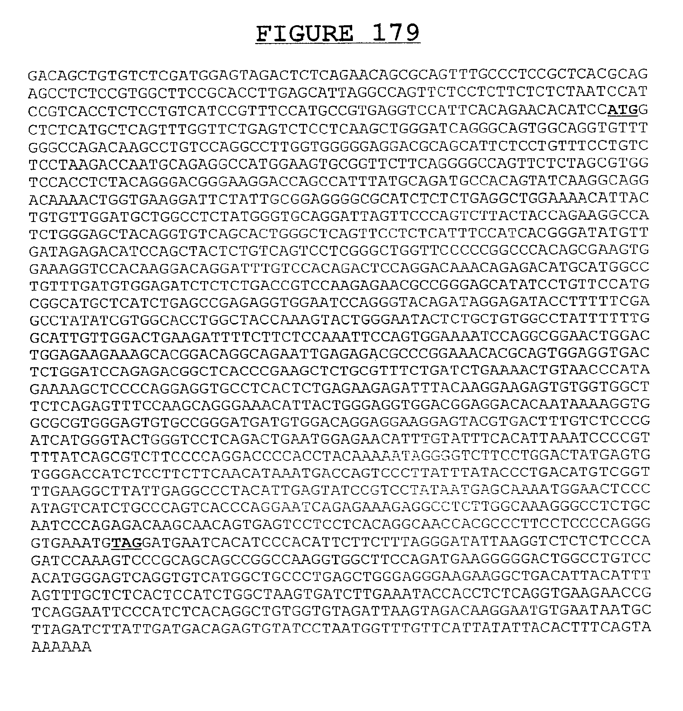

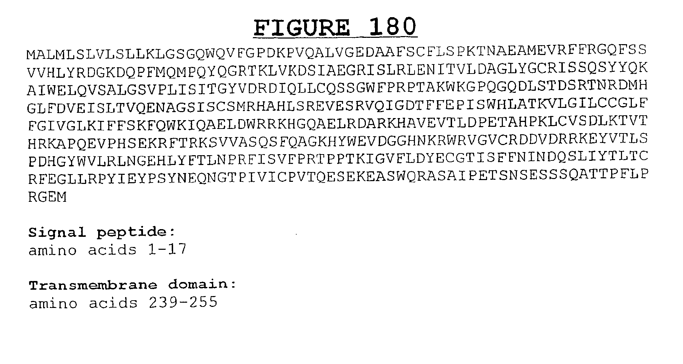

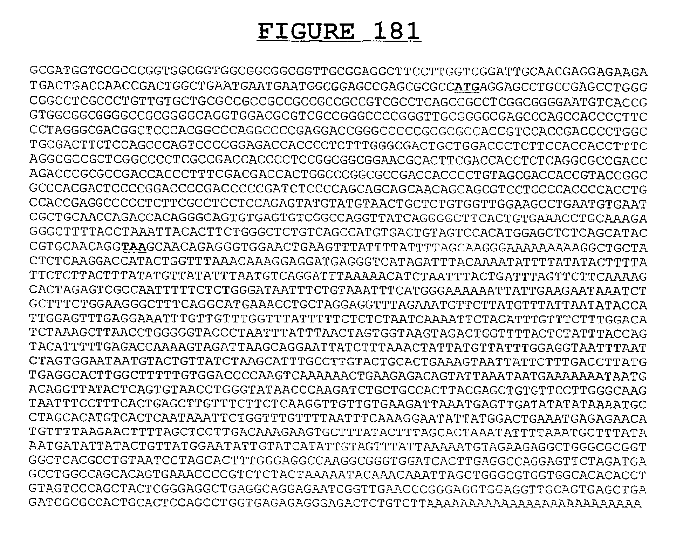

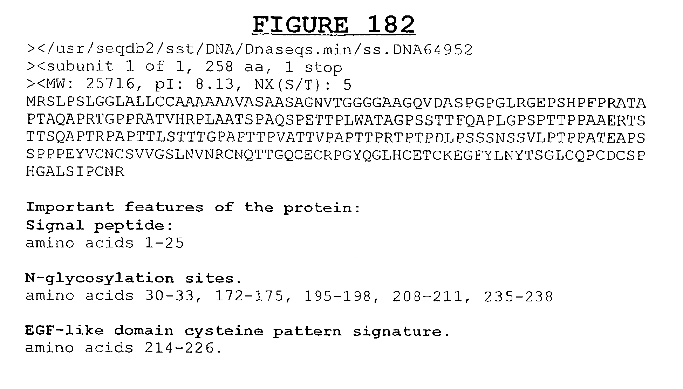

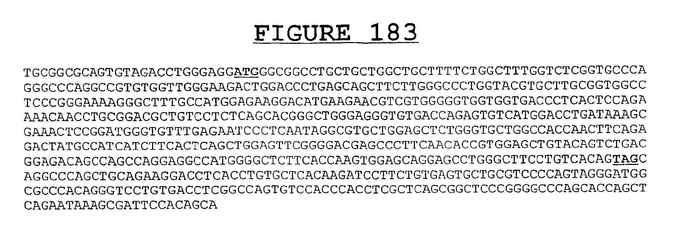

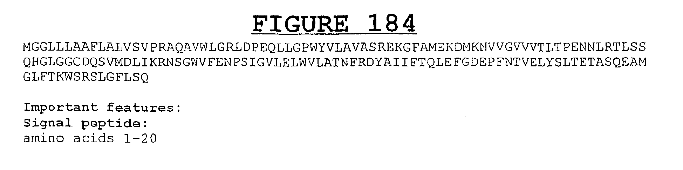

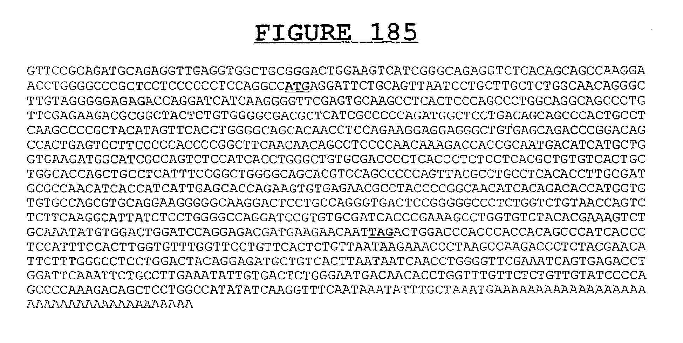

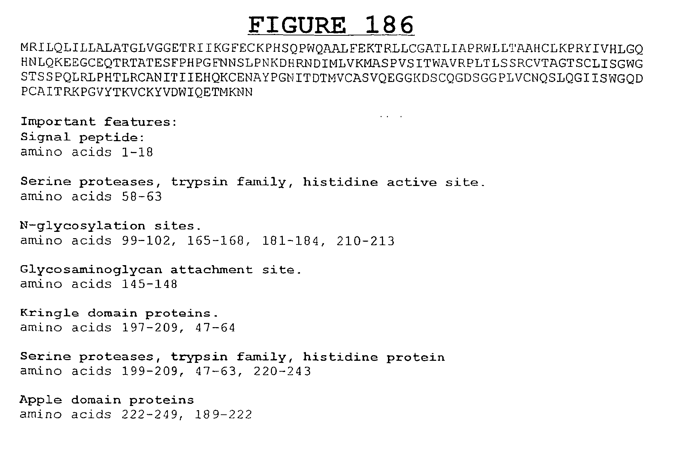

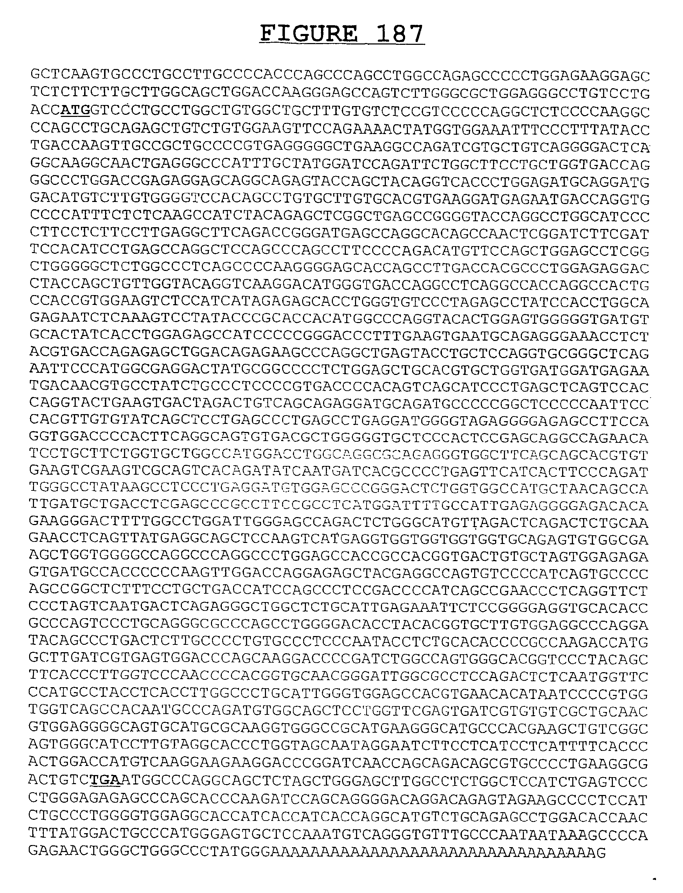

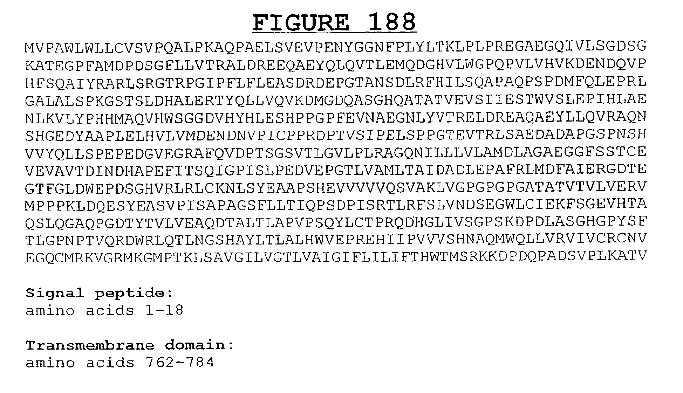

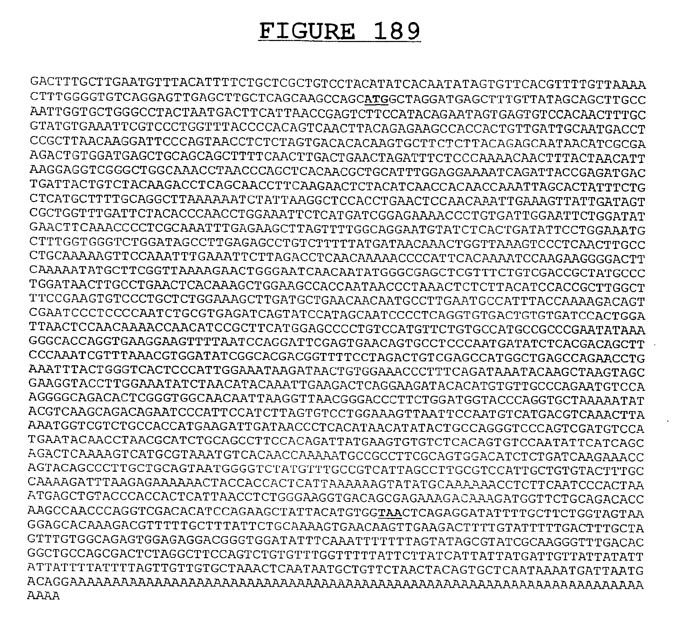

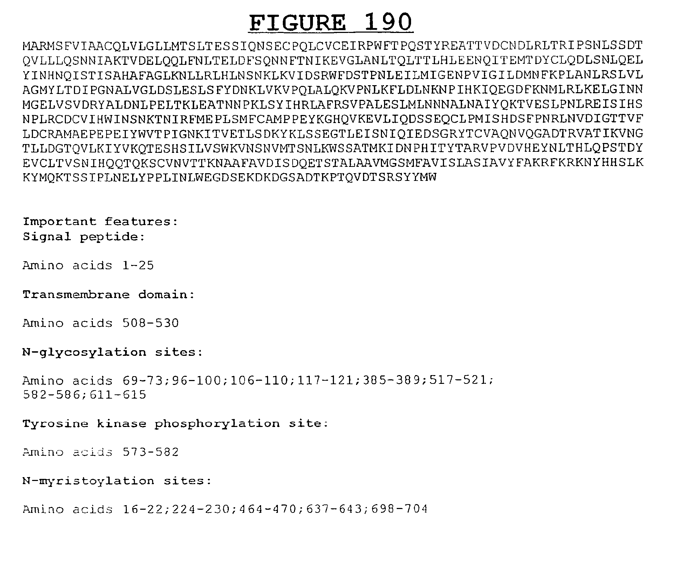

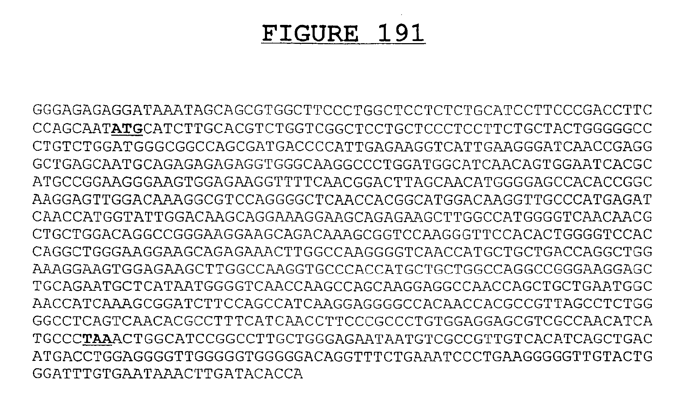

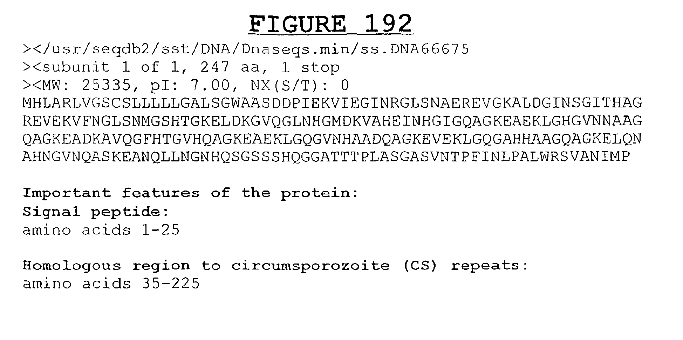

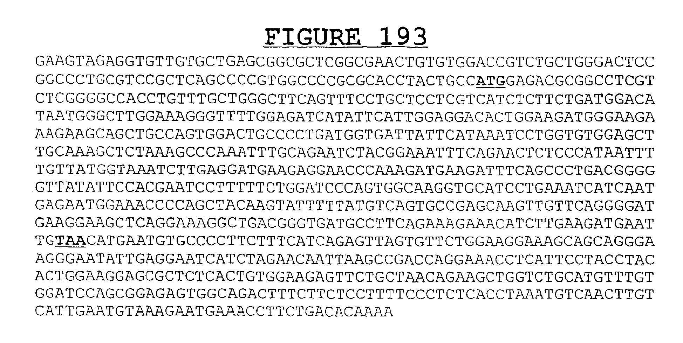

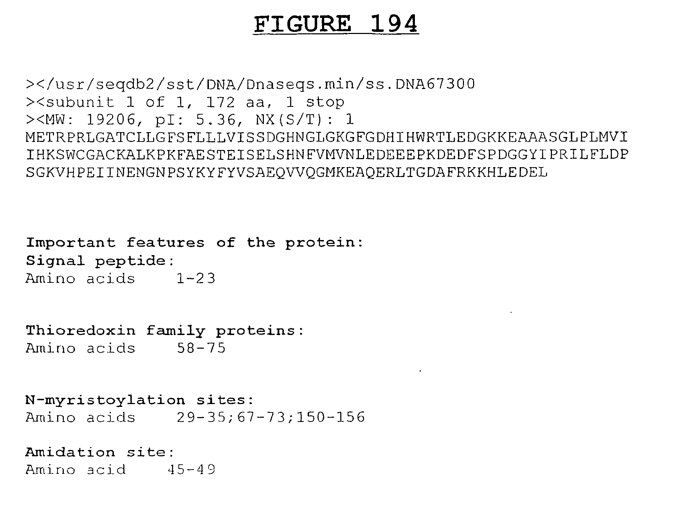

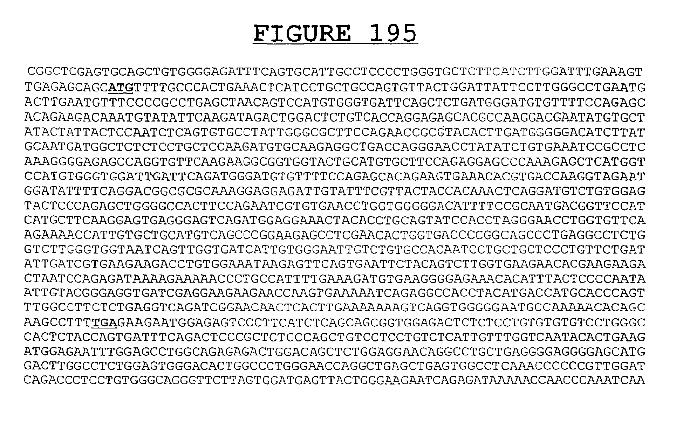

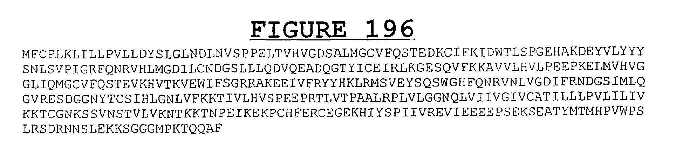

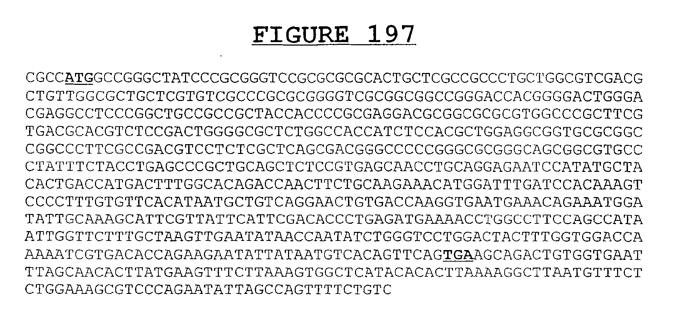

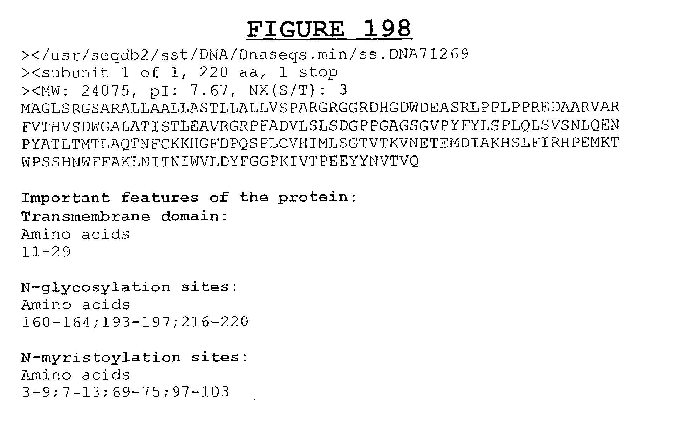

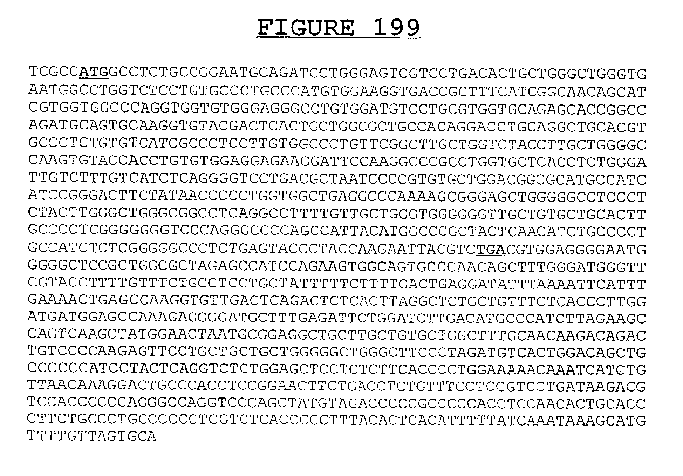

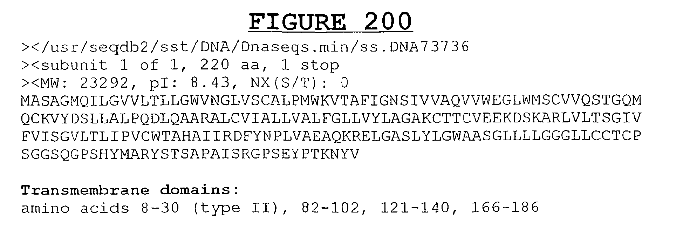

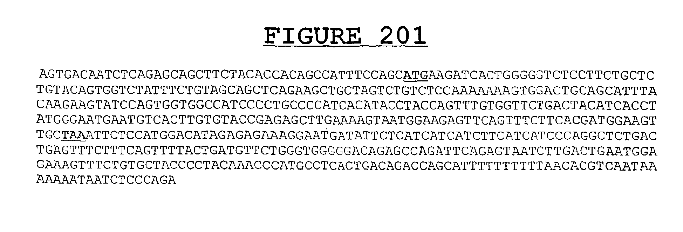

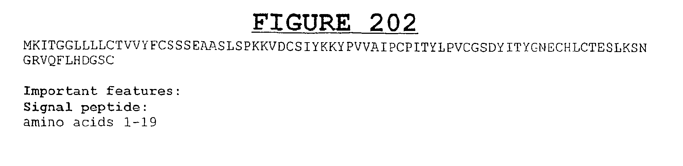

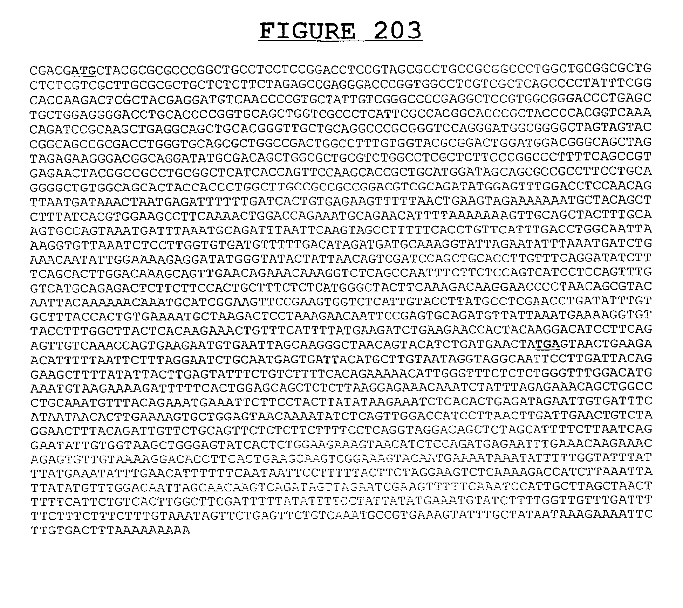

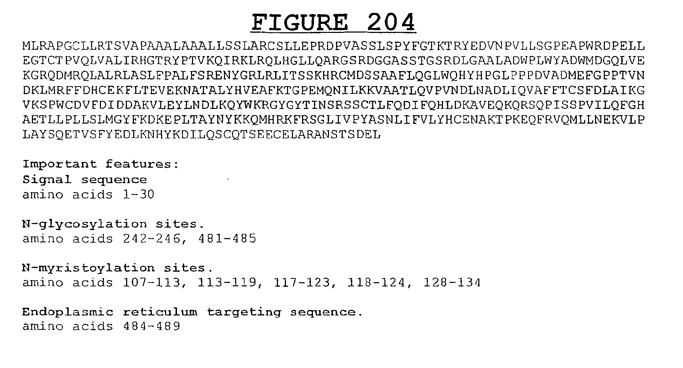

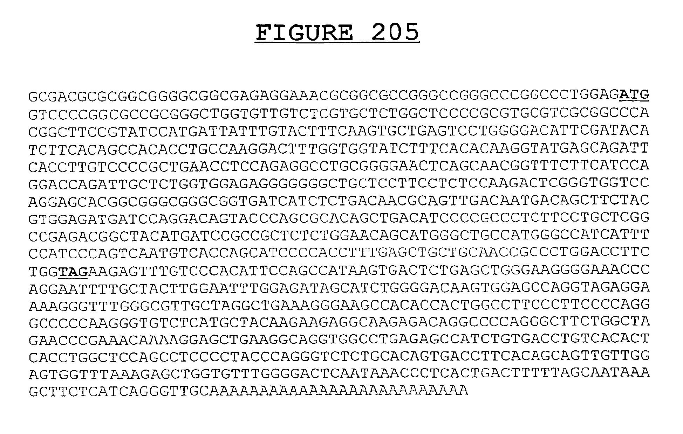

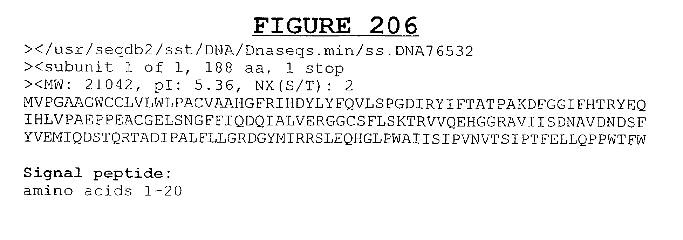

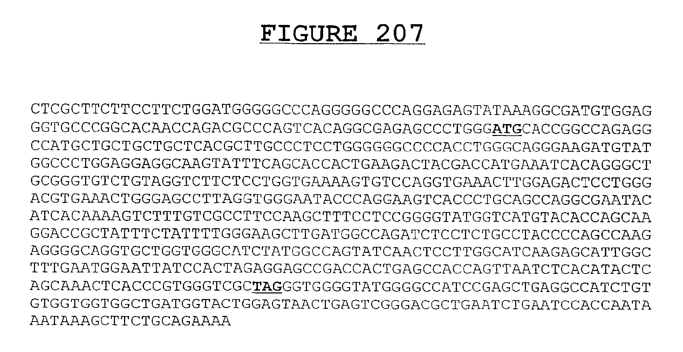

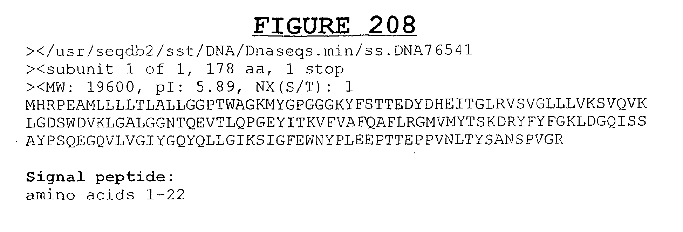

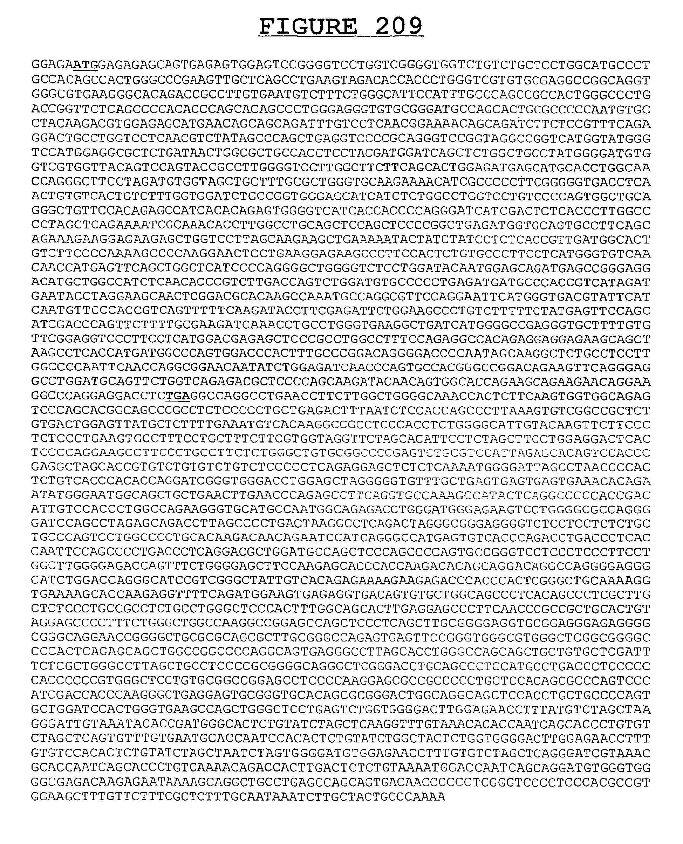

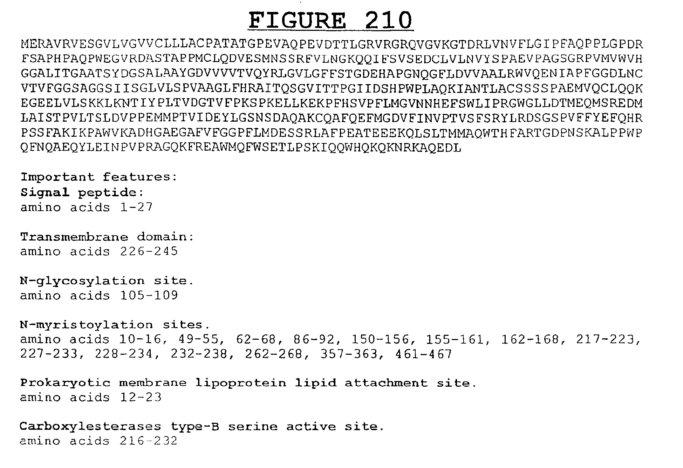

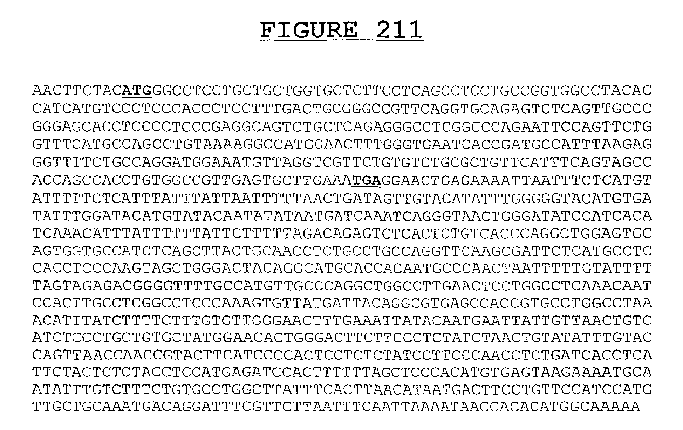

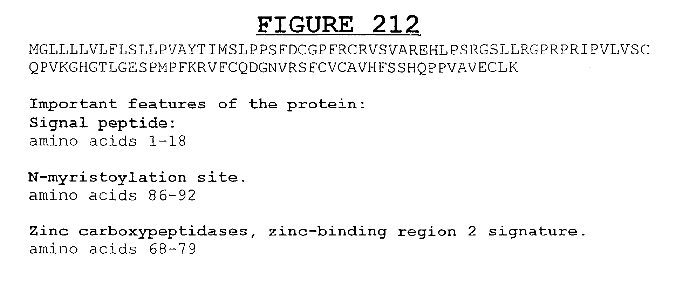

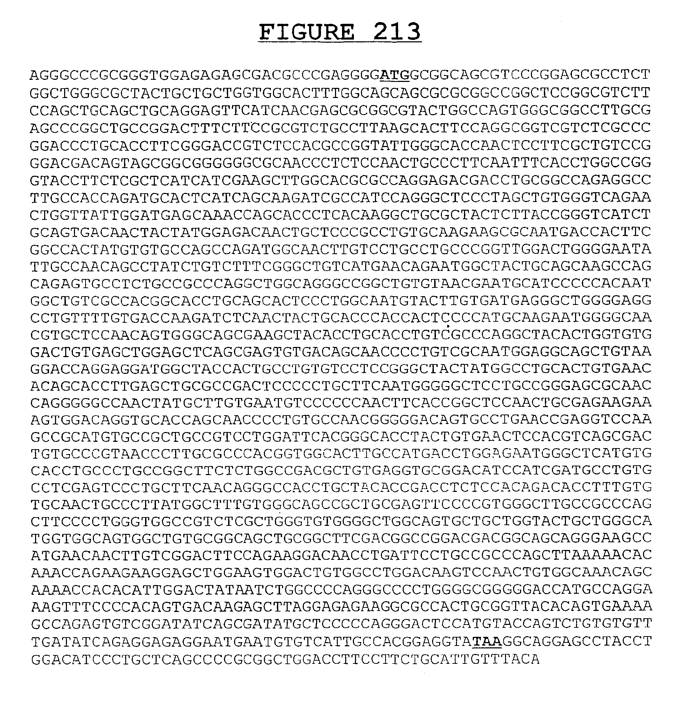

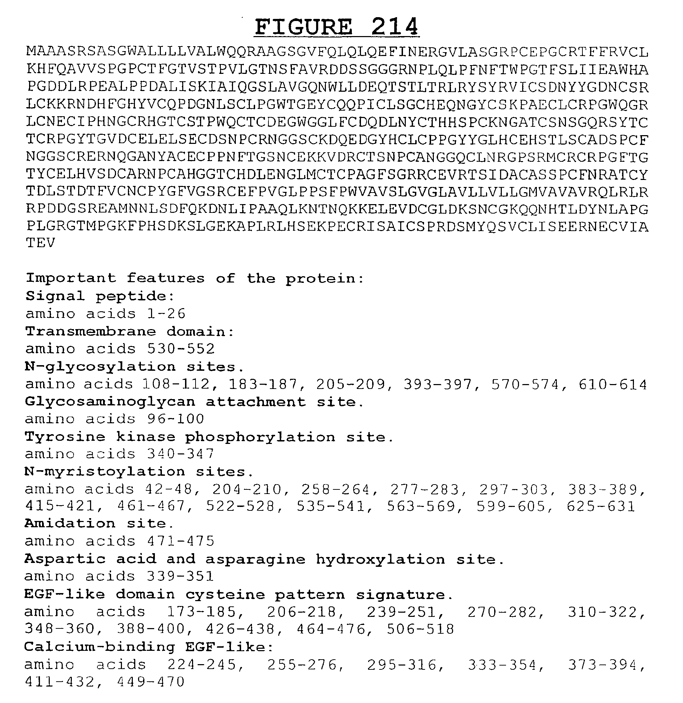

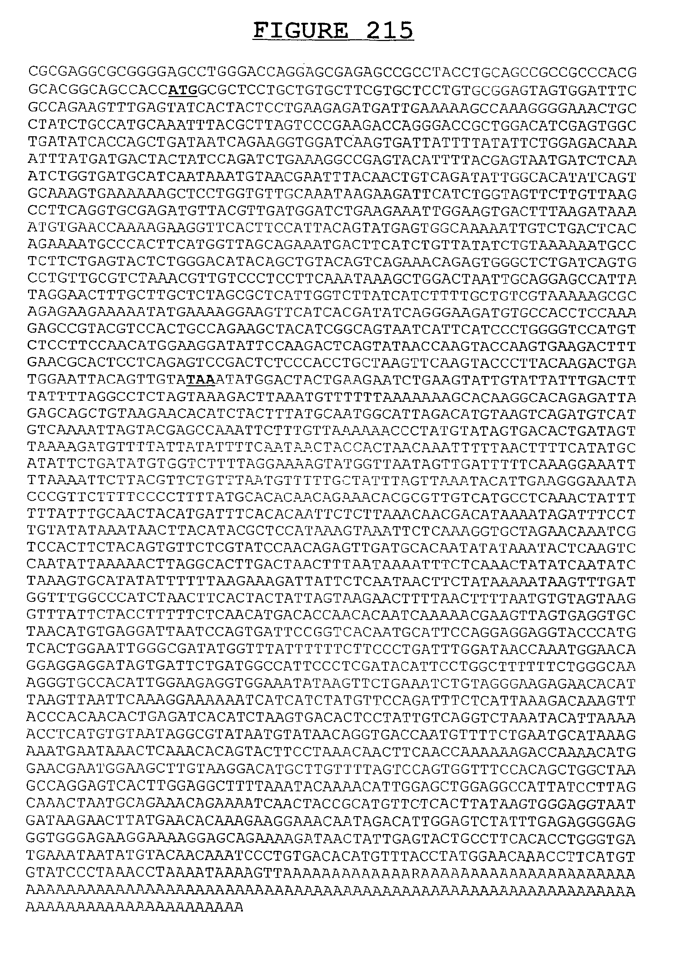

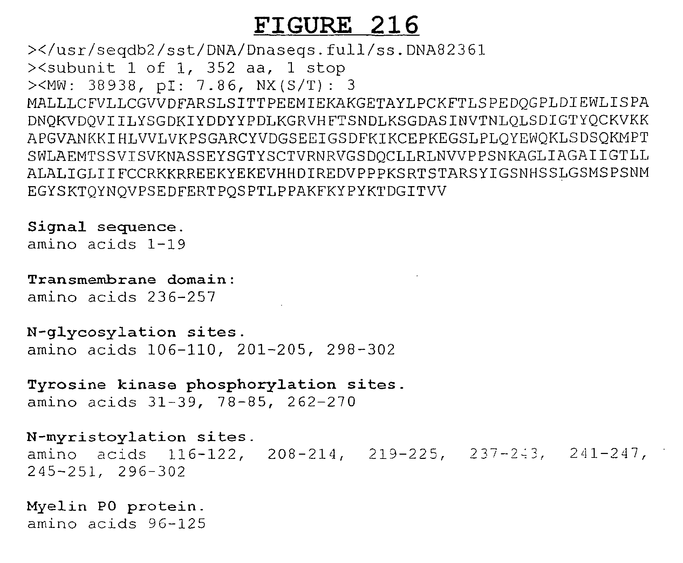

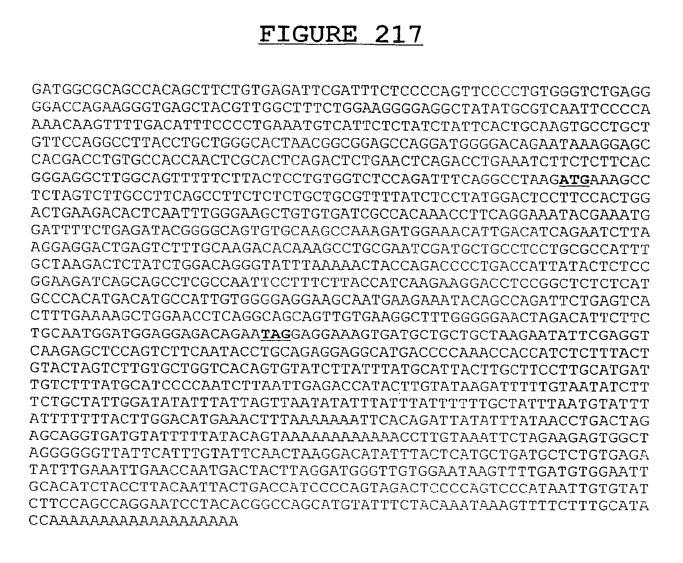

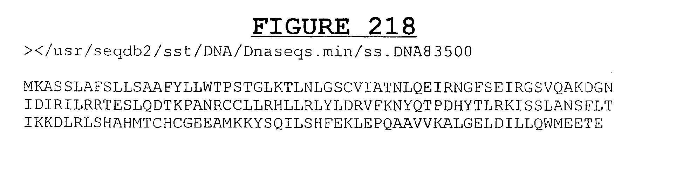

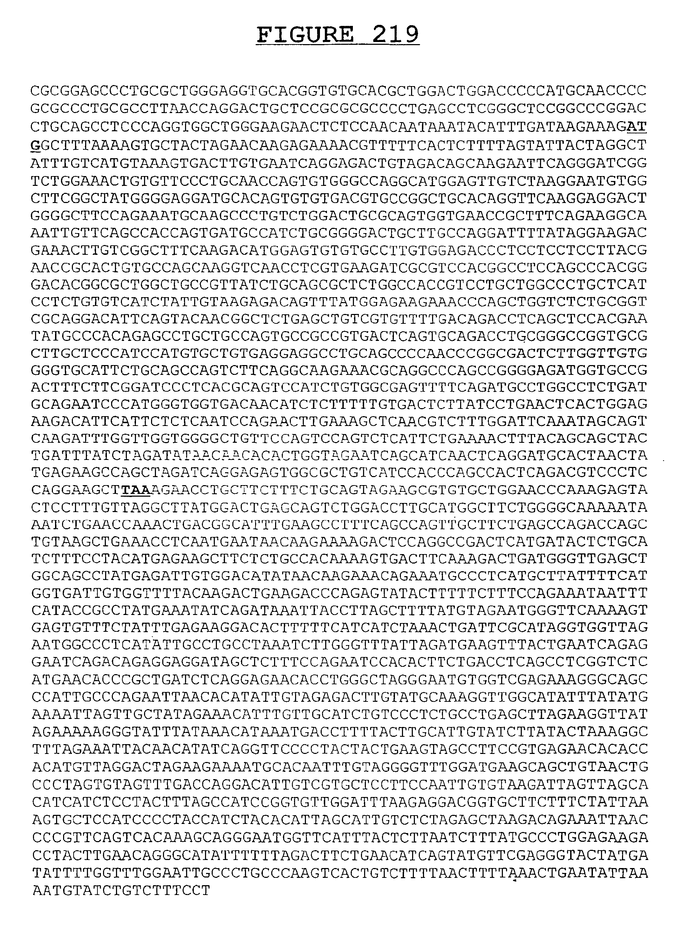

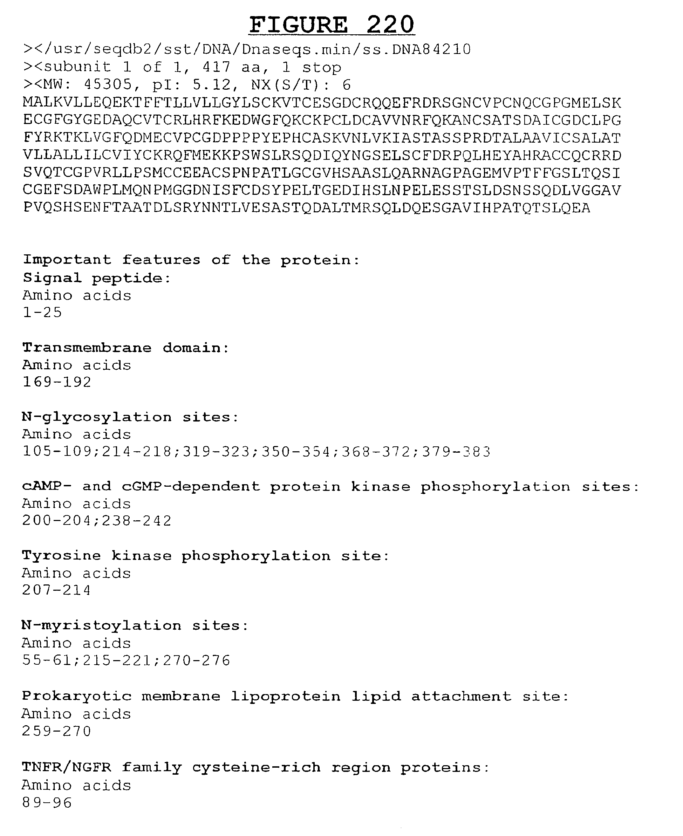

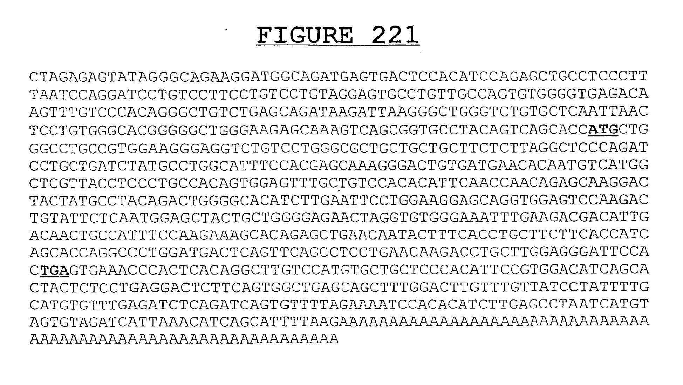

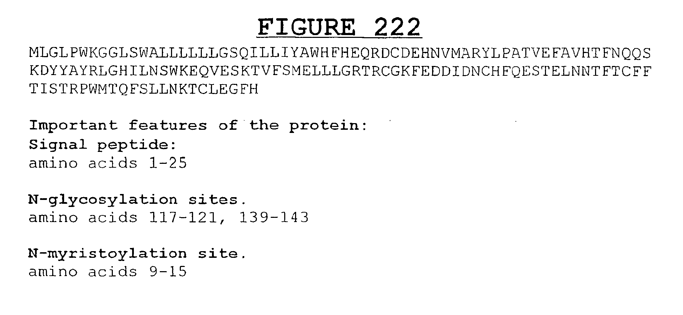

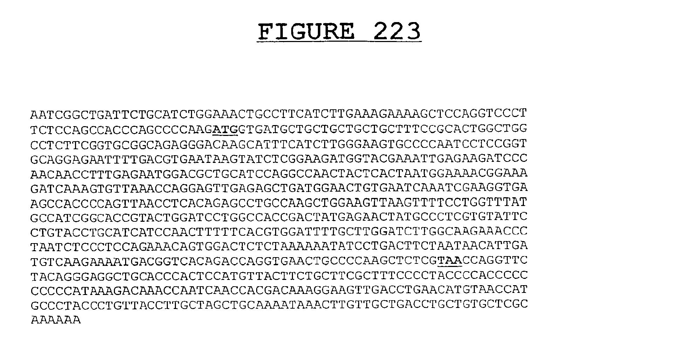

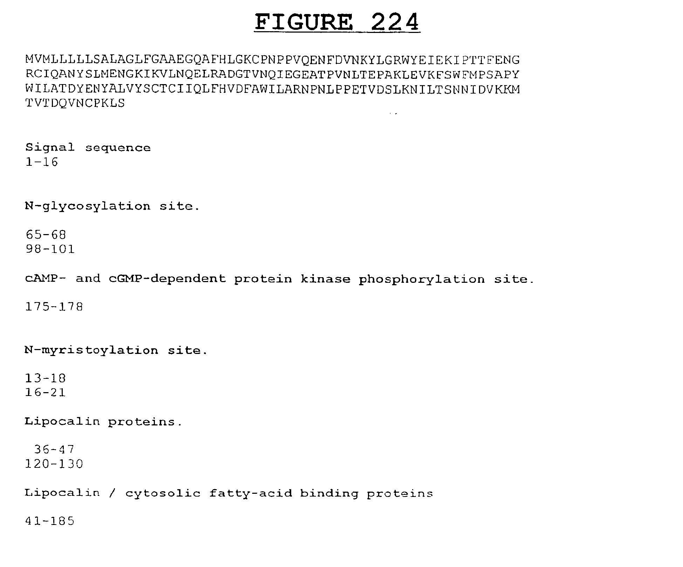

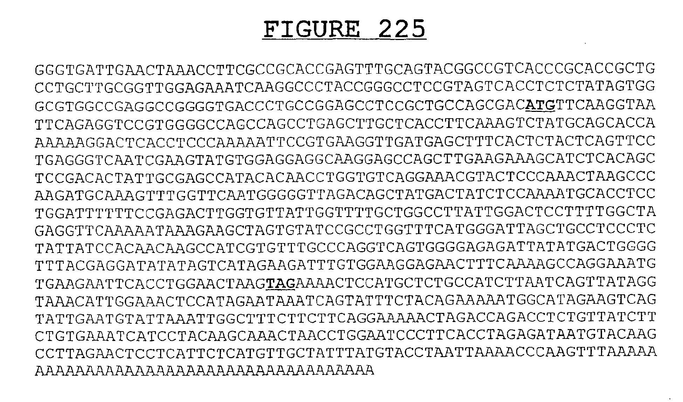

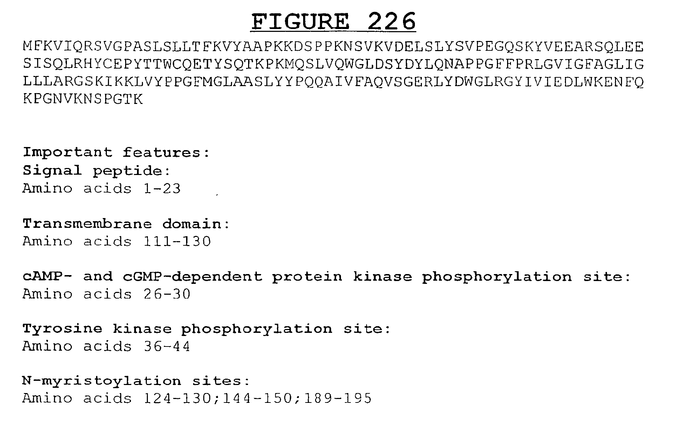

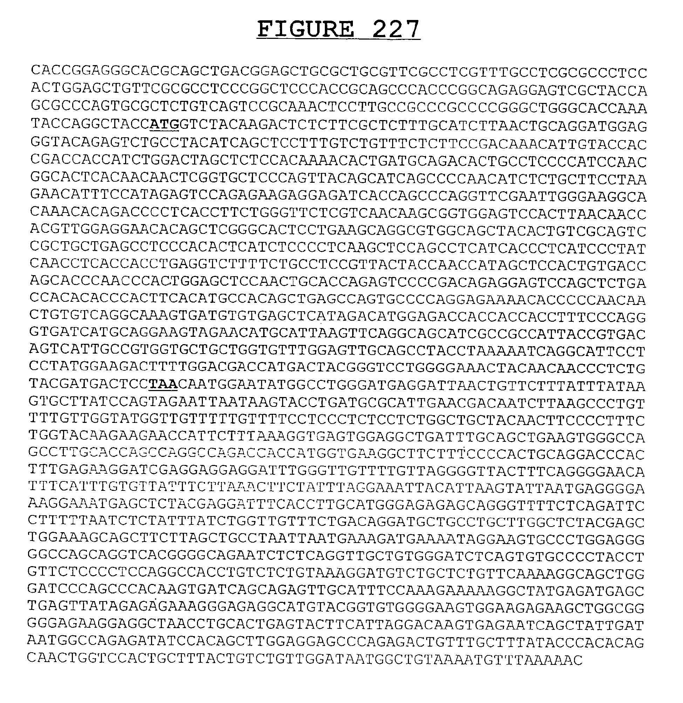

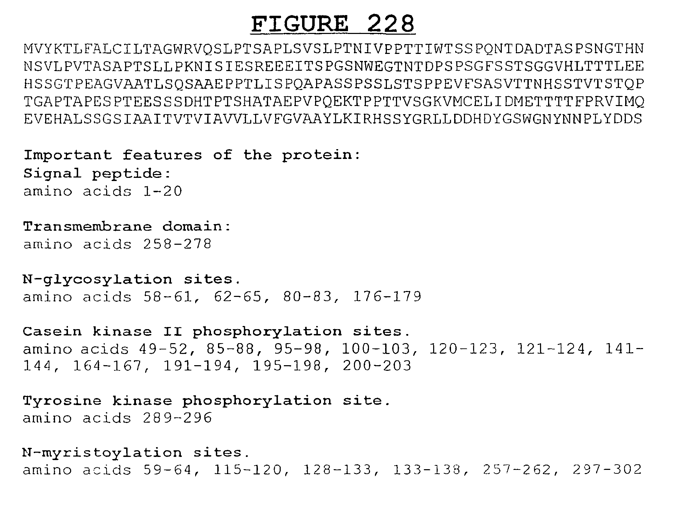

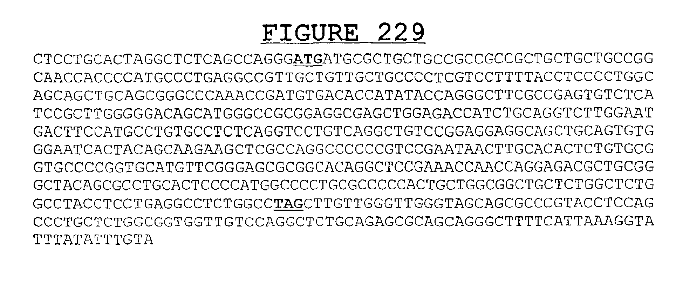

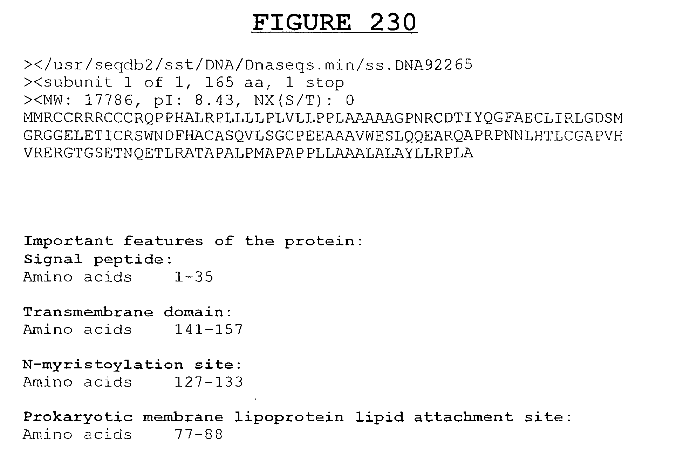

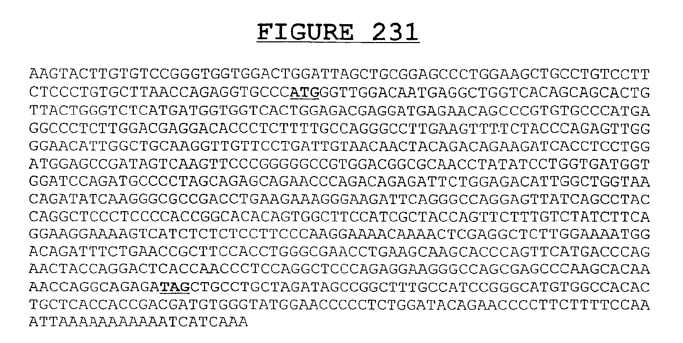

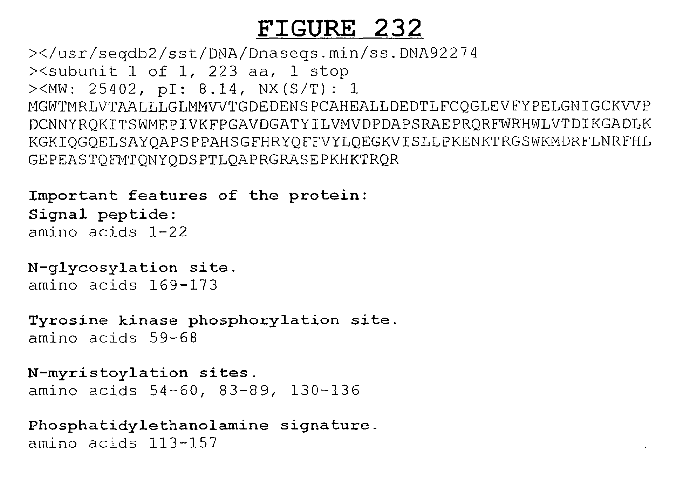

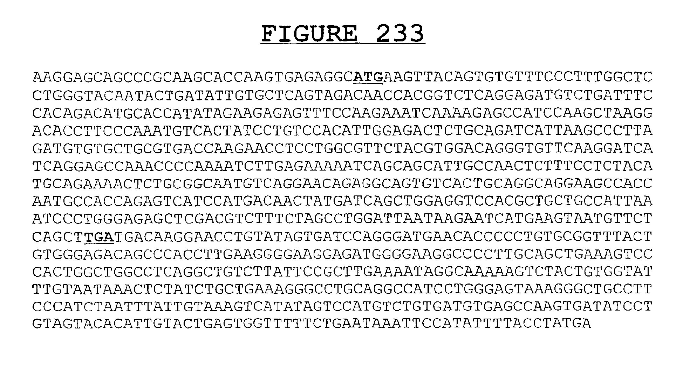

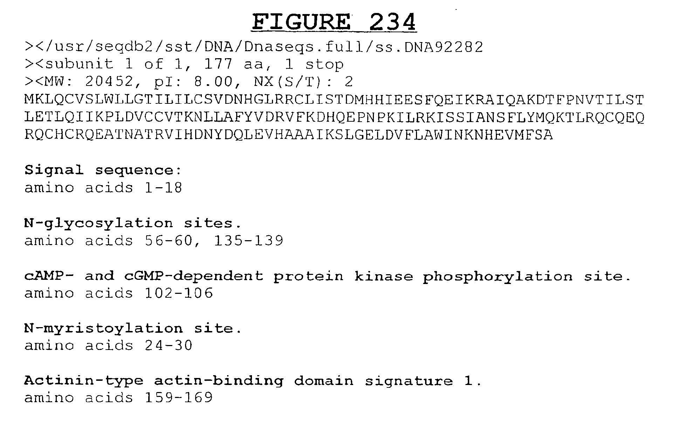

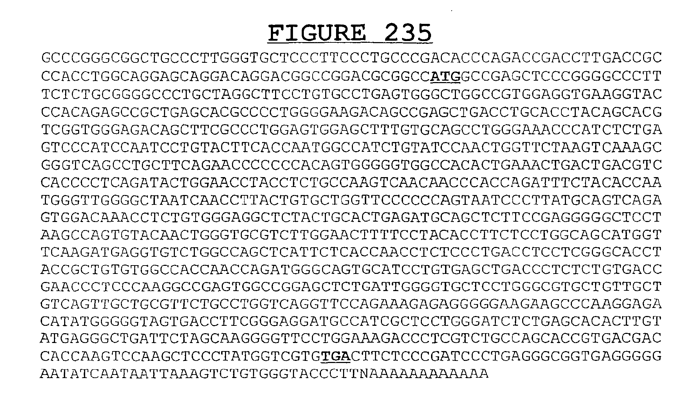

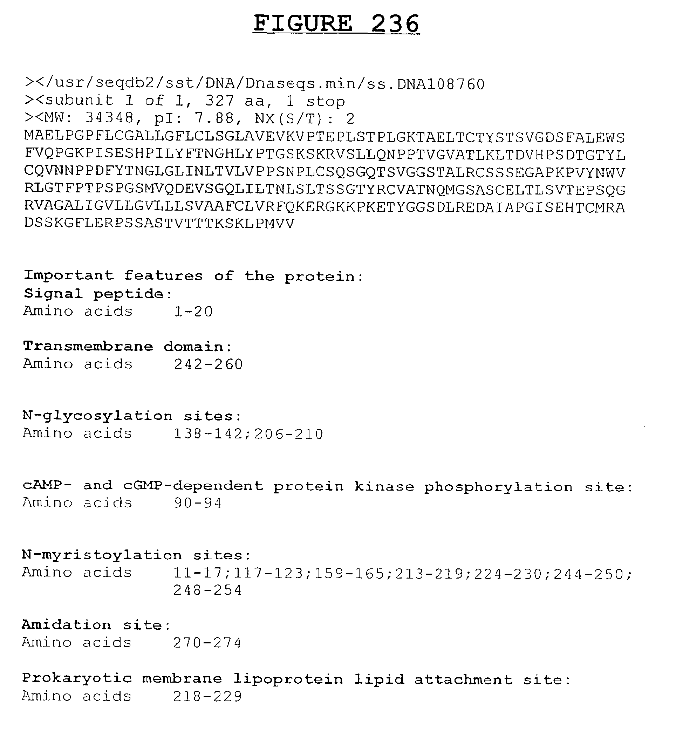

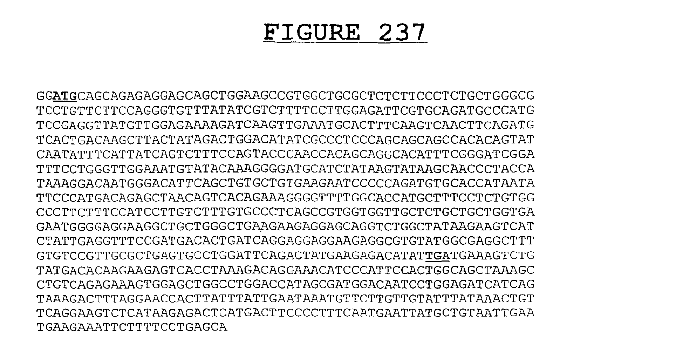

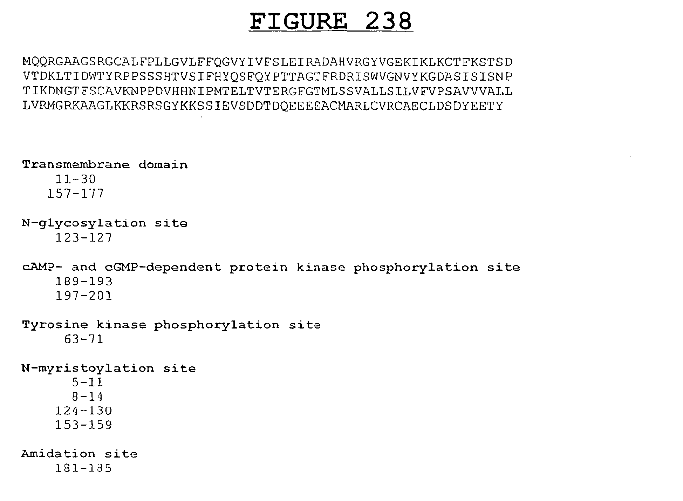

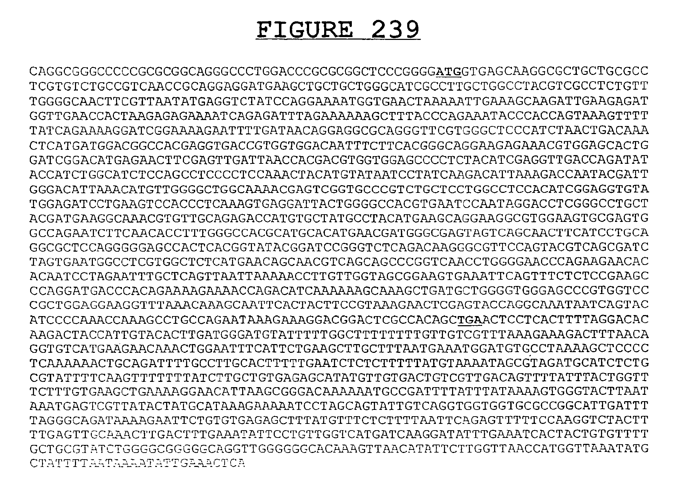

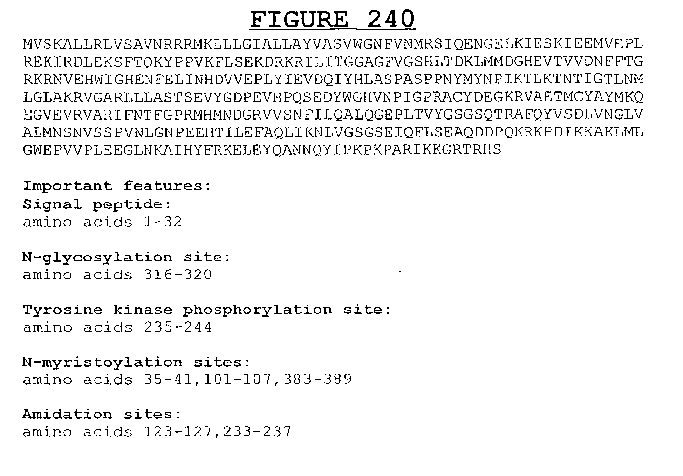

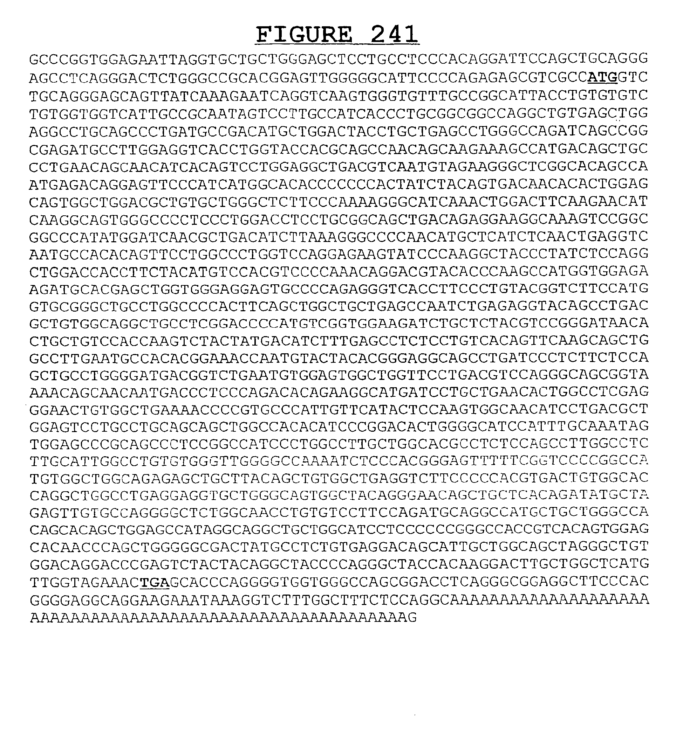

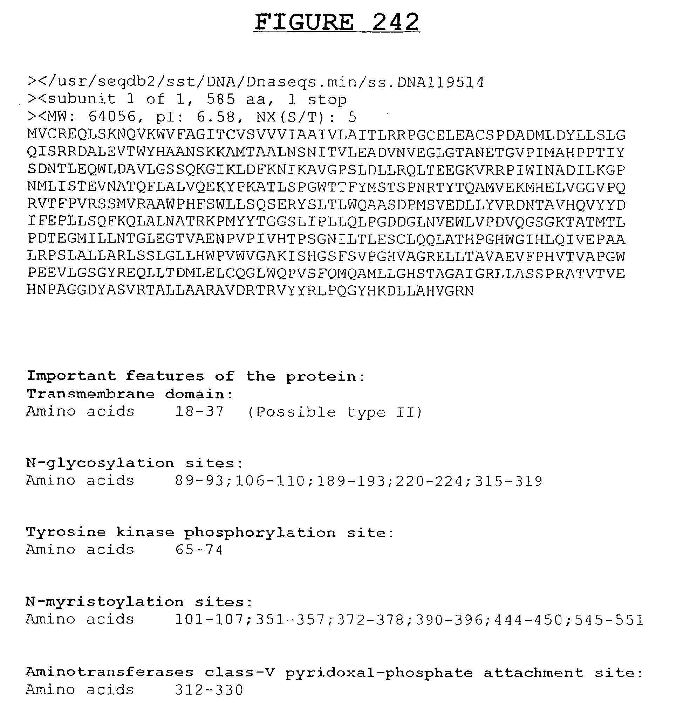

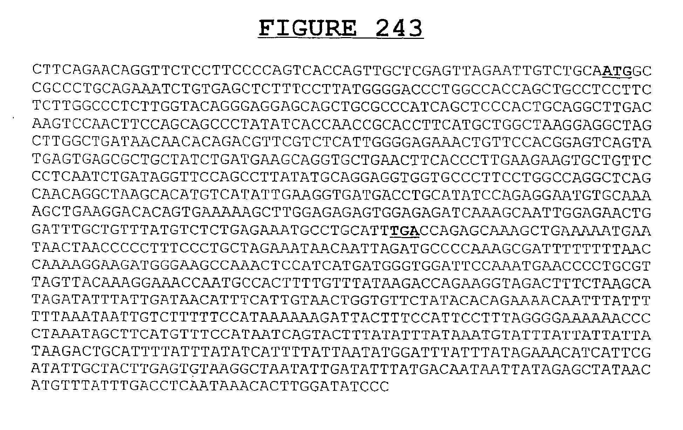

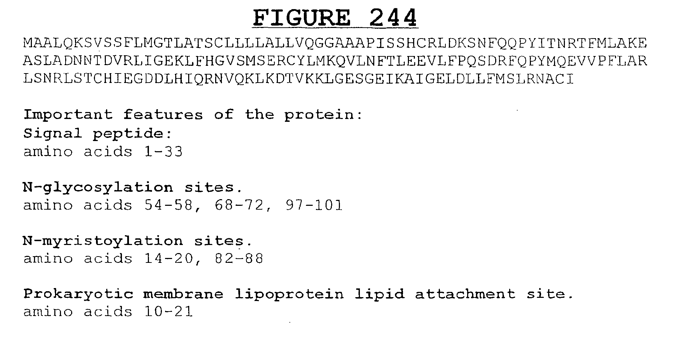

The following clauses also define aspects and embodiments of the invention:
1. Isolated nucleic acid having at least 80% nucleic acid sequence identity to a nucleotide sequence that encodes an amino acid sequence selected from the group consisting of the amino acid sequence shown in Figure 2 (SEQ ID NO:2), Figure 4 (SEQ ID NO:4), Figure 6 (SEQ ID NO:6), Figure 8 (SEQ ID NO:8), Figure 10 (SEQ ID NO:10), Figure 12 (SEQ ID NO:12), Figure 14 (SEQ ID NO:14), Figure 16 (SEQ ID NO:16), Figure 18 (SEQ ID NO:18), Figure 20 (SEQ ID NO:20), Figure 22 (SEQ ID NO:22), Figure 24 (SEQ ID NO:24), Figure 26 (SEQ ID NO:26), Figure 28 (SEQ ID NO:28), Figure 30 (SEQ ID NO:30), Figure 32 (SEQ ID NO:32), Figure 34 (SEQ ID NO:34), Figure 36 (SEQ ID NO:36), Figure 38 (SEQ ID NO:38), Figure 40 (SEQ ID NO:40), Figure 42 (SEQ ID NO:42), Figure 44 (SEQ ID NO:44), Figure 46 (SEQ ID NO:46), Figure 48 (SEQ ID NO:48), Figure 50 (SEQ ID NO:50), Figure 52 (SEQ ID NO:52), Figure 54 (SEQ ID NO:54), Figure 56 (SEQ ID NO:56), Figure 58 (SEQ ID NO:58), Figure 60 (SEQ ID NO:60), Figure 62 (SEQ ID NO:62), Figure 64 (SEQ ID NO:64), Figure 66 (SEQ ID NO:66), Figure 68 (SEQ ID NO:68), Figure 70 (SEQ ID NO:70), Figure 72 (SEQ ID N0:72), Figure 74 (SEQ ID NO:74), Figure 76 (SEQ ID NO:76), Figure 78 (SEQ ID NO:78), Figure 80 (SEQ ID NO:80), Figure 82 (SEQ ID NO:82), Figure 84 (SEQ ID NO:84), Figure 86 (SEQ ID NO:86), Figure 88 (SEQ ID NO:88), Figure 90 (SEQ ID NO:90), Figure 92 (SEQ ID NO:92), Figure 94 (SEQ ID NO:94), Figure 96 (SEQ ID NO:96), Figure 98 (SEQ ID NO:98), Figure 100 (SEQ ID NO:100), Figure 102 (SEQ ID NO:102), Figure 104 (SEQ ID NO:104), Figure 106 (SEQ ID NO:106), Figure 108 (SEQ ID NO:108), Figure 110 (SEQ ID NO:110), Figure 112 (SEQ ID NO:112), Figure 114 (SEQ ID NO:114), Figure 116 (SEQ ID NO:116), Figure 118 (SEQ ID NO:118), Figure 120 (SEQ ID NO:120), Figure 122 (SEQ ID NO:122), Figure 124 (SEQ ID NO:124), Figure 126 (SEQ ID NO:126), Figure 128 (SEQ ID NO:128), Figure 130 (SEQ ID NO:130), Figure 132 (SEQ ID NO:132), Figure 134 (SEQ ID NO:134), Figure 136 (SEQ ID NO:136), Figure 138 (SEQ ID NO:138), Figure 140 (SEQ ID NO:140), Figure 142 (SEQ ID NO:142), Figure 144 (SEQ ID NO:144), Figure 146 (SEQ ID NO:146), Figure 148 (SEQ ID NO:148), Figure 150 (SEQ ID NO:150), Figure 152 (SEQ ID NO:152), Figure 154 (SEQ ID NO:154), Figure 156 (SEQ ID NO:156), Figure 158 (SEQ ID NO:158), Figure 160 (SEQ ID NO:160), Figure 162 (SEQ ID NO:162), Figure 164 (SEQ ID NO:164), Figure 166 (SEQ ID NO:166), Figure 168 (SEQ ID NO:168), Figure 170 (SEQ ID NO:170), Figure 172 (SEQ ID NO:172), Figure 174 (SEQ ID NO:174), Figure 176 (SEQ ID NO:176), Figure 178 (SEQ ID NO:178), Figure 180 (SEQ ID NO:180), Figure 182 (SEQ ID NO:182), Figure 184 (SEQ ID NO:184), Figure 186 (SEQ ID NO:186), Figure 188 (SEQ ID NO:188), Figure 190 (SEQ ID NO:190), Figure 192 (SEQ ID NO:192), Figure 194 (SEQ ID NO:194), Figure 196 (SEQ ID NO:196), Figure 198 (SEQ ID NO:198), Figure 200 (SEQ ID NO:200), Figure 202 (SEQ ID NO:202), Figure 204 (SEQ ID NO:204), Figure 206 (SEQ ID NO:206), Figure 208 (SEQ ID NO:208), Figure 210 (SEQ ID NO:210), Figure 212 (SEQ ID NO:212), Figure 214 (SEQ ID NO:214), Figure 216 (SEQ ID NO:216), Figure 218 (SEQ ID NO:218), Figure 220 (SEQ ID NO:220), Figure 222 (SEQ ID NO:222), Figure 224 (SEQ ID NO:224), Figure 226 (SEQ ID NO:226), Figure 228 (SEQ ID NO:228), Figure 230 (SEQ ID NO:230), Figure 232 (SEQ ID NO:232), Figure 234 (SEQ ID NO:234), Figure 236 (SEQ ID NO:236), Figure 238 (SEQ ID NO:238), Figure 240 (SEQ ID NO:240), Figure 242 (SEQ ID NO:242), and Figure 244 (SEQ ID NO:244).
2. Isolated nucleic acid having at least 80% nucleic acid sequence identity to a nucleotide sequence selected from the group consisting of the nucleotide sequence shown in Figures 1A-1B (SEQ ID NO:1), Figure 3 (SEQ ID NO:3), Figure 5 (SEQ ID NO:5), Figure 7 (SEQ ID NO:7), Figure 9 (SEQ ID NO:9), Figure 11 (SEQ ID NO:11), Figure 13 (SEQ ID NO:13), Figure 15 (SEQ ID NO:15), Figure 17 (SEQ ID NO:17), Figure 19 (SEQ ID NO:19), Figure 21 (SEQ ID NO:21), Figure 23 (SEQ ID NO:23), Figure 25 (SEQ ID NO:25), Figure 27 (SEQ ID NO:27), Figure 29 (SEQ ID NO:29), Figure 31 (SEQ ID NO:31), Figure 33 (SEQ ID NO:33), Figure 35 (SEQ ID NO:35), Figure 37 (SEQ ID NO:37), Figure 39 (SEQ ID NO:39), Figure 41 (SEQ ID NO:41) Figure 43 (SEQ ID NO:43), Figure 45 (SEQ ID NO:45) Figure 47 (SEQ ID NO:47), Figure 49 (SEQ ID NO:49), Figure 51 (SEQ ID NO:51), Figure 53 (SEQ ID NO:53), Figure 55 (SEQ ID NO:55), Figure 57 (SEQ ID NO:57), Figures 59A-59B (SEQ ID NO:59), Figure 61 (SEQ ID NO:61), Figure 63 (SEQ ID NO:63), Figure 65 (SEQ ID NO:65), Figure 67 (SEQ ID NO:67), Figure 69 (SEQ ID NO:69), Figure 71 (SEQ ID NO:71), Figure 73 (SEQ ID NO:73), Figure 75 (SEQ ID NO:75), Figure 77 (SEQ ID NO:77), Figure 79 (SEQ ID NO:79), Figure 81 (SEQ ID NO:81), Figure 83 (SEQ ID NO:83), Figure 85 (SEQ ID NO:85), Figure 87 (SEQ ID NO:87), Figure 89 (SEQ ID NO:89), Figure 91 (SEQ ID NO:91), Figure 93 (SEQ ID NO:93), Figure 95 (SEQ ID NO:95), Figure 97 (SEQ ID NO:97), Figure 99 (SEQ ID NO:99), Figure 101 (SEQ ID NO:101), Figure 103 (SEQ ID NO:103), Figure 105 (SEQ ID NO:105), Figure 107 (SEQ ID NO:107), Figure 109 (SEQ ID NO:109), Figure 111 (SEQ ID NO:111), Figure 113 (SEO ID NO:113), Figure 115 (SEQ ID NO:115), Figure 117 (SEQ ID NO:117), Figure 119 (SEQ ID NO:119), Figure 121 (SEQ ID NO:121), Figure 123 (SEQ ID NO:123), Figure 125 (SEQ ID NO:125), Figure 127 (SEQ ID NO:127), Figure 129 (SEQ ID NO:129), Figure 131 (SEQ ID NO:131), Figure 133 (SEQ ID NO:133), Figure 135 (SEQ ID NO:135), Figure 137 (SEQ ID NO:137), Figure 139 (SEQ ID NO:139), Figure 141 (SEQ ID NO:141), Figure 143 (SEQ ID NO:143), Figure 145 (SEQ ID NO:145), Figure 147 (SEQ ID NO:147), Figure 149 (SEQ ID NO:149), Figure 151 (SEQ ID NO:151), Figure 153 (SEQ ID NO:153), Figure 155 (SEQ ID NO:155), Figure 157 (SEQ ID NO:157), Figure 159 (SEQ ID NO:159), Figure 161 (SEQ ID NO:161), Figure 163 (SEQ ID NO:163), Figure 165 (SEQ ID NO:165), Figure 167 (SEQ ID NO:167), Figure 169 (SEQ ID NO:169), Figure 171 (SEQ ID NO:171), Figure 173 (SEQ ID NO:173), Figure 175 (SEQ ID NO:175), Figure 177 (SEQ ID NO:177), Figure 179 (SEQ ID NO:179), Figure 181 (SEQ ID NO:181), Figure 183 (SEQ ID NO:183), Figure 185 (SEQ ID NO:185), Figure 187 (SEQ ID NO:187), Figure 189 (SEQ ID NO:189), Figure 191 (SEQ ID NO:191 Figure 193 (SEQ ID NO:193), Figure 195 (SEQ ID NO:195), Figure 197 (SEQ ID NO:197), Figure 199 (SEQ ID NO:199), Figure 201 (SEQ ID NO:201), Figure 203 (SEQ ID NO:203), Figure 205 (SEQ ID NO:205), Figure 207 (SEQ ID NO:207), Figure 209 (SEQ ID NO:209), Figure 211 (SEQ ID NO:211), Figure 213 (SEO ID NO:213), Figure 215 (SEQ ID NO:215), Figure 217 (SEQ ID NO:217), Figure 219 (SEQ ID NO:219), Figure 221 (SEQ ID NO:221), Figure 223 (SEQ ID NO:223), Figure 225 (SEQ ID NO:225), Figure 227 (SEQ ID NO:227), Figure 229 (SEQ ID NO:229), Figure 231 (SEQ ID NO:231), Figure 233 (SEQ ID NO:233), Figure 235 (SEQ ID NO:235), Figure 237 (SEQ ID NO:237), Figure 239 (SEQ ID NO:239), Figure 241 (SEQ ID NO:241), and Figure 243 (SEQ ID NO:243).
3. Isolated nucleic acid having at least 80% nucleic acid sequence identity to a nucleotide sequence selected from the group consisting of the full-length coding sequence of the nucleotide sequence shown in Figures 1A-1B (SEQ ID NO:1), Figure 3 (SEQ ID NO:3), Figure 5 (SEQ ID NO:5), Figure 7 (SEQ ID NO:7), Figure 9 (SEQ ID NO:9), Figure 11 (SEQ ID NO:11), Figure 13 (SEQ ID NO:13), Figure 15 (SEQ ID NO:15), Figure 17 (SEQ ID NO:17), Figure 19 (SEQ ID NO:19), Figure 21 (SEQ ID NO:21), Figure 23 (SEQ ID NO:23), Figure 25 (SEQ ID NO:25), Figure 27 (SEQ ID NO:27), Figure 29 (SEQ ID NO:29), Figure 31 (SEQ ID NO:31), Figure 33 (SEQ ID NO:33), Figure 35 (SEQ ID NO:35), Figure 37 (SEQ ID NO:37), Figure 39 (SEQ ID NO:39), Figure 41 (SEQ ID NO:41), Figure 43 (SEQ ID NO:43), Figure 45 (SEQ ID NO:45), Figure 47 (SEQ ID NO:47), Figure 49 (SEQ ID NO:49), Figure 51 (SEQ ID NO:51), Figure 53 (SEQ ID NO:53), Figure 55 (SEQ ID NO:55), Figure 57 (SEQ ID NO:57), Figures 59A-59B (SEQ ID NO:59), Figure 61 (SEQ ID NO:61), Figure 63 (SEQ ID NO:63), Figure 65 (SEQ ID NO:65), Figure 67 (SEQ ID NO:67), Figure 69 (SEQ ID NO:69), Figure 71 (SEQ ID NO:71), Figure 73 (SEQ ID NO:73), Figure 75 (SEQ ID NO:75), Figure 77 (SEQ ID NO:77), Figure 79 (SEQ ID NO:79), Figure 81 (SEQ ID NO:81), Figure 83 (SEQ ID NO:83), Figure 85 (SEQ ID NO:85), Figure 87 (SEQ ID NO:87), Figure 89 (SEQ ID NO:89), Figure 91 (SEQ ID NO:91), Figure 93 (SEQ ID NO:93), Figure 95 (SEQ ID NO:95), Figure 97 (SEQ ID NO:97), Figure 99 (SEQ ID NO:99), Figure 101 (SEQ ID NO:101), Figure 103 (SEQ ID NO:103), Figure 105 (SEQ ID NO:105), Figure 107 (SEQ ID NO:107), Figure 109 (SEQ ID NO:109), Figure 111 (SEQ ID NO:111), Figure 113 (SEQ ID NO:113), Figure 115 (SEQ ID NO:115), Figure 117 (SEQ ID NO:117), Figure 119 (SEQ ID NO:119), Figure 121 (SEQ ID NO:121), Figure 123 (SEQ ID NO:123), Figure 125 (SEQ ID NO:125), Figure 127 (SEQ ID NO:127), Figure 129 (SEQ ID NO:129), Figure 131 (SEQ ID NO:131), Figure 133 (SEO ID NO:133), Figure 135 (SEQ ID NO:135), Figure 137 (SEQ ID NO:137), Figure 139 (SEQ ID NO:139), Figure 141 (SEQ ID NO:141), Figure 143 (SEQ ID NO:143), Figure 145 (SEQ ID NO:145), Figure 147 (SEQ ID NO:147), Figure 149 (SEQ ID NO:149), Figure 151 (SEQ ID NO:151), Figure 153 (SEQ ID NO:153), Figure 155 (SEQ ID NO:155), Figure 157 (SEQ ID NO:157), Figure 159 (SEQ ID NO:159), Figure 161 (SEQ ID NO:161), Figure 163 (SEQ ID NO:163), Figure 165 (SEQ ID NO:165), Figure 167 (SEQ ID NO:167), Figure 169 (SEQ ID NO:169), Figure 171 (SEQ ID NO:171), Figure 173 (SEQ ID NO:173), Figure 175 (SEQ ID NO:175), Figure 177 (SEQ ID NO:177), Figure 179 (SEQ ID NO:179), Figure 181 (SEQ ID NO:181), Figure 183 (SEQ ID NO:183), Figure 185 (SEQ ID NO:185), Figure 187 (SEQ ID NO:187), Figure 189 (SEQ ID NO:189), Figure 191 (SEQ ID NO:191), Figure 193 (SEQ ID NO:193), Figure 195 (SEQ ID NO:195), Figure 197 (SEQ ID NO:197), Figure 199 (SEQ ID NO:199), Figure 201 (SEQ ID NO:201), Figure 203 (SEQ ID NO:203), Figure 205 (SEQ ID NO:205), Figure 207 (SEQ ID NO:207), Figure 209 (SEQ ID NO:209), Figure 211 (SEQ ID NO:211), Figure 213 (SEQ ID NO:213), Figure 215 (SEO ID NO:215), Figure 217 (SEQ ID NO:217), Figure 219 (SEQ ID NO:219), Figure 221 (SEQ ID NO:221), Figure 223 (SEQ ID NO:223), Figure 225 (SEQ ID NO:225), Figure 227 (SEQ ID NO:227), Figure 229 (SEQ ID NO:229), Figure 231 (SEQ ID NO:231), Figure 233 (SEQ ID NO:233), Figure 235 (SEQ ID NO:235), Figure 237 (SEQ ID NO:237), Figure 239 (SEQ ID NO:239), Figure 241 (SEQ ID NO:241), and Figure 243 (SEQ ID NO:243).
4. Isolated nucleic acid having at least 80% nucleic acid sequence identity to the full-length coding sequence of the DNA deposited under any ATCC accession number shown in Table 7.
5. A vector comprising the nucleic acid of Clause 1.
6. A host cell comprising the vector of Clause 5.
7. The host cell of Clause 6, wherein said cell is a CHO cell.
8. The host cell of Clause 6, wherein said cell is an E. *coli.*
9. The host cell of Clause 6, wherein said cell is a yeast cell.
10. A process for producing a PRO polypeptide comprising culturing the host cell of Clause 6 under conditions suitable for expression of said PRO polypeptide and recovering said PRO polypeptide from the cell culture.
11. An isolated polypeptide having at least 80% amino acid sequence identity to an amino acid sequence selected from the group consisting of the amino acid sequence shown in Figure 2 (SEQ ID NO:2), Figure 4 (SEQ ID NO:4), Figure 6 (SEQ ID NO:6), Figure 8 (SEQ ID NO:8), Figure 10 (SEQ ID NO:10), Figure 12 (SEQ ID NO:12), Figure 14 (SEQ ID NO:14), Figure 16 (SEQ ID NO:16), Figure 18 (SEQ ID NO:18), Figure 20 (SEQ ID NO:20), Figure 22 (SEQ ID NO:22), Figure 24 (SEQ ID NO:24), Figure 26 (SEQ ID NO:26), Figure 28 (SEQ ID NO:28), Figure 30 (SEQ ID NO:30), Figure 32 (SEQ ID NO:32), Figure 34 (SEQ ID NO:34), Figure 36 (SEQ ID NO:36), Figure 38 (SEQ ID NO:38), Figure 40 (SEQ ID NO:40), Figure 42 (SEQ ID NO:42), Figure 44 (SEQ ID NO:44), Figure 46 (SEQ ID NO:46), Figure 48 (SEQ ID NO:48), Figure 50 (SEQ ID NO:50), Figure 52 (SEQ ID NO:52), Figure 54 (SEQ ID NO:54), Figure 56 (SEQ ID NO:56), Figure 58 (SEQ ID NO:58), Figure 60 (SEQ ID NO:60), Figure 62 (SEQ ID NO:62), Figure 64 (SEQ ID NO:64), Figure 66 (SEQ ID NO:66), Figure 68 (SEQ ID NO:68), Figure 70 (SEQ ID NO:70), Figure 72 (SEQ ID NO:72), Figure 74 (SEQ ID NO:74), Figure 76 (SEQ ID NO:76), Figure 78 (SEQ ID NO:78), Figure 80 (SEQ ID NO:80), Figure 82 (SEQ ID NO:82), Figure 84 (SEQ ID NO:84), Figure 86 (SEQ ID NO:86), Figure 88 (SEQ ID NO:88), Figure 90 (SEQ ID NO:90), Figure 92 (SEQ ID NO:92), Figure 94 (SEQ ID NO:94), Figure 96 (SEQ ID NO:96), Figure 98 (SEQ ID NO:98), Figure 100 (SEQ ID NO:100), Figure 102 (SEQ ID NO:102), Figure 104 (SEQ ID NO:104), Figure 106 (SEQ ID NO:106), Figure 108 (SEQ ID NO:108), Figure 110 (SEQ ID NO:110), Figure 112 (SEQ ID NO:112), Figure 114 (SEQ ID NO:114), Figure 116 (SEQ ID NO:116), Figure 118 (SEQ ID NO:118), Figure 120 (SEQ ID NO:120), Figure 122 (SEQ ID NO:122), Figure 124 (SEQ ID NO:124), Figure 126 (SEQ ID NO:126), Figure 128 (SEQ ID NO:128), Figure 130 (SEQ ID NO:130), Figure 132 (SEQ ID NO:132), Figure 134 (SEQ ID NO:134), Figure 136 (SEQ ID NO:136), Figure 138 (SEQ ID NO:138), Figure 140 (SEQ ID NO:140), Figure 142 (SEQ ID NO:142), Figure 144 (SEQ ID NO:144), Figure 146 (SEQ ID NO:146), Figure 148 (SEQ ID NO:148), Figure 150 (SEQ ID NO:150), Figure 152 (SEQ ID NO:152), Figure 154 (SEQ ID NO:154), Figure 156 (SEQ ID NO:156), Figure 158 (SEQ ID NO:158), Figure 160 (SEQ ID NO:160), Figure 162 (SEQ ID NO:162), Figure 164 (SEQ ID NO:164), Figure 166 (SEQ ID NO:166), Figure 168 (SEQ ID NO:168), Figure 170 (SEQ ID NO:170), Figure 172 (SEQ ID NO:172), Figure 174 (SEQ ID NO:174), Figure 176 (SEQ ID NO:176), Figure 178 (SEQ ID NO:178), Figure 180 (SEQ ID NO:180), Figure 182 (SEQ ID NO:182), Figure 184 (SEQ ID NO:184), Figure 186 (SEQ ID NO:186), Figure 188 (SEQ ID NO:188), Figure 190 (SEQ ID NO:190), Figure 192 (SEQ ID NO:192), Figure 194 (SEQ ID NO:194), Figure 196 (SEQ ID NO:196), Figure 198 (SEQ ID NO:198), Figure 200 (SEQ ID NO:200), Figure 202 (SEQ ID NO:202), Figure 204 (SEQ ID NO:204), Figure 206 (SEQ ID NO:206), Figure 208 (SEQ ID NO:208), Figure 210 (SEQ ID NO:210), Figure 212 (SEQ ID NO:212), Figure 214 (SEQ ID NO:214), Figure 216 (SEQ ID NO:216), Figure 218 (SEQ ID NO:218), Figure 220 (SEQ ID NO:220), Figure 222 (SEQ ID NO:222), Figure 224 (SEQ ID NO:224), Figure 226 (SEQ ID NO:226), Figure 228 (SEQ ID NO:228), Figure 230 (SEQ ID NO:230), Figure 232 (SEQ ID NO:232), Figure 234 (SEQ ID NO:234), Figure 236 (SEQ ID NO:236), Figure 238 (SEQ ID NO:238), Figure 240 (SEQ ID NO:240), Figure 242 (SEQ ID NO:242), and Figure 244 (SEQ ID NO:244).
12. An isolated polypeptide having at least 80% amino acid sequence identity to an amino acid sequence encoded by the full-length coding sequence of the DNA deposited under any ATCC accession number shown in Table 7.
13. A chimeric molecule comprising a polypeptide according to Clause 11 fused to a heterologous amino acid sequence.
14. The chimeric molecule of Clause 13, wherein said heterologous amino acid sequence is an epitope tag sequence.
15. The chimeric molecule of Clause 13, wherein said heterologous amino acid sequence is a Fc region of an immunoglobulin.
16. An antibody which specifically binds to a polypeptide according to Clause 11.
17. The antibody of Clause 16, wherein said antibody is a monoclonal antibody, a humanized antibody or a single-chain antibody.
18. Isolated nucleic acid having at least 80% nucleic acid sequence identity to:

(a) a nucleotide sequence encoding the polypeptide shown in Figure 2 (SEQ ID NO:2), Figure 4 (SEQ ID NO:4), Figure 6 (SEQ ID NO:6), Figure 8 (SEQ ID NO:8), Figure 10 (SEQ ID NO:10), Figure 12 (SEQ ID NO:12), Figure 14 (SEQ ID NO:14), Figure 16 (SEQ ID NO:16), Figure 18 (SEQ ID NO:18), Figure 20 (SEQ ID NO:20), Figure 22 (SEQ ID NO:22), Figure 24 (SEQ ID NO:24), Figure 26 (SEQ ID NO:26), Figure 28 (SEQ ID NO:28), Figure 30 (SEQ ID NO:30), Figure 32 (SEQ ID NO:32), Figure 34 (SEQ ID NO:34), Figure 36 (SEQ ID NO:36), Figure 38 (SEQ ID NO:38), Figure 40 (SEQ ID NO:40), Figure 42 (SEQ ID NO:42), Figure 44 (SEQ ID NO:44), Figure 46 (SEQ ID NO:46), Figure 48 (SEQ ID NO:48), Figure 50 (SEQ ID NO:50), Figure 52 (SEQ ID NO:52), Figure 54 (SEQ ID NO:54), Figure 56 (SEQ ID NO:56), Figure 58 (SEQ ID NO:58), Figure 60 (SEQ ID NO:60), Figure 62 (SEQ ID NO:62), Figure 64 (SEQ ID NO:64), Figure 66 (SEQ ID NO:66), Figure 68 (SEQ ID NO:68), Figure 70 (SEQ ID NO:70), Figure 72 (SEQ ID NO:72), Figure 74 (SEQ ID NO:74), Figure 76 (SEQ ID NO:76), Figure 78 (SEQ ID NO:78), Figure 80 (SEQ ID NO:80), Figure 82 (SEQ ID NO:82), Figure 84 (SEQ ID NO:84), Figure 86 (SEQ ID NO:86), Figure 88 (SEQ ID NO:88), Figure 90 (SEQ ID NO:90), Figure 92 (SEQ ID NO:92), Figure 94 (SEQ ID NO:94), Figure 96 (SEQ ID NO:96), Figure 98 (SEQ ID NO:98), Figure 100 (SEQ ID NO:100), Figure 102 (SEQ ID NO:102), Figure 104 (SEQ ID NO:104), Figure 106 (SEQ ID NO:106), Figure 108 (SEQ ID NO:108), Figure 110 (SEQ ID NO:110), Figure 112 (SEQ ID NO:112), Figure 114 (SEQ ID NO:114), Figure 116 (SEQ ID NO:116), Figure 118 (SEQ ID NO:118), Figure 120 (SEQ ID NO:120), Figure 122 (SEQ ID NO:122), Figure 124 (SEQ ID NO:124), Figure 126 (SEQ ID NO:126), Figure 128 (SEQ ID NO:128), Figure 130 (SEQ ID NO:130), Figure 132 (SEQ ID NO:132), Figure 134 (SEQ ID NO:134), Figure 136 (SEQ ID NO:136), Figure 138 (SEQ ID NO:138), Figure 140 (SEQ ID NO:140), Figure 142 (SEQ ID NO:142), Figure 144 (SEQ ID NO:144), Figure 146 (SEQ ID NO:146), Figure 148 (SEQ ID NO:148), Figure 150 (SEQ ID NO:150), Figure 152 (SEQ ID NO:152), Figure 154 (SEQ ID NO:154), Figure 156 (SEQ ID NO:156), Figure 158 (SEQ ID NO:158), Figure 160 (SEQ ID NO:160), Figure 162 (SEQ ID NO:162), Figure 164 (SEQ ID NO:164), Figure 166 (SEQ ID NO:166), Figure 168 (SEQ ID NO:168), Figure 170 (SEQ ID NO:170), Figure 172 (SEQ ID NO:172), Figure 174 (SEQ ID NO:174), Figure 176 (SEQ ID NO:176), Figure 178 (SEQ ID NO:178), Figure 180 (SEQ ID NO:180), Figure 182 (SEQ ID NO:182), Figure 184 (SEQ ID NO:184), Figure 186 (SEQ ID NO:186), Figure 188 (SEQ ID NO:188), Figure 190 (SEQ ID NO:190), Figure 192 (SEQ ID NO:192), Figure 194 (SEQ ID NO:194), Figure 196 (SEQ ID NO:196), Figure 198 (SEQ ID NO:198), Figure 200 (SEQ ID NO:200), Figure 202 (SEQ ID NO:202), Figure 204 (SEQ ID NO:204), Figure 206 (SEQ ID NO:206), Figure 208 (SEQ ID NO:208), Figure 210 (SEQ ID NO:210), Figure 212 (SEQ ID NO:212), Figure 214 (SEQ ID NO:214), Figure 216 (SEQ ID NO:216), Figure 218 (SEQ ID NO:218), Figure 220 (SEQ ID NO:220), Figure 222 (SEQ ID NO:222), Figure 224 (SEQ ID NO:224), Figure 226 (SEQ ID NO:226), Figure 228 (SEQ ID NO:228), Figure 230 (SEQ ID NO:230), Figure 232 (SEQ ID NO:232), Figure 234 (SEQ ID NO:234), Figure 236 (SEQ ID NO:236), Figure 238 (SEQ ID NO:238), Figure 240 (SEQ ID NO:240), Figure 242 (SEQ ID NO:242), or Figure 244 (SEQ ID NO:244), lacking its associated signal peptide;
(b) a nucleotide sequence encoding an extracellular domain of the polypeptide shown in Figure 2 (SEQ ID NO:2), Figure 4 (SEQ ID NO:4), Figure 6 (SEQ ID NO:6), Figure 8 (SEQ ID NO:8), Figure 10 (SEQ ID NO:10), Figure 12 (SEQ ID NO:12), Figure 14 (SEQ ID NO:14), Figure 16 (SEQ ID NO:16), Figure 18 (SEQ ID NO:18), Figure 20 (SEQ ID NO:20), Figure 22 (SEQ ID NO:22), Figure 24 (SEQ ID NO:24), Figure 26 (SEQ ID NO:26), Figure 28 (SEQ ID NO:28), Figure 30 (SEQ ID NO:30), Figure 32 (SEQ ID NO:32), Figure 34 (SEQ ID NO:34), Figure 36 (SEQ ID NO:36), Figure 38 (SEQ ID NO:38), Figure 40 (SEQ ID NO:40), Figure 42 (SEQ ID NO:42), Figure 44 (SEQ ID NO:44), Figure 46 (SEQ ID NO:46), Figure 48 (SEQ ID NO:48), Figure 50 (SEQ ID NO:50), Figure 52 (SEQ ID NO:52), Figure 54 (SEQ ID NO:54), Figure 56 (SEQ ID NO:56), Figure 58 (SEQ ID NO:58), Figure 60 (SEQ ID NO:60), Figure 62 (SEQ ID NO:62), Figure 64 (SEQ ID NO:64), Figure 66 (SEQ ID NO:66), Figure 68 (SEQ ID NO:68), Figure 70 (SEQ ID NO:70), Figure 72 (SEQ ID NO:72), Figure 74 (SEQ ID NO:74), Figure 76 (SEQ ID NO:76), Figure 78 (SEQ ID NO:78), Figure 80 (SEQ ID NO:80), Figure 82 (SEQ ID NO:82), Figure 84 (SEQ ID NO:84), Figure 86 (SEQ ID NO:86), Figure 88 (SEQ ID NO:88), Figure 90 (SEQ ID NO:90), Figure 92 (SEQ ID NO:92), Figure 94 (SEQ ID NO:94), Figure 96 (SEQ ID NO:96), Figure 98 (SEQ ID NO:98), Figure 100 (SEQ ID NO:100), Figure 102 (SEQ ID NO:102), Figure 104 (SEQ ID NO:104), Figure 106 (SEQ ID NO:106), Figure 108 (SEQ ID NO:108), Figure 110 (SEQ ID NO:110), Figure 112 (SEQ ID NO:112), Figure 114 (SEQ ID NO:114), Figure 116 (SEQ ID NO:116), Figure 118 (SEQ ID NO:118), Figure 120 (SEQ ID NO:120), Figure 122 (SEQ ID NO:122), Figure 124 (SEQ ID NO:124), Figure 126 (SEQ ID NO:126), Figure 128 (SEQ ID NO:128), Figure 130 (SEQ ID NO:130), Figure 132 (SEQ ID NO:132), Figure 134 (SEQ ID NO:134), Figure 136 (SEQ ID NO:136), Figure 138 (SEQ ID NO:138), Figure 140 (SEQ ID NO:140), Figure 142 (SEQ ID NO:142), Figure 144 (SEQ ID NO:144), Figure 146 (SEQ ID NO:146), Figure 148 (SEQ ID NO:148), Figure 150 (SEQ ID NO:150), Figure 152 (SEQ ID NO:152), Figure 154 (SEQ ID NO:154), Figure 156 (SEQ ID NO:156), Figure 158 (SEQ ID NO:158), Figure 160 (SEQ ID NO:160), Figure 162 (SEQ ID NO:162), Figure 164 (SEQ ID NO:164), Figure 166 (SEQ ID NO:166), Figure 168 (SEQ ID NO:168), Figure 170 (SEQ ID NO:170), Figure 172 (SEQ ID NO:172), Figure 174 (SEQ ID NO:174), Figure 176 (SEQ ID NO:176), Figure 178 (SEQ ID NO:178), Figure 180 (SEQ ID NO:180), Figure 182 (SEQ ID NO:182), Figure 184 (SEQ ID NO:184), Figure 186 (SEQ ID NO:186), Figure 188 (SEQ ID NO:188), Figure 190 (SEQ ID NO:190), Figure 192 (SEQ ID NO:192), Figure 194 (SEQ ID NO:194), Figure 196 (SEQ ID NO:196), Figure 198 (SEQ ID NO:198), Figure 200 (SEQ ID NO:200), Figure 202 (SEQ ID NO:202), Figure 204 (SEQ ID NO:204), Figure 206 (SEQ ID NO:206), Figure 208 (SEQ ID NO:208), Figure 210 (SEQ ID NO:210), Figure 212 (SEQ ID NO:212), Figure 214 (SEO ID NO:214), Figure 216 (SEQ ID NO:216), Figure 218 (SEQ ID NO:218), Figure 220 (SEQ ID NO:220), Figure 222 (SEQ ID NO:222), Figure 224 (SEQ ID NO:224), Figure 226 (SEQ ID NO:226), Figure 228 (SEQ ID NO:228), Figure 230 (SEQ ID NO:230), Figure 232 (SEQ ID NO:232), Figure 234 (SEQ ID NO:234), Figure 236 (SEQ ID NO:236), Figure 238 (SEQ ID NO:238), Figure 240 (SEQ ID NO:240), Figure 242 (SEQ ID NO:242), or Figure 244 (SEQ ID NO:244), with its associated signal peptide; or
(c) a nucleotide sequence encoding an extracellular domain of the polypeptide shown in Figure 2 (SEQ ID NO:2), Figure 4 (SEQ ID NO:4), Figure 6 (SEQ ID NO:6), Figure 8 (SEQ ID NO:8), Figure 10 (SEQ ID NO:10), Figure 12 (SEQ ID NO:12), Figure 14 (SEQ ID NO:14), Figure 16 (SEQ ID NO:16), Figure 18 (SEQ ID NO:18), Figure 20 (SEQ ID NO:20), Figure 22 (SEQ ID NO:22), Figure 24 (SEQ ID NO:24), Figure 26 (SEQ ID NO:26), Figure 28 (SEQ ID NO:28), Figure 30 (SEQ ID NO:30), Figure 32 (SEQ ID NO:32), Figure 34 (SEQ ID NO:34), Figure 36 (SEQ ID NO:36), Figure 38 (SEQ ID NO:38), Figure 40 (SEQ ID NO:40), Figure 42 (SEQ ID NO:42), Figure 44 (SEQ ID NO:44), Figure 46 (SEQ ID NO:46), Figure 48 (SEQ ID NO:48), Figure 50 (SEQ ID NO:50), Figure 52 (SEQ ID NO:52), Figure 54 (SEQ ID NO:54), Figure 56 (SEQ ID NO:56), Figure 58 (SEQ ID NO:58), Figure 60 (SEQ ID NO:60), Figure 62 (SEQ ID NO:62), Figure 64 (SEQ ID NO:64), Figure 66 (SEQ ID NO:66), Figure 68 (SEQ ID NO:68), Figure 70 (SEQ ID NO:70), Figure 72 (SEQ ID NO:72), Figure 74 (SEQ ID NO:74), Figure 76 (SEQ ID NO:76), Figure 78 (SEQ ID NO:78), Figure 80 (SEQ ID NO:80), Figure 82 (SEQ ID NO:82), Figure 84 (SEQ ID NO:84), Figure 86 (SEQ ID NO:86), Figure 88 (SEQ ID NO:88), Figure 90 (SEQ ID NO:90), Figure 92 (SEQ ID NO:92), Figure 94 (SEQ ID NO:94), Figure 96 (SEQ ID NO:96), Figure 98 (SEQ ID NO:98), Figure 100 (SEQ ID NO:100), Figure 102 (SEQ ID NO:102), Figure 104 (SEQ ID NO:104), Figure 106 (SEQ ID NO:106), Figure 108 (SEQ ID NO:108), Figure 110 (SEQ ID NO:110), Figure 112 (SEQ ID NO:112), Figure 114 (SEQ ID NO:114), Figure 116 (SEQ ID NO:116), Figure 118 (SEQ ID NO:118), Figure 120 (SEQ ID NO:120), Figure 122 (SEQ ID NO:122), Figure 124 (SEQ ID NO:124), Figure 126 (SEQ ID NO:126), Figure 128 (SEQ ID NO:128), Figure 130 (SEQ ID NO:130), Figure 132 (SEQ ID NO:132), Figure 134 (SEQ ID NO:134), Figure 136 (SEQ ID NO:136), Figure 138 (SEQ ID NO:138), Figure 140 (SEQ ID NO:140), Figure 142 (SEQ ID NO:142), Figure 144 (SEQ ID NO:144), Figure 146 (SEQ ID NO:146), Figure 148 (SEQ ID NO:148), Figure 150 (SEQ ID NO:150), Figure 152 (SEQ ID NO:152), Figure 154 (SEQ ID NO:154), Figure 156 (SEQ ID NO:156), Figure 158 (SEQ ID NO:158), Figure 160 (SEQ ID NO:160), Figure 162 (SEQ ID NO:162), Figure 164 (SEQ ID NO:164), Figure 166 (SEQ ID NO:166), Figure 168 (SEQ ID NO:168), Figure 170 (SEQ ID NO:170), Figure 172 (SEQ ID NO:172), Figure 174 (SEQ ID NO:174), Figure 176 (SEQ ID NO:176), Figure 178 (SEQ ID NO:178), Figure 180 (SEQ ID NO:180), Figure 182 (SEQ ID NO:182), Figure 184 (SEQ ID NO:184), Figure 186 (SEQ ID NO:186), Figure 188 (SEQ ID NO:188), Figure 190 (SEQ ID NO:190), Figure 192 (SEQ ID NO:192), Figure 194 (SEQ ID NO:194), Figure 196 (SEQ ID NO:196), Figure 198 (SEQ ID NO:198), Figure 200 (SEQ ID NO:200), Figure 202 (SEQ ID NO:202), Figure 204 (SEQ ID NO:204), Figure 206 (SEQ ID NO:206), Figure 208 (SEQ ID NO:208), Figure 210 (SEQ ID NO:210), Figure 212 (SEQ ID NO:212), Figure 214 (SEQ ID NO:214), Figure 216 (SEQ ID NO:216), Figure 218 (SEQ ID NO:218), Figure 220 (SEQ ID NO:220), Figure 222 (SEQ ID NO:222), Figure 224 (SEQ ID NO:224), Figure 226 (SEQ ID NO:226), Figure 228 (SEQ ID NO:228), Figure 230 (SEQ ID NO:230), Figure 232 (SEQ ID NO:232), Figure 234 (SEQ ID NO:234), Figure 236 (SEQ ID NO:236), Figure 238 (SEQ ID NO:238), Figure 240 (SEQ ID NO:240), Figure 242 (SEQ ID NO:242), or Figure 244 (SEQ ID NO:244), lacking its associated signal peptide.
19. An isolated polypeptide having at least 80% amino acid sequence identity to:
   (a) an amino acid sequence of the polypeptide shown in Figure 2 (SEQ ID NO:2), Figure 4 (SEQ ID NO:4), Figure 6 (SEQ ID NO:6), Figure 8 (SEQ ID NO:8), Figure 10 (SEQ ID NO:10), Figure 12 (SEQ ID NO:12), Figure 14 (SEQ ID NO:14), Figure 16 (SEQ ID NO:16), Figure 18 (SEQ ID NO:18), Figure 20 (SEQ ID NO:20), Figure 22 (SEQ ID NO:22), Figure 24 (SEQ ID NO:24), Figure 26 (SEQ ID NO:26), Figure 28 (SEQ ID NO:28), Figure 30 (SEQ ID NO:30), Figure 32 (SEQ ID NO:32), Figure 34 (SEQ ID NO:34), Figure 36 (SEQ ID NO:36), Figure 38 (SEQ ID NO:38), Figure 40 (SEQ ID NO:40), Figure 42 (SEQ ID NO:42), Figure 44 (SEQ ID NO:44), Figure 46 (SEQ ID NO:46), Figure 48 (SEQ ID NO:48), Figure 50 (SEQ ID NO:50), Figure 52 (SEQ ID NO:52), Figure 54 (SEQ ID NO:54), Figure 56 (SEQ ID NO:56), Figure 58 (SEQ ID NO:58), Figure 60 (SEQ ID NO:60), Figure 62 (SEQ ID NO:62), Figure 64 (SEQ ID NO:64), Figure 66 (SEQ ID NO:66), Figure 68 (SEQ ID NO:68), Figure 70 (SEQ ID NO:70), Figure 72 (SEQ ID NO:72), Figure 74 (SEQ ID NO:74), Figure 76 (SEQ ID NO:76), Figure 78 (SEQ ID NO:78), Figure 80 (SEQ ID NO:80), Figure 82 (SEQ ID NO:82), Figure 84 (SEQ ID NO:84), Figure 86 (SEQ ID NO:86), Figure 88 (SEQ ID NO:88), Figure 90 (SEQ ID NO:90), Figure 92 (SEQ ID NO:92), Figure 94 (SEQ ID NO:94), Figure 96 (SEQ ID NO:96), Figure 98 (SEQ ID NO:98), Figure 100 (SEQ ID NO:100), Figure 102 (SEQ ID NO:102), Figure 104 (SEQ ID NO:104), Figure 106 (SEQ ID NO:106), Figure 108 (SEQ ID NO:108), Figure 110 (SEQ ID NO:110), Figure 112 (SEQ ID NO:112), Figure 114 (SEQ ID NO:114), Figure 116 (SEQ ID NO:116), Figure 118 (SEQ ID NO:118), Figure 120 (SEQ ID NO:120), Figure 122 (SEQ ID NO:122), Figure 124 (SEQ ID NO:124), Figure 126 (SEQ ID NO:126), Figure 128 (SEQ ID NO:128), Figure 130 (SEQ ID NO:130), Figure 132 (SEQ ID NO:132), Figure 134 (SEQ ID NO:134), Figure 136 (SEQ ID NO:136), Figure 138 (SEQ ID NO:138), Figure 140 (SEQ ID NO:140), Figure 142 (SEQ ID NO:142), Figure 144 (SEQ ID NO:144), Figure 146 (SEQ ID NO:146), Figure 148 (SEQ ID NO:148), Figure 150 (SEQ ID NO:150), Figure 152 (SEQ ID NO:152), Figure 154 (SEQ ID NO:154), Figure 156 (SEQ ID NO:156), Figure 158 (SEQ ID NO:158), Figure 160 (SEQ ID NO:160), Figure 162 (SEQ ID NO:162), Figure 164 (SEQ ID NO:164), Figure 166 (SEQ ID NO:166), Figure 168 (SEQ ID NO:168), Figure 170 (SEQ ID NO:170), Figure 172 (SEQ ID NO:172), Figure 174 (SEQ ID NO:174), Figure 176 (SEQ ID NO:176), Figure 178 (SEQ ID NO:178), Figure 180 (SEQ ID NO:180), Figure 182 (SEQ ID NO:182), Figure 184 (SEQ ID NO:184), Figure 186 (SEQ ID NO:186), Figure 188 (SEQ ID NO:188), Figure 190 (SEQ ID NO:190), Figure 192 (SEQ ID NO:192), Figure 194 (SEQ ID NO:194), Figure 196 (SEQ ID NO:196), Figure 198 (SEQ ID NO:198), Figure 200 (SEQ ID NO:200), Figure 202 (SEQ ID NO:202), Figure 204 (SEQ ID NO:204), Figure 206 (SEQ ID NO:206), Figure 208 (SEQ ID NO:208), Figure 210 (SEQ ID NO:210), Figure 212 (SEQ ID NO:212), Figure 214 (SEQ ID NO:214), Figure 216 (SEQ ID NO:216), Figure 218 (SEQ ID NO:218), Figure 220 (SEQ ID NO:220), Figure 222 (SEQ ID NO:222), Figure 224 (SEQ ID NO:224), Figure 226 (SEQ ID NO:226), Figure 228 (SEQ ID NO:228), Figure 230 (SEQ ID NO:230), Figure 232 (SEQ ID NO:232), Figure 234 (SEQ ID NO:234), Figure 236 (SEQ ID NO:236), Figure 238 (SEQ ID NO:238), Figure 240 (SEQ ID NO:240), Figure 242 (SEQ ID NO:242), or Figure 244 (SEQ ID NO:244), lacking its associated signal peptide;
   (b) an amino acid sequence of an extracellular domain of the polypeptide shown in Figure 2 (SEQ ID NO:2), Figure 4 (SEQ ID NO:4), Figure 6 (SEQ ID NO:6), Figure 8 (SEQ ID NO:8), Figure 10 (SEQ ID NO:10), Figure 12 (SEQ ID NO:12), Figure 14 (SEQ ID NO:14), Figure 16 (SEQ ID NO:16), Figure 18 (SEQ ID NO:18), Figure 20 (SEQ ID NO:20), Figure 22 (SEQ ID NO:22), Figure 24 (SEQ ID NO:24), Figure 26 (SEQ ID NO:26), Figure 28 (SEQ ID NO:28), Figure 30 (SEQ ID NO:30), Figure 32 (SEQ ID NO:32), Figure 34 (SEQ ID NO:34), Figure 36 (SEQ ID NO:36), Figure 38 (SEQ ID NO:38), Figure 40 (SEQ ID NO:40), Figure 42 (SEQ ID NO:42), Figure 44 (SEQ ID NO:44), Figure 46 (SEQ ID NO:46), Figure 48 (SEQ ID NO:48), Figure 50 (SEQ ID NO:50), Figure 52 (SEQ ID NO:52), Figure 54 (SEQ ID NO:54), Figure 56 (SEQ ID NO:56), Figure 58 (SEQ ID NO:58), Figure 60 (SEQ ID NO:60), Figure 62 (SEQ ID NO:62), Figure 64 (SEQ ID NO:64), Figure 66 (SEQ ID NO:66), Figure 68 (SEQ ID NO:68), Figure 70 (SEQ ID NO:70), Figure 72 (SEQ ID NO:72), Figure 74 (SEQ ID NO:74), Figure 76 (SEQ ID NO:76), Figure 78 (SEQ ID NO:78), Figure 80 (SEQ ID NO:80), Figure 82 (SEQ ID NO:82), Figure 84 (SEQ ID NO:84), Figure 86 (SEQ ID NO:86), Figure 88 (SEQ ID NO:88), Figure 90 (SEQ ID NO:90), Figure 92 (SEQ ID NO:92), Figure 94 (SEQ ID NO:94), Figure 96 (SEQ ID NO:96), Figure 98 (SEQ ID NO:98), Figure 100 (SEQ ID NO:100), Figure 102 (SEQ ID NO:102), Figure 104 (SEQ ID NO:104), Figure 106 (SEQ ID NO:106), Figure 108 (SEQ ID NO:108), Figure 110 (SEQ ID NO:110), Figure 112 (SEQ ID NO:112), Figure 114 (SEQ ID NO:114), Figure 116 (SEQ ID NO:116), Figure 118 (SEQ ID NO:118), Figure 120 (SEQ ID NO:120), Figure 122 (SEQ ID NO:122), Figure 124 (SEQ ID NO:124), Figure 126 (SEQ ID NO:126), Figure 128 (SEQ ID NO:128), Figure 130 (SEQ ID NO:130), Figure 132 (SEQ ID NO:132), Figure 134 (SEQ ID NO:134), Figure 136 (SEQ ID NO:136), Figure 138 (SEQ ID NO:138), Figure 140 (SEQ ID NO:140), Figure 142 (SEQ ID NO:142), Figure 144 (SEQ ID NO:144), Figure 146 (SEQ ID NO:146), Figure 148 (SEQ ID NO:148), Figure 150 (SEQ ID NO:150), Figure 152 (SEQ ID NO:152), Figure 154 (SEQ ID NO:154), Figure 156 (SEQ ID NO:156), Figure 158 (SEQ ID NO:158), Figure 160 (SEQ ID NO:160), Figure 162 (SEQ ID NO:162), Figure 164 (SEQ ID NO:164), Figure 166 (SEQ ID NO:166), Figure 168 (SEQ ID NO:168), Figure 170 (SEQ ID NO:170), Figure 172 (SEQ ID NO:172), Figure 174 (SEQ ID NO:174), Figure 176 (SEQ ID NO:176), Figure 178 (SEQ ID NO:178), Figure 180 (SEQ ID NO:180), Figure 182 (SEQ ID NO:182), Figure 184 (SEQ ID NO:184), Figure 186 (SEQ ID NO:186), Figure 188 (SEQ ID NO:188), Figure 190 (SEQ ID NO:190), Figure 192 (SEQ ID NO:192), Figure 194 (SEQ ID NO:194), Figure 196 (SEQ ID NO:196), Figure 198 (SEQ ID NO:198), Figure 200 (SEQ ID NO:200), Figure 202 (SEQ ID NO:202), Figure 204 (SEQ ID NO:204), Figure 206 (SEQ ID NO:206), Figure 208 (SEQ ID NO:208), Figure 210 (SEQ ID NO:210), Figure 212 (SEQ ID NO:212), Figure 214 (SEQ ID NO:214), Figure 216 (SEQ ID NO:216), Figure 218 (SEQ ID NO:218), Figure 220 (SEQ ID NO:220), Figure 222 (SEQ ID NO:222), Figure 224 (SEQ ID NO:224), Figure 226 (SEQ ID NO:226), Figure 228 (SEQ ID NO:228), Figure 230 (SEQ ID NO:230), Figure 232 (SEQ ID NO:232), Figure 234 (SEQ ID NO:234), Figure 236 (SEQ ID NO:236), Figure 238 (SEQ ID NO:238), Figure 240 (SEQ ID NO:240), Figure 242 (SEQ ID NO:242), or Figure 244 (SEQ ID NO:244), with its associated signal peptide; or
   (c) an amino acid sequence of an extracellular domain of the polypeptide shown in Figure 2 (SEQ ID NO:2), Figure 4 (SEQ ID NO:4), Figure 6 (SEQ ID NO:6), Figure 8 (SEQ ID NO:8), Figure 10 (SEQ ID NO:10), Figure 12 (SEQ ID NO: 12), Figure 14 (SEQ ID NO:14), Figure 16 (SEQ ID NO:16), Figure 18 (SEQ ID NO:18), Figure 20 (SEQ ID NO:20), Figure 22 (SEQ ID NO:22), Figure 24 (SEQ ID NO:24), Figure 26 (SEQ ID NO:26), Figure 28 (SEQ ID NO:28), Figure 30 (SEQ ID NO:30), Figure 32 (SEQ ID NO:32), Figure 34 (SEQ ID NO:34), Figure 36 (SEQ ID NO:36), Figure 38 (SEQ ID NO:38), Figure 40 (SEQ ID NO:40), Figure 42 (SEQ ID NO:42), Figure 44 (SEQ ID NO:44), Figure 46 (SEQ ID NO:46), Figure 48 (SEQ ID NO:48) Figure 50 (SEQ ID NO:50), Figure 52 (SEQ ID NO:52), Figure 54 (SEQ ID NO:54), Figure 56 (SEQ ID NO:56), Figure 58 (SEQ ID NO:58), Figure 60 (SEQ ID NO:60), Figure 62 (SEQ ID NO:62), Figure 64 (SEQ ID NO:64). Figure 66 (SEQ ID NO:66), Figure 68 (SEQ ID NO:68), Figure 70 (SEQ ID NO:70), Figure 72 (SEQ ID NO:72), Figure 74 (SEQ ID NO:74), Figure 76 (SEQ ID NO:76), Figure 78 (SEQ ID NO:78), Figure 80 (SEQ ID NO:80), Figure 82 (SEQ ID NO:82), Figure 84 (SEQ ID NO:84), Figure 86 (SEQ ID NO:86), Figure 88 (SEQ ID NO:88), Figure 90 (SEQ ID NO:90), Figure 92 (SEQ ID NO:92), Figure 94 (SEQ ID NO:94), Figure 96 (SEQ ID NO:96), Figure 98 (SEQ ID NO:98), Figure 100 (SEQ ID NO:100), Figure 102 (SEQ ID NO:102), Figure 104 (SEQ ID NO:104), Figure 106 (SEQ ID NO:106), Figure 108 (SEQ ID NO:108), Figure 110 (SEQ ID NO:110), Figure 112 (SEQ ID NO:112), Figure 114 (SEQ ID NO:114), Figure 116 (SEQ ID NO:116), Figure 118 (SEQ ID NO:118), Figure 120 (SEQ ID NO:120), Figure 122 (SEQ ID NO:122), Figure 124 (SEQ ID NO:124), Figure 126 (SEQ ID NO:126), Figure 128 (SEQ ID NO:128), Figure 130 (SEQ ID NO:130), Figure 132 (SEQ ID NO:132), Figure 134 (SEQ ID NO:134), Figure 136 (SEQ ID NO:136), Figure 138 (SEQ ID NO:138), Figure 140 (SEQ ID NO:140), Figure 142 (SEQ ID NO:142), Figure 144 (SEQ ID NO:144), Figure 146 (SEQ ID NO:146), Figure 148 (SEQ ID NO:148), Figure 150 (SEQ ID NO:150), Figure 152 (SEQ ID NO:152), Figure 154 (SEQ ID NO:154), Figure 156 (SEQ ID NO:156), Figure 158 (SEQ ID NO:158), Figure 160 (SEQ ID NO:160), Figure 162 (SEQ ID NO:162), Figure 164 (SEQ ID NO:164), Figure 166 (SEQ ID NO:166), Figure 168 (SEQ ID NO:168), Figure 170 (SEQ ID NO:170), Figure 172 (SEQ ID NO:172), Figure 174 (SEQ ID NO:174), Figure 176 (SEQ ID NO:176), Figure 178 (SEQ ID NO:178), Figure 180 (SEQ ID NO:180), Figure 182 (SEQ ID NO:182), Figure 184 (SEQ ID NO:184), Figure 186 (SEQ ID NO:186), Figure 188 (SEQ ID NO:188), Figure 190 (SEQ ID NO:190), Figure 192 (SEQ ID NO:192), Figure 194 (SEQ ID NO:194), Figure 196 (SEQ ID NO:196), Figure 198 (SEQ ID NO:198), Figure 200 (SEQ ID NO:200), Figure 202 (SEQ ID NO:202), Figure 204 (SEQ ID NO:204), Figure 206 (SEQ ID NO:206), Figure 208 (SEQ ID NO:208), Figure 210 (SEQ ID NO:210), Figure 212 (SEQ ID NO:212), Figure 214 (SEQ ID NO:214), Figure 216 (SEQ ID NO:216), Figure 218 (SEO ID NO:218), Figure 220 (SEQ ID NO:220), Figure 222 (SEQ ID NO:222), Figure 224 (SEQ ID NO:224), Figure 226 (SEQ ID NO:226), Figure 228 (SEQ ID NO:228), Figure 230 (SEQ ID NO:230), Figure 232 (SEQ ID NO:232), Figure 234 (SEQ ID NO:234), Figure 236 (SEQ ID NO:236), Figure 238 (SEQ ID NO:238), Figure 240 (SEQ ID NO:240), Figure 242 (SEQ ID NO:242), or Figure 244 (SEQ ID NO:244), lacking its associated signal peptide.
20. A method for stimulating the proliferation of or gene expression in pericyte cells, said method comprising contacting said cells with a PRO982, PRO1160, PRO1187, or PRO1329 polypeptide, wherein the proliferation of or gene expression in said cells is stimulated.
21. A method for stimulating the proliferation or differentiation of chondrocyte cells, said method comprising contacting said cells with a PRO357, PRO229, PRO1272 or PRO4405 polypeptide, wherein the proliferation or differentiation of said cells is stimulated.
22. A method for stimulating the release of TNF-α from human blood, said method comprising contacting said blood with a PRO231, PRO357, PRO725, PRO1155, PRO1306 or PRO1419 polypeptide, wherein the release of TNF-α from said blood is stimulated.
23. A method for stimulating the proliferation of normal human dermal fibroblast cells, said method comprising contacting said cells with a PRO982, PRO357, PRO725, PRO1306 PRO1419, PRO229, PRO1272, PRO181, PRO214, PRO247, PRO337, PRO526, PRO363, PRO531, PRO1083, PRO840, PRO1080, PRO788, PRO1478, PRO1134, PRO826, PRO1005, PRO809, PRO1194 PRO1071, PRO1411, PRO1309, PRO1025, PRO1181, PRO1126, PRO1186, PRO1192, PRO1244, PRO1274, PRO1412, PRO1286, PRO1330, PRO1347, PRO1305, PRO1273, PRO1279, PRO1340 PRO1338, PRO1343, PRO1376, PRO1387, PRO1409, PRO1488, PRO1474, PR01917, PRO1760, PRO1567, PRO1887, PRO1928, PRO4341, PRO5723, PRO1801, PRO4333, PRO3543, PRO3444, PRO4302, PRO4322, PRO5725, PRO4408, PRO9940, PRO7154, PRO7425, PRO6079, PRO9836 or PRO10096 polypeptide, wherein the proliferation of said cells is stimulated.
24. A method for detecting the presence of tumor in an mammal, said method comprising comparing the level of expression of any PRO polypeptide shown in Table 8 in (a) a test sample of cells taken from said mammal and (b) a control sample of normal cells of the same cell type, wherein a higher level of expression of said PRO polypeptide in the test sample as compared to the control sample is indicative of the presence of tumor in said mammal.
25. The method of Clause 24, wherein said tumor is lung tumor, colon tumor, breast tumor, prostate tumor, rectal tumor, or liver tumor.
26. An oligonucleotide probe derived from any of the nucleotide sequences shown in the accompanying figures.

### BRIEF DESCRIPTION OF THE DRAWINGS

Figures 1A-1B show a nucleotide sequence (SEQ ID NO:1) of a native sequence PRO6004 cDNA, wherein SEQ ID NO:1 is a clone designated herein as "DNA92259".
Figure 2 shows the amino acid sequence (SEQ ID NO:2) derived from the coding sequence of SEQ ID NO:1 shown in Figures 1A-1B.
Figure 3 shows a nucleotide sequence (SEQ ID NO:3) of a native sequence PRO4981 cDNA, wherein SEQ ID NO:3 is a clone designated herein as "DNA94849-2960".
Figure 4 shows the amino acid sequence (SEQ ID NO:4) derived from the coding sequence of SEQ ID NO:3 shown in Figure 3.
Figure 5 shows a nucleotide sequence (SEQ ID NO:5) of a native sequence PRO7174 cDNA, wherein SEQ ID NO:5 is a clone designated herein as "DNA96883-2745".
Figure 6 shows the amino acid sequence (SEQ ID NO:6) derived from the coding sequence of SEQ ID NO:5 shown in Figure 5.
Figure 7 shows a nucleotide sequence (SEQ ID NO:7) of a native sequence PRO5778 cDNA, wherein SEQ ID NO:7 is a clone designated herein as "DNA96894-2675".
Figure 8 shows the amino acid sequence (SEQ ID NO:8) derived from the coding sequence of SEQ ID NO:7 shown in Figure 7.
Figure 9 shows a nucleotide sequence (SEQ ID NO:9) of a native sequence PRO433 cDNA, wherein SEQ ID NO:9 is a clone designated herein as "DNA 100272-2969"
Figure 10 shows the amino acid sequence (SEQ ID NO:10) derived from the coding sequence of SEQ ID NO:9 shown in Figure 9.
Figure 11 shows a nucleotide sequence (SEQ ID NO:11) of a native sequence PRO9799 cDNA, wherein SEQ ID NO:11 is a clone designated herein as "DNA108696-2966".
Figure 12 shows the amino acid sequence (SEQ ID NO:12) derived from the coding sequence of SEQ ID NO:11 shown in Figure 11.
Figure 13 shows a nucleotide sequence (SEQ ID NO:13) of a native sequence PRO9909 cDNA, wherein SEQ ID NO:13 is a clone designated herein as "DNA117935-2801".
Figure 14 shows the amino acid sequence (SEQ ID NO:14) derived from the coding sequence of SEQ ID NO:13 shown in Figure 13.
Figure 15 shows a nucleotide sequence (SEQ ID NO:15) of a native sequence PRO9917 cDNA, wherein SEQ ID NO:15 is a clone designated herein as "DNA119474-2803".
Figure 16 shows the amino acid sequence (SEQ ID NO: 16) derived from the coding sequence of SEQ ID NO:15 shown in Figure 15.
Figure 17 shows a nucleotide sequence (SEQ ID NO:17) of a native sequence PRO9771 cDNA, wherein SEQ ID NO:17 is a clone designated herein as "DNA119498-2965".
Figure 18 shows the amino acid sequence (SEQ ID NO:18) derived from the coding sequence of SEQ ID NO:17 shown in Figure 17.
Figure 19 shows a nucleotide sequence (SEQ ID NO:19) of a native sequence PRO9877 cDNA, wherein SEQ ID NO:19 is a clone designated herein as "DNA119502-2789".
Figure 20 shows the amino acid sequence (SEQ ID NO:20) derived from the coding sequence of SEQ ID NO: 19 shown in Figure 19.
Figure 21 shows a nucleotide sequence (SEQ ID NO:21) of a native sequence PRO9903 cDNA, wherein SEQ ID NO:21 is a clone designated herein as "DNA119516-2797".
Figure 22 shows the amino acid sequence (SEQ ID NO:22) derived from the coding sequence of SEQ ID NO:21 shown in Figure 21.
Figure 23 shows a nucleotide sequence (SEQ ID NO:23) of a native sequence PRO9830 cDNA, wherein SEQ ID NO:23 is a clone designated herein as "DNA119530-2968".
Figure 24 shows the amino acid sequence (SEQ ID NO:24) derived from the coding sequence of SEQ ID NO:23 shown in Figure 23.
Figure 25 shows a nucleotide sequence (SEQ ID NO:25) of a native sequence PRO7155 cDNA, wherein SEQ ID NO:25 is a clone designated herein as "DNA121772-2741".
Figure 26 shows the amino acid sequence (SEQ ID NO:26) derived from the coding sequence of SEQ ID NO:25 shown in Figure 25.
Figure 27 shows a nucleotide sequence (SEQ ID NO:27) of a native sequence PRO9862 cDNA, wherein SEQ ID NO:27 is a clone designated herein as "DNA125148-2782".
Figure 28 shows the amino acid sequence (SEQ ID NO:28) derived from the coding sequence of SEQ ID NO:27 shown in Figure 27.
Figure 29 shows a nucleotide sequence (SEQ ID NO:29) of a native sequence PRO9882 cDNA, wherein SEQ ID NO:29 is a clone designated herein as "DNA125150-2793".
Figure 30 shows the amino acid sequence (SEQ ID NO:30) derived from the coding sequence of SEQ ID NO:29 shown in Figure 29.
Figure 31 shows a nucleotide sequence (SEQ ID NO:31) of a native sequence PRO9864 cDNA, wherein SEQ ID NO:31 is a clone designated herein as "DNA125151-2784".
Figure 32 shows the amino acid sequence (SEQ ID NO:32) derived from the coding sequence of SEQ ID NO:31 shown in Figure 31.
Figure 33 shows a nucleotide sequence (SEQ ID NO:33) of a native sequence PRO10013 cDNA, wherein. SEQ ID NO:33 is a clone designated herein as "DNA125181-2804".
Figure 34 shows the amino acid sequence (SEQ ID NO:34) derived from the coding sequence of SEQ ID NO:33 shown in Figure 33.
Figure 35 shows a nucleotide sequence (SEQ ID NO:35) of a native sequence PRO9885 cDNA, wherein. SEQ ID NO:35 is a clone designated herein as "DNA125192-2794".
Figure 36 shows the amino acid sequence (SEQ ID NO:36) derived from the coding sequence of SEQ ID NO:35 shown in Figure 35.
Figure 37 shows a nucleotide sequence (SEQ ID NO:37) of a native sequence PRO9879 cDNA, wherein SEQ ID NO:37 is a clone designated herein as "DNA125196-2792".
Figure 38 shows the amino acid sequence (SEQ ID NO:38) derived from the coding sequence of SEQ ID NO:37 shown in Figure 37.
Figure 39 shows a nucleotide sequence (SEQ lD NO:39) of a native sequence PRO10111 cDNA, wherein SEQ ID NO:39 is a clone designated herein as "DNA125200-2810".
Figure 40 shows the amino acid sequence (SEQ ID NO:40) derived from the coding sequence of SEQ ID NO:39 shown in Figure 39.
Figure 41 shows a nucleotide sequence (SEQ ID NO:41) of a native sequence PRO9925 cDNA, wherein SEQ ID NO:41 is a clone designated herein as "DNA125214-2814".
Figure 42 shows the amino acid sequence (SEQ ID NO:42) derived from the coding sequence of SEQ ID NO:41 shown in Figure 41.
Figure 43 shows a nucleotide sequence (SEQ ID NO:43) of a native sequence PRO9905 cDNA. wherein SEQ ID NO:43 is a clone designated herein as "DNA125219-2799".
Figure 44 shows the amino acid sequence (SEQ ID NO:44) derived from the coding sequence of SEQ ID NO:43 shown in Figure 43.
Figure 45 shows a nucleotide sequence (SEQ ID NO:45) of a native sequence PRO 10276 cDNA, wherein SEQ ID NO:45 is a clone designated herein as "DNA128309-2825".
Figure 46 shows the amino acid sequence (SEQ ID NO:46) derived from the coding sequence of SEQ ID NO:45 shown in Figure 45.
Figure 47 shows a nucleotide sequence (SEQ ID NO:47) of a native sequence PRO9898 cDNA, wherein SEQ ID NO:47 is a clone designated herein as "DNA129535-2796".
Figure 48 shows the amino acid sequence (SEQ ID NO:48) derived from the coding sequence of SEQ ID NO:47 shown in Figure 47.
Figure 49 shows a nucleotide sequence (SEQ ID NO:49) of a native sequence PRO9904 cDNA, wherein SEQ ID NO:49 is a clone designated herein as "DNA129549-2798".
Figure 50 shows the amino acid sequence (SEQ ID NO:50) derived from the coding sequence of SEQ ID NO:49 shown in Figure 49.
Figure 51 shows a nucleotide sequence (SEQ ID NO:51) of a native sequence PRO19632 cDNA, wherein SEQ ID NO:51 is a clone designated herein as "DNA129580-2863".
Figure 52 shows the amino acid sequence (SEQ ID NO:52) derived from the coding sequence of SEQ ID NO:51 shown in Figure 51.
Figure 53 shows a nucleotide sequence (SEQ ID NO:53) of a native sequence PRO19672 cDNA, wherein SEQ ID NO:53 is a clone designated herein as "DNA129794-2967".
Figure 54 shows the amino acid sequence (SEQ ID NO:54) derived from the coding sequence of SEQ ID NO:53 shown in Figure 53.
Figure 55 shows a nucleotide sequence (SEQ ID NO:55) of a native sequence PR09783 cDNA, wherein SEQ ID NO:55 is a clone designated herein as "DNA131590-2962".
Figure 56 shows the amino acid sequence (SEQ ID NO:56) derived from the coding sequence of SEQ ID NO:55 shown in Figure 55.
Figure 57 shows a nucleotide sequence (SEQ ID NO:57) of a native sequence PRO10112 cDNA, wherein SEQ ID NO:57 is a clone designated herein as "DNA135173-2811".
Figure 58 shows the amino acid sequence (SEQ ID NO:58) derived from the coding sequence of SEQ ID NO:57 shown in Figure 57.
Figures 59A-59B show a nucleotide sequence (SEQ ID NO:59) of a native sequence PRO 10284 cDNA, wherein SEQ ID NO:59 is a clone designated herein as "DNA138039-2828".
Figure 60 shows the amino acid sequence (SEQ ID NO:60) derived from the coding sequence of SEQ ID NO:59 shown in Figures 59A-59B.
Figure 61 shows a nucleotide sequence (SEQ ID NO:61) of a native sequence PRO10100 cDNA, wherein SEQ ID NO:61 is a clone designated herein as " DNA 139540-2807".
Figure 62 shows the amino acid sequence (SEQ ID NO:62) derived from the coding sequence of SEQ ID NO:61 shown in Figure 61.
Figure 63 shows a nucleotide sequence (SEQ ID NO:63) of a native sequence PRO 19628 cDNA, wherein SEQ ID NO:63 is a clone designated herein as "DNA139602-2859".
Figure 64 shows the amino acid sequence (SEQ ID NO:64) derived from the coding sequence of SEQ ID NO:63 shown in Figure 63.
Figure 65 shows a nucleotide sequence (SEQ ID NO:65) of native sequence PRO 19684 cDNA, wherein SEQ ID NO:65 is a clone designated herein as "DNA139632-2880".
Figure 66 shows the amino acid sequence (SEQ ID NO:66) derived from the coding sequence of SEQ ID NO:65 shown in Figure 65.
Figure 67 shows a nucleotide sequence (SEQ ID NO:67) of a native sequence PRO10274 cDNA, wherein SEQ ID NO:67 is a clone designated herein as "DNA139686-2823".
Figure 68 shows the amino acid sequence (SEQ ID NO:68) derived from the coding sequence of SEQ ID NO:67 shown in Figure 67.
Figure 69 shows a nucleotide sequence (SEQ ID NO:69) of a native sequence PRO9907 cDNA, wherein SEQ ID NO:69 is a clone designated herein as "DNA142392-2800".
Figure 70 shows the amino acid sequence (SEQ ID NO:70) derived from the coding sequence of SEQ ID NO:69 shown in Figure 69.
Figure 71 shows a nucleotide sequence (SEQ ED NO:71) of a native sequence PRO9873 cDNA, wherein SEQ ID NO:71 is a clone designated herein as "DNA143076-2787".
Figure 72 shows the amino acid sequence (SEQ ID NO:72) derived from the coding sequence of SEQ ID NO:71 shown in Figure 71.
Figure 73 shows a nucleotide sequence (SEQ ID NO:73) of a native sequence PRO10201 cDNA, wherein SEQ ID NO:73 is a clone designated herein as "DNA143294-2818".
Figure 74 shows the amino acid sequence (SEQ ID NO:74) derived from the coding sequence of SEQ ID NO:73 shown in Figure 73.
Figure 75 shows a nucleotide sequence (SEQ ID NO:75) of a native sequence PRO10200 cDNA, wherein SEQ ID NO:75 is a clone designated herein as "DNA143514-2817".
Figure 76 shows the amino acid sequence (SEQ ID NO:76) derived from the coding sequence of SEQ ID NO:75 shown in Figure 75.
Figure 77 shows a nucleotide sequence (SEQ ID NO:77) of a native sequence PRO10196 cDNA, wherein SEQ ID NO:77 is a clone designated herein as "DNA144841-2816".
Figure 78 shows the amino acid sequence (SEQ ID NO:78) derived from the coding sequence of SEQ ID NO:77 shown in Figure 77.
Figure 79 shows a nucleotide sequence (SEQ ID NO:79) of a native sequence PRO10282 cDNA, wherein SEQ ID NO:79 is a clone designated herein as "DNA148380-2827".
Figure 80 shows the amino acid sequence (SEQ ID NO:80) derived from the coding sequence of SEQ ID NO:79 shown in Figure 79.
Figure 81 shows a nucleotide sequence (SEQ ID NO:81) of a native sequence PRO19650 cDNA, wherein SEQ ID NO:81 is a clone designated herein as "DNA149995-2871"
Figure 82 shows the amino acid sequence (SEQ ID NO:82) derived from the coding sequence of SEQ ID NO:81 shown in Figure 81.
Figure 83 shows a nucleotide sequence (SEQ ID NO:83) of a native sequence PRO21194 cDNA, wherein SEQ ID NO:83 is a clone designated herein as "DNA167678-2963".
Figure 84 shows the amino acid sequence (SEQ ID NO:84) derived from the coding sequence of SEQ ID NO:83 shown in Figure 83.
Figure 85 shows a nucleotide sequence (SEQ ID NO:85) of a native sequence PRO21201 cDNA, wherein SEQ ID NO:85 is a clone designated herein as "DNA168028-2956".
Figure 86 shows the amino acid sequence (SEQ ID NO:86) derived from the coding sequence of SEQ ID NO:85 shown in Figure 85.
Figure 87 shows a nucleotide sequence (SEQ ID NO:87) of a native sequence PRO21175 cDNA, wherein SEQ ID NO:87 is a clone designated herein as "DNA173894-2947".
Figure 88 shows the amino acid sequence (SEQ ID NO:88) derived from the coding sequence of SEQ ID NO:87 shown in Figure 87.
Figure 89 shows a nucleotide sequence (SEQ ID NO:89) of a native sequence PRO21340 cDNA, wherein SEQ ID NO:89 is a clone designated herein as "DNA176775-2957".
Figure 90 shows the amino acid sequence (SEQ ID NO:90) derived from the coding sequence of SEQ ID NO:89 shown in Figure 89.
Figure 91 shows a nucleotide sequence (SEQ ID NO:91) of a native sequence PRO21384 cDNA, wherein SEQ ID NO:91 is a clone designated herein as "DNA177313-2982".
Figure 92 shows the amino acid sequence (SEQ ID NO:92) derived from the coding sequence of SEQ ID NO:91 shown in Figure 91,
Figure 93 shows a nucleotide sequence (SEQ ID NO:93) of a native sequence PRO982 cDNA, wherein SEQ ID NO:93 is a clone designated herein as "DNA57700-1408".
Figure 94 shows the amino acid sequence (SEQ ID NO:94) derived from the coding sequence of SEQ ID NO:93 shown in Figure 93.
Figure 95 shows a nucleotide sequence (SEQ ID NO:95) of a native sequence PRO1160 cDNA, wherein SEQ ID NO:95 is a clone designated herein as "DNA62872-1509".
Figure 96 shows the amino acid sequence (SEQ ID NO:96) derived from the coding sequence of SEQ ID NO:95 shown in Figure 95.
Figure 97 shows a nucleotide sequence (SEQ lD NO:97) of a native sequence PRO1187 cDNA, wherein SEQ ID NO:97 is a clone designated herein as "DNA62876 1517".
Figure 98 shows the amino acid sequence (SEQ ID NO:98) derived from the coding sequence of SEQ ID NO:97 shown in Figure 97.
Figure 99 shows a nucleotide sequence (SEQ ID NO:99) of a native sequence PRO1329 cDNA, wherein SEQ lD NO:99 is a clone designated herein as "DNA66660-1585".
Figure 100 shows the amino acid sequence (SEQ ID NO:100) derived from the coding sequence of SEQ lD NO:99 shown in Figure 99.
Figure 101 shows a nucleotide sequence (SEQ lD NO: 101) of a native sequence PRO231 cDNA, wherein SEQ ID NO:101 is a clone designated herein as "DNA34434-1139".
Figure 102 shows the amino acid sequence (SEQ ID NO:102) derived from the coding sequence of SEQ ID NO:101 shown in Figure 101.
Figure 103 shows a nucleotide sequence (SEQ ID NO:103) of a native sequence PRO357 cDNA, wherein SEQ ID NO:103 is a clone designated herein as "DNA44804-1248".
Figure 104 shows the amino acid sequence (SEQ ID NO:104) derived from the coding sequence of SEQ ID NO:103 shown in Figure 103.
Figure 105 shows a nucleotide sequence (SEQ ID NO:105) of a native sequence PRO725 cDNA, wherein SEQ ID NO:105 is a clone designated herein as "DNA52758-1399".
Figure 106 shows the amino acid sequence (SEQ ID NO:106) derived from the coding sequence of SEQ ID NO:105 shown in Figure 105.
Figure 107 shows a nucleotide sequence (SEQ ID NO:107) of a native sequence PRO1155 cDNA, wherein SEQ ID NO:107 is a clone designated herein as "DNA59849-1504".
Figure 108 shows the amino acid sequence (SEQ ID NO:108) derived from the coding sequence of SEQ ID NO:107 shown in Figure 107.
Figure 109 shows a nucleotide sequence (SEQ ID NO:109) of a native sequence PRO1306 cDNA, wherein SEQ ID NO:109 is a clone designated herein as "DNA65410-1569".
Figure 110 shows the amino acid sequence (SEQ ID NO:110) derived from the coding sequence of SEQ ID NO:109 shown in Figure 109.
Figure 111 shows a nucleotide sequence (SEQ ID NO:111) of a native sequence PRO1419 cDNA, wherein SEQ ID NO: 111 is a clone designated herein as "DNA71290-1630".
Figure 112 shows the amino acid sequence (SEQ ID NO:112) derived from the coding sequence of SEQ ID NO:111 shown in Figure 111.
Figure 113 shows a nucleotide sequence (SEQ ID NO: 113) of a native sequence PRO229 cDNA, wherein SEQ ID NO:113 is a clone designated herein as "DNA33100-1159".
Figure 114 shows the amino acid sequence (SEQ ID NO: 114) derived from the coding sequence of SEQ ID NO:113 shown in Figure 113.
Figure 115 shows a nucleotide sequence (SEQ ID NO: 115) of a native sequence PRO1272 cDNA, wherein SEQ ID NO:115 is a clone designated herein as "DNA64896-1539".
Figure 116 shows the amino acid sequence (SEQ ID NO:116) derived from the coding sequence of SEQ ID NO:115 shown in Figure 115.
Figure 117 shows a nucleotide sequence (SEQ ID NO:117) of a native sequence PRO4405 cDNA, wherein SEQ ID NO:117 is a clone designated herein as "DNA84920-2614".
Figure 118 shows the amino acid sequence (SEQ ID NO:118) derived from the coding sequence of SEQ ID NO:117 shown in Figure 117.
Figure 119 shows a nucleotide sequence (SEQ ID NO:119) of a native sequence PRO181 cDNA, wherein SEQ ID NO:119 is a clone designated herein as "DNA23330-1390".
Figure 120 shows the amino acid sequence (SEQ ID NO:120) derived from the coding sequence of SEQ ID NO:119 shown in Figure 119.
Figure 121 shows a nucleotide sequence (SEQ ID NO:121) of a native sequence PRO214 cDNA, wherein SEQ ID NO:121 is a clone designated herein as "DNA32286-1191".
Figure 122 shows the amino acid sequence (SEQ ID NO: 122) derived from the coding sequence of SEQ ID NO: 121 shown in Figure 121.
Figure 123 shows a nucleotide sequence (SEQ ID NO:123) of a native sequence PRO247 cDNA, wherein SEQ ID NO:123 is a clone designated herein as "DNA35673-1201".
Figure 124 shows the amino acid sequence (SEQ ID NO:124) derived from the coding sequence of SEQ ID NO:123 shown in Figure 123.
Figure 125 shows a nucleotide sequence (SEQ ID NO:125) of a native sequence PRO337 cDNA, wherein SEQ ID NO:125 is a clone designated herein as "DNA43316-1237".
Figure 126 shows the amino acid sequence (SEQ ID NO:126) derived from the coding sequence of SEQ ID NO:125 shown in Figure 125.
Figure 127 shows a nucleotide sequence (SEQ ID NO:127) of a native sequence PRO526 cDNA, wherein SEQ ID NO:127 is a clone designated herein as "DNA44184-1319".
Figure 128 shows the amino acid sequence (SEQ ID NO:128) derived from the coding sequence of SEQ ID NO:127 shown in Figure 127.
Figure 129 shows a nucleotide sequence (SEQ ID NO:129) of a native sequence PRO363 cDNA, wherein SEQ ID NO:129 is a clone designated herein as "DNA45419-1252".
Figure 130 shows the amino acid sequence (SEQ ID NO:130) derived from the coding sequence of SEQ ID NO:129 shown in Figure 129.
Figure 131 shows a nucleotide sequence (SEQ ID NO:131) of a native sequence PRO531 cDNA, wherein SEQ ID NO:131 is a clone designated herein as "DNA48314-1320".
Figure 132 shows the amino acid sequence (SEQ ID NO:132) derived from the coding sequence of SEQ ID NO:131 shown in Figure 131.
Figure 133 shows a nucleotide sequence (SEQ ID NO:133) of a native sequence PRO1083 cDNA, wherein SEQ ID NO:133 is a clone designated herein as "DNA50921-1458".
Figure 134 shows the amino acid sequence (SEQ ID NO:134) derived from the coding sequence of SEQ ID NO:133 shown in Figure 133.
Figure 135 shows a nucleotide sequence (SEQ ID NO:135) of a native sequence PRO840 cDNA, wherein SEQ ID NO:135 is a clone designated herein as "DNAS3987".
Figure 136 shows the amino acid sequence (SEQ ID NO:136) derived from the coding sequence of SEQ ID NO:135 shown in Figure 135.
Figure 137 shows a nucleotide sequence (SEQ ID NO:137) of a native sequence PRO1080 cDNA, wherein SEQ ID NO:137 is a clone designated herein as "DNA56047-1456".
Figure 138 shows the amino acid sequence (SEQ ID NO:138) derived from the coding sequence of SEQ ID NO:137 shown in Figure 137.
Figure 139 shows a nucleotide sequence (SEQ ID NO:139) of a native sequence PRO788 cDNA, wherein SEQ ID NO:139 is a clone designated herein as "DNAS6405-1357".
Figure 140 shows the amino acid sequence (SEQ ID NO:140) derived from the coding sequence of SEQ ID NO:139 shown in Figure 139.
Figure 141 shows a nucleotide sequence (SEQ ID NO:141) of a native sequence PRO1478 cDNA, wherein SEQ ID NO:141 is a clone designated herein as "DNA56531-1648".
Figure 142 shows the amino acid sequence (SEQ ID NO:142) derived from the coding sequence of SEQ ID NO:141 shown in Figure 141.
Figure 143 shows a nucleotide sequence (SEQ ID NO:143) of a native sequence PRO1134 cDNA, wherein SEQ ID NO:143 is a clone designated herein as "DNA56865-1491"
Figure 144 shows the amino acid sequence (SEQ ID NO:144) derived from the coding sequence of SEQ ID NO: 143 shown in Figure 143.
Figure 145 shows a nucleotide sequence (SEQ ID NO:145) of a native sequence PRO826 cDNA, wherein SEQ ID NO: 145 is a clone designated herein as "DNA57694-1341".
Figure 146 shows the amino acid sequence (SEQ ID NO:146) derived from the coding sequence of SEQ ID NO: 145 shown in Figure 145.
Figure 147 shows a nucleotide sequence (SEQ ID NO:147) of a native sequence PRO1005 cDNA, wherein SEQ ID NO:147 is a clone designated herein as "DNA57708-1411".
Figure 148 shows the amino acid sequence (SEQ ID NO:148) derived from the coding sequence of SEQ ID NO:147 shown in Figure 147.
Figure 149 shows a nucleotide sequence (SEQ ID NO:149) of a native sequence PRO809 cDNA, wherein SEQ ID NO:149 is a clone designated herein as "DNA57836-1338".
Figure 150 shows the amino acid sequence (SEQ ID NO: 150) derived from the coding sequence of SEQ ID NO: 149 shown in Figure 149.
Figure 151 shows a nucleotide sequence (SEQ ID NO:151) of a native sequence PRO1194 cDNA, wherein SEQ ID NO:151 is a clone designated herein as "DNA57841-1522".
Figure 152 shows the amino acid sequence (SEQ ID NO:152) derived from the coding sequence of SEQ ID NO:151 shown in Figure 151.
Figure 153 shows a nucleotide sequence (SEQ ID NO:153) of a native sequence PRO1071 cDNA, wherein SEQ ID NO:153 is a clone designated herein as "DNA58847-1383".
Figure 154 shows the amino acid sequence (SEQ ID NO:154) derived from the coding sequence of SEQ ID NO:153 shown in Figure 153.
Figure 155 shows a nucleotide sequence (SEQ ID NO:155) of a native sequence PRO1411 cDNA, wherein SEQ ID NO:155 is a clone designated herein as "DNA59212-1627".
Figure 156 shows the amino acid sequence (SEQ ID NO:156) derived from the coding sequence of SEQ ID NO:155 shown in Figure 155.
Figure 157 shows a nucleotide sequence (SEQ ID NO:157) of a native sequence PRO1309 cDNA, wherein SEQ ID NO:157 is a clone designated herein as "DNA59588-1571".
Figure 158 shows the amino acid sequence (SEQ ID NO:158) derived from the coding sequence of SEQ ID NO:157 shown in Figure 157.
Figure 159 shows a nucleotide sequence (SEQ ID NO:159) of a native sequence PRO1025 cDNA, wherein SEQ ID NO:159 is a clone designated herein as "DNA59622-1334".
Figure 160 shows the amino acid sequence (SEQ ID NO:160) derived from the coding sequence of SEQ ID NO:159 shown in Figure 159.
Figure 161 shows a nucleotide sequence (SEQ ID NO:161) of a native sequence PRO1181 cDNA, wherein SEQ ID NO:161 is a clone designated herein as "DNA59847-251.0".
Figure 162 shows the amino acid sequence (SEQ ID NO:162) derived from the coding sequence of SEQ ID NO:161 shown in Figure 161.
Figure 163 shows a nucleotide sequence (SEQ ID NO:163) of a native sequence PRO1126 cDNA, wherein SEQ ID NO: 163 is a clone designated herein as "DNA60615-1483".
Figure 164 shows the amino acid sequence (SEQ ID NO: 164) derived from the coding sequence of SEQ ID NO:163 shown in Figure 163.
Figure 165 shows a nucleotide sequence (SEQ ID NO:165) of a native sequence PRO1186 cDNA, wherein SEQ ID NO: 165 is a clone designated herein as "DNA60621-1516".
Figure 166 shows the amino acid sequence (SEQ ID NO:166) derived from the coding sequence of SEQ ID NO:165 shown in Figure 165.
Figure 167 shows a nucleotide sequence (SEQ ID NO:167) of a native sequence PRO1192 cDNA, wherein SEQ ID NO: 167 is a clone designated herein as "DNA62814-1521".
Figure 168 shows the amino acid sequence (SEQ ID NO:168) derived from the coding sequence of SEQ ID NO: 167 shown in Figure 167.
Figure 169 shows a nucleotide sequence (SEQ ID NO:169) of a native sequence PRO 1244 cDNA, wherein SEQ ID NO: 169 is a clone designated herein as "DNA64883-1526".
Figure 170 shows the amino acid sequence (SEQ ID NO:170) derived from the coding sequence of SEQ ID NO: 169 shown in Figure 169.
Figure 171 shows a nucleotide sequence (SEQ ID NO: 17 1) of a native sequence PRO L274 cDNA, wherein SEQ ID NO: 171 is a clone designated herein as "DNA64889-1541".
Figure 172 shows the amino acid sequence (SEQ ID NO:172) derived from the coding sequence of SEQ ID NO:171 shown in Figure 171.
Figure 173 shows a nucleotide sequence (SEQ ID NO:173) of a native sequence PRO1412 cDNA, wherein SEQ ID NO:173 is a clone designated herein as "DNA64897-1628".
Figure 174 shows the amino acid sequence (SEQ ID NO:174) derived from the coding sequence of SEQ ID NO:173 shown in Figure 173.
Figure 175 shows a nucleotide sequence (SEQ ID NO:175) of a native sequence PRO1286 cDNA, wherein SEQ ID NO: 175 is a clone designated herein as "DNA64903-1553".
Figure 176 shows the amino acid sequence (SEQ ID NO:176) derived from the coding sequence of SEQ ID NO: 175 shown in Figure 175.
Figure 177 shows a nucleotide sequence (SEQ ID NO:177) of a native sequence PRO1330 cDNA, wherein SEQ ID NO:177 is a clone designated herein as "DNA64907-1163-1".
Figure 178 shows the amino acid sequence (SEQ ID NO:178) derived from the coding sequence of SEQ ID NO:177 shown in Figure 177.
Figure 179 shows a nucleotide sequence (SEQ ID NO: 179) of a native sequence PRO1347 cDNA, wherein SEQ ID NO: 179 is a clone designated herein as "DNA64950-1590".
Figure 180 shows the amino acid sequence (SEQ ID NO:180) derived from the coding sequence of SEQ ID NO: 179 shown in Figure 179.
Figure 181 shows a nucleotide sequence (SEQ ID NO: 181) of a native sequence PRO1305 cDNA, wherein SEQ ID NO:181 is a clone designated herein as "DNA64952-1568".
Figure 182 shows the amino acid sequence (SEQ ID NO: 182) derived from the coding sequence of SEQ ID NO:181 shown in Figure 181.
Figure 183 shows a nucleotide sequence (SEQ ID NO:183) of a native sequence PRO1273 cDNA, wherein SEQ ID NO:183 is a clone designated herein as "DNA65402-1540".
Figure 184 shows the amino acid sequence (SEQ ID NO:184) derived from the coding sequence of SEQ ID NO:183 shown in Figure 183.
Figure 185 shows a nucleotide sequence (SEQ ID NO:185) of a native sequence PRO1279 cDNA, wherein SEQ ID NO:185 is a clone designated herein as "DNA65405-1547".
Figure 186 shows the amino acid sequence (SEQ ID NO:186) derived from the coding sequence of SEQ ID NO:185 shown in Figure 185.
Figure 187 shows a nucleotide sequence (SEQ ID NO:187) of a native sequence PRO1340 cDNA, wherein SEQ ID NO:187 is a clone designated herein as "DNA66663-1598".
Figure 188 shows the amino acid sequence (SEQ ID NO: 188) derived from the coding sequence of SEQ ID NO:187 shown in Figure 187.
Figure 189 shows a nucleotide sequence (SEQ ID NO: 189) of a native sequence PRO 1338 cDNA, wherein SEQ ID NO:189 is a clone designated herein as "DNA66667".
Figure 190 shows the amino acid sequence (SEQ ID NO:190) derived from the coding sequence of SEQ ID NO:189 shown in Figure 189.
Figure 191 shows a nucleotide sequence (SEQ ID NO:191) of a native sequence PRO1343 cDNA, wherein SEQ ID NO: 191 is a clone designated herein as "DNA66675-1587".
Figure 192 shows the amino acid sequence (SEQ ID NO:192) derived from the coding sequence of SEQ ID NO:191 shown in Figure 191.
Figure 193 shows a nucleotide sequence (SEQ ID NO:193) of a native sequence PRO1376 cDNA, wherein SEQ ID NO:193 is a clone designated herein as "DNA67300-1605".
Figure 194 shows the amino acid sequence (SEQ ID NO:194) derived from the coding sequence of SEQ ID NO:193 shown in Figure 193.
Figure 195 shows a nucleotide sequence (SEQ ID NO:195) of a native sequence PRO1387 cDNA, wherein SEQ ID NO:195 is a clone designated herein as "DNA68872-1620".
Figure 196 shows the amino acid sequence (SEQ ID NO : 196) derived from the coding sequence of SEQ ID NO:195 shown in Figure 195.
Figure 197 shows a nucleotide sequence (SEQ ID NO:197) of a native sequence PRO1409 cDNA, wherein SEQ ID NO:197 is a clone designated herein as "DNA71269-1621".
Figure 198 shows the amino acid sequence (SEQ ID NO:198) derived from the coding sequence of SEQ ID NO:197 shown in Figure 197.
Figure 199 shows a nucleotide sequence (SEQ ID NO:199) of a native sequence PRO1488 cDNA, wherein SEQ ID NO:199 is a clone designated herein as "DNA73736-1657".
Figure 200 shows the amino acid sequence (SEQ ID NO:200) derived from the coding sequence of SEQ ID NO:199 shown in Figure 199.
Figure 201 shows a nucleotide sequence (SEQ ID NO:201) of a native sequence PRO1474 cDNA, wherein SEQ ID NO:201 is a clone designated herein as "DNA73739-1645".
Figure 202 shows the amino acid sequence (SEQ ID NO:202) derived from the coding sequence of SEQ ID NO:201 shown in Figure 201.
Figure 203 shows a nucleotide sequence (SEQ ID NO:203) of a native sequence PRO191 cDNA, wherein SEQ ID NO:203 is a clone designated herein as "DNA76400-2528".
Figure 204 shows the amino acid sequence (SEQ ID NO:204) derived from the coding sequence of SEQ ID NO:203 shown in Figure 203.
Figure 205 shows a nucleotide sequence (SEQ ID NO:205) of a native sequence PRO1760 cDNA, wherein SEQ ID NO:205 is a clone designated herein as "DNA76532-1702".
Figure 206 shows the amino acid sequence (SEQ ID NO:206) derived from the coding sequence of SEQ ID NO:205 shown in Figure 205.
Figure 207 shows a nucleotide sequence (SEQ ID NO:207) of a native sequence PRO 1567 cDNA, wherein SEQ ID NO:207 is a clone designated herein as "DNA76541-1675".
Figure 208 shows the amino acid sequence (SEQ ID NO:208) derived from the coding sequence of SEQ ID NO:207 shown in Figure 207.
Figure 209 shows a nucleotide sequence (SEQ ID NO:209) of a native sequence PRO1887 cDNA, wherein SEQ ID NO:209 is a clone designated herein as "DNA79862-2522".
Figure 210 shows the amino acid sequence (SEQ ID NO:210) derived from the coding sequence of SEQ ID NO:209 shown in Figure 209.
Figure 211 shows a nucleotide sequence (SEQ ID NO:211) of a native sequence PRO1928 cDNA, wherein SEQ ID NO:211 is a clone designated herein as " DNA81754-2532 ".
Figure 212 shows the amino acid sequence (SEQ ID NO:212) derived from the coding sequence of SEQ ID NO:211 shown in Figure 211.
Figure 213 shows a nucleotide sequence (SEQ ID NO:213) of a native sequence PRO4341 cDNA, wherein SEQ ID NO:213 is a clone designated herein as "DNA81761-2583".
Figure 214 shows the amino acid sequence (SEQ ID NO:214) derived from the coding sequence of SEQ ID NO:213 shown in Figure 213.
Figure 215 shows a nucleotide sequence (SEQ ID NO:215) of a native sequence PRO5723 cDNA, wherein SEQ ID NO:215 is a clone designated herein as "DNA82361".
Figure 216 shows the amino acid sequence (SEQ ID NO:216) derived from the coding sequence of SEQ ID NO:215 shown in Figure 215.
Figure 217 shows a nucleotide sequence (SEQ [D NO:217) of a native sequence PRO1801 cDNA,
   wherein SEQ ID NO:217 is a clone designated herein as "DNA83500-2506"
Figure 218 shows the amino acid sequence (SEQ ID NO:218) derived from the coding sequence of SEQ ID NO:217 shown in Figure 217.
Figure 219 shows a nucleotide sequence (SEQ ID NO:219) of a native sequence PRO4333 cDNA, wherein SEQ ID NO:219 is a clone designated herein as "DNA84210-2576".
Figure 220 shows the amino acid sequence (SEQ ID NO:220) derived from the coding sequence of SEQ ID NO:219 shown in Figure 219.
Figure 221 shows a nucleotide sequence (SEQ ID NO:221) of a native sequence PRO3543 cDNA, wherein SEQ ID NO:221 is a clone designated herein as "DNA86571-2551".
Figure 222 shows the amino acid sequence (SEQ ID NO:222) derived from the coding sequence of SEQ ID NO:221 shown in Figure 221.
Figure 223 shows a nucleotide sequence (SEQ ID NO:223) of a native sequence PRO3444 cDNA, wherein SEQ ID NO:223 is a clone designated herein as "DNA87997".
Figure 224 shows the amino acid sequence (SEQ ID NO:224) derived from the coding sequence of SEQ ID NO:223 shown in Figure 223.
Figure 225 shows a nucleotide sequence (SEQ ID NO:225) of a native sequence PRO4302 cDNA, wherein SEQ ID NO:225 is a clone designated herein as "DNA92218-2554".
Figure 226 shows the amino acid sequence (SEQ ID NO:226) derived from the coding sequence of SEQ ID NO:225 shown in Figure 225.
Figure 227 shows a nucleotide sequence (SEQ ID NO:227) of a native sequence PRO4322 cDNA, wherein SEQ ID NO:227 is a clone designated herein as "DNA92223-2567".
Figure 228 shows the amino acid sequence (SEQ ID NO:228) derived from the coding sequence of SEQ ID NO:227 shown in Figure 227.
Figure 229 shows a nucleotide sequence (SEQ ID NO:229) of a native sequence PRO5725 cDNA, wherein SEQ ID NO:229 is a clone designated herein as "DNA92265-2669".
Figure 230 shows the amino acid sequence (SEQ ID NO:230) derived from the coding sequence of SEQ ID NO:229 shown in Figure 229.
Figure 231 shows a nucleotide sequence (SEQ ID NO:231) of a native sequence PRO4408 cDNA, wherein SEQ ID NO:231 is a clone designated herein as "DNA92274-2617".
Figure 232 shows the amino acid sequence (SEQ ID NO:232) derived from the coding sequence of SEQ ID NO:231 shown in Figure 231.
Figure 233 shows a nucleotide sequence (SEQ ID NO:233) of a native sequence PRO9940 cDNA, wherein SEQ ID NO:223 is a clone designated herein as "DNA92282".
Figure 234 shows the amino acid sequence (SEQ ID NO:234) derived from the coding sequence of SEQ ID NO:233 shown in Figure 233.
Figure 235 shows a nucleotide sequence (SEQ ID NO:235) of a native sequence PRO7154 cDNA, wherein SEQ ID NO:235 is a clone designated herein as "DNA108760-2740".
Figure 236 shows the amino acid sequence (SEQ ID NO:236) derived from the coding sequence of SEQ ID NO:235 shown in Figure 235.
Figure 237 shows a nucleotide sequence (SEQ ID NO:237) of a native sequence PRO7425 cDNA, wherein SEQ ID NO:237 is a clone designated herein as "DNA108792-2753".
Figure 238 shows the amino acid sequence (SEQ ID NO:238) derived from the coding sequence of SEQ ID NO:237 shown in Figure 237.
Figure 239 shows a nucleotide sequence (SEQ ID NO:239) of a native sequence PRO6079 cDNA, wherein SEQ ID NO:239 is a clone designated herein as "DNA111750-2706".
Figure 240 shows the amino acid sequence (SEQ ID NO:240) derived from the coding sequence of SEQ ID NO:239 shown in Figure 239.
Figure 241 shows a nucleotide sequence (SEQ ID NO:241) of a native sequence PRO9836 cDNA, wherein SEQ ID NO:241 is a clone designated herein as "DNA119514-2772".
Figure 242 shows the amino acid sequence (SEQ ID NO:242) derived from the coding sequence of SEQ ID NO:241 shown in Figure 241.
Figure 243 shows a nucleotide sequence (SEQ ID NO:243) of a native sequence PRO10096 cDNA, wherein SEQ ID NO:243 is a clone designated herein as "DNA125185-2806".
Figure 244 shows the amino acid sequence (SEQ LD NO:244) derived from the coding sequence of SEQ
ID NO:243 shown in Figure 243.

### DETAILED DESCRIPTION OF THE PREFERRED EMBODIMENTS

### I. Definitions

The terms "PRO polypeptide" and "PRO" as used herein and when immediately followed by a numerical designation refer to various polypeptides, Wherein the complete designation (i.e., PRO/number) refers to specific polypeptide sequences as described herein. The terms "PRO/number polypeptide" and "PRO/number" wherein the term "number" is provided as an actual numerical designation as used herein encompass native sequence polypeptides and polypeptide variants (which are further defined herein). The PRO polypeptides described herein may be isolated from a variety of sources, such as from human tissue types or from another source, or prepared by recombinant or synthetic methods. The term "PRO polypeptide" refers to each individual PRO/number polypeptide disclosed herein. All disclosures in this specification which refer to the "PRO polypeptide" refer to each of the polypeptides individually as well as jointly. For example, descriptions of the preparation of, purification of, derivation of, formation of antibodies to or against, administration of, compositions containing, treatment of a disease with, etc., pertain to each polypeptide of the invention individually. The term "PRO polypeptide" also includes variants of the PRO/number polypeptides disclosed herein.

A "native sequence PRO polypeptide" comprises a polypeptide having the same amino acid sequence as the corresponding PRO polypeptide derived from nature. Such native sequence PRO polypeptides can be isolated from nature or can be produced by recombinant or synthetic means. The term "native sequence PRO polypeptide" specifically encompasses naturally-occurring truncated or secreted forms of the specific PRO polypeptide (e.g., an extracellular domain sequence), naturally-occurring variant forms (e.g., alternatively spliced forms) and naturally-occurring allelic variants of the polypeptide. In various embodiments of the invention, the native sequence PRO polypeptides disclosed herein are mature or full-length native sequence polypeptides comprising the full-length amino acids sequences shown in the accompanying figures. Start and stop codons are shown in bold font and underlined in the figures. However, while the PRO polypeptide disclosed in the accompanying figures are shown to begin with methionine residues designated herein as amino acid position 1 in the figures, it is conceivable and possible that other methionine residues located either upstream or downstream from the amino acid position 1 in the figures may be employed as the starting amino acid residue for the PRO polypeptides.

The PRO polypeptide "extracellular domain" or "ECD" refers to a form of the PRO polypeptide which is essentially free of the transmembrane and cytoplasmic domains. Ordinarily, a PRO polypeptide ECD will have less than 1% of such transmembrane and/or cytoplasmic domains and preferably, will have less than 0.5% of such domains. It will be understood that any transmembrane domains identified for the PRO polypeptides of the present invention are identified pursuant to criteria routinely employed in the art for identifying that type of hydrophobic domain. The exact boundaries of a transmembrane domain may vary but most likely by no more than about 5 amino acids at either end of the domain as initially identified herein. Optionally, therefore, an extracellular domain of a PRO polypeptide may contain from about 5 or fewer amino acids on either side of the transmembrane domain/extracellular domain boundary as identified in the Examples or specification and such polypeptides, with or without the associated signal peptide, and nucleic acid encoding them, are comtemplated by the present invention.

The approximate location of the "signal peptides" of the various PRO polypeptides disclosed herein are shown in the present specification and/or the accompanying figures. It is noted, however, that the C-terminal boundary of a signal peptide may vary, but most likely by no more than about 5 amino acids on either side of the signal peptide C-terminal boundary as initially identified herein, wherein the C-terminal boundary of the signal peptide may be identified pursuant to criteria routinely employed in the art for identifying that type of amino acid sequence element (e.g., Nielsen et al., Prot. Eng. 10:1-6 (1997) and von Heinje et al., Nucl. Acids. Res. 14:4683-4690 (1986)). Moreover, it is also recognized that, in some cases, cleavage of a signal sequence from a secreted polypeptide is not entirely uniform, resulting in more than one secreted species. These mature polypeptides, where the signal peptide is cleaved within no more than about 5 amino acids on either side of the C-terminal boundary of the signal peptide as identified herein, and the polynucleotides encoding them, are contemplated by the present invention.

"PRO polypeptide variant" means an active PRO polypeptide as defined above or below having at least about 80% amino acid sequence identity with a full-length native sequence PRO polypeptide sequence as disclosed herein, a PRO polypeptide sequence lacking the signal peptide as disclosed herein, an extracellular domain of a PRO polypeptide, with or without the signal peptide, as disclosed herein or any other fragment of a full-length PRO polypeptide sequence as disclosed herein. Such PRO polypeptide variants include, for instance, PRO polypeptides wherein one or more amino acid residues are added, or deleted, at the N- or C-terminus of the full-length native amino acid sequence. Ordinarily, a PRO polypeptide variant will have at least about 80% amino acid sequence identity, alternatively at least about 81 % amino acid sequence identity, alternatively at least about 82% amino acid sequence identity, alternatively at least about 83% amino acid sequence identity, alternatively at least about 84% amino acid sequence identity, alternatively at least about 85% amino acid sequence identity, alternatively at least about 86% amino acid sequence identity, alternatively at least about 87% amino acid sequence identity, alternatively at least about 88 % amino acid sequence identity, alternatively at least about 89 % amino acid sequence identity, alternatively at least about 90% amino acid sequence identity, alternatively at least about 91% amino acid sequence identity, alternatively at least about 92% amino acid sequence identity, alternatively at least about 93% amino acid sequence identity, alternatively at least about 94 % amino acid sequence identity, alternatively at least about 95 % amino acid sequence identity, alternatively at least about 96% amino acid sequence identity, alternatively at least about 97% amino acid sequence identity, alternatively at least about 98 % amino acid sequence identity and alternatively at least about 99 % amino acid sequence identity to a full-length native sequence PRO polypeptide sequence as disclosed herein, a PRO polypeptide sequence lacking the signal peptide as disclosed herein, an extracellular domain of a PRO polypeptide, with or without the signal peptide, as disclosed herein or any other specifically defined fragment of a full-length PRO polypeptide sequence as disclosed herein. Ordinarily, PRO variant polypeptides are at least about 10 amino acids in length, alternatively at least about 20 amino acids in length, alternatively at least about 30 amino acids in length, alternatively at least about 40 amino acids in length, alternatively at least about 50 amino acids in length, alternatively at least about 60 amino acids in length, alternatively at least about 70 amino acids in length, alternatively at least about 80 amino acids in length, alternatively at least about 90 amino acids in length, alternatively at least about 100 amino acids in length, alternatively at least about 150 amino acids in length, alternatively at least about 200 amino acids in length, alternatively at least about 300 amino acids in length, or more.

"Percent (%) amino acid sequence identity" with respect to the PRO polypeptide sequences identified herein is defined as the percentage of amino acid residues in a candidate sequence that are identical with the amino acid residues in the specific PRO polypeptide sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN or Megalign (DNASTAR) software. Those skilled in the art can determine appropriate parameters for measuring alignment, including any algorithms needed to achieve maximal alignment over the full length of the sequences being compared. For purposes herein, however, % amino acid sequence identity values are generated using the sequence comparison computer program ALIGN-2, wherein the complete source code for the ALIGN-2 program is provided in Table 1 below. The ALIGN-2 sequence comparison computer program was authored by Genentech, Inc. and the source code shown in Table 1 below has been filed with user documentation in the U.S. Copyright Office, Washington D.C., 20559, where it is registered under U.S. Copyright Registration No. TXU510087. The ALIGN-2 program is publicly available through Genentech, Inc., South San Francisco, California or may be compiled from the source code provided in Table 1 below. The ALIGN-2 program should be compiled for use on a UNIX operating system, preferably digital UNIX V4.0D. All sequence comparison parameters are set by the ALIGN-2 program and do not vary.

In situations where ALIGN-2 is employed for amino acid sequence comparisons, the % amino acid sequence identity of a given amino acid sequence A to, with, or against a given amino acid sequence B (which can alternatively be phrased as a given amino acid sequence A that has or comprises a certain % amino acid sequence identity to, with, or against a given amino acid sequence B) is calculated as follows:
100 times the fraction X/Y
where X is the number of amino acid residues scored as identical matches by the sequence alignment program ALIGN-2 in that program's alignment of A and B, and where Y is the total number of amino acid residues in B. It will be appreciated that where the length of amino acid sequence A is not equal to the length of amino acid sequence B, the % amino acid sequence identity of A to B will not equal the % amino acid sequence identity of B to A. As examples of % amino acid sequence identity calculations using this method, Tables 2 and 3 demonstrate how to calculate the % amino acid sequence identity of the amino acid sequence designated "Comparison Protein" to the amino acid sequence designated "PRO", wherein "PRO" represents the amino acid sequence of a hypothetical PRO polypeptide of interest, "Comparison Protein" represents the amino acid sequence of a polypeptide against which the "PRO" polypeptide of interest is being compared, and "X, "Y" and "Z" each represent different hypothetical amino acid residues.

Unless specifically stated otherwise, all % amino acid sequence identity values used herein are obtained as described in the immediately preceding paragraph using the ALIGN-2 computer program. However, % amino acid sequence identity values may also be obtained as described below by using the WU-BLAST-2 computer program (Altschul et al., Methods in Enzymology 266:460-480 (1996)). Most of the WLT-BLAST-2 search parameters are set to the default values. Those not set to default values, i.e., the adjustable parameters, are set with the following values: overlap span = 1, overlap fraction = 0.125, word threshold (T) = 11, and scoring matrix = BLOSUM62. When WU-BLAST-2 is employed, a % amino acid sequence identity value is determined by dividing (a) the number of matching identical amino acid residues between the amino acid sequence of the PRO polypeptide of interest having a sequence derived from the native PRO polypeptide and the comparison amino acid sequence of interest (i.e., the sequence against which the PRO polypeptide of interest is being compared which may be a PRO variant polypeptide) as determined by WU-BLAST-2 by (b) the total number of amino acid residues of the PRO polypeptide of interest. For example, in the statement "a polypeptide comprising an the amino acid sequence A which has or having at least 80% amino acid sequence identity to the amino acid sequence B", the amino acid sequence A is the comparison amino acid sequence of interest and the amino acid sequence B is the amino acid sequence of the PRO polypeptide of interest.

Percent amino acid sequence identity may also be determined using the sequence comparison program NCBI-BLAST2 (Altschul et al., Nucleic Acids Res. 25:3389-3402 (1997)). The NCBI-BLAST2 sequence comparison program may be downloaded from http://www.ncbi.nlm.nih.gov or otherwise obtained from the National Institute of Health, Bethesda, MD. NCBI-BLAST2 uses several search parameters, wherein all of those search parameters are set to default values including, for example, unmask = yes, strand = all, expected occurrences = 10, minimum low complexity length = 15/5, multi-pass e-value = 0.01, constant for multi-pass = 25, dropoff for final gapped alignment = 25 and scoring matrix = BLOSUM62.

In situations where NCBI-BLAST2 is employed for amino acid sequence comparisons, the % amino acid sequence identity of a given amino acid sequence A to, with, or against a given amino acid sequence B (which can alternatively be phrased as a given amino acid sequence A that has or comprises a certain % amino acid sequence identity to, with, or against a given amino acid sequence B) is calculated as follows:
100 times the fraction X/Y
where X is the number of amino acid residues scored as identical matches by the sequence alignment program NCBI-BLAST2 in that program's alignment of A and B, and where Y is the total number of amino acid residues in B. It will be appreciated that where the length of amino acid sequence A is not equal to the length of amino acid sequence B, the % amino acid sequence identity of A to B will not equal the % amino acid sequence identity of B to A.

"PRO variant polynucleotide" or "PRO variant nucleic acid sequence" means a nucleic acid molecule which encodes an active PRO polypeptide as defined below and which has at least about 80% nucleic acid sequence identity with a nucleotide acid sequence encoding a full-length native sequence PRO polypeptide sequence as disclosed herein, a full-length native sequence PRO polypeptide sequence lacking the signal peptide as disclosed herein, an extracellular domain of a PRO polypeptide, with or without the signal peptide, as disclosed herein or any other fragment of a full-length PRO polypeptide sequence as disclosed herein. Ordinarily, a PRO variant polynucleotide will have at least about 80 % nucleic acid sequence identity, alternatively at least about 81 % nucleic acid sequence identity, alternatively at least about 82 % nucleic acid sequence identity, alternatively at least about 83% nucleic acid sequence identity, alternatively at least about 84% nucleic acid sequence identity, alternatively at least about 85% nucleic acid sequence identity, alternatively at least about 86% nucleic acid sequence identity, alternatively at least about 87 % nucleic acid sequence identity, alternatively at least about 88 % nucleic acid sequence identity, alternatively at least about 89% nucleic acid sequence identity, alternatively at least about 90% nucleic acid sequence identity, alternatively at least about 91 % nucleic acid sequence identity, alternatively at least about 92% nucleic acid sequence identity, alternatively at least about 93% nucleic acid sequence identity, alternatively at least about 94 % nucleic acid sequence identity, alternatively at least about 95 % nucleic acid sequence identity, alternatively at least about 96 % nucleic acid sequence identity, alternatively at least about 97% nucleic acid sequence identity, alternatively at least about 98% nucleic acid sequence identity and alternatively at least about 99% nucleic acid sequence identity with a nucleic acid sequence encoding a full-length native sequence PRO polypeptide sequence as disclosed herein, a full-length native sequence PRO polypeptide sequence lacking the signal peptide as disclosed herein, an extracellular domain of a PRO polypeptide, with or without the signal sequence, as disclosed herein or any other fragment of a full-length PRO polypeptide sequence as disclosed herein. Variants do not encompass the native nucleotide sequence.

Ordinarily, PRO variant polynucleotides are at least about 30 nucleotides in length, alternatively at least about 60 nucleotides in length, alternatively at least about 90 nucleotides in length, alternatively at least about 120 nucleotides in length, alternatively at least about 150 nucleotides in length, alternatively at least about 180 nucleotides in length, alternatively at least about 210 nucleotides in length, alternatively at least about 240 nucleotides in length, alternatively at least about 270 nucleotides in length, alternatively at least about 300 nucleotides in length, alternatively at least about 450 nucleotides in length, alternatively at least about 600 nucleotides in length, alternatively at least about 900 nucleotides in length, or more.

"Percent (%) nucleic acid sequence identity" with respect to PRO-encoding nucleic acid sequences identified herein is defined as the percentage of nucleotides in a candidate sequence that are identical with the nucleotides in the PRO nucleic acid sequence of interest, after aligning the sequences and introducing gaps, if necessary, to achieve the maximum percent sequence identity. Alignment for purposes of determining percent nucleic acid sequence identity can be achieved in various ways that are within the skill in the art, for instance, using publicly available computer software such as BLAST, BLAST-2, ALIGN or Megalign (DNASTAR) software. For purposes herein, however, % nucleic acid sequence identity values are generated using the sequence comparison computer program ALIGN-2, wherein the complete source code for the ALIGN-2 program is provided in Table 1 below. The ALIGN-2 sequence comparison computer program was authored by Genentech, Inc. and the source code shown in Table 1 below has been filed with user documentation in the U.S. Copyright Office, Washington D.C., 20559, where it is registered under U.S. Copyright Registration No. TXU510087. The ALIGN-2 program is publicly available through Genentech, Inc., South San Francisco, California or may be compiled from the source code provided in Table 1 below. The ALIGN-2 program should be compiled for use on a UNIX operating system, preferably digital UNIX V4.0D. All sequence comparison parameters are set by the ALIGN-2 program and do not vary.

In situations where ALIGN-2 is employed for nucleic acid sequence comparisons, the % nucleic acid sequence identity of a given nucleic acid sequence C to, with, or against a given nucleic acid sequence D (which can alternatively be phrased as a given nucleic acid sequence, C that has or comprises a certain % nucleic acid sequence identity to, with, or against a given nucleic acid sequence D) is calculated as follows:
100 times the fraction W/Z
where W is the number of nucleotides scored as identical matches by the sequence alignment program ALIGN-2 in that program's alignment of C and D, and where Z is the total number of nucleotides in D. It will be appreciated that where the length of nucleic acid sequence C is not equal to the length of nucleic acid sequence D, the % nucleic acid sequence identity of C to D will not equal the % nucleic acid sequence identity of D to C. As examples of % nucleic acid sequence identity calculations. Tables 4 and 5, demonstrate how to calculate the % nucleic acid sequence identity of the nucleic acid sequence designated "Comparison DNA" to the nucleic acid sequence designated "PRO-DNA", wherein "PRO-DNA" represents a hypothetical PRO-encoding nucleic acid sequence of interest, "Comparison DNA" represents the nucleotide sequence of a nucleic acid molecule against which the "PRO-DNA" nucleic acid molecule of interest is being compared, and "N", "L" and "V" each represent different hypothetical nucleotides.

Unless specifically stated otherwise, all % nucleic acid sequence identity values used herein are obtained as described in the immediately preceding paragraph using the ALIGN-2 computer program. However, % nucleic acid sequence identity values may also be obtained as described below by using the WU-BLAST-2 computer program (Altschul et al., Methods in Enzymology 266:460-480 (1996)). Most of the WU-BLAST-2 search parameters are set to the default values. Those not set to default values, i.e., the adjustable parameters, are set with the following values: overlap span = 1, overlap fraction = 0.125, word threshold (T) = 11, and scoring matrix = BLOSUM62. When WU-BLAST-2 is employed, a % nucleic acid sequence identity value is determined by dividing (a) the number of matching identical nucleotides between the nucleic acid sequence of the PRO polypeptide-encoding nucleic acid molecule of interest having a sequence derived from the native sequence PRO polypeptide-encoding nucleic acid and the comparison nucleic acid molecule of interest (i.e., the sequence against which the PRO polypeptide-encoding nucleic acid molecule of interest is being compared which may be a variant PRO polynucleotide) as determined by WU-BLAST-2 by (b) the total number of nucleotides of the PRO polypeptide-encoding nucleic acid molecule of interest. For example, in the statement "an isolated nucleic acid molecule comprising a nucleic acid sequence A which has or having at least 80% nucleic acid sequence identity to the nucleic acid sequence B", the nucleic acid sequence A is the comparison nucleic acid molecule of interest and the nucleic acid sequence B is the nucleic acid sequence of the PRO polypeptide-encoding nucleic acid molecule of interest.

Percent nucleic acid sequence identity may also be determined using the sequence comparison program NCBI-BLAST2 (Altschul et al., Nucleic Acids Res. 25:3389-3402 (1997)). The NCBI-BLAST2 sequence comparison program may be downloaded from http://www.ncbi.nlm.nih.gov or otherwise obtained from the National Institute of Health, Bethesda, MD. NCBI-BLAST2 uses several search parameters, wherein all of those search parameters are set to default values including, for example, unmask = yes, strand = all, expected occurrences = 10, minimum low complexity length = 15/5, multi-pass e-value = 0.01, constant for multi-pass = 25, dropoff for final gapped alignment = 25 and scoring matrix = BLOSUM62.

In situations where NCBI-BLAST2 is employed for sequence comparisons, the % nucleic acid sequence identity of a given nucleic acid sequence C to, with, or against a given nucleic acid sequence D (which can alternatively be phrased as a given nucleic acid sequence C that has or comprises a certain % nucleic acid sequence identity to, with, or against a given nucleic acid sequence D) is calculated as follows:
100 times the fraction W/Z
where W is the number of nucleotides scored as identical matches by the sequence alignment program NCBI-BLAST2 in that program's alignment of C and D, and where Z is the total number of nucleotides in D. It will be appreciated that where the length of nucleic acid sequence C is not equal to the length of nucleic acid sequence D, the % nucleic acid sequence identity of C to D will not equal the % nucleic acid sequence identity of D to C.

In other embodiments, PRO variant polynucleotides are nucleic acid molecules that encode an active PRO polypeptide and which are capable of hybridizing, preferably under stringent hybridization and wash conditions, to nucleotide sequences encoding a full-length PRO polypeptide as disclosed herein. PRO variant polypeptides may be those that are encoded by a PRO variant polynucleotide.

"Isolated," when used to describe the various polypeptides disclosed herein, means polypeptide that has been identified and separated and/or recovered from a component of its natural environment. Contaminant components of its natural environment are materials that would typically interfere with diagnostic or therapeutic uses for the polypeptide, and may include enzymes, hormones, and other proteinaceous or non-proteinaceous solutes. In preferred embodiments, the polypeptide will be purified (1) to a degree sufficient to obtain at least 15 residues of N-terminal or internal amino acid sequence by use of a spinning cup sequenator, or (2) to homogeneity by SDS-PAGE under non-reducing or reducing conditions using Coomassie blue or, preferably, silver stain. Isolated polypeptide includes polypeptide in *situ* within recombinant cells, since at least one component of the PRO polypeptide natural environment will not be present. Ordinarily, however, isolated polypeptide will be prepared by at least one purification step.

An "isolated" PRO polypeptide-encoding nucleic acid or other polypeptide-encoding nucleic acid is a nucleic acid molecule that is identified and separated from at least one contaminant nucleic acid molecule with which it is ordinarily associated in the natural source of the polypeptide-encoding nucleic acid. An isolated polypeptide-encoding nucleic acid molecule is other than in the form or setting in which it is found in nature. Isolated polypeptide-encoding nucleic acid molecules therefore are distinguished from the specific polypeptide-encoding nucleic acid molecule as it exists in natural cells. However, an isolated polypeptide-encoding nucleic acid molecule includes polypeptide-encoding nucleic acid molecules contained in cells that ordinarily express the polypeptide where, for example, the nucleic acid molecule is in a chromosomal location different from that of natural cells.

The term "control sequences" refers to DNA sequences necessary for the expression of an operably linked coding sequence in a particular host organism. The control sequences that are suitable for prokaryotes, for example, include a promoter, optionally an operator sequence, and a ribosome binding site. Eukaryotic cells are known to utilize promoters, polyadenylation signals, and enhancers.

Nucleic acid is "operably linked" when it is placed into a functional relationship with another nucleic acid sequence. For example, DNA for a presequence or secretory leader is operably linked to DNA for a polypeptide if it is expressed as a preprotein that participates in the secretion of the polypeptide; a promoter or enhancer is operably linked to a coding sequence if it affects the transcription of the sequence; or a ribosome binding site is operably linked to a coding sequence if it is positioned so as to facilitate translation. Generally, "operably linked" means that the DNA sequences being linked are contiguous, and, in the case of a secretory leader, contiguous and in reading phase. However, enhancers do not have to be contiguous. Linking is accomplished by ligation at convenient restriction sites. If such sites do not exist, the synthetic oligonucleotide adaptors or linkers are used in accordance with conventional practice.

The term "antibody" is used in the broadest sense and specifically covers, for example, single anti-PRO monoclonal antibodies (including agonist, antagonist, and neutralizing antibodies), anti-PRO antibody compositions with polyepitopic specificity, single chain anti-PRO antibodies, and fragments of anti-PRO antibodies (see below). The term "monoclonal antibody" as used herein refers to an antibody obtained from a population of substantially homogeneous antibodies, i.e., the individual antibodies comprising the population are identical except for possible naturally-occurring mutations that may be present in minor amounts.

"Stringency" of hybridization reactions is readily determinable by one of ordinary skill in the art, and generally is an empirical calculation dependent upon probe length, washing temperature, and salt concentration. In general, longer probes require higher temperatures for proper annealing, while shorter probes need lower temperatures. Hybridization generally depends on the ability of denatured DNA to reanneal when complementary strands are present in an environment below their melting temperature. The higher the degree of desired homology between the probe and hybridizable sequence, the higher the relative temperature which can be used. As a result, it follows that higher relative temperatures would tend to make the reaction conditions more stringent, while lower temperatures less so. For additional details and explanation of stringency of hybridization reactions, see Ausubel et al., Current Protocols in Molecular Biology, Wiley Interscience Publishers, (1995).

"Stringent conditions" or "high stringency conditions", as defined herein, may be identified by those that:
(1) employ low ionic strength and high temperature for washing, for example 0.015 M sodium chloride/0.0015 M sodium citrate/0.1 % sodium dodecyl sulfate at 50°C; (2) employ during hybridization a denaturing agent, such as formamide, for example, 50% (v/v) formamide with 0.1% bovine serum albumin/0.1% Ficoll/0.1% polyvinylpyrrolidone/50mM sodium phosphate buffer at pH 6.5 with 750 mM sodium chloride, 75 mM sodium citrate at 42°C; or (3) employ 50% formamide, 5 x SSC (0.75 M NaCl, 0.075 M sodium citrate), 50 mM sodium phosphate (pH 6.8), 0.1 % sodium pyrophosphate, 5 x Denhardt's solution, sonicated salmon sperm DNA (50 µg/ml), 0.1% SDS, and 10% dextran sulfate at 42°C, with washes at 42°C in 0.2 x SSC (sodium chloride/sodium citrate) and 50% formamide at 55°C, followed by a high-stringency wash consisting of 0.1 x SSC containing EDTA at 55°C.

"Moderately stringent conditions" may be identified as described by Sambrook et al., Molecular Cloning:
A Laboratory Manual, New York: Cold Spring Harbor Press, 1989, and include the use of washing solution and hybridization conditions (e.g., temperature, ionic strength and % SDS) less stringent that those described above. An example of moderately stringent conditions is overnight incubation at 37°C in a solution comprising: 20% formamide, 5 x SSC (150 mM NaCl, 15 mM trisodium citrate), 50 mM sodium phosphate (pH 7.6), 5 x Denhardt's solution, 10% dextran sulfate, and 20 mg/ml denatured sheared salmon sperm DNA, followed by washing the filters in 1 x SSC at about 37-50°C. The skilled artisan will recognize how to adjust the temperature, ionic strength, etc. as necessary to accommodate factors such as probe length and the like.

The term "epitope tagged" when used herein refers to a chimeric polypeptide comprising a PRO polypeptide fused to a "tag polypeptide". The tag polypeptide has enough residues to provide an epitope against which an antibody can be made, yet is short enough such that it does not interfere with activity of the polypeptide to which it is fused. The tag polypeptide preferably also is fairly unique so that the antibody does not substantially cross-react with other epitopes. Suitable tag polypeptides generally have at least six amino acid residues and usually between about 8 and 50 amino acid residues (preferably, between about 10 and 20 amino acid residues).

As used herein, the term "immunoadhesin" designates antibody-like molecules which combine the binding specificity of a heterologous protein (an "adhesin") with the effector functions of immunoglobulin constant domains. Structurally, the immunoadhesins comprise a fusion of an amino acid sequence with the desired binding specificity which is other than the antigen recognition and binding site of an antibody (i.e., is "heterologous"), and an immunoglobulin constant domain sequence. The adhesin part of an immunoadhesin molecule typically is a contiguous amino acid sequence comprising at least the binding site of a receptor or a ligand. The immunoglobulin constant domain sequence in the immunoadhesin may be obtained from any immunoglobulin, such as IgG-1, IgG-2, IgG-3, or IgG-4 subtypes, IgA (including IgA-1 and IgA-2), IgE, IgD or IgM.

"Active" or "activity" for the purposes herein refers to form(s) of a PRO polypeptide which retain a biological and/or an immunological activity of native or naturally-occurring PRO, wherein "biological" activity refers to a biological function (either inhibitory or stimulatory) caused by a native or naturally-occurring PRO other than the ability to induce the production of an antibody against an antigenic epitope possessed by a native or naturally-occurring PRO and an "immunological" activity refers to the ability to induce the production of an antibody against an antigenic epitope possessed by a native or naturally-occurring PRO.

The term "antagonist" is used in the broadest sense, and includes any molecule that partially or fully blocks, inhibits, or neutralizes a biological activity of a native PRO polypeptide disclosed herein. In a similar manner, the term "agonist" is used in the broadest sense and includes any molecule that mimics a biological activity of a native PRO polypeptide disclosed herein. Suitable agonist or antagonist molecules specifically include agonist or antagonist antibodies or antibody fragments, fragments or amino acid sequence variants of native PRO polypeptides, peptides, antisense oligonucleotides, small organic molecules, etc. Methods for identifying agonists or antagonists of a PRO polypeptide may comprise contacting a PRO polypeptide with a candidate agonist or antagonist molecule and measuring a detectable change in one or more biological activities normally associated with the PRO polypeptide.

"Treatment" refers to both therapeutic treatment and prophylactic or preventative measures, wherein the object is to prevent or slow down (lessen) the targeted pathologic condition or disorder. Those in need of treatment include those already with the disorder as well as those prone to have the disorder or those in whom the disorder is to be prevented.

"Chronic" administration refers to administration of the agent(s) in a continuous mode as opposed to an acute mode, so as to maintain the initial therapeutic effect (activity) for an extended period of time. "Intermittent" administration is treatment that is not consecutively done without interruption, but rather is cyclic in nature.

"Mammal" for purposes of treatment refers to any animal classified as a mammal, including humans, domestic and farm animals, and zoo, sports, or pet animals, such as dogs, cats, cattle, horses, sheep, pigs, goats, rabbits, etc. Preferably, the mammal is human.

Administration "in combination with" one or more further therapeutic agents includes simultaneous (concurrent) and consecutive administration in any order.

"Carriers" as used herein include pharmaceutically acceptable carriers, excipients, or stabilizers which are nontoxic to the cell or manunal being exposed thereto at the dosages and concentrations employed. Often the physiologically acceptable carrier is an aqueous pH buffered solution. Examples of physiologically acceptable carriers include buffers such as phosphate, citrate, and other organic acids; antioxidants including ascorbic acid; low molecular weight (less than about 10 residues) polypeptide; proteins, such as serum albumin, gelatin, or immunoglobulins; hydrophilic polymers such as polyvinylpyrrolidone; amino acids such as glycine, glutamine, asparagine, arginine or lysine; monosaccharides, disaccharides, and other carbohydrates including glucose, mannose, or dextrins; chelating agents such as EDTA; sugar alcohols such as mannitol or sorbitol; salt-forming counterions such as sodium; and/or nonionic surfactants such as TWEEN™, polyethylene glycol (PEG), and PLURONICS™.

"Antibody fragments" comprise a portion of an intact antibody, preferably the antigen binding or variable region of the intact antibody. Examples of antibody fragments include Fab, Fab', F(ab')₂, and Fv fragments; diabodies; linear antibodies (Zapata et al., Protein Eng. 8(10): 1057-1062 [1995]); single-chain antibody molecules; and multispecific antibodies formed from antibody fragments.

Papain digestion of antibodies produces two identical antigen-binding fragments, called "Fab" fragments, each with a single antigen-binding site, and a residual "Fc" fragment, a designation reflecting the ability to crystallize readily. Pepsin treatment yields an F(ab')₂ fragment that has two antigen-combining sites and is still capable of cross-linking antigen.

"Fv" is the minimum antibody fragment which contains a complete antigen-recognition and -binding site. This region consists of a dimer of one heavy- and one light-chain variable domain in tight, non-covalent association. It is in this configuration that the three CDRs of each variable domain interact to define an antigen-binding site on the surface of the V_{H}-V_{L} dimer. Collectively, the six CDRs confer antigen-binding specificity to the antibody. However, even a single variable domain (or half of an Fv comprising only three CDRs specific for an antigen) has the ability to recognize and bind antigen, although at a lower affinity than the entire binding site.

The Fab fragment also contains the constant domain of the light chain and the first constant domain (CH1) of the heavy chain. Fab fragments differ from Fab' fragments by the addition of a few residues at the carboxy terminus of the heavy chain CH1 domain including one or more cysteines from the antibody hinge region. Fab'-SH is the designation herein for Fab' in which the cysteine residue(s) of the constant domains bear a free thiol group. F(ab')₂ antibody fragments originally were produced as pairs of Fab' fragments which have hinge cysteines between them. Other chemical couplings of antibody fragments are also known.

The "light chains" of antibodies (immunoglobulins) from any vertebrate species can be assigned to one of two clearly distinct types, called kappa and lambda, based on the amino acid sequences of their constant domains.

Depending on the amino acid sequence of the constant domain of their heavy chains, immunoglobulins can be assigned to different classes. There are five major classes of immunoglobulins: IgA, IgD, IgE, IgG, and IgM, and several of these may be further divided into subclasses (isotypes), e.g., IgG1, IgG2, IgG3, IgG4, IgA, and IgA2.

"Single-chain Fv" or "sFv" antibody fragments comprise the V_{H} and V_{L} domains of antibody, wherein these domains are present in a single polypeptide chain. Preferably, the Fv polypeptide further comprises a polypeptide linker between the V_{H} and V_{L} domains which enables the sFv to form the desired structure for antigen binding. For a review of sFv, see Pluckthun in The Pharmacology of Monoclonal Antibodies, vol. 113, Rosenburg and Moore eds., Springer-Verlag, New York, pp. 269-315 (1994).

The term "diabodies" refers to small antibody fragments with two antigen-binding sites, which fragments comprise a heavy-chain variable domain (V_{H}) connected to a light-chain variable domain (V_{L}) in the same polypeptide chain (V_{H}-V_{L}). By using a linker that is too short to allow pairing between the two domains on the same chain, the domains are forced to pair with the complementary domains of another chain and create two antigen-binding sites. Diabodies are described more fully in, for example, EP 404,097; WO 93/11161; and Hollinger et al., Proc. Natl. Acad. Sci. USA, 90:6444-6448 (1993).

An "isolated" antibody is one which has been identified and separated and/or recovered from a component of its natural environment. Contaminant components of its natural environment are materials which would interfere with diagnostic or therapeutic uses for the antibody, and may include enzymes, hormones, and other proteinaceous or nonproteinaceous solutes. In preferred embodiments, the antibody will be purified (1) to greater than 95 % by weight of antibody as determined by the Lowry method, and most preferably more than 99 % by weight, (2) to a degree sufficient to obtain at least 15 residues of N-terminal or internal amino acid sequence by use of a spinning cup sequenator, or (3) to homogeneity by SDS-PAGE under reducing or nonreducing conditions using Coomassie blue or, preferably, silver stain. Isolated antibody includes the antibody in situ within recombinant cells since at least one component of the antibody's natural environment will not be present.

Ordinarily, however, isolated antibody will be prepared by at least one purification step.

An antibody that "specifically binds to" or is "specific for" a particular polypeptide or an epitope on a particular polypeptide is one that binds to that particular polypeptide or epitope on a particular polypeptide without substantially binding to any other polypeptide or polypeptide epitope.

The word "label" when used herein refers to a detectable compound or composition which is conjugated directly or indirectly to the antibody so as to generate a "labeled" antibody. The label may be detectable by itself (e.g. radioisotope labels or fluorescent labels) or, in the case of an enzymatic label, may catalyze chemical alteration of a substrate compound or composition which is detectable.

By "solid phase" is meant a non-aqueous matrix to which the antibody of the present invention can adhere. Examples of solid phases encompassed herein include those formed partially or entirely of glass (e.g., controlled pore glass), polysaccharides (e.g., agarose), polyacrylamides, polystyrene, polyvinyl alcohol and silicones. In certain embodiments, depending on the context, the solid phase can comprise the well of an assay plate; in others it is a purification column (e.g., an affinity chromatography column). This term also includes a discontinuous solid phase of discrete particles, such as those described in U.S. Patent No. 4,275,149.

A "liposome" is a small vesicle composed of various types of lipids, phospholipids and/or surfactant which is useful for delivery of a drug (such as a PRO polypeptide or antibody thereto) to a mammal. The components of the liposome are commonly arranged in a bilayer formation, similar to the lipid arrangement of biological membranes.

A "small molecule" is defined herein to have a molecular weight below about 500 Daltons.

An "effective amount" of a polypeptide disclosed herein or an agonist or antagonist thereof is an amount sufficient to carry out a specifically stated purpose. An "effective amount" may be determined empirically and in a routine manner, in relation to the stated purpose.

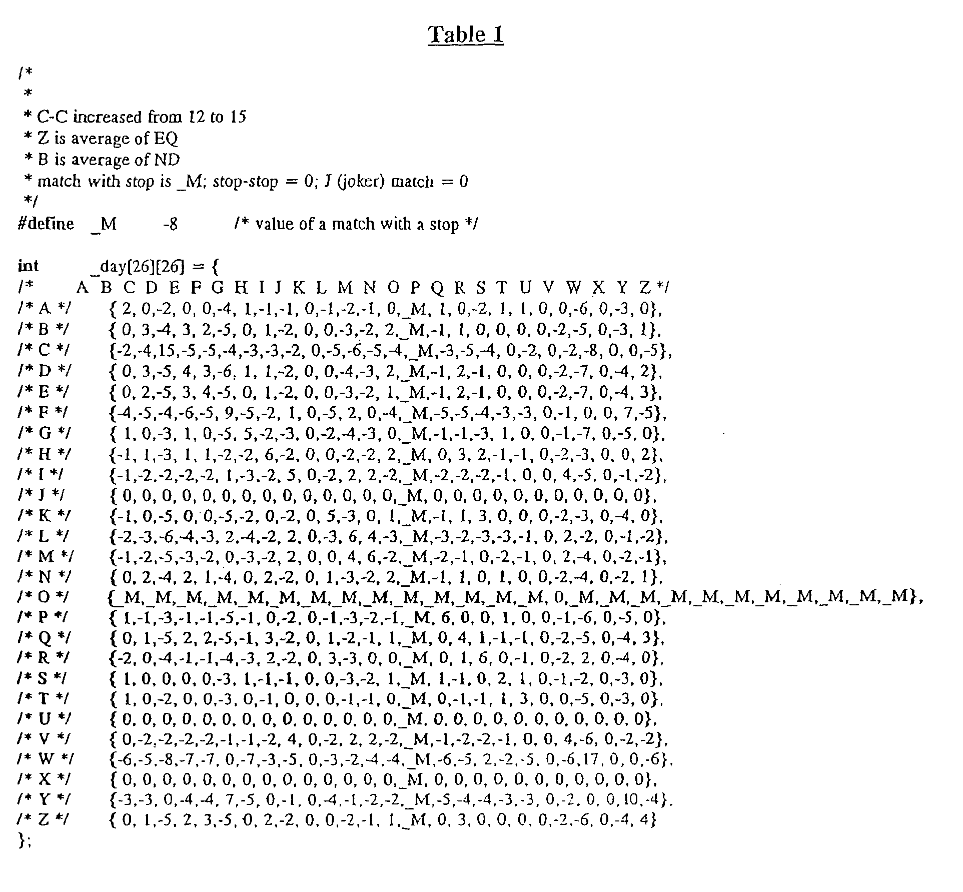

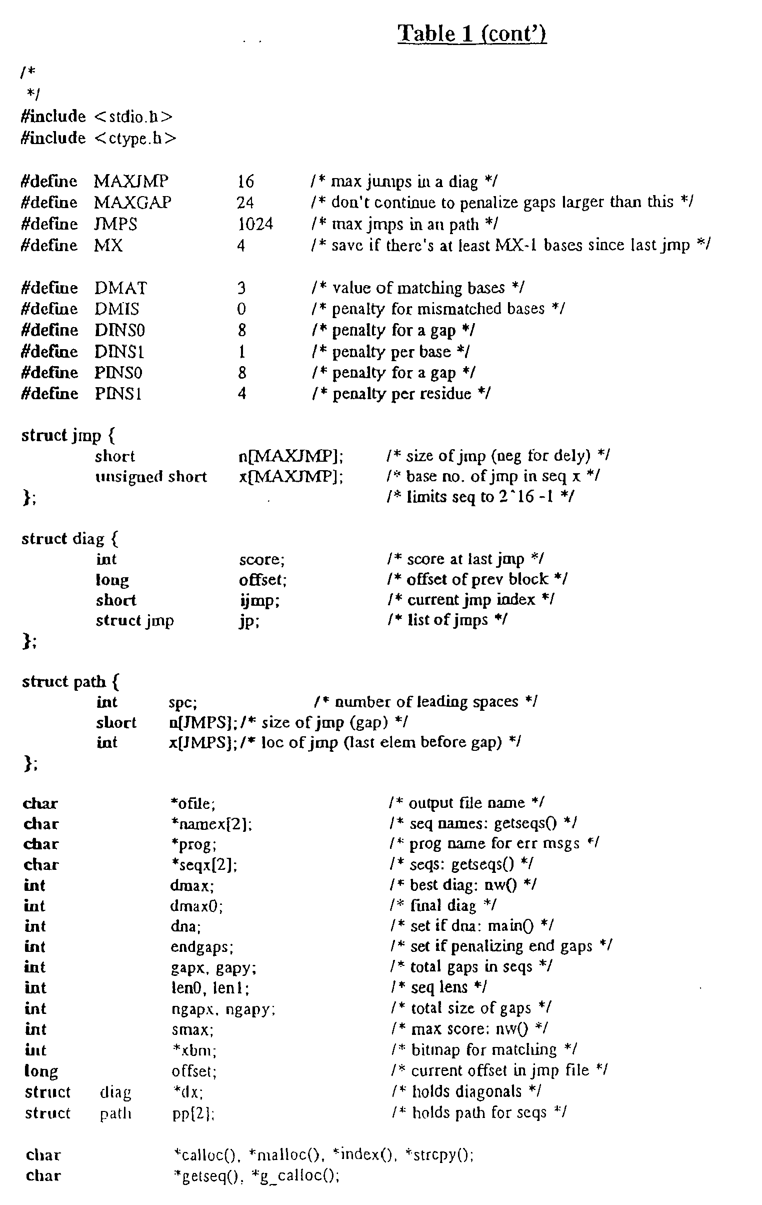

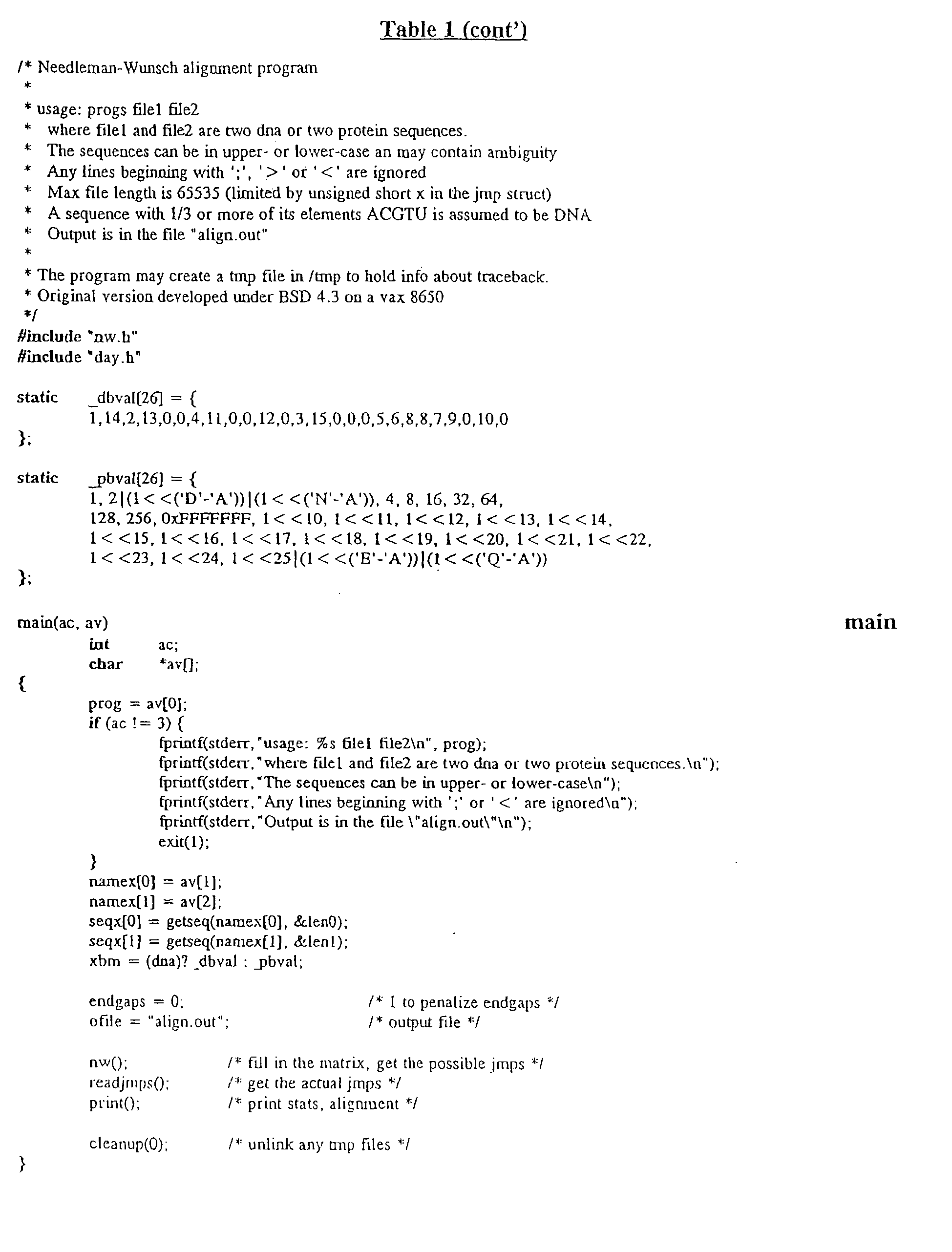

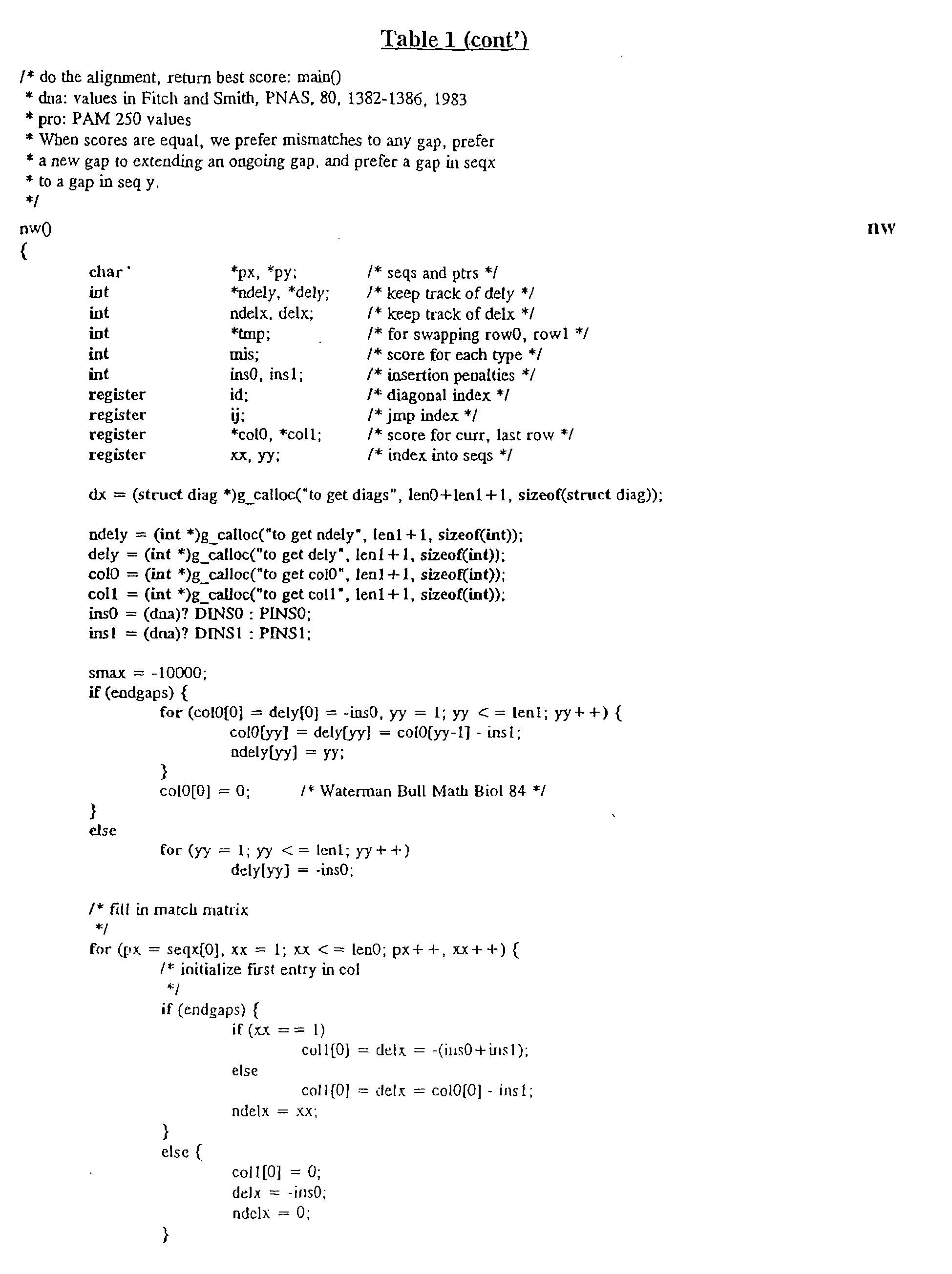

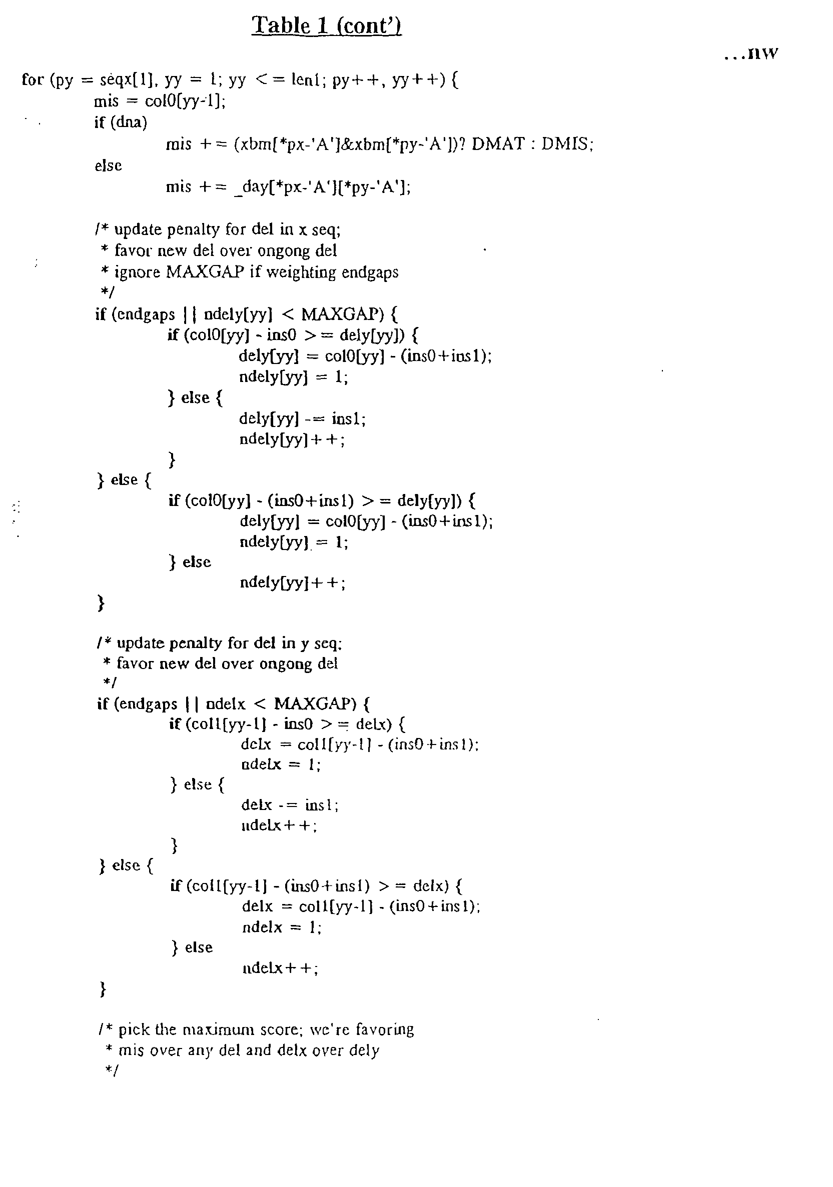

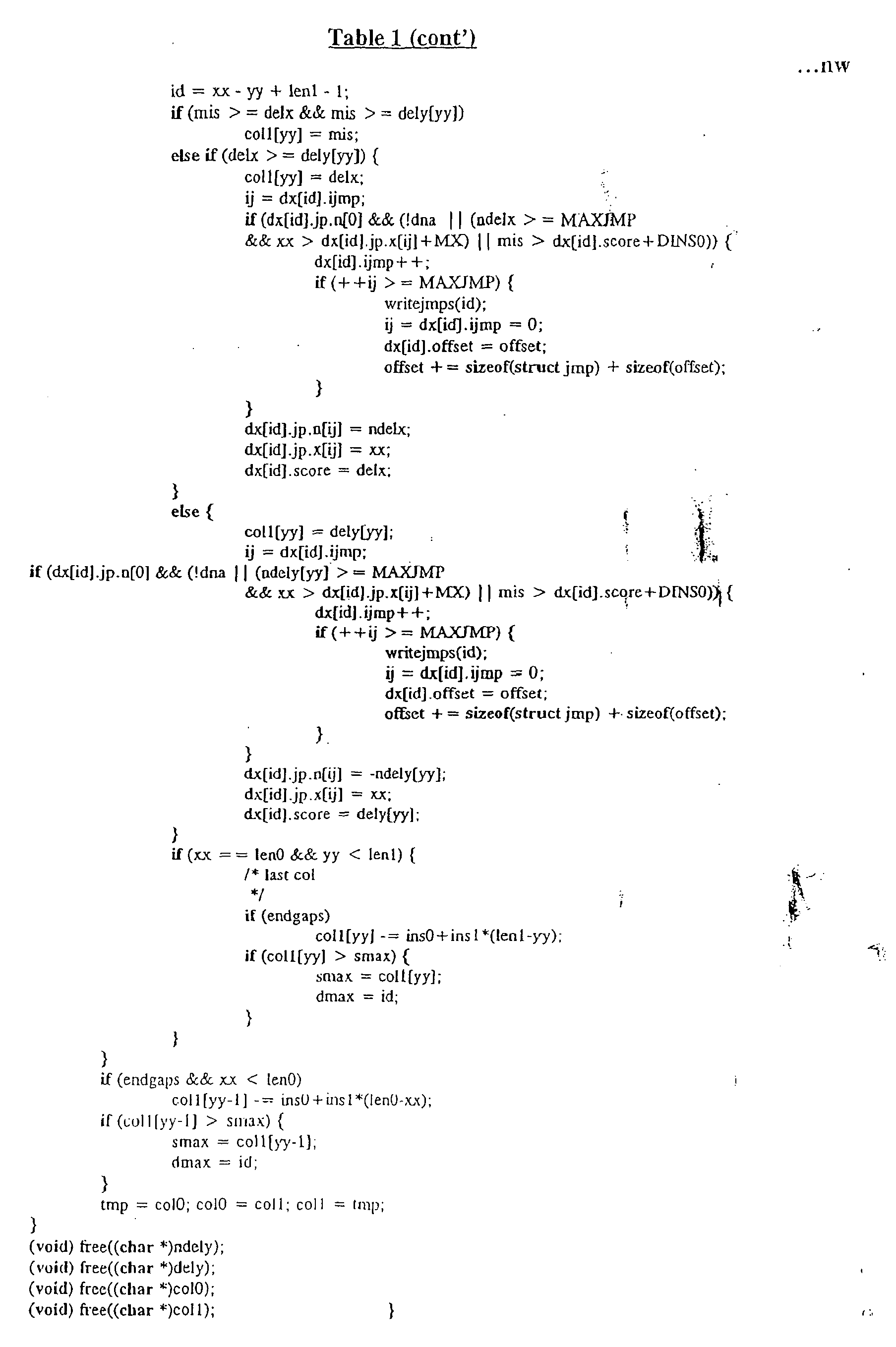

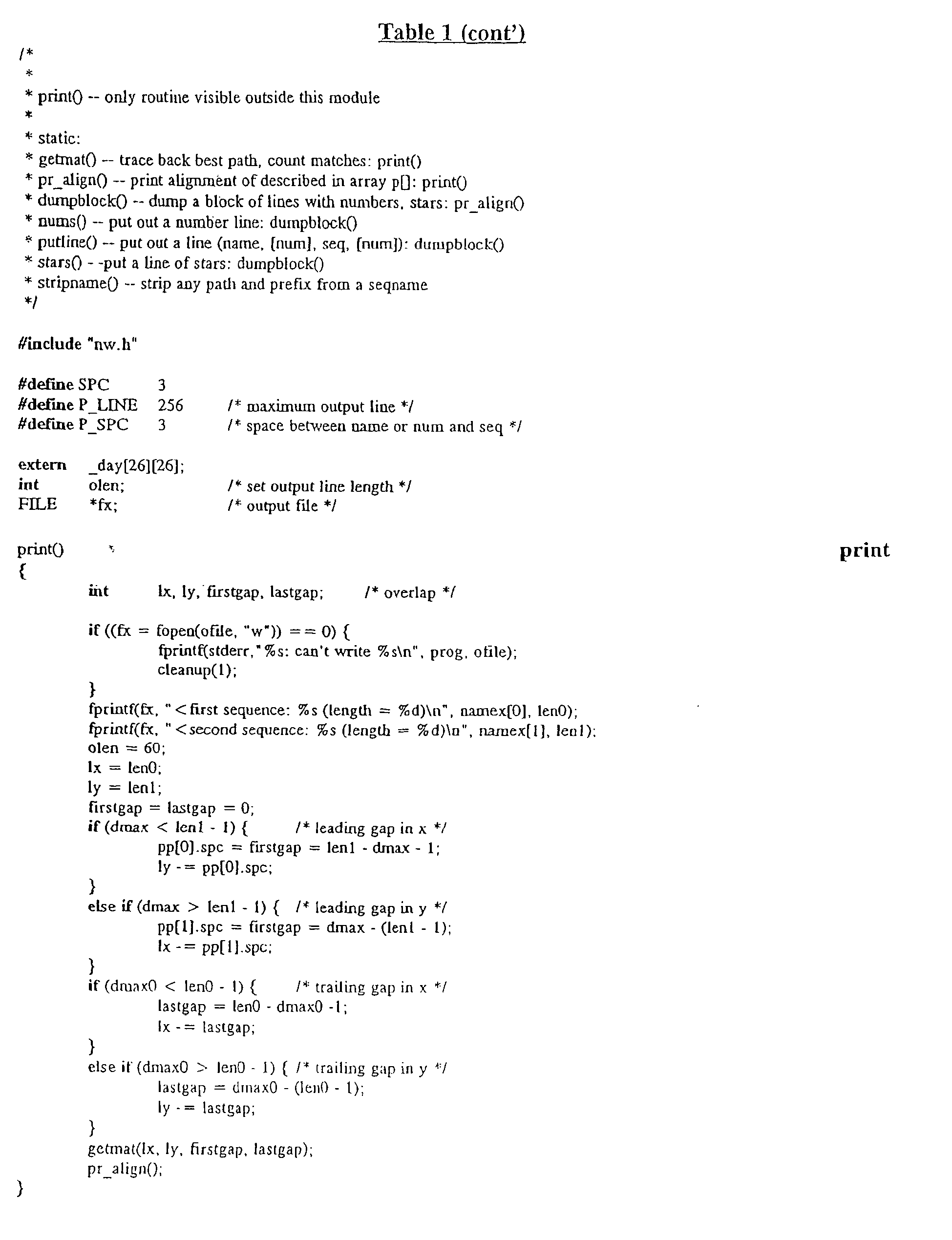

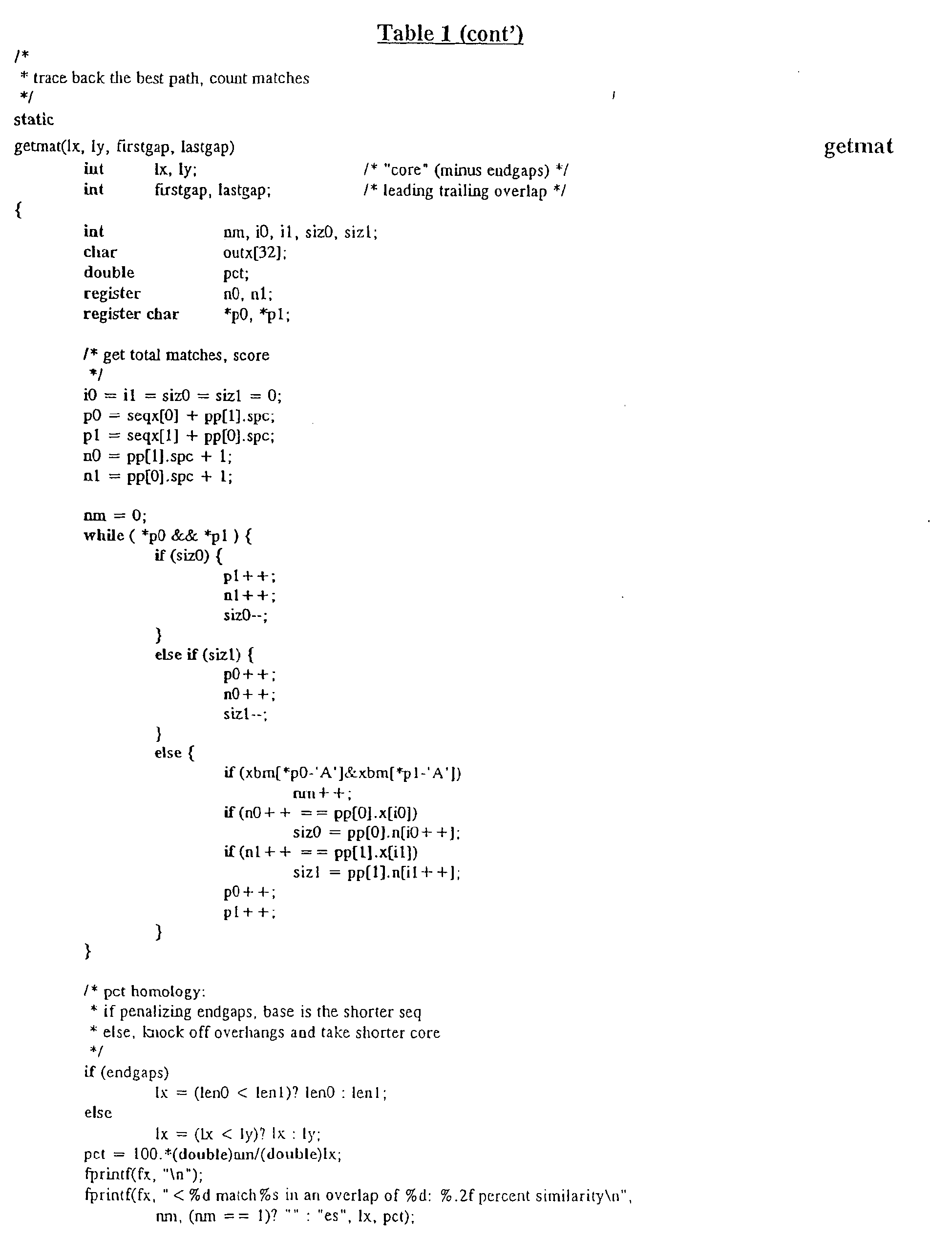

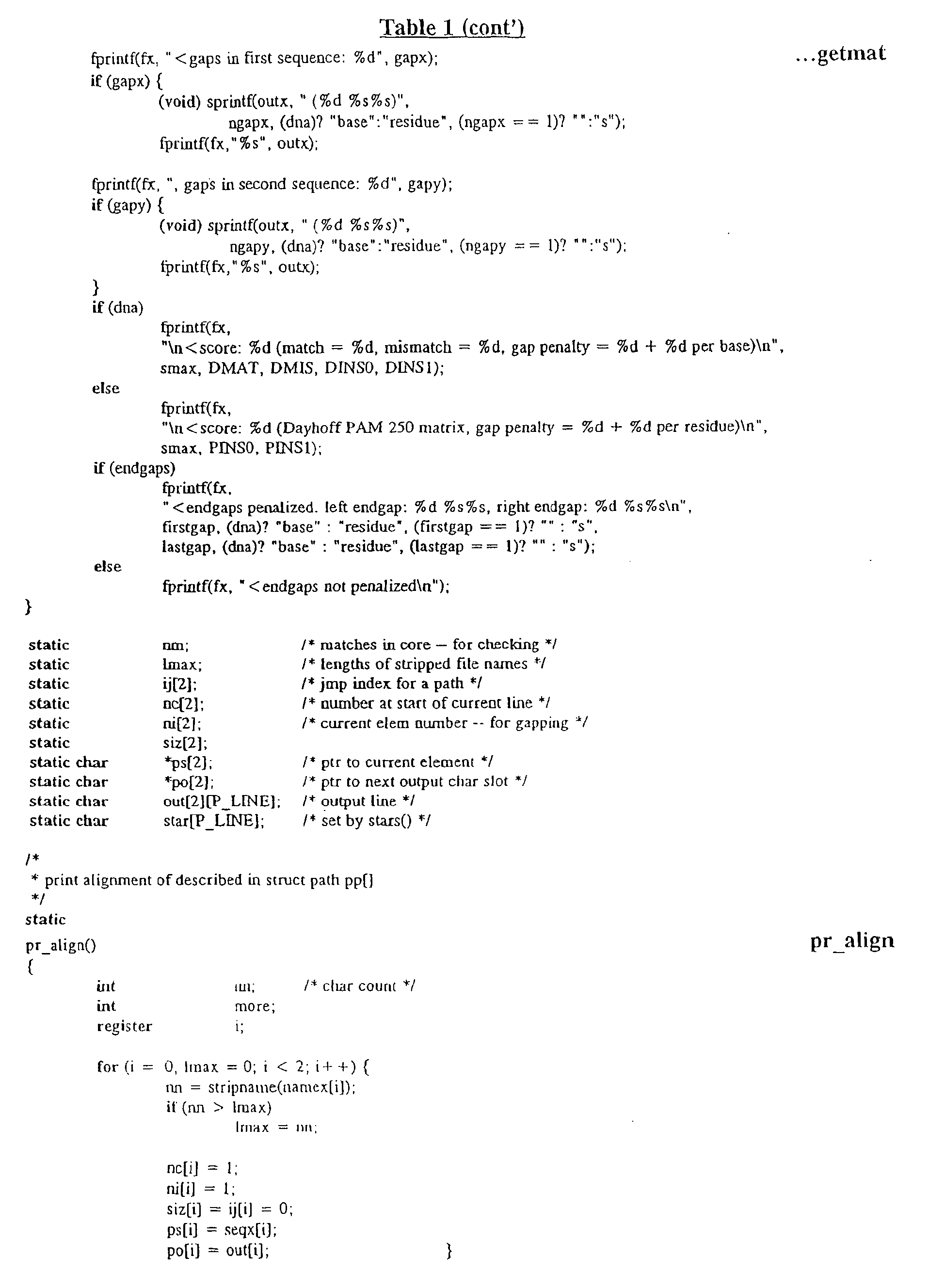

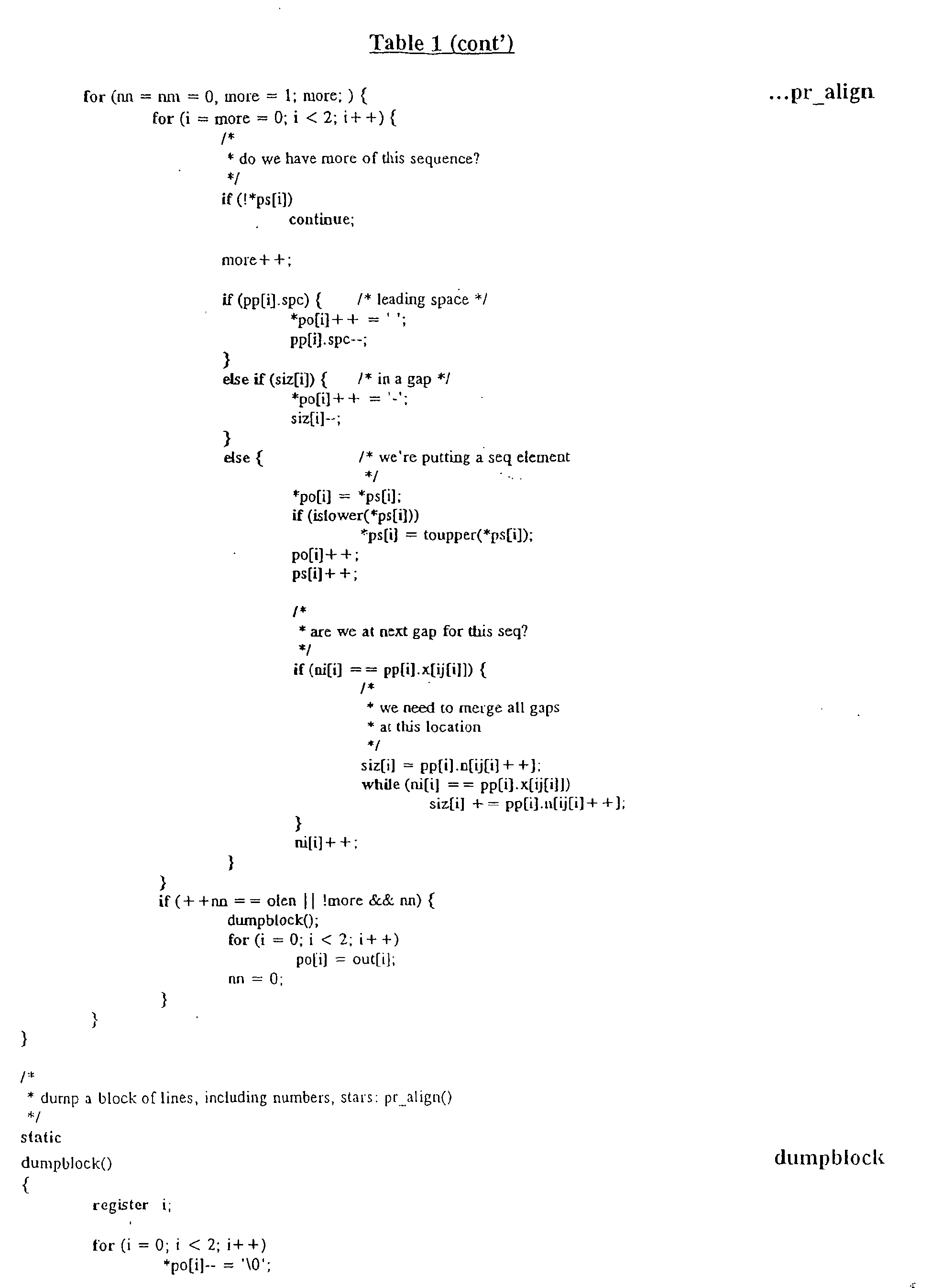

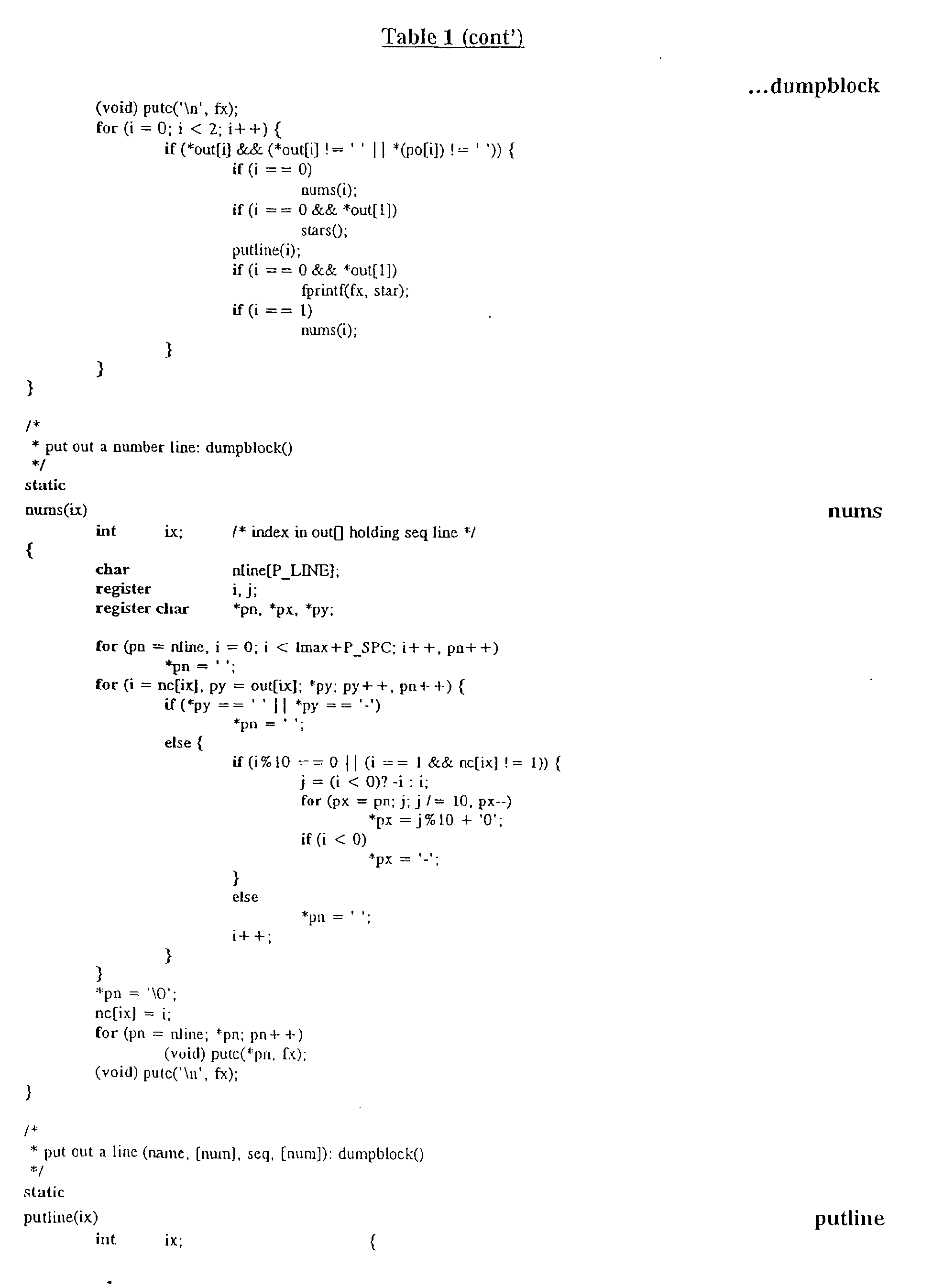

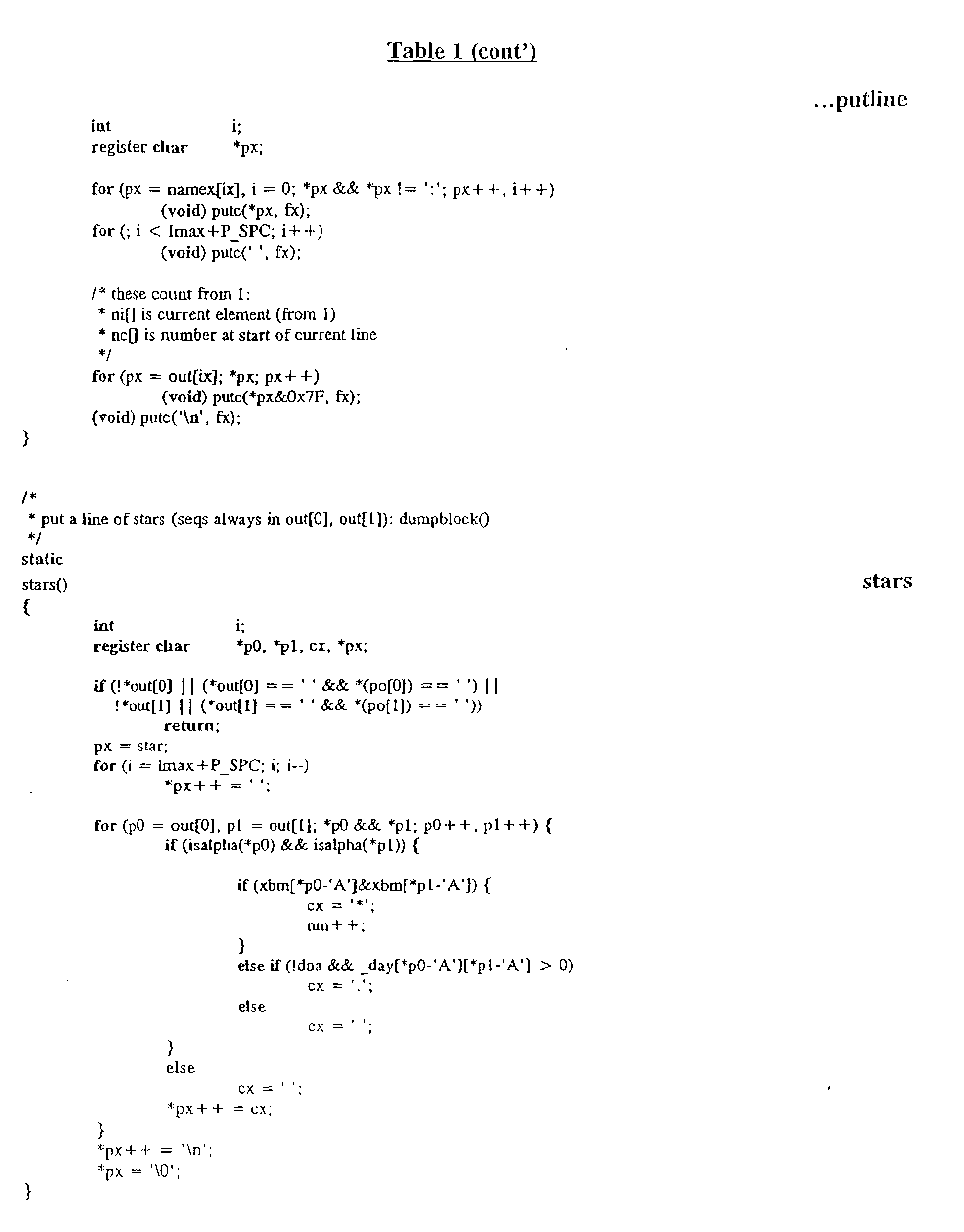

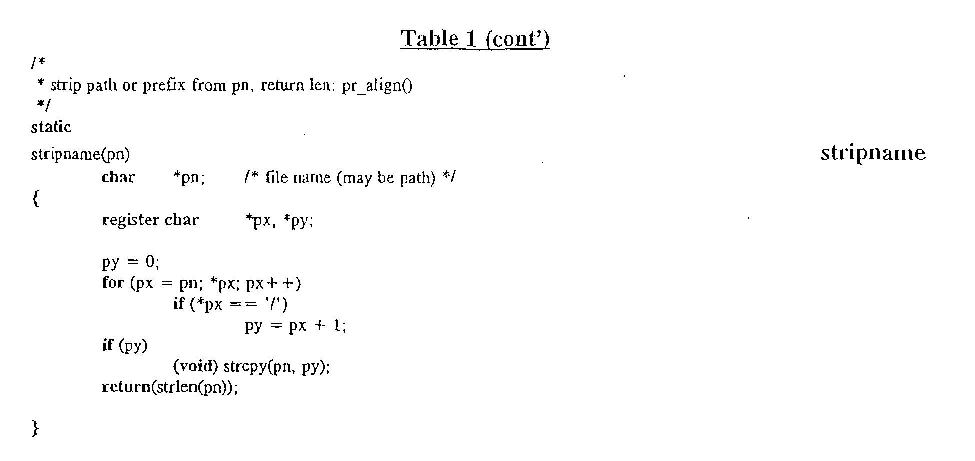

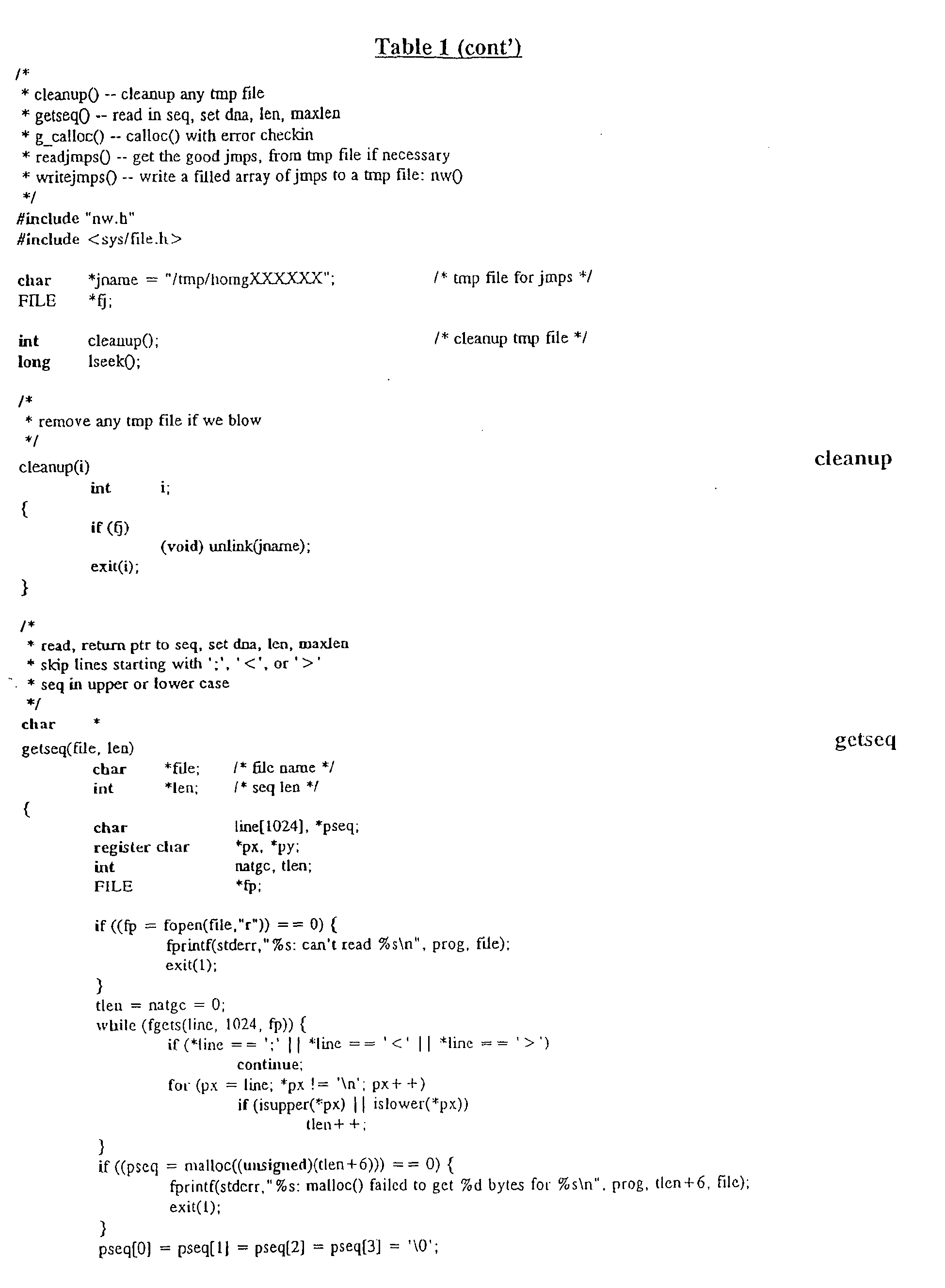

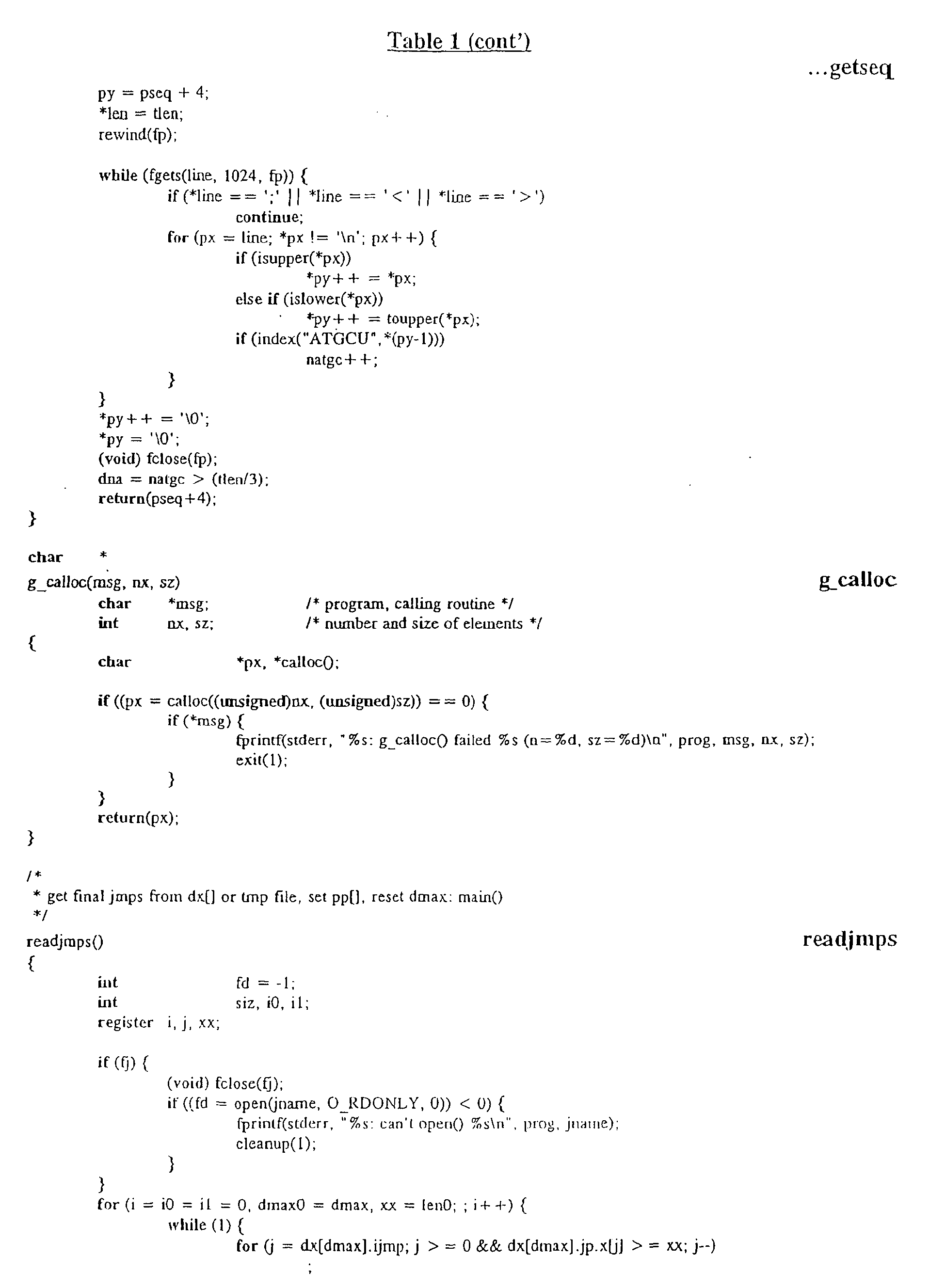

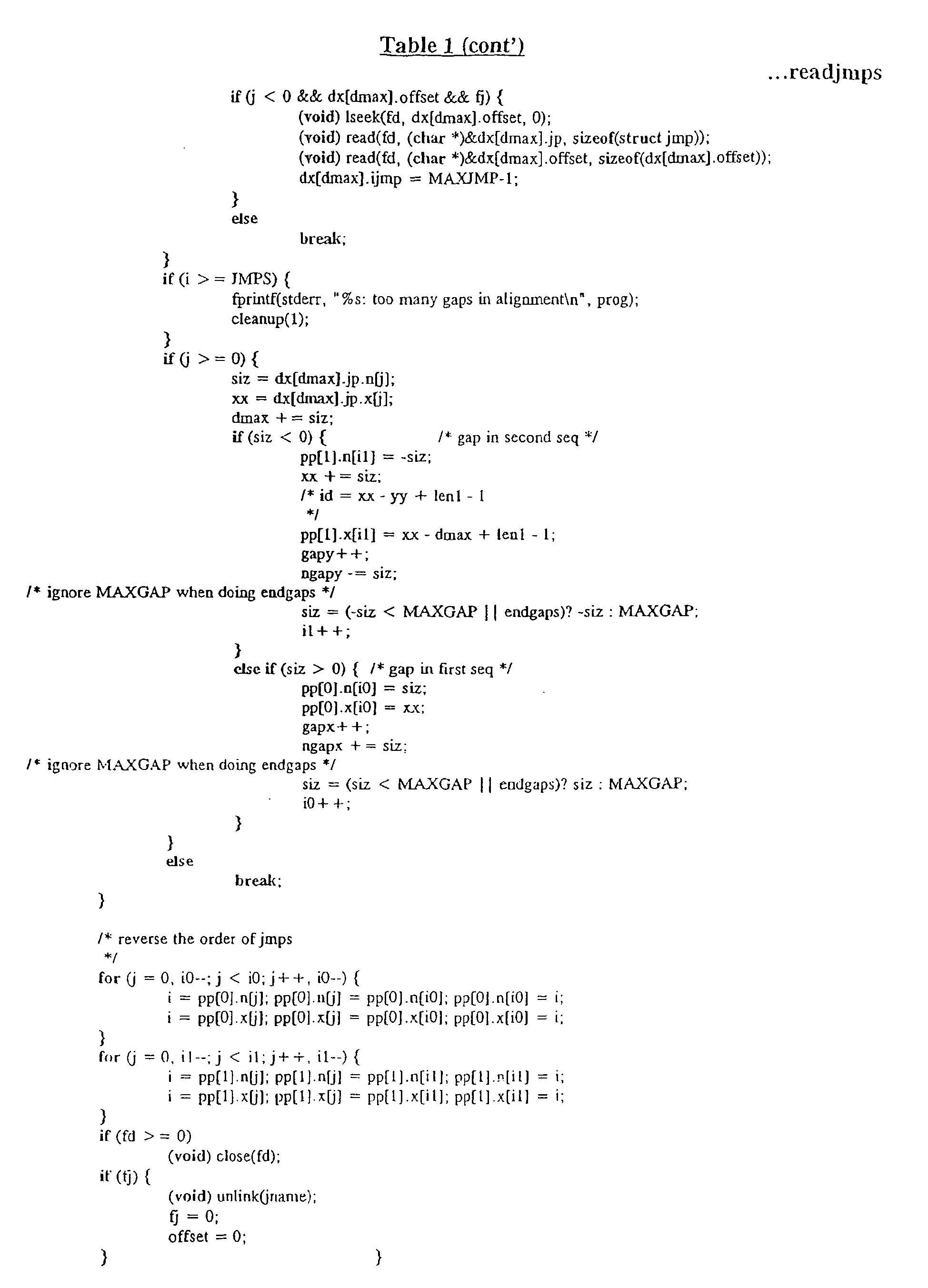

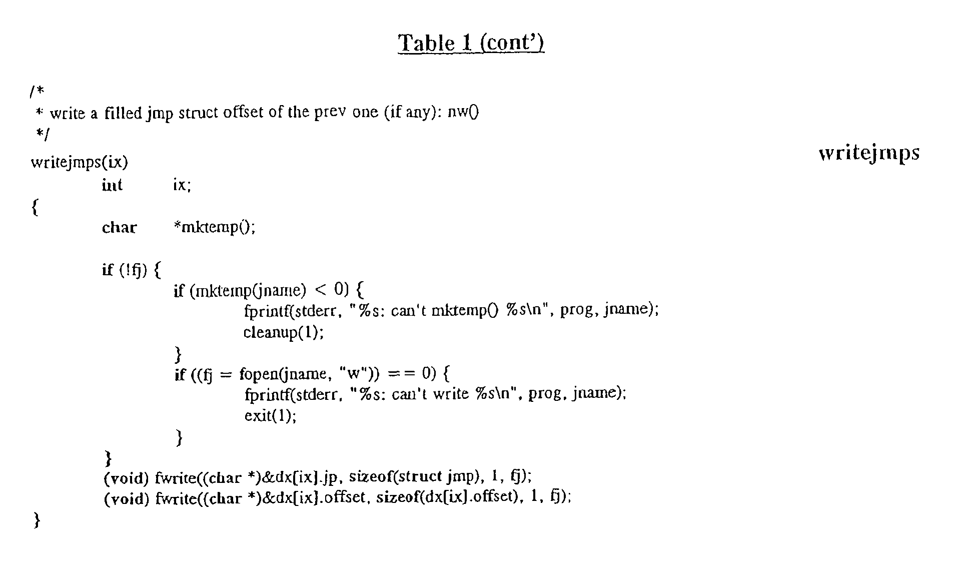

**Table 2**

| | | |
|---|---|---|
| PRO | XXXXXXXXXXXXXXX | (Length = 15 amino acids) |
| Comparison Protein | XXXXXYYYYYYY | (Length = 12 amino acids) |
| % amino acid sequence identity = | | |
| (the number of identically matching amino acid residues between the two polypeptide sequences as determined by ALIGN-2) divided by (the total number of amino acid residues of the PRO polypeptide) = | | |
| 5 divided by 15 = 33.3% | | |

**Table 3**

| | | |
|---|---|---|
| PRO | XXXXXXXXXX | (Length = 10 amino acids) |
| Comparison Protein | XXXXXYYYYYYZZYZ | (Length = 15 amino acids) |
| % amino acid sequence identity = | | |
| (the number of identically matching amino acid residues between the two polypeptide sequences as determined by ALIGN-2) divided by (the total number of amino acid residues of the PRO polypeptide) = | | |
| 5 divided by 10 = 50% | | |

**Table 4**

| | | |
|---|---|---|
| PRO-DNA | NNNNNNNNNNNNNN | (Length = 14 nucleotides) |
| Comparison DNA | NNNNNNLLLLLLLLLL | (Length = 16 nucleotides) |
| % nucleic acid sequence identity = | | |
| (the number of identically matching nucleotides between the two nucleic acid sequences as determined by ALIGN-2) divided by (the total number of nucleotides of the PRO-DNA nucleic acid sequence) = | | |
| 6 divided by 14 = 42.9% | | |

**Table 5**

| | | |
|---|---|---|
| PRO-DNA | NNNNNNNNNNNN | (Length = 12 nucleotides) |
| Comparison DNA | NNNNLLLVV | (Length = 9 nucleotides) |
| % nucleic acid sequence identity = | | |
| (the number of identically matching nucleotides between the two nucleic acid sequences as determined by ALIGN-2) divided by (the total number of nucleotides of the PRO-DNA nucleic acid sequence) = | | |
| 4 divided by 12 = 33.3 % | | |

### II. Compositions and Methods of the Invention

### A. Full-Length PRO Polypeptides

The present invention provides newly identified and isolated nucleotide sequences encoding polypeptides referred to in the present application as PRO polypeptides. In particular, cDNAs encoding various PRO polypeptides have been identified and isolated, as disclosed in further detail in the Examples below. It is noted that proteins produced in separate expression rounds may be given different PRO numbers but the UNQ number is unique for any given DNA and the encoded protein, and will not be changed. However, for sake of simplicity, in the present specification the protein encoded by the full length native nucleic acid molecules disclosed herein as well as all further native homologues and variants included in the foregoing definition of PRO, will be referred to as "PRO/number", regardless of their origin or mode of preparation.

As disclosed in the Examples below, various cDNA clones have been deposited with the ATCC. The actual nucleotide sequences of those clones can readily be determined by the skilled artisan by sequencing of the deposited clone using routine methods in the art. The predicted amino acid sequence can be determined from the nucleotide sequence using routine skill. For the PRO polypeptides and encoding nucleic acids described herein, Applicants have identified what is believed to be the reading frame best identifiable with the sequence information available at the time.

### B. PRO Polypeptide Variants

In addition to the full-length native sequence PRO polypeptides described herein, it is contemplated that PRO variants can be prepared. PRO variants can be prepared by introducing appropriate nucleotide changes into the PRO DNA, and/or by synthesis of the desired PRO polypeptide. Those skilled in the art will appreciate that amino acid changes may alter post-translational processes of the PRO, such as changing the number or position of glycosylation sites or altering the membrane anchoring characteristics.

Variations in the native full-length sequence PRO or in various domains of the PRO described herein, can be made, for example, using any of the techniques and guidelines for conservative and non-conservative mutations set forth, for instance, in U.S. Patent No. 5,364,934. Variations may be a substitution, deletion or insertion of one or more codons encoding the PRO that results in a change in the amino acid sequence of the PRO as compared with the native sequence PRO. Optionally the variation is by substitution of at least one amino acid with any other amino acid in one or more of the domains of the PRO. Guidance in determining which amino acid residue may be inserted, substituted or deleted without adversely affecting the desired activity may be found by comparing the sequence of the PRO with that of homologous known protein molecules and minimizing the number of amino acid sequence changes made in regions of high homology. Amino acid substitutions can be the result of replacing one amino acid with another amino acid having similar structural and/or chemical properties, such as the replacement of a leucine with a serine, i.e., conservative amino acid replacements. Insertions or deletions may optionally be in the range of about 1 to 5 amino acids. The variation allowed may be determined by systematically making insertions, deletions or substitutions of amino acids in the sequence and testing the resulting variants for activity exhibited by the full-length or mature native sequence.

PRO polypeptide fragments are provided herein. Such fragments may be truncated at the N-terminus or C-terminus, or may lack internal residues, for example, when compared with a full length native protein. Certain fragments lack amino acid residues that are not essential for a desired biological activity of the PRO polypeptide.

PRO fragments may be prepared by any of a number of conventional techniques. Desired peptide fragments may be chemically synthesized. An alternative approach involves generating PRO fragments by enzymatic digestion, e.g., by treating the protein with an enzyme known to cleave proteins at sites defined by particular amino acid residues, or by digesting the DNA with suitable restriction enzymes and isolating the desired fragment. Yet another suitable technique involves isolating and amplifying a DNA fragment encoding a desired polypeptide fragment, by polymerase chain reaction (PCR). Oligonucleotides that define the desired termini of the DNA fragment are employed at the 5' and 3' primers in the PCR. Preferably, PRO polypeptide fragments share at least one biological and/or immunological activity with the native PRO polypeptide disclosed herein.

In particular embodiments, conservative substitutions of interest are shown in Table 6 under the heading of preferred substitutions. If such substitutions result in a change in biological activity, then more substantial changes, denominated exemplary substitutions in Table 6, or as further described below in reference to amino acid classes, are introduced and the products screened.

**Table 6**

| Original Residue | Exemplary Substitutions | Preferred Substitutions |
|---|---|---|
| Ala (A) | val; leu; ile | val |
| Arg (R) | lys; gln; asn | lys |
| Asn (N) | gln; his; lys; arg | gln |
| Asp (D) | glu | glu |
| Cys (C) | ser | ser |
| Gln (Q) | asn | asn |
| Glu (E) | asp | asp |
| Gly (G) | pro; ala | ala |
| His (H) | asn; gin; lys; arg | arg |
| Ile (I) | leu; val; met; ala; phe; norleucine | leu |
| Leu (L) | norleucine; ile; val; met; ala; phe | ile |
| Lys (K) | arg; gln; asn | arg |
| Met (M) | leu; phe; ile | leu |
| Phe (F) | leu; val; ile; ala; tyr | leu |
| Pro (P) | ala | ala |
| Ser (S) | thr | thr |
| Thr (T) | ser | ser |
| Trp (W) | tyr; phe | tyr |
| Tyr (Y) | trp; phe; thr; ser | phe |
| Val (V) | ile; leu; met; phe; ala; norleucine | leu |

Substantial modifications in function or immunological identity of the PRO polypeptide are accomplished by selecting substitutions that differ significantly in their effect on maintaining (a) the structure of the polypeptide backbone in the area of the substitution, for example, as a sheet or helical conformation, (b) the charge or hydrophobicity of the molecule at the target site, or (c) the bulk of the side chain. Naturally occurring residues are divided into groups based on common side-chain properties:
(1) hydrophobic: norleucine, met, ala, val, leu, ile;
(2) neutral hydrophilic: cys, ser, thr;
(3) acidic: asp, glu;
(4) basic: asn, gln, his, lys, arg;
(5) residues that influence chain orientation: gly, pro; and
(6) aromatic: trp, tyr, phe.

Non-conservative substitutions will entail exchanging a member of one of these classes for another class. Such substituted residues also may be introduced into the conservative substitution sites or, more preferably, into the remaining (non-conserved) sites.

The variations can be made using methods known in the art such as oligonucleotide-mediated (site-directed) mutagenesis, alanine scanning, and PCR mutagenesis. Site-directed mutagenesis [Carter et al., Nucl. Acids Res., 13:4331 (1986); Zoller et al., Nucl. Acids Res., 10:6487 (1987)], cassette mutagenesis [Wells et al., Gene, 34:315 (1985)], restriction selection mutagenesis [Wells et al., Philos. Trans. R. Soc. London SerA, 317:415 (1986)] or other known techniques can be performed on the cloned DNA to produce the PRO variant DNA.

Scanning amino acid analysis can also be employed to identify one or more amino acids along a contiguous sequence. Among the preferred scanning amino acids are relatively small, neutral amino acids. Such amino acids include alanine, glycine, serine, and cysteine. Alanine is typically a preferred scanning amino acid among this group because it eliminates the side-chain beyond the beta-carbon and is less likely to alter the mainchain conformation of the variant [Cunningham and Wells, Science, 244: 1081-1085 (1989)]. Alanine is also typically preferred because it is the most common amino acid. Further, it is frequently found in both buried and exposed positions [Creighton, The Proteins, (W.H. Freeman & Co., N.Y.); Chothia, J. Mol. Biol., 150:1 (1976)]. If alanine substitution does not yield adequate amounts of variant, an isoteric amino acid can be used.

### C. Modifications of PRO

Covalent modifications of PRO are included within the scope of this invention. One type of covalent modification includes reacting targeted amino acid residues of a PRO polypeptide with an organic derivatizing agent that is capable of reacting with selected side chains or the N- or C- terminal residues of the PRO. Derivatization with bifunctional agents is useful, for instance, for crosslinking PRO to a water-insoluble support matrix or surface for use in the method for purifying anti-PRO antibodies, and vice-versa. Commonly used crosslinking agents include, e.g., 1,1-bis(diazoacetyl)-2-phenylethane, glutaraldehyde, N-hydroxysuccinimide esters, for example, esters with 4-azidosalicylic acid, homobifunctional imidoesters, including disuccinimidyl esters such as 3,3'-dithiobis(succinimidylpropionate), bifunctional maleimides such as bis-N-maleimido-1,8-octane and agents such as methyl-3-[(p-azidophenyl)dithiol]propioimidate.

Other modifications include deamidation of glutaminyl and asparaginyl residues to the corresponding glutamyl and aspartyl residues, respectively, hydroxylation of proline and lysine, phosphorylation of hydroxyl groups of seryl or threonyl residues, methylation of the α-amino groups of lysine, arginine, and histidine side chains [T.E. Creighton, Proteins: Structure and Molecular Properties W.H. Freeman & Co., San Francisco, pp. 79-86 (1983)], acetylation of the N-terminal amine, and amidation of any C-terminal carboxyl group.

Another type of covalent modification of the PRO polypeptide included within the scope of this invention comprises altering the native glycosylation pattern of the polypeptide. "Altering the native glycosylation pattern" is intended for purposes herein to mean deleting one or more carbohydrate moieties found in native sequence PRO (either by removing the underlying glycosylation site or by deleting the glycosylation by chemical and/or enzymatic means), and/or adding one or more glycosylation sites that are not present in the native sequence PRO. In addition, the phrase includes qualitative changes in the glycosylation of the native proteins, involving a change in the nature and proportions of the various carbohydrate moieties present.

Addition of glycosylation sites to the PRO polypeptide may be accomplished by altering the amino acid sequence. The alteration may be made, for example, by the addition of, or substitution by, one or more serine or threonine residues to the native sequence PRO (for O-linked glycosylation sites). The PRO amino acid sequence may optionally be altered through changes at the DNA level, particularly by mutating the DNA encoding the PRO polypeptide at preselected bases such that codons are generated that will translate into the desired amino acids.

Another means of increasing the number of carbohydrate moieties on the PRO polypeptide is by chemical or enzymatic coupling of glycosides to the polypeptide. Such methods are described in the art, e.g., in WO 87/05330 published 11 September 1987, and in Aplin and Wriston, CRC Crit. Rev. Biochem., pp. 259-306 (1981).

Removal of carbohydrate moieties present on the PRO polypeptide may be accomplished chemically or enzymatically or by mutational substitution of codons encoding for amino acid residues that serve as targets for glycosylation. Chemical deglycosylation techniques are known in the art and described, for instance, by Hakimuddin, et al., Arch. Biochem. Biophys., 259:52 (1987) and by Edge et al., Anal. Biochem., 118:131 (1981). Enzymatic cleavage of carbohydrate moieties on polypeptides can be achieved by the use of a variety of endo- and exo-glycosidases as described by Thotakura et al., Meth. Enzymol., 138:350 (1987).

Another type of covalent modification of PRO comprises linking the PRO polypeptide to one of a variety of nonproteinaceous polymers, e.g., polyethylene glycol (PEG), polypropylene glycol, or polyoxyalkylenes, in the manner set forth in U.S. Patent Nos. 4,640,835; 4,496,689; 4,301,144; 4,670,417; 4,791,192 or 4,179,337.

The PRO of the present invention may also be modified in a way to form a chimeric molecule comprising PRO fused to another, heterologous polypeptide or amino acid sequence.

In one embodiment, such a chimeric molecule comprises a fusion of the PRO with a tag polypeptide which provides an epitope to which an anti-tag antibody can selectively bind. The epitope tag is generally placed at the amino- or carboxyl- terminus of the PRO. The presence of such epitope-tagged forms of the PRO can be detected using an antibody against the tag polypeptide. Also, provision of the epitope tag enables the PRO to be readily purified by affinity purification using an anti-tag antibody or another type of affinity matrix that binds to the epitope tag. Various tag polypeptides and their respective antibodies are well known in the art. Examples include poly-histidine (poly-his) or poly-histidine-glycine (poly-his-gly) tags; the flu HA tag polypeptide and its antibody 12CA5 [Field et al., Mol. Cell. Biol., 8:2159-2165 (1988)]; the c-myc tag and the 8F9, 3C7, 6E10, G4, B7 and 9E10 antibodies thereto [Evan et al., Molecular and Cellular Biology, 5:3610-3616 (1985)]; and the Herpes Simplex virus glycoprotein D (gD) tag and its antibody [Paborsky et al., Protein Engineering, 3(6):547-553 (1990)]. Other tag polypeptides include the Flag-peptide [Hopp et al., BioTechnology, 6:1204-1210 (1988)]; the KT3 epitope peptide [Martin et al., Science, 255:192-194 (1992)]; an α-tubulin epitope peptide [Skinner et al., J. Biol. Chem., 266:15163-15166 (1991)]; and the T7 gene 10 protein peptide tag [Lutz-Freyermuth et al., Proc. Natl. Acad. Sci. USA, 87:6393-6397 (1990)].

In an alternative embodiment, the chimeric molecule may comprise a fusion of the PRO with an immunoglobulin or a particular region of an immunoglobulin. For a bivalent form of the chimeric molecule (also referred to as an "immunoadhesin"), such a fusion could be to the Fc region of an IgG molecule. The Ig fusions preferably include the substitution of a soluble (transmembrane domain deleted or inactivated) form of a PRO polypeptide in place of at least one variable region within an Ig molecule. In a particularly preferred embodiment, the immunoglobulin fusion includes the hinge, CH2 and CH3, or the hinge, CH1, CH2 and CH3 regions of an IgG1 molecule. For the production of immunoglobulin fusions see also US Patent No. 5,428,130 issued June 27, 1995.

### D. Preparation of PRO

The description below relates primarily to production of PRO by culturing cells transformed or transfected with a vector containing PRO nucleic acid. It is, of course, contemplated that alternative methods, which are well known in the art, may be employed to prepare PRO. For instance, the PRO sequence, or portions thereof, may be produced by direct peptide synthesis using solid-phase techniques [see, e.g., Stewart et al., Solid-Phase Peptide Synthesis, W.H. Freeman Co., San Francisco, CA (1969); Merrifield, J. Am. Chem. Soc., 85:2149-2154 (1963)]. *In vitro* protein synthesis may be performed using manual techniques or by automation. Automated synthesis may be accomplished, for instance, using an Applied Biosystems Peptide Synthesizer (Foster City, CA) using manufacturer's instructions. Various portions of the PRO may be chemically synthesized separately and combined using chemical or enzymatic methods to produce the full-length PRO.

### 1. Isolation of DNA Encoding PRO

DNA encoding PRO may be obtained from a cDNA library prepared from tissue believed to possess the PRO mRNA and to express it at a detectable level. Accordingly, human PRO DNA can be conveniently obtained from a cDNA library prepared from human tissue, such as described in the Examples. The PRO-encoding gene may also be obtained from a genomic library or by known synthetic procedures (e.g., automated nucleic acid synthesis).

Libraries can be screened with probes (such as antibodies to the PRO or oligonucleotides of at least about 20-80 bases) designed to identify the gene of interest or the protein encoded by it. Screening the cDNA or genomic library with the selected probe may be conducted using standard procedures, such as described in Sambrook et al., Molecular Cloning: A Laboratory Manual (New York: Cold Spring Harbor Laboratory Press, 1989). An alternative means to isolate the gene encoding PRO is to use PCR methodology [Sambrook et al., supra; Dieffenbach et al., PCR Primer: A Laboratory Manual (Cold Spring Harbor Laboratory Press, 1995)].

The Examples below describe techniques for screening a cDNA library. The oligonucleotide sequences selected as probes should be of sufficient length and sufficiently unambiguous that false positives are minimized. The oligonucleotide is preferably labeled such that it can be detected upon hybridization to DNA in the library being screened. Methods of labeling are well known in the art, and include the use of radiolabels like ³²P-labeled ATP, biotinylation or enzyme labeling. Hybridization conditions, including moderate stringency and high stringency, are provided in Sambrook et al., supra.

Sequences identified in such library screening methods can be compared and aligned to other known sequences deposited and available in public databases such as GenBank or other private sequence databases. Sequence identity (at either the amino acid or nucleotide level) within defined regions of the molecule or across the full-length sequence can be determined using methods known in the art and as described herein.

Nucleic acid having protein coding sequence may be obtained by screening selected cDNA or genomic libraries using the deduced amino acid sequence disclosed herein for the first time, and, if necessary, using conventional primer extension procedures as described in Sambrook et al., supra, to detect precursors and processing intermediates of mRNA that may not have been reverse-transcribed into cDNA.

### 2. Selection and Transformation of Host Cells

Host cells are transfected or transformed with expression or cloning vectors described herein for PRO production and cultured in conventional nutrient media modified as appropriate for inducing promoters, selecting transformants, or amplifying the genes encoding the desired sequences. The culture conditions, such as media, temperature, pH and the like, can be selected by the skilled artisan without undue experimentation. In general, principles, protocols, and practical techniques for maximizing the productivity of cell cultures can be found in Mammalian Cell Biotechnology: a Practical Approach, M. Butler, ed. (IRL Press, 1991) and Sambrook et al., supra.

Methods of eukaryotic cell transfection and prokaryotic cell transformation are known to the ordinarily skilled artisan, for example, CaCl₂, CAPO₄, liposome-mediated and electroporation. Depending on the host cell used, transformation is performed using standard techniques appropriate to such cells. The calcium treatment employing calcium chloride, as described in Sambrook et al., supra, or electroporation is generally used for prokaryotes. Infection with *Agrobacterium tumefaciens* is used for transformation of certain plant cells, as described by Shaw et al., Gene, 23:315 (1983) and WO 89/05859 published 29 June 1989. For mammalian cells without such cell walls, the calcium phosphate precipitation method of Graham and van der Eb, Virology, 52:456-457 (1978) can be employed. General aspects of mammalian cell host system transfections have been described in U.S. Patent No. 4,399,216. Transformations into yeast are typically carried out according to the method of Van Solingen et al., J. Bact., 130:946 (1977) and Hsiao et al., Proc. Natl. Acad. Sci. (USA), 76:3829 (1979). However, other methods for introducing DNA into cells, such as by nuclear microinjection, electroporation, bacterial protoplast fusion with intact cells, or polycations, e.g., polybrene, polyornithine, may also be used. For various techniques for transforming mammalian cells, see Keown et al., Methods in Enzymology, 185:527-537 (1990) and Mansour et al., Nature, 336:348-352 (1988).

Suitable host cells for cloning or expressing the DNA in the vectors herein include prokaryote, yeast, or higher eukaryote cells. Suitable prokaryotes include but are not limited to eubacteria, such as Gram-negative or Gram-positive organisms, for example, Enterobacteriaceae such as *E. coli.* Various E. *coli* strains are publicly available, such as *E. coli* K12 strain MM294 (ATCC 31,446); *E. coli* X1776 (ATCC 31,537); *E. coli* strain W3110 (ATCC 27,325) and K5 772 (ATCC 53,635). Other suitable prokaryotic host cells include Enterobacteriaceae such as *Escherichia,* e.g., *E. coli, Enterobacter, Erwinia, Klebsiella, Proteus, Salmonella,* e.g., *Salmonella typhimurium*, *Serratia,* e.g., *Serratia marcescans,* and *Shigella,* as well as *Bacilli* such as *B. subtilis* and *B. licheniformis* (e.g., *B. licheniformis* 41P disclosed in DD 266, 710 published 12 April 1989), *Pseudomonas* such as *P. aeruginosa,* and *Streptomyces.* These examples are illustrative rather than limiting. Strain W3110 is one particularly preferred host or parent host because it is a common host strain for recombinant DNA product fermentations. Preferably, the host cell secretes minimal amounts of proteolytic enzymes. For example, strain W3110 may be modified to effect a genetic mutation in the genes encoding proteins endogenous to the host, with examples of such hosts including *E. coli* W3110 strain 1A2, which has the complete genotype *tonA* ; *E. coli* W3110 strain 9E4, which has the complete genotype *tonA ptr3*; *E. coli* W3110 strain 27C7 (ATCC 55,244), which has the complete genotype *tonA ptr3 phoA E15* (*argF-lac*)*169 degP ompT kan*^{*r*}; *E. coli* W3110 strain 37D6, which has the complete genotype *tonA ptr3 phoA E15 (argF-lac)169 degP ompT rbs7 ilvG kan*^{*r*}*; E. coli* W3110 strain 40B4, which is strain 37D6 with a non-kanamycin resistant *degP* deletion mutation; and an *E. coli* strain having mutant periplasmic protease disclosed in U.S. Patent No. 4,946,783 issued 7 August 1990. Alternatively, *in vitro* methods of cloning, e.g., PCR or other nucleic acid polymerase reactions, are suitable.

In addition to prokaryotes, eukaryotic microbes such as filamentous fungi or yeast are suitable cloning or expression hosts for PRO-encoding vectors. *Saccharomyces cerevisiae* is a commonly used lower eukaryotic host microorganism. Others include *Schizosaccharomyces pombe* (Beach and Nurse, Nature, 290: 140 [1981]; EP 139,383 published 2 May 1985); *Kluyveromyces* hosts (U.S. Patent No. 4,943,529; Fleer et al., Bio/Technology, 9:968-975 (1991)) such as, e.g., K. *lactis* (MW98-8C, CBS683, CBS4574; Louvencourt et al., J. Bacteriol., 154(2):737-742 (1983)), *K. fragilis* (ATCC 12,424), *K. bulgaricus* (ATCC 16,045), *K. wickeramii* (ATCC 24,178), *K. waltii* (ATCC 56,500), *K. drosophilarum* (ATCC 36,906; Van den Berg et al., Bio/Technology, 8:135 (1990)), *K. thermotolerans,* and *K. marxianus; yarrowia* (EP 402,226); *Pichia pastoris* (EP 183,070; Sreekrishna et al., J. Basic Microbiol., 28:265-278 [1988]); *Candida; Trichoderma reesia* (EP *244,234); Neurospora crassa* (Case et al., Proc. Natl. Acad. Sci. USA, 76:5259-5263 [1979]); *Schwanniomyces* such as *Schwanniomyces occidentalis* (EP 394,538 published 31 October 1990); and filamentous fungi such as, *e.g., Neurospora, Penicillium, Tolypocladium* (WO 91/00357 published 10 January 1991), and *Aspergillus* hosts such as *A. nidulans* (Ballance et al., Biochem. Biophys. Res. Commun.. 112:284-289 [1983]; Tilburn et al., Gene, 26:205-221 [1983]; Yelton et al., Proc. Natl. Acad. Sci. USA, 81: 1470-1474 [1984]) and *A. niger* (Kelly and Hynes, EMBO *J*., 4:475-479 [1985]). Methylotropic yeasts are suitable herein and include, but are not limited to, yeast capable of growth on methanol selected from the genera consisting of *Hansenula, Candida, Kloeckera, Pichia, Saccharomyces, Torulopsis,* and *Rhodotorula.* A list of specific species that are exemplary of this class of yeasts may be found in C. Anthony, The Biochemistry of Methylotrophs, 269 (1982).

Suitable host cells for the expression of glycosylated PRO are derived from multicellular organisms. Examples of invertebrate cells include insect cells such as Drosophila S2 and Spodoptera Sf9, as well as plant cells. Examples of useful mammalian host cell lines include Chinese hamster ovary (CHO) and COS cells. More specific examples include monkey kidney CV1 line transformed by SV40 (COS-7, ATCC CRL 1651); human embryonic kidney line (293 or 293 cells subcloned for growth in suspension culture, Graham et al., J. Gen Virol., 36:59 (1977)); Chinese hamster ovary cells/-DHFR (CHO, Urlaub and Chasin, Proc. Natl. Acad. Sci. USA, 77:4216 (1980)); mouse sertoli cells (TM4, Mather, Biol. Reprod., 23:243-251 (1980)); human lung cells (W 138, ATCC CCL 75); human liver cells (Hep G2, HB 8065); and mouse mammary tumor (MMT 060562, ATCC CCL51). The selection of the appropriate host cell is deemed to be within the skill in the art.

### 3. Selection and Use of a Replicable Vector

The nucleic acid (e.g., cDNA or genomic DNA) encoding PRO may be inserted into a replicable vector for cloning (amplification of the DNA) or for expression. Various vectors are publicly available. The vector may, for example, be in the form of a plasmid, cosmid, viral particle, or phage. The appropriate nucleic acid sequence may be inserted into the vector by a variety of procedures. In general, DNA is inserted into an appropriate restriction endonuclease site(s) using techniques known in the art. Vector components generally include, but are not limited to, one or more of a signal sequence, an origin of replication, one or more marker genes, an enhancer element, a promoter, and a transcription termination sequence. Construction of suitable vectors containing one or more of these components employs standard ligation techniques which are known to the skilled artisan.

The PRO may be produced recombinantly not only directly, but also as a fusion polypeptide with a heterologous polypeptide, which may be a signal sequence or other polypeptide having a specific cleavage site at the N-terminus of the mature protein or polypeptide. In general, the signal sequence may be a component of the vector, or it may be a part of the PRO-encoding DNA that is inserted into the vector. The signal sequence may be a prokaryotic signal sequence selected, for example, from the group of the alkaline phosphatase, penicillinase, Ipp, or heat-stable enterotoxin II leaders. For yeast secretion the signal sequence may be, e.g., the yeast invertase leader, alpha factor leader (including *Saccharomyces* and *Kluyveromyces* α-factor leaders, the latter described in U.S. Patent No. 5,010,182), or acid phosphatase leader, the C. *albicans* glucoamylase leader (EP 362,179 published 4 April 1990), or the signal described in WO 90/13646 published 15 November 1990. In mammalian cell expression, mammalian signal sequences may be used to direct secretion of the protein, such as signal sequences from secreted polypeptides of the same or related species, as well as viral secretory leaders.

Both expression and cloning vectors contain a nucleic acid sequence that enables the vector to replicate in one or more selected host cells. Such sequences are well known for a variety of bacteria, yeast, and viruses. The origin of replication from the plasmid pBR322 is suitable for most Gram-negative bacteria, the 2µ plasmid origin is suitable for yeast, and various viral origins (SV40, polyoma, adenovirus, VSV or BPV) are useful for cloning vectors in mammalian cells.

Expression and cloning vectors will typically contain a selection gene, also termed a selectable marker Typical selection genes encode proteins that (a) confer resistance to antibiotics or other toxins, e.g., ampicillin, neomycin, methotrexate, or tetracycline, (b) complement auxotrophic deficiencies, or (c) supply critical nutrients not available from complex media, e.g., the gene encoding D-alanine racemase for *Bacilli.*

An example of suitable selectable markers for mammalian cells are those that enable the identification of cells competent to take up the PRO-encoding nucleic acid, such as DHFR or thymidine kinase. An appropriate host cell when wild-type DHFR is employed is the CHO cell line deficient in DHFR activity, prepared and propagated as described by Urlaub et al., Proc. Natl. Acad. Sci. USA, 77:4216 (1980). A suitable selection gene for use in yeast is the *trp*1 gene present in the yeast plasmid YRp7 [Stinchcomb et al., Nature, 282:39 (1979); Kingsman et al., Gene, 7:141 (1979); Tschemper et al., Gene, 10: 157 (1980)]. The *trp*1 gene provides a selection marker for a mutant strain of yeast lacking the ability to grow in tryptophan, for example, ATCC No. 44076 or PEP4-1 [Jones, Genetics, 85:12 (1977)].

Expression and cloning vectors usually contain a promoter operably linked to the PRO-encoding nucleic acid sequence to direct mRNA synthesis. Promoters recognized by a variety of potential host cells are well known. Promoters suitable for use with prokaryotic hosts include the β-lactamase and lactose promoter systems [Chang et al., Nature, 275:615 (1978); Goeddel et al., Nature, 281:544 (1979)], alkaline phosphatase, a tryptophan (trp) promoter system [Goeddel, Nucleic Acids Res., 8:4057 (1980); EP 36,776], and hybrid promoters such as the tac promoter [deBoer et al., Proc. Natl. Acad. Sci. USA, 80:21-25 (1983)]. Promoters for use in bacterial systems also will contain a Shine-Dalgarno (S.D.) sequence operably linked to the DNA encoding PRO.

Examples of suitable promoting sequences for use with yeast hosts include the promoters for 3-phosphoglycerate kinase [Hitzeman et al., J. Biol. Chem., 255:2073 (1980)] or other glycolytic enzymes [Hess et al., J. Adv. Enzyme Reg., 7:149 (1968); Holland, Biochemistry, 17:4900 (1978)], such as enolase, glyceraldehyde-3-phosphate dehydrogenase, hexokinase, pyruvate decarboxylase, phosphofructokinase, glucose-6-phosphate isomerase, 3-phosphoglycerate mutase, pyruvate kinase, triosephosphate isomerase, phosphoglucose isomerase, and glucokinase.

Other yeast promoters, which are inducible promoters having the additional advantage of transcription controlled by growth conditions, are the promoter regions for alcohol dehydrogenase 2, isocytochrome C, acid phosphatase, degradative enzymes associated with nitrogen metabolism, metallothionein, glyceraldehyde-3-phosphate dehydrogenase, and enzymes responsible for maltose and galactose utilization. Suitable vectors and promoters for use in yeast expression are further described in EP 73,657.

PRO transcription from vectors in mammalian host cells is controlled, for example, by promoters obtained from the genomes of viruses such as polyoma virus, fowlpox virus (UK 2,211,504 published 5 July 1989), adenovirus (such as Adenovirus 2), bovine papilloma virus, avian sarcoma virus, cytomegalovirus, a retrovirus, hepatitis-B virus and Simian Virus 40 (SV40), from heterologous mammalian promoters, e.g., the actin promoter or an immunoglobulin promoter, and from heat-shock promoters, provided such promoters are compatible with the host cell systems.

Transcription of a DNA encoding the PRO by higher eukaryotes may be increased by inserting an enhancer sequence into the vector. Enhancers are cis-acting elements of DNA, usually about from 10 to 300 bp, that act on a promoter to increase its transcription. Many enhancer sequences are now known from mammalian genes (globin, elastase, albumin, α-fetoprotein, and insulin). Typically, however, one will use an enhancer from a eukaryotic cell virus. Examples include the SV40 enhancer on the late side of the replication origin (bp 100-270), the cytomegalovirus early promoter enhancer, the polyoma enhancer on the late side of the replication origin, and adenovirus enhancers. The enhancer may be spliced into the vector at a position 5' or 3' to the PRO coding sequence, but is preferably located at a site 5' from the promoter.

Expression vectors used in eukaryotic host cells (yeast, fungi, insect, plant, animal, human, or nucleated cells from other multicellular organisms) will also contain sequences necessary for the termination of transcription and for stabilizing the mRNA. Such sequences are commonly available from the 5' and, occasionally 3', untranslated regions of eukaryotic or viral DNAs or cDNAs. These regions contain nucleotide segments transcribed as polyadenylated fragments in the untranslated portion of the mRNA encoding PRO.

Still other methods, vectors, and host cells suitable for adaptation to the synthesis of PRO in recombinant vertebrate cell culture are described in Gething et al., Nature, 293:620-625 (1981); Mantei et al., Nature, 281:40-46 (1979); EP 117,060; and EP 117,058.

### 4. Detecting Gene Amplification/Expression

Gene amplification and/or expression may be measured in a sample directly, for example, by conventional Southern blotting, Northern blotting to quantitate the transcription of mRNA [Thomas, Proc. Natl. Acad. Sci. USA, 77:5201-5205 (1980)], dot blotting (DNA analysis), or in situ hybridization, using an appropriately labeled probe, based on the sequences provided herein. Alternatively, antibodies may be employed that can recognize specific duplexes, including DNA duplexes, RNA duplexes, and DNA-RNA hybrid duplexes or DNA-protein duplexes. The antibodies in turn may be labeled and the assay may be carried out where the duplex is bound to a surface, so that upon the formation of duplex on the surface, the presence of antibody bound to the duplex can be detected.

Gene expression, alternatively, may be measured by immunological methods, such as immunohistochemical staining of cells or tissue sections and assay of cell culture or body fluids, to quantitate directly the expression of gene product. Antibodies useful for immunohistochemical staining and/or assay of sample fluids may be either monoclonal or polyclonal, and may be prepared in any mammal. Conveniently, the antibodies may be prepared against a native sequence PRO polypeptide or against a synthetic peptide based on the DNA sequences provided herein or against exogenous sequence fused to PRO DNA and encoding a specific antibody epitope.

### 5. Purification of Polypeptide

Forms of PRO may be recovered from culture medium or from host cell lysates. If membrane-bound, it can be released from the membrane using a suitable detergent solution (e.g. Triton-X 100) or by enzymatic cleavage. Cells employed in expression of PRO can be disrupted by various physical or chemical means, such as freeze-thaw cycling, sonication, mechanical disruption, or cell lysing agents.

It may be desired to purify PRO from recombinant cell proteins or polypeptides. The following procedures are exemplary of suitable purification procedures: by fractionation on an ion-exchange column; ethanol precipitation; reverse phase HPLC; chromatography on silica or on a cation-exchange resin such as DEAE; chromatofocusing; SDS-PAGE; ammonium sulfate precipitation; gel filtration using, for example, Sephadex G-75; protein A Sepharose columns to remove contaminants such as IgG; and metal chelating columns to bind epitope-tagged forms of the PRO. Various methods of protein purification may be employed and such methods are known in the art and described for example in Deutscher, Methods in Enzymology, 182 (1990); Scopes, Protein Purification: Principles and Practice, Springer-Verlag, New York (1982). The purification step(s) selected will depend, for example, on the nature of the production process used and the particular PRO produced.

### E. Uses for PRO

Nucleotide sequences (or their complement) encoding PRO have various applications in the art of molecular biology, including uses as hybridization probes, in chromosome and gene mapping and in the generation of anti-sense RNA and DNA. PRO nucleic acid will also be useful for the preparation of PRO polypeptides by the recombinant techniques described herein.

The full-length native sequence PRO gene, or portions thereof, may be used as hybridization probes for a cDNA library to isolate the full-length PRO cDNA or to isolate still other cDNAs (for instance, those encoding naturally-occurring variants of PRO or PRO from other species) which have a desired sequence identity to the native PRO sequence disclosed herein. Optionally, the length of the probes will be about 20 to about 50 bases. The hybridization probes may be derived from at least partially novel regions of the full length native nucleotide sequence wherein those regions may be determined without undue experimentation or from genomic sequences including promoters, enhancer elements and introns of native sequence PRO. By way of example, a screening method will comprise isolating the coding region of the PRO gene using the known DNA sequence to synthesize a selected probe of about 40 bases. Hybridization probes may be labeled by a variety of labels, including radionucleotides such as ³²P or ³⁵S, or enzymatic labels such as alkaline phosphatase coupled to the probe via avidin/biotin coupling systems. Labeled probes having a sequence complementary to that of the PRO gene of the present invention can be used to screen libraries of human cDNA, genomic DNA or mRNA to determine which members of such libraries the probe hybridizes to. Hybridization techniques are described in further detail in the Examples below.

Any EST sequences disclosed in the present application may similarly be employed as probes, using the methods disclosed herein.

Other useful fragments of the PRO nucleic acids include antisense or sense oligonucleotides comprising a singe-stranded nucleic acid sequence (either RNA or DNA) capable of binding to target PRO mRNA (sense) or PRO DNA (antisense) sequences. Antisense or sense oligonucleotides, according to the present invention, comprise a fragment of the coding region of PRO DNA. Such a fragment generally comprises at least about 14 nucleotides, preferably from about 14 to 30 nucleotides. The ability to derive an antisense or a sense oligonucleotide, based upon a cDNA sequence encoding a given protein is described in, for example, Stein and Cohen (Cancer Res, 48:2659, 1988) and van der Krol et al. (BioTechniques 6:958, 1988).

Binding of antisense or sense oligonucleotides to target nucleic acid sequences results in the formation of duplexes that block transcription or translation of the target sequence by one of several means, including enhanced degradation of the duplexes, premature termination of transcription or translation, or by other means. The antisense oligonucleotides thus may be used to block expression of PRO proteins. Antisense or sense oligonucleotides further comprise oligonucleotides having modified sugar-phosphodiester backbones (or other sugar linkages, such as those described in WO 91/06629) and wherein such sugar linkages are resistant to endogenous nucleases. Such oligonucleotides with resistant sugar linkages are stable *in vivo* (i.e., capable of resisting enzymatic degradation) but retain sequence specificity to be able to bind to target nucleotide sequences.

Other examples of sense or antisense oligonucleotides include those oligonucleotides which are covalently linked to organic moieties, such as those described in WO 90/10048, and other moieties that increases affinity of the oligonucleotide for a target nucleic acid sequence, such as poly-(L-lysine). Further still, intercalating agents, such as ellipticine, and alkylating agents or metal complexes may be attached to sense or antisense oligonucleotides to modify binding specificities of the antisense or sense oligonucleotide for the target nucleotide sequence.

Antisense or sense oligonucleotides may be introduced into a cell containing the target nucleic acid sequence by any gene transfer method, including, for example, CaPO₄-mediated DNA transfection, electroporation, or by using gene transfer vectors such as Epstein-Barr virus. In a preferred procedure, an antisense or sense oligonucleotide is inserted into a suitable retroviral vector. A cell containing the target nucleic acid sequence is contacted with the recombinant retroviral vector, either *in* vivo or *ex vivo.* Suitable retroviral vectors include, but are not limited to, those derived from the murine retrovirus M-MuLV, N2 (a retrovirus derived from M-MuLV), or the double copy vectors designated DCT5A, DCT5B and DCT5C (see WO 90/13641).

Sense or antisense oligonucleotides also may be introduced into a cell containing the target nucleotide sequence by formation of a conjugate with a ligand binding molecule, as described in WO 91/04753. Suitable ligand binding molecules include, but are not limited to, cell surface receptors, growth factors, other cytokines, or other ligands that bind to cell surface receptors. Preferably, conjugation of the ligand binding molecule does not substantially interfere with the ability of the ligand binding molecule to bind to its corresponding molecule or receptor, or block entry of the sense or antisense oligonucleotide or its conjugated version into the cell.

Alternatively, a sense or an antisense oligonucleotide may be introduced into a cell containing the target nucleic acid sequence by formation of an oligonucleotide-lipid complex, as described in WO 90/10448. The sense or antisense oligonucleotide-lipid complex is preferably dissociated within the cell by an endogenous lipase.

Antisense or sense RNA or DNA molecules are generally at least about 5 bases in length, about 10 bases in length, about 15 bases in length, about 20 bases in length, about 25 bases in length, about 30 bases in length, about 35 bases in length, about 40 bases in length, about 45 bases in length, about 50 bases in length, about 55 bases in length, about 60 bases in length, about 65 bases in length, about 70 bases in length, about 75 bases in length, about 80 bases in length, about 85 bases in length, about 90 bases in length, about 95 bases in length, about 100 bases in length, or more.

The probes may also be employed in PCR techniques to generate a pool of sequences for identification of closely related PRO coding sequences.

Nucleotide sequences encoding a PRO can also be used to construct hybridization probes for mapping the gene which encodes that PRO and for the genetic analysis of individuals with genetic disorders. The nucleotide sequences provided herein may be mapped to a chromosome and specific regions of a chromosome using known techniques, such as *in situ* hybridization, linkage analysis against known chromosomal markers, and hybridization screening with libraries.

When the coding sequences for PRO encode a protein which binds to another protein (example, where the PRO is a receptor), the PRO can be used in assays to identify the other proteins or molecules involved in the binding interaction. By such methods, inhibitors of the receptor/ligand binding interaction can be identified. Proteins involved in such binding interactions can also be used to screen for peptide or small molecule inhibitors or agonists of the binding interaction. Also, the receptor PRO can be used to isolate correlative ligand(s). Screening assays can be designed to find lead compounds that mimic the biological activity of a native PRO or a receptor for PRO. Such screening assays will include assays amenable to high-throughput screening of chemical libraries, making them particularly suitable for identifying small molecule drug candidates. Small molecules contemplated include synthetic organic or inorganic compounds. The assays can be performed in a variety of formats, including protein-protein binding assays, biochemical screening assays, immunoassays and cell based assays, which are well characterized in the art.

Nucleic acids which encode PRO or its modified forms can also be used to generate either transgenic animals or "knock out" animals which, in turn, are useful in the development and screening of therapeutically useful reagents. A transgenic animal (e.g., a mouse or rat) is an animal having cells that contain a transgene, which transgene was introduced into the animal or an ancestor of the animal at a prenatal, e.g., an embryonic stage. A transgene is a DNA which is integrated into the genome of a cell from which a transgenic animal develops. In one embodiment, cDNA encoding PRO can be used to clone genomic DNA encoding PRO in accordance with established techniques and the genomic sequences used to generate transgenic animals that contain cells which express DNA encoding PRO. Methods for generating transgenic animals, particularly animals such as mice or rats, have become conventional in the art and are described, for example, in U.S. Patent Nos: 4,736,866 and 4,870,009. Typically, particular cells would be targeted for PRO transgene incorporation with tissue-specific enhancers. Transgenic animals that include a copy of a transgene encoding PRO introduced into the germ line of the animal at an embryonic stage can be used to examine the effect of increased expression of DNA encoding PRO. Such animals can be used as tester animals for reagents thought to confer protection from, for example, pathological conditions associated with its overexpression. In accordance with this facet of the invention, an animal is treated with the reagent and a reduced incidence of the pathological condition, compared to untreated animals bearing the transgene, would indicate a potential therapeutic intervention for the pathological condition.

Alternatively, non-human homologues of PRO can be used to construct a PRO "knock out" animal which has a defective or altered gene encoding PRO as a result of homologous recombination between the endogenous gene encoding PRO and altered genomic DNA encoding PRO introduced into an embryonic stem cell of the animal. For example, cDNA encoding PRO can be used to clone genomic DNA encoding PRO in accordance with established techniques. A portion of the genomic DNA encoding PRO can be deleted or replaced with another gene, such as a gene encoding a selectable marker which can be used to monitor integration. Typically, several kilobases of unaltered flanking DNA (both at the 5' and 3' ends) are included in the vector [see e.g., Thomas and Capecchi, Cell, 51:503 (1987) for a description of homologous recombination vectors]. The vector is introduced into an embryonic stem cell line (e.g., by electroporation) and cells in which the introduced DNA has homologously recombined with the endogenous DNA are selected [see e.g., Li et al., Cell, 69:915 (1992)]. The selected cells are then injected into a blastocyst of an animal (e.g., a mouse or rat) to form aggregation chimeras [see e.g., Bradley, in *Teratocarcinomas and Embryonic Stem Cells: A Practical Approach,* E. J. Robertson, ed. (IRL, Oxford, 1987), pp. 113-152]. A chimeric embryo can then be implanted into a suitable pseudopregnant female foster animal and the embryo brought to term to create a "knock out" animal. Progeny harboring the homologously recombined DNA in their germ cells can be identified by standard techniques and used to breed animals in which all cells of the animal contain the homologously recombined DNA. Knockout animals can be characterized for instance, for their ability to defend against certain pathological conditions and for their development of pathological conditions due to absence of the PRO polypeptide.

Nucleic acid encoding the PRO polypeptides may also be used in gene therapy. In gene therapy applications, genes are introduced into cells in order to achieve in *vivo* synthesis of a therapeutically effective genetic product, for example for replacement of a defective gene. "Gene therapy" includes both conventional gene therapy where a lasting effect is achieved by a single treatment, and the administration of gene therapeutic agents, which involves the one time or repeated administration of a therapeutically effective DNA or mRNA. Antisense RNAs and DNAs can be used as therapeutic agents for blocking the expression of certain genes in vivo. It has already been shown that short antisense oligonucleotides can be imported into cells where they act as inhibitors, despite their low intracellular concentrations caused by their restricted uptake by the cell membrane. (Zamecnik *et al.,* Proc. Natl. Acad. Sci. USA 83:4143-4146 [1986]). The oligonucleotides can be modified to enhance their uptake, e.g. by substituting their negatively charged phosphodiester groups by uncharged groups.

There are a variety of techniques available for introducing nucleic acids into viable cells. The techniques vary depending upon whether the nucleic acid is transferred into cultured cells *in vitro*, or *in vivo* in the cells of the intended host. Techniques suitable for the transfer of nucleic acid into mammalian cells *in vitro* include the use of liposomes, electroporation, microinjection, cell fusion, DEAE-dextran, the calcium phosphate precipitation method, etc. The currently preferred *in vivo* gene transfer techniques include transfection with viral (typically retroviral) vectors and viral coat protein-liposome mediated transfection (Dzau et al., Trends in Biotechnology 11, 205-210 [1993]). In some situations it is desirable to provide the nucleic acid source with an agent that targets the target cells, such as an antibody specific for a cell surface membrane protein or the target cell, a ligand for a receptor on the target cell, etc. Where liposomes are employed, proteins which bind to a cell surface membrane protein associated with endocytosis may be used for targeting and/or to facilitate uptake, e.g. capsid proteins or fragments thereof tropic for a particular cell type, antibodies for proteins which undergo internalization in cycling, proteins that target intracellular localization and enhance intracellular half-life. The technique of receptor-mediated endocytosis is described, for example, by Wu et al., J. Biol. Chem. 262, 4429-4432 (1987); and Wagner et al., Proc. Natl. Acad. Sci. USA 87, 3410-3414 (1990). For review of gene marking and gene therapy protocols see Anderson et al., Science 256, 808-813 (1992).

The PRO polypeptides described herein may also be employed as molecular weight markers for protein electrophoresis purposes and the isolated nucleic acid sequences may be used for recombinantly expressing those markers.

The nucleic acid molecules encoding the PRO polypeptides or fragments thereof described herein are useful for chromosome identification. In this regard, there exists an ongoing need to identify new chromosome markers, since relatively few chromosome marking reagents, based upon actual sequence data are presently available. Each PRO nucleic acid molecule of the present invention can be used as a chromosome marker.

The PRO polypeptides and nucleic acid molecules of the present invention may also be used diagnostically for tissue typing, wherein the PRO polypeptides of the present invention may be differentially expressed in one tissue as compared to another, preferably in a diseased tissue as compared to a normal tissue of the same tissue type. PRO nucleic acid molecules will find use for generating probes for PCR, Northern analysis, Southern analysis and Western analysis.

The PRO polypeptides described herein may also be employed as therapeutic agents. The PRO polypeptides of the present invention can be formulated according to known methods to prepare pharmaceutically useful compositions, whereby the PRO product hereof is combined in admixture with a pharmaceutically acceptable carrier vehicle. Therapeutic formulations are prepared for storage by mixing the active ingredient having the desired degree of purity with optional physiologically acceptable carriers, excipients or stabilizers (Remington's Pharmaceutical Sciences 16th edition, Osol, A. Ed. (1980)), in the form of lyophilized formulations or aqueous solutions. Acceptable carriers, excipients or stabilizers are nontoxic to recipients at the dosages and concentrations employed, and include buffers such as phosphate, citrate and other organic acids; antioxidants including ascorbic acid; low molecular weight (less than about 10 residues) polypeptides; proteins, such as serum albumin, gelatin or immunoglobulins; hydrophilic polymers such as polyvinylpyrrolidone, amino acids such as glycine, glutamine, asparagine, arginine or lysine; monosaccharides, disaccharides and other carbohydrates including glucose, mannose, or dextrins; chelating agents such as EDTA; sugar alcohols such as mannitol or sorbitol; salt-forming counterions such as sodium; and/or nonionic surfactants such as TWEEN™, PLURONICS™ or PEG.

The formulations to be used for in vivo administration must be sterile. This is readily accomplished by filtration through sterile filtration membranes, prior to or following lyophilization and reconstitution.

Therapeutic compositions herein generally are placed into a container having a sterile access port, for example, an intravenous solution bag or vial having a stopper pierceable by a hypodermic injection needle.

The route of administration is in accord with known methods, e.g. injection or infusion by intravenous, intraperitoneal, intracerebral, intramuscular, intraocular, intraarterial or intralesional routes, topical administration, or by sustained release systems.

Dosages and desired drug concentrations of pharmaceutical compositions of the present invention may vary depending on the particular use envisioned. The determination of the appropriate dosage or route of administration is well within the skill of an ordinary physician. Animal experiments provide reliable guidance for the determination of effective doses for human therapy. Interspecies scaling of effective doses can be performed following the principles laid down by Mordenti, J. and Chappell, W. "The use of interspecies scaling in toxicokinetics" In Toxicokinetics and New Drug Development, Yacobi et al., Eds., Pergamon Press, New York 1989, pp. 42-96.

When *in vivo* administration of a PRO polypeptide or agonist or antagonist thereof is employed, normal dosage amounts may vary from about 10 ng/kg to up to 100 mg/kg of mammal body weight or more per day, preferably about 1 µg/kg/day to 10 mg/kg/day, depending upon the route of administration. Guidance as to particular dosages and methods of delivery is provided in the literature; see, for example, U.S. Pat. Nos. 4,657,760; 5,206,344; or 5,225,212. It is anticipated that different formulations will be effective for different treatment compounds and different disorders, that administration targeting one organ or tissue, for example, may necessitate delivery in a manner different from that to another organ or tissue.

Where sustained-release administration of a PRO polypeptide is desired in a formulation with release characteristics suitable for the treatment of any disease or disorder requiring administration of the PRO polypeptide, microencapsulation of the PRO polypeptide is contemplated. Microencapsulation of recombinant proteins for sustained release has been successfully performed with human growth hormone (rhGH), interferon-(rhIFN-) , interleukin-2, and MN rgp120. Johnson et al., Nat. Med., 2:795-799 (1996); Yasuda, Biomed. Ther., 27:1221-1223 (1993); Hora et al., Bio/Technology, 8:755-758 (1990); Cleland, "Design and Production of Single Immunization Vaccines Using Polylactide Polyglycolide Microsphere Systems," in Vaccine Design: The Subunit and Adjuvant Approach, Powell and Newman, eds, (Plenum Press: New York, 1995), pp. 439-462; WO 97/03692, WO 96/40072, WO 96/07399; and U.S. Pat. No. 5,654,010.

The sustained-release formulations of these proteins were developed using poly-lactic-coglycolic acid (PLGA) polymer due to its biocompatibility and wide range of biodegradable properties. The degradation products of PLGA, lactic and glycolic acids, can be cleared quickly within the human body. Moreover, the degradability of this polymer can be adjusted from months to years depending on its molecular weight and composition. Lewis, "Controlled release of bioactive agents from lactide/glycolide polymer," in: M. Chasin and R. Langer (Eds.), Biodegradable Polymers as Drug Delivery Systems (Marcel Dekker: New York, 1990), pp. 1-41.

This invention encompasses methods of screening compounds to identify those that mimic the PRO polypeptide (agonists) or prevent the effect of the PRO polypeptide (antagonists). Screening assays for antagonist drug candidates are designed to identify compounds that bind or complex with the PRO polypeptides encoded by the genes identified herein, or otherwise interfere with the interaction of the encoded polypeptides with other cellular proteins. Such screening assays will include assays amenable to high-throughput screening of chemical libraries, making them particularly suitable for identifying small molecule drug candidates.

The assays can be performed in a variety of formats, including protein-protein binding assays, biochemical screening assays, immunoassays, and cell-based assays, which are well characterized in the art.

All assays for antagonists are common in that they call for contacting the drug candidate with a PRO polypeptide encoded by a nucleic acid identified herein under conditions and for a time sufficient to allow these two components to interact.

In binding assays, the interaction is binding and the complex formed can be isolated or detected in the reaction mixture. In a particular embodiment, the PRO polypeptide encoded by the gene identified herein or the drug candidate is immobilized on a solid phase, e.g., on a microtiter plate, by covalent or non-covalent attachments. Non-covalent attachment generally is accomplished by coating the solid surface with a solution of the PRO polypeptide and drying. Alternatively, an immobilized antibody, e.g., a monoclonal antibody, specific for the PRO polypeptide to be immobilized can be used to anchor it to a solid surface. The assay is performed by adding the non-immobilized component, which may be labeled by a detectable label, to the immobilized component, e.g., the coated surface containing the anchored component. When the reaction is complete, the non-reacted components are removed, e.g., by washing, and complexes anchored on the solid surface are detected. When the originally non-immobilized component carries a detectable label, the detection of label immobilized on the surface indicates that complexing occurred. Where the originally non-immobilized component does not carry a label, complexing can be detected, for example, by using a labeled antibody specifically binding the immobilized complex.

If the candidate compound interacts with but does not bind to a particular PRO polypeptide encoded by a gene identified herein, its interaction with that polypeptide can be assayed by methods well known for detecting protein-protein interactions. Such assays include traditional approaches, such as, e.g., cross-linking, coimmunoprecipitation, and co-purification through gradients or chromatographic columns. In addition, protein-protein interactions can be monitored by using a yeast-based genetic system described by Fields and co-workers (Fields and Song, Nature (London), 340:245-246 (1989); Chien et al., Proc. Natl. Acad. Sci. USA, 88:9578-9582 (1991)) as disclosed by Chevray and Nathans, Proc. Natl. Acad. Sci. USA, 89: 5789-5793 (1991). Many transcriptional activators, such as yeast GAL4, consist of two physically discrete modular domains, one acting as the DNA-binding domain, the other one functioning as the transcription-activation domain. The yeast expression system described in the foregoing publications (generally referred to as the "two-hybrid system") takes advantage of this property, and employs two hybrid proteins, one in which the target protein is fused to the DNA-binding domain of GAL4, and another, in which candidate activating proteins are fused to the activation domain. The expression of a GALI-*lac*Z reporter gene under control of a GAL4-activated promoter depends on reconstitution of GAL4 activity via protein-protein interaction. Colonies containing interacting polypeptides are detected with a chromogenic substrate for β-galactosidase. A complete kit (MATCHMAKER™) for identifying protein-protein interactions between two specific proteins using the two-hybrid technique is commercially available from Clontech. This system can also be extended to map protein domains involved in specific protein interactions as well as to pinpoint amino acid residues that are crucial for these interactions.

Compounds that interfere with the interaction of a gene encoding a PRO polypeptide identified herein and other intra- or extracellular components can be tested as follows: usually a reaction mixture is prepared containing the product of the gene and the intra- or extracellular component under conditions and for a time allowing for the interaction and binding of the two products. To test the ability of a candidate compound to inhibit binding, the reaction is run in the absence and in the presence of the test compound. In addition, a placebo may be added to a third reaction mixture, to serve as positive control. The binding (complex formation) between the test compound and the intra- or extracellular component present in the mixture is monitored as described hereinabove. The formation of a complex in the control reaction(s) but not in the reaction mixture containing the test compound indicates that the test compound interferes with the interaction of the test compound and its reaction partner.

To assay for antagonists, the PRO polypeptide may be added to a cell along with the compound to be screened for a particular activity and the ability of the compound to inhibit the activity of interest in the presence of the PRO polypeptide indicates that the compound is an antagonist to the PRO polypeptide. Alternatively, antagonists may be detected by combining the PRO polypeptide and a potential antagonist with membrane-bound PRO polypeptide receptors or recombinant receptors under appropriate conditions for a competitive inhibition assay. The PRO polypeptide can be labeled, such as by radioactivity, such that the number of PRO polypeptide molecules bound to the receptor can be used to determine the effectiveness of the potential antagonist. The gene encoding the receptor can be identified by numerous methods known to those of skill in the art, for example, ligand panning and FACS sorting. Coligan et al., Current Protocols in Immun., 1(2): Chapter 5 (1991). Preferably, expression cloning is employed wherein polyadenylated RNA is prepared from a cell responsive to the PRO polypeptide and a cDNA library created from this RNA is divided into pools and used to transfect COS cells or other cells that are not responsive to the PRO polypeptide. Transfected cells that are grown on glass slides are exposed to labeled PRO polypeptide. The PRO polypeptide can be labeled by a variety of means including iodination or inclusion of a recognition site for a site-specific protein kinase. Following fixation and incubation, the slides are subjected to autoradiographic analysis. Positive pools are identified and sub-pools are prepared and re-transfected using an interactive sub-pooling and re-screening process, eventually yielding a single clone that encodes the putative receptor.

As an alternative approach for receptor identification, labeled PRO polypeptide can be photoaffinity-linked with cell membrane or extract preparations that express the receptor molecule. Cross-linked material is resolved by PAGE and exposed to X-ray film. The labeled complex containing the receptor can be excised, resolved into peptide fragments, and subjected to protein micro-sequencing. The amino acid sequence obtained from micro- sequencing would be used to design a set of degenerate oligonucleotide probes to screen a cDNA library to identify the gene encoding the putative receptor.

In another assay for antagonists, mammalian cells or a membrane preparation expressing the receptor would be incubated with labeled PRO polypeptide in the presence of the candidate compound. The ability of the compound to enhance or block this interaction could then be measured.

More specific examples of potential antagonists include an oligonucleotide that binds to the fusions of immunoglobulin with PRO polypeptide, and, in particular, antibodies including, without limitation, poly- and monoclonal antibodies and antibody fragments, single-chain antibodies, anti-idiotypic antibodies, and chimeric or humanized versions of such antibodies or fragments, as well as human antibodies and antibody fragments. Alternatively, a potential antagonist may be a closely related protein, for example, a mutated form of the PRO polypeptide that recognizes the receptor but imparts no effect, thereby competitively inhibiting the action of the PRO polypeptide.

Another potential PRO polypeptide antagonist is an antisense RNA or DNA construct prepared using antisense technology, where, e.g., an antisense RNA or DNA molecule acts to block directly the translation of mRNA by hybridizing to targeted mRNA and preventing protein translation. Antisense technology can be used to control gene expression through triple-helix formation or antisense DNA or RNA, both of which methods are based on binding of a polynucleotide to DNA or RNA. For example, the 5' coding portion of the polynucleotide sequence, which encodes the mature PRO polypeptides herein, is used to design an antisense RNA oligonucleotide of from about 10 to 40 base pairs in length. A DNA oligonucleotide is designed to be complementary to a region of the gene involved in transcription (triple helix - see Lee et al., Nucl. Acids Res., 6:3073 (1979); Cooney et al., Science, 241: 456 (1988); Dervan et al., Science, 251:1360 (1991)), thereby preventing transcription and the production of the PRO polypeptide. The antisense RNA, oligonucleotide hybridizes to the mRNA *in vivo* and blocks translation of the mRNA molecule into the PRO polypeptide (antisense - Okano, Neurochem., 56:560 (1991); Oligo de oxy nucleotides as Antisense Inhibitors of Gene Expression (CRC Press: Boca Raton, FL, 1988). The oligonucleotides described above can also be delivered to cells such that the antisense RNA or DNA may be expressed *in vivo* to inhibit production of the PRO polypeptide. When antisense DNA is used, oligodeoxyribonucleotides derived from the translation-initiation site, e.g., between about -10 and + 10 positions of the target gene nucleotide sequence, are preferred.

Potential antagonists include small molecules that bind to the active site, the receptor binding site, or growth factor or other relevant binding site of the PRO polypeptide, thereby blocking the normal biological activity of the PRO polypeptide. Examples of small molecules include, but are not limited to, small peptides or peptide-like molecules, preferably soluble peptides, and synthetic non-peptidyl organic or inorganic compounds.

Ribozymes are enzymatic RNA molecules capable of catalyzing the specific cleavage of RNA. Ribozymes act by sequence-specific hybridization to the complementary target RNA, followed by endonucleolytic cleavage. Specific ribozyme cleavage sites within a potential RNA target can be identified by known techniques. For further details see, e.g., Rossi, <Current Biology, 4:469-471 (1994), and PCT publication No. WO 97/33551 (published September 18, 1997).

Nucleic acid molecules in triple-helix formation used to inhibit transcription should be single-stranded and composed of deoxynucleotides. The base composition of these oligonucleotides is designed such that it promotes triple-helix formation via Hoogsteen base-pairing rules, which generally require sizeable stretches of purines or pyrimidines on one strand of a duplex. For further details see, e.g., PCT publication No. WO *97*/*33551, supra*.

These small molecules can be identified by any one or more of the screening assays discussed hereinabove and/or by any other screening techniques well known for those skilled in the art.

Diagnostic and therapeutic uses of the herein disclosed molecules may also be based upon the positive functional assay hits disclosed and described below.

### F. Anti-PRO Antibodies

The present invention further provides anti-PRO antibodies. Exemplary antibodies include polyclonal, monoclonal, humanized, bispecific, and heteroconjugate antibodies.

### 1. Polyclonal Antibodies

The anti-PRO antibodies may comprise polyclonal antibodies. Methods of preparing polyclonal antibodies are known to the skilled artisan. Polyclonal antibodies can be raised in a mammal, for example, by one or more injections of an immunizing agent and, if desired, an adjuvant. Typically, the immunizing agent and/or adjuvant will be injected in the mammal by multiple subcutaneous or intraperitoneal injections. The immunizing agent may include the PRO polypeptide or a fusion protein thereof. It may be useful to conjugate the immunizing agent to a protein known to be immunogenic in the mammal being immunized. Examples of such immunogenic proteins include but are not limited to keyhole limpet hemocyanin, serum albumin, bovine thyroglobulin, and soybean trypsin inhibitor. Examples of adjuvants which may be employed include Freund's complete adjuvant and MPL-TDM adjuvant (monophosphoryl Lipid A, synthetic trehalose dicorynomycolate). The immunization protocol may be selected by one skilled in the art without undue experimentation.

### 2. Monoclonal Antibodies

The anti-PRO antibodies may, alternatively, be monoclonal antibodies. Monoclonal antibodies may be prepared using hybridoma methods, such as those described by Kohler and Milstein, Nature, 256:495 (1975). In a hybridoma method, a mouse, hamster, or other appropriate host animal, is typically immunized with an immunizing agent to elicit lymphocytes that produce or are capable of producing antibodies that will specifically bind to the immunizing agent. Alternatively, the lymphocytes may be immunized *in vitro.*

The immunizing agent will typically include the PRO polypeptide or a fusion protein thereof. Generally, either peripheral blood lymphocytes ("PBLs") are used if cells of human origin are desired, or spleen cells or lymph node cells are used if non-human mammalian sources are desired. The lymphocytes are then fused with an immortalized cell line using a suitable fusing agent, such as polyethylene glycol, to form a hybridoma cell [Goding, Monoclonal Antibodies: Principles and Practice, Academic Press, (1986) pp. 59-103]. Immortalized cell lines are usually transformed mammalian cells, particularly myeloma cells of rodent, bovine and human origin. Usually, rat or mouse myeloma cell lines are employed. The hybridoma cells may be cultured in a suitable culture medium that preferably contains one or more substances that inhibit the growth or survival of the unfused, immortalized cells. For example, if the parental cells lack the enzyme hypoxanthine guanine phosphoribosyl transferase (HGPRT or HPRT), the culture medium for the hybridomas typically will include hypoxanthine, aminopterin, and thymidine ("HAT medium"), which substances prevent the growth of HGPRT-deficient cells.

Preferred immortalized cell lines are those that fuse efficiently, support stable high level expression of antibody by the selected antibody-producing cells, and are sensitive to a medium such as HAT medium. More preferred immortalized cell lines are murine myeloma lines, which can be obtained, for instance, from the Salk Institute Cell Distribution Center, San Diego, California and the American Type Culture Collection, Manassas, Virginia. Human myeloma and mouse-human heteromyeloma cell lines also have been described for the production of human monoclonal antibodies [Kozbor, J. Immunol., 133:3001 (1984); Brodeur et al., Monoclonal Antibody Production Techniques and Applications, Marcel Dekker, Inc., New York, (1987) pp. 51-63].

The culture medium in which the hybridoma cells are cultured can then be assayed for the presence of monoclonal antibodies directed against PRO. Preferably, the binding specificity of monoclonal antibodies produced by the hybridoma cells is determined by immunoprecipitation or by an *in vitro* binding assay, such as radioimmunoassay (RIA) or enzyme-linked immunoabsorbent assay (ELISA). Such techniques and assays are known in the art. The binding affinity of the monoclonal antibody can, for example, be determined by the Scatchard analysis of Munson and Pollard, Anal. Biochem., 107:220 (1980).

After the desired hybridoma cells are identified, the clones may be subcloned by limiting dilution procedures and grown by standard methods [Goding, supra]. Suitable culture media for this purpose include, for example, Dulbecco's Modified Eagle's Medium and RPMI-1640 medium. Alternatively, the hybridoma cells may be grown in *vivo* as ascites in a mammal.

The monoclonal antibodies secreted by the subclones may be isolated or purified from the culture medium or ascites fluid by conventional immunoglobulin purification procedures such as, for example, protein A-Sepharose, hydroxylapatite chromatography, gel electrophoresis, dialysis, or affinity chromatography.

The monoclonal antibodies may also be made by recombinant DNA methods, such as those described in U.S. Patent No. 4,816,567. DNA encoding the monoclonal antibodies of the invention can be readily isolated and sequenced using conventional procedures (e.g., by using oligonucleotide probes that are capable of binding specifically to genes encoding the heavy and light chains of murine antibodies). The hybridoma cells of the invention serve as a preferred source of such DNA. Once isolated, the DNA may be placed into expression vectors, which are then transfected into host cells such as simian COS cells, Chinese hamster ovary (CHO) cells, or myeloma cells that do not otherwise produce immunoglobulin protein, to obtain the synthesis of monoclonal antibodies in the recombinant host cells. The DNA also may be modified, for example, by substituting the coding sequence for human heavy and light chain constant domains in place of the homologous murine sequences [U.S. Patent No. 4,816,567; Morrison et al., supra] or by covalently joining to the immunoglobulin coding sequence all or part of the coding sequence for a non-immunoglobulin polypeptide. Such a non-immunoglobulin polypeptide can be substituted for the constant domains of an antibody of the invention, or can be substituted for the variable domains of one antigen-combining site of an antibody of the invention to create a chimeric bivalent antibody.

The antibodies may be monovalent antibodies. Methods for preparing monovalent antibodies are well known in the art. For example, one method involves recombinant expression of immunoglobulin light chain and modified heavy chain. The heavy chain is truncated generally at any point in the Fc region so as to prevent heavy chain crosslinking. Alternatively, the relevant cysteine residues are substituted with another amino acid residue or are deleted so as to prevent crosslinking.

*In vitro* methods are also suitable for preparing monovalent antibodies. Digestion of antibodies to produce fragments thereof, particularly, Fab fragments, can be accomplished using routine techniques known in the art.

### 3. Human and Humanized Antibodies

The anti-PRO antibodies of the invention may further comprise humanized antibodies or human antibodies. Humanized forms of non-human (e.g., murine) antibodies are chimeric immunoglobulins, immunoglobulin chains or fragments thereof (such as Fv, Fab, Fab', F(ab')₂, or other antigen-binding subsequences of antibodies) which contain minimal sequence derived from non-human immunoglobulin. Humanized antibodies include human immunoglobulins (recipient antibody) in which residues from a complementary determining region (CDR) of the recipient are replaced by residues from a CDR of a non-human species (donor antibody) such as mouse, rat or rabbit having the desired specificity, affinity and capacity. In some instances, Fv framework residues of the human immunoglobulin are replaced by corresponding non-human residues. Humanized antibodies may also comprise residues which are found neither in the recipient antibody nor in the imported CDR or framework sequences. In general, the humanized antibody will comprise substantially all of at least one, and typically two, variable domains, in which all or substantially all of the CDR regions correspond to those of a non-human immunoglobulin and all or substantially all of the FR regions are those of a human immunoglobulin consensus sequence. The humanized antibody optimally also will comprise at least a portion of an immunoglobulin constant region (Fc), typically that of a human immunoglobulin [Jones et al., Nature, 321:522-525 (1986); Riechmannetal., Nature, 332:323-329 (1988); and Presta, Curr. Op. Struct. Biol., 2:593-596 (1992)].

Methods for humanizing non-human antibodies arc well known in the art. Generally, a humanized antibody has one or more amino acid residues introduced into it from a source which is non-human. These non-human amino acid residues are often referred to as "import" residues, which are typically taken from an "import" variable domain. Humanization can be essentially performed following the method of Winter and co-workers [Jones et al., Nature, 321:522-525 (1986); Riechmann et al., Nature, 332:323-327 (1988); Verhoeyen et al., Science, 239:1534-1536 (1988)], by substituting rodent CDRs or CDR sequences for the corresponding sequences of a human antibody. Accordingly, such "humanized" antibodies are chimeric antibodies (U.S. Patent No. 4,816,567), wherein substantially less than an intact human variable domain has been substituted by the corresponding sequence from a non-human species. In practice, humanized antibodies are typically human antibodies in which some CDR residues and possibly some FR residues are substituted by residues from analogous sites in rodent antibodies.

Human antibodies can also be produced using various techniques known in the art, including phage display libraries [Hoogenboom and Winter, J. Mol. Biol., 227:381 (1991); Marks et al., J. Mol. Biol., 222:581 (1991)]. The techniques of Cole et al. and Boerner et al. are also available for the preparation of human monoclonal antibodies (Cole et al., Monoclonal Antibodies and Cancer Therapy, Alan R. Liss, p. 77 (1985) and Boerner et al., J. Immunol., 147(1):86-95 (1991)]. Similarly, human antibodies can be made by introducing of human immunoglobulin loci into transgenic animals, e.g., mice in which the endogenous immunoglobulin genes have been partially or completely inactivated. Upon challenge, human antibody production is observed, which closely resembles that seen in humans in all respects, including gene rearrangement, assembly, and antibody repertoire. This approach is described, for example, in U.S. Patent Nos. 5,545,807; 5,545,806; 5,569,825; 5,625,126; 5,633,425; 5,661,016, and in the following scientific publications: Marks *et al.*, Bio/Technology 10, 779-783 (1992); Lonberg *et al.,* Nature 368 856-859 (1994); Morrison, Nature 368, 812-13 (1994); Fishwild et al., Nature Biotechnology 14, 845-51 (1996); Neuberger, Nature Biotechnology 14, 826 (1996); Lonberg and Huszar, Intern. Rev. Immunol. 13 65-93 (1995).

The antibodies may also be affinity matured using known selection and/or mutagenesis methods as described above. Preferred affinity matured antibodies have an affinity which is five times, more preferably 10 times, even more preferably 20 or 30 times greater than the starting antibody (generally murine, humanized or human) from which the matured antibody is prepared.

### 4. Bispecific Antibodies

Bispecific antibodies are monoclonal, preferably human or humanized, antibodies that have binding specificities for at least two different antigens. In the present case, one of the binding specificities is for the PRO, the other one is for any other antigen, and preferably for a cell-surface protein or receptor or receptor subunit.

Methods for making bispecific antibodies are known in the art. Traditionally, the recombinant production of bispecific antibodies is based on the co-expression of two immunoglobulin heavy-chain/light-chain pairs, where the two heavy chains have different specificities [Milstein and Cuello, Nature, 305:537-539 (1983)]. Because of the random assortment of immunoglobulin heavy and light chains, these hybridomas (quadromas) produce a potential mixture of ten different antibody molecules, of which only one has the correct bispecific structure. The purification of the correct molecule is usually accomplished by affinity chromatography steps. Similar procedures are disclosed in WO 93/08829, published 13 May 1993, and in Traunecker et al., EMBO J., 10:3655-3659 (1991).

Antibody variable domains with the desired binding specificities (antibody-antigen combining sites) can be fused to immunoglobulin constant domain sequences. The fusion preferably is with an immunoglobulin heavy-chain constant domain, comprising at least part of the hinge, CH2, and CH3 regions. It is preferred to have the first heavy-chain constant region (CH1) containing the site necessary for light-chain binding present in at least one of the fusions. DNAs encoding the immunoglobulin heavy-chain fusions and, if desired, the immunoglobulin light chain, are inserted into separate expression vectors, and are co-transfected into a suitable host organism. For further details of generating bispecific antibodies see, for example, Suresh et al., Methods in Enzymology, 121:210 (1986).

According to another approach described in WO 96/27011, the interface between a pair of antibody molecules can be engineered to maximize the percentage of heterodimers which are recovered from recombinant cell culture. The preferred interface comprises at least a part of the CH3 region of an antibody constant domain. In this method, one or more small amino acid side chains from the interface of the first antibody molecule are replaced with larger side chains (e.g. tyrosine or tryptophan). Compensatory "cavities" of identical or similar size to the large side chain(s) are created on the interface of the second antibody molecule by replacing large amino acid side chains with smaller ones (e.g. alanine or threonine). This provides a mechanism for increasing the yield of the heterodimer over other unwanted end-products such as homodimers.

Bispecific antibodies can be prepared as full length antibodies or antibody fragments (e.g. F(ab')₂ bispecific antibodies). Techniques for generating bispecific antibodies from antibody fragments have been described in the literature. For example, bispecific antibodies can be prepared can be prepared using chemical linkage. Brennan *et al*., Science 229:81 (1985) describe a procedure wherein intact antibodies are proteolytically cleaved to generate F(ab')₂ fragments. These fragments are reduced in the presence of the dithiol complexing agent sodium arsenite to stabilize vicinal dithiols and prevent intermolecular disulfide formation. The Fab' fragments generated are then converted to thionitrobenzoate (TNB) derivatives. One of the Fab'-TNB derivatives is then reconverted to the Fab'-thiol by reduction with mercaptoethylamine and is mixed with an equimolar amount of the other Fab'-TNB derivative to form the bispecific antibody- The bispecific antibodies produced can be used as agents for the selective immobilization of enzymes.

Fab' fragments may be directly recovered from *E. coli* and chemically coupled to form bispecific antibodies. Shalaby *et al.,* *J.* Exp. Med. 175:217-225 (1992) describe the production of a fully humanized bispecific antibody F(ab')₂ molecule. Each Fab' fragment was separately secreted from *E. coli* and subjected to directed chemical coupling *in vitro* to form the bispecific antibody. The bispecific antibody thus formed was able to bind to cells overexpressing the ErbB2 receptor and normal human T cells, as well as trigger the lytic activity of human cytotoxic lymphocytes against human breast tumor targets.

Various technique for making and isolating bispecific antibody fragments directly from recombinant cell culture have also been described. For example, bispecific antibodies have been produced using leucine zippers. Kostelny *et al.*, J. Immunol. 148(5):1547-1553 (1992). The leucine zipper peptides from the Fos and Jun proteins were linked to the Fab' portions of two different antibodies by gene fusion. The antibody homodimers were reduced at the hinge region to form monomers and then re-oxidized to form the antibody heterodimers. This method can also be utilized for the production of antibody homodimers. The "diabody" technology described by Hollinger *et al.,* Proc. Natl. Acad. Sci. USA 90:6444-6448 (1993) has provided an alternative mechanism for making bispecific antibody fragments. The fragments comprise a heavy-chain variable domain (V_{H}) connected to a light-chain variable domain (V_{L}) by a linker which is too short to allow pairing between the two domains on the same chain. Accordingly, the V_{H} and V_{L} domains of one fragment are forced to pair with the complementary V_{L} and V_{H} domains of another fragment, thereby forming two antigen-binding sites. Another strategy for making bispecific antibody fragments by the use of single-chain Fv (sFv) dimers has also been reported. See, Gruber et *al.,* J. Immunol. 152:5368 (1994).
Antibodies with more than two valencies are contemplated. For example, trispecific antibodies can be prepared. *Tutt et al.,* J. Immunol. 147:60 (1991).

Exemplary bispecific antibodies may bind to two different epitopes on a given PRO polypeptide herein. Alternatively, an anti-PRO polypeptide arm may be combined with an arm which binds to a triggering molecule on a leukocyte such as a T-cell receptor molecule (e.g. CD2, CD3, CD28, or B7), or Fc receptors for IgG (FcγR), such as FcγRI (CD64), FcγRII (CD32) and FcγRIII (CD16) so as to focus cellular defense mechanisms to the cell expressing the particular PRO polypeptide. Bispecific antibodies may also be used to localize cytotoxic agents to cells which express a particular PRO polypeptide. These antibodies possess a PRO-binding arm and an arm which binds a cytotoxic agent or a radionuclide chelator, such as EOTUBE, DPTA, DOTA, or TETA. Another bispecific antibody of interest binds the PRO polypeptide and further binds tissue factor (TF).

### 5. Heteroconjugate Antibodies

Heteroconjugate antibodies are also within the scope of the present invention. Heteroconjugate antibodies are composed of two covalently joined antibodies. Such antibodies have, for example, been proposed to target immune system cells to unwanted cells [U.S. Patent No. 4,676,980], and for treatment of HIV infection [WO 91/00360; WO92/200373; EP 03089]. It is contemplated that the antibodies may be prepared *in* vitro using known methods in synthetic protein chemistry, including those involving crosslinking agents. For example, immunotoxins may be constructed using a disulfide exchange reaction or by forming a thioether bond. Examples of suitable reagents for this purpose include iminothiolate and methyl-4-mercaptobutyrimidate and those disclosed, for example, in U.S. Patent No. 4,676,980.

### 6. Effector Function Engineering

It may be desirable to modify the antibody of the invention with respect to effector function, so as to enhance, e.g., the effectiveness of the antibody in treating cancer. For example, cysteine residue(s) may be introduced into the Fc region, thereby allowing interchain disulfide bond formation in this region. The homodimeric antibody thus generated may have improved internalization capability and/or increased complement-mediated cell killing and antibody-dependent cellular cytotoxicity (ADCC). See Caron *et al.,* *J.* Exp Med., 176: 1191-1195 (1992) and Shopes, J. Immunol., 148: 2918-2922 (1992). Homodimeric antibodies with enhanced antitumor activity may also be prepared using heterobifunctional cross-linkers as described in Wolff *et al.* Cancer Research, 53: 2560-2565 (1993). Alternatively, an antibody can be engineered that has dual Fc regions and may thereby have enhanced complement lysis and ADCC capabilities. See Stevenson *et al.,* Anti-Cancer Drug Design, 3: 219-230 (1989).

### 7. Immunoconjugates

The invention also pertains to immunoconjugates comprising an antibody conjugated to a cytotoxic agent such as a chemotherapeutic agent, toxin *(e.g.,* an enzymatically active toxin of bacterial, fungal, plant, or animal origin, or fragments thereof), or a radioactive isotope (i.e., a radioconjugate).

Chemotherapeutic agents useful in the generation of such immunoconjugates have been described above. Enzymatically active toxins and fragments thereof that can be used include diphtheria A chain, nonbinding active fragments of diphtheria toxin, exotoxin A chain (from *Pseudomonas aeruginosa),* ricin A chain, abrin A chain, modeccin A chain, alpha-sarcin, *Aleuritesfordii* proteins, dianthin proteins, *Phytolaca americana* proteins (PAPI, PAPII, and PAP-S), momordica charantia inhibitor, curcin, crotin, sapaonaria officinalis inhibitor, gelonin, mitogellin, restrictociu, phenomycin, enomycin, and the tricothecenes. A variety of radionuclides are available for the production of radioconjugated antibodies. Examples include ²¹²Bi, ¹³¹I, ¹³¹In, ⁹⁰Y, and ¹⁸⁶Re.
Conjugates of the antibody and cytotoxic agent are made using a variety of bifunctional protein-coupling agents such as N-succinimidyl-3-(2-pyridyldithiol) propionate (SPDP), iminothiolane (IT), bifunctional derivatives of imidoesters (such as dimethyl adipimidate HCL), active esters (such as disuccinimidyl suberate), aldehydes (such as glutareldehyde), bis-azido compounds (such as bis (p-azidobenzoyl) hexanediamine), bis-diazonium derivatives (such as bis-(p-diazoniumbenzoyl)-ethylenediamine), diisocyanates (such as tolyene 2,6-diisocyanate), and bis-active fluorine compounds (such as 1,5-difluoro-2,4-dinitrobenzene). For example, a ricin immunotoxin can be prepared as described in Vitetta *et al.,* Science, 238: 1098 (1987). Carbon-14-labeled 1-isothiocyanatobenzyl-3-methyldiethylene triaminepentaacetic acid (MX-DTPA) is an exemplary chelating agent for conjugation of radionucleotide to the antibody. See W094/11026.

In another embodiment, the antibody may be conjugated to a "receptor" (such streptavidin) for utilization in tumor pretargeting wherein the antibody-receptor conjugate is administered to the patient, followed by removal of unbound conjugate from the circulation using a clearing agent and then administration of a "ligand" (e.g., avidin) that is conjugated to a cytotoxic agent (e.g., a radionucleotide).

### 8. Immunoliposomes

The antibodies disclosed herein may also be formulated as immunoliposomes. Liposomes containing the antibody are prepared by methods known in the art, such as described in Epstein *et al.,* Proc. Natl. Acad. Sci. USA, 82: 3688 (1985); *Hwang et al*., Proc. Natl Acad. Sci. USA, 77: 4030 (1980); and U.S. Pat. Nos. 4,485,045 and 4,544,545. Liposomes with enhanced circulation time are disclosed in U.S. Patent No. 5,013,556.

Particularly useful liposomes can be generated by the reverse-phase evaporation method with a lipid composition comprising phosphatidylcholine, cholesterol, and PEG-derivatized phosphatidylethanolamine (PEG-PE). Liposomes are extruded through filters of defined pore size to yield liposomes with the desired diameter. Fab' fragments of the antibody of the present invention can be conjugated to the liposomes as described in Martin *et al.,* J. Biol. Chem., 257: 286-288 (1982) via a disulfide-interchange reaction. A chemotherapeutic agent (such as Doxorubicin) is optionally contained within the liposome. See *Gabizon et al. ,* J. National Cancer Inst., 81(19): 1484 (1989).

### 9. Pharmaceutical Compositions of Antibodies

Antibodies specifically binding a PRO polypeptide identified herein, as well as other molecules identified by the screening assays disclosed hereinbefore, can be administered for the treatment of various disorders in the form of pharmaceutical compositions.

If the PRO polypeptide is intracellular and whole antibodies are used as inhibitors, internalizing antibodies are preferred. However, lipofections or liposomes can also be used to deliver the antibody, or an antibody fragment, into cells. Where antibody fragments are used, the smallest inhibitory fragment that specifically binds to the binding domain of the target protein is preferred. For example, based upon the variable-region sequences of an antibody, peptide molecules can be designed that retain the ability to bind the target protein sequence. Such peptides can be synthesized chemically and/or produced by recombinant DNA technology. See, e.g., *Marasco et al.,* Proc. Natl. Acad. Sci. USA, 90: 7889-7893 (1993). The formulation herein may also contain more than one active compound as necessary for the particular indication being treated, preferably those with complementary activities that do not adversely affect each other. Alternatively, or in addition, the composition may comprise an agent that enhances its function, such as, for example, a cytotoxic agent, cytokine, chemotherapeutic agent, or growth-inhibitory agent. Such molecules are suitably present in combination in amounts that are effective for the purpose intended.

The active ingredients may also be entrapped in microcapsules prepared, for example, by coacervation techniques or by interfacial polymerization, for example, hydroxymethylcellulose or gelatin-microcapsules and poly-(methylmethacylate) microcapsules, respectively, in colloidal drug delivery systems (for example, liposomes, albumin microspheres, microemulsioris, nano-particles, and nanocapsules) or in macroernulsions. Such techniques are disclosed in Remington's Pharmaceutical Sciences, *supra.*

The formulations to be used for in *vivo* administration must be sterile. This is readily accomplished by filtration through sterile filtration membranes.

Sustained-release preparations may be prepared. Suitable examples of sustained-release preparations include semipermeable matrices of solid hydrophobic polymers containing the antibody, which matrices are in the form of shaped articles, e.g., films, or microcapsules. Examples of sustained-release matrices include polyesters, hydrogels (for example, poly(2-hydroxyethyl-methacrylate), or poly(vinylalcohol)), polylactides (U.S. Pat. No. 3,773,919), copolymers of L-glutamic acid and γ ethyl-L-glutamate, non-degradable ethylene-vinyl acetate, degradable lactic acid-glycolic acid copolymers such as the LUPRON DEPOT ™ (injectable microspheres composed of lactic acid-glycolic acid copolymer and leuprolide acetate), and poly-D-(-)-3-hydroxybutyric acid. While polymers such as ethylene-vinyl acetate and lactic acid-glycolic acid enable release of molecules for over 100 days, certain hydrogels release proteins for shorter time periods. When encapsulated antibodies remain in the body for a long time, they may denature or aggregate as a result of exposure to moisture at 37°C, resulting in a loss of biological activity and possible changes in immunogenicity. Rational strategies can be devised for stabilization depending on the mechanism involved. For example, if the aggregation mechanism is discovered to be intermolecular S-S bond formation through thio-disulfide interchange, stabilization may be achieved by modifying sulfhydryl residues, lyophilizing from acidic solutions, controlling moisture content, using appropriate additives, and developing specific polymer matrix compositions.

### G. Uses for anti-PRO Antibodies

The anti-PRO antibodies of the invention have various utilities. For example, anti-PRO antibodies may be used in diagnostic assays for PRO, e.g., detecting its expression (and in some cases, differential expression) in specific cells, tissues, or serum. Various diagnostic assay techniques known in the art may be used, such as competitive binding assays, direct or indirect sandwich assays and immunoprecipitation assays conducted in either heterogeneous or homogeneous phases [Zola, Monoclonal Antibodies: A Manual of Techniques, CRC Press, Inc. (1987) pp. 147-158]. The antibodies used in the diagnostic assays can be labeled with a detectable moiety. The detectable moiety should be capable of producing, either directly or indirectly, a detectable signal. For example, the detectable moiety may be a radioisotope, such as ³H, ¹⁴C, ³²P, ³⁵S or ¹²⁵I, a fluorescent or chemiluminescent compound, such as fluorescein isothiocyanate, rhodamine, or luciferin, or an enzyme, such as alkaline phosphatase, beta-galactosidase or horseradish peroxidase. Any method known in the art for conjugating the antibody to the detectable moiety may be employed, including those methods described by Hunter et al., Nature, 144:945 (1962); David et al., Biochemistry, 13: 1014 (1974); Pain et al., J. Immunol. Meth., 40:219 (1981); and Nygren, J. Histochem. and Cytochem., 30:407 (1982).

Anti-PRO antibodies also are useful for the affinity purification of PRO from recombinant cell culture or natural sources. In this process, the antibodies against PRO are immobilized on a suitable support, such a Sephadex resin or filter paper, using methods well known in the art. The immobilized antibody then is contacted with a sample containing the PRO to be purified, and thereafter the support is washed with a suitable solvent that will remove substantially all the material in the sample except the PRO, which is bound to the immobilized antibody. Finally, the support is washed with another suitable solvent that will release the PRO from the antibody.

The following examples are offered for illustrative purposes only, and are not intended to limit the scope of the present invention in any way.

All patent and literature references cited in the present specification are hereby incorporated by reference in their entirety.

### EXAMPLES

Commercially available reagents referred to in the examples were used according to manufacturer's instructions unless otherwise indicated. The source of those cells identified in the following examples, and throughout the specification, by ATCC accession numbers is the American Type Culture Collection, Manassas, VA.

### EXAMPLE 1: Extracellular Domain Homology Screening to Identify Novel Polypeptides and cDNA Encoding Therefor

The extracellular domain (ECD) sequences (including the secretion signal sequence, if any) from about 950 known secreted proteins from the Swiss-Prot public database were used to search EST databases. The EST databases included public databases (*e*.*g*., Dayhoff, GenBank), and proprietary databases (e.g. LIFESEQ™, Incyte Pharmaceuticals, Palo Alto, CA). The search was performed using the computer program BLAST or BLAST-2 (Altschul *et al*., Methods in Enzymology, 266:460-480 (1996)) as a comparison of the ECD protein sequences to a 6 frame translation of the EST sequences. Those comparisons with a BLAST score of 70 (or in some cases 90) or greater that did not encode known proteins were clustered and assembled into consensus DNA sequences with the program "phrap" (Phil Green, University of Washington, Seattle, WA).

Using this extracellular domain homology screen, consensus DNA sequences were assembled relative to the other identified EST sequences using phrap. In addition, the consensus DNA sequences obtained were often (but not always) extended using repeated cycles of BLAST or BLAST-2 and phrap to extend the consensus sequence as far as possible using the sources of EST sequences discussed above.

Based upon the consensus sequences obtained as described above, oligonucleotides were then synthesized and used to identify by PCR a cDNA library that contained the sequence of interest and for use as probes to isolate a clone of the full-length coding sequence for a PRO polypeptide. Forward and reverse PCR primers generally range from 20 to 30 nucleotides and are often designed to give a PCR product of about 100-1000 bp in length. The probe sequences are typically 40-55 bp in length. In some cases, additional oligonucleotides are synthesized when the consensus sequence is greater than about 1-1.5kbp. In order to screen several libraries for a full-length clone, DNA from the libraries was screened by PCR amplification, as per Ausubel *et al.,* Current Protocols in Molecular Biology, with the PCR primer pair. A positive library was then used to isolate clones encoding the gene of interest using the probe oligonucleotide and one of the primer pairs.

The cDNA libraries used to isolate the cDNA clones were constructed by standard methods using commercially available reagents such as those from Invitrogen, San Diego, CA. The cDNA was primed with oligo dT containing a NotI site, linked with blunt to SalI hemikinased adaptors, cleaved with NotI, sized appropriately by gel electrophoresis, and cloned in a defined orientation into a suitable cloning vector (such as pRKB or pRKD; pRK5B is a precursor of pRK5D that does not contain the SfiI site; see, Holmes *et al*., Science, 253:1278-1280 (1991)) in the unique XhoI and NotI sites.

### EXAMPLE 2: Isolation of cDNA clones by Amylase Screening

### 1. Preparation of oligo dT primed cDNA library

mRNA was isolated from a human tissue of interest using reagents and protocols from Invitrogen, San Diego, CA (Fast Track 2). This RNA was used to generate an oligo dT primed cDNA library in the vector pRK5D using reagents and protocols from Life Technologies, Gaithersburg, MD (Super Script Plasmid System). In this procedure, the double stranded cDNA was sized to greater than 1000 bp and the SalI/NotI linkered cDNA was cloned into XhoI/NotI cleaved vector. pRK5D is a cloning vector that has an sp6 transcription initiation site followed by an SfiI restriction enzyme site preceding the XhoI/NotI cDNA cloning sites.

### 2. Preparation of random primed cDNA library

A secondary cDNA library was generated in order to preferentially represent the 5' ends of the primary cDNA clones. Sp6 RNA was generated from the primary library (described above), and this RNA was used to generate a random primed cDNA library in the vector pSST-AMY.0 using reagents and protocols from Life Technologies (Super Script Plasmid System, referenced above). In this procedure the double stranded cDNA was sized to 500-1000 bp, linkered with blunt to NotI adaptors, cleaved with SfiI, and cloned into SfiI/NotI cleaved vector. pSST-AMY.0 is a cloning vector that has a yeast alcohol dehydrogenase promoter preceding the cDNA cloning sites and the mouse amylase sequence (the mature sequence without the secretion signal) followed by the yeast alcohol dehydrogenase terminator, after the cloning sites. Thus, cDNAs cloned into this vector that are fused in frame with amylase sequence will lead to the secretion of amylase from appropriately transfected yeast colonies.

### 3. Transformation and Detection

DNA from the library described in paragraph 2 above was chilled on ice to which was added electrocompetent DH10B bacteria (Life Technologies, 20 ml). The bacteria and vector mixture was then electroporated as recommended by the manufacturer. Subsequently, SOC media (Life Technologies, 1 ml) was added and the mixture was incubated at 37°C for 30 minutes. The transformants were then plated onto 20 standard 150 mm LB plates containing ampicillin and incubated for 16 hours (37°C). Positive colonies were scraped off the plates and the DNA was isolated from the bacterial pellet using standard protocols, e.g. CsCl-gradient. The purified DNA was then carried on to the yeast protocols below.

The yeast methods were divided into three categories: (1) Transformation of yeast with the plasmid/cDNA combined vector; (2) Detection and isolation of yeast clones secreting amylase; and (3) PCR amplification of the insert directly from the yeast colony and purification of the DNA for sequencing and further analysis.

The yeast strain used was HD56-5A (ATCC-90785). This strain has the following genotype: MAT alpha, ura3-52, leu2-3, leu2-112, his3-11, his3-15, MAL⁺, SUC⁺, GAL⁺. Preferably, yeast mutants can be employed that have deficient post-translational pathways. Such mutants may have translocation deficient alleles in *sec*71, sec72, *sec*62, with truncated *sec71* being most preferred. Alternatively, antagonists (including antisense nucleotides and/or ligands) which interfere with the normal operation of these genes, other proteins implicated in this post translation pathway (e.g., SEC61p, SEC72p, SEC62p, SEC63p, TDJ1p or SSAlp-4p) or the complex formation of these proteins may also be preferably employed in combination with the amylase-expressing yeast.

Transformation was performed based on the protocol outlined by Gietz *et al*., Nucl. Acid. Res., 20:1425 (1992). Transformed cells were then inoculated from agar into YEPD complex media broth (100 ml) and grown overnight at 30°C. The YEPD broth was prepared as described in Kaiser *et al*., Methods in Yeast Genetics, Cold Spring Harbor Press, Cold Spring Harbor, NY, p. 207 (1994). The overnight culture was then diluted to about 2 x 10⁶ cells/ml (approx. OD₆₀₀=0.1) into fresh YEPD broth (500 ml) and regrown to 1 x 10⁷ cells/ml (approx. OD₆₀₀=0.4-0.5).

The cells were then harvested and prepared for transformation by transfer into GS3 rotor bottles in a Sorval GS3 rotor at 5,000 rpm for 5 minutes, the supernatant discarded, and then resuspended into sterile water, and centrifuged again in 50 ml falcon tubes at 3,500 rpm in a Beckman GS-6KR centrifuge. The supernatant was discarded and the cells were subsequently washed with LiAc/TE (10 ml, 10 mM Tris-HCl, 1 mM EDTA pH 7.5, 100 mM Li₂OOCCH₃), and resuspended into LiAc/TE (2.5 ml).

Transformation took place by mixing the prepared cells (100 *µ*l) with freshly denatured single stranded salmon testes DNA (Lofstrand Labs, Gaithersburg, MD) and transforming DNA (1 µg, vol. < 10 µl) in microfuge tubes. The mixture was mixed briefly by vortexing, then 40% PEG/TE (600 µl, 40% polyethylene glycol-4000, 10 mM Tris-HCl, I mM EDTA, 100 mM Li₂OOCCH₃, pH 7.5) was added. This mixture was gently mixed and incubated at 30°C while agitating for 30 minutes. The cells were then heat shocked at 42°C for 15 minutes, and the reaction vessel centrifuged in a microfuge at 12,000 rpm for 5-10 seconds, decanted and resuspended into TE (500 *µ*l, 10 mM Tris-HCl, 1 mM EDTA pH 7.5) followed by recentrifugation. The cells were then diluted into TE (1 ml) and aliquots (200 *µ*l) were spread onto the selective media previously prepared in 150 mm growth plates (VWR).

Alternatively, instead of multiple small reactions, the transformation was performed using a single, large scale reaction, wherein reagent amounts were scaled up accordingly.

The selective media used was a synthetic complete dextrose agar lacking uracil (SCD-Ura) prepared as described in Kaiser *et al. ,* Methods in Yeast Genetics, Cold Spring Harbor Press, Cold Spring Harbor, NY, p. 208-210 (1994). Transformants were grown at 30°C for 2-3 days.

The detection of colonies secreting amylase was performed by including red starch in the selective growth media. Starch was coupled to the red dye (Reactive Red-120, Sigma) as per the procedure described by Biely *et al.,* Anal. Biochem., 172: 176-179 (1988). The coupled starch was incorporated into the SCD-Ura agar plates at a final concentration of 0.15 % (w/v), and was buffered with potassium phosphate to a pH of 7.0 (50-100 mM final concentration).

The positive colonies were picked and streaked across fresh selective media (onto 150 mm plates) in order to obtain well isolated and identifiable single colonies. Well isolated single colonies positive for amylase secretion were detected by direct incorporation of red starch into buffered SCD-Ura agar. Positive colonies were determined by their ability to break down starch resulting in a clear halo around the positive colony visualized directly.

### 4. Isolation of DNA by PCR Amplification

When a positive colony was isolated, a portion of it was picked by a toothpick and diluted into sterile water (30 µl) in a 96 well plate. At this time, the positive colonies were either frozen and stored for subsequent analysis or immediately amplified. An aliquot of cells (5 µl) was used as a template for the PCR reaction in a 25 µl volume containing: 0.5 µl Klentaq (Clontech, Palo Alto, CA); 4.0 µl 10 mM dNTP's (Perkin Elmer-Cetus); 2.5 µl Kentaq buffer (Clontech); 0.25 µl forward oligo 1; 0.25 µl reverse oligo 2; 12.5 µl distilled water. The sequence of the forward oligonucleotide 1 was: 5'-TGTAAAACGACGGCCAGTTAAATAGACCTGCAATTATTAATCT-3' (SEQ ID NO:245) The sequence of reverse oligonucleotide 2 was: 5'-CAGGAAACAGCTATGACCACCTGCACACCTGCAAATCCATT-3' (SEQ ID NO:246) PCR was then performed as follows:

| | | | | |
|---|---|---|---|---|
| a. | | Denature | 92°C, | 5 minutes |
| b. | 3 cycles of: | Denature | 92°C, | 30 seconds |
| | | Anneal | 59°C, | 30 seconds |
| | | Extend | 72°C, | 60 seconds |
| c. | 3 cycles of: | Denature | 92°C, | 30 seconds |
| | | Anneal | 57°C, | 30 seconds |
| | | Extend | 72°C, | 60 seconds |
| d. | 25 cycles of: | Denature | 92°C, | 30 seconds |
| | | Anneal | 55°C, | 30 seconds |
| | | Extend | 72°C, | 60 seconds |
| e. | | Hold | 4°C | |

The underlined regions of the oligonucleotides annealed to the ADH promoter region and the amylase region, respectively, and amplified a 307 bp region from vector pSST-AMY.0 when no insert was present. Typically, the first 18 nucleotides of the 5' end of these oligonucleotides contained annealing sites for the sequencing primers. Thus, the total product of the PCR reaction from an empty vector was 343 bp. However, signal sequence-fused cDNA resulted in considerably longer nucleotide sequences.

Following the PCR, an aliquot of the reaction (5 *µ*l) was examined by agarose gel electrophoresis in a 1% agarose gel using a Tris-Borate-EDTA (TBE) buffering system as described by Sambrook *et al*., *supra*. Clones resulting in a single strong PCR product larger than 400 bp were further analyzed by DNA sequencing after purification with a 96 Qiaquick PCR clean-up column (Qiagen Inc., Chatsworth, CA).

### EXAMPLE 3: Isolation of cDNA Clones Using Signal Algorithm Analysis

Various polypeptide-encoding nucleic acid sequences were identified by applying a proprietary signal sequence finding algorithm developed by Genentech, Inc. (South San Francisco. CA) upon ESTs as well as clustered and assembled EST fragments from public (e.g., GenBank) and/or private (LIFESEQ®, Incyte Pharmaceuticals, Inc., Palo Alto, CA) databases. The signal sequence algorithm computes a secretion signal score based on the character of the DNA nucleotides surrounding the first and optionally the second methionine codon(s) (ATG) at the 5'-end of the sequence or sequence fragment under consideration. The nucleotides following the first ATG must code for at least 35 unambiguous amino acids without any stop codons. If the first ATG has the required amino acids, the second is not examined. If neither meets the requirement, the candidate sequence is not scored. In order to determine whether the EST sequence contains an authentic signal sequence, the DNA and corresponding amino acid sequences surrounding the ATG codon are scored using a set of seven sensors (evaluation parameters) known to be associated with secretion signals. Use of this algorithm resulted in the identification of numerous polypeptide-encoding nucleic acid sequences.

### EXAMPLE 4: Isolation of cDNA clones Encoding Human PRO Polypeptides

Using the techniques described in Examples 1 to 3 above, numerous full-length cDNA clones were identified as encoding PRO polypeptides as disclosed herein. These cDNAs were then deposited under the terms of the Budapest Treaty with the American Type Culture Collection, 10801 University Blvd., Manassas, VA 20110-2209, USA (ATCC) as shown in Table 7 below.

**Table 7**

| Material | ATCC Dep. No. | Deposit Date |
|---|---|---|
| DNA94849-2960 | PTA-2306 | July 25, 2000 |
| DNA96883-2745 | PTA-544 | August 17, 1999 |
| DNA96894-2675 | PTA-260 | June 22, 1999 |
| DNA100272-2969 | PTA-2299 | July 25, 2000 |
| DNA108696-2966 | PTA-2315 | August 1, 2000 |
| DNA117935-2801 | PTA-1088 | December 22, 1999 |
| DNA119474-2803 | PTA-1097 | December 22, 1999 |
| DNA119498-2965 | PTA-2298 | July 25, 2000 |
| DNA119502-2789 | PTA-1082 | December 22, 1999 |
| DNA119516-2797 | PTA-1083 | December 22, 1999 |
| DNA119530-2968 | PTA-2396 | August 8, 2000 |
| DNA121772-2741 | PTA-1030 | December 7, 1999 |
| DNA125148-2782 | PTA-955 | November 16, 1999 |
| DNA125150-2793 | PTA-1085 | December 22, 1999 |
| DNA125151-2784 | PTA-1029 | December 7, 1999 |
| DNA125181-2804 | PTA-1096 | December 22, 1999 |
| DNA125192-2794 | PTA-1086 | December 22, 1999 |
| DNA125196-2792 | PTA-1091 | December 22, 1999 |
| DNA125200-2810 | PTA-1186 | January 11, 2000 |
| DNA125214-2814 | PTA-1270 | February 2, 2000 |
| DNA125219-2799 | PTA-1084 | December 22, 1999 |
| DNA128309-2825 | PTA-1340 | February 8, 2000 |
| DNA129535-2796 | PTA-1087 | December 22, 1999 |
| DNA129549-2798 | PTA-1099 | December 22, 1999 |
| DNA129580-2863 | PTA-1584 | March 28, 2000 |
| DNA129794-2967 | PTA-2305 | July 25, 2000 |
| DNA131590-2962 | PTA-2297 | July 25, 2000 |
| DNA135173-2811 | PTA-1184 | January 11, 2000 |
| DNA138039-2828 | PTA-1343 | February 8, 2000 |
| DNA139540-2807 | PTA-1187 | January 11, 2000 |
| DNA139602-2859 | PTA-1588 | March 28, 2000 |
| DNA139632-2880 | PTA-1629 | April 4, 2000 |
| DNA139686-2823 | PTA-1264 | February 2, 2000 |
| DNA142392-2800 | PTA-1092 | December 22, 1999 |
| DNA143076-2787 | PTA-1028 | December 7, 1999 |
| DNA143294-2818 | PTA-1182 | January 11, 2000 |
| DNA143514-2817 | PTA-1266 | February 2, 2000 |
| DNA144841-2816 | PTA-1188 | January 11, 2000 |
| DNA148380-2827 | PTA-1181 | January 11, 2000 |
| DNA149995-2871 | PTA-1971 | May 31, 2000 |
| DNA167678-2963 | PTA-2302 | July 25, 2000 |
| DNA168028-2956 | PTA-2304 | July 25, 2000 |
| DNA173894-2947 | PTA-2108 | June 20, 2000 |
| DNA176775-2957 | PTA-2303 | July 25, 2000 |
| DNA177313-2982 | PTA-2251 | July 19, 2000 |
| DNA57700-1408 | 203583 | January 12, 1999 |
| DNA62872-1509 | 203100 | August 4, 1998 |
| DNA62876-1517 | 203095 | August 4, 1998 |
| DNA66660-1585 | 203279 | September 22, 1998 |
| DNA34434-1139 | 209252 | September 16, 1997 |
| DNA44804-1248 | 209527 | December 10, 1997 |
| DNA52758-1399 | 209773 | April 14, 1998 |
| DNA59849-1504 | 209986 | June 16, 1998 |
| DNA65410-1569 | 203231 | September 15, 1998 |
| DNA71290-1630 | 203275 | September 22, 1998 |
| DNA33100-1159 | 209377 | October 16, 1997 |
| DNA64896-1539 | 203238 | September 9, 1998 |
| DNA84920-2614 | 203966 | April 27, 1999 |
| DNA23330-1390 | 209775 | April 14, 1998 |
| DNA32286-1191 | 209385 | October 16, 1997 |
| DNA35673-1201 | 209418 | October 28, 1997 |
| DNA43316-1237 | 209487 | November 21, 1997 |
| DNA44184-1319 | 209704 | March 26, 1998 |
| DNA45419-1252 | 209616 | February 5, 1998 |
| DNA48314-1320 | 209702 | March 26, 1998 |
| DNA50921-1458 | 209859 | May 12, 1998 |
| DNA53987 | 209858 | May 12, 1998 |
| DNA56047-1456 | 209948 | June 9, 1998 |
| DNA56405-1357 | 209849 | May 6, 1998 |
| DNA56531-1648 | 203286 | September 29, 1998 |
| DNA56865-1491 | 203022 | June 23, 1998 |
| DNA57694-1341 | 203017 | June 23, 1998 |
| DNA57708-1411 | 203021 | June 23, 1998 |
| DNA57836-1338 | 203025 | June 23, 1998 |
| DNA57841-1522 | 203458 | November 3, 1998 |
| DNA58847-1383 | 209879 | May 20, 1998 |
| DNA59212-1627 | 203245 | September 9, 1998 |
| DNA59588-1571 | 203106 | August 11, 1998 |
| DNA59622-1334 | 209984 | June 16, 1998 |
| DNA59847-2510 | 203576 | January 12, 1999 |
| DNA60615-1483 | 209980 | June 16, 1998 |
| DNA60621-1516 | 203091 | August 4, 1998 |
| DNA62814-1521 | 203093 | August 4, 1998 |
| DNA64883-1526 | 203253 | September 9, 1998 |
| DNA64889-1541 | 203250 | September 9, 1998 |
| DNA64897-1628 | 203216 | September 15, 1998 |
| DNA64903-1553 | 203223 | September 15, 1998 |
| DNA64907-1163-1 | 203242 | September 9, 1998 |
| DNA64950-1590 | 203224 | September 15, 1998 |
| DNA64952-1568 | 203222 | September 15, 1998 |
| DNA65402-1540 | 203252 | September 9, 1998 |
| DNA65405-1547 | 203476 | November 17, 1998 |
| DNA66663-1598 | 203268 | September 22, 1998 |
| DNA66667 | 203267 | September 22, 1998 |
| DNA66675-1587 | 203282 | September 22, 1998 |
| DNA67300-1605 | 203163 | August 25, 1998 |
| DNA68872-1620 | 203160 | August 25, 1998 |
| DNA71269-1621 | 203284 | September 22, 1998 |
| DNA73736-1657 | 203466 | November 17, 1998 |
| DNA73739-1645 | 203270 | September 22, 1998 |
| DNA76400-2528 | 203573 | January 12, 1999 |
| DNA76532-1702 | 203473 | November 17, 1998 |
| DNA76541-1675 | 203409 | October 27, 1998 |
| DNA79862-2522 | 203550 | December 22, 1998 |
| DNA81754-2532 | 203542 | December 15, 1998 |
| DNA81761-2583 | 203862 | March 23, 1999 |
| DNA83500-2506 | 203391 | October 29, 1998 |
| DNA84210-2576 | 203818 | March 2, 1999 |
| DNA86571-2551 | 203660 | February 9, 1999 |
| DNA92218-2554 | 203834 | March 9, 1999 |
| DNA92223-2567 | 203851 | March 16, 1999 |
| DNA92265-2669 | PTA-256 | June 22, 1999 |
| DNA92274-2617 | 203971 | April 27, 1999 |
| DNA108760-2740 | PTA-548 | August 17, 1999 |
| DNA108792-2753 | PTA-617 | August 31, 1999 |
| DNA111750-2706 | PTA-489 | August 3, 1999 |
| DNA119514-2772 | PTA-946 | November 9, 1999 |
| DNA125185-2806 | PTA-1031 | December 7, 1999 |

These deposits were made under the provisions of the Budapest Treaty on the International Recognition of the Deposit of Microorganisms for the Purpose of Patent Procedure and the Regulations thereunder (Budapest Treaty). This assures maintenance of a viable culture of the deposit for 30 years from the date of deposit. The deposits will be made available by ATCC under the terms of the Budapest Treaty, and subject to an agreement between Genentech, Inc. and ATCC, which assures permanent and unrestricted availability of the progeny of the culture of the deposit to the public upon issuance of the pertinent U.S. patent or upon laying open to the public of any U.S. or foreign patent application, whichever comes first, and assures availability of the progeny to one determined by the U.S. Commissioner of Patents and Trademarks to be entitled thereto according to 35 USC § 122 and the Commissioner's rules pursuant thereto (including 37 CFR § 1.14 with particular reference to 886 OG 638).

The assignee of the present application has agreed that if a culture of the materials on deposit should die or be lost or destroyed when cultivated under suitable conditions, the materials will be promptly replaced on notification with another of the same. Availability of the deposited material is not to be construed as a license to practice the invention in contravention of the rights granted under the authority of any government in accordance with its patent laws.

### EXAMPLE 5: Isolation of cDNA clones Encoding Human PRO6004, PRO5723, PRO3444, and PRO9940

DNA molecules encoding the PRO840, PRO1338, PRO6004, PRO5723, PRO3444, and PRO9940 polypeptides shown in the accompanying figures were obtained through GenBank.

### EXAMPLE 6: Use of PRO as a hybridization probe

The following method describes use of a nucleotide sequence encoding PRO as a hybridization probe.

DNA comprising the coding sequence of full-length or mature PRO as disclosed herein is employed as a probe to screen for homologous DNAs (such as those encoding naturally-occurring variants of PRO) in human tissue cDNA libraries or human tissue genomic libraries.

Hybridization and washing of filters containing either library DNAs is performed under the following high stringency conditions. Hybridization of radiolabeled PRO-derived probe to the filters is performed in a solution of 50% formamide, 5x SSC, 0.1 % SDS, 0.1 % sodium pyrophosphate, 50 mM sodium phosphate, pH 6.8, 2x Denhardt's solution, and 10% dextran sulfate at 42°C for 20 hours. Washing of the filters is performed in an aqueous solution of 0.1x SSC and 0.1% SDS at 42°C.

DNAs having a desired sequence identity with the DNA encoding full-length native sequence PRO can then be identified using standard techniques known in the art.

### EXAMPLE 7: Expression of PRO in E. coli

This example illustrates preparation of an unglycosylated form of PRO by recombinant expression in E. *coli.*

The DNA sequence encoding PRO is initially amplified using selected PCR primers. The primers should contain restriction enzyme sites which correspond to the restriction enzyme sites on the selected expression vector. A variety of expression vectors may be employed. An example of a suitable vector is pBR322 (derived from *E. coli;* see Bolivar et al., Gene, 2:95 (1977)) which contains genes for ampicillin and tetracycline resistance. The vector is digested with restriction enzyme and dephosphorylated. The PCR amplified sequences are then ligated into the vector. The vector will preferably include sequences which encode for an antibiotic resistance gene, a trp promoter, a polyhis leader (including the first six STII codons, polyhis sequence, and enterokinase cleavage site), the PRO coding region, lambda transcriptional terminator, and an argU gene.

The ligation mixture is then used to transform a selected E. *coli* strain using the methods described in Sambrook et al., supra. Transformants are identified by their ability to grow on LB plates and antibiotic resistant colonies are then selected. Plasmid DNA can be isolated and confirmed by restriction analysis and DNA sequencing.

Selected clones can be grown overnight in liquid culture medium such as LB broth supplemented with antibiotics. The overnight culture may subsequently be used to inoculate a larger scale culture. The cells are then grown to a desired optical density, during which the expression promoter is turned on.

After culturing the cells for several more hours, the cells can be harvested by centrifugation. The cell pellet obtained by the centrifugation can be solubilized using various agents known in the art, and the solubilized PRO protein can then be purified using a metal chelating column under conditions that allow tight binding of the protein.

PRO may be expressed in *E. coli* in a poly-His tagged form, using the following procedure. The DNA encoding PRO is initially amplified using selected PCR primers. The primers will contain restriction enzyme sites which correspond to the restriction enzyme sites on the selected expression vector, and other useful sequences providing for efficient and reliable translation initiation, rapid purification on a metal chelation column, and proteolytic removal with enterokinase. The PCR-amplified, poly-His tagged sequences are then ligated into an expression vector, which is used to transform an E. *coli* host based on strain 52 (W3110 fuhA(tonA) Ion galE rpoHts(htpRts) clpP(lacIq). Transformants are first grown in LB containing 50 mg/ml carbenicillin at 30°C with shaking until an O.D.600 of 3-5 is reached. Cultures are then diluted 50-100 fold into CRAP media (prepared by mixing 3.57 g (NH₄)₂SO₄, 0.71 g sodium citrate·2H2O, 1.07 g KCI, 5.36 g Difco yeast extract, 5.36 g Sheffield hycase SF in 500 mL water, as well as 110 mM MPOS, pH 7.3, 0.55% (w/v) glucose and 7 mM MgSO₄) and grown for approximately 20-30 hours at 30°C with shaking. Samples are removed to verify expression by SDS-PAGE analysis, and the bulk culture is centrifuged to pellet the cells. Cell pellets are frozen until purification and refolding.

*E, coli* paste from 0.5 to 1 L fermentations (6-10 g pellets) is resuspended in 10 volumes (w/v) in 7 M guanidine, 20 mM Tris, pH 8 buffer. Solid sodium sulfite and sodium tetrathionate is added to make final concentrations of 0.1M and 0.02 M, respectively, and the solution is stirred overnight at 4°C. This step results in a denatured protein with all cysteine residues blocked by sulfitolization. The solution is centrifuged at 40,000 rpm in a Beckman Ultracentifuge for 30 min. The supernatant is diluted with 3-5 volumes of metal chelate column buffer (6 M guanidine, 20 mM Tris, pH 7.4) and filtered through 0.22 micron filters to clarify. The clarified extract is loaded onto a 5 ml Qiagen Ni-NTA metal chelate column equilibrated in the metal chelate column buffer. The column is washed with additional buffer containing 50 mM imidazole (Calbiochem, Utrol grade), pH 7.4. The protein is eluted with buffer containing 250 mM imidazole. Fractions containing the desired protein are pooled and stored at 4°C. Protein concentration is estimated by its absorbance at 280 nm using the calculated extinction coefficient based on its amino acid sequence.

The proteins are refolded by diluting the sample slowly into freshly prepared refolding buffer consisting of: 20 mM Tris, pH 8.6, 0.3 M NaCl, 2.5 M urea, 5 mM cysteine, 20 mM glycine and 1 mM EDTA. Refolding volumes are chosen so that the final protein concentration is between 50 to 100 micrograms/ml. The refolding solution is stirred gently at 4°C for 12-36 hours. The refolding reaction is quenched by the addition of TFA to a final concentration of 0.4 % (pH of approximately 3). Before further purification of the protein, the solution is filtered through a 0.22 micron filter and acetonitrile is added to 2-10% final concentration. The refolded protein is chromatographed on a Poros R1/H reversed phase column using a mobile buffer of 0.1 % TFA with elution with a gradient of acetonitrile from 10 to 80%. Aliquots of fractions with A280 absorbance are analyzed on SDS polyacrylamide gels and fractions containing homogeneous refolded protein are pooled. Generally, the properly refolded species of most proteins are eluted at the lowest concentrations of acetonitrile since those species are the most compact with their hydrophobic interiors shielded from interaction with the reversed phase resin. Aggregated species are usually eluted at higher acetonitrile concentrations. In addition to resolving misfolded forms of proteins from the desired form, the reversed phase step also removes endotoxin from the samples.

Fractions containing the desired folded PRO polypeptide are pooled and the acetonitrile removed using a gentle stream of nitrogen directed at the solution. Proteins are formulated into 20 mM Hepes, pH 6.8 with 0.14 M sodium chloride and 4% mannitol by dialysis or by gel filtration using G25 Superfine (Pharmacia) resins equilibrated in the formulation buffer and sterile filtered.

Many of the PRO polypeptides disclosed herein were successfully expressed as described above.

### EXAMPLE 8: Expression of PRO in mammalian cells

This example illustrates preparation of a potentially glycosylated form of PRO by recombinant expression in mammalian cells.

The vector, pRK5 (see EP 307,247, published March 15, 1989), is employed as the expression vector. Optionally, the PRO DNA is ligated into pRK5 with selected restriction enzymes to allow insertion of the PRO DNA using ligation methods such as described in Sambrook et al., supra. The resulting vector is called pRK5-PRO.

In one embodiment, the selected host cells may be 293 cells. Human 293 cells (ATCC CCL 1573) are grown to confluence in tissue culture plates in medium such as DMEM supplemented with fetal calf serum and optionally, nutrient components and/or antibiotics. About 10 *µ*g pRK5-PRO DNA is mixed with about 1 *µ*g DNA encoding the VA RNA gene [Thimmappaya et al., Cell, 31:543 (1982)] and dissolved in 500 *µ*l of 1 mM Tris-HCl, 0.1 mM EDTA, 0.227 M CaCl₂. To this mixture is added, dropwise, 500 *µ*l of 50 mM HEPES (pH 7.35), 280 mM NaCl, 1.5 mM NaPO₄, and a precipitate is allowed to form for 10 minutes at 25°C. The precipitate is suspended and added to the 293 cells and allowed to settle for about four hours at 37°C. The culture medium is aspirated off and 2 ml of 20 % glycerol in PBS is added for 30 seconds. The 293 cells are then washed with serum free medium, fresh medium is added and the cells are incubated for about 5 days.

Approximately 24 hours after the transfections, the culture medium is removed and replaced with culture medium (alone) or culture medium containing 200 *µ*Ci/ml ³⁵S-cysteine and 200 /*µ*Ci/ml ³⁵S-methionine. After a 12 hour incubation, the conditioned medium is collected, concentrated on a spin filter, and loaded onto a 15 % SDS gel. The processed gel may be dried and exposed to film for a selected period of time to reveal the presence of PRO polypeptide. The cultures containing transfected cells may undergo further incubation (in serum free medium) and the medium is tested in selected bioassays.

In an alternative technique, PRO may be introduced into 293 cells transiently using the dextran sulfate method described by Somparyrac et al., Proc. Natl. Acad. Sci., 12:7575 (1981). 293 cells are grown to maximal density in a spinner flask and 700 *µ*g pRK5-PRO DNA is added. The cells are first concentrated from the spinner flask by centrifugation and washed with PBS. The DNA-dextran precipitate is incubated on the cell pellet for four hours. The cells are treated with 20% glycerol for 90 seconds, washed with tissue culture medium, and reintroduced into the spinner flask containing tissue culture medium, 5 µg/ml bovine insulin and 0.1 µg/ml bovine transferrin. After about four days, the conditioned media is centrifuged and filtered to remove cells and debris. The sample containing expressed PRO can then be concentrated and purified by any selected method, such as dialysis and/or column chromatography.

In another embodiment, PRO can be expressed in CHO cells. The pRK5-PRO can be transfected into CHO cells using known reagents such as CaPO₄ or DEAE-dextran. As described above, the cell cultures can be incubated, and the medium replaced with culture medium (alone) or medium containing a radiolabel such as ³⁵S-methionine. After determining the presence of PRO polypeptide, the culture medium may be replaced with serum free medium. Preferably, the cultures are incubated for about 6 days, and then the conditioned medium is harvested. The medium containing the expressed PRO can then be concentrated and purified by any selected method.

Epitope-tagged PRO may also be expressed in host CHO cells. The PRO may be subcloned out of the pRK5 vector. The subclone insert can undergo PCR to fuse in frame with a selected epitope tag such as a poly-his tag into a Baculovirus expression vector. The poly-his tagged PRO insert can then be subcloned into a SV40 driven vector containing a selection marker such as DHFR for selection of stable clones. Finally, the CHO cells can be transfected (as described above) with the SV40 driven vector. Labeling may be performed, as described above, to verify expression. The culture medium containing the expressed poly-His tagged PRO can then be concentrated and purified by any selected method, such as by Ni²⁺-chelate affinity chromatography.

PRO may also be expressed in CHO and/or COS cells by a transient expression procedure or in CHO cells by another stable expression procedure.

Stable expression in CHO cells is performed using the following procedure. The proteins are expressed as an IgG construct (immunoadhesin), in which the coding sequences for the soluble forms (e.g. extracellular domains) of the respective proteins are fused to an IgGl constant region sequence containing the hinge, CH2 and CH2 domains and/or is a poly-His tagged form.

Following PCR amplification, the respective DNAs are subcloned in a CHO expression vector using standard techniques as described in Ausubel et al., Current Protocols of Molecular Biology, Unit 3.16, John Wiley and Sons (1997). CHO expression vectors are constructed to have compatible restriction sites 5' and 3' of the DNA of interest to allow the convenient shuttling of cDNA's. The vector used expression in CHO cells is as described in Lucas et al., Nucl. Acids Res. 24:9 (1774-1779 (1996), and uses the SV40 early promoter/enhancer to drive expression of the cDNA of interest and dihydrofolate reductase (DHFR). DHFR expression permits selection for stable maintenance of the plasmid following transfection.

Twelve micrograms of the desired plasmid DNA is introduced into approximately 10 million CHO cells using commercially available transfection reagents Superfect* (Qiagen), Dosper* or Fugene* (Boehringer Mannheim). The cells are grown as described in Lucas et al., supra. Approximately 3 × 10⁷ cells are frozen in an ampule for further growth and production as described below.

The ampules containing the plasmid DNA are thawed by placement into water bath and mixed by vortexing. The contents are pipetted into a centrifuge tube containing 10 mLs of media and centrifuged at 1000 rpm for 5 minutes. The supernatant is aspirated and the cells are resuspended in 10 mL of selective media (0.2 µm filtered PS20 with 5 % 0.2 µm diafiltered fetal bovine serum). The cells are then aliquoted into a 100 mL spinner containing 90 mL of selective media. After 1-2 days, the cells are transferred into a 250 mL spinner filled with 150 mL selective growth medium and incubated at 37°C. After another 2-3 days, 250 mL, 500 mL and 2000 mL spinners are seeded with 3 x 10⁵ cells/mL. The cell media is exchanged with fresh media by centrifugation and resuspension in production medium. Although any suitable CHO media may be employed, a production medium described in U.S. Patent No. 5,122,469, issued June 16, 1992 may actually be used. A 3L production spinner is seeded at 1.2 x 10⁶ cells/mL. On day 0, the cell number pH ie determined. On day 1, the spinner is sampled and sparging with filtered air is commenced. On day 2, the spinner is sampled, the temperature shifted to 33°C, and 30 mL of 500 g/L glucose and 0.6 mL of 10% antifoam (e.g., 35 % polydimethylsiloxane emulsion, Dow Corning 365 Medical Grade Emulsion) taken. Throughout the production, the pH is adjusted as necessary to keep it at around 7.2. After 10 days, or until the viability dropped below 70% , the cell culture is harvested by centrifugation and filtering through a 0.22 µm filter. The filtrate was either stored at 4°C or immediately loaded onto columns for purification.

For the poly-His tagged constructs, the proteins are purified using a Ni-NTA column (Qiagen). Before purification, imidazole is added to the conditioned media to a concentration of 5 mM. The conditioned media is pumped onto a 6 ml Ni-NTA column equilibrated in 20 mM Hepes, pH 7.4, buffer containing 0.3 M NaCl and 5 mM imidazole at a flow rate of 4-5 ml/min, at 4°C. After loading, the column is washed with additional equilibration buffer and the protein eluted with equilibration buffer containing 0.25 M imidazole. The highly purified protein is subsequently desalted into a storage buffer containing 10 mM Hepes, 0.14 M NaCl and 4% mannitol, pH 6.8, with a 25 ml G25 Superfine (Pharmacia) column and stored at -80°C.

Immunoadhesin (Fc-containing) constructs are purified from the conditioned media as follows. The conditioned medium is pumped onto a 5 ml Protein A column (Pharmacia) which had been equilibrated in 20 mM Na phosphate buffer, pH 6.8. After loading, the column is washed extensively with equilibration buffer before elution with 100 mM citric acid, pH 3.5. The eluted protein is immediately neutralized by collecting 1 ml fractions into tubes containing 275 *µ*L of 1 M Tris buffer, pH 9. The highly purified protein is subsequently desalted into storage buffer as described above for the poly-His tagged proteins. The homogeneity is assessed by SDS polyacrylamide gels and by N-terminal amino acid sequencing by Edman degradation.

Many of the PRO polypeptides disclosed herein were successfully expressed as described above.

### EXAMPLE 9: Expression of PRO in Yeast

The following method describes recombinant expression of PRO in yeast.

First, yeast expression vectors are constructed for intracellular production or secretion of PRO from the ADH2/GAPDH promoter. DNA encoding PRO and the promoter is inserted into suitable restriction enzyme sites in the selected plasmid to direct intracellular expression of PRO. For secretion, DNA encoding PRO can be cloned into the selected plasmid, together with DNA encoding the ADH2/GAPDH promoter, a native PRO signal peptide or other mammalian signal peptide, or, for example, a yeast alpha-factor or invertase secretory signal/leader sequence, and linker sequences (if needed) for expression of PRO.

Yeast cells, such as yeast strain AB 110, can then be transformed with the expression plasmids described above and cultured in selected fermentation media. The transformed yeast supernatants can be analyzed by precipitation with 10% trichloroacetic acid and separation by SDS-PAGE, followed by staining of the gels with Coomassie Blue stain.

Recombinant PRO can subsequently be isolated and purified by removing the yeast cells from the fermentation medium by centrifugation and then concentrating the medium using selected cartridge filters. The concentrate containing PRO may further be purified using selected column chromatography resins.

Many of the PRO polypeptides disclosed herein were successfully expressed as described above.

### EXAMPLE 10: Expression of PRO in Baculovirus-Infected Insect Cells

The following method describes recombinant expression of PRO in Baculovirus-infected insect cells.

The sequence coding for PRO is fused upstream of an epitope tag contained within a baculovirus expression vector. Such epitope tags include poly-his tags and immunoglobulin tags (like Fc regions of IgG). A variety of plasmids may be employed, including plasmids derived from commercially available plasmids such as pVL1393 (Novagen). Briefly, the sequence encoding PRO or the desired portion of the coding sequence of PRO such as the sequence encoding the extracellular domain of a transmembrane protein or the sequence encoding the mature protein if the protein is extracellular is amplified by PCR with primers complementary to the 5' and 3' regions. The 5' primer may incorporate flanking (selected) restriction enzyme sites. The product is then digested with those selected restriction enzymes and subcloned into the expression vector.

Recombinant baculovirus is generated by co-transfecting the above plasmid and BaculoGold^{TM} virus DNA (Pharmingen) into *Spodoptera frugiperda* ("Sf9") cells (ATCC CRL 1711) using lipofectin (commercially available from GIBCO-BRL). After 4 - 5 days of incubation at 28°C, the released viruses are harvested and used for further amplifications. Viral infection and protein expression are performed as described by O'Reilley et al., Baculovirus expression vectors: A Laboratory Manual, Oxford: Oxford University Press (1994).

Expressed poly-his tagged PRO can then be purified, for example, by Ni²⁺-chelate affinity chromatography as follows. Extracts are prepared from recombinant virus-infected Sf9 cells as described by Rupert et al., Nature, 362:175-179 (1993). Briefly, Sf9 cells are washed, resuspended in sonication buffer (25 mL Hepes, pH 7.9; 12.5 mM MgCl₂; 0.1 mM EDTA; 10% glycerol; 0.1 % NP-40; 0.4 M KCl), and sonicated twice for 20 seconds on ice. The sonicates are cleared by centrifugation, and the supernatant is diluted 50-fold in loading buffer (50 mM phosphate, 300 mM NaCl, 10% glycerol, pH 7.8) and filtered through a 0.45 µm filter. A Ni²⁺-NTA agarose column (commercially available from Qiagen) is prepared with a bed volume of 5 mL, washed with 25 mL of water and equilibrated with 25 mL of loading buffer. The filtered cell extract is loaded onto the column at 0.5 mL per minute. The column is washed to baseline A₂₈₀ with loading buffer, at which point fraction collection is started. Next, the column is washed with a secondary wash buffer (50 mM phosphate; 300 mM NaCl, 10% glycerol, pH 6.0), which elutes nonspecifically bound protein. After reaching A₂₈₀ baseline again, the column is developed with a 0 to 500 mM limidazole gradient in the secondary wash buffer. One mL fractions are collected and analyzed by SDS-PAGE and silver staining or Western blot with Ni²⁺ -NTA-conjugated to alkaline phosphatase (Qiagen). Fractions containing the eluted His₁₀-tagged PRO are pooled and dialyzed against loading buffer.

Alternatively, purification of the IgG tagged (or Fc tagged) PRO can be performed using known chromatography techniques, including for instance, Protein A or protein G column chromatography.

Many of the PRO polypeptides disclosed herein were successfully expressed as described above.

### EXAMPLE 11: Preparation of Antibodies that Bind PRO

This example illustrates preparation of monoclonal antibodies which can specifically bind PRO.

Techniques for producing the monoclonal antibodies are known in the art and are described, for instance, in Goding, supra. Immunogens that may be employed include purified PRO, fusion proteins containing PRO, and cells expressing recombinant PRO on the cell surface. Selection of the immunogen can be made by the skilled artisan without undue experimentation.

Mice, such as Balb/c, are immunized with the PRO immunogen emulsified in complete Freund's adjuvant and injected subcutaneously or intraperitoneally in an amount from 1-100 micrograms. Alternatively, the immunogen is emulsified in MPL-TDM adjuvant (Ribi Immunochemical Research, Hamilton, MT) and injected into the animal's hind foot pads. The immunized mice are then boosted 10 to 12 days later with additional immunogen emulsified in the selected adjuvant. Thereafter, for several weeks, the mice may also be boosted with additional immunization injections. Serum samples may be periodically obtained from the mice by retro-orbital bleeding for testing in ELISA assays to detect anti-PRO antibodies.

After a suitable antibody titer has been detected, the animals "positive" for antibodies can be injected with a final intravenous injection of PRO. Three to four days later, the mice are sacrificed and the spleen cells are harvested. The spleen cells are then fused (using 35 % polyethylene glycol) to a selected murine myeloma cell line such as P3X63AgU.1, available from ATCC, No. CRL 1597. The fusions generate hybridoma cells which can then be plated in 96 well tissue culture plates containing HAT (hypoxanthine, aminopterin, and thymidine) medium to inhibit proliferation of non-fused cells, myeloma hybrids, and spleen cell hybrids.

The hybridoma cells will be screened in an ELISA for reactivity against PRO. Determination of "positive" hybridoma cells secreting the desired monoclonal antibodies against PRO is within the skill in the art.

The positive hybridoma cells can be injected intraperitoneally into syngeneic Balb/c mice to produce ascites containing the anti-PRO monoclonal antibodies. Alternatively, the hybridoma cells can be grown in tissue culture flasks or roller bottles. Purification of the monoclonal antibodies produced in the ascites can be accomplished using ammonium sulfate precipitation, followed by gel exclusion chromatography. Alternatively, affinity chromatography based upon binding of antibody to protein A or protein G can be employed.

### EXAMPLE 12: Purification of PRO Polypeptides Using Specific Antibodies

Native or recombinant PRO polypeptides may be purified by a variety of standard techniques in the art of protein purification. For example, pro-PRO polypeptide, mature PRO polypeptide, or pre-PRO polypeptide is purified by immunoaffinity chromatography using antibodies specific for the PRO polypeptide of interest. In general, an immunoaffinity column is constructed by covalently coupling the anti-PRO polypeptide antibody to an activated chromatographic resin.

Polyclonal immunoglobulins are prepared from immune sera either by precipitation with ammonium sulfate or by purification on immobilized Protein A (Pharmacia LKB Biotechnology, Piscataway, N.J.). Likewise, monoclonal antibodies are prepared from mouse ascites fluid by ammonium sulfate precipitation or chromatography on immobilized Protein A. Partially purified immunoglobulin is covalently attached to a chromatographic resin such as CnBr-activated SEPHAROSE™ (Pharmacia LKB Biotechnology). The antibody is coupled to the resin, the resin is blocked, and the derivative resin is washed according to the manufacturer's instructions.

Such an immunoaffinity column is utilized in the purification of PRO polypeptide by preparing a fraction from cells containing PRO polypeptide in a soluble form. This preparation is derived by solubilization of the whole cell or of a subcellular fraction obtained via differential centrifugation by the addition of detergent or by other methods well known in the art. Alternatively, soluble PRO polypeptide containing a signal sequence may be secreted in useful quantity into the medium in which the cells are grown.

A soluble PRO polypeptide-containing preparation is passed over the immunoaffinity column, and the column is washed under conditions that allow the preferential absorbance of PRO polypeptide (e.g., high ionic strength buffers in the presence of detergent). Then, the column is eluted under conditions that disrupt antibody/PRO polypeptide binding (e. g. , a low pH buffer such as approximately pH 2-3, or a high concentration of a chaotrope such as urea or thiocyanate ion), and PRO polypeptide is collected.

### EXAMPLE 13: Drug Screening

This invention is particularly useful for screening compounds by using PRO polypeptides or binding fragment thereof in any of a variety of drug screening techniques. The PRO polypeptide or fragment employed in such a test may either be free in solution, affixed to a solid support, borne on a cell surface, or located intracellularly. One method of drug screening utilizes eukaryotic or prokaryotic host cells which are stably transformed with recombinant nucleic acids expressing the PRO polypeptide or fragment. Drugs are screened against such transformed cells in competitive binding assays. Such cells, either in viable or fixed form, can be used for standard binding assays. One may measure, for example, the formation of complexes between PRO polypeptide or a fragment and the agent being tested. Alternatively, one can examine the diminution in complex formation between the PRO polypeptide and its target cell or target receptors caused by the agent being tested.

Thus, the present invention provides methods of screening for drugs or any other agents which can affect a PRO polypeptide-associated disease or disorder. These methods comprise contacting such an agent with an PRO polypeptide or fragment thereof and assaying (1) for the presence of a complex between the agent and the PRO polypeptide or fragment, or (ii) for the presence of a complex between the PRO polypeptide or fragment and the cell, by methods well known in the art. In such competitive binding assays, the PRO polypeptide or fragment is typically labeled. After suitable incubation, free PRO polypeptide or fragment is separated from that present in bound form, and the amount of free or uncomplexed label is a measure of the ability of the particular agent to bind to PRO polypeptide or to interfere with the PRO polypeptide/cell complex.

Another technique for drug screening provides high throughput screening for compounds having suitable binding affinity to a polypeptide and is described in detail in WO 84/03564, published on September 13, 1984. Briefly stated, large numbers of different small peptide test compounds are synthesized on a solid substrate, such as plastic pins or some other surface. As applied to a PRO polypeptide, the peptide test compounds are reacted with PRO polypeptide and washed. Bound PRO polypeptide is detected by methods well known in the art. Purified PRO polypeptide can also be coated directly onto plates for use in the aforementioned drug screening techniques. In addition, non-neutralizing antibodies can be used to capture the peptide and immobilize it on the solid support.

This invention also contemplates the use of competitive drug screening assays in which neutralizing antibodies capable of binding PRO polypeptide specifically compete with a test compound for binding to PRO polypeptide or fragments thereof. In this manner, the antibodies can be used to detect the presence of any peptide which shares one or more antigenic determinants with PRO polypeptide.

### EXAMPLE 14: Rational Drug Design

The goal of rational drug design is to produce structural analogs of biologically active polypeptide of interest *(i.e. ,* a PRO polypeptide) or of small molecules with which they interact, *e.g. ,* agonists, antagonists, or inhibitors. Any of these examples can be used to fashion drugs which are more active or stable forms of the PRO polypeptide or which enhance or interfere with the function of the PRO polypeptide *in vivo (c.f.,* Hodgson, Bio/Technology, 9: 19-21 (1991)).

In one approach, the three-dimensional structure of the PRO polypeptide, or of an PRO polypeptide-inhibitor complex, is determined by x-ray crystallography, by computer modeling or, most typically, by a combination of the two approaches. Both the shape and charges of the PRO polypeptide must be ascertained to elucidate the structure and to determine active site(s) of the molecule. Less often, useful information regarding the structure of the PRO polypeptide may be gained by modeling based on the structure of homologous proteins. In both cases, relevant structural information is used to design analogous PRO polypeptide-like molecules or to identify efficient inhibitors. Useful examples of rational drug design may include molecules which have improved activity or stability as shown by Braxton and Wells. Biochemistry. 31:7796-7801 (1992) or which act as inhibitors, agonists, or antagonists of native peptides as shown by Athauda *et al.,* J. Biochem., 113:742-746 (1993).

It is also possible to isolate a target-specific antibody, selected by functional assay, as described above, and then to solve its crystal structure. This approach, in principle, yields a pharmacore upon which subsequent drug design can be based. It is possible to bypass protein crystallography altogether by generating anti-idiotypic antibodies (anti-ids) to a functional, pharmacologically active antibody. As a mirror image of a mirror image, the binding site of the anti-ids would be expected to be an analog of the original receptor. The anti-id could then be used to identify and isolate peptides from banks of chemically or biologically produced peptides. The isolated peptides would then act as the pharmacore.

By virtue of the present invention, sufficient amounts of the PRO polypeptide may be made available to perform such analytical studies as X-ray crystallography. In addition, knowledge of the PRO polypeptide amino acid sequence provided herein will provide guidance to those employing computer modeling techniques in place of or in addition to x-ray crystallography.

### EXAMPLE 15: Pericyte c-Fos Induction (Assay 93)

This assay shows that certain polypeptides of the invention act to induce the expression of c-fos in pericyte cells and, therefore, are useful not only as diagnostic markers for particular types of pericyte-associated tumors but also for giving rise to antagonists which would be expected to be useful for the therapeutic treatment of pericyte-associated tumors. Induction of c-fos expression in pericytes is also indicative of the induction of angiogenesis and, as such, PRO polypeptides capable of inducing the expression of c-fos would be expected to be useful for the treatment of conditions where induced angiogenesis would be beneficial including, for example, wound healing, and the like. Specifically, on day 1, pericytes are received from VEC Technologies and all but 5 ml of media is removed from flask. On day 2, the pericytes are trypsinized, washed, spun and then plated onto 96 well plates. On day 7, the media is removed and the pericytes are treated with 100 µl of PRO polypeptide test samples and controls (positive control = DME+5 % serum +/- PDGF at 500 ng/ml; negative control = protein 32). Replicates are averaged and SD/CV are determined. Fold increase over Protein 32 (buffer control) value indicated by chemiluminescence units (RLU) luminometer reading verses frequency is plotted on a histogram. Two-fold above Protein 32 value is considered positive for the assay. ASY Matrix: Growth media = low glucose DMEM = 20% FBS + 1X pen strep + 1X fungizone. Assay Media = low glucose DMEM +5% FBS.

The following polypeptides tested positive in this assay: PRO982, PRO1160, PRO1187, and PRO1329.

### EXAMPLE 16: Chondrocyte Re-differentiation Assay (Assay 110)

This assay shows that certain polypeptides of the invention act to induce redifferentiation of chondrocytes, therefore, are expected to be useful for the treatment of various bone and/or cartilage disorders such as, for example, sports injuries and arthritis. The assay is performed as follows. Porcine chondrocytes are isolated by overnight collagenase digestion of articulary cartilage of metacarpophalangeal joints of 4-6 month old female pigs. The isolated cells are then seeded at 25,000 cells/cm² in Ham F-12 containing 10% FBS and 4 *µ*g/ml gentamycin. The culture media is changed every third day and the cells are then seeded in 96 well plates at 5,000 cells/well in 100µl of the same media without serum and 100 µl of the test PRO polypeptide, 5 nM staurosporin (positive control) or medium alone (negative control) is added to give a final volume of 200 µl/well. After 5 days of incubation at 37°C, a picture of each well is taken and the differentiation state of the chondrocytes is determined.
A positive result in the assay occurs when the redifferentiation of the chondrocytes is determined to be more similar to the positive control than the negative control.

The following polypeptide tested positive in this assay: PRO357.

### EXAMPLE 17: Identification of PRO Polypeptides That Stimulate TNF-α Release In Human Blood (Assay 128)

This assay shows that certain PRO polypeptides of the present invention act to stimulate the release of TNF-α in human blood. PRO polypeptides testing positive in this assay are useful for, among other things, research purposes where stimulation of the release of TNF-α would be desired and for the therapeutic treatment of conditions wherein enhanced TNF-α release would be beneficial. Specifically, 200 *µ*l of human blood supplemented with 50mM Hepes buffer (pH 7.2) is aliquoted per well in a 96 well test plate. To each well is then added 300*µ*l of either the test PRO polypeptide in 50 mM Hepes buffer (at various concentrations) or 50 mM Hepes buffer alone (negative control) and the plates are incubated at 37°C for 6 hours. The samples are then centrifuged and 50*µ*l of plasma is collected from each well and tested for the presence of TNF-α by ELISA assay. A positive in the assay is a higher amount of TNF-α in the PRO polypeptide treated samples as compared to the negative control samples.

The following PRO polypeptides tested positive in this assay: PRO231, PR0357, PRO725, PRO1155, PR01306, and PR01419.

### EXAMPLE 18: Promotion of Chondrocyte Redifferentiation (Assay 129)

This assay is designed to determine whether PRO polypeptides of the present invention show the ability to induce the proliferation and/or redifferentiation of chondrocytes in culture. PRO polypeptides testing positive in this assay would be expected to be useful for the therapeutic treatment of various bone and/or cartilage disorders such as, for example, sports injuries and arthritis.

Porcine chondrocytes are isolated by overnight collagenase digestion of articular cartilage of the metacarpophalangeal joint of 4-6 month old female pigs. The isolated cells are then seeded at 25,000 cells/cm² in Ham F-12 containing 10% FBS and 4 µg/ml gentamycin. The culture media is changed every third day. On day 12, the cells are seeded in 96 well plates at 5,000 cells/well in 100µl of the same media without serum and 100 µl of either serum-free medium (negative control), staurosporin (final concentration of 5 nM; positive control) or the test PRO polypeptide are added to give a final volume of 200 µl/well. After 5 days at 37 °C, 22 µl of media comtaining 100µg/ml Hoechst 33342 and 50 µg/ml 5-CFDA is added to each well and incubated for an additional 10 minutes at 37°C. A picture of the green fluorescence is taken for each well and the differentiation state of the chondrocytes is calculated by morphometric analysis. A positive result in the assay is obtained when the >50% of the PRO polypeptide treated cells are differentiated (compared to the background obtained by the negative control).

The following PRO polypeptides tested positive in this assay: PRO229, PRO1272, and PRO4405.

### EXAMPLE 19: Normal Human Dermal Fibroblast Proliferation (Assay 141)

This assay is designed to determine whether PRO polypeptides of the present invention show the ability to induce proliferation of human dermal fibroblast cells in culture and, therefore, function as useful growth factors.

On day 0, human dermal fibroblast cells (from cell lines, maximum of 12-14 passages) were plated in 96-well plates at 1000 cells/well per 100 microliter and incubated overnight in complete media [fibroblast growth media (FGM, Clonetics), plus supplements: insulin, human epithelial growth factor (hEGF), gentamicin (GA-1000), and fetal bovine serum (FBS, Clonetics)]. On day 1, complete media was replaced by basal media [FGM plus 1% FBS] and addition of PRO polypeptides at 1%, 0.1 % and 0.01%. On day 7, an assessment of cell proliferation was performed by Alamar Blue assay followed by Crystal Violet. Results are expresses as % of the cell growth observed with control buffer.

The following PRO polypeptides tested positive in this assay: PRO982, PRO357, PRO725, PRO1306, PRO1419, PRO229, PRO1272, PRO181, PRO214, PRO247, PRO337, PRO526, PRO363, PRO531, PRO1083 , PRO840, PRO1080, PRO788, PRO1478, PRO1134, PRO826, PRO1005, PRO809, PRO1194, PRO1071, PRO1411, PRO1309 PRO1025, PRO1181, PRO1126, PRO1186, PRO1192, PRO1244, PRO1274, PRO1412, PRO1286, PRO1330, PRO1347, PRO1305, PRO1273, PRO1279, PRO1340, PRO1338, PRO1343, PRO1376, PRO1387, PRO1409, PRO1488, PRO1474, PRO1917, PRO1760, PRO1567, PRO1887, PRO1928, PRO4341, PRO5723, PRO1801, PRO4333, PRO3543, PRO3444, PRO4302, PRO4322, PRO5725, PRO4408, PRO9940, PRO7154, PRO7425, PRO6079, PRO9836 and PRO10096.

### EXAMPLE 20: Microarray Analysis to Detect Overexpression of PRO Polypeptides in Cancerous Tumors

Nucleic acid microarrays, often containing thousands of gene sequences, are useful for identifying differentially expressed genes in diseased tissues as compared to their normal counterparts. Using nucleic acid microarrays, test and control mRNA samples from test and control tissue samples are reverse transcribed and labeled to generate cDNA probes. The cDNA probes are then hybridized to an array of nucleic acids immobilized on a solid support. The array is configured such that the sequence and position of each member of the array is known. For example, a selection of genes known to be expressed in certain disease states may be arrayed on a solid support. Hybridization of a labeled probe with a particular array member indicates that the sample from which the probe was derived expresses that gene. If the hybridization signal of a probe from a test (disease tissue) sample is greater than hybridization signal of a probe from a control (normal tissue) sample, the gene or genes overexpressed in the disease tissue are identified. The implication of this result is that an overexpressed protein in a diseased tissue is useful not only as a diagnostic marker for the presence of the disease condition, but also as a therapeutic target for treatment of the disease condition.

The methodology of hybridization of nucleic acids and microarray technology is well known in the art. In the present example, the specific preparation of nucleic acids for hybridization and probes, slides, and hybridization conditions are all detailed in U.S. Provisional Patent Application Serial No. 60/193,767, filed on March 31, 2000 and which is herein incorporated by reference.

In the present example, cancerous tumors derived from various human tissues were studied for PRO polypeptide-encoding gene expression relative to non-cancerous human tissue in an attempt to identify those PRO polypeptides which are overexpressed in cancerous tumors. Cancerous human tumor tissue from any of a variety of different human tumors was obtained and compared to a "universal" epithelial control sample which was prepared by pooling non-cancerous human tissues of epithelial origin, including liver, kidney, and lung. mRNA isolated from the pooled tissues represents a mixture of expressed gene products from these different tissues. Microarray hybridization experiments using the pooled control samples generated a linear plot in a 2-color analysis. The slope of the line generated in a 2-color analysis was then used to normalize the ratios of (test:control detection) within each experiment. The normalized ratios from various experiments were then compared and used to identify clustering of gene expression. Thus, the pooled "universal control" sample not only allowed effective relative gene expression determinations in a simple 2-sample comparison, it also allowed multi-sample comparisons across several experiments.

In the present experiments, nucleic acid probes derived from the herein described PRO polypeptide-encoding nucleic acid sequences were used in the creation of the microarray and RNA from a panel of nine different tumor tissues (listed below) were used for the hybridization thereto. A value based upon the normalized rado:experimental ratio was designated as a "cutoff ratio". Only values that were above this cutoff ratio were determined to be significant. Table 8 below shows the results of these experiments, demonstrating that various PRO polypeptides of the present invention are significantly overexpressed in various human tumor tissues, as compared to a non-cancerous human tissue control or other human tumor tissues. As described above, these data demonstrate that the PRO polypeptides of the present invention are useful not only as diagnostic markers for the presence of one or more cancerous tumors, but also serve as therapeutic targets for the treatment of those tumors.

**TABLE 8**

| Molecule | is overexpressed in: | as compared to normal control: |
|---|---|---|
| PRO6004 | colon tumor | universal normal control |
| PRO4981 | colon tumor | universal normal control |
| PRO4981 | lung tumor | universal normal control |
| PRO7174 | colon tumor | universal normal control |
| PRO5778 | lung tumor | universal normal control |
| PRO5778 | breast tumor | universal normal control |
| PRO5778 | liver tumor | universal normal control |
| PRO4332 | colon tumor | universal normal control |
| PRO9799 | colon tumor | universal normal control |
| PRO9909 | colon tumor | universal normal control |
| PRO9917 | colon tumor | universal normal control |
| PRO9917 | lung tumor | universal normal control |
| PRO9917 | breast tumor | universal normal control |
| PRO9771 | colon tumor | universal normal control |
| PRO9877 | colon tumor | universal normal control |
| PRO9903 | colon tumor | universal normal control |
| PRO9830 | colon tumor | universal normal control |
| PRO7155 | colon tumor | universal normal control |
| PRO7155 | lung tumor | universal normal control |
| PRO7155 | prostate tumor | universal normal control |
| PRO9862 | colon tumor | universal normal control |
| PRO9882 | colon tumor | universal normal control |
| PRO9864 | colon tumor | universal normal control |
| PRO10013 | colon tumor | universal normal control |
| PRO9885 | colon tumor | universal normal control |
| PRO9879 | colon tumor | universal normal control |
| PRO10111 | colon tumor | universal normal control |
| PRO10111 | rectal tumor | universal normal control |
| PRO9925 | breast tumor | universal normal control |
| PRO9925 | rectal tumor | universal normal control |
| PRO9925 | colon tumor | universal normal control |
| PRO9925 | lung tumor | universal normal control |
| PRO9905 | colon tumor | universal normal control |
| PRO10276 | colon tumor | universal normal control |
| PRO9898 | colon tumor | universal normal control |
| PRO9904 | colon tumor | universal normal control |
| PRO 19632 | colon tumor | universal normal control |
| PRO19672 | colon tumor | universal normal control |
| PRO9783 | colon tumor | universal normal control |
| PRO9783 | lung tumor | universal normal control |
| PRO9783 | breast tumor | universal normal control |
| PRO9783 | prostate tumor | universal normal control |
| PRO9783 | rectal tumor | universal normal control |
| PRO10112 | colon tumor | universal normal control |
| PRO10284 | colon tumor | universal normal control |
| PRO10100 | colon tumor | universal normal control |
| PRO19628 | colon tumor | universal normal control |
| PRO19684 | colon tumor | universal normal control |
| PRO10274 | colon tumor | universal normal control |
| PRO9907 | colon tumor | universal normal control |
| PRO9873 | colon tumor | universal normal control |
| PRO10201 | colon tumor | universal normal control |
| PRO10200 | colon tumor | universal normal control |
| PRO10196 | colon tumor | universal normal control |
| PRO10282 | lung tumor | universal normal control |
| PRO10282 | breast tumor | universal normal control |
| PRO10282 | colon tumor | universal normal control |
| PRO10282 | rectal tumor | universal normal control |
| PRO19650 | colon tumor | universal normal control |
| PRO21184 | lung tumor | universal normal control |
| PR021184 | breast tumor | universal normal control |
| PRO21184 | colon tumor | universal normal control |
| PRO21201 | breast tumor | universal normal control |
| PRO21201 | colon tumor | universal normal control |
| PRO21175 | breast tumor | universal normal control |
| PRO21175 | colon tumor | universal normal control |
| PRO21175 | lung tumor | universal normal control |
| PRO21340 | colon tumor | universal normal control |
| PRO21340 | prostate tumor | universal normal control |
| PRO21384 | colon tumor | universal normal control |
| PRO21384 | lung tumor | universal normal control |
| PRO21384 | breast tumor | universal normal control |

### EXAMPLE 21: Tissue Expression Distribution

Oligonucleotide probes were constructed from the PRO polypeptide-encoding nucleotide sequence shown in the accompanying figures for use in quantitative PCR amplification reactions. The oligonucleotide probes were chosen so as to give an approximately 200-600 base pair amplified fragment from the 3' end of its associated template in a standard PCR reaction. The oligonucleotide probes were employed in standard quantitative PCR amplification reactions with cDNA Libraries isolated from different human adult and/or fetal tissue sources and analyzed by agarose gel electrophoresis so as to obtain a quantitative determination of the level of expression of the PRO polypeptide-encoding nucleic acid in the various tissues tested. Knowledge of the expression pattern or the differential expression of the PRO polypeptide-encoding nucleic acid in various different human tissue types provides a diagnostic marker useful for tissue typing, with or without other tissue-specific markers, for determining the primary tissue source of a metastatic tumor, and the like. The results of these assays demonstrated the following:
(1) the DNA94849-2960 molecule is significantly expressed in the following tissues: cartilage, testis, colon tumor, heart, placenta, bone marrow, adrenal gland, prostate, spleen aortic endothelial cells and uterus, and not significantly expressed in the following tissues: HUVEC.
(2) the DNA100272-2969 molecule is significantly expressed in cartilage, testis, human umblilical vein endothelial cells (HUVEC), colon tumor, heart, placenta, bone marrow, adrenal gland, prostate, spleen and aortic endothelial cells; and not significantly expressed in uterus. Among a panel of normal and tumor cells examined, the DNA100272-2969 was found to be expressed in normal esophagus, esophageal tumor, normal stomach, stomach tumor, normal kidney, kidney tumor, normal lung, lung tumor, normal rectum, rectal tumor, normal liver and liver tumor.
(3) the DNA108696-2966 molecule is highly expressed in prostate and also expressed in testis, bone marrow and spleen. The DNA108696-2966 molecule is expressed in normal stomach, but not expressed in stomach tumor. The DNA108696-2966 molecule is not expressed in normal kidney, kidney tumor, normal lung, or lung tumor. The DNA108696-2966 molecule is highly expressed in normal rectum, lower expression in rectal tumor. The DNA108696-2966 molecule is not expressed in normal liver or liver tumor. The DNA108696-2966 molecule is not expressed in normal esophagus, esophagial tumor, cartilage, HUVEC, colon tumor, heart, placenta, adrenal gland, aortic endothelial cells and uterus.
(4) the DNA119498-2965 molecule is significantly expressed in the following tissues: highly expressed in aortic endothelial cells, and also significantly expressed in cartilage, testis, HUVEC, colon tumor, heart, placenta, bone marrow, adrenal galnd, prostate and spleen. It is not significantly expressed in uterus.
(5) the DNA119530-2968 molecule is expressed in the following tissues: normal esophagus and not expressed in the following tissues: esophageal tumors, stomach tumors, normal stomach, normal kidney, kidney tumor, normal lung, lung tumor, normal rectum, rectal tumors, normal liver or liver tumors.
(6) the DNA129794-2967 molecule is significantly expressed in testis and adrenal gland; and not significantly expressed in cartilage, human umblilical vein endothelial cells (HUVEC), colon tumor, heart, placenta, bone marrow, prostate, spleen, aortic endothelial cells and uterus.
(7) the DNA131590-2962 molecule is significantly expressed in the following tissues: bone marrow, adrenal gland, prostate, spleen, uterus, cartilage, testis, colon tumor, heart, and placenta, and not significantly expressed in the following tissues: HUVEC, and aortic endothelial cells.
(8) the DNA149995-2871 molecule is highly expressed in testis, and adrenal gland; expressed in cartilage, human umblilical vein endothelial cells (HUVEC), colon tumor, heart, prostate and uterus; weakly expressed in bone marrow, spleen and aortic endothelial cells; and not significantly expressed in placenta.
(9) the DNA167678-2963 molecule is significantly expressed in the following tissues: normal esophagus, esophagial tumor, highly expressed in normal stomach, stomach tumor, highly expressed in normal kidney, kidney tumor, expressed in lung, lung tumor, normal rectum, rectal tumor, weakly expressed in normal liver, and not significantly expressed in liver tumor.
(10) the DNA168028-2956 molecule is highly expressed in bone marrow; expressed in testis, human umblilical vein endothelial cells (HUVEC), colon tumor, heart, placenta, adrenal gland, prostate, spleen, aortic endothelial cells and uterus; and is weakly expressed in cartilage. Among a panel of normal and tumor samples examined, the DNA 168028-2956 was found to be expressed in stomach tumor, normal kidney, kidney tumor, lung tumor, normal rectum and rectal tumor; and not expressed in normal esophagus, esophageal tumor, normal stomach, normal lung, normal liver and liver tumor.
(11) the DNA176775-2957 molecule is highly expressed in testis; expressed in cartilage and prostate; weakly expressed in adrenal gland, spleen and uterus; and not significantly expressed in human umblilical vein endothelial cells (HUVEC), colon tumor, heart, placenta, bone marrow and aortic endothelial cells.
(12) the DNA177313-2982 molecule is significantly expressed in prostate and aortic endothelial cells; and not significantly expressed in cartilage, testis, human umbilical vein endothelial cells (HUVEC), colon tumor, heart, placenta, bone marrow, adrenal gland, spleen and uterus. Among a panel of normal and tumor cells, the DNA177313-2982 molecule was found to be expressed in esophageal tumor but not in normal esophagus, normal stomach, stomach tumor, normal kidney, kidney tumor, normal lung, lung tumor, normal rectum, rectal tumor, normal liver and liver tumor.

## Claims

1. Isolated nucleic acid having at least 80% nucleic acid sequence identity to:
(a) a nucleotide sequence that encodes the amino acid sequence shown in Figure 74 (SEQ ID NO:74);
(b) a nucleotide sequence encoding the polypeptide shown in Figure 74 (SEQ ID NO:74), lacking its associated signal peptide;
(c) a nucleotide sequence encoding an extracellular domain of the polypeptide shown in Figure 74 (SEQ ID NO:74), with its associated signal peptide; or (d) a nucleotide sequence encoding an extracellular domain of the polypeptide shown in Figure 74 (SEQ ID NO:74), lacking its associated signal peptide.

2. Isolated nucleic acid having at least 80% nucleic acid sequence identity to the nucleotide sequence shown in Figure 73 (SEQ ID NO:73).

3. Isolated nucleic acid having at least 80% nucleic acid sequence identity to the full-length coding sequence of the nucleotide sequence shown in Figure 73 (SEQ ID NO:73).

4. Isolated nucleic acid having at least 80% nucleic acid sequence identity to the full-length coding sequence of the DNA (DNA143294-2818) deposited under ATCC accession number PTA-1182.

5. A vector comprising the nucleic acid of Claim 1.

6. A host cell comprising the vector of Claim 5.

7. The host cell of Claim 6, wherein said cell is a CHO cell.

8. The host cell of Claim 6, wherein said cell is an *E*. *coli.*

9. The host cell of Claim 6, wherein said cell is a yeast cell.

10. A process for producing a PRO polypeptide comprising culturing the host cell of Claim 6 under conditions suitable for expression of said PRO polypeptide and recovering said PRO polypeptide from the cell culture.

11. An isolated polypeptide having at least 80% amino acid sequence identity to:
(a) the amino acid sequence shown in Figure 74 (SEQ ID NO:74);
(b) the amino acid sequence of the polypeptide shown in Figure 74 (SEQ ID NO:74), lacking its associated signal peptide;
(c) the amino acid sequence of an extracellular domain of the polypeptide shown in Figure 74 (SEQ ID NO:74), with its associated signal peptide; or
(d) the amino acid sequence of an extracellular domain of the polypeptide shown in Figure 74 (SEQ ID NO:74), lacking its associated signal peptide.

12. An isolated polypeptide having at least 80% amino acid sequence identity to the amino acid sequence encoded by the full-length coding sequence of the DNA (DNA143294-2818) deposited under ATCC accession number PTA-1182.

13. A chimeric molecule comprising a polypeptide according to Claim 11 fused to a heterologous amino acid sequence.

14. The chimeric molecule of Claim 13, wherein said heterologous amino acid sequence is an epitope tag sequence.

15. The chimeric molecule of Claim 13, wherein said heterologous amino acid sequence is a Fc region of an immunoglobulin.

16. An antibody which specifically binds to a polypeptide according to Claim 11.

17. The antibody of Claim 16, wherein said antibody is a monoclonal antibody, a humanized antibody or a single-chain antibody.

18. A method for detecting the presence of tumor in a mammal, said method comprising comparing the level of expression of PRO 10201 polypeptide in (a) a test sample of cells taken from said mammal and (b) a control sample of normal cells of the same cell type, wherein a higher level of expression of said PRO 10201 polypeptide in the test sample as compared to the control sample is indicative of the presence of tumor in said mammal.

19. The method of Claim 18, wherein said tumor is lung tumor, colon tumor, breast tumor, prostate tumor, rectal tumor, or liver tumor.

20. An oligonucleotide probe derived from the nucleotide sequence shown in Figure 73.


### Annex to the application documents - subsequently filed sequences listing

Sequence Listing

<110> Genentech, Inc.

<120> SECRETED AND TRANSMEMBRANE POLYPEPTIDES AND NUCLEIC
      ACIDS ENCODING THE SAME

<140> EP 04005726.7
<141> 2001-06-01

<150> PCT/US00/15264
<151> 2000-06-02

<150> US 60/209832
<151> 2000-06-05

<150> US 60/212901
<151> 2000-06-20

<150> US 60/213807
<151> 2000-06-22

<150> US 60/219556
<151> 2000-07-20

<150> US 60/220585
<151> 2000-07-25

<150> US 60/220605
<151> 2000-07-25

<150> US 60/220607
<151> 2000-07-25

<150> US 60/220624
<151> 2000-07-25

<150> US 60/220638
<151> 2000-07-25

<150> US 60/220664
<151> 2000-07-25

<150> US 60/220666
<151> 2000-07-25

<150> US 60/220893
<151> 2000-07-26

<150> PCT/US00/20710
<151> 2000-07-28

<150> US 60/222425
<151> 2000-08-01

<150> US 60/227133
<151> 2000-08-22

<150> PCT/US00/23522
<151> 2000-08-23

<150> PCT/US00/23328
<151> 2000-08-24

<150> US 60/000000
<151> 2000-09-15

<150> PCT/US00/30873

<151> 2000-11-10

<150> US 60/253646
<151> 2000-11-28

<150> PCT/US00/32678
<151> 2000-12-01

<150> US 09/747259
<151> 2000-12-20

<150> PCT/US00/34956
<151> 2000-12-20

<150> PCT/US01/06520
<151> 2001-02-28

<150> PCT/US01/06666
<151> 2001-03-01

<150> US 09/816744
<151> 2001-03-22

<150> 09/854208
<151> 2001-05-10

<150> US 09/854280
<151> 2001-05-10

<150> PCT/US01/17092
<151> 2001-05-25

<160> 246

<210> 1
<211> 4834
<212> DNA
<213> Homo Sapien

<220>
<221> unsure
<222> 3784
<223> unknown base

<400> 1
gcagccctag cagggatgga catgatgctg ttggtgcagg gtgcttgttg 50

ctcgaaccag tggctggcgg cggtgctcct cagcctgtgc tgcctgctac 100

cctcctgcct cccggctgga cagagtgtgg acttcccctg ggcggccgtg 150

gacaacatga tggtcagaaa aggggacacg gcggtgctta ggtgttattt 200

ggaagatgga gcttcaaagg gtgcctggct gaaccggtca agtattattt 250

ttgcgggagg tgataagtgg tcagtggatc ctcgagtttc aatttcaaca 300

ttgaataaaa gggactacag cctccagata cagaatgtag atgtgacaga 350

tgatggccca tacacgtgtt ctgttcagac tcaacataca cccagaacaa 400

tgcaggtgca tctaactgtg caagttcctc ctaagatata tgacatctca 450

aatgatatga ccgtcaatga aggaaccaac gtcactctta cttgtttggc 500

cactgggaaa ccagagcctt ccatttcttg gcgacacatc tccccatcag 550

caaaaccatt tgaaaatgga caatatttgg acatttatgg aattacaagg 600

gaccaggctg gggaatatga atgcagtgcg gaaaatgatg tgtcattccc 650
agatgtgagg aaagtaaaag ttgttgtcaa ctttgctcct actattcagg 700
aaattaaatc tggcaccgtg acccccggac gcagtggcct gataagatgt 750
gaaggtgcag gtgtgccgcc tccagccttt gaatggtaca aaggagagaa 800
gaagctcttc aatggccaac aaggaattat tattcaaaat tttagcacaa 850
gatccattct cactgttacc aacgtgacac aggagcactt cggcaattat 900
acttgtgtgg ctgccaacaa gctaggcaca accaatgcga gcctgcctct 950
taaccctcca agtacagccc agtatggaat taccgggagc gctgatgttc 1000
ttttctcctg ctggtacctt gtgttgacac tgtcctcttt caccagcata 1050
ttctacctga agaatgccat tctacaataa attcaaagac ccataaaagg 1100
cttttaagga ttctctgaaa gtgctgatgg ctggatccaa tctggtacag 1150
tttgttaaaa gcagcgtggg atataatcag cagtgcttac atggggatga 1200
tcgccttctg tagaattgct cattatgtaa atactttaat tctactcttt 1250
tttgattagc tacattacct tgtgaagcag tacacattgt ccttttttta 1300
agacgtgaaa gctctgaaat tacttttaga ggatattaat tgtgatttca 1350
tgtttgtaat ctacaacttt tcaaaagcat tcagtcatgg tctgctaggt 1400
tgcaggctgt agtttacaaa aacgaatatt gcagtgaata tgtgattctt 1450
taaggctgca atacaagcat tcagttccct gtttcaataa gagtcaatcc 1500
acatttacaa agatgcattt ttttcttttt tgataaaaaa gcaaataata 1550
ttgccttcag attatttctt caaaatataa cacatatcta gatttttctg 1600
ctcgcatgat attcaggttt caggaatgag ccttgtaata taactggctg 1650
tgcagctctg cttctctttc ctgtaagttc agcatgggtg tgccttcata 1700
caataatatt tttctctttg tctccaacta atataaaatg ttttgctaaa 1750
tcttacaatt tgaaagtaaa aataaaccag agtgatcaag ttaaaccata 1800
cactatctct aagtaacgaa ggagctattg gactgtaaaa atctcttcct 1850
gcactgacaa tggggtttga gaattttgcc ccacactaac tcagttcttg 1900
tgatgagaga caatttaata acagtatagt aaatatacca tatgatttct 1950
ttagttgtag ctaaatgtta gatccaccgt gggaaatcat tccctttaaa 2000
atgacagcac agtccactca aaggattgcc tagcaataca gcatcttttc 2050
ctttcactag tccaagccaa aaattttaag atgatttgtc agaaagggca 2100
caaagtccta tcacctaata ttacaagagt tggtaagcgc tcatcattaa 2150
ttttattttg tggcagctaa gttagtatga cagaggcagt gctcctgtgg 2200
acaggagcat tttgcatatt ttccatctga aagtatcact cagttgatag 2250
tctggaatgc atgttatata ttttaaaact tccaaaatat attataacaa 2300

acattctata tcggtatgta gcagaccaat ctctaaaata gctaattctt 2350

caataaaatc tttctatata gccatttcag tgcaaacaag taaaatcaaa 2400

aaagaccatc ctttattttt ccttacatga tatatgtaag atgcgatcaa 2450

ataaagacaa aacaccagtg atgagaatat cttaagataa gtaattatca 2500

aattattgtg aatgttaaat tatttctact ataaagaagc aaaactacat 2550

ttttgaagga aaatgctgtt actctaacat taatttacag gaatagtttg 2600

atggtttcac tctttactaa agaaaggcca tcaccttgaa agccatttta 2650

caggtttgat gaagttacca atttcagtac acctaaattt ctacaaatag 2700

tcccctttta caagttgtaa caacaaagac cctataataa aattagatac 2750

aagaaatttt gcagtggtta tacatatttg agatatctag tatgttgccc 2800

tagcagggat ggcttaaaaa ctgtgatttt ttttcttcaa gtaaaactta 2850

gtcccaaagt acatcataaa tcaattttaa ttagaaaaat gaatcttaaa 2900

tgaggggaca taagtatact ctttccacaa aatggcaata ataaggcata 2950

aagctagtaa atctactaac tgtaataaat gtatgacatt attttgattg 3000

atacattaaa aaagagtttt tagaacaaat atggcattta actttattat 3050

ttatttgctt ttaagaaata ttctttgtgg aattgttgaa taaactataa 3100

aatattattt tgtattgcag ctttaaagtg gcacactcca taataatcta 3150

cttactagaa atagtggtgc taccacaaaa aatgttaacc atcagtacca 3200

ttgtttggga gaaagaaaca gatcaagaat gcatattatt cagtgaccgc 3250

tttcctagag ttaaaatacc tcctctttgt aaggtttgta ggtaaattga 3300

ggtataaact atggatgaac caaataatta gttcaaagtg ttgtcatgat 3350

tccaaatttg tggagtctgg tgtttttacc atagaatgtg acagaagtac 3400

agtcatagct cagtagctat atgtatttgc ctttatgtta gaagagactt 3450

tcttgagtga catttttaaa tagaggaggt attcactatg tttttctgta 3500

tcacagcagc attcctagtc cttaggccct cggacagagt gaaatcatga 3550

gtatttatga gttcaatatt gtcaaataag gctacagtat ttgctttttt 3600

gtgtgaatgt attgcatata atgttcaagt agatgatttt acatttatgg 3650

acatataaaa tgtctgatta ccccatttta tcagtcctga ctgtacaaga 3700

ttgttgcaat ttcagaatag cagttttata aattgattta tcttttaatc 3750

tataacaatt tgtgttagct gttcatttca ggantatatt ttctacaagt 3800

tccacttgtg ggactccttt tgttgcccct attttttttt aaagaaggaa 3850

gaaagaaaaa taagtagcag tttaaaaatg agaatggaga gaaaagaaaa 3900

agaatgaaaa ggaaaggcag taaagaggga aaaaaaagga aggatggaag 3950

gaatgaagga aggaagggag gaaggggaga aggtaggaag aaagaaagga 4000

tgagagggaa ggaagaatca gagtattagg gtagttaact tacacatttg 4050

cattcttagt ttaactgcaa gtggtgtaac tatgtttttc aatgatcgca 4100

tttgaaacat aagtcctatt ataccattaa gttcctatta tgcagcaatt 4150

atataataaa aagtactgcc caagttatag taatgtgggt gtttttgaga 4200

cactaaaaga tttgagaggg agaatttcaa acttaaagcc acttttgggg 4250

ggtttataac ttaactgaaa aattaatgct tcatcataac atttaagcta 4300

tatctagaaa gtagactgga gaactgagaa aattacccag gtaattcagg 4350

gaaaaaaaaa aatatatata tatataaata cccctacatt tgaagtcaga 4400

aaactctgaa aaactgaatt atcaaagtca atcatctata atgatcaaat 4450

ttactgaaca attgttaatt tatccattgt gcttagcttt gtgacacagc 4500

caaaagttac ctatttaatc ttttcaataa aaattgtttt ttgaaatcca 4550

gaaatgattt aaaaagaggt caggttttta actatttatt gaagtatgtg 4600

gatgtacagt atttcaatag atatgaatat gaataaatgg tatgccttaa 4650

gattctttga atatgtattt actttaaaga ctggaaaaag ctcttcctgt 4700

cttttagtaa aacatccata tttcataacc tgatgtaaaa tatgttgtac 4750

tgtttccaat aggtgaatat aaactcagtt tatcaattaa aaaaaaaaaa 4800

aaaaaaaaaa aaaaaaaaaa aaaaaaaaaa aaaa 4834

<210> 2
<211> 354
<212> PRT
<213> Homo Sapien

<400> 2
Met Asp Met Met Leu Leu Val Gln Gly Ala Cys Cys Ser Asn Gln
1               5                   10                  15

Trp Leu Ala Ala Val Leu Leu Ser Leu Cys Cys Leu Leu Pro Ser
                20                  25                  30

Cys Leu Pro Ala Gly Gln Ser Val Asp Phe Pro Trp Ala Ala Val
                35                  40                  45

Asp Asn Met Met Val Arg Lys Gly Asp Thr Ala Val Leu Arg Cys
                50                  55                  60

Tyr Leu Glu Asp Gly Ala Ser Lys Gly Ala Trp Leu Asn Arg Ser
                65                  70                  75

Ser Ile Ile Phe Ala Gly Gly Asp Lys Trp Ser Val Asp Pro Arg
                80                  85                  90

Val Ser Ile Ser Thr Leu Asn Lys Arg Asp Tyr Ser Leu Gln Ile
                95                  100                 105

Gln Asn Val Asp Val Thr Asp Asp Gly Pro Tyr Thr Cys Ser Val
                110                 115                 120

Gln Thr Gln His Thr Pro Arg Thr Met Gln Val His Leu Thr Val
                125                 130                 135

Gln Val Pro Pro Lys Ile Tyr Asp Ile Ser Asn Asp Met Thr Val
                140                 145                 150

Asn Glu Gly Thr Asn Val Thr Leu Thr Cys Leu Ala Thr Gly Lys
                155                 160                 165

Pro Glu Pro Ser Ile Ser Trp Arg His Ile Ser Pro Ser Ala Lys
                170                 175                 180

Pro Phe Glu Asn Gly Gln Tyr Leu Asp Ile Tyr Gly Ile Thr Arg
                185                 190                 195

Asp Gln Ala Gly Glu Tyr Glu Cys Ser Ala Glu Asn Asp Val Ser
                200                 205                 210

Phe Pro Asp Val Arg Lys Val Lys Val Val Val Asn Phe Ala Pro
                215                 220                 225

Thr Ile Gln Glu Ile Lys Ser Gly Thr Val Thr Pro Gly Arg Ser
                230                 235                 240

Gly Leu Ile Arg Cys Glu Gly Ala Gly Val Pro Pro Pro Ala Phe
                245                 250                 255

Glu Trp Tyr Lys Gly Glu Lys Lys Leu Phe Asn Gly Gln Gln Gly
                260                 265                 270

Ile Ile Ile Gln Asn Phe Ser Thr Arg Ser Ile Leu Thr Val Thr
                275                 280                 285

Asn Val Thr Gln Glu His Phe Gly Asn Tyr Thr Cys Val Ala Ala
                290                 295                 300

Asn Lys Leu Gly Thr Thr Asn Ala Ser Leu Pro Leu Asn Pro Pro
                305                 310                 315

Ser Thr Ala Gln Tyr Gly Ile Thr Gly Ser Ala Asp Val Leu Phe
                320                 325                 330

Ser Cys Trp Tyr Leu Val Leu Thr Leu Ser Ser Phe Thr Ser Ile
                335                 340                 345

Phe Tyr Leu Lys Asn Ala Ile Leu Gln
                350

<210> 3
<211> 2506
<212> DNA
<213> Homo Sapien

<400> 3
cactgcccgt ccgctcttca gcagccggtc gcgggcggtg gaaaagcgag 50

tgaagagagc gcgacggcgg cggcggcggc ggcgcagcta ttgctggacg 100

gccagtggga gagcgaggcc tgagcctctg cgtctaggat caaaatggtt 150

tcaatcccag aatactatga aggcaagaac gtcctcctca caggagctac 200

cggttttcta gggaaggtgc ttctggaaaa gttgctgagg tcttgtccta 250

aggtgaattc agtatatgtt ttggtgaggc agaaagctgg acagacacca 300

caagagcgag tggaagaagt ccttagtggc aagctttttg acagattgag 350

agatgaaaat ccagatttta gagagaaaat tatagcaatc aacagcgaac 400

tcacccaacc taaactggct ctcagtgaag aagataaaga ggtgatcata 450

gattctacca atattatatt ccactgtgca gctacagtaa ggtttaatga 500

aaatttaaga gatgctgttc agttaaatgt gattgcaacg cgacagctta 550

ttctccttgc acaacaaatg aagaatctgg aagtgttcat gcatgtatca 600

acagcatatg cctactgtaa tcgcaagcat attgatgaag tagtctatcc 650

accacctgtg gatcccaaga agctgattga ttctttagag tggatggatg 700

atggcctagt aaatgatatc acgccaaaat tgataggaga cagacctaat 750

acatacatat acacaaaagc attggcagaa tatgttgtac aacaagaagg 800

agcaaaacta aatgtggcaa ttgtaaggcc atcgattgtt ggtgccagtt 850

ggaaagaacc ttttccagga tggattgata actttaatgg accaagtggt 900

ctctttattg cggcagggaa aggaattctt cgaacaatac gtgcctccaa 950

caatgccctt gcagatcttg ttcctgtaga tgtagttgtc aacatgagtc 1000

ttgcggcagc ctggtattcc ggagttaata gaccaagaaa catcatggtg 1050

tataattgta caacaggcag cactaatcct ttccactggg gtgaagttga 1100

gtaccatgta atttccactt tcaagaggaa tcctctcgaa caggccttca 1150

gacggcccaa tgtaaatcta acctccaatc atcttttata tcattactgg 1200

attgctgtaa gccataaggc cccagcattc ctgtatgata tctacctcag 1250

gatgactgga agaagcccaa ggatgatgaa aacaataact cgtcttcaca 1300

aagctatggt gtttcttgaa tatttcacaa gtaattcttg ggtttggaat 1350

actgagaatg tcaatatgtt aatgaatcaa ctaaaccctg aagataaaaa 1400

gaccttcaat attgatgtac ggcagttaca ttgggcagaa tatatagaga 1450

actactgctt gggaactaag aagtacgtat tgaatgaaga aatgtctggc 1500

ctccctgcag ccagaaaaca tctgaacaag ttgcggaata tacgttatgg 1550

ttttaatact atccttgtga tcctcatctg gcgcattttt attgcaagat 1600

cacaaatggc aagaaatatc tggtactttg tggttagtct gtgttacaag 1650

tttttgtcat acttccgagc atccagcact atgagatact gaagaccaag 1700

gattcagcat tagaacatct atacatatgg tgatctaaat gtacaaaatg 1750

taaaatgtat aagtcatctc actttttgtc aagacattaa accatcttag 1800

atcggagtgt gaagtaaatt atggtatatt ttatgtaaca ttttaatgtt 1850

tatgctcata aaacttagtg aacacactgt gttatgccag ctcaaatcta 1900

cagtagccac caaaaccatg acttaatatt ttgagcccta gaagaaaggg 1950

gtgtgctgag gacaagagtg gggaaatagg aacactgacc agtataactg 2000

tgcaattctg gaacatatta attaaaataa tatgccttaa catatagtga 2050

atttctaatt ctaatgttca gtgcaatgga agacatttat ttggacagta 2100

tactagcaaa gttggtagat atttgattct tcattttttg tttttttcat 2150

tagttgaagt gggttttagt tttgtttaaa attataacca gcgtattttc 2200

acatcattct gtaagttaaa tgatatcaaa catgaaagag atgttctcat 2250

ttttcttttt ctgattaaac gtctgatgca tatcattttt ctataagtaa 2300

tcagttgctt ttaaaatcag aaggctatat tattctaatg accctattcg 2350

atctaaatgg gtttgagaat ccatatcagc aacatacgtg ttttttgaca 2400

gaaagtgaaa acaaattccg taaaactgtt agtatcaaaa agaataggaa 2450

tacagttttc ttttccacat tatgatcaaa taaaaatctt gtgagattgt 2500

taaaaa 2506

<210> 4
<211> 515
<212> PRT
<213> Homo Sapien

<400> 4

<210> 5
<211> 1759
<212> DNA
<213> Homo Sapien

<400> 5
cgatgccggc ggtcagtggt ccaggtccct tattctgcct tctcctcctg 50

ctcctggacc cccacagccc tgagacgggg tgtcctcctc tacgcaggtt 100
tgagtacaag ctcagcttca aaggcccaag gctggcattg cctggggctg 150
gaataccctt ctggagccat catggagacg ccatcctggg cctggaggaa 200
gtgcggctga cgccatccat gaggaaccgg agtggcgccg tgtggagcag 250
ggcctctgtc cccttctctg cctgggaagt agaggtgcag atgagggtga 300
cgggactggg gcgccgggga gcccagggca tggccgtgtg gtacacccgg 350
ggcaggggcc atgtaggctc tgtccttggg gggctggctt cgtgggacgg 400
catcgggatc ttctttgact ctccggcaga ggatactcag gacagtcctg 450
ccatccgtgt gctggccagc gacgggcaca tcccctctga gcagcctggg 500
gatggagcta gccaagggct gggctcctgt cattgggact tccggaaccg 550
gccacactcc ttcagagcac ggatcaccta ctgggggcag aggctgcgca 600
tgtccttgaa cagtggcctc actcccagtg atccaggtga gttctgtgtg 650
gatgtggggc ccctgctttt ggtccctgga ggtttctttg gggtctcagc 700
agccaccggc accctggcag gtgaggatcc cactggacag gttccccctc 750
agcccttcct ggagatgcag cagctccgcc tggcgaggca gctggaaggg 800
ctgtgggcaa ggctgggctt gggcaccagg gaggatgtaa ctccaaaatc 850
agactctgaa gctcaaggag aaggggaaag gctctttgac ctggaggaga 900
cgctgggcag acaccgccgg atcctgcagg ctctgcgggg tctctccaag 950
cagctggccc aggctgagag acaatggaag aagcagctgg ggcccccagg 1000
ccaagccagg cctgacggag gctgggccct ggatgcttcc tgccagattc 1050
catccacccc agggaggggt ggccacctct ccatgtcact caataaggac 1100
tctgccaagg tcggtgccct gctccatgga cagtggactc tgctccaggc 1150
cctgcaagag atgagggatg cagctgtccg catggctgca gaagcccagg 1200
tctcctacct gcctgtgggc attgagcatc atttcttaga gctggaccac 1250
atcctgggcc tcctgcagga ggagcttcgg ggcccggcga aggcagcagc 1300
caaggccccc cgcccacctg gccagccccc aagggcctcc tcgtgcctgc 1350
agcctggcat cttcctgttc tacctcctca ttcagactgt aggcttcttc 1400
ggctacgtgc acttcaggca ggagctgaac aagagccttc aggagtgtct 1450
gtccacaggc agccttcctc tgggtcctgc accacacacc cccagggccc 1500
tggggattct gaggaggcag cctctccctg ccagcatgcc tgcctgaccc 1550
acctcagagc ctgctttgca tcactgggaa gcaggcagtg tcttgggtgg 1600
gggcttggtc agtatcctct ccgtctgggt gcccagctcc cacgcacacc 1650
tgagctttcg gcatgctccc acctcgttaa aggtgatttc cctctcccca 1700
aaaaaaaaaa aaaaaaaaaa aaaaaaaaaa aaaaaaaaaa aaaaaaaaaa 1750

aaaaaaaaa 1759

<210> 6
<211> 514
<212> PRT
<213> Homo Sapien

<400> 6

<210> 7
<211> 3010
<212> DNA
<213> Homo Sapien

<400> 7
gcccccagca tggcttggca gggctggccc gcggcgtggc agtgggtcgc 50

cggctgctgg ctcctcctcg tccttgtcct cgtcctactt gtgagccccc 100

gcggctgccg agcgcggcgg ggcctccgcg gtctgctcat ggcgcacagc 150

cagcggctgc tcttccgaat cgggtacagc ctgtacaccc gcacctggct 200

cgggtacctc ttctaccgac agcagctgcg cagggctcgg aatcgctacc 250

ctaaaggcca ctcgaaaacc cagccccgcc tcttcaatgg agtgaaggtg 300

cttcccatcc ctgtcctctc ggacaactac agctacctca tcatcgacac 350

ccaggcccag ctggctgtgg ctgtggaccc ttcagaccct cgggctgtgc 400

aggcttccat tgaaaaggaa ggggtcacct tggtcgccat tctgtgtact 450

cacaagcact gggaccacag tggagggaac cgtgacctca gccggcggca 500
ccgggactgt cgggtgtacg ggagccctca ggacggcatc ccctacctca 550
cccatcccct gtgtcatcaa gatgtggtca gcgtgggacg gcttcagatc 600
cgggccctgg ctacacctgg ccacacacaa ggccatctgg tctacctact 650
ggatggggag ccctacaagg gtccctcctg cctcttctca ggggacctgc 700
tcttcctctc tggctgtggg cggacctttg agggcaatgc agagaccatg 750
ctgagctcac tggacactgt gctggggcta ggggatgaca cccttctgtg 800
gcctggtcat gagtatgcag aggagaacct gggctttgca ggtgtggtgg 850
agcccgagaa cctggcccgg gagaggaaga tgcagtgggt gcagcggcag 900
cggctggagc gcaagggcac gtgcccatct accctgggag aggagcgctc 950
ctacaacccg ttcctgagaa cccactgcct ggcgctacag gaggctctgg 1000
ggccggggcc gggccccact ggggatgatg actactcccg ggcccagctc 1050
ctggaagagc tccgccggct gaaggatatg cacaagagca agtgatgccc 1100
ccagcgcccc cagcccagcc cactccccgc atggggaggc cgccaccacc 1150
aacacctcat catccttctc atcgctaaca ccaccacctc catcggcacc 1200
caagcgggca tcatcccccc acactgctca ggggagggga gggatcaggc 1250
gatgagactg tgaggccaaa agaagggggc ctgttggagg ctgggaaccc 1300
cgcagcgcga ggctgcctca tcaacggcaa gaggaaagga ggggtctcgg 1350
gacatctcca gaccctacca actgggaggg tcccctcctc cttccctact 1400
cctgggacgg cagcaaggac atgggggctg ctgttagctt ctccgtcagg 1450
aggcctcatc tcactgtagc cctggaaccc agggtccatc ttgcccttcc 1500
cccatccatg gttgggaaag aagctcagcc cctcacagtg gcctcaagtg 1550
tgatgcctta caaaagcacc actcagatgg gcagctggac tctggtgtcc 1600
tgagactctg ccctcttccc acagcctccc tgccccaccc atccctgcaa 1650
agccattttt cagacagagc cattcctaag aacactgaag ggctggaatg 1700
ctggctggcc actctctgcc tcagtggcct ccctacagcc tggaagaagg 1750
agggtcctga ttgccaagga aacctcctca ttgggctaag gagacactgg 1800
agtctggagt gtggagcccc acagtcttgc aggtcacatg ctctccttgc 1850
acatctggcc tggttgtacc cactggcctc tgcctctgcc ctgggccaaa 1900
agggcccctc cttgccaggg gagagacagc cacggtcctc tttggccgat 1950
gctgtattct cattttggcc cttgttctta ggcccgtctg cccgccctcc 2000
tccatctaac ctttcctgtt ttatccgcag cccttttctt ctttgagtta 2050
gtaaagattt attctgtaac ctgacactca tctggccctt tgcagtttgc 2100
cagccatatt cccatgtgat ttcccactgg atccaggccc ccatccggct 2150

ggcaggaggg ggctctgacg tacaggttgg aaatcagaag tctgtgagag 2200

cgcgggagtg catggcagct ctgggtccca gacctggccc gacccctctg 2250

cttcacctcc agctctgctg ctcctctact cttgggtcga gatccctttg 2300

gagccacagc gaggaaccct gtggtcctca ggcaggtgta ccttgagtca 2350

gccaggagcc ctcttttcct gtgtcaaagc ctgccctcgg gctctgctca 2400

cctctggtga ccctccaaga tgcccctgcc ctcagtttcc cctcatgatc 2450

tggcctctgc ccccttctct agccacagcc tctagtacac tttagcaata 2500

ccaccagact agttagagtt ccccactcac caagcaagac atgcagtttc 2550

atgcctctgt gccttcgctc atgctgtttc ttccgactgg aatgccttcc 2600

cctgctcctc ctgccttgtc tgcctggcaa gttcatctct cacgatcccc 2650

tcaaaggccc cctcctccag gaaggcaacc cctgtgcccc tcccctccag 2700

gctacctctg cactttgtca atgcttctct tgtggcactt atcacactgt 2750

attttacttg tttacatgtt tgtctcccct tctagactgt gaatccttaa 2800

gggcatggac tgtatcttat gcatctctgt atttctgcgc ctagcacggt 2850

gcctagcaca cagtaggcgc tcaataaatg ttgaatgaat gaaaaaaaaa 2900

aaaaaaaaaa aaaaaaaaaa aaaaaaaaaa aaaaaaaaaa aaaaaaaaaa 2950

aaaaaaaaaa aaaaaaaaaa aaaaaaaaaa aaaaaaaaaa aaaaaaaaaa 3000

aaaaaaaaaa 3010

<210> 8
<211> 361
<212> PRT
<213> Homo Sapien

<400> 8
Met Ala Trp Gln Gly Trp Pro Ala Ala Trp Gln Trp Val Ala Gly
1               5                   10                  15

Cys Trp Leu Leu Leu Val Leu Val Leu Val Leu Leu Val Ser Pro
                20                  25                  30

Arg Gly Cys Arg Ala Arg Arg Gly Leu Arg Gly Leu Leu Met Ala
                35                  40                  45

His Ser Gln Arg Leu Leu Phe Arg Ile Gly Tyr Ser Leu Tyr Thr
                50                  55                  60

Arg Thr Trp Leu Gly Tyr Leu Phe Tyr Arg Gln Gln Leu Arg Arg
                65                  70                  75

Ala Arg Asn Arg Tyr Pro Lys Gly His Ser Lys Thr Gln Pro Arg
                80                  85                  90

Leu Phe Asn Gly Val Lys Val Leu Pro Ile Pro Val Leu Ser Asp
                95                  100                 105

Asn Tyr Ser Tyr Leu Ile Ile Asp Thr Gln Ala Gln Leu Ala Val
                110                 115                 120

Ala Val Asp Pro Ser Asp Pro Arg Ala Val Gln Ala Ser Ile Glu

                125                 130                 135

Lys Glu Gly Val Thr Leu Val Ala Ile Leu Cys Thr His Lys His
                140                 145                 150

Trp Asp His Ser Gly Gly Asn Arg Asp Leu Ser Arg Arg His Arg
                155                 160                 165

Asp Cys Arg Val Tyr Gly Ser Pro Gln Asp Gly Ile Pro Tyr Leu
                170                 175                 180

Thr His Pro Leu Cys His Gln Asp Val Val Ser Val Gly Arg Leu
                185                 190                 195

Gln Ile Arg Ala Leu Ala Thr Pro Gly His Thr Gln Gly His Leu
                200                 205                 210

Val Tyr Leu Leu Asp Gly Glu Pro Tyr Lys Gly Pro Ser Cys Leu
                215                 220                 225

Phe Ser Gly Asp Leu Leu Phe Leu Ser Gly Cys Gly Arg Thr Phe
                230                 235                 240

Glu Gly Asn Ala Glu Thr Met Leu Ser Ser Leu Asp Thr Val Leu
                245                 250                 255

Gly Leu Gly Asp Asp Thr Leu Leu Trp Pro Gly His Glu Tyr Ala
                260                 265                 270

Glu Glu Asn Leu Gly Phe Ala Gly Val Val Glu Pro Glu Asn Leu
                275                 280                 285

Ala Arg Glu Arg Lys Met Gln Trp Val Gln Arg Gln Arg Leu Glu
                290                 295                 300

Arg Lys Gly Thr Cys Pro Ser Thr Leu Gly Glu Glu Arg Ser Tyr
                305                 310                 315

Asn Pro Phe Leu Arg Thr His Cys Leu Ala Leu Gln Glu Ala Leu
                320                 325                 330

Gly Pro Gly Pro Gly Pro Thr Gly Asp Asp Asp Tyr Ser Arg Ala
                335                 340                 345

Gln Leu Leu Glu Glu Leu Arg Arg Leu Lys Asp Met His Lys Ser
                350                 355                 360

Lys

<210> 9
<211> 872
<212> DNA
<213> Homo Sapien

<400> 9
gctgacaatc cccttgacgt tctatcccgg aagctccacc tggggcccaa 50

tgttgggcgt gatgttcctc gcctgtctct gcctggaaaa ctggtcttcc 100

caagctccac tggcagccac ttctccatgt tgggcatcgg agacatcgtt 150

atgcctggtc tcctactatg ctttgtcctt cgctatgaca actacaaaaa 200

gcaagccagt ggggactcct gtggggcccc tggacctgcc aacatctccg 250

ggcgcatgca gaaggtctcc tactctcact gcaccctcat cggatacttt 300

gtaggcctgc tcactgctac tgtggcgtct cgcattcacc gggccgccca 350

gcccgccctt ctctatttgg tgccatttac tttattgcca ctcctcacga 400

tggcctattt aaagggcgac ctccggcgga tgtggtctga gcctttccac 450

tccaagtcca gcagctcccg attcctggaa gtatgatgga tcacgtggaa 500

agtgaccaga tggccgtcat agtccttttc tctcaactca tggtttgttt 550

cctcttagag ctggcctggt actcagaaat gtacctgtgt ttaaggaact 600

gccgtgtgac tggatttggc attgaaaggg agctcgtttg caggagagag 650

gtgctggagc cctgtttggt tccttctctt cctgcggatg tagaggtggg 700

gccccttcca agagggacag gcctctcccc agcgcgcctt cctcccacgt 750

ttttatggat ctgcaccaga ctgttacctt ctgggggaga tggagatttg 800

actgtttaaa aactgaaaac agcgaggagt ctttctagaa cttttgaaca 850

ctaaaaggat gaaaaaatta gc 872

<210> 10
<211> 108
<212> PRT
<213> Homo Sapien

<400> 10
Met Met Asp His Val Glu Ser Asp Gln Met Ala Val Ile Val Leu
1               5                   10                  15

Phe Ser Gln Leu Met Val Cys Phe Leu Leu Glu Leu Ala Trp Tyr
                20                  25                  30

Ser Glu Met Tyr Leu Cys Leu Arg Asn Cys Arg Val Thr Gly Phe
                35                  40                  45

Gly Ile Glu Arg Glu Leu Val Cys Arg Arg Glu Val Leu Glu Pro
                50                  55                  60

Cys Leu Val Pro Ser Leu Pro Ala Asp Val Glu Val Gly Pro Leu
                65                  70                  75

Pro Arg Gly Thr Gly Leu Ser Pro Ala Arg Leu Pro Pro Thr Phe
                80                  85                  90

Leu Trp Ile Cys Thr Arg Leu Leu Pro Ser Gly Gly Asp Gly Asp
                95                  100                 105

Leu Thr Val

<210> 11
<211> 1739
<212> DNA
<213> Homo Sapien

<400> 11
tcgcacactg gtggcttcag aagaaattct caacacctag ctcgccagag 50

agtctatgta tgggattgaa caatctgtaa actaaaggat cctaatcatg 100

aaaataagta tgataaatta taagtcacta ttggcactgt tgtttatatt 150

agcctcctgg atcattttta cagttttcca gaactccaca aaggtttggt 200

ctgctctaaa cttatccatc tccctccatt actggaacaa ctccacaaag 250

tccttattcc ctaaaacacc actgatatca ttaaagccac taacagagac 300

tgaactcaga ataaaggaaa tcatagagaa actagatcag cagatcccac 350

ccagaccttt cacccacgtg aacaccacca ccagcgccac acatagcaca 400

gccaccatcc tcaaccctcg agatacgtac tgcaggggag accagctgca 450

catcctgctg gaggtgaggg accacttggg acgcaggaag caatatggcg 500

gggatttcct gagggccagg atgtcttccc cagcgctgat ggcaggtgct 550

tcaggaaagg tgactgactt caacaacggc acctacctgg tcagcttcac 600

tctgttctgg gagggccagg tctctctgtc tctgctgctc atccacccca 650

gtgaaggggt gtcagctctc tggagtgcaa ggaaccaagg ctatgacagg 700

gtgatcttca ctggccagtt tgtcaatggc acttcccaag tccactctga 750

atgtggcctg atcctaaaca caaatgctga attgtgccag tacctggaca 800

acagagacca agaaggcttc tactgtgtga ggcctcaaca catgccctgt 850

gctgcactca ctcacatgta ttctaagaac aagaaagttt cttatcttag 900

caaacaagaa aagagcctct ttgaaaggtc aaatgtgggt gtagagatta 950

tggaaaaatt caatacaatt agtgtctcca aatgcaacaa agaaacagtt 1000

gcaatgaaag agaaatgcaa gtttggaatg acatccacaa tccccagtgg 1050

gcatgtctgg agaaacacat ggaatcctgt ctcctgtagt ttggctacag 1100

tcaaaatgaa ggaatgcctg agaggaaaac tcatatacct aatgggagat 1150

tccacgatcc gccagtggat ggaatacttc aaagccagta tcaacacact 1200

gaagtcagtg gatctgcatg aatctggaaa attgcaacac cagcttgctg 1250

tggatttgga taggaacatc aacatccagt ggcaaaaata ttgttatccc 1300

ttgataggat caatgaccta ttcagtcaaa gagatggagt acctcacccg 1350

ggccattgac agaactggag gagaaaaaaa tactgtcatt gttatttccc 1400

tgggccagca tttcagaccc tttcccattg atgtttttat ccgaagggcc 1450

ctcaatgtcc acaaagccat tcagcatctt cttctgagaa gcccagacac 1500

tatggttatc atcaaaacag aaaacatcag ggagatgtac aatgatgcag 1550

aaagatttag tgactttcat ggttacattc aatatctcat cataaaggac 1600

attttccagg atctcagtgt gagtatcatt gatgcctggg atataacaat 1650

tgcatatggc acaaataatg tacacccacc tcaacatgta gtcggaaatc 1700

agattaatat attattaaac tatatttgtt aaataacaa 1739

<210> 12
<211> 544
<212> PRT
<213> Homo Sapien

<400> 12

Met Lys Ile Ser Met Ile Asn Tyr Lys Ser Leu Leu Ala Leu Leu
1               5                   10                  15

Phe Ile Leu Ala Ser Trp Ile Ile Phe Thr Val Phe Gln Asn Ser
                20                  25                  30

Thr Lys Val Trp Ser Ala Leu Asn Leu Ser Ile Ser Leu His Tyr
                35                  40                  45

Trp Asn Asn Ser Thr Lys Ser Leu Phe Pro Lys Thr Pro Leu Ile
                50                  55                  60

Ser Leu Lys Pro Leu Thr Glu Thr Glu Leu Arg Ile Lys Glu Ile
                65                  70                  75

Ile Glu Lys Leu Asp Gln Gln Ile Pro Pro Arg Pro Phe Thr His
                80                  85                  90

Val Asn Thr Thr Thr Ser Ala Thr His Ser Thr Ala Thr Ile Leu
                95                  100                 105

Asn Pro Arg Asp Thr Tyr Cys Arg Gly Asp Gln Leu His Ile Leu
                110                 115                 120

Leu Glu Val Arg Asp His Leu Gly Arg Arg Lys Gln Tyr Gly Gly
                125                 130                 135

Asp Phe Leu Arg Ala Arg Met Ser Ser Pro Ala Leu Met Ala Gly
                140                 145                 150

Ala Ser Gly Lys Val Thr Asp Phe Asn Asn Gly Thr Tyr Leu Val
                155                 160                 165

Ser Phe Thr Leu Phe Trp Glu Gly Gln Val Ser Leu Ser Leu Leu
                170                 175                 180

Leu Ile His Pro Ser Glu Gly Val Ser Ala Leu Trp Ser Ala Arg
                185                 190                 195

Asn Gln Gly Tyr Asp Arg Val Ile Phe Thr Gly Gln Phe Val Asn
                200                 205                 210

Gly Thr Ser Gln Val His Ser Glu Cys Gly Leu Ile Leu Asn Thr
                215                 220                 225

Asn Ala Glu Leu Cys Gln Tyr Leu Asp Asn Arg Asp Gln Glu Gly
                230                 235                 240

Phe Tyr Cys Val Arg Pro Gln His Met Pro Cys Ala Ala Leu Thr
                245                 250                 255

His Met Tyr Ser Lys Asn Lys Lys Val Ser Tyr Leu Ser Lys Gln
                260                 265                 270

Glu Lys Ser Leu Phe Glu Arg Ser Asn Val Gly Val Glu Ile Met
                275                 280                 285

Glu Lys Phe Asn Thr Ile Ser Val Ser Lys Cys Asn Lys Glu Thr
                290                 295                 300

Val Ala Met Lys Glu Lys Cys Lys Phe Gly Met Thr Ser Thr Ile
                305                 310                 315

Pro Ser Gly His Val Trp Arg Asn Thr Trp Asn Pro Val Ser Cys
                320                 325                 330

Ser Leu Ala Thr Val Lys Met Lys Glu Cys Leu Arg Gly Lys Leu
                335                 340                 345

Ile Tyr Leu Met Gly Asp Ser Thr Ile Arg Gln Trp Met Glu Tyr
                350                 355                 360

Phe Lys Ala Ser Ile Asn Thr Leu Lys Ser Val Asp Leu His Glu
                365                 370                 375

Ser Gly Lys Leu Gln His Gln Leu Ala Val Asp Leu Asp Arg Asn
                380                 385                 390

Ile Asn Ile Gln Trp Gln Lys Tyr Cys Tyr Pro Leu Ile Gly Ser
                395                 400                 405

Met Thr Tyr Ser Val Lys Glu Met Glu Tyr Leu Thr Arg Ala Ile
                410                 415                 420

Asp Arg Thr Gly Gly Glu Lys Asn Thr Val Ile Val Ile Ser Leu
                425                 430                 435

Gly Gln His Phe Arg Pro Phe Pro Ile Asp Val Phe Ile Arg Arg
                440                 445                 450

Ala Leu Asn Val His Lys Ala Ile Gln His Leu Leu Leu Arg Ser
                455                 460                 465

Pro Asp Thr Met Val Ile Ile Lys Thr Glu Asn Ile Arg Glu Met
                470                 475                 480

Tyr Asn Asp Ala Glu Arg Phe Ser Asp Phe His Gly Tyr Ile Gln
                485                 490                 495

Tyr Leu Ile Ile Lys Asp Ile Phe Gln Asp Leu Ser Val Ser Ile
                500                 505                 510

Ile Asp Ala Trp Asp Ile Thr Ile Ala Tyr Gly Thr Asn Asn Val
                515                 520                 525

His Pro Pro Gln His Val Val Gly Asn Gln Ile Asn Ile Leu Leu
                530                 535                 540

Asn Tyr Ile Cys

<210> 13
<211> 1893
<212> DNA
<213> Homo Sapien

<400> 13
gcaaagagaa gactgaaaga caaacctggg tgcagccaga gaggtccaga 50

tagatgagct tgtggcatcc attccccaag ttcagcctag ggactccacg 100

taccccagct gggtctcatt gttccagaac tgcattagtt aagattaccc 150

agacttggat ttcaaaggaa tactttcatt gttccgtctg taacacgaag 200

taattggggc cagctggatg tcaggatgcg tgtggttacc attgtaatct 250

tgctctgctt ttgcaaagcg gctgagctgc gcaaagcaag cccaggcagt 300

gtgagaagcc gagtgaatca tggccgggcg ggtggaggcc ggagaggctc 350

caacccggtc aaacgctacg caccaggcct cccgtgtgac gtgtacacat 400

atctccatga gaaatactta gattgtcaag aaagaaaatt agtttatgtg 450

ctgcctggtt ggcctcagga tttgctgcac atgctgctag caagaaacaa 500

gatccgcaca ttgaagaaca acatgttttc caagtttaaa aagctgaaaa 550

gcctggatct gcagcagaat gagatctcta aaattgagag tgaggcgttc 600

tttggtttaa acaaactcac caccctctta ctgcagcaca accagatcaa 650

agtcttgacg gaggaagtgt tcatttacac acctctcttg agctacctgc 700

gtctttatga caacccctgg cactgtactt gtgagataga aacgcttatt 750

tcaatgttgc agattcccag gaaccggaat ttggggaact acgccaagtg 800

tgaaagtcca caagaacaaa aaaataaaaa actgcggcag ataaaatctg 850

aacagttgtg taatgaagaa aaggaacaat tggacccgaa accccaagtg 900

tcagggagac ccccagtcat caagcctgag gtggactcaa ctttttgcca 950

caattatgtg tttcccatac aaacactgga ctgcaaaagg aaagagttga 1000

aaaaagtgcc aaacaacatc cctccagata ttgttaaact tgacttgtca 1050

tacaataaaa tcaaccaact tcgacccaag gaatttgaag atgttcatga 1100

gctgaagaaa ttaaacctca gcagcaatgg cattgaattc atcgatcctg 1150

ccgctttttt agggctcaca catttagaag aattagattt atcaaacaac 1200

agtctgcaaa actttgacta tggcgtatta gaagacttgt attttttgaa 1250

actcttgtgg ctcagagata acccttggag atgtgactac aacattcact 1300

acctctacta ctggttaaag caccactaca atgtccattt taatggcctg 1350

gaatgcaaaa cgcctgaaga atacaaagga tggtctgtgg gaaaatatat 1400

tagaagttac tatgaagaat gccccaaaga caagttacca gcatatcctg 1450

agtcatttga ccaagacaca gaagatgatg aatgggaaaa aaaacataga 1500

gatcacaccg caaagaagca aagcgtaata attactatag taggataagg 1550

tagaaattgt tctgattgta attagttttg tattttctat actggtgtta 1600

gaaaacatat gtttacattt gattaactgt gttgcctatt tatgcagggt 1650

aatccagcta aaggaagctt tctttaatta taagtattat tgtgactatt 1700

atagtaatca agagaatgct atcatcctgc ttgcctgtcc atttgtggaa 1750

cagcatctgg tgatatgcaa ttccacactg gtaacctgca gcagttgggt 1800

cctaatgatg gcattagact ttcataatgt cctgtataaa tgtttttact 1850

gcttttagaa aataaagaaa aaaaacttgg ttcatgttta aaa 1893

<210> 14
<211> 440
<212> PRT
<213> Homo Sapien

<400> 14
Met Arg Val Val Thr Ile Val Ile Leu Leu Cys Phe Cys Lys Ala
1               5                   10                  15

Ala Glu Leu Arg Lys Ala Ser Pro Gly Ser Val Arg Ser Arg Val
                20                  25                  30

Asn His Gly Arg Ala Gly Gly Gly Arg Arg Gly Ser Asn Pro Val
                35                  40                  45

Lys Arg Tyr Ala Pro Gly Leu Pro Cys Asp Val Tyr Thr Tyr Leu
                50                  55                  60

His Glu Lys Tyr Leu Asp Cys Gln Glu Arg Lys Leu Val Tyr Val
                65                  70                  75

Leu Pro Gly Trp Pro Gln Asp Leu Leu His Met Leu Leu Ala Arg
                80                  85                  90

Asn Lys Ile Arg Thr Leu Lys Asn Asn Met Phe Ser Lys Phe Lys
                95                  100                 105

Lys Leu Lys Ser Leu Asp Leu Gln Gln Asn Glu Ile Ser Lys Ile
                110                 115                 120

Glu Ser Glu Ala Phe Phe Gly Leu Asn Lys Leu Thr Thr Leu Leu
                125                 130                 135

Leu Gln His Asn Gln Ile Lys Val Leu Thr Glu Glu Val Phe Ile
                140                 145                 150

Tyr Thr Pro Leu Leu Ser Tyr Leu Arg Leu Tyr Asp Asn Pro Trp
                155                 160                 165

His Cys Thr Cys Glu Ile Glu Thr Leu Ile Ser Met Leu Gln Ile
                170                 175                 180

Pro Arg Asn Arg Asn Leu Gly Asn Tyr Ala Lys Cys Glu Ser Pro
                185                 190                 195

Gln Glu Gln Lys Asn Lys Lys Leu Arg Gln Ile Lys Ser Glu Gln
                200                 205                 210

Leu Cys Asn Glu Glu Lys Glu Gln Leu Asp Pro Lys Pro Gln Val
                215                 220                 225

Ser Gly Arg Pro Pro Val Ile Lys Pro Glu Val Asp Ser Thr Phe
                230                 235                 240

Cys His Asn Tyr Val Phe Pro Ile Gln Thr Leu Asp Cys Lys Arg
                245                 250                 255

Lys Glu Leu Lys Lys Val Pro Asn Asn Ile Pro Pro Asp Ile Val
                260                 265                 270

Lys Leu Asp Leu Ser Tyr Asn Lys Ile Asn Gln Leu Arg Pro Lys
                275                 280                 285

Glu Phe Glu Asp Val His Glu Leu Lys Lys Leu Asn Leu Ser Ser
                290                 295                 300

Asn Gly Ile Glu Phe Ile Asp Pro Ala Ala Phe Leu Gly Leu Thr
                305                 310                 315

His Leu Glu Glu Leu Asp Leu Ser Asn Asn Ser Leu Gln Asn Phe
                320                 325                 330

Asp Tyr Gly Val Leu Glu Asp Leu Tyr Phe Leu Lys Leu Leu Trp
                335                 340                 345

Leu Arg Asp Asn Pro Trp Arg Cys Asp Tyr Asn Ile His Tyr Leu
                350                 355                 360

Tyr Tyr Trp Leu Lys His His Tyr Asn Val His Phe Asn Gly Leu

365                 370                 375

Glu Cys Lys Thr Pro Glu Glu Tyr Lys Gly Trp Ser Val Gly Lys
                380                 385                 390

Tyr Ile Arg Ser Tyr Tyr Glu Glu Cys Pro Lys Asp Lys Leu Pro
                395                 400                 405

Ala Tyr Pro Glu Ser Phe Asp Gln Asp Thr Glu Asp Asp Glu Trp
                410                 415                 420

Glu Lys Lys His Arg Asp His Thr Ala Lys Lys Gln Ser Val Ile
                425                 430                 435

Ile Thr Ile Val Gly
                440

<210> 15
<211> 1524
<212> DNA
<213> Homo Sapien

<400> 15
gcggcagcag cgcgggcccc agcagcctcg gcagccacag ccgctgcagc 50

cggggcagcc tccgctgctg tcgcctcctc tgatgcgctt gccctctccc 100

ggccccggga ctccgggaga atgtgggtcc taggcatcgc ggcaactttt 150

tgcggattgt tcttgcttcc aggctttgcg ctgcaaatcc agtgctacca 200

gtgtgaagaa ttccagctga acaacgactg ctcctccccc gagttcattg 250

tgaattgcac ggtgaacgtt caagacatgt gtcagaaaga agtgatggag 300

caaagtgccg ggatcatgta ccgcaagtcc tgtgcatcat cagcggcctg 350

tctcatcgcc tctgccgggt accagtcctt ctgctcccca gggaaactga 400

actcagtttg catcagctgc tgcaacaccc ctctttgtaa cgggccaagg 450

cccaagaaaa ggggaagttc tgcctcggcc ctcaggccag ggctccgcac 500

caccatcctg ttcctcaaat tagccctctt ctcggcacac tgctgaagct 550

gaaggagatg ccaccccctc ctgcattgtt cttccagccc tcgcccccaa 600

ccccccacct ccctgagtga gtttcttctg ggtgtccttt tattctgggt 650

agggagcggg agtccgtgtt ctcttttgtt cctgtgcaaa taatgaaaga 700

gctcggtaaa gcattctgaa taaattcagc ctgactgaat tttcagtatg 750

tacttgaagg aaggaggtgg agtgaaagtt cacccccatg tctgtgtaac 800

cggagtcaag gccaggctgg cagagtcagt ccttagaagt cactgaggtg 850

ggcatctgcc ttttgtaaag cctccagtgt ccattccatc cctgatgggg 900

gcatagtttg agactgcaga gtgagagtga cgttttctta gggctggagg 950

gccagttccc actcaaggct ccctcgcttg acattcaaac ttcatgctcc 1000

tgaaaaccat tctctgcagc agaattggct ggtttcgcgc ctgagttggg 1050

ctctagtgac tcgagactca atgactggga cttagactgg ggctcggcct 1100

cgctctgaaa agtgcttaag aaaatcttct cagttctcct tgcagaggac 1150

tggcgccggg acgcgaagag caacgggcgc tgcacaaagc gggcgctgtc 1200

ggtggtggag tgcgcatgta cgcgcaggcg cttctcgtgg ttggcgtgct 1250

gcagcgacag gcggcagcac agcacctgca cgaacacccg ccgaaactgc 1300

tgcgaggaca ccgtgtacag gagcgggttg atgaccgagc tgaggtagaa 1350

aaacgtctcc gagaagggga ggaggatcat gtacgcccgg aagtaggacc 1400

tcgtccagtc gtgcttgggt ttggccgcag ccatgatcct ccgaatctgg 1450

ttgggcatcc agcatacggc caatgtcaca acaatcagcc ctgggcagac 1500

acgagcagga gggagagaca gaga 1524

<210> 16
<211> 141
<212> PRT
<213> Homo Sapien

<400> 16
Met Trp Val Leu Gly Ile Ala Ala Thr Phe Cys Gly Leu Phe Leu
1               5                   10                  15

Leu Pro Gly Phe Ala Leu Gln Ile Gln Cys Tyr Gln Cys Glu Glu
                20                  25                  30

Phe Gln Leu Asn Asn Asp Cys Ser Ser Pro Glu Phe Ile Val Asn
                35                  40                  45

Cys Thr Val Asn Val Gln Asp Met Cys Gln Lys Glu Val Met Glu
                50                  55                  60

Gln Ser Ala Gly Ile Met Tyr Arg Lys Ser Cys Ala Ser Ser Ala
                65                  70                  75

Ala Cys Leu Ile Ala Ser Ala Gly Tyr Gln Ser Phe Cys Ser Pro
                80                  85                  90

Gly Lys Leu Asn Ser Val Cys Ile Ser Cys Cys Asn Thr Pro Leu
                95                  100                 105

Cys Asn Gly Pro Arg Pro Lys Lys Arg Gly Ser Ser Ala Ser Ala
                110                 115                 120

Leu Arg Pro Gly Leu Arg Thr Thr Ile Leu Phe Leu Lys Leu Ala
                125                 130                 135

Leu Phe Ser Ala His Cys
                140

<210> 17
<211> 1402
<212> DNA
<213> Homo Sapien

<400> 17
cgcaaagccg ccctcggggc gctcatggcg ggacgcctcc tgggaaaggc 50

tttagccgcg gtgtctctct ctctggcctt ggcctctgtg actatcaggt 100

cctcgcgctg ccgcggcatc caggcgttca gaaactcgtt ttcatcttct 150

tggtttcatc ttaataccaa cgtcatgtct ggttctaatg gttccaaaga 200

aaattctcac aataaggctc ggacgtctcc ttacccaggt tcaaaagttg 250

aacgaagcca ggttcctaat gagaaagtgg gctggcttgt tgagtggcaa 300

gactataagc ctgtggaata cactgcagtc tctgtcttgg ctggacccag 350

gtgggcagat cctcagatca gtgaaagtaa tttttctccc aagtttaacg 400

aaaaggatgg gcatgttgag agaaagagca agaatggcct gtatgagatt 450

gaaaatggaa gaccgagaaa tcctgcagga cggactggac tggtgggccg 500

gggggctttg ggcgatgggg gcccaaatca cgctgcagat cccattataa 550

ccagatggaa aagggatagc agtggaaata aaatcatgca tcctgtttct 600

gggaagcata tcttacaatt tgttgcaata aaaaggaaag actgtggaga 650

atgggcaatc ccagggggga tggtggatcc aggagagaag attagtgcca 700

cactgaaaag agaatttggt gaggaagctc tcaactcctt acagaaaacc 750

agtgctgaga agagagaaat agaggaaaag ttgcacaaac tcttcagcca 800

agaccaccta gtgatatata agggatatgt tgatgatcct cgaaacactg 850

ataatgcatg gatggagaca gaagctgtga actaccatga cgaaacaggt 900

gagataatgg ataatcttat gctagaagct ggagatgatg ctggaaaagt 950

gaaatgggtg gacatcaatg ataaactgaa gctttatgcc agtcactctc 1000

aattcatcaa acttgtggct gagaaacgag atgcacactg gagcgaggac 1050

tctgaagctg actgccatgc gttgtagctg atggtctccg tgtaagccaa 1100

aggcccacag aggagcatat actgaaaaga aggcagtatc acagaattta 1150

tactataaaa agggcagggt aggccacttg gcctatttac tttcaaaaca 1200

atttgcattt agagtgtttc gcatcagaat aacatgagta agatgaactg 1250

gaacacaaaa ttttcagctc tttggtcaaa aggaatataa gtaatcatat 1300

tttgtatgta ttcgatttaa gcatggctta aattaaattt aaacaactaa 1350

tgctctttga agaatcataa tcagaataaa gataaattct tgatcagcta 1400

ta 1402

<210> 18
<211> 350
<212> PRT
<213> Homo Sapien

<400> 18
Met Ala Gly Arg Leu Leu Gly Lys Ala Leu Ala Ala Val Ser Leu
1               5                   10                  15

Ser Leu Ala Leu Ala Ser Val Thr Ile Arg Ser Ser Arg Cys Arg
                20                  25                  30

Gly Ile Gln Ala Phe Arg Asn Ser Phe Ser Ser Ser Trp Phe His
                35                  40                  45

Leu Asn Thr Asn Val Met Ser Gly Ser Asn Gly Ser Lys Glu Asn
                50                  55                  60

Ser His Asn Lys Ala Arg Thr Ser Pro Tyr Pro Gly Ser Lys Val

65                  70                  75

Glu Arg Ser Gln Val Pro Asn Glu Lys Val Gly Trp Leu Val Glu
                80                  85                  90

Trp Gln Asp Tyr Lys Pro Val Glu Tyr Thr Ala Val Ser Val Leu
                95                  100                 105

Ala Gly Pro Arg Trp Ala Asp Pro Gln Ile Ser Glu Ser Asn Phe
                110                 115                 120

Ser Pro Lys Phe Asn Glu Lys Asp Gly His Val Glu Arg Lys Ser
                125                 130                 135

Lys Asn Gly Leu Tyr Glu Ile Glu Asn Gly Arg Pro Arg Asn Pro
                140                 145                 150

Ala Gly Arg Thr Gly Leu Val Gly Arg Gly Leu Leu Gly Arg Trp
                155                 160                 165

Gly Pro Asn His Ala Ala Asp Pro Ile Ile Thr Arg Trp Lys Arg
                170                 175                 180

Asp Ser Ser Gly Asn Lys Ile Met His Pro Val Ser Gly Lys His
                185                 190                 195

Ile Leu Gln Phe Val Ala Ile Lys Arg Lys Asp Cys Gly Glu Trp
                200                 205                 210

Ala Ile Pro Gly Gly Met Val Asp Pro Gly Glu Lys Ile Ser Ala
                215                 220                 225

Thr Leu Lys Arg Glu Phe Gly Glu Glu Ala Leu Asn Ser Leu Gln
                230                 235                 240

Lys Thr Ser Ala Glu Lys Arg Glu Ile Glu Glu Lys Leu His Lys
                245                 250                 255

Leu Phe Ser Gln Asp His Leu Val Ile Tyr Lys Gly Tyr Val Asp
                260                 265                 270

Asp Pro Arg Asn Thr Asp Asn Ala Trp Met Glu Thr Glu Ala Val
                275                 280                 285

Asn Tyr His Asp Glu Thr Gly Glu Ile Met Asp Asn Leu Met Leu
                290                 295                 300

Glu Ala Gly Asp Asp Ala Gly Lys Val Lys Trp Val Asp Ile Asn
                305                 310                 315

Asp Lys Leu Lys Leu Tyr Ala Ser His Ser Gln Phe Ile Lys Leu
                320                 325                 330

Val Ala Glu Lys Arg Asp Ala His Trp Ser Glu Asp Ser Glu Ala
                335                 340                 345

Asp Cys His Ala Leu
                350

<210> 19
<211> 2138
<212> DNA
<213> Homo Sapien

<400> 19
cgagggctcc tgctggtact gtgttcgctg ctgcacagca aggccctgcc 50

acccaccttc aggccatgca gccatgttcc gggagcccta attgcacaga 100

agcccatggg gagctccaga ctggcagccc tgctcctgcc tctcctcctc 150

atagtcatcg acctctctga ctctgctggg attggctttc gccacctgcc 200

ccactggaac acccgctgtc ctctggcctc ccacacggat gacagtttca 250

ctggaagttc tgcctatatc ccttgccgca cctggtgggc cctcttctcc 300

acaaagcctt ggtgtgtgcg agtctggcac tgttcccgct gtttgtgcca 350

gcatctgctg tcaggtggct caggtcttca acggggcctc ttccacctcc 400

tggtgcagaa atccaaaaag tcttccacat tcaagttcta taggagacac 450

aagatgccag cacctgctca gaggaagctg ctgcctcgtc gtcacctgtc 500

tgagaagagc catcacattt ccatcccctc cccagacatc tcccacaagg 550

gacttcgctc taaaaggacc caaccttcgg atccagagac atgggaaagt 600

cttcccagat tggactcaca aaggcatgga ggacccgagt tctcctttga 650

tttgctgcct gaggcccggg ctattcgggt gaccatatct tcaggccctg 700

aggtcagcgt gcgtctttgt caccagtggg cactggagtg tgaagagctg 750

agcagtccct atgatgtcca gaaaattgtg tctgggggcc acactgtaga 800

gctgccttat gaattccttc tgccctgtct gtgcatagag gcatcctacc 850

tgcaagagga cactgtgagg cgcaaaaaat gtcccttcca gagctggcca 900

gaagcctatg gctcggactt ctggaagtca gtgcacttca ctgactacag 950

ccagcacact cagatggtca tggccctgac actccgctgc ccactgaagc 1000

tggaagctgc cctctgccag aggcacgact ggcataccct ttgcaaagac 1050

ctcccgaatg ccacggctcg agagtcagat gggtggtatg ttttggagaa 1100

ggtggacctg cacccccagc tctgcttcaa gttctctttt ggaaacagca 1150

gccatgttga atgcccccac cagactgggt ctctcacatc ctggaatgta 1200

agcatggata cccaagccca gcagctgatt cttcacttct cctcaagaat 1250

gcatgccacc ttcagtgctg cctggagcct cccaggcttg gggcaggaca 1300

ctttggtgcc ccccgtgtac actgtcagcc aggcccgggg ctcaagccca 1350

gtgtcactag acctcatcat tcccttcctg aggccagggt gctgtgtcct 1400

ggtgtggcgg tcagatgtcc agtttgcctg gaagcacctc ttgtgtccag 1450

atgtctctta cagacacctg gggctcttga tcctggcact gctggccctc 1500

ctcaccctac tgggtgttgt tctggccctc acctgccggc gcccacagtc 1550

aggcccgggc ccagcgcggc cagtgctcct cctgcacgcg gcggactcgg 1600

aggcgcagcg gcgcctggtg ggagcgctgg ctgaactgct acgggcagcg 1650

ctgggcggcg ggcgcgacgt gatcgtggac ctgtgggagg ggaggcacgt 1700

ggcgcgcgtg ggcccgctgc cgtggctctg ggcggcgcgg acgcgcgtag 1750

cgcgggagca gggcactgtg ctgctgctgt ggagcggcgc cgaccttcgc 1800

ccggtcagcg gccccgaccc ccgcgccgcg cccctgctcg ccctgctcca 1850

cgctgccccg cgcccgctgc tgctgctcgc ttacttcagt cgcctctgcg 1900

ccaagggcga catccccccg ccgctgcgcg ccctgccgcg ctaccgcctg 1950

ctgcgcgacc tgccgcgtct gctgcgggcg ctggacgcgc ggcctttcgc 2000

agaggccacc agctggggcc gccttggggc gcggcagcgc aggcagagcc 2050

gcctagagct gtgcagccgg cttgaacgag aggccgcccg acttgcagac 2100

ctaggttgag cagagctcca ccgcagtccc gggtgtct 2138

<210> 20
<211> 667
<212> PRT
<213> Homo Sapien

<400> 20
Met Gly Ser Ser Arg Leu Ala Ala Leu Leu Leu Pro Leu Leu Leu
1               5                   10                  15

Ile Val Ile Asp Leu Ser Asp Ser Ala Gly Ile Gly Phe Arg His
                20                  25                  30

Leu Pro His Trp Asn Thr Arg Cys Pro Leu Ala Ser His Thr Asp
                35                  40                  45

Asp Ser Phe Thr Gly Ser Ser Ala Tyr Ile Pro Cys Arg Thr Trp
                50                  55                  60

Trp Ala Leu Phe Ser Thr Lys Pro Trp Cys Val Arg Val Trp His
                65                  70                  75

Cys Ser Arg Cys Leu Cys Gln His Leu Leu Ser Gly Gly Ser Gly
                80                  85                  90

Leu Gln Arg Gly Leu Phe His Leu Leu Val Gln Lys Ser Lys Lys
                95                  100                 105

Ser Ser Thr Phe Lys Phe Tyr Arg Arg His Lys Met Pro Ala Pro
                110                 115                 120

Ala Gln Arg Lys Leu Leu Pro Arg Arg His Leu Ser Glu Lys Ser
                125                 130                 135

His His Ile Ser Ile Pro Ser Pro Asp Ile Ser His Lys Gly Leu
                140                 145                 150

Arg Ser Lys Arg Thr Gln Pro Ser Asp Pro Glu Thr Trp Glu Ser
                155                 160                 165

Leu Pro Arg Leu Asp Ser Gln Arg His Gly Gly Pro Glu Phe Ser
                170                 175                 180

Phe Asp Leu Leu Pro Glu Ala Arg Ala Ile Arg Val Thr Ile Ser
                185                 190                 195

Ser Gly Pro Glu Val Ser Val Arg Leu Cys His Gln Trp Ala Leu
                200                 205                 210

Glu Cys Glu Glu Leu Ser Ser Pro Tyr Asp Val Gln Lys Ile Val
                215                 220                 225

Ser Gly Gly His Thr Val Glu Leu Pro Tyr Glu Phe Leu Leu Pro
                230                 235                 240

Cys Leu Cys Ile Glu Ala Ser Tyr Leu Gln Glu Asp Thr Val Arg
                245                 250                 255

Arg Lys Lys Cys Pro Phe Gln Ser Trp Pro Glu Ala Tyr Gly Ser
                260                 265                 270

Asp Phe Trp Lys Ser Val His Phe Thr Asp Tyr Ser Gln His Thr
                275                 280                 285

Gln Met Val Met Ala Leu Thr Leu Arg Cys Pro Leu Lys Leu Glu
                290                 295                 300

Ala Ala Leu Cys Gln Arg His Asp Trp His Thr Leu Cys Lys Asp
                305                 310                 315

Leu Pro Asn Ala Thr Ala Arg Glu Ser Asp Gly Trp Tyr Val Leu
                320                 325                 330

Glu Lys Val Asp Leu His Pro Gln Leu Cys Phe Lys Phe Ser Phe
                335                 340                 345

Gly Asn Ser Ser His Val Glu Cys Pro His Gln Thr Gly Ser Leu
                350                 355                 360

Thr Ser Trp Asn Val Ser Met Asp Thr Gln Ala Gln Gln Leu Ile
                365                 370                 375

Leu His Phe Ser Ser Arg Met His Ala Thr Phe Ser Ala Ala Trp
                380                 385                 390

Ser Leu Pro Gly Leu Gly Gln Asp Thr Leu Val Pro Pro Val Tyr
                395                 400                 405

Thr Val Ser Gln Ala Arg Gly Ser Ser Pro Val Ser Leu Asp Leu
                410                 415                 420

Ile Ile Pro Phe Leu Arg Pro Gly Cys Cys Val Leu Val Trp Arg
                425                 430                 435

Ser Asp Val Gln Phe Ala Trp Lys His Leu Leu Cys Pro Asp Val
                440                 445                 450

Ser Tyr Arg His Leu Gly Leu Leu Ile Leu Ala Leu Leu Ala Leu
                455                 460                 465

Leu Thr Leu Leu Gly Val Val Leu Ala Leu Thr Cys Arg Arg Pro
                470                 475                 480

Gln Ser Gly Pro Gly Pro Ala Arg Pro Val Leu Leu Leu His Ala
                485                 490                 495

Ala Asp Ser Glu Ala Gln Arg Arg Leu Val Gly Ala Leu Ala Glu
                500                 505                 510

Leu Leu Arg Ala Ala Leu Gly Gly Gly Arg Asp Val Ile Val Asp
                515                 520                 525

Leu Trp Glu Gly Arg His Val Ala Arg Val Gly Pro Leu Pro Trp
                530                 535                 540

Leu Trp Ala Ala Arg Thr Arg Val Ala Arg Glu Gln Gly Thr Val
                545                 550                 555

Leu Leu Leu Trp Ser Gly Ala Asp Leu Arg Pro Val Ser Gly Pro
                560                 565                 570

Asp Pro Arg Ala Ala Pro Leu Leu Ala Leu Leu His Ala Ala Pro

                575                 580                 585

Arg Pro Leu Leu Leu Leu Ala Tyr Phe Ser Arg Leu Cys Ala Lys
                590                 595                 600

Gly Asp Ile Pro Pro Pro Leu Arg Ala Leu Pro Arg Tyr Arg Leu
                605                 610                 615

Leu Arg Asp Leu Pro Arg Leu Leu Arg Ala Leu Asp Ala Arg Pro
                620                 625                 630

Phe Ala Glu Ala Thr Ser Trp Gly Arg Leu Gly Ala Arg Gln Arg
                635                 640                 645

Arg Gln Ser Arg Leu Glu Leu Cys Ser Arg Leu Glu Arg Glu Ala
                650                 655                 660

Ala Arg Leu Ala Asp Leu Gly
                665

<210> 21
<211> 2714
<212> DNA
<213> Homo Sapien

<400> 21
cggctcgagg ccctttgtga gggctgtgag ctgcgcctga cggtggcacc 50

atgagcagct caggtggggc gcccggggcg tccgccagct ctgcgccgcc 100

cgcgcaggaa gagggcatga cgtggtggta ccgctggctg tgtcgcctgt 150

ctggggtgct gggggcagtc tcttgcgcga tctctggcct cttcaactgc 200

atcaccatcc accctctgaa catcgcggcc ggcgtgtgga tgatcatgaa 250

tgccttcatc ttgttgctgt gtgaggcgcc cttctgctgc cagttcatcg 300

agtttgcaaa cacagtggcg gagaaggtgg accggctgcg ctcctggcag 350

aaggctgtct tctactgcgg gatggcggtc gttcccatcg tcatcagcct 400

gaccctgacc acgctgctgg gcaacgccat cgcctttgct acgggggtgc 450

tgtacggact ctctgctctg ggcaaaaagg gcgatgcgat ctcctatgcc 500

aggatccagc agcagaggca gcaggcggat gaggagaagc tcgcggagac 550

cctggagggg gagctgtgaa gggctgggcg cccctccctc cctgtcccct 600

cttctggctc tgtgtgggtc caagtgaggc ctggactgtc cacgctgagg 650

cacagcctgg agaggggcct ttgcacgtgt ccctacacct ggagtcctct 700

gctcctttct ccagactggc ttaagccagg agccactggc tgctggtgtg 750

agggtctggg ctgctggact tgaggcagag cctgcagcag ctgtgtggac 800

actacccagc cctactcctc tgctgggtgg gtctgcagat ctcacaccac 850

agacagggct gcctgtgacc tgctgtgacc tgggagcagc ttcccctgga 900

gatgctggtc ctggcttgag gggaggggca agtgggaccc tgccacctgg 950

gcactgagca gagggacctc ccccagctct cttagcaggt ggagccccag 1000

ggcctgggac agcctgccgc tgccagcaac ctcccactgc tgcctagggt 1050

gcagcgccca ctgtcaccct gccttctgaa gaagcccaca gggctcctaa 1100
ggtgcacccc ggtacctgga actgcagcct tggcagtgac tggacagctg 1150
ggtgggggat gctccctgct ggccctggga accttggaca ggccacctca 1200
aggcccctcg gctgcccctc ctccctgggc ctgctggggc ccctaggttc 1250
tacccatcac cccccgcccc tgctggcctt ggtgctaagg aagtggggag 1300
agcaggctct ccctggcacc gagggtgccc accctctccc tggtgtggcc 1350
ccgtcaacat cagccacagc ccagccccat tagtgggtta gtgggtctga 1400
cctcagcccc actcaggtgc tcctgctggc ctgcccaagc cctgccctca 1450
gggagcttct gccttttaag aactgggcag aggccacagt cacctcccca 1500
cacagagctg tccccactgc cctgggtgcc aggctgtccg gagccaggcc 1550
tacccaggga ggatgcagag agctggtgcc caggatgtgc acccccatat 1600
tccctctgcc ctgtggcctc agcccgctgg cctctctgac cgtgaggctg 1650
gctctcagcc atcgggcagg tgcctggtca ggcctggctt agcccaggtg 1700
gggcttggca gaagcgggcg ggtgtggaag atattccatc tggggccaac 1750
cccaggctgg gcctgcgctg agcttctgga gcgcaggtac tgggtcttgc 1800
taagtgaact gtttcccagg aacacctctc gggcccatct gcgtctgagg 1850
ctgggagtgg catctgaggc cgggagtggc atctgaggcc aggagtggca 1900
ggctggtggg ctgggcgtgg ggttttctgg gccctgccca gtactgccct 1950
ggggacttgg tgggctcctg ggtcagcagc atcccacccc tgggagtctg 2000
gccagctgag ccccagggtg gcaggggcat tatagcctgg tggacatgtg 2050
ccttcagggt tcctccgggg ccaccttcct caggccagtg ctgggttcaa 2100
agggctgtgt gtgtgtgtgt ttgtgtgtgt atgtatatgt gtgtgggtgc 2150
acacatctgt cccatgtatg cagtgagacc tgtctacctc ccacaaggag 2200
caagggctct gcccgccctc tgctcattcc tacccaggta gtgggacccc 2250
gggccccctt ctgcctggct tgcctgcttc tgccctttcc agaggggtct 2300
cactgacagc cagagacagc aggagaaggg ttggctgtgg atcaaggaag 2350
gctgcccctg taccctgtgg ggaaatggtg ggtgcatggc tggatgcaga 2400
ggtggaaggc cctgggccac aggcgagagt gggcgtgtca cctgtcccag 2450
gttcccagca agtctgcagc tgtgcagtcc tggggtccct gaccctgtcg 2500
cccagggggc gtgctgtcca gcaggggccc tgccttgcaa ggaacgtctc 2550
tccggcggct gggccgctcc tgcctggtct gggctgtgtg tggcgccctt 2600
tcctccttgt ttgttcctct gtgttctgtg tgcgtcttaa gcaataaagc 2650
gtggccgtgg gaaaaaaaaa aaaaaaaaaa aaaaaaaaaa aaaaaaaaaa 2700
aaaaaaaaaa aaaa 2714

<210> 22
<211> 172
<212> PRT
<213> Homo Sapien

<400> 22
Met Ser Ser Ser Gly Gly Ala Pro Gly Ala Ser Ala Ser Ser Ala
1               5                   10                  15

Pro Pro Ala Gln Glu Glu Gly Met Thr Trp Trp Tyr Arg Trp Leu
                20                  25                  30

Cys Arg Leu Ser Gly Val Leu Gly Ala Val Ser Cys Ala Ile Ser
                35                  40                  45

Gly Leu Phe Asn Cys Ile Thr Ile His Pro Leu Asn Ile Ala Ala
                50                  55                  60

Gly Val Trp Met Ile Met Asn Ala Phe Ile Leu Leu Leu Cys Glu
                65                  70                  75

Ala Pro Phe Cys Cys Gln Phe Ile Glu Phe Ala Asn Thr Val Ala
                80                  85                  90

Glu Lys Val Asp Arg Leu Arg Ser Trp Gln Lys Ala Val Phe Tyr
                95                  100                 105

Cys Gly Met Ala Val Val Pro Ile Val Ile Ser Leu Thr Leu Thr
                110                 115                 120

Thr Leu Leu Gly Asn Ala Ile Ala Phe Ala Thr Gly Val Leu Tyr
                125                 130                 135

Gly Leu Ser Ala Leu Gly Lys Lys Gly Asp Ala Ile Ser Tyr Ala
                140                 145                 150

Arg Ile Gln Gln Gln Arg Gln Gln Ala Asp Glu Glu Lys Leu Ala
                155                 160                 165

Glu Thr Leu Glu Gly Glu Leu
                170

<210> 23
<211> 888
<212> DNA
<213> Homo Sapien

<400> 23
gtgaaacacc catggtttta tgctctattt ctcttttcct catcttcttc 50

cacatcctct ttctgaatgt atcaaactac ttccttgaag tggggcacca 100

ggagggccac tccagtctcc aatgcaggga ctcaggggca gggatctctg 150

agaaagtggc catctcgtta ttaaagctct gtcctctgct tccctctcac 200

ctcagaagca gcccgtttat tcaacagagc tccaggttgc cagctagggg 250

ttttcgggac catagaccaa gcaaccccga gagactgagt actgacctgc 300

agttgttcca gaaactctgc tgggaattag gttgtgacct agaagtgaac 350

tgacactaac agtgagaagg cagggtaaga atgcagtcta gagcgcaacc 400

tttctccact agacttgtaa gtaatttaag tgaatcctgt ccccctgggg 450

ttctatcctg gctggctctg ctggtgaact tgactggcca gcatagggca 500

cttgatgaga ccctggaatg ctgaggccag ttgggcagca agctttcacc 550

tcatccttct gcccatctat ccagccattc aaacattcat tcgcctgaag 600

acatttatca agctcctgca atgggtcagg catctgctag gcactgggga 650

cacagagctc acagtctcct ggagggggtg agagatgact gacaggtggt 700

ctgtggtgca gtgtgacctg ggaatgcaca cagtactgtg gaaacacggg 750

agaggcatct agcacaacct gagagggcca ggggaggctt cctggcaggt 800

ttccctttaa ccatcttaag ggaaagaggc actaggtagg aaaataaagg 850

gacagtggtg tcccagacag agggcactct acatggaa 888

<210> 24
<211> 113
<212> PRT
<213> Homo Sapien

<400> 24
Met Val Leu Cys Ser Ile Ser Leu Phe Leu Ile Phe Phe His Ile
1               5                   10                  15

Leu Phe Leu Asn Val Ser Asn Tyr Phe Leu Glu Val Gly His Gln
                20                  25                  30

Glu Gly His Ser Ser Leu Gln Cys Arg Asp Ser Gly Ala Gly Ile
                35                  40                  45

Ser Glu Lys Val Ala Ile Ser Leu Leu Lys Leu Cys Pro Leu Leu
                50                  55                  60

Pro Ser His Leu Arg Ser Ser Pro Phe Ile Gln Gln Ser Ser Arg
                65                  70                  75

Leu Pro Ala Arg Gly Phe Arg Asp His Arg Pro Ser Asn Pro Glu
                80                  85                  90

Arg Leu Ser Thr Asp Leu Gln Leu Phe Gln Lys Leu Cys Trp Glu
                95                  100                 105

Leu Gly Cys Asp Leu Glu Val Asn
                110

<210> 25
<211> 2534
<212> DNA
<213> Homa Sapien

<400> 25
cgggctccgc gcggtcccac ttcccggctc ccttcgcctc caggatgcgc 50

tgagccctac aacaccccca gcggccgccg gctcccccac gaggtgtgaa 100

tgacagaggt ggtgccatcc agcgcgctca gcgaggtcag cctgcgcctc 150

ctctgccacg atgacataga cactgtgaag cacctgtgtg gcgactggtt 200

ccccatcgag tacccagact catggtatcg tgatatcaca tccaacaaga 250

agttcttttc ccttgctgca acctacagag gtgccattgt gggaatgata 300

gtagctgaaa ttaagaacag gaccaaaata cataaagagg atggagatat 350

tctagcaacc aacttctctg ttgacacaca agtcgcgtac atcctaagtc 400

tgggcgtcgt gaaagagttc aggaagcacg gcataggttc cctcttactt 450
gaaagtttaa aggatcacat atcaaccacc gcccaggacc actgcaaagc 500
catttacctg catgtcctca ccaccaacaa cacagcaata aacttctatg 550
aaaacagaga cttcaagcag caccactatc tcccctatta ctactccatt 600
cgaggggtcc tcaaagatgg cttcacctat gtcctctaca tcaacggcgg 650
gcaccctccc tggacgattt tggactacat ccagcacctg ggctctgcac 700
tagccagcct gagcccctgc tccattccgc acagagtcta ccgccaggcc 750
cacagcctgc tctgcagctt cctgccatgg tcgggcatct cttccaagag 800
tggcatcgag tacagccgga ccatgtgatg tcggctgggc agccgccacc 850
aggccccacc cttcagccgc ccgcagagcc cgccttcctg tccatctgac 900
cccttctgtt ttctgcaagg agctgccagc catctaactg ggctcgtcgg 950
cctgccccag ctgcaggccc ggtgctacac gggctcggga acagaacatc 1000
gtgggcatgc gcagagcatg cccatccgtg gcaggctctt cagctcccct 1050
ccctgcttct ggaaacctct gcctgctgcc ctggccctgc ccccctgcgc 1100
atgcaccatc cccagggctg acccagtgtg gctgcattca ctgggagggg 1150
cctgccctca ctgggcctct cccactccgc tgcctgttct tgcagctcct 1200
tcctggaaag ctggagggga ctttctcctg caagggagga acgcaagtat 1250
tatggacaca cttgaccgta aaggcacagg agcctcggaa caagggggcg 1300
caataaaggg aatggcccgt ccccttccag aaccagccca aagaagcctg 1350
gggggtgagg agtggccccc actcctccat gaggggctga tgaggggtgg 1400
gcagcctggg ggaggctttc ctcgcaagca cagagctctg aggctcagcc 1450
ccctggcaca ggcggtcacg catcaggacg gttcctactc ctcagcacct 1500
tccgtgcagt taccagtgcc ctgggaggtc acactgcccg tcggaccttg 1550
gcatgctcca ttcagctgac ctgctgagga caggcatcgc cgagactcct 1600
tgggtcctcc ccgccctccc tcatgctgcc acaagctgct gctccaaggc 1650
ctggccacat gcagacagga ggaagctgag ctcgacatta ggcctcaagg 1700
ctgccatctg tcttgtaggg cctggccttg tgggcagggg gcagtcctgt 1750
gccttgtggg ccctcagcct ctgagggcag agatgctgtc agtgccgcag 1800
gtgcatcaca tacttctagc atcctctcca ccctgcattc caaatgctgc 1850
ttgctgcctg ccctgccctc cgatgcaggg gtggggtggg gggcggagtc 1900
ccgcccagca tagctgcagt gtcacaaagc catggcagag ggtcctagcg 1950
gcgccaccct gccccagcct gaggaggagg gagagggagg aacaaccctg 2000
ggcagacggg gtctcaggga cctgtgtcct tccgcctcca gagctgccca 2050
gccacgggct ctcagggtgc tggggcagcc ccaggtcccc tcttgaactc 2100

agctggggcc aggggccctc agaatgaagg caggcaccag gcaggagcag 2150

catccccctc cttgacggtg ctggcaggag ggccgcgcca tgctgactgc 2200

ttgaacctct gctgacctga cagtgctggc gggagggccg caccatgctg 2250

actgcctgaa tctctgctga ggctgcctgc ctgccgggcc cagctcagcg 2300

ccctctccac tgcgaatcag tggcgatcat gtgatttcta tttctgcccc 2350

acagggtaag ggacgagtct tctggaaggc tctgccatgg acatttgtcc 2400

tcgggctcag aggccccacc ctgccccaca cctgccccta atcactgcag 2450

tgtccagccc agtgttgaac agattgtagc gttctgtctc attacgagca 2500

aataaataga ctttcattgg gaaaaaaaaa aaaa 2534

<210> 26
<211> 242
<212> PRT
<213> Homo Sapien

<400> 26

<210> 27
<211> 991
<212> DNA
<213> Homo Sapien

<400> 27
gttgggcagc agccacccgc tcacctccat ccccaggact tagagggacg 50

cagggcgttg ggaacagagg acactccagg cgctgaccct gggaggccag 100

gaccagggcc aaagtcccgt gggcaagagg agtcctcaga ggtccttcat 150

tcagcggttc cgggaggtct gggaagccca cggcctggct ggggcagggt 200

caacgccgcc aggccgccat ggtcctgtgc tggctgctgc ttctggtgat 250

ggctctgccc ccaggcacga cgggcgtcaa ggactgcgtc ttctgtgagc 300

tcaccgactc catgcagtgt cctggtacct acatgcactg tggcgatgac 350

gaggactgct tcacaggcca cggggtcgcc ccgggcactg gtccggtcat 400

caacaaaggc tgcctgcgag ccaccagctg cggccttgag gaacccgtca 450

gctacagggg cgtcacctac agcctcacca ccaactgctg caccggccgc 500

ctgtgtaaca gagccccgag cagccagaca gtgggggcca ccaccagcct 550

ggcactgggg ctgggtatgc tgcttcctcc acgtttgctg tgaccaacag 600

ggaggacagg gcctgggact gttctcccag atccgccact ccccatgtcc 650

ccatgtcctt cccccactaa atggccagag aggccctgga caacctcttg 700

cggccctggc ttcatccctt ctaaggctgt ccaccaggag cccggtgcta 750

ggggaagcat ccccaggcct gactgagcgg caggggagca cggcccgtgg 800

gtttgattgt attactctgt tccactggtt ctaagacgca gagcttctca 850

catctcaatc aggatgcttc tctccattgg tagcacttta gagtccatga 900

aatatggtaa aaaatatata tatatcataa taaatgacag ctgatgttca 950

tgggggaaaa aaaaaaaaaa aaaaaaaaaa aaaaaaaaaa a 991

<210> 28
<211> 124
<212> PRT
<213> Homo Sapien

<400> 28
Met Val Leu Cys Trp Leu Leu Leu Leu Val Met Ala Leu Pro Pro
1               5                   10                  15

Gly Thr Thr Gly Val Lys Asp Cys Val Phe Cys Glu Leu Thr Asp
                20                  25                  30

Ser Met Gln Cys Pro Gly Thr Tyr Met His Cys Gly Asp Asp Glu
                35                  40                  45

Asp Cys Phe Thr Gly His Gly Val Ala Pro Gly Thr Gly Pro Val
                50                  55                  60

Ile Asn Lys Gly Cys Leu Arg Ala Thr Ser Cys Gly Leu Glu Glu
                65                  70                  75

Pro Val Ser Tyr Arg Gly Val Thr Tyr Ser Leu Thr Thr Asn Cys
                80                  85                  90

Cys Thr Gly Arg Leu Cys Asn Arg Ala Pro Ser Ser Gln Thr Val
                95                  100                 105

Gly Ala Thr Thr Ser Leu Ala Leu Gly Leu Gly Met Leu Leu Pro
                110                 115                 120

Pro Arg Leu Leu

<210> 29
<211> 2020
<212> DNA
<213> Homo Sapien

<400> 29
ggcattttga aagcccagtg ttgcccaggg ggcatctcct ttgtgtttat 50

gagagacctg cattctccct ggctcagttc tctcaggctc tccagagctc 100

aggacctctg agaagaatgg agccctcctg gcttcaggaa ctcatggctc 150

accccttctt gctgctgatc ctcctctgca tgtctctgct gctgtttcag 200

gtaatcaggt tgtaccagag gaggagatgg atgatcagag ccctgcacct 250

gtttcctgca ccccctgccc actggttcta tggccacaag gagttttacc 300

cagtaaagga gtttgaggtg tatcataagc tgatggaaaa atacccatgt 350

gctgttccct tgtgggttgg accctttacg atgttcttca gtgtccatga 400

cccagactat gccaagattc tcctgaaaag acaagatccc aaaagtgctg 450

ttagccacaa aatccttgaa tcctgggttg gtcgaggact tgtgaccctg 500

gatggttcta aatggaaaaa gcaccgccag attgtgaaac ctggcttcaa 550

catcagcatt ctgaaaatat tcatcaccat gatgtctgag agtgttcgga 600

tgatgctgaa caaatgggag gaacacattg cccaaaactc acgtctggag 650

ctctttcaac atgtctccct gatgaccctg gacagcatca tgaagtgtgc 700

cttcagccac cagggcagca tccagttgga cagtaccctg gactcatacc 750

tgaaagcagt gttcaacctt agcaaaatct ccaaccagcg catgaacaat 800

tttctacatc acaacgacct ggttttcaaa ttcagctctc aaggccaaat 850

cttttctaaa tttaaccaag aacttcatca gttcacagag aaagtaatcc 900

aggaccggaa ggagtctctt aaggataagc taaaacaaga tactactcag 950

aaaaggcgct gggatttctt ggacatactt ttgagtgcca aaagcgaaaa 1000

caccaaagat ttctctgaag cagatctcca ggctgaagtg aaaacgttca 1050

tgtttgcagg acatgacacc acatccagtg ctatctcctg gatcctttac 1100

tgcttggcaa agtaccctga gcatcagcag agatgccgag atgaaatcag 1150

ggaactccta ggggatgggt cttctattac ctgggaacac ctgagccaga 1200

tgccttacac cacgatgtgc atcaaggaat gcctccgcct ctacgcaccg 1250

gtagtaaaca tatcccggtt actcgacaaa cccatcacct ttccagatgg 1300

acgctcctta cctgcaggaa taactgtgtt tatcaatatt tgggctcttc 1350

accacaaccc ctatttctgg gaagaccctc aggtctttaa ccccttgaga 1400

ttctccaggg aaaattctga aaaaatacat ccctatgcct tcataccatt 1450

ctcagctgga ttaaggaact gcattgggca gcattttgcc ataattgagt 1500

gtaaagtggc agtggcatta actctgctcc gcttcaagct ggctccagac 1550

cactcaaggc ctccccagcc tgttcgtcaa gttgtcctca agtccaagaa 1600

tggaatccat gtgtttgcaa aaaaagtttg ctaattttaa gtcctttcgt 1650

ataagaatta atgagacaat tttcctacca aaggaagaac aaaaggataa 1700

atataataca aaatatatgt atatggttgt ttgacaaatt atataactta 1750

ggatacttct gactggtttt gacatccatt aacagtaatt ttaatttctt 1800

tgctgtatct ggtgaaaccc acaaaaacac ctgaaaaaac tcaagctgac 1850

ttccactgcg aagggaaatt attggtttgt gtaactagtg gtagagtggc 1900

tttcaagcat agtttgatca aaactccact cagtatctgc attactttta 1950

tctctgcaaa tatctgcatg atagctttat tctcagttat ctttccccat 2000

aataaaaaat atctgccaaa 2020

<210> 30
<211> 505
<212> PRT
<213> Homo Sapien

<400> 30
Met Glu Pro Ser Trp Leu Gln Glu Leu Met Ala His Pro Phe Leu
1               5                   10                  15

Leu Leu Ile Leu Leu Cys Met Ser Leu Leu Leu Phe Gln Val Ile
                20                  25                  30

Arg Leu Tyr Gln Arg Arg Arg Trp Met Ile Arg Ala Leu His Leu
                35                  40                  45

Phe Pro Ala Pro Pro Ala His Trp Phe Tyr Gly His Lys Glu Phe
                50                  55                  60

Tyr Pro Val Lys Glu Phe Glu Val Tyr His Lys Leu Met Glu Lys
                65                  70                  75

Tyr Pro Cys Ala Val Pro Leu Trp Val Gly Pro Phe Thr Met Phe
                80                  85                  90

Phe Ser Val His Asp Pro Asp Tyr Ala Lys Ile Leu Leu Lys Arg
                95                  100                 105

Gln Asp Pro Lys Ser Ala Val Ser His Lys Ile Leu Glu Ser Trp
                110                 115                 120

Val Gly Arg Gly Leu Val Thr Leu Asp Gly Ser Lys Trp Lys Lys
                125                 130                 135

His Arg Gln Ile Val Lys Pro Gly Phe Asn Ile Ser Ile Leu Lys
                140                 145                 150

Ile Phe Ile Thr Met Met Ser Glu Ser Val Arg Met Met Leu Asn
                155                 160                 165

Lys Trp Glu Glu His Ile Ala Gln Asn Ser Arg Leu Glu Leu Phe
                170                 175                 180

Gln His Val Ser Leu Met Thr Leu Asp Ser Ile Met Lys Cys Ala
                185                 190                 195

Phe Ser His Gln Gly Ser Ile Gln Leu Asp Ser Thr Leu Asp Ser
                200                 205                 210

Tyr Leu Lys Ala Val Phe Asn Leu Ser Lys Ile Ser Asn Gln Arg
                215                 220                 225

Met Asn Asn Phe Leu His His Asn Asp Leu Val Phe Lys Phe Ser
                230                 235                 240

Ser Gln Gly Gln Ile Phe Ser Lys Phe Asn Gln Glu Leu His Gln
                245                 250                 255

Phe Thr Glu Lys Val Ile Gln Asp Arg Lys Glu Ser Leu Lys Asp
                260                 265                 270

Lys Leu Lys Gln Asp Thr Thr Gln Lys Arg Arg Trp Asp Phe Leu
                275                 280                 285

Asp Ile Leu Leu Ser Ala Lys Ser Glu Asn Thr Lys Asp Phe Ser
                290                 295                 300

Glu Ala Asp Leu Gln Ala Glu Val Lys Thr Phe Met Phe Ala Gly
                305                 310                 315

His Asp Thr Thr Ser Ser Ala Ile Ser Trp Ile Leu Tyr Cys Leu
                320                 325                 330

Ala Lys Tyr Pro Glu His Gln Gln Arg Cys Arg Asp Glu Ile Arg
                335                 340                 345

Glu Leu Leu Gly Asp Gly Ser Ser Ile Thr Trp Glu His Leu Ser
                350                 355                 360

Gln Met Pro Tyr Thr Thr Met Cys Ile Lys Glu Cys Leu Arg Leu
                365                 370                 375

Tyr Ala Pro Val Val Asn Ile Ser Arg Leu Leu Asp Lys Pro Ile
                380                 385                 390

Thr Phe Pro Asp Gly Arg Ser Leu Pro Ala Gly Ile Thr Val Phe
                395                 400                 405

Ile Asn Ile Trp Ala Leu His His Asn Pro Tyr Phe Trp Glu Asp
                410                 415                 420

Pro Gln Val Phe Asn Pro Leu Arg Phe Ser Arg Glu Asn Ser Glu
                425                 430                 435

Lys Ile His Pro Tyr Ala Phe Ile Pro Phe Ser Ala Gly Leu Arg
                440                 445                 450

Asn Cys Ile Gly Gln His Phe Ala Ile Ile Glu Cys Lys Val Ala
                455                 460                 465

Val Ala Leu Thr Leu Leu Arg Phe Lys Leu Ala Pro Asp His Ser
                470                 475                 480

Arg Pro Pro Gln Pro Val Arg Gln Val Val Leu Lys Ser Lys Asn
                485                 490                 495

Gly Ile His Val Phe Ala Lys Lys Val Cys
                500                 505

<210> 31
<211> 1118
<212> DNA
<213> Homo Sapien

<400> 31
tccgctgtcg cccagtcccg gccgctggcg ggaactgacc tggagcaagc 50

aggaccttcc ctcccacctc tcccgcctgg cctccgcggg agtcccctac 100

gatcccgctc agcagtgggg cactcgctga ggacagcgag tcctgggagt 150

gagcccaagg ccacccctgg ccagcccagg agagatagcc agggcaggcc 200

cagcagcccg aggccaggct ctggccacgg cggtctccga catggagaga 250

cattgtctgc tttttatcct gttaacctgt cttcggtggt tgtgccacga 300

cattccccag ggttcaggtg cccggtggcc gagggtcagt ccagtggtag 350

agccttgctc tcctaggctc atcctgctgg cggtcctcct gcttctgctg 400

tgtggtgtca cagctggttg tgtccggttc tgctgcctcc ggaagcaggc 450

acaggcccag ccacatctgc caccagcacg gcagccctgc gacgtggcag 500

tcatccctat ggacagtgac agccctgtac acagcactgt gacctcctac 550

agctccgtgc agtacccact gggcatgcgg ttgcccctgc cctttgggga 600

gctggacctg gactccacgg ctcctcctgc ctacagcctg tacaccccgg 650

agcctccacc ctcctacgat gaagctgtca agatggccaa gcccagagag 700

gaaggaccag cactctccca gaaacccagc cctctccttg gggcctcggg 750

cctagagacc actccagtgc cccaggagtc gggccccaat actcaactac 800

caccttgtag ccctggtgcc ccttgaagga ggtaggagaa cggaccagag 850

cttggagaac taatgcttgg agccaagggc cccagcccac cccaccgtcc 900

cacacattgc tgtggcccca acctcggtgc catgttacac cggcccctgg 950

cgtcacccac taggcaggct gctgctttca gcctcagccc ctggcccagc 1000

cccagcaggc cctcagcctg gaagaggccc cttgggccta agcctcgggt 1050

gggagctcag ggccacctgt gacgtctgca tcttcttgga gagagaataa 1100

agtttgtatt taagtggt 1118

<210> 32
<211> 194
<212> PRT
<213> Homo Sapien

<400> 32

<210> 33
<211> 1976
<212> DNA
<213> Homo Sapien

<400> 33
ccttgcttgg tgcttggcac acacaaatcc agtgggctac acaggttttc 50

cagaagcccc acgaggtggt aatggtgctg ctgattcaga ccctgggggc 100

cctcatgccc tcgctgccct cctgcctcag caacggcgtg gagagggcag 150

ggcccgagca ggagctcacc aggctgctgg agttctacga cgccaccgcc 200

cacttcgcca agggcttgga gatggcactg ctcccccacc tacatgaaca 250

caatctggta aaagtcacgg agctggtgga tgctgtgtat gatccataca 300

aaccctacca gctgaagtat ggcgacatgg aagagagcaa cctcctcatc 350

cagatgagtg ctgtgcctct ggagcatggg gaagtgattg actgtgtgca 400

ggagctgagc cactccgtga acaagctgtt tggtctggcg tctgcagccg 450

ttgacagatg cgtcagattc accaatggcc tggggacctg cggcctgttg 500

tcagccctga aatccctctt tgccaagtat gtgtctgatt tcaccagcac 550

tctccagtcc atacgaaaga agtgcaaact ggaccacatt cctcccaact 600

ccctcttcca ggaagattgg acggcttttc agaactccat taggataata 650

gccacctgtg gagagctttt gcggcattgt ggggacttcg agcagcagct 700

agccaacagg attttgtcca cagctgggaa gtatctatct gattcctgca 750

gcccccggag cctggctggt tttcaggaga gcatcttgac agacaagaag 800

aactctgcca agaacccatg gcaagaatat aattacctcc agaaagataa 850

ccctgctgaa tatgccagtt taatggaaat actttatacc cttaaggaaa 900

aagggtcaag caaccacaac ctgctggctg cacctcgagc agcgctgact 950

cggcttaacc agcaggccca ccagctggct ttcgattccg tgttcctgcg 1000

catcaaacaa cagctgttgc ttatttcgaa gatggacagc tggaatacgg 1050

ctggcatcgg agaaaccctc acagatgaac tgcccgcctt tagtctcacc 1100

cctctcgagt acatcagcaa catcgggcag tacatcatgt ccctccccct 1150

gaatcttgag ccatttgtga ctcaggagga ctctgcctta gagttggcat 1200

tgcacgctgg aaagctgcca tttcctcctg agcaggggga tgaattgccc 1250

gagctggaca acatggctga caactggctg ggctcgatcg ccagagccac 1300

aatgcagacc tactgtgatg cgatcctaca gatccctgag ctgagcccac 1350

actctgccaa gcagctggcc actgacatcg actatctgat caacgtgatg 1400

gatgccctgg gcctgcagcc gtcccgcacc ctccagcaca tcgtgacgct 1450

actgaagacc aggcctgagg actatagaca ggtcagcaaa ggcctgcccc 1500

gtcgcctggc caccaccgtg gccaccatgc ggagtgtgaa ttactgaccc 1550

caccacacac cggaccacca agagagccag ggctgctgtt tcgtgactca 1600

ccagcacaga tttgctcaga aactctgccc aagattgggc agaagttact 1650

ttaaaaagac ttggttcagc tggtcacggt ggctcacgcc tgtaatccca 1700

gcactttggg aggccaagcc agatggatca tgaggccagg agttcgagac 1750

cagcctgacc aacatggtga aaccccatct ctactaaaaa tacaaaaatt 1800

aacagcagag cgagactctg tctcaaaaaa aaaaaaaaaa agacttggtt 1850

catttgtata atcaaaaaga gttgtaaatt aaagatgtat tatttatcag 1900

agaagacttt ttagataatt tttttaaagg atcagatctt gaaaatggaa 1950

taaataacta ctgtgaaatg caaaaa 1976

<210> 34
<211> 491
<212> PRT
<213> Homo Sapien

<400> 34
Met Val Leu Leu Ile Gln Thr Leu Gly Ala Leu Met Pro Ser Leu
1               5                   10                  15

Pro Ser Cys Leu Ser Asn Gly Val Glu Arg Ala Gly Pro Glu Gln
                20                  25                  30

Glu Leu Thr Arg Leu Leu Glu Phe Tyr Asp Ala Thr Ala His Phe
                35                  40                  45

Ala Lys Gly Leu Glu Met Ala Leu Leu Pro His Leu His Glu His
                50                  55                  60

Asn Leu Val Lys Val Thr Glu Leu Val Asp Ala Val Tyr Asp Pro
                65                  70                  75

Tyr Lys Pro Tyr Gln Leu Lys Tyr Gly Asp Met Glu Glu Ser Asn
                80                  85                  90

Leu Leu Ile Gln Met Ser Ala Val Pro Leu Glu His Gly Glu Val
                95                  100                 105

Ile Asp Cys Val Gln Glu Leu Ser His Ser Val Asn Lys Leu Phe
                110                 115                 120

Gly Leu Ala Ser Ala Ala Val Asp Arg Cys Val Arg Phe Thr Asn
                125                 130                 135

Gly Leu Gly Thr Cys Gly Leu Leu Ser Ala Leu Lys Ser Leu Phe
                140                 145                 150

Ala Lys Tyr Val Ser Asp Phe Thr Ser Thr Leu Gln Ser Ile Arg
                155                 160                 165

Lys Lys Cys Lys Leu Asp His Ile Pro Pro Asn Ser Leu Phe Gln
                170                 175                 180

Glu Asp Trp Thr Ala Phe Gln Asn Ser Ile Arg Ile Ile Ala Thr
                185                 190                 195

Cys Gly Glu Leu Leu Arg His Cys Gly Asp Phe Glu Gln Gln Leu
                200                 205                 210

Ala Asn Arg Ile Leu Ser Thr Ala Gly Lys Tyr Leu Ser Asp Ser
                215                 220                 225

Cys Ser Pro Arg Ser Leu Ala Gly Phe Gln Glu Ser Ile Leu Thr
                230                 235                 240

Asp Lys Lys Asn Ser Ala Lys Asn Pro Trp Gln Glu Tyr Asn Tyr
                245                 250                 255

Leu Gln Lys Asp Asn Pro Ala Glu Tyr Ala Ser Leu Met Glu Ile
                260                 265                 270

Leu Tyr Thr Leu Lys Glu Lys Gly Ser Ser Asn His Asn Leu Leu
                275                 280                 285

Ala Ala Pro Arg Ala Ala Leu Thr Arg Leu Asn Gln Gln Ala His
                290                 295                 300

Gln Leu Ala Phe Asp Ser Val Phe Leu Arg Ile Lys Gln Gln Leu
                305                 310                 315

Leu Leu Ile Ser Lys Met Asp Ser Trp Asn Thr Ala Gly Ile Gly
                320                 325                 330

Glu Thr Leu Thr Asp Glu Leu Pro Ala Phe Ser Leu Thr Pro Leu
                335                 340                 345

Glu Tyr Ile Ser Asn Ile Gly Gln Tyr Ile Met Ser Leu Pro Leu
                350                 355                 360

Asn Leu Glu Pro Phe Val Thr Gln Glu Asp Ser Ala Leu Glu Leu

                365                 370                 375

Ala Leu His Ala Gly Lys Leu Pro Phe Pro Pro Glu Gln Gly Asp
                380                 385                 390

Glu Leu Pro Glu Leu Asp Asn Met Ala Asp Asn Trp Leu Gly Ser
                395                 400                 405

Ile Ala Arg Ala Thr Met Gln Thr Tyr Cys Asp Ala Ile Leu Gln
                410                 415                 420

Ile Pro Glu Leu Ser Pro His Ser Ala Lys Gln Leu Ala Thr Asp
                425                 430                 435

Ile Asp Tyr Leu Ile Asn Val Met Asp Ala Leu Gly Leu Gln Pro
                440                 445                 450

Ser Arg Thr Leu Gln His Ile Val Thr Leu Leu Lys Thr Arg Pro
                455                 460                 465

Glu Asp Tyr Arg Gln Val Ser Lys Gly Leu Pro Arg Arg Leu Ala
                470                 475                 480

Thr Thr Val Ala Thr Met Arg Ser Val Asn Tyr
                485                 490

<210> 35
<211> 1227
<212> DNA
<213> Homo Sapien

<400> 35
gcaagtgcca ccatgctagt gtgatttgga cttcagtaaa agttagtttg 50

cttccttccc gttgtcccat ctcactcctg ggccacccat ggggctgctg 100

gtagctggtg tgtggctgct gctggactgt gtggcagtcc atccatctgt 150

cagcagccac tgcgggccta cttgctgggt gcccagcacc gcactcacca 200

ctgcaggcgt ggccaggagc gtgagatccc cagagcccat ggccagtgag 250

aggcggccag ggataggtac ccagggaatg ccacaggagt ttgctgggct 300

cacggagctc tttcactggt cagagaggag tgtgtgtagg agaggacttc 350

tacttggtgt tgaaggacag atggggtttg gctgggagag aggaggaatg 400

tgggcgggcc ttataggcag gcgagaaggt gagagccaag gccctctgtg 450

ggcagggcga ggtggcgtgt tgaggagact cgtccagctg ggcagaggct 500

catgttgagg gatgaggcag agctggggga ggagggagcc cagaaatggc 550

aggtccttga atgcaggttt ggaagcaggg acgccctgtg agggtacaga 600

gtctgggctg ttaccttctg tggcttttgc tagaaggtga gatgtcaggg 650

aggaagacag gactccagga tgtctcctgt ctctctctgg aaaaaggagg 700

tgggcccctt tctcagcagt cagctgctgt ttttgaggtc ttctccatgg 750

ataatccacg gtgttggaag tggttaaggt aatggatcct catgggctta 800

ccataaaaat atctggaggc tggaccattt tccttaaaac gttataaaag 850

ctggaattga atgccatcgg tgtcacccct gggaagtgtg ctttctcttg 900

agctcttttg gccccgagat agcagtcact ccatagtttc gtgaagacca 950

gcctggtgtt gcctggtttt ctgccattag ggagcagcta gaggtcttcc 1000

agtagctcct gtgtaaagtg atgaaagaaa agggctgggt gctgactgct 1050

cctggagaaa agcaacacac tcccaaagtc ttaattgcct gcttccaggg 1100

agctgtggtg gtttcccttg ggcagggcac acgccccagt ggttgactta 1150

ataaggatac attttaatca gaggacaaaa atgtgccctg acttgatttc 1200

cgcatgggct tccagcatgg tcaaagg 1227

<210> 36
<211> 139
<212> PRT
<213> Homo Sapien

<400> 36
Met Gly Leu Leu Val Ala Gly Val Trp Leu Leu Leu Asp Cys Val
1               5                   10                  15

Ala Val His Pro Ser Val Ser Ser His Cys Gly Pro Thr Cys Trp
                20                  25                  30

Val Pro Ser Thr Ala Leu Thr Thr Ala Gly Val Ala Arg Ser Val
                35                  40                  45

Arg Ser Pro Glu Pro Met Ala Ser Glu Arg Arg Pro Gly Ile Gly
                50                  55                  60

Thr Gln Gly Met Pro Gln Glu Phe Ala Gly Leu Thr Glu Leu Phe
                65                  70                  75

His Trp Ser Glu Arg Ser Val Cys Arg Arg Gly Leu Leu Leu Gly
                80                  85                  90

Val Glu Gly Gln Met Gly Phe Gly Trp Glu Arg Gly Gly Met Trp
                95                  100                 105

Ala Gly Leu Ile Gly Arg Arg Glu Gly Glu Ser Gln Gly Pro Leu
                110                 115                 120

Trp Ala Gly Arg Gly Gly Val Leu Arg Arg Leu Val Gln Leu Gly
                125                 130                 135

Arg Gly Ser Cys

<210> 37
<211> 1225
<212> DNA
<213> Homo Sapien

<400> 37
ggccaggaat ggggtccccg ggcatggtgc tgggcctcct ggtgcagatc 50

tgggccctgc aagaagcctc aagcctgagc gtgcagcagg ggcccaactt 100

gctgcaggtg aggcagggca gtcaggcgac cctggtctgc caggtggacc 150

aggccacagc ctgggaacgg ctccgtgtta agtggacaaa ggatggggcc 200

atcctgtgtc aaccgtacat caccaacggc agcctcagcc tgggggtctg 250

cgggccccag ggacggctct cctggcaggc acccagccat ctcaccctgc 300

agctggaccc tgtgagcctc aaccacagcg gggcgtacgt gtgctgggcg 350

gccgtagaga ttcctgagtt ggaggaggct gagggcaaca taacaaggct 400

ctttgtggac ccagatgacc ccacacagaa cagaaaccgg atcgcaagct 450

tcccaggatt cctcttcgtg ctgctggggg tgggaagcat gggtgtggct 500

gcgatcgtgt ggggtgcctg gttctggggc cgccgcagct gccagcaaag 550

ggactcagga aatgcattct acagcaacgt cctataccgg ccccgggggg 600

ccccaaagaa gagtgaggac tgctctggag aggggaagga ccagaggggc 650

cagagcattt attcaacctc cttcccgcaa ccggcccccc gccagccgca 700

cctggcgtca agaccctgcc ccagcccgag accctgcccc agccccaggc 750

ccggccaccc cgtctctatg gtcagggtct ctcctagacc aagccccacc 800

cagcagccga ggccaaaagg gttccccaaa gtgggagagg agtgagagat 850

cccaggagac ctcaacagga ccccacccat aggtacacac aaaaaagggg 900

ggatcgaggc cagacacggt ggctcacgcc tgtaatccca gcagtttggg 950

aagccgaggc gggtggaaca cttgaggtca ggggtttgag accagcctgg 1000

cttgaacctg ggaggcggag gttgcagtga gccgagattg cgccactgca 1050

ctccagcctg ggcgacagag tgagactccg tctcaaaaaa aaaacaaaaa 1100

gcaggaggat tgggagcctg tcagccccat cctgagaccc cgtcctcatt 1150

tctgtaatga tggatctcgc tcccactttc ccccaagaac ctaataaagg 1200

cttgtgaaga aaaaaaaaaa aaaaa 1225

<210> 38
<211> 278
<212> PRT
<213> Homo Sapien

<400> 38
Met Gly Ser Pro Gly Met Val Leu Gly Leu Leu Val Gln Ile Trp
1               5                   10                  15

Ala Leu Gln Glu Ala Ser Ser Leu Ser Val Gln Gln Gly Pro Asn
                20                  25                  30

Leu Leu Gln Val Arg Gln Gly Ser Gln Ala Thr Leu Val Cys Gln
                35                  40                  45

Val Asp Gln Ala Thr Ala Trp Glu Arg Leu Arg Val Lys Trp Thr
                50                  55                  60

Lys Asp Gly Ala Ile Leu Cys Gln Pro Tyr Ile Thr Asn Gly Ser
                65                  70                  75

Leu Ser Leu Gly Val Cys Gly Pro Gln Gly Arg Leu Ser Trp Gln
                80                  85                  90

Ala Pro Ser His Leu Thr Leu Gln Leu Asp Pro Val Ser Leu Asn
                95                  100                 105

His Ser Gly Ala Tyr Val Cys Trp Ala Ala Val Glu Ile Pro Glu
                110                 115                 120

Leu Glu Glu Ala Glu Gly Asn Ile Thr Arg Leu Phe Val Asp Pro
                125                 130                 135

Asp Asp Pro Thr Gln Asn Arg Asn Arg Ile Ala Ser Phe Pro Gly
                140                 145                 150

Phe Leu Phe Val Leu Leu Gly Val Gly Ser Met Gly Val Ala Ala
                155                 160                 165

Ile Val Trp Gly Ala Trp Phe Trp Gly Arg Arg Ser Cys Gln Gln
                170                 175                 180

Arg Asp Ser Gly Asn Ala Phe Tyr Ser Asn Val Leu Tyr Arg Pro
                185                 190                 195

Arg Gly Ala Pro Lys Lys Ser Glu Asp Cys Ser Gly Glu Gly Lys
                200                 205                 210

Asp Gln Arg Gly Gln Ser Ile Tyr Ser Thr Ser Phe Pro Gln Pro
                215                 220                 225

Ala Pro Arg Gln Pro His Leu Ala Ser Arg Pro Cys Pro Ser Pro
                230                 235                 240

Arg Pro Cys Pro Ser Pro Arg Pro Gly His Pro Val Ser Met Val
                245                 250                 255

Arg Val Ser Pro Arg Pro Ser Pro Thr Gln Gln Pro Arg Pro Lys
                260                 265                 270

Gly Phe Pro Lys Val Gly Glu Glu
                275

<210> 39
<211> 1837
<212> DNA
<213> Homo Sapien

<400> 39
accagcagaa ggctgggagt ctgtagtttg ttcctgctgc caggctccac 50

tgaggggaac ggggacctgt ctgaagagaa gatgcccctg ctgacactct 100

acctgctcct cttctggctc tcaggctact ccattgccac tcaaatcacc 150

ggtccaacaa cagtgaatgg cttggagcgg ggctccttga ccgtgcagtg 200

tgtttacaga tcaggctggg agacctactt gaagtggtgg tgtcgaggag 250

ctatttggcg tgactgcaag atccttgtta aaaccagtgg gtcagagcag 300

gaggtgaaga gggaccgggt gtccatcaag gacaatcaga aaaaccgcac 350

gttcactgtg accatggagg atctcatgaa aactgatgct gacacttact 400

ggtgtggaat tgagaaaact ggaaatgacc ttggggtcac agttcaagtg 450

accattgacc cagcaccagt cacccaagaa gaaactagca gctccccaac 500

tctgaccggc caccacttgg acaacaggca caagctcctg aagctcagtg 550

tcctcctgcc cctcatcttc accatattgc tgctgctttt ggtggccgcc 600

tcactcttgg cttggaggat gatgaagtac cagcagaaag cagccgggat 650

gtccccagag caggtactgc agcccctgga gggcgacctc tgctatgcag 700

acctgaccct gcagctggcc ggaacctccc cgcgaaaggc taccacgaag 750

ctttcctctg cccaggttga ccaggtggaa gtggaatatg tcaccatggc 800

ttccttgccg aaggaggaca tttcctatgc atctctgacc ttgggtgctg 850

aggatcagga accgacctac tgcaacatgg gccacctcag tagccacctc 900

cccggcaggg gccctgagga gcccacggaa tacagcacca tcagcaggcc 950

ttagcctgca ctccaggctc cttcttggac cccaggctgt gagcacactc 1000

ctgcctcatc gaccgtctgc cccctgctcc cctcatcagg accaacccgg 1050

ggactggtgc ctctgcctga tcagccagca ttgcccctag ctctgggttg 1100

ggcttggggc caagtctcag ggggcttcta ggagttgggg ttttctaaac 1150

gtcccctcct ctcctacata gttgaggagg gggctaggga tatgctctgg 1200

ggctttcatg ggaatgatga agatgataat gagaaaaatg ttatcattat 1250

tatcatgaag taccattatc ataatacaat gaacctttat ttattgccta 1300

ccacatgtta tgggctgaat aatggccccc aaagatatct gtgtcctaat 1350

cctcagaact tgtgactgtt accttctgtg gcagaaaggg acagtgcaga 1400

tgtatgtaag ttaaggactt tgagatagag aggttattct tgctgattca 1450

ggtgggccca aaatatcacc acaagggtcc tcataagaaa gaggccagaa 1500

ggtcaaagag gtagagacaa agtgatgatg gaagtggacg tgggtgtgac 1550

gtgagcaggg gccatgaatg ccgcagcctt cagatgccag aaagggaaag 1600

gaatggattc ccctgcctgg agcctccaaa agaaaccagc cctgcccacg 1650

ccttgacttg agcccattga aactgatctt gagctcctgg cctccagaat 1700

tgcaggagaa taaatttgtg ttgtttttaa tgaaaaaaaa aaaaaaaaaa 1750

aaaaaaaaaa aaaaaaaaaa aaaaaaaaaa aaaaaaaaaa aaaaaaaaaa 1800

aaaaaaaaaa aaaaaaaaaa aaaaaaaaaa aaaaaag 1837

<210> 40
<211> 290
<212> PRT
<213> Homo Sapien

<400> 40

<210> 41
<211> 2683
<212> DNA
<213> Homo Sapien

<400> 41
aagaacactg ttgctcttgg tggacgggcc cagaggaatt cagagttaaa 50

ccttgagtgc ctgcgtccgt gagaattcag catggaatgt ctctactatt 100

tcctgggatt tctgctcctg gctgcaagat tgccacttga tgccgccaaa 150

cgatttcatg atgtgctggg caatgaaaga ccttctgctt acatgaggga 200

gcacaatcaa ttaaatggct ggtcttctga tgaaaatgac tggaatgaaa 250

aactctaccc agtgtggaag cggggagaca tgaggtggaa aaactcctgg 300

aagggaggcc gtgtgcaggc ggtcctgacc agtgactcac cagccctcgt 350

gggctcaaat ataacatttg cggtgaacct gatattccct agatgccaaa 400

aggaagatgc caatggcaac atagtctatg agaagaactg cagaaatgag 450

gctggtttat ctgctgatcc gtatgtttac aactggacag catggtcaga 500

ggacagtgac ggggaaaatg gcaccggcca aagccatcat aacgtcttcc 550
ctgatgggaa accttttcct caccaccccg gatggagaag atggaatttc 600
atctacgtct tccacacact tggtcagtat ttccagaaat tgggacgatg 650
ttcagtgaga gtttctgtga acacagccaa tgtgacactt gggcctcaac 700
tcatggaagt gactgtctac agaagacatg gacgggcata tgttcccatc 750
gcacaagtga aagatgtgta cgtggtaaca gatcagattc ctgtgtttgt 800
gactatgttc cagaagaacg atcgaaattc atccgacgaa accttcctca 850
aagatctccc cattatgttt gatgtcctga ttcatgatcc tagccacttc 900
ctcaattatt ctaccattaa ctacaagtgg agcttcgggg ataatactgg 950
cctgtttgtt tccaccaatc atactgtgaa tcacacgtat gtgctcaatg 1000
gaaccttcag ccttaacctc actgtgaaag ctgcagcacc aggaccttgt 1050
ccgccaccgc caccaccacc cagaccttca aaacccaccc cttctttagc 1100
aactactcta aaatcttatg attcaaacac cccaggacct actggtgaca 1150
accccctgga gctgagtagg attcctgatg aaaactgcca gattaacaga 1200
tatggccact ttcaagccac catcacaatt gtagagggaa tcttagaggt 1250
taacatcatc cagatgacag acgtcctgat gccggtgcca tggcctgaaa 1300
gctccctaat agactttgtc gtgacctgcc aagggagcat tcccacggag 1350
gtctgtacca tcatttctga ccccacctgc gagatcaccc agaacacagt 1400
ctgcagccct gtggatgtgg atgagatgtg tctgctgact gtgagacgaa 1450
ccttcaatgg gtctgggacg tactgtgtga acctcaccct gggggatgac 1500
acaagcctgg ctctcacgag caccctgatt tctgttcctg acagagaccc 1550
agcctcgcct ttaaggatgg caaacagtgc cctgatctcc gttggctgct 1600
tggccatatt tgtcactgtg atctccctct tggtgtacaa aaaacacaag 1650
gaatacaacc caatagaaaa tagtcctggg aatgtggtca gaagcaaagg 1700
cctgagtgtc tttctcaacc gtgcaaaagc cgtgttcttc ccgggaaacc 1750
aggaaaagga tccgctactc aaaaaccaag aatttaaagg agtttcttaa 1800
atttcgacct tgtttctgaa gctcactttt cagtgccatt gatgtgagat 1850
gtgctggagt ggctattaac ctttttttcc taaagattat tgttaaatag 1900
atattgtggt ttggggaagt tgaatttttt ataggttaaa tgtcatttta 1950
gagatgggga gagggattat actgcaggca gcttcagcca tgttgtgaaa 2000
ctgataaaag caacttagca aggcttcttt tcattatttt ttatgtttca 2050
cttataaagt cttaggtaac tagtaggata gaaacactgt gtcccgagag 2100
taaggagaga agctactatt gattagagcc taacccaggt taactgcaag 2150
aagaggcggg atactttcag ctttccatgt aactgtatgc ataaagccaa 2200

tgtagtccag tttctaagat catgttccaa gctaactgaa tcccacttca 2250

atacacactc atgaactcct gatggaacaa taacaggccc aagcctgtgg 2300

tatgatgtgc acacttgcta gactcagaaa aaatactact ctcataaatg 2350

ggtgggagta ttttggtgac aacctacttt gcttggctga gtgaaggaat 2400

gatattcata tattcattta ttccatggac atttagttag tgctttttat 2450

ataccaggca tgatgctgag tgacactctt gtgtatattt ccaaattttt 2500

gtacagtcgc tgcacatatt tgaaatcata tattaagact ttccaaagat 2550

gaggtccctg gtttttcatg gcaacttgat cagtaaggat ttcacctctg 2600

tttgtaacta aaaccatcta ctatatgtta gacatgacat tctttttctc 2650

tccttcctga aaaataaagt gtgggaagag aca 2683

<210> 42
<211> 572
<212> PRT
<213> Homo Sapien

<400> 42

<210> 43
<211> 1434
<212> DNA
<213> Homo Sapien

<400> 43
gcatagatga atgtatcagt ggatggatag ttggctagat gggtgggttg 50
gtggatgaat ggcagagctt gcacctgcca gtccatctga catcaaagcc 100
agtgtctcta atggtgacac caccctcctc tgcagcagga ggcagagctg 150
tgggatgaat gaggttcgcc aggtctccct tacctatcct gggtccccag 200
ctccttctca ctctcttccc ttgcagcctc gaagcggagg atccctgtgt 250
cccagccggg catggccgac ccccaccagc ttttcgatga cacaagttca 300
gcccagagcc ggggctatgg ggcccagcgg gcacctggtg gcctgagtta 350
tcctgcagcc tctcccacgc cccatgcagc cttcctggct gacccggtgt 400
ccaacatggc catggcctat gggagcagcc tggccgcgca gggcaaggag 450
ctggtggata agaacatcga ccgcttcatc cccatcacca agctcaagta 500
ttactttgct gtggacacca tgtatgtggg cagaaagctg ggcctgctgt 550
tcttccccta cctacaccag gactgggaag tgcagtacca acaggacacc 600
ccggtggccc cccgctttga cgtcaatgcc ccggacctct acattccagc 650
aatggctttc atcacctacg ttttggtggc tggtcttgcg ctggggaccc 700
aggataggtt ctccccagac ctcctggggc tgcaagcgag ctcagccctg 750
gcctggctga ccctggaggt gctggccatc ctgctcagcc tctatctggt 800
cactgtcaac accgacctca ccaccatcga cctggtggcc ttcttgggct 850
acaaatatgt cgggatgatt ggcggggtcc tcatgggcct gctcttcggg 900
aagattggct actacctggt gctgggctgg tgctgcgtag ccatctttgt 950
gttcatgatc cggacgctgc ggctgaagat cttggcagac gcagcagctg 1000
agggggtccc ggtgcgtggg gcccggaacc agctgcgcat gtacctgacc 1050
atggcggtgg cggcggcgca gcctatgctc atgtactggc tcaccttcca 1100
cctggtgcgg tgagcgcgcc cgctgaacct cccgctgctg ctgctgctgc 1150
tgggggccac tgtggccgcc gaactcatct cctgcctgca ggccccaagg 1200
tccaccctgt ctggccacag gcaccgcctc catcccatgt cccgcccagc 1250
cccgccccca acccaaggtg ctgagagatc tccagctgca caggccaccg 1300
ccccagggcg tggccgctgt tacagaaaca ataaaccctg atgggcatgg 1350

caaaaaaaaa aaaaaaaaaa aaaaaaaaaa aaaaaaaaaa aaaaaaaaaa 1400

aaaaaaaaaa aaaaaaaaaa aaaaaaaaaa aaga 1434

<210> 44
<211> 283
<212> PRT
<213> Homo Sapien

<400> 44
Met Ala Asp Pro His Gln Leu Phe Asp Asp Thr Ser Ser Ala Gln
1               5                   10                  15

Ser Arg Gly Tyr Gly Ala Gln Arg Ala Pro Gly Gly Leu Ser Tyr
                20                  25                  30

Pro Ala Ala Ser Pro Thr Pro His Ala Ala Phe Leu Ala Asp Pro
                35                  40                  45

Val Ser Asn Met Ala Met Ala Tyr Gly Ser Ser Leu Ala Ala Gln
                50                  55                  60

Gly Lys Glu Leu Val Asp Lys Asn Ile Asp Arg Phe Ile Pro Ile
                65                  70                  75

Thr Lys Leu Lys Tyr Tyr Phe Ala Val Asp Thr Met Tyr Val Gly
                80                  85                  90

Arg Lys Leu Gly Leu Leu Phe Phe Pro Tyr Leu His Gln Asp Trp
                95                  100                 105

Glu Val Gln Tyr Gln Gln Asp Thr Pro Val Ala Pro Arg Phe Asp
                110                 115                 120

Val Asn Ala Pro Asp Leu Tyr Ile Pro Ala Met Ala Phe Ile Thr
                125                 130                 135

Tyr Val Leu Val Ala Gly Leu Ala Leu Gly Thr Gln Asp Arg Phe
                140                 145                 150

Ser Pro Asp Leu Leu Gly Leu Gln Ala Ser Ser Ala Leu Ala Trp
                155                 160                 165

Leu Thr Leu Glu Val Leu Ala Ile Leu Leu Ser Leu Tyr Leu Val
                170                 175                 180

Thr Val Asn Thr Asp Leu Thr Thr Ile Asp Leu Val Ala Phe Leu
                185                 190                 195

Gly Tyr Lys Tyr Val Gly Met Ile Gly Gly Val Leu Met Gly Leu
                200                 205                 210

Leu Phe Gly Lys Ile Gly Tyr Tyr Leu Val Leu Gly Trp Cys Cys
                215                 220                 225

Val Ala Ile Phe Val Phe Met Ile Arg Thr Leu Arg Leu Lys Ile
                230                 235                 240

Leu Ala Asp Ala Ala Ala Glu Gly Val Pro Val Arg Gly Ala Arg
                245                 250                 255

Asn Gln Leu Arg Met Tyr Leu Thr Met Ala Val Ala Ala Ala Gln
                260                 265                 270

Pro Met Leu Met Tyr Trp Leu Thr Phe His Leu Val Arg
                275                 280

<210> 45

<211> 1205
<212> DNA
<213> Homo Sapien

<400> 45
gctgagcacc aacaggaact attccagtga agagcaagtg ctgcccgacc 50

caggaccctg tgccaggctg gcagccctcc agctccctcc agagaggaaa 100

cctctgtctg gctgagggtg ggactagctg ggatgtctca ctccagttgc 150

tcaggttcac ccaggaagct cctccgtgga gtggccagcc tgattctagc 200

cctgtcctct ctggcagcac atgccacacc tgcctgggcc ttctgctccc 250

tgatgcttga tgagcccctg cctcctcaat gtttctcaaa gacagacccc 300

cctgaggcca gcttgaatgt gaagactgct gaagtcagct ggcttcactt 350

gagctgcaga aaaggtggct gggatggccc aggtgcaccc agaggcccca 400

gccctttggc tgcctttggg ttgtgacttg ggttgtctct gaggccctgc 450

cagagctggg cctgcgggtg gtgggcggtc cgacctcggg cagtcagtgc 500

tccgcagcct cagcactgca tcccagaccc agtgtcctca gagggaagag 550

ccagcctccc tgcctcatgg aaccaggagt cccaaaaagt caggagcctg 600

gaggctctga aaggagcagg gattccatag tgcgtgaagc tgaaataggc 650

gccctcctgg ggagccccca gcaaaactgt tttcataccc cactcccaga 700

actgccccgc tccagctcca gcgccagcgc cagctggttg ccaggcgtca 750

ttggagaggc ctggctgccc caggggcagc agggagtggt ggacctgtat 800

gggctggcag gaggccattg gccatgctga caagtgtcac ctgccttcct 850

agcctggagc cacccctcag gtggcctgct tgcacctcct atccggaggt 900

agcctgcccc acctgtaggc agagggggct cttgcttgag gcctgcacag 950

gaagcaagta tagccccggt gccccagagt gggttccact tagccctggc 1000

gagatggcct gtcctgagat ctctgctccc agaccccacc atctggggag 1050

cacagtcctt aggctgcctg gtccaggaag ggggtgcggc tctgtcagga 1100

aacctggact ctcaaggccc accagcctct ccgtgagtgt tagaaatcac 1150

agatacagta tatacttaat tacactactc actactcaaa aaaaaaaaaa 1200

aaaaa 1205

<210> 46
<211> 97
<212> PRT
<213> Homo Sapien

<400> 46
Met Ser His Ser Ser Cys Ser Gly Ser Pro Arg Lys Leu Leu Arg
1               5                   10                  15

Gly Val Ala Ser Leu Ile Leu Ala Leu Ser Ser Leu Ala Ala His
                20                  25                  30

Ala Thr Pro Ala Trp Ala Phe Cys Ser Leu Met Leu Asp Glu Pro

                35                  40                  45

Leu Pro Pro Gln Cys Phe Ser Lys Thr Asp Pro Pro Glu Ala Ser
                50                  55                  60

Leu Asn Val Lys Thr Ala Glu Val Ser Trp Leu His Leu Ser Cys
                65                  70                  75

Arg Lys Gly Gly Trp Asp Gly Pro Gly Ala Pro Arg Gly Pro Ser
                80                  85                  90

Pro Leu Ala Ala Phe Gly Leu
                95

<210> 47
<211> 983
<212> DNA
<213> Homo Sapien

<400> 47
ttccgggccc tggcgtctcg tctccttacc ctggggctac ccttgcccgg 50

tcctactgcc cgcggttaac ccgccgcgag ccgcctctcc cctccccgcc 100

cgactcaacc ctgccctccc ccgtgctttg cagacgccgc ccgggggccc 150

aggcggctga tgcgtgtggg cctcgcgctg atcttggtgg gccacgtgaa 200

cctgctgctg ggggccgtgc tgcatggcac cgtcctgcgg cacgtggcca 250

atccccgcgg cgctgtcacg ccggagtaca ccgtagccaa tgtcatctct 300

gtcggctcgg ggctgctgag cgtttccgtg ggacttgtgg ccctcctggc 350

gtccaggaac cttcttcgcc ctccactgca ctgggtcctg ctggcactag 400

ctctggtgaa cctgctcttg tccgttgcct gctccctggg cctccttctt 450

gctgtgtcac tcactgtggc caacggtggc cgccgcctta ttgctgactg 500

ccacccagga ctgctggatc ctctggtacc actggatgag ggcccgggac 550

atactgactg cccctttgac cccacaagaa tctatgatac agccttggct 600

ctctggatcc cttctttgct catgtctgca ggggaggctg ctctatctgg 650

ttactgctgt gtggctgcac tcactctacg tggagttggg ccctgcagga 700

aggacggact tcaggggcag ctagaggaaa tgacagagct tgaatctcct 750

aaatgtaaaa ggcaggaaaa tgagcagcta ctggatcaaa atcaagaaat 800

ccgggcatca cagagaagtt gggtttagga caggtgctgt tccgagactc 850

agtcctaaag ggtttttttt cccactaagc aaggggccct gacctcggga 900

tgagataaca aattgtaata aagtaacttc tcttttcttc taaaaaaaaa 950

aaaaaaaaaa aaaaaaaaaa aaaaaaaaaa aaa 983

<210> 48
<211> 222
<212> PRT
<213> Homo Sapien

<400> 48
Met Arg Val Gly Leu Ala Leu Ile Leu Val Gly His Val Asn Leu
1               5                   10                  15

Leu Leu Gly Ala Val Leu His Gly Thr Val Leu Arg His Val Ala
                20                  25                  30

Asn Pro Arg Gly Ala Val Thr Pro Glu Tyr Thr Val Ala Asn Val
                35                  40                  45

Ile Ser Val Gly Ser Gly Leu Leu Ser Val Ser Val Gly Leu Val
                50                  55                  60

Ala Leu Leu Ala Ser Arg Asn Leu Leu Arg Pro Pro Leu His Trp
                65                  70                  75

Val Leu Leu Ala Leu Ala Leu Val Asn Leu Leu Leu Ser Val Ala
                80                  85                  90

Cys Ser Leu Gly Leu Leu Leu Ala Val Ser Leu Thr Val Ala Asn
                95                  100                 105

Gly Gly Arg Arg Leu Ile Ala Asp Cys His Pro Gly Leu Leu Asp
                110                 115                 120

Pro Leu Val Pro Leu Asp Glu Gly Pro Gly His Thr Asp Cys Pro
                125                 130                 135

Phe Asp Pro Thr Arg Ile Tyr Asp Thr Ala Leu Ala Leu Trp Ile
                140                 145                 150

Pro Ser Leu Leu Met Ser Ala Gly Glu Ala Ala Leu Ser Gly Tyr
                155                 160                 165

Cys Cys Val Ala Ala Leu Thr Leu Arg Gly Val Gly Pro Cys Arg
                170                 175                 180

Lys Asp Gly Leu Gln Gly Gln Leu Glu Glu Met Thr Glu Leu Glu
                185                 190                 195

Ser Pro Lys Cys Lys Arg Gln Glu Asn Glu Gln Leu Leu Asp Gln
                200                 205                 210

Asn Gln Glu Ile Arg Ala Ser Gln Arg Ser Trp Val
                215                 220

<210> 49
<211> 847
<212> DNA
<213> Homo Sapien

<400> 49
cgtcagtcta gaaggataag agaaagaaag ttaagcaact acaggaaatg 50

gctttgggag ttccaatatc agtctatctt ttattcaacg caatgacagc 100

actgaccgaa gaggcagccg tgactgtaac acctccaatc acagcccagc 150

aagctgacaa catagaagga cccatagcct tgaagttctc acacctttgc 200

ctggaagatc ataacagtta ctgcatcaac ggtgcttgtg cattccacca 250

tgagctagag aaagccatct gcaggtgttt tactggttat actggagaaa 300

ggtgtgagca cttgacttta acttcatatg ctgtggattc ttatgaaaaa 350

tacattgcaa ttgggattgg tgttggatta ctattaagtg gttttcttgt 400

tattttttac tgctatataa gaaagaggta tgaaaaagac aaaatatgaa 450

gtcacttcat atgcaatcgt ttgacaaata gttattcagg ccctataatg 500

tgtcaggcac tgacatgtaa aattttttta attaaaaaag agctgtaatc 550

tggcaaaaag tttctatgta atatttttca tgccttttct cataaaccca 600

gacgagtggt aaaaatttgc cttcagttgt aataggagag ttcaaacgta 650

cagtctccct tcaacctatc tctgtctgcc catatcaaaa ttataaatga 700

ggaggacagc aggccccaag aaagtaggga ctaagtatgt cttgttcaaa 750

attgtatatt cagtgactta cactatgcct agcacacaac acacactgag 800

taaatatttg ttgagtgaaa taaaatcaag aaacaagtaa aaactga 847

<210> 50
<211> 133
<212> PRT
<213> Homo Sapien

<400> 50
Met Ala Leu Gly Val Pro Ile Ser Val Tyr Leu Leu Phe Asn Ala
1               5                   10                  15

Met Thr Ala Leu Thr Glu Glu Ala Ala Val Thr Val Thr Pro Pro
                20                  25                  30

Ile Thr Ala Gln Gln Ala Asp Asn Ile Glu Gly Pro Ile Ala Leu
                35                  40                  45

Lys Phe Ser His Leu Cys Leu Glu Asp His Asn Ser Tyr Cys Ile
                50                  55                  60

Asn Gly Ala Cys Ala Phe His His Glu Leu Glu Lys Ala Ile Cys
                65                  70                  75

Arg Cys Phe Thr Gly Tyr Thr Gly Glu Arg Cys Glu His Leu Thr
                80                  85                  90

Leu Thr Ser Tyr Ala Val Asp Ser Tyr Glu Lys Tyr Ile Ala Ile
                95                  100                 105

Gly Ile Gly Val Gly Leu Leu Leu Ser Gly Phe Leu Val Ile Phe
                110                 115                 120

Tyr Cys Tyr Ile Arg Lys Arg Tyr Glu Lys Asp Lys Ile
                125                 130

<210> 51
<211> 978
<212> DNA
<213> Homo Sapien

<400> 51
ggctcgagct tggctctcag accatcctgg tggaagaaac actagcagtc 50

tgcccaatct gaatgcaaat ccagaataat cttttctttt gttgttacac 100

agttatgagt gcaattttta aatggctgct actctacagc ctgcctgcct 150

tatgctttct cctgggcacg caggaaagtg agagcttcca ctccaaagca 200

gagatcctag tgacactaag tcaggtaata atctctccag ctggacctca 250

tgcactcaca tggacaacac acttctctcc ttcagtgatc atcatccttg 300

taccatgttg gtggcatgct gtaatcgtga ctcaacatcc ggttgccaat 350

tgctatgtaa caaaccacct caacattcag tggcttgaat tgaaagcagg 400

gtcttgaaga gatatttgca catttcatcc tcccagcagc attattcaca 450

acagccaata ggcagaagca acccaatgtc caaccataga tgagtggata 500

accaaaatgt agtccatcca tacaatgaaa tatgattcag ccttaacaag 550

gaaggaagtc ccgccacgtg ctacaacatg gatggacctt gaggacacta 600

tgctaagtga agtaagccag gcacaaaagg acaaatactc tatgattcca 650

ttttataggg taccaaagag aatcaaactc acagagatag aaagtagact 700

ggggtggcca gggactcggg gagagaggaa agggcagtta ttgtttaaaa 750

ggtacagagt ttcagtttgg gaagatgaaa atgttctgga aacggttaat 800

ggtgatttta cattgtttat gttaccacga tttgtaaaag agcagctgcg 850

ctgagaatga gcatgcttgt cattggcagc tctctgagat tttcagtgcc 900

tcttactggc ttgttaagaa gacggcaaaa aaaaaaaaaa aaaaaaaaaa 950

aaaaaaaaaa aaaaaaaaaa aaaaaaaa 978

<210> 52
<211> 114
<212> PRT
<213> Homo Sapien

<400> 52
Met Gln Ile Gln Asn Asn Leu Phe Phe Cys Cys Tyr Thr Val Met
1               5                   10                  15

Ser Ala Ile Phe Lys Trp Leu Leu Leu Tyr Ser Leu Pro Ala Leu
                20                  25                  30

Cys Phe Leu Leu Gly Thr Gln Glu Ser Glu Ser Phe His Ser Lys
                35                  40                  45

Ala Glu Ile Leu Val Thr Leu Ser Gln Val Ile Ile Ser Pro Ala
                50                  55                  60

Gly Pro His Ala Leu Thr Trp Thr Thr His Phe Ser Pro Ser Val
                65                  70                  75

Ile Ile Ile Leu Val Pro Cys Trp Trp His Ala Val Ile Val Thr
                80                  85                  90

Gln His Pro Val Ala Asn Cys Tyr Val Thr Asn His Leu Asn Ile
                95                  100                 105

Gln Trp Leu Glu Leu Lys Ala Gly Ser
                110

<210> 53
<211> 1664
<212> DNA
<213> Homo Sapien

<400> 53
ttttgaaatg gtttatgacc tcttccccac ttccccgctt gctttgctca 50

ttagtgttcc taggtggctg ctgggtgacg ggcttttcat catctctgat 100

gtgggccagt gcgaaagagc agctgcaaca tctgtttcta attgggtcgt 150

gcctttataa atacttcttg cctatttgtc acattgcttc cctcccaccc 200

tgtcttcctt ggagtactgc agaatctgta agcgtccctg gaatgcacac 250

gtggaccttg tcattcccaa acagactttc tgctggtcag cactttgtaa 300

tgttcggctg ttacaggcat tagtcacttg tgctcagaga gagactgtgg 350

tctttggaaa ctgaagaaaa tgtctttttt gttgttgtta attcttggca 400

tccagttaga tttaacttct caagagttta cacagacttt tagaaaaaca 450

ttctgtctct aagaaaaaag tgctctagct ttgtacagtt tttggatttt 500

cacacttggt ggttgtttgc tgaaatgctg ttttgctagt gattcccctc 550

ctccccctat ctggggttgt aagcagctct ggggctctgt tcacttcgga 600

tacctgtttc tggggactgc ttttcaacag cgtttttcct aagggcatat 650

gagaaattta atttctgatg gaatgaaggt gaaactctag tcccaggtaa 700

acctgggtag gctgtagaga cagaaagggg gctgcaggtc taggtggaag 750

aacgagaacg aatgcagcat ggtatttcca ggccttttag attcggcttc 800

atccacaacc aatgtgagtt cttatctgca aagcgggcct aagtgtaatg 850

gagggaaggt gggccaggca ccagggtcct gggttctccc gcgcctcact 900

ctgtctccac ctggcccatg cataaagaac actagtcaag tagccattgt 950

acctgtttcc ttatctgaaa atgagaaggt tggagagtat gacttctgtt 1000

gaaacaacaa ggcgcttaca aattttggtg aagtcgaatg agggcagcgt 1050

taagagaaat atcaaagtta gtcattggat ttcagggctt agggatggaa 1100

accagctggt agtagactgg ttgtagttat gtccaaaggg cagagtggga 1150

aaaatttggc cgaaaagagt gtggtgggtg accagcaaat gttagaggta 1200

tacatcaggg cacagaggag aaaagctaac atgatactga tgacttcaag 1250

tcttcactgt ccaattcaga ggatagggga gggtttaagc tgattaaaca 1300

gtgggctttt tttctcctgc aagagggtgg aggtctataa ctgtgcagat 1350

tttatcagat gcatgctaat acatgttatt ctgggggact ctcttatacc 1400

ttgaagtaga cattgctgct atttgcgtga aaaaaatagg aggacttatt 1450

tgaattgaga atggggatag gctgagttcc accgagatgt tggcttagag 1500

atgcctgggc catgctgtac agtaggaagc ccagcagagg agattgggct 1550

gtgtgggtca tgacaaaggg agttgttagc ttatggttgg ctattaaagt 1600

catgggcaag gatgggcaag aaaagtgtgt aaaatgagct gacaaaagat 1650

aaatatgtta atta 1664

<210> 54
<211> 102
<212> PRT
<213> Homo Sapien

<400> 54

Met Thr Ser Ser Pro Leu Pro Arg Leu Leu Cys Ser Leu Val Phe
1               5                   10                  15

Leu Gly Gly Cys Trp Val Thr Gly Phe Ser Ser Ser Leu Met Trp
                20                  25                  30

Ala Ser Ala Lys Glu Gln Leu Gln His Leu Phe Leu Ile Gly Ser
                35                  40                  45

Cys Leu Tyr Lys Tyr Phe Leu Pro Ile Cys His Ile Ala Ser Leu
                50                  55                  60

Pro Pro Cys Leu Pro Trp Ser Thr Ala Glu Ser Val Ser Val Pro
                65                  70                  75

Gly Met His Thr Trp Thr Leu Ser Phe Pro Asn Arg Leu Ser Ala
                80                  85                  90

Gly Gln His Phe Val Met Phe Gly Cys Tyr Arg His
                95                  100

<210> 55
<211> 1824
<212> DNA
<213> Homo Sapien

<400> 55
acactggcca aacactcgca tcccagggcg tctccggctg ctcccattga 50

gctgtctgct cgctgtgccc gctgtgcctg ctgtgcccgc gctgtcgccg 100

ctgctaccgc gtctgctgga cgcgggagac gccagcgagc tggtgattgg 150

agccctgcgg agagctcaag cgcccagctc tgcccgagga gcccaggctg 200

ccccgtgagt cccatagttg ctgcaggagt ggagccatga gctgcgtcct 250

gggtggtgtc atccccttgg ggctgctgtt cctggtctgc ggatcccaag 300

gctacctcct gcccaacgtc actctcttag aggagctgct cagcaaatac 350

cagcacaacg agtctcactc ccgggtccgc agagccatcc ccagggagga 400

caaggaggag atcctcatgc tgcacaacaa gcttcggggc caggtgcagc 450

ctcaggcctc caacatggag tacatgacct gggatgacga actggagaag 500

tctgctgcag cgtgggccag tcagtgcatc tgggagcacg ggcccaccag 550

tctgctggtg tccatcgggc agaacctggg cgctcactgg ggcaggtatc 600

gctctccggg gttccatgtg cagtcctggt atgacgaggt gaaggactac 650

acctacccct acccgagcga gtgcaacccc tggtgtccag agaggtgctc 700

ggggcctatg tgcacgcact acacacagat agtttgggcc accaccaaca 750

agatcggttg tgctgtgaac acctgccgga agatgactgt ctggggagaa 800

gtttgggaga acgcggtcta ctttgtctgc aattattctc caaaggggaa 850

ctggattgga gaagccccct acaagaatgg ccggccctgc tctgagtgcc 900

cacccagcta tggaggcagc tgcaggaaca acttgtgtta ccgagaagaa 950

acctacactc caaaacctga aacggacgag atgaatgagg tggaaacggc 1000

tcccattcct gaagaaaacc atgtttggct ccaaccgagg gtgatgagac 1050

ccaccaagcc caagaaaacc tctgcggtca actacatgac ccaagtcgtc 1100

agatgtgaca ccaagatgaa ggacaggtgc aaagggtcca cgtgtaacag 1150

gtaccagtgc ccagcaggct gcctgaacca caaggcgaag atctttggaa 1200

gtctgttcta tgaaagctcg tctagcatat gccgcgccgc catccactac 1250

gggatcctgg atgacaaggg aggcctggtg gatatcacca ggaacgggaa 1300

ggtccccttc ttcgtgaagt ctgagagaca cggcgtgcag tccctcagca 1350

aatacaaacc ttccagctca ttcatggtgt caaaagtgaa agtgcaggat 1400

ttggactgct acacgaccgt tgctcagctg tgcccgtttg aaaagccagc 1450

aactcactgc ccaagaatcc attgtccggc acactgcaaa gacgaacctt 1500

cctactgggc tccggtgttt ggaaccaaca tctatgcaga tacctcaagc 1550

atctgcaaga cagctgtgca cgcgggagtc atcagcaacg agagtggggg 1600

tgacgtggac gtgatgcccg tggataaaaa gaagacctac gtgggctcgc 1650

tcaggaatgg agttcagtct gaaagcctgg ggactcctcg ggatggaaag 1700

gccttccgga tctttgctgt caggcagtga atttccagca ccaggggaga 1750

aggggcgtct tcaggagggc ttcggggttt tgcttttatt tttattttgt 1800

cattgcgggg tatatggaga gtca 1824

<210> 56
<211> 497
<212> PRT
<213> Homo Sapien

<400> 56

<210> 57
<211> 486
<212> DNA
<213> Homo Sapien

<400> 57
gcacgaggcc aaacacagca gcctcaacat gaaggtggtt atggtcctcc 50

tgcttgctgc cctccccctt tactgctatg caggttctgg ttgcgttctt 100

ctggagagcg tcgtggaaaa gaccatcgat ccatcggttt ctgtggagga 150

atacaaagca gatcttcaga ggttcatcga cactgagcaa accgaagcag 200

ctgtagagga gttcaaggag tgcttcctca gccagagcaa tgagactctg 250

gccaacttcc gagtcatggt gcatacgata tatgacagcc tttactgtgc 300

tgcgtattaa ctgtcacaag aactttggct cagaggaatc cagacgatgc 350

tcacaacccg actgtggact ggcagaaatc tcaacttttc cttttgactt 400

tcccctttga tcagtaatat ggaagacgtt gttgaaacct gaagtatagt 450

taatttaaat aaacccactg caagaaaaaa aaaaaa 486

<210> 58
<211> 93
<212> PRT
<213> Homo Sapien

<400> 58

<210> 59
<211> 3976
<212> DNA
<213> Homo Sapien

<400> 59
caagtccgtt gaggctgcca ggcgagtcag gtctctctgg acctcgcctg 50

actcggctgg gctgtgcctg aaattgaccc agctccacca tactccttga 100

ttatgagaaa acaaggagta agctcaaagc ggctgcaatc ttccggccgc 150
agccagtcta aggggcggcg cggggcctcc ctcgcccggg agccggaggt 200
agaggaggag gtggaaaagt cggtcctagg cggcgggaaa ctgccaaggg 250
gcgcctggag gtcctccccg gggaggatcc aaagtctgaa agagcgaaaa 300
ggcttggagc tagaggtggt ggccaagacc tttcttctcg gccccttcca 350
gttcgtccgt aattccctgg cgcagctccg ggaaaaggtg caggaactgc 400
aggcgcggcg gttctccagc agaaccactc tcggcatcgc tgtctttgtg 450
gcaattttac attggttaca tttagtaaca ctttttgaaa atgatcgtca 500
tttctctcac ctctcatctt tggaacggga gatgactttt cgcactgaaa 550
tgggacttta ttattcatac ttcaagacca ttattgaagc accttcgttt 600
ttggaaggac tgtggatgat tatgaatgac aggcttactg aatatcctct 650
tataattaat gcaataaaac gcttccatct ttatccagag gtaatcatag 700
cctcctggta ttgcacattc atgggaataa tgaatttatt tggactagaa 750
actaagacct gctggaatgt caccagaata gaacctctta atgaagttca 800
aagctgtgaa ggattgggag atcctgcttg cttttatgtt ggtgtaatct 850
ttattttaaa tggactaatg atgggattgt tcttcatgta tggagcatac 900
ctgagtggga ctcaactggg aggtcttatt acagtactgt gcttcttttt 950
caaccatgga gaggccaccc gtgtgatgtg gacaccacct ctccgtgaaa 1000
gtttttccta tcctttcctt gtacttcaga tgtgtatttt aactttgatt 1050
ctcaggacct caagcaatga tagaaggccc ttcattgcac tctgtctttc 1100
caatgttgct tttatgcttc cctggcaatt tgctcagttt atacttttta 1150
cacagatagc atcattattt cccatgtatg ttgtgggata cattgaacca 1200
agcaaatttc agaagatcat ttatatgaac atgatttcag ttacccttag 1250
tttcattttg atgtttggaa attcaatgta cttatcttct tattattctt 1300
catctttgtt aatgacgtgg gcaataattc taaagagaaa tgaaattcaa 1350
aaactgggag tatctaaact caacttttgg ctaattcaag gtagtgcctg 1400
gtggtgtgga acaatcattt tgaaatttct gacatctaaa atcttaggcg 1450
tttcagacca cattcgcctg agtgatctta tagcagccag aatcttaagg 1500
tatacagatt ttgatacttt aatatatacc tgtgctcccg aatttgactt 1550
catggaaaaa gcgactccgc tgagatacac aaagacatta ttgcttccag 1600
ttgttatggt gattacatgt tttatcttta aaaagactgt tcgtgatatt 1650
tcatatgttt tagctacaaa catttatcta agaaaacagc tccttgaaca 1700
cagtgagctg gcttttcaca cattgcagtt gttagtgttt actgcccttg 1750
ccattttaat tatgaggcta aagatgtttt tgacaccgca catgtgtgtt 1800

atggcttcct tgatatgctc tcgacagctc tttggctggc tttttcgcag 1850

agttcgtttt gagaaggtta tctttggcat tttaacagtg atgtcaatac 1900

aaggttatgc aaacctccgt aatcaatgga gcataatagg agaatttaat 1950

aatttgcctc aggaagaact tttacagtgg atcaaataca gtaccacatc 2000

agatgctgtc tttgcaggtg ccatgcctac aatggcaagc atcaagctgt 2050

ctacacttca tcccattgtg aatcatccac attacgaaga tgcagacttg 2100

agggctcgga caaaaatagt ttattctaca tatagtcgaa aatctgccaa 2150

agaagtaaga gataaattgt tggagttaca tgtgaattat tatgttttag 2200

aagaggcatg gtgtgttgtg agaactaagc ctggttgcag tatgcttgaa 2250

atctgggatg tggaagaccc ttccaatgca gctaaccctc ccttatgtag 2300

cgtcctgctc gaagacgcca ggccttactt caccacagta tttcagaata 2350

gtgtgtacag agtattaaag gttaactgag aaggatacta cccattttac 2400

tatggcacaa tgccgtgtgt caaaaacaat caccctttgg cttattcaca 2450

ttaataaaaa tcacaagctt taataacaga cacttaaaaa taagataaaa 2500

atggattgga aatttttctg attactaaaa ggtaaattac ttttctgttc 2550

attgaatgtc agccttatta agcttgtcat ataagttatt aaatcattca 2600

tgtcatactg cataaacaaa tgttcatttc agaattttaa agagaaatgt 2650

atataaagaa cmatgatttt aataatcagg ggtatgtaag tcctttttca 2700

tccaactagg tgaattgctt cagattttct ctagtaccag agggtacctc 2750

ctcaaactct ttgaaccact taaggcagaa gaatgcaagc tctgaaatga 2800

catccttaaa atgctgatac tggtcacagc ctctttacct ctgtgaggaa 2850

attgtaacag tgtgtctttt aaggtgtttt tattttacca gcccttaaga 2900

aagatctcta atacctttta atactttttt ttaataattt caagttgaag 2950

tgtttttaaa aacactttgt tttgtaatgt tttgaatctc ttgagatgtg 3000

tttaccccac tagatacata tttgccactg gttagttctc catctaagct 3050

caagaggtta ttcatctctc tttagattcc agtggctttt cttttaacat 3100

ccaggtaaaa cagaaactgc tatggtatac aaccaagttt tggggttaaa 3150

cataatcaga aaagaaaatc cagttaaatt tatgaagtga gattttcaga 3200

tcctagatct tgaataaagg aaaggtcttt tcatcttgat ggccccaaag 3250

cttgttggtc atggtcttta tttctggcca ctatcttctt aaataatata 3300

tttttaagcc ctcatttatt tttggttttg ggtgaggaaa gtcatgtttt 3350

ctaagtcctc tcccctaata aaacctaccc aacaatagtg ctttgaaaag 3400

tggtagttat cttgaagata ctcttgccaa atgcaaagat aaacattctt 3450

tttgtctgct ttataaatat gaaatatgcc agatctatag tattttaatg 3500

tgcatctact ttaaatgagt catcttgggg tttttataat tcccttatgt 3550

tcttgcccct ctacacttga aataacaaaa tgccttaatt ttatggatta 3600

gttctcttat agtagacagg cagctatatg cagcaaaacc aataaagtta 3650

tttttcaact ttcatagttg taaaatatct tataccagaa tacaaaacag 3700

ctaagaaaac atgccacatt ttattttagc attttcaaat aatttgtttt 3750

tggtgtaagc acaggataaa aaaggagagc gtcaaagaaa agagacataa 3800

cacctaacat tcataaaaat taacaaagta tattttggat gatgttttta 3850

caggaaatat tttaaataag ttggtagaac ttttaaaatg gtactgtatt 3900

agctaataaa atattcagta caaatatatg tttggattta tgcattaaaa 3950

aactaataaa attatttcca acttta 3976

<210> 60
<211> 758
<212> PRT
<213> Homo Sapien

<400> 60
Met Arg Lys Gln Gly Val Ser Ser Lys Arg Leu Gln Ser Ser Gly
1               5                   10                  15

Arg Ser Gln Ser Lys Gly Arg Arg Gly Ala Ser Leu Ala Arg Glu
                20                  25                  30

Pro Glu Val Glu Glu Glu Val Glu Lys Ser Val Leu Gly Gly Gly
                35                  40                  45

Lys Leu Pro Arg Gly Ala Trp Arg Ser Ser Pro Gly Arg Ile Gln
                50                  55                  60

Ser Leu Lys Glu Arg Lys Gly Leu Glu Leu Glu Val Val Ala Lys
                65                  70                  75

Thr Phe Leu Leu Gly Pro Phe Gln Phe Val Arg Asn Ser Leu Ala
                80                  85                  90

Gln Leu Arg Glu Lys Val Gln Glu Leu Gln Ala Arg Arg Phe Ser
                95                  100                 105

Ser Arg Thr Thr Leu Gly Ile Ala Val Phe Val Ala Ile Leu His
                110                 115                 120

Trp Leu His Leu Val Thr Leu Phe Glu Asn Asp Arg His Phe Ser
                125                 130                 135

His Leu Ser Ser Leu Glu Arg Glu Met Thr Phe Arg Thr Glu Met
                140                 145                 150

Gly Leu Tyr Tyr Ser Tyr Phe Lys Thr Ile Ile Glu Ala Pro Ser
                155                 160                 165

Phe Leu Glu Gly Leu Trp Met Ile Met Asn Asp Arg Leu Thr Glu
                170                 175                 180

Tyr Pro Leu Ile Ile Asn Ala Ile Lys Arg Phe His Leu Tyr Pro
                185                 190                 195

Glu Val Ile Ile Ala Ser Trp Tyr Cys Thr Phe Met Gly Ile Met
                200                 205                 210

Asn Leu Phe Gly Leu Glu Thr Lys Thr Cys Trp Asn Val Thr Arg
                215                 220                 225

Ile Glu Pro Leu Asn Glu Val Gln Ser Cys Glu Gly Leu Gly Asp
                230                 235                 240

Pro Ala Cys Phe Tyr Val Gly Val Ile Phe Ile Leu Asn Gly Leu
                245                 250                 255

Met Met Gly Leu Phe Phe Met Tyr Gly Ala Tyr Leu Ser Gly Thr
                260                 265                 270

Gln Leu Gly Gly Leu Ile Thr Val Leu Cys Phe Phe Phe Asn His
                275                 280                 285

Gly Glu Ala Thr Arg Val Met Trp Thr Pro Pro Leu Arg Glu Ser
                290                 295                 300

Phe Ser Tyr Pro Phe Leu Val Leu Gln Met Cys Ile Leu Thr Leu
                305                 310                 315

Ile Leu Arg Thr Ser Ser Asn Asp Arg Arg Pro Phe Ile Ala Leu
                320                 325                 330

Cys Leu Ser Asn Val Ala Phe Met Leu Pro Trp Gln Phe Ala Gln
                335                 340                 345

Phe Ile Leu Phe Thr Gln Ile Ala Ser Leu Phe Pro Met Tyr Val
                350                 355                 360

Val Gly Tyr Ile Glu Pro Ser Lys Phe Gln Lys Ile Ile Tyr Met
                365                 370                 375

Asn Met Ile Ser Val Thr Leu Ser Phe Ile Leu Met Phe Gly Asn
                380                 385                 390

Ser Met Tyr Leu Ser Ser Tyr Tyr Ser Ser Ser Leu Leu Met Thr
                395                 400                 405

Trp Ala Ile Ile Leu Lys Arg Asn Glu Ile Gln Lys Leu Gly Val
                410                 415                 420

Ser Lys Leu Asn Phe Trp Leu Ile Gln Gly Ser Ala Trp Trp Cys
                425                 430                 435

Gly Thr Ile Ile Leu Lys Phe Leu Thr Ser Lys Ile Leu Gly Val
                440                 445                 450

Ser Asp His Ile Arg Leu Ser Asp Leu Ile Ala Ala Arg Ile Leu
                455                 460                 465

Arg Tyr Thr Asp Phe Asp Thr Leu Ile Tyr Thr Cys Ala Pro Glu
                470                 475                 480

Phe Asp Phe Met Glu Lys Ala Thr Pro Leu Arg Tyr Thr Lys Thr
                485                 490                 495

Leu Leu Leu Pro Val Val Met Val Ile Thr Cys Phe Ile Phe Lys
                500                 505                 510

Lys Thr Val Arg Asp Ile Ser Tyr Val Leu Ala Thr Asn Ile Tyr
                515                 520                 525

Leu Arg Lys Gln Leu Leu Glu His Ser Glu Leu Ala Phe His Thr
                530                 535                 540

Leu Gln Leu Leu Val Phe Thr Ala Leu Ala Ile Leu Ile Met Arg
                545                 550                 555

Leu Lys Met Phe Leu Thr Pro His Met Cys Val Met Ala Ser Leu
                560                 565                 570

Ile Cys Ser Arg Gln Leu Phe Gly Trp Leu Phe Arg Arg Val Arg
                575                 580                 585

Phe Glu Lys Val Ile Phe Gly Ile Leu Thr Val Met Ser Ile Gln
                590                 595                 600

Gly Tyr Ala Asn Leu Arg Asn Gln Trp Ser Ile Ile Gly Glu Phe
                605                 610                 615

Asn Asn Leu Pro Gln Glu Glu Leu Leu Gln Trp Ile Lys Tyr Ser
                620                 625                 630

Thr Thr Ser Asp Ala Val Phe Ala Gly Ala Met Pro Thr Met Ala
                635                 640                 645

Ser Ile Lys Leu Ser Thr Leu His Pro Ile Val Asn His Pro His
                650                 655                 660

Tyr Glu Asp Ala Asp Leu Arg Ala Arg Thr Lys Ile Val Tyr Ser
                665                 670                 675

Thr Tyr Ser Arg Lys Ser Ala Lys Glu Val Arg Asp Lys Leu Leu
                680                 685                 690

Glu Leu His Val Asn Tyr Tyr Val Leu Glu Glu Ala Trp Cys Val
                695                 700                 705

Val Arg Thr Lys Pro Gly Cys Ser Met Leu Glu Ile Trp Asp Val
                710                 715                 720

Glu Asp Pro Ser Asn Ala Ala Asn Pro Pro Leu Cys Ser Val Leu
                725                 730                 735

Leu Glu Asp Ala Arg Pro Tyr Phe Thr Thr Val Phe Gln Asn Ser
                740                 745                 750

Val Tyr Arg Val Leu Lys Val Asn
                755

<210> 61
<211> 2449
<212> DNA
<213> Homo Sapien

<400> 61
ggcgcggcca catcctttaa atatggtctt tcttgggcgc gcgcgacaat 50

gtgaggagtg gggtggagcg tgtgtggtgt gtggctgcgg cctgggcaag 100

agccgccgcg gaccatgagc tgagtaagtt ctggagggat cctgcctctt 150

ggagccttcg cagccaggca gctgtgaact gtgagctaga gtgaagcaga 200

aatctaggaa gatgagctcc aagatggtca taagtgaacc aggactgaat 250

tgggatattt cccccaaaaa tggccttaag acatttttct ctcgagaaaa 300

ttataaagat cattccatgg ctccaagttt aaaagaacta cgtgttttat 350

ccaacagacg tataggagaa aatttgaatg cctcagcaag ttctgtagaa 400

aatgagccgg cagttagttc agcaactcaa gcaaaggaaa aagttaaaac 450

cacaattgga atggttcttc ttccaaaacc aagagttcct tatcctcgtt 500

tctctcgttt ctcacagaga gagcagagga gttatgtgga cttgttggtt 550
aaatacgcaa agattcctgc aaattccaaa gctgttggaa taaataaaaa 600
tgactacttg cagtacttgg atatgaaaaa acatgtgaac gaagaagtta 650
ctgagttcct aaagtttttg cagaattctg caaagaaatg tgcgcaggat 700
tataatatgc tttctgatga tgcccgtctc ttcacagaga aaattttaag 750
agcttgcatt gaacaagtga aaaagtattc agaattctat actctccacg 800
aggtcaccag cttaatggga ttcttcccat tcagagtaga gatgggatta 850
aagttagaaa aaactcttct cgcattgggc agtgtaaaat atgtgaaaac 900
agtatttccc tcaatgccta taaagttgca gctgtcaaag gacgatatag 950
ctaccattga aacgtcagaa caaacagctg aagctatgca ttatgatatt 1000
agtaaagatc caaatgcaga gaagcttgtt tccagatatc accctcagat 1050
agctctaact agtcagtcat tatttacctt attaaataat catggaccaa 1100
cgtacaagga acagtgggaa attccagtgt gtattcaagt aatacctgtt 1150
gcaggttcaa aaccagttaa agtaatatat attaattcac cacttcccca 1200
aaagaaaatg actatgagag agagaaatca aatctttcat gaagttccat 1250
taaaatttat gatgtccaaa aacacatctg ttccagtctc tgcagtcttt 1300
atggacaaac ctgaagagtt tatatctgaa atggacatgt cctgtgaagt 1350
caacgagtgc cgaaaaattg agagtcttga aaacttgtat ttggattttg 1400
atgatgatgt cacagaactt gaaacttttg gagtaaccac caccaaagta 1450
tcaaaatcac caagtccagc aagtacttcc acagtaccta acatgacaga 1500
tgctcctaca gcccccaaag caggaactac aactgtggca ccaagtgcac 1550
cagacatttc tgctaattct agaagtttat ctcagattct gatggaacaa 1600
ttgcaaaagg agaaacagct ggtcactggt atggatggtg gccctgagga 1650
atgcaaaaat aaagatgatc agggatttga atcatgtgaa aaggtatcaa 1700
attctgacaa gcctttgata caagatagtg acttgaaaac atctgatgcc 1750
ttacagttag aaaattctca ggaaattgaa acttctaata aaaatgatat 1800
gactatagat atactacatg ctgatggtga aagacctaat gttctagaaa 1850
acctagacaa ctcaaaggaa aagactgttg gatcagaagc agcaaaaact 1900
gaagatacag ttctctgcag cagtgataca gatgaggagt gtttaatcat 1950
tgatacagaa tgtaaaaaaa ccagttataa cagtgtttaa tttagataag 2000
tttgagggaa aataatcagt aggcaagagg aacatttttc ctgtagtagc 2050
tagagtgcct tgaaaaaatg tgttggctat gtgaaggaat atttcaacta 2100
aaatggaatg gtatgctttt cacccttaaa gtttgaggag gatcttgata 2150
tgttttaaca ttatcatggc agggaaatat ataaagaaga aaaatatttt 2200

tacattaaac cttttctaaa aattgtaaat agaaaaataa tttggttttt 2250

tatcaagaac aacacttatc gttatgtatt gtgttagtta tattgccagt 2300

ctgttgcgac tgactcaaaa agttaaatgt tgccactgct gaagatgatt 2350

atgagcatcg caaactttgt ttctgaccca ttttgacagt ttttatatac 2400

tcctttaaaa tgatgaatgt tacaggttaa taaagttaat acctttaaa 2449

<210> 62
<211> 592
<212> PRT
<213> Homo Sapien

<400> 62

<210> 63
<211> 1451
<212> DNA
<213> Homo Sapien

<400> 63
tttttaactt gaacttccaa ggccacgtgc gtctcctggc tcctgcacgg 50

actgtgtgac tgtccccgac agctttcctg tctcgtctca tgaggggtcc 100

agcacatggc attctgggtc ggcacctgaa gtccacctct atgagaccct 150

ctgggagcgt gacggggcct tggcatgggt cggccgaggc ccttctgtcc 200

caggtcactg gtgtggtcgg cccaggccct cctgtcccac atcacctgtg 250

tggtcggccc aggccctcct gtcccaggtc accggtgtgg tcggcccagg 300

ccctcctgtc caggtcctcc tgtccaggtc actggtgtgg tcggcccagg 350

cccttctgtc ccaggtcacc tgtgtggtcg gcccagggcc ctcctgtacc 400

atgtcactgt tgaggggctg gctctggaag agggcaggga cttggcattg 450

gtgggggcag ggttccaagg tgtggcctgt cagcaggaag gggcaggtgg 500

catgggtcca ggcgggactc agggctgggg tgccactgct ggagactgtc 550

cggaggcccc tccagggcac cttgccattg ccattgtcgc tcatggccat 600

ctggtcccgt ttcagggaac aagaggagga tcagatgctg cgggacatga 650

ttgagaagct gggtgactgg gccggggatg ctgagggctg ggctggctgg 700

ctgggtgggc cggggatgct gagtgctggg ctggctggct gggtggaccg 750

ggcctccagc tgggggtggg gggggggcgg gtatcgggtc cccccctcag 800

ccttggtgac aggacaggca ggttcaccct gagggtgaga gctccctccc 850

gcccctaaga gagccagggg cagctggtga ccgtgtggtc atggtgggga 900

ccagccctcc ggggcaccca gtcggggcag gttctcacgt gggagggcac 950

agggcttcct gcaggctcgg aggcccaggg cggattgtgg ccagtggaag 1000

ggaaggatgt ttctggcagg gggacttgtg tgggccacgg ctgtgcggct 1050

gcggcgttga gcacggcctc actgtccacc tgtcccctag gcctccagag 1100

gaagaagtcc aagttccgct tgtccaagat ctggtcacca aaaagcaaaa 1150

gcagcccctc ccagtagtag ccagtagggc cgtgggctcg gcccggacct 1200

ggcatccgga cttggactcg ggccatgggg cttggcccgg acccggaacc 1250

cggacttgta ctcggggccg tgggctcggc ccggacccgg cattcggact 1300

tggactcggg aagggcctcc tgtccctaca aggggcatgt ggacagcagg 1350

gacctgcgct accgtctgtg gtctcaataa agaaaccgac cacatggccc 1400

cggaaaaaaa aaaaaaaaaa aaaaaaaaaa aaaaaaaaaa aaaaaaaaac 1450

a 1451

<210> 64

<211> 159
<212> PRT
<213> Homo Sapien

<400> 64
Met Gly Arg Pro Arg Pro Phe Cys Pro Arg Ser Leu Val Trp Ser
1               5                   10                  15

Ala Gln Ala Leu Leu Ser His Ile Thr Cys Val Val Gly Pro Gly
                20                  25                  30

Pro Pro Val Pro Gly His Arg Cys Gly Arg Pro Arg Pro Ser Cys
                35                  40                  45

Pro Gly Pro Pro Val Gln Val Thr Gly Val Val Gly Pro Gly Pro
                50                  55                  60

Ser Val Pro Gly His Leu Cys Gly Arg Pro Arg Ala Leu Leu Tyr
                65                  70                  75

His Val Thr Val Glu Gly Leu Ala Leu Glu Glu Gly Arg Asp Leu
                80                  85                  90

Ala Leu Val Gly Ala Gly Phe Gln Gly Val Ala Cys Gln Gln Glu
                95                  100                 105

Gly Ala Gly Gly Met Gly Pro Gly Gly Thr Gln Gly Trp Gly Ala
                110                 115                 120

Thr Ala Gly Asp Cys Pro Glu Ala Pro Pro Gly His Leu Ala Ile
                125                 130                 135

Ala Ile Val Ala His Gly His Leu Val Pro Phe Gln Gly Thr Arg
                140                 145                 150

Gly Gly Ser Asp Ala Ala Gly His Asp
                155

<210> 65
<211> 1629
<212> DNA
<213> Homo Sapien

<400> 65
ggcgaccacc gccgcctcct cacctggcca ttggtgcagc ccgttcccgg 50

cggcgagaga aggcaggcgc gctccttgcg ccacgccaca ccgtcgggcc 100

cccgtcgggt ccccctcggg ccgcaatggt gggctccgcg cggctgggtc 150

cggcactctt gacccccttt gtaaccaccg cggcgggcac ccagggagtt 200

cgagcaacga agttggtgac ctgccccgct cccaggcagt ttgctgttgg 250

ggctttcacg gctgctggaa gggcatggct gtttgtccca tcactgggcg 300

ccagcttctc aaagctacgt tcacagcaac gcagtaggga ctttcgtggc 350

aggctttttt taagagctga aagaagggcg ggagggttta cgtcctaggg 400

tgatgatttc ctcaccagac agcgaagtat ctattgggaa actccaggtg 450

accgcacctc cttccgacag ttcgccccgg ggcaagttta ccagctgcgt 500

cagaaagcag gtttgcaaaa tccttggaga acggcctgag ctaaggactg 550

gggtcaggag ggttttaaac tcattctgat tttcttgcaa tcatatctct 600

tgaaagtttt tatattttcc ccaatatttt tctgagttgc tatatccaat 650

gaaaacaatg ctgatgtaga ggtccaccag ccaatgcttt attggaagtc 700

aacgaatgag accgagggtg gcccataatc aatctcggca cgcgggaatg 750

tgaacctctt ccaaggtctg ggcgagtccc tagagttacg cagatgaagg 800

acattggccc tcgagaatct cacaccagca aagaagagca caacgaagcg 850

caaactactt atgatcattg tggctttggg caagttgttg tagctcccag 900

caacaatttc ttcacctgga gtgcagcaat aaatgatact ggtgctgcag 950

ggcagctaat aagcttctga ataatatatg caaagtactt ggcaccatga 1000

gcagaactca gtataccgtc actgaagaaa tagcttattt aatgattaca 1050

cttttcatat gtgcaagtaa aagtttgact tttagggaga gcctcaccta 1100

cggaatgtct tttttaaatt tcttttttaa ttatacttta agttctggga 1150

tacatgtgca gaacgtgcag gtttgttaca caggtataca tgtgccatgg 1200

tggtttgcag cacccatcaa cccttcatct aggttttaag ctccgcatgc 1250

attagttatt tgtcctaatg ctctccctcc ccttgtcccc caccccccaa 1300

caggcctcag ggtgtgatgt tcccctccct gggtccatat gttctcattg 1350

ttcaactccc acttatgatg agaacatgca gtgtttggtt ttctgttcct 1400

gtgttagttt gctgagaatg atggtttcca gcatcatcca cgtccctgca 1450

aaggacatga attcattctt ttttatggct gcatggtatt ccatggtgta 1500

tatgtgccac attttcttca tccagtctat cattgatggg cacttgggtt 1550

ggttccaaga ctttgttatt gtgaacagtg ctgcaataaa catacgtttg 1600

tatgtgtcaa aaaaaaaaaa aaaaaaaaa 1629

<210> 66
<211> 90
<212> PRT
<213> Homo Sapien

<400> 66

<210> 67
<211> 2067

<212> DNA
<213> Homo Sapien

<400> 67
catgtctaga ctgggagccc tgggtggtgc ccgtgccggg ctgggactgt 50

tgctgggtac cgccgccggc cttggattcc tgtgcctcct ttacagccag 100

cgatggaaac ggacccagcg tcatggccgc agccagagcc tgcccaactc 150

cctggactat acgcagactt cagatcccgg acgccacgtg atgctcctgc 200

gggctgtccc aggtggggct ggagatgcct cagtgctgcc cagccttcca 250

cgggaaggac aggagaaggt gctggaccgc ctggactttg tgctgaccag 300

ccttgtggcg ctgcggcggg aggtggagga gctgagaagc agcctgcgag 350

ggcttgcggg ggagattgtt ggggaggtcc gatgccacat ggaagagaac 400

cagagagtgg ctcggcggcg aaggtttccg tttgtccggg agaggagtga 450

ctccactggc tccagctctg tctacttcac ggcctcctcg ggagccacgt 500

tcacagatgc tgagagtgaa gggggttaca caacagccaa tgcggagtct 550

gacaatgagc gggactctga caaagaaagt gaggacgggg aagatgaagt 600

gagctgtgag actgtgaaga tggggagaaa ggattctctt gacttggagg 650

aagaggcagc ttcaggtgcc tccagtgccc tggaggctgg aggttcctca 700

ggcttggagg atgtgctgcc cctcctgcag caggccgacg agctgcacag 750

gggtgatgag caaggcaagc gggagggctt ccagctgctg ctcaacaaca 800

agctggtgta tggaagccgg caggactttc tctggcgcct ggcccgagcc 850

tacagtgaca tgtgtgagct cactgaggag gtgagcgaga agaagtcata 900

tgccctagat ggaaaagaag aagcagaggc tgctctggag aagggggatg 950

agagtgctga ctgtcacctg tggtatgcgg tgctttgtgg tcagctggct 1000

gagcatgaga gcatccagag gcgcatccag agtggcttta gcttcaagga 1050

gcatgtggac aaagccattg ctctccagcc agaaaacccc atggctcact 1100

ttcttcttgg caggtggtgc tatcaggtct ctcacctgag ctggctagaa 1150

aaaaaaactg ctacagcctt gcttgaaagc cctctcagtg ccactgtgga 1200

agatgccctc cagagcttcc taaaggctga agaactacag ccaggatttt 1250

ccaaagcagg aagggtatat atttccaagt gctacagaga actagggaaa 1300

aactctgaag ctagatggtg gatgaagttg gccctggagc tgccagatgt 1350

cacgaaggag gatttggcta tccagaagga cctggaagaa ctggaagtca 1400

ttttacgaga ctaaccacgt ttcactggcc ttcatgactt gatgccacta 1450

tttaaggtgg gggggcgggg aggctttttt ccttagacct tgctgagatc 1500

aggaaaccac acaaatctgt ctcctgggtc tgactgctac ccactaccac 1550

tccccattag ttaatttatt ctaacctcta acctaatcta gaattggggc 1600

agtactcatg gcttccgttt ctgttgttct ctcccttgag taatctctta 1650

aaaaaatcaa gattcacacc tgccccagga ttacacatgg gtagagcctg 1700

caagacctga gaccttccaa ttgctggtga ggtggatgaa cttcaaagct 1750

ataggaacaa agcacataac ttgtcacttt aatctttttc actgactaat 1800

aggactcagt acatatagtc ttaagatcat accttaccta ccaaggtaaa 1850

aagagggatc agagtggccc acagacattg ctttcttatc acctatcatg 1900

tgaattctac ctgtattcct gggctggacc acttgataac ttccagtgtc 1950

ctggcagctt ttggaatgac agcagtggta tggggtttat gatgctataa 2000

aacaatgtct gaaaagttgc ctagaatata ttttgttaca aacttgaaat 2050

aaaccaaatt tgatgtt 2067

<210> 68
<211> 470
<212> PRT
<213> Homo Sapien

<400> 68
Met Ser Arg Leu Gly Ala Leu Gly Gly Ala Arg Ala Gly Leu Gly
1               5                   10                  15

Leu Leu Leu Gly Thr Ala Ala Gly Leu Gly Phe Leu Cys Leu Leu
                20                  25                  30

Tyr Ser Gln Arg Trp Lys Arg Thr Gln Arg His Gly Arg Ser Gln
                35                  40                  45

Ser Leu Pro Asn Ser Leu Asp Tyr Thr Gln Thr Ser Asp Pro Gly
                50                  55                  60

Arg His Val Met Leu Leu Arg Ala Val Pro Gly Gly Ala Gly Asp
                65                  70                  75

Ala Ser Val Leu Pro Ser Leu Pro Arg Glu Gly Gln Glu Lys Val
                80                  85                  90

Leu Asp Arg Leu Asp Phe Val Leu Thr Ser Leu Val Ala Leu Arg
                95                  100                 105

Arg Glu Val Glu Glu Leu Arg Ser Ser Leu Arg Gly Leu Ala Gly
                110                 115                 120

Glu Ile Val Gly Glu Val Arg Cys His Met Glu Glu Asn Gln Arg
                125                 130                 135

Val Ala Arg Arg Arg Arg Phe Pro Phe Val Arg Glu Arg Ser Asp
                140                 145                 150

Ser Thr Gly Ser Ser Ser Val Tyr Phe Thr Ala Ser Ser Gly Ala
                155                 160                 165

Thr Phe Thr Asp Ala Glu Ser Glu Gly Gly Tyr Thr Thr Ala Asn
                170                 175                 180

Ala Glu Ser Asp Asn Glu Arg Asp Ser Asp Lys Glu Ser Glu Asp
                185                 190                 195

Gly Glu Asp Glu Val Ser Cys Glu Thr Val Lys Met Gly Arg Lys
                200                 205                 210

Asp Ser Leu Asp Leu Glu Glu Glu Ala Ala Ser Gly Ala Ser Ser
                215                 220                 225

Ala Leu Glu Ala Gly Gly Ser Ser Gly Leu Glu Asp Val Leu Pro
                230                 235                 240

Leu Leu Gln Gln Ala Asp Glu Leu His Arg Gly Asp Glu Gln Gly
                245                 250                 255

Lys Arg Glu Gly Phe Gln Leu Leu Leu Asn Asn Lys Leu Val Tyr
                260                 265                 270

Gly Ser Arg Gln Asp Phe Leu Trp Arg Leu Ala Arg Ala Tyr Ser
                275                 280                 285

Asp Met Cys Glu Leu Thr Glu Glu Val Ser Glu Lys Lys Ser Tyr
                290                 295                 300

Ala Leu Asp Gly Lys Glu Glu Ala Glu Ala Ala Leu Glu Lys Gly
                305                 310                 315

Asp Glu Ser Ala Asp Cys His Leu Trp Tyr Ala Val Leu Cys Gly
                320                 325                 330

Gln Leu Ala Glu His Glu Ser Ile Gln Arg Arg Ile Gln Ser Gly
                335                 340                 345

Phe Ser Phe Lys Glu His Val Asp Lys Ala Ile Ala Leu Gln Pro
                350                 355                 360

Glu Asn Pro Met Ala His Phe Leu Leu Gly Arg Trp Cys Tyr Gln
                365                 370                 375

Val Ser His Leu Ser Trp Leu Glu Lys Lys Thr Ala Thr Ala Leu
                380                 385                 390

Leu Glu Ser Pro Leu Ser Ala Thr Val Glu Asp Ala Leu Gln Ser
                395                 400                 405

Phe Leu Lys Ala Glu Glu Leu Gln Pro Gly Phe Ser Lys Ala Gly
                410                 415                 420

Arg Val Tyr Ile Ser Lys Cys Tyr Arg Glu Leu Gly Lys Asn Ser
                425                 430                 435

Glu Ala Arg Trp Trp Met Lys Leu Ala Leu Glu Leu Pro Asp Val
                440                 445                 450

Thr Lys Glu Asp Leu Ala Ile Gln Lys Asp Leu Glu Glu Leu Glu
                455                 460                 465

Val Ile Leu Arg Asp
                470

<210> 69
<211> 2250
<212> DNA
<213> Homo Sapien

<400> 69
cccacgcgtc cgaaacactt taaacctgac cagctaaatg gataaaccta 50

gcctgcatag cttttaaact ggggtctcat acagcacagg aggcctactt 100

gcttcaagaa ctgaaaatcc agaggatgaa ttgctttatc tgggaatggc 150

aaaagccagc acaataagga atgccagttt gtatggggct actagctcac 200

atgcgggatc agaatggtgt gaatgacagc cgcactgtgt catgaaggtg 250
gtggtggttt ccgcacaaga gaccaaataa gaagaaagct gagagagggg 300
ggaaacgttt ttggatgaca aaggatgggt ttccatttaa ttacgcagct 350
gaaaggcatg agtgtggtgc tggtgctact tcctacactg ctgcttgtta 400
tgctcacggg tgctcagaga gcttgcccaa agaactgcag atgtgatggc 450
aaaattgtgt actgtgagtc tcatgctttc gcagatatcc ctgagaacat 500
ttctggaggg tcacaaggct tatcattaag gttcaacagc attcagaagc 550
tcaaatccaa tcagtttgcc ggccttaacc agcttatatg gctttatctt 600
gaccataatt acattagctc agtggatgaa gatgcatttc aagggatccg 650
tagactgaaa gaattaattc taagctccaa caaaattact tatctgcaca 700
ataaaacatt tcacccagtt cccaatctcc gcaatctgga cctctcctac 750
aataagcttc agacattgca atctgaacaa tttaaaggcc ttcggaaact 800
catcattttg cacttgagat ctaactcact aaagactgtg cccataagag 850
tttttcaaga ctgtcggaat cttgattttt tggatttggg ttacaatcgt 900
cttcgaagct tgtcccgaaa tgcatttgct ggcctcttga agttaaagga 950
gctccacctg gagcacaacc agttttccaa gatcaacttt gctcattttc 1000
cacgtctctt caacctccgc tcaatttact tacaatggaa caggattcgc 1050
tccattagcc aaggtttgac atggacttgg agttccttac acaacttgga 1100
tttatcaggg aatgacatcc aaggaattga gccgggcaca tttaaatgcc 1150
tccccaattt acaaaaattg aatttggatt ccaacaagct caccaatatc 1200
tcacaggaaa ctgtcaatgc gtggatatca ttaatatcca tcacattgtc 1250
tggaaatatg tgggaatgca gtcggagcat ttgtccttta ttttattggc 1300
ttaagaattt caaaggaaat aaggaaagca ccatgatatg tgcgggacct 1350
aagcacatcc agggtgaaaa ggttagtgat gcagtggaaa catataatat 1400
ctgttctgaa gtccaggtgg tcaacacaga aagatcacac ctggtgcccc 1450
aaactcccca gaaacctctg attatcccta gacctaccat cttcaaacct 1500
gacgtcaccc aatccacctt tgaaacacca agcccttccc cagggtttca 1550
gattcctggc gcagagcaag agtatgagca tgtttcattt cacaaaatta 1600
ttgccgggag tgtggctctc tttctctcag tggccatgat cctcttggtg 1650
atctatgtgt cttggaaacg ctacccagcc agcatgaaac aactccagca 1700
acactctctt atgaagaggc ggcggaaaaa ggccagagag tctgaaagac 1750
aaatgaattc ccctttacag gagtattatg tggactacaa gcctacaaac 1800
tctgagacca tggatatatc ggttaatgga tctgggccct gcacatatac 1850
catctctggc tccagggaat gtgagatgcc acaccacatg aagcccttgc 1900

catattacag ctatgaccag cctgtgatcg ggtactgcca ggcccaccag 1950

ccactccatg tcaccaaggg ctatgagaca gtgtctccag agcaggacga 2000

aagccccggc ctggagctgg gccgagacca cagcttcatc gccaccatcg 2050

ccaggtcggc agcaccggcc atctacctag agagaattgc aaactaacgc 2100

tgaagccaac tcctcactgg ggagctccat gggggggagg gagggccttc 2150

atcttaaagg agaatgggtg tccacaatcg cgcaatcgag caagctcatc 2200

gttcctgtta aaacatttat ggcataggga aaaaaaaaaa aaaaaaaaaa 2250

<210> 70
<211> 590
<212> PRT
<213> Homo Sapien

<400> 70
Met Gly Phe His Leu Ile Thr Gln Leu Lys Gly Met Ser Val Val
1               5                   10                  15

Leu Val Leu Leu Pro Thr Leu Leu Leu Val Met Leu Thr Gly Ala
                20                  25                  30

Gln Arg Ala Cys Pro Lys Asn Cys Arg Cys Asp Gly Lys Ile Val
                35                  40                  45

Tyr Cys Glu Ser His Ala Phe Ala Asp Ile Pro Glu Asn Ile Ser
                50                  55                  60

Gly Gly Ser Gln Gly Leu Ser Leu Arg Phe Asn Ser Ile Gln Lys
                65                  70                  75

Leu Lys Ser Asn Gln Phe Ala Gly Leu Asn Gln Leu Ile Trp Leu
                80                  85                  90

Tyr Leu Asp His Asn Tyr Ile Ser Ser Val Asp Glu Asp Ala Phe
                95                  100                 105

Gln Gly Ile Arg Arg Leu Lys Glu Leu Ile Leu Ser Ser Asn Lys
                110                 115                 120

Ile Thr Tyr Leu His Asn Lys Thr Phe His Pro Val Pro Asn Leu
                125                 130                 135

Arg Asn Leu Asp Leu Ser Tyr Asn Lys Leu Gln Thr Leu Gln Ser
                140                 145                 150

Glu Gln Phe Lys Gly Leu Arg Lys Leu Ile Ile Leu His Leu Arg
                155                 160                 165

Ser Asn Ser Leu Lys Thr Val Pro Ile Arg Val Phe Gln Asp Cys
                170                 175                 180

Arg Asn Leu Asp Phe Leu Asp Leu Gly Tyr Asn Arg Leu Arg Ser
                185                 190                 195

Leu Ser Arg Asn Ala Phe Ala Gly Leu Leu Lys Leu Lys Glu Leu
                200                 205                 210

His Leu Glu His Asn Gln Phe Ser Lys Ile Asn Phe Ala His Phe
                215                 220                 225

Pro Arg Leu Phe Asn Leu Arg Ser Ile Tyr Leu Gln Trp Asn Arg
                230                 235                 240

Ile Arg Ser Ile Ser Gln Gly Leu Thr Trp Thr Trp Ser Ser Leu
                245                 250                 255

His Asn Leu Asp Leu Ser Gly Asn Asp Ile Gln Gly Ile Glu Pro
                260                 265                 270

Gly Thr Phe Lys Cys Leu Pro Asn Leu Gln Lys Leu Asn Leu Asp
                275                 280                 285

Ser Asn Lys Leu Thr Asn Ile Ser Gln Glu Thr Val Asn Ala Trp
                290                 295                 300

Ile Ser Leu Ile Ser Ile Thr Leu Ser Gly Asn Met Trp Glu Cys
                305                 310                 315

Ser Arg Ser Ile Cys Pro Leu Phe Tyr Trp Leu Lys Asn Phe Lys
                320                 325                 330

Gly Asn Lys Glu Ser Thr Met Ile Cys Ala Gly Pro Lys His Ile
                335                 340                 345

Gln Gly Glu Lys Val Ser Asp Ala Val Glu Thr Tyr Asn Ile Cys
                350                 355                 360

Ser Glu Val Gln Val Val Asn Thr Glu Arg Ser His Leu Val Pro
                365                 370                 375

Gln Thr Pro Gln Lys Pro Leu Ile Ile Pro Arg Pro Thr Ile Phe
                380                 385                 390

Lys Pro Asp Val Thr Gln Ser Thr Phe Glu Thr Pro Ser Pro Ser
                395                 400                 405

Pro Gly Phe Gln Ile Pro Gly Ala Glu Gln Glu Tyr Glu His Val
                410                 415                 420

Ser Phe His Lys Ile Ile Ala Gly Ser Val Ala Leu Phe Leu Ser
                425                 430                 435

Val Ala Met Ile Leu Leu Val Ile Tyr Val Ser Trp Lys Arg Tyr
                440                 445                 450

Pro Ala Ser Met Lys Gln Leu Gln Gln His Ser Leu Met Lys Arg
                455                 460                 465

Arg Arg Lys Lys Ala Arg Glu Ser Glu Arg Gln Met Asn Ser Pro
                470                 475                 480

Leu Gln Glu Tyr Tyr Val Asp Tyr Lys Pro Thr Asn Ser Glu Thr
                485                 490                 495

Met Asp Ile Ser Val Asn Gly Ser Gly Pro Cys Thr Tyr Thr Ile
                500                 505                 510

Ser Gly Ser Arg Glu Cys Glu Met Pro His His Met Lys Pro Leu
                515                 520                 525

Pro Tyr Tyr Ser Tyr Asp Gln Pro Val Ile Gly Tyr Cys Gln Ala
                530                 535                 540

His Gln Pro Leu His Val Thr Lys Gly Tyr Glu Thr Val Ser Pro
                545                 550                 555

Glu Gln Asp Glu Ser Pro Gly Leu Glu Leu Gly Arg Asp His Ser
                560                 565                 570

Phe Ile Ala Thr Ile Ala Arg Ser Ala Ala Pro Ala Ile Tyr Leu
                575                 580                 585

Glu Arg Ile Ala Asn
                590

<210> 71
<211> 521
<212> DNA
<213> Homo Sapien

<400> 71
ttccagtcag agttaagtta aaacagaaaa aaggaagatg gcaagaatat 50

tgttactttt cctcccgggt cttgtggctg tatgtgctgt gcatggaata 100

tttatggacc gtctagcttc caagaagctc tgtgcagatg atgagtgtgt 150

ctatactatt tctctggcta gtgctcaaga agattataat gccccggact 200

gtagattcat taacgttaaa aaagggcagc agatctatgt gtactcaaag 250

ctggtaaaag aaaatggagc tggagaattt tgggctggca gtgtttatgg 300

tgatggccag gacgagatgg gagtcgtggg ttatttcccc aggaacttgg 350

tcaaggaaca gcgtgtgtac caggaagcta ccaaggaagt tcccaccacg 400

gatattgact tcttctgcga gtaataaatt agttaaaact gcaaatagaa 450

agaaaacacc aaaaataaag aaaagagcaa aagtggccaa aaaatgcatg 500

tctgtaattt tggactgacg t 521

<210> 72
<211> 128
<212> PRT
<213> Homo Sapien

<400> 72
Met Ala Arg Ile Leu Leu Leu Phe Leu Pro Gly Leu Val Ala Val
  1               5                  10                  15

Cys Ala Val His Gly Ile Phe Met Asp Arg Leu Ala Ser Lys Lys
                 20                  25                  30

Leu Cys Ala Asp Asp Glu Cys Val Tyr Thr Ile Ser Leu Ala Ser
                 35                  40                  45

Ala Gln Glu Asp Tyr Asn Ala Pro Asp Cys Arg Phe Ile Asn Val
                 50                  55                  60

Lys Lys Gly Gln Gln Ile Tyr Val Tyr Ser Lys Leu Val Lys Glu
                 65                  70                  75

Asn Gly Ala Gly Glu Phe Trp Ala Gly Ser Val Tyr Gly Asp Gly
                 80                  85                  90

Gln Asp Glu Met Gly Val Val Gly Tyr Phe Pro Arg Asn Leu Val
                 95                 100                 105

Lys Glu Gln Arg Val Tyr Gln Glu Ala Thr Lys Glu Val Pro Thr
                110                 115                 120

Thr Asp Ile Asp Phe Phe Cys Glu
                125

<210> 73
<211> 720
<212> DNA

<213> Homo Sapien

<400> 73
ctcagatttg ccatggagaa attttcagtc tcggcaatcc tgcttcttgt 50

ggccatctct ggtactctgg ccaaagacac cacagtcaaa tctggatcca 100

aaaaggaccc aaaggactct cgacccaaac taccccagac cctgtccaga 150

ggttggggag atcagctcat ctggactcag acttacgaag aagccttata 200

caaatccaag acaagcaaca gacccttgat ggtcattcat cacttggacg 250

aatgcccgca cagtcaagct ttaaagaaag tgtttgctga aaataaggag 300

atccagaaat tggcagagca gtttgttctc ctcaacttga tctatgaaac 350

aactgacaag cacctttctc ctgatggcca gtacgtcccc agaattgtgt 400

ttgtggaccc ttccctgacg gtgagggcag acatcaccgg aagatactca 450

aaccgtctct acgcttatga accttctgac acagctctgt tgcacgacaa 500

catgaagaaa gctctcaagt tgctgaagac agagttgtag agtcaactgt 550

acagtgcctc aggagccggg aaggcagaag cactgtggac ctgccgatga 600

cattacagtt taatgttaca acaaatgtat tttttaaaca cccacgtgtg 650

gggaaacaat attattatct actacagaca catgattttc tagaaaataa 700

agtcttgtga gaactccaaa 720

<210> 74
<211> 175
<212> PRT
<213> Homo Sapien

<400> 74
Met Glu Lys Phe Ser Val Ser Ala Ile Leu Leu Leu Val Ala Ile
1               5                   10                  15

Ser Gly Thr Leu Ala Lys Asp Thr Thr Val Lys Ser Gly Ser Lys
                20                  25                  30

Lys Asp Pro Lys Asp Ser Arg Pro Lys Leu Pro Gln Thr Leu Ser
                35                  40                  45

Arg Gly Trp Gly Asp Gln Leu Ile Trp Thr Gln Thr Tyr Glu Glu
                50                  55                  60

Ala Leu Tyr Lys Ser Lys Thr Ser Asn Arg Pro Leu Met Val Ile
                65                  70                  75

His His Leu Asp Glu Cys Pro His Ser Gln Ala Leu Lys Lys Val
                80                  85                  90

Phe Ala Glu Asn Lys Glu Ile Gln Lys Leu Ala Glu Gln Phe Val
                95                  100                 105

Leu Leu Asn Leu Ile Tyr Glu Thr Thr Asp Lys His Leu Ser Pro
                110                 115                 120

Asp Gly Gln Tyr Val Pro Arg Ile Val Phe Val Asp Pro Ser Leu
                125                 130                 135

Thr Val Arg Ala Asp Ile Thr Gly Arg Tyr Ser Asn Arg Leu Tyr
                140                 145                 150

Ala Tyr Glu Pro Ser Asp Thr Ala Leu Leu His Asp Asn Met Lys
                155                 160                 165

Lys Ala Leu Lys Leu Leu Lys Thr Glu Leu
                170                 175

<210> 75
<211> 776
<212> DNA
<213> Homo Sapien

<400> 75
gccggcgcca gggcaggcgg gcggctggca gctgtggcgc cgacatggct 50

gcgctggtgg agccgctggg gctggagcgg gacgtgtccc gggcggttga 100

gctcctcgag cggctccagc gcagcgggga gctgccgccg cagaagctgc 150

aggccctcca gcgagttctg cagagccgct tctgctccgc tatccgagag 200

gtgtatgagc agctttatga cacgctggac atcaccggca gcgccgagat 250

ccgagcccat gccacagcca aggccacagt ggctgccttc acagccagcg 300

agggccacgc acatcccagg gtagtggagc tacccaagac ggatgagggc 350

ctaggcttca acatcatggg tggcaaagag caaaactcgc ccatctacat 400

ctcccgggtc atcccagggg gtgtggctga ccgccatgga ggcctcaagc 450

gtggggatca actgttgtcg gtgaacggtg tgagcgttga gggtgagcag 500

catgagaagg cggtggagct gctgaaggcg gcccagggct cggtgaagct 550

ggttgtccgt tacacaccgc gagtgctgga ggagatggag gcccggttcg 600

agaagatgcg ctctgcccgc cggcgccaac agcatcagag ctactcgtcc 650

ttggagtctc gaggttgaaa ccacagatct ggacgttcac gtgcactctc 700

ttcctgtaca gtatttattg ttcctggcac tttatttaaa gatatttgac 750

cctcaaaaaa aaaaaaaaaa aaaaaa 776

<210> 76
<211> 207
<212> PRT
<213> Homo Sapien

<400> 76
Met Ala Ala Leu Val Glu Pro Leu Gly Leu Glu Arg Asp Val Ser
  1               5                  10                  15

Arg Ala Val Glu Leu Leu Glu Arg Leu Gln Arg Ser Gly Glu Leu
                 20                  25                  30

Pro Pro Gln Lys Leu Gln Ala Leu Gln Arg Val Leu Gln Ser Arg
                 35                  40                  45

Phe Cys Ser Ala Ile Arg Glu Val Tyr Glu Gln Leu Tyr Asp Thr
                 50                  55                  60

Leu Asp Ile Thr Gly Ser Ala Glu Ile Arg Ala His Ala Thr Ala
                 65                  70                  75

Lys Ala Thr Val Ala Ala Phe Thr Ala Ser Glu Gly His Ala His
                 80                  85                  90

Pro Arg Val Val Glu Leu Pro Lys Thr Asp Glu Gly Leu Gly Phe
                95                  100                 105

Asn Ile Met Gly Gly Lys Glu Gln Asn Ser Pro Ile Tyr Ile Ser
                110                 115                 120

Arg Val Ile Pro Gly Gly Val Ala Asp Arg His Gly Gly Leu Lys
                125                 130                 135

Arg Gly Asp Gln Leu Leu Ser Val Asn Gly Val Ser Val Glu Gly
                140                 145                 150

Glu Gln His Glu Lys Ala Val Glu Leu Leu Lys Ala Ala Gln Gly
                155                 160                 165

Ser Val Lys Leu Val Val Arg Tyr Thr Pro Arg Val Leu Glu Glu
                170                 175                 180

Met Glu Ala Arg Phe Glu Lys Met Arg Ser Ala Arg Arg Arg Gln
                185                 190                 195

Gln His Gln Ser Tyr Ser Ser Leu Glu Ser Arg Gly
                200                 205

<210> 77
<211> 939
<212> DNA
<213> Homo Sapien

<400> 77
ctgtcagctg aggatccagc cgaaagagga gccaggcact caggccacct 50

gagtctactc acctggacaa ctggaatctg gcaccaattc taaaccactc 100

agcttctccg agctcacacc ccggagatca cctgaggacc cgagccattg 150

atggactcgg acgagaccgg gttcgagcac tcaggactgt gggtttctgt 200

gctggctggt ctgctgggag cctgccaggc acaccccatc cctgactcca 250

gtcctctcct gcaattcggg ggccaagtcc ggcagcggta cctctacaca 300

gatgatgccc agcagacaga agcccacctg gagatcaggg aggatgggac 350

ggtggggggc gctgctgacc agagccccga aagtctcctg cagctgaaag 400

ccttgaagcc gggagttatt caaatcttgg gagtcaagac atccaggttc 450

ctgtgccagc ggccagatgg ggccctgtat ggatcgctcc actttgaccc 500

tgaggcctgc agcttccggg agctgcttct tgaggacgga tacaatgttt 550

accagtccga agcccacggc ctcccgctgc acctgccagg gaacaagtcc 600

ccacaccggg accctgcacc ccgaggacca gctcgcttcc tgccactacc 650

aggcctgccc cccgcactcc cggagccacc cggaatcctg gccccccagc 700

cccccgatgt gggctcctcg gaccctctga gcatggtggg accttcccag 750

ggccgaagcc ccagctacgc ttcctgaagc cagaggctgt ttactatgac 800

atctcctctt tatttattag gttatttatc ttatttattt ttttattttt 850

cttacttgag ataataaaga gttccagagg agaaaaaaaa aaaaaaaaaa 900

aaaaaaaaaa aaaaaaaaaa aaaaaaaaaa aaaaaaaag 939

<210> 78
<211> 208
<212> PRT
<213> Homo Sapien

<400> 78
Met Asp Ser Asp Glu Thr Gly Phe Glu His Ser Gly Leu Trp Val
1               5                   10                  15

Ser Val Leu Ala Gly Leu Leu Gly Ala Cys Gln Ala His Pro Ile
                20                  25                  30

Pro Asp Ser Ser Pro Leu Leu Gln Phe Gly Gly Gln Val Arg Gln
                35                  40                  45

Arg Tyr Leu Tyr Thr Asp Asp Ala Gln Gln Thr Glu Ala His Leu
                50                  55                  60

Glu Ile Arg Glu Asp Gly Thr Val Gly Gly Ala Ala Asp Gln Ser
                65                  70                  75

Pro Glu Ser Leu Leu Gln Leu Lys Ala Leu Lys Pro Gly Val Ile
                80                  85                  90

Gln Ile Leu Gly Val Lys Thr Ser Arg Phe Leu Cys Gln Arg Pro
                95                  100                 105

Asp Gly Ala Leu Tyr Gly Ser Leu His Phe Asp Pro Glu Ala Cys
                110                 115                 120

Ser Phe Arg Glu Leu Leu Leu Glu Asp Gly Tyr Asn Val Tyr Gln
                125                 130                 135

Ser Glu Ala His Gly Leu Pro Leu His Leu Pro Gly Asn Lys Ser
                140                 145                 150

Pro His Arg Asp Pro Ala Pro Arg Gly Pro Ala Arg Phe Leu Pro
                155                 160                 165

Leu Pro Gly Leu Pro Pro Ala Leu Pro Glu Pro Pro Gly Ile Leu
                170                 175                 180

Ala Pro Gln Pro Pro Asp Val Gly Ser Ser Asp Pro Leu Ser Met
                185                 190                 195

Val Gly Pro Ser Gln Gly Arg Ser Pro Ser Tyr Ala Ser
                200                 205

<210> 79
<211> 2732
<212> DNA
<213> Homo Sapien

<400> 79
agtcccagac gggcttttcc cagagagcta aaagagaagg gccagagaat 50

gtcgtcccag ccagcaggga accagacctc ccccggggcc acagaggact 100

actcctatgg cagctggtac atcgatgagc cccagggggg cgaggagctc 150

cagccagagg gggaagtgcc ctcctgccac accagcatac cacccggcct 200

gtaccacgcc tgcctggcct cgctgtcaat ccttgtgctg ctgctcctgg 250

ccatgctggt gaggcgccgc cagctctggc ctgactgtgt gcgtggcagg 300

cccggcctgc ccagccctgt ggatttcttg gctggggaca ggccccgggc 350

agtgcctgct gctgttttca tggtcctcct gagctccctg tgtttgctgc 400

tccccgacga ggacgcattg cccttcctga ctctcgcctc agcacccagc 450

caagatggga aaactgaggc tccaagaggg gcctggaaga tactgggact 500

gttctattat gctgccctct actaccctct ggctgcctgt gccacggctg 550

gccacacagc tgcacacctg ctcggcagca cgctgtcctg ggcccacctt 600

ggggtccagg tctggcagag ggcagagtgt ccccaggtgc ccaagatcta 650

caagtactac tccctgctgg cctccctgcc tctcctgctg ggcctcggat 700

tcctgagcct ttggtaccct gtgcagctgg tgagaagctt cagccgtagg 750

acaggagcag gctccaaggg gctgcagagc agctactctg aggaatatct 800

gaggaacctc ctttgcagga agaagctggg aagcagctac cacacctcca 850

agcatggctt cctgtcctgg gcccgcgtct gcttgagaca ctgcatctac 900

actccacagc caggattcca tctcccgctg aagctggtgc tttcagctac 950

actgacaggg acggccattt accaggtggc cctgctgctg ctggtgggcg 1000

tggtacccac tatccagaag gtgagggcag gggtcaccac ggatgtctcc 1050

tacctgctgg ccggctttgg aatcgtgctc tccgaggaca agcaggaggt 1100

ggtggagctg gtgaagcacc atctgtgggc tctggaagtg tgctacatct 1150

cagccttggt cttgtcctgc ttactcacct tcctggtcct gatgcgctca 1200

ctggtgacac acaggaccaa ccttcgagct ctgcaccgag gagctgccct 1250

ggacttgagt cccttgcatc ggagtcccca tccctcccgc caagccatat 1300

tctgttggat gagcttcagt gcctaccaga cagcctttat ctgccttggg 1350

ctcctggtgc agcagatcat cttcttcctg ggaaccacgg ccctggcctt 1400

cctggtgctc atgcctgtgc tccatggcag gaacctcctg ctcttccgtt 1450

ccctggagtc ctcgtggccc ttctggctga ctttggccct ggctgtgatc 1500

ctgcagaaca tggcagccca ttgggtcttc ctggagactc atgatggaca 1550

cccacagctg accaaccggc gagtgctcta tgcagccacc tttcttctct 1600

tccccctcaa tgtgctggtg ggtgccatgg tggccacctg gcgagtgctc 1650

ctctctgccc tctacaacgc catccacctt ggccagatgg acctcagcct 1700

gctgccaccg agagccgcca ctctcgaccc cggctactac acgtaccgaa 1750

acttcttgaa gattgaagtc agccagtcgc atccagccat gacagccttc 1800

tgctccctgc tcctgcaagc gcagagcctc ctacccagga ccatggcagc 1850

cccccaggac agcctcagac caggggagga agacgaaggg atgcagctgc 1900

tacagacaaa ggactccatg gccaagggag ctaggcccgg ggccagccgc 1950

ggcagggctc gctggggtct ggcctacacg ctgctgcaca acccaaccct 2000

gcaggtcttc cgcaagacgg ccctgttggg tgccaatggt gcccagccct 2050

gagggcaggg aaggtcaacc cacctgccca tctgtgctga ggcatgttcc 2100

tgcctaccat cctcctccct ccccggctct cctcccagca tcacaccagc 2150

catgcagcca gcaggtcctc cggatcactg tggttgggtg gaggtctgtc 2200

tgcactggga gcctcaggag ggctctgctc cacccacttg gctatgggag 2250

agccagcagg ggttctggag aaaaaaactg gtgggttagg gccttggtcc 2300

aggagccagt tgagccaggg cagccacatc caggcgtctc cctaccctgg 2350

ctctgccatc agccttgaag ggcctcgatg aagccttctc tggaaccact 2400

ccagcccagc tccacctcag ccttggcctt cacgctgtgg aagcagccaa 2450

ggcacttcct caccccctca gcgccacgga cctctctggg gagtggccgg 2500

aaagctcccg gtcctctggc ctgcagggca gcccaagtca tgactcagac 2550

caggtcccac actgagctgc ccacactcga gagccagata tttttgtagt 2600

ttttatgcct ttggctatta tgaaagaggt tagtgtgttc cctgcaataa 2650

acttgttcct gagaaaaaaa aaaaaaaaaa aaaaaaaaaa aaaaaaaaaa 2700

aaaaaaaaaa aaaaaaaaaa aaaaaaaaaa aa 2732

<210> 80
<211> 667
<212> PRT
<213> Homo Sapien

<400> 80

<210> 81
<211> 1186
<212> DNA
<213> Homo Sapien

<400> 81
aaaaaataca gcaggtgaag gaggttggag agtagggggt ggagggccca 50

cgcagcactt gtccttcacc ctggagggga tctgttacat gccccagatt 100

gctggtcccc tagaaatgtt actgaggcag cctctgcatt tttgcaggga 150

ttgttttcta ctgtttgaca ttcacgtaac ctcctaacgc tgtctgggga 200

agatgctacc ccctgctctc cccgtctttc ctgcactctc agcaatggga 250

tgggctgact gatgccctgt gggctggaaa gctgaccaca gttgctgcag 300

accagacccc ctcacatagt gagtgctggg ctgaggaatc caggagagcc 350

cgagggggga cactgaaggt gtatcgttgg ccctgccagc tgcaagtgaa 400

ctgcttctga tgaattttaa tagggagaaa gaagtatttg ctaagaatgg 450

caatcctgac gctcagcctt caactcatct tgttattaat accatcaata 500

tcccatgagg ctcataaaac gagtctttct tcttggaaac atgaccaaga 550

ttgggcaaac gtctccaaca tgactttcag caacggaaaa ctaagagtca 600

aaggcattta ttaccggaat gccgacattt gctctcgaca tcgcgtaacc 650

tcagcaggcc taactctgca ggaccttcag ctatggtgta atttgaggtc 700

agtggccaga ggacagatcc cgtctacatt atgagtgaag cggagagcta 750

ctgcagggtt ctgagcagag tcctaattta tattttagaa gaatcatcat 800

ggctcctaga ttaggaataa aacgaagggg cccagggatg gaaacgatga 850

gtccagttgg gttactgcaa agatccaggc cagaaatcca ggcacagtgg 900

cacacacctg agtcccagat aattccacct actggtcctg ctctgtggcc 950

tactggtccg agtccagccc cgactgattt ctgggcctgt aatgtctaaa 1000

aacgctccct gctgatgttt tgcaagtgac tgtgttactt gaaggcagtt 1050

cctaggataa actagtcgct ttatcattac agaatcattc actgagcatc 1100

aactatgtaa ccagcattgg gttgggtgcc agagatccaa agctaagaca 1150

ccaaaacctg ctctccagga aacgagaggc tgagaa 1186

<210> 82
<211> 95
<212> PRT
<213> Homo Sapien

<400> 82

<210> 83
<211> 1677
<212> DNA
<213> Homo Sapien

<400> 83
aatagaagtc ctcaggacgg agcagaggtg gccggcgggc ccggctgact 50

gcgcctctgc tttctttcca taaccttttc tttcggactc gaatcacggc 100

tgctgcgaag ggtctagttc cggacactag ggtgcccgaa cgcgctgatg 150

ccccgagtgc tcgcagggct tcccgctaac catgctgccg ccgccgcggc 200

ccgcagctgc cttggcgctg cctgtgctcc tgctactgct ggtggtgctg 250

acgccgcccc cgaccggcgc aaggccatcc ccaggcccag attacctgcg 300

gcgcggctgg atgcggctgc tagcggaggg cgagggctgc gctccctgcc 350

ggccagaaga gtgcgccgcg ccgcggggct gcctggcggg cagggtgcgc 400

gacgcgtgcg gctgctgctg ggaatgcgcc aacctcgagg gccagctctg 450
cgacctggac cccagtgctc acttctacgg gcactgcggc gagcagcttg 500
agtgccggct ggacacaggc ggcgacctga gccgcggaga ggtgccggaa 550
cctctgtgtg cctgtcgttc gcagagtccg ctctgcgggt ccgacggtca 600
cacctactcc cagatctgcc gcctgcagga ggcggcccgc gctcggcccg 650
atgccaacct cactgtggca cacccggggc cctgcgaatc ggggccccag 700
atcgtgtcac atccatatga cacttggaat gtgacagggc aggatgtgat 750
ctttggctgt gaagtgtttg cctaccccat ggcctccatc gagtggagga 800
aggatggctt ggacatccag ctgccagggg atgaccccca catctctgtg 850
cagtttaggg gtggacccca gaggtttgag gtgactggct ggctgcagat 900
ccaggctgtg cgtcccagtg atgagggcac ttaccgctgc cttggccgca 950
atgccctggg tcaagtggag gcccctgcta gcttgacagt gctcacacct 1000
gaccagctga actctacagg catcccccag ctgcgatcac taaacctggt 1050
tcctgaggag gaggctgaga gtgaagagaa tgacgattac tactaggtcc 1100
agagctctgg cccatggggg tgggtgagcg gctatagtgt tcatccctgc 1150
tcttgaaaag acctggaaag gggagcaggg tcccttcatc gactgctttc 1200
atgctgtcag tagggatgat catgggaggc ctatttgact ccaaggtagc 1250
agtgtggtag gatagagaca aaagctggag gagggtaggg agagaagctg 1300
agaccaggac cggtggggta caaaggggcc catgcaggag atgccctggc 1350
cagtaggacc tccaacaggt tgtttcccag gctggggtgg gggcctgagc 1400
agacacagag gtgcaggcac caggattctc cacttcttcc agccctgctg 1450
ggccacagtt ctaactgccc ttcctcccag gccctggttc ttgctatttc 1500
ctggtcccca acgtttatct agcttgtttg ccctttcccc aaactcatct 1550
tccagaactt ttccctctct cctaagcccc agttgcacct actaactgca 1600
gtcccttttg ctgtctgccg tcttttgtac aagagagaga acagcggagc 1650
atgacttagt tcagtgcaga gagattt 1677

<210> 84
<211> 304
<212> PRT
<213> Homo Sapien

<400> 84
Met Leu Pro Pro Pro Arg Pro Ala Ala Ala Leu Ala Leu Pro Val
1               5                   10                  15

Leu Leu Leu Leu Leu Val Val Leu Thr Pro Pro Pro Thr Gly Ala
                20                  25                  30

Arg Pro Ser Pro Gly Pro Asp Tyr Leu Arg Arg Gly Trp Met Arg
                35                  40                  45

Leu Leu Ala Glu Gly Glu Gly Cys Ala Pro Cys Arg Pro Glu Glu
                50                  55                  60

Cys Ala Ala Pro Arg Gly Cys Leu Ala Gly Arg Val Arg Asp Ala
                65                  70                  75

Cys Gly Cys Cys Trp Glu Cys Ala Asn Leu Glu Gly Gln Leu Cys
                80                  85                  90

Asp Leu Asp Pro Ser Ala His Phe Tyr Gly His Cys Gly Glu Gln
                95                  100                 105

Leu Glu Cys Arg Leu Asp Thr Gly Gly Asp Leu Ser Arg Gly Glu
                110                 115                 120

Val Pro Glu Pro Leu Cys Ala Cys Arg Ser Gln Ser Pro Leu Cys
                125                 130                 135

Gly Ser Asp Gly His Thr Tyr Ser Gln Ile Cys Arg Leu Gln Glu
                140                 145                 150

Ala Ala Arg Ala Arg Pro Asp Ala Asn Leu Thr Val Ala His Pro
                155                 160                 165

Gly Pro Cys Glu Ser Gly Pro Gln Ile Val Ser His Pro Tyr Asp
                170                 175                 180

Thr Trp Asn Val Thr Gly Gln Asp Val Ile Phe Gly Cys Glu Val
                185                 190                 195

Phe Ala Tyr Pro Met Ala Ser Ile Glu Trp Arg Lys Asp Gly Leu
                200                 205                 210

Asp Ile Gln Leu Pro Gly Asp Asp Pro His Ile Ser Val Gln Phe
                215                 220                 225

Arg Gly Gly Pro Gln Arg Phe Glu Val Thr Gly Trp Leu Gln Ile
                230                 235                 240

Gln Ala Val Arg Pro Ser Asp Glu Gly Thr Tyr Arg Cys Leu Gly
                245                 250                 255

Arg Asn Ala Leu Gly Gln Val Glu Ala Pro Ala Ser Leu Thr Val
                260                 265                 270

Leu Thr Pro Asp Gln Leu Asn Ser Thr Gly Ile Pro Gln Leu Arg
                275                 280                 285

Ser Leu Asn Leu Val Pro Glu Glu Glu Ala Glu Ser Glu Glu Asn
                290                 295                 300

Asp Asp Tyr Tyr

<210> 85
<211> 1285
<212> DNA
<213> Homo Sapien

<400> 85
caaagcggcg gctgtccgcg gtgccggctg ggggcggaga ggcggcggtg 50

ggctccctgg ggtgtgtgag cccggtgatg gagccgggcc cgacagccgc 100

gcagcggagg tgttcgttgc cgccgtggct gccgctgggg ctgctgctgt 150

ggtcggggct ggccctgggc gcgctcccct tcggcagcag tccgcacagg 200

gtcttccacg acctcctgtc ggagcagcag ttgctggagg tggaggactt 250

gtccctgtcc ctcctgcagg gtggagggct ggggcctctg tcgctgcccc 300

cggacctgcc ggatctggat cctgagtgcc gggagctcct gctggacttc 350

gccaacagca gcgcagagct gacagggtgt ctggtgcgca gcgcccggcc 400

cgtgcgcctc tgtcagacct gctaccccct cttccaacag gtcgtcagca 450

agatggacaa catcagccga gccgcgggga atacttcaga gagtcagagt 500

tgtgccagaa gtctcttaat ggcagataga atgcaaatag ttgtgattct 550

ctcagaattt tttaatacca catggcagga ggcaaattgt gcaaattgtt 600

taacaaacaa cagtgaagaa ttatcaaaca gcacagtata tttccttaat 650

ctatttaatc acaccctgac ctgctttgaa cataaccttc aggggaatgc 700

acatagtctt ttacagacaa aaaattattc agaagtatgc aaaaactgcc 750

gtgaagcata caaaactctg agtagtctgt acagtgaaat gcaaaaaatg 800

aatgaacttg agaataaggc tgaacctgga acacatttat gcattgatgt 850

ggaagatgca atgaacatca ctcgaaaact atggagtcga actttcaact 900

gttcagtccc ttgcagtgac acagtgcctg taattgctgt ttctgtgttc 950

attctctttc tacctgttgt cttctacctt agtagctttc ttcactcaga 1000

gcaaaagaaa cgcaaactca ttctgcccaa acgtctcaag tccagtacca 1050

gttttgcaaa tattcaggaa aattcaaact gagacctaca aaatggagaa 1100

ttgacatatc acgtgaatga atggtggaag acacaacttg gtttcagaaa 1150

gaagataaac tgtgatttga caagtcaagc tcttaagaaa tacaaggact 1200

tcagatccat ttttaaataa gaattttcga tttttctttc cttttccact 1250

tctttctaac agatttggat atttttaatt tccag 1285

<210> 86
<211> 334
<212> PRT
<213> Homo Sapien

<400> 86
Met Glu Pro Gly Pro Thr Ala Ala Gln Arg Arg Cys Ser Leu Pro
1               5                   10                  15

Pro Trp Leu Pro Leu Gly Leu Leu Leu Trp Ser Gly Leu Ala Leu
                20                  25                  30

Gly Ala Leu Pro Phe Gly Ser Ser Pro His Arg Val Phe His Asp
                35                  40                  45

Leu Leu Ser Glu Gln Gln Leu Leu Glu Val Glu Asp Leu Ser Leu
                50                  55                  60

Ser Leu Leu Gln Gly Gly Gly Leu Gly Pro Leu Ser Leu Pro Pro
                65                  70                  75

Asp Leu Pro Asp Leu Asp Pro Glu Cys Arg Glu Leu Leu Leu Asp
                80                  85                  90

Phe Ala Asn Ser Ser Ala Glu Leu Thr Gly Cys Leu Val Arg Ser
                95                  100                 105

Ala Arg Pro Val Arg Leu Cys Gln Thr Cys Tyr Pro Leu Phe Gln
                110                 115                 120

Gln Val Val Ser Lys Met Asp Asn Ile Ser Arg Ala Ala Gly Asn
                125                 130                 135

Thr Ser Glu Ser Gln Ser Cys Ala Arg Ser Leu Leu Met Ala Asp
                140                 145                 150

Arg Met Gln Ile Val Val Ile Leu Ser Glu Phe Phe Asn Thr Thr
                155                 160                 165

Trp Gln Glu Ala Asn Cys Ala Asn Cys Leu Thr Asn Asn Ser Glu
                170                 175                 180

Glu Leu Ser Asn Ser Thr Val Tyr Phe Leu Asn Leu Phe Asn His
                185                 190                 195

Thr Leu Thr Cys Phe Glu His Asn Leu Gln Gly Asn Ala His Ser
                200                 205                 210

Leu Leu Gln Thr Lys Asn Tyr Ser Glu Val Cys Lys Asn Cys Arg
                215                 220                 225

Glu Ala Tyr Lys Thr Leu Ser Ser Leu Tyr Ser Glu Met Gln Lys
                230                 235                 240

Met Asn Glu Leu Glu Asn Lys Ala Glu Pro Gly Thr His Leu Cys
                245                 250                 255

Ile Asp Val Glu Asp Ala Met Asn Ile Thr Arg Lys Leu Trp Ser
                260                 265                 270

Arg Thr Phe Asn Cys Ser Val Pro Cys Ser Asp Thr Val Pro Val
                275                 280                 285

Ile Ala Val Ser Val Phe Ile Leu Phe Leu Pro Val Val Phe Tyr
                290                 295                 300

Leu Ser Ser Phe Leu His Ser Glu Gln Lys Lys Arg Lys Leu Ile
                305                 310                 315

Leu Pro Lys Arg Leu Lys Ser Ser Thr Ser Phe Ala Asn Ile Gln
                320                 325                 330

Glu Asn Ser Asn

<210> 87
<211> 1754
<212> DNA
<213> Homo Sapien

<400> 87
atgctggtag ccggcttcct gctggcgctg ccgccgagct gggccgcggg 50

cgcccccagg gcgggcaggc gccccgcgcg gccgcggggc tgcgcggacc 100

ggccggagga gctactggag cagctgtacg ggcgcctggc ggccggcgtg 150

ctcagtgcct tccaccacac gctgcagctg gggccgcgtg agcaggcgcg 200

caacgcgagc tgcccggcag ggggcaggcc cggcgaccgc cgcttccggc 250

cgcccaccaa cctgcgcagc gtgtcgccct gggcctacag aatctcctac 300

gacccggcga ggtaccccag gtacctgcct gaagcctact gcctgtgccg 350

gggctgcctg accgggctgt tcggcgagga ggacgtgcgc ttccgcagcg 400

cccctgtcta catgcccacc gtcgtcctgc gccgcacccc cgcctgcgcc 450

ggcggccgtt ccgtctacac cgaggcctac gtcaccatcc ccgtgggctg 500

cacctgcgtc cccgagccgg agaaggacgc agacagcatc aactccagca 550

tcgacaaaca gggcgccaag ctcctgctgg gccccaacga cgcgcccgct 600

ggcccctgag gccggtcctg ccccgggagg tctccccggc ccgcatcccg 650

aggcgcccaa gctggagccg cctggagggc tcggtcggcg acctctgaag 700

agagtgcacc gagcaaacca agtgccggag caccagcgcc gcctttccat 750

ggagactcgt aagcagcttc atctgacacg ggcatccctg gcttgctttt 800

agctacaagc aagcagcgtg gctggaagct gatgggaaac gacccggcac 850

gggcatcctg tgtgcggccc gcatggaggg tttggaaaag ttcacggagg 900

ctccctgagg agcctctcag atcggctgct gcgggtgcag ggcgtgactc 950

accgctgggt gcttgccaaa gagataggga cgcatatgct ttttaaagca 1000

atctaaaaat aataataagt atagcgacta tatacctact tttaaaatca 1050

actgttttga atagaggcag agctatttta tattatcaaa tgagagctac 1100

tctgttacat ttcttaacat ataaacatcg ttttttactt cttctggtag 1150

aattttttaa agcataattg gaatccttgg ataaattttg tagctggtac 1200

actctggcct gggtctctga attcagcctg tcaccgatgg ctgactgatg 1250

aaatggacac gtctcatctg acccactctt ccttccactg aaggtcttca 1300

cgggcctcca ggtggaccaa agggatgcac aggcggctcg catgccccag 1350

ggccagctaa gagttccaaa gatctcagat ttggttttag tcatgaatac 1400

ataaacagtc tcaaactcgc acaatttttt cccccttttg aaagccactg 1450

gggccaattt gtggttaaga ggtggtgaga taagaagtgg aacgtgacat 1500

ctttgccagt tgtcagaaga atccaagcag gtattggctt agttgtaagg 1550

gctttaggat caggctgaat atgaggacaa agtgggccac gttagcatct 1600

gcagagatca atctggaggc ttctgtttct gcattctgcc acgagagcta 1650

ggtccttgat cttttcttta gattgaaagt ctgtctctga acacaattat 1700

ttgtaaaagt tagtagttct tttttaaatc attaaaagag gcttgctgaa 1750

ggat 1754

<210> 88
<211> 202
<212> PRT
<213> Homo Sapien

<400> 88

<210> 89
<211> 2601
<212> DNA
<213> Homo Sapien

<400> 89
ccggggcctc cggagaacgc tgtcccatga acgtgcgggg agcggccccc 50

ggcgtccgcg cgtccccgcg tccctggcaa ttcccgactt cccaacggct 100

tcccgctggc agccccgaag ccgcaccatg ttccgcctct ggttgctgct 150

ggccgggctc tgcggcctcc tggcgtcaag acccggtttt caaaattcac 200

ttctacagat cgtaattcca gagaaaatcc aaacaaatac aaatgacagt 250

tcagaaatag aatatgaaca aatatcctat attattccaa tagatgagaa 300

actgtacact gtgcacctta aacaaagata ttttttagca gataatttta 350

tgatctattt gtacaatcaa ggatctatga atacttattc ttcagatatt 400

cagactcaat gctactatca aggaaatatt gaaggatatc cagattccat 450

ggtcacactc agcacgtgct ctggactaag aggaatactg caatttgaaa 500

atgtttctta tggaattgag cctctggaat ctgcagttga atttcagcat 550

gttctttaca aattaaagaa tgaagacaat gatattgcaa tttttattga 600
cagaagcctg aaagaacaac caatggatga caacattttt ataagtgaaa 650
aatcagaacc agctgttcca gatttatttc ctctttatct agaaatgcat 700
attgtggtgg acaaaacttt gtatgattac tggggctctg atagcatgat 750
agtaacaaat aaagtcatcg aaattgttgg ccttgcaaat tcaatgttca 800
cccaatttaa agttactatt gtgctgtcat cattggagtt atggtcagat 850
gaaaataaga tttctacagt tggtgaggca gatgaattat tgcaaaaatt 900
tttagaatgg aaacaatctt atcttaacct aaggcctcat gatattgcat 950
atctactaat ttatatggat tatcctcgtt atttgggagc agtgtttcct 1000
ggaacaatgt gtattactcg ttattctgca ggagttgcat tgtaccccaa 1050
ggagataact ctggaggcat ttgcagttat tgtcacccag atgctggcac 1100
tcagtctggg aatatcatat gacgacccaa agaaatgtca atgttcagaa 1150
tccacctgta taatgaatcc agaagttgtg caatccaatg gtgtgaagac 1200
ttttagcagt tgcagtttga ggagctttca aaatttcatt tcaaatgtgg 1250
gtgtcaaatg tcttcagaat aagccacaaa tgcaaaaaaa atctccgaaa 1300
ccagtctgtg gcaatggcag attggaggga aatgaaatct gtgattgtgg 1350
tactgaggct caatgtggac ctgcaagctg ttgtgatttt cgaacttgtg 1400
tactgaaaga cggagcaaaa tgttataaag gactgtgctg caaagactgt 1450
caaattttac aatcaggcgt tgaatgtagg ccgaaagcac atcctgaatg 1500
tgacatcgct gaaaattgta atggaagctc accagaatgt ggtcctgaca 1550
taactttaat caatggactt tcatgcaaaa ataataagtt tatttgttat 1600
gacggagact gccatgatct cgatgcacgt tgtgagagtg tatttggaaa 1650
aggttcaaga aatgctccat ttgcctgcta tgaagaaata caatctcaat 1700
cagacagatt tgggaactgt ggtagggata gaaataacaa atatgtgttc 1750
tgtggatgga ggaatcttat atgtggaaga ttagtttgta cctaccctac 1800
tcgaaagcct ttccatcaag aaaatggtga tgtgatttat gctttcgtac 1850
gagattctgt atgcataact gtagactaca aattgcctcg aacagttcca 1900
gatccactgg ctgtcaaaaa tggctctcag tgtgatattg ggagggtttg 1950
tgtaaatcgt gaatgtgtag aatcaaggat aattaaggct tcagcacatg 2000
tttgttcaca acagtgttct ggacatggag tgtgtgattc cagaaacaag 2050
tgccattgtt cgccaggcta taagcctcca aactgccaaa tacgttccaa 2100
aggattttcc atatttcctg aggaagatat gggttcaatc atggaaagag 2150
catctgggaa gactgaaaac acctggcttc taggtttcct cattgctctt 2200
cctattctca ttgtaacaac cgcaatagtt ttggcaagga aacagttgaa 2250

aaagtggttc gccaaggaag aggaattccc aagtagcgaa tctaaatcgg 2300

aaggtagcac acagacatat gccagccaat ccagctcaga aggcagcact 2350

cagacatatg ccagccaaac cagatcagaa agcagcagtc aagctgatac 2400

tagcaaatcc aaatcagaag atagtgctga agcatatact agcagatcca 2450

aatcacagga cagtacccaa acacaaagca gtagtaacta gtgattcctt 2500

cagaaggcaa cggataacat cgagagtctc gctaagaaat gaaaattctg 2550

tctttccttc cgtggtcaca gctgaaagaa acaataaatt gagtgtggat 2600

c 2601

<210> 90
<211> 787
<212> PRT
<213> Homo Sapien

<400> 90
Met Phe Arg Leu Trp Leu Leu Leu Ala Gly Leu Cys Gly Leu Leu
1               5                   10                  15

Ala Ser Arg Pro Gly Phe Gln Asn Ser Leu Leu Gln Ile Val Ile
                20                  25                  30

Pro Glu Lys Ile Gln Thr Asn Thr Asn Asp Ser Ser Glu Ile Glu
                35                  40                  45

Tyr Glu Gln Ile Ser Tyr Ile Ile Pro Ile Asp Glu Lys Leu Tyr
                50                  55                  60

Thr Val His Leu Lys Gln Arg Tyr Phe Leu Ala Asp Asn Phe Met
                65                  70                  75

Ile Tyr Leu Tyr Asn Gln Gly Ser Met Asn Thr Tyr Ser Ser Asp
                80                  85                  90

Ile Gln Thr Gln Cys Tyr Tyr Gln Gly Asn Ile Glu Gly Tyr Pro
                95                  100                 105

Asp Ser Met Val Thr Leu Ser Thr Cys Ser Gly Leu Arg Gly Ile
                110                 115                 120

Leu Gln Phe Glu Asn Val Ser Tyr Gly Ile Glu Pro Leu Glu Ser
                125                 130                 135

Ala Val Glu Phe Gln His Val Leu Tyr Lys Leu Lys Asn Glu Asp
                140                 145                 150

Asn Asp Ile Ala Ile Phe Ile Asp Arg Ser Leu Lys Glu Gln Pro
                155                 160                 165

Met Asp Asp Asn Ile Phe Ile Ser Glu Lys Ser Glu Pro Ala Val
                170                 175                 180

Pro Asp Leu Phe Pro Leu Tyr Leu Glu Met His Ile Val Val Asp
                185                 190                 195

Lys Thr Leu Tyr Asp Tyr Trp Gly Ser Asp Ser Met Ile Val Thr
                200                 205                 210

Asn Lys Val Ile Glu Ile Val Gly Leu Ala Asn Ser Met Phe Thr
                215                 220                 225

Gln Phe Lys Val Thr Ile Val Leu Ser Ser Leu Glu Leu Trp Ser
                230                 235                 240

Asp Glu Asn Lys Ile Ser Thr Val Gly Glu Ala Asp Glu Leu Leu
                245                 250                 255

Gln Lys Phe Leu Glu Trp Lys Gln Ser Tyr Leu Asn Leu Arg Pro
                260                 265                 270

His Asp Ile Ala Tyr Leu Leu Ile Tyr Met Asp Tyr Pro Arg Tyr
                275                 280                 285

Leu Gly Ala Val Phe Pro Gly Thr Met Cys Ile Thr Arg Tyr Ser
                290                 295                 300

Ala Gly Val Ala Leu Tyr Pro Lys Glu Ile Thr Leu Glu Ala Phe
                305                 310                 315

Ala Val Ile Val Thr Gln Met Leu Ala Leu Ser Leu Gly Ile Ser
                320                 325                 330

Tyr Asp Asp Pro Lys Lys Cys Gln Cys Ser Glu Ser Thr Cys Ile
                335                 340                 345

Met Asn Pro Glu Val Val Gln Ser Asn Gly Val Lys Thr Phe Ser
                350                 355                 360

Ser Cys Ser Leu Arg Ser Phe Gln Asn Phe Ile Ser Asn Val Gly
                365                 370                 375

Val Lys Cys Leu Gln Asn Lys Pro Gln Met Gln Lys Lys Ser Pro
                380                 385                 390

Lys Pro Val Cys Gly Asn Gly Arg Leu Glu Gly Asn Glu Ile Cys
                395                 400                 405

Asp Cys Gly Thr Glu Ala Gln Cys Gly Pro Ala Ser Cys Cys Asp
                410                 415                 420

Phe Arg Thr Cys Val Leu Lys Asp Gly Ala Lys Cys Tyr Lys Gly
                425                 430                 435

Leu Cys Cys Lys Asp Cys Gln Ile Leu Gln Ser Gly Val Glu Cys
                440                 445                 450

Arg Pro Lys Ala His Pro Glu Cys Asp Ile Ala Glu Asn Cys Asn
                455                 460                 465

Gly Ser Ser Pro Glu Cys Gly Pro Asp Ile Thr Leu Ile Asn Gly
                470                 475                 480

Leu Ser Cys Lys Asn Asn Lys Phe Ile Cys Tyr Asp Gly Asp Cys
                485                 490                 495

His Asp Leu Asp Ala Arg Cys Glu Ser Val Phe Gly Lys Gly Ser
                500                 505                 510

Arg Asn Ala Pro Phe Ala Cys Tyr Glu Glu Ile Gln Ser Gln Ser
                515                 520                 525

Asp Arg Phe Gly Asn Cys Gly Arg Asp Arg Asn Asn Lys Tyr Val
                530                 535                 540

Phe Cys Gly Trp Arg Asn Leu Ile Cys Gly Arg Leu Val Cys Thr
                545                 550                 555

Tyr Pro Thr Arg Lys Pro Phe His Gln Glu Asn Gly Asp Val Ile
                560                 565                 570

Tyr Ala Phe Val Arg Asp Ser Val Cys Ile Thr Val Asp Tyr Lys
                575                 580                 585

Leu Pro Arg Thr Val Pro Asp Pro Leu Ala Val Lys Asn Gly Ser
                590                 595                 600

Gln Cys Asp Ile Gly Arg Val Cys Val Asn Arg Glu Cys Val Glu
                605                 610                 615

Ser Arg Ile Ile Lys Ala Ser Ala His Val Cys Ser Gln Gln Cys
                620                 625                 630

Ser Gly His Gly Val Cys Asp Ser Arg Asn Lys Cys His Cys Ser
                635                 640                 645

Pro Gly Tyr Lys Pro Pro Asn Cys Gln Ile Arg Ser Lys Gly Phe
                650                 655                 660

Ser Ile Phe Pro Glu Glu Asp Met Gly Ser Ile Met Glu Arg Ala
                665                 670                 675

Ser Gly Lys Thr Glu Asn Thr Trp Leu Leu Gly Phe Leu Ile Ala
                680                 685                 690

Leu Pro Ile Leu Ile Val Thr Thr Ala Ile Val Leu Ala Arg Lys
                695                 700                 705

Gln Leu Lys Lys Trp Phe Ala Lys Glu Glu Glu Phe Pro Ser Ser
                710                 715                 720

Glu Ser Lys Ser Glu Gly Ser Thr Gln Thr Tyr Ala Ser Gln Ser
                725                 730                 735

Ser Ser Glu Gly Ser Thr Gln Thr Tyr Ala Ser Gln Thr Arg Ser
                740                 745                 750

Glu Ser Ser Ser Gln Ala Asp Thr Ser Lys Ser Lys Ser Glu Asp
                755                 760                 765

Ser Ala Glu Ala Tyr Thr Ser Arg Ser Lys Ser Gln Asp Ser Thr
                770                 775                 780

Gln Thr Gln Ser Ser Ser Asn
                785

<210> 91
<211> 2445
<212> DNA
<213> Homo Sapien

<220>
<221> unsure
<222> 2424
<223> unknown base

<400> 91
caccagacag cactccagca ctctgtttgg ggggcattcg aaacagcaaa 50

atcactcata aaaggcaaaa aattgcaaaa aaaaatagta ataaccagca 100

tggcactaaa tagaccatga aaagacatgt gtgtgcagta tgaaaattga 150

gacaggaagg cagagtgtca gcttgttcca cctcagctgg gaatgtgcat 200

caggcaactc aagtttttca ccacggcatg tgtctgtgaa tgtccgcaaa 250

acattctctc tccccagcct tcatgtgtta acctggggat gatgtggacc 300

tgggcactgt ggatgctccc ttcactctgc aaattcagcc tggcagctct 350
gccagctaag cctgagaaca tttcctgtgt ctactactat aggaaaaatt 400
taacctgcac ttggagtcca ggaaaggaaa ccagttatac ccagtacaca 450
gttaagagaa cttacgcttt tggagaaaaa catgataatt gtacaaccaa 500
tagttctaca agtgaaaatc gtgcttcgtg ctcttttttc cttccaagaa 550
taacgatccc agataattat accattgagg tggaagctga aaatggagat 600
ggtgtaatta aatctcatat gacatactgg agattagaga acatagcgaa 650
aactgaacca cctaagattt tccgtgtgaa accagttttg ggcatcaaac 700
gaatgattca aattgaatgg ataaagcctg agttggcgcc tgtttcatct 750
gatttaaaat acacacttcg attcaggaca gtcaacagta ccagctggat 800
ggaagtcaac ttcgctaaga accgtaagga taaaaaccaa acgtacaacc 850
tcacggggct gcagcctttt acagaatatg tcatagctct gcgatgtgcg 900
gtcaaggagt caaagttctg gagtgactgg agccaagaaa aaatgggaat 950
gactgaggaa gaagctccat gtggcctgga actgtggaga gtcctgaaac 1000
cagctgaggc ggatggaaga aggccagtgc ggttgttatg gaagaaggca 1050
agaggagccc cagtcctaga gaaaacactt ggctacaaca tatggtacta 1100
tccagaaagc aacactaacc tcacagaaac aatgaacact actaaccagc 1150
agcttgaact gcatctggga ggcgagagct tttgggtgtc tatgatttct 1200
tataattctc ttgggaagtc tccagtggcc accctgagga ttccagctat 1250
tcaagaaaaa tcatttcagt gcattgaggt catgcaggcc tgcgttgctg 1300
aggaccagct agtggtgaag tggcaaagct ctgctctaga cgtgaacact 1350
tggatgattg aatggtttcc ggatgtggac tcagagccca ccaccctttc 1400
ctgggaatct gtgtctcagg ccacgaactg gacgatccag caagataaat 1450
taaaaccttt ctggtgctat aacatctctg tgtatccaat gttgcatgac 1500
aaagttggcg agccatattc catccaggct tatgccaaag aaggcgttcc 1550
atcagaaggt cctgagacca aggtggagaa cattggcgtg aagacggtca 1600
cgatcacatg gaaagagatt cccaagagtg agagaaaggg tatcatctgc 1650
aactacacca tcttttacca agctgaaggt ggaaaaggat tctgtaagca 1700
cgcccatagc gaagtggaaa aaaaccccaa gccccagata gatgctatgg 1750
atagacctgt tgtaggcatg gctcccccat ctcattgtga cttgcaacct 1800
ggcatgaatc acttagcttc tttaaatctc tctgaaaatg gggccaagag 1850
cacccacctt ttggggtttt gggggttaaa tgagagtgaa gtgacagtac 1900
ctgagaggag agtcctgagg aaatggaagg agttgttata atttgtcctg 1950
gttaggccct gaattgacct cccgggagct ccccgaccat cattcccagg 2000

aatggcgtgc ctggcttaaa gagtgaggag gaacagaccc tgtcaccatg 2050

acttctactg cccctgccaa atcatgcttt tgtttttcag tccaccttat 2100

ctcctgacat cttaaatact gggcaaggct tggattcttg cttaggctaa 2150

ataatttttt cttatggtaa aatacacgta aaatattttt ccagtttaaa 2200

catttgaaag tgtacaattt agtggcatta gaagcattca caatattgtg 2250

caaccatcac cactatttcc agaactcttc tatttctgcc caaatagaag 2300

ccctataccc attcattagt cactccccat tcctctcctc ccacagcccc 2350

tggcaactac caaactgctt tgtgtctcta tggattgcct attttggata 2400

tttcatatac atagaatcat aaantaaaaa aaaaaaaaaa aaaaa 2445

<210> 92
<211> 582
<212> PRT
<213> Homo Sapien

<400> 92
Met Cys Ile Arg Gln Leu Lys Phe Phe Thr Thr Ala Cys Val Cys
1               5                   10                  15

Glu Cys Pro Gln Asn Ile Leu Ser Pro Gln Pro Ser Cys Val Asn
                20                  25                  30

Leu Gly Met Met Trp Thr Trp Ala Leu Trp Met Leu Pro Ser Leu
                35                  40                  45

Cys Lys Phe Ser Leu Ala Ala Leu Pro Ala Lys Pro Glu Asn Ile
                50                  55                  60

Ser Cys Val Tyr Tyr Tyr Arg Lys Asn Leu Thr Cys Thr Trp Ser
                65                  70                  75

Pro Gly Lys Glu Thr Ser Tyr Thr Gln Tyr Thr Val Lys Arg Thr
                80                  85                  90

Tyr Ala Phe Gly Glu Lys His Asp Asn Cys Thr Thr Asn Ser Ser
                95                  100                 105

Thr Ser Glu Asn Arg Ala Ser Cys Ser Phe Phe Leu Pro Arg Ile
                110                 115                 120

Thr Ile Pro Asp Asn Tyr Thr Ile Glu Val Glu Ala Glu Asn Gly
                125                 130                 135

Asp Gly Val Ile Lys Ser His Met Thr Tyr Trp Arg Leu Glu Asn
                140                 145                 150

Ile Ala Lys Thr Glu Pro Pro Lys Ile Phe Arg Val Lys Pro Val
                155                 160                 165

Leu Gly Ile Lys Arg Met Ile Gln Ile Glu Trp Ile Lys Pro Glu
                170                 175                 180

Leu Ala Pro Val Ser Ser Asp Leu Lys Tyr Thr Leu Arg Phe Arg
                185                 190                 195

Thr Val Asn Ser Thr Ser Trp Met Glu Val Asn Phe Ala Lys Asn
                200                 205                 210

Arg Lys Asp Lys Asn Gln Thr Tyr Asn Leu Thr Gly Leu Gln Pro

                215                 220                 225

Phe Thr Glu Tyr Val Ile Ala Leu Arg Cys Ala Val Lys Glu Ser
                230                 235                 240

Lys Phe Trp Ser Asp Trp Ser Gln Glu Lys Met Gly Met Thr Glu
                245                 250                 255

Glu Glu Ala Pro Cys Gly Leu Glu Leu Trp Arg Val Leu Lys Pro
                260                 265                 270

Ala Glu Ala Asp Gly Arg Arg Pro Val Arg Leu Leu Trp Lys Lys
                275                 280                 285

Ala Arg Gly Ala Pro Val Leu Glu Lys Thr Leu Gly Tyr Asn Ile
                290                 295                 300

Trp Tyr Tyr Pro Glu Ser Asn Thr Asn Leu Thr Glu Thr Met Asn
                305                 310                 315

Thr Thr Asn Gln Gln Leu Glu Leu His Leu Gly Gly Glu Ser Phe
                320                 325                 330

Trp Val Ser Met Ile Ser Tyr Asn Ser Leu Gly Lys Ser Pro Val
                335                 340                 345

Ala Thr Leu Arg Ile Pro Ala Ile Gln Glu Lys Ser Phe Gln Cys
                350                 355                 360

Ile Glu Val Met Gln Ala Cys Val Ala Glu Asp Gln Leu Val Val
                365                 370                 375

Lys Trp Gln Ser Ser Ala Leu Asp Val Asn Thr Trp Met Ile Glu
                380                 385                 390

Trp Phe Pro Asp Val Asp Ser Glu Pro Thr Thr Leu Ser Trp Glu
                395                 400                 405

Ser Val Ser Gln Ala Thr Asn Trp Thr Ile Gln Gln Asp Lys Leu
                410                 415                 420

Lys Pro Phe Trp Cys Tyr Asn Ile Ser Val Tyr Pro Met Leu His
                425                 430                 435

Asp Lys Val Gly Glu Pro Tyr Ser Ile Gln Ala Tyr Ala Lys Glu
                440                 445                 450

Gly Val Pro Ser Glu Gly Pro Glu Thr Lys Val Glu Asn Ile Gly
                455                 460                 465

Val Lys Thr Val Thr Ile Thr Trp Lys Glu Ile Pro Lys Ser Glu
                470                 475                 480

Arg Lys Gly Ile Ile Cys Asn Tyr Thr Ile Phe Tyr Gln Ala Glu
                485                 490                 495

Gly Gly Lys Gly Phe Cys Lys His Ala His Ser Glu Val Glu Lys
                500                 505                 510

Asn Pro Lys Pro Gln Ile Asp Ala Met Asp Arg Pro Val Val Gly
                515                 520                 525

Met Ala Pro Pro Ser His Cys Asp Leu Gln Pro Gly Met Asn His
                530                 535                 540

Leu Ala Ser Leu Asn Leu Ser Glu Asn Gly Ala Lys Ser Thr His
                545                 550                 555

Leu Leu Gly Phe Trp Gly Leu Asn Glu Ser Glu Val Thr Val Pro
                560                 565                 570

Glu Arg Arg Val Leu Arg Lys Trp Lys Glu Leu Leu
                575                 580

<210> 93
<211> 662
<212> DNA
<213> Homo Sapien

<400> 93
attctcctag agcatctttg gaagcatgag gccacgatgc tgcatcttgg 50

ctcttgtctg ctggataaca gtcttcctcc tccagtgttc aaaaggaact 100

acagacgctc ctgttggctc aggactgtgg ctgtgccagc cgacacccag 150

gtgtgggaac aagatctaca acccttcaga gcagtgctgt tatgatgatg 200

ccatcttatc cttaaaggag acccgccgct gtggctccac ctgcaccttc 250

tggccctgct ttgagctctg ctgtcccgag tcttttggcc cccagcagaa 300

gtttcttgtg aagttgaggg ttctgggtat gaagtctcag tgtcacttat 350

ctcccatctc ccggagctgt accaggaaca ggaggcacgt cctgtaccca 400

taaaaacccc aggctccact ggcagacggc agacaagggg agaagagacg 450

aagcagctgg acatcggaga ctacagttga acttcggaga gaagcaactt 500

gacttcagag ggatggctca atgacatagc tttggagagg agcccagctg 550

gggatggcca gacttcaggg gaagaatgcc ttcctgcttc atcccctttc 600

cagctcccct tcccgctgag agccactttc atcggcaata aaatccccca 650

catttaccat ct 662

<210> 94
<211> 125
<212> PRT
<213> Homo Sapien

<400> 94
Met Arg Pro Arg Cys Cys Ile Leu Ala Leu Val Cys Trp Ile Thr
  1               5                  10                  15

Val Phe Leu Leu Gln Cys Ser Lys Gly Thr Thr Asp Ala Pro Val
                 20                  25                  30

Gly Ser Gly Leu Trp Leu Cys Gln Pro Thr Pro Arg Cys Gly Asn
                 35                  40                  45

Lys Ile Tyr Asn Pro Ser Glu Gln Cys Cys Tyr Asp Asp Ala Ile
                 50                  55                  60

Leu Ser Leu Lys Glu Thr Arg Arg Cys Gly Ser Thr Cys Thr Phe
                 65                  70                  75

Trp Pro Cys Phe Glu Leu Cys Cys Pro Glu Ser Phe Gly Pro Gln
                 80                  85                  90

Gln Lys Phe Leu Val Lys Leu Arg Val Leu Gly Met Lys Ser Gln
                 95                 100                 105

Cys His Leu Ser Pro Ile Ser Arg Ser Cys Thr Arg Asn Arg Arg

110                 115                 120

His Val Leu Tyr Pro
                125

<210> 95
<211> 471
<212> DNA
<213> Homo Sapien

<400> 95
gcatttttgt ctgtgctccc tgatcttcag gtcaccacca tgaagttctt 50

agcagtcctg gtactcttgg gagtttccat ctttctggtc tctgcccaga 100

atccgacaac agctgctcca gctgacacgt atccagctac tggtcctgct 150

gatgatgaag cccctgatgc tgaaaccact gctgctgcaa ccactgcgac 200

cactgctgct cctaccactg caaccaccgc tgcttctacc actgctcgta 250

aagacattcc agttttaccc aaatgggttg gggatctccc gaatggtaga 300

gtgtgtccct gagatggaat cagcttgagt cttctgcaat tggtcacaac 350

tattcatgct tcctgtgatt tcatccaact acttaccttg cctacgatat 400

cccctttatc tctaatcagt ttattttctt tcaaataaaa aataactatg 450

agcaacataa aaaaaaaaaa a 471

<210> 96
<211> 90
<212> PRT
<213> Homo Sapien

<400> 96
Met Lys Phe Leu Ala Val Leu Val Leu Leu Gly Val Ser Ile Phe
  1               5                  10                  15

Leu Val Ser Ala Gln Asn Pro Thr Thr Ala Ala Pro Ala Asp Thr
                 20                  25                  30

Tyr Pro Ala Thr Gly Pro Ala Asp Asp Glu Ala Pro Asp Ala Glu
                 35                  40                  45

Thr Thr Ala Ala Ala Thr Thr Ala Thr Thr Ala Ala Pro Thr Thr
                 50                  55                  60

Ala Thr Thr Ala Ala Ser Thr Thr Ala Arg Lys Asp Ile Pro Val
                 65                  70                  75

Leu Pro Lys Trp Val Gly Asp Leu Pro Asn Gly Arg Val Cys Pro
                 80                  85                  90

<210> 97
<211> 907
<212> DNA
<213> Homo Sapien

<400> 97
ggactctgaa ggtcccaagc agctgctgag gcccccaagg aagtggttcc 50

aaccttggac ccctaggggt ctggatttgc tggttaacaa gataacctga 100

gggcaggacc ccatagggga atgctacctc ctgcccttcc acctgccctg 150

gtgttcacgg tggcctggtc cctccttgcc gagagagtgt cctgggtcag 200

ggacgcagag gacgctcaca gactccagcc ctttgttacc gagaggacac 250

ttggcaaggt ccagcgatgg tccggagtcc acacacagac tggcggcagg 300

gcaggagggg gacagttctg ttgtgcttgg ttggacagta agagggtctt 350

ggccagtcca gggtgggggg cggcaaactc cataaagaac cagagggtct 400

gggccccggc cacagagtca tctgcccagc tcctctgctg ctggccagtg 450

ggagtggcac gaggtggggc tttgtgccag taaaaccaca ggctggattt 500

gcctgcgggc catggtccct gtctagggca gcaattctca accttcttgc 550

tctcaggacc ccaaagagct ttcattgtat ctattgattt ttaccacatt 600

agcaattaaa actgagaaat gggccgggca cggtggctca cgcctgtaat 650

cccagcactt tgggaggccg aggcgggtgg atcacctgag atcaggagtt 700

caagaccagc ctggccaaca tggtgaaacc ttgtctacta aaaatacaaa 750

aaattagcca ggcacagtgg tgtgcactgg tagtcccagt tactcgggag 800

gctgaggcag gaaaatcgct tgaacccagg aggcggacgt tgcggtgagc 850

cgagatcgcg ccgctgattc cagcctgggc gacaagagtg agactccatc 900

tcacaca 907

<210> 98
<211> 120
<212> PRT
<213> Homo Sapien

<400> 98
Met Leu Pro Pro Ala Leu Pro Pro Ala Leu Val Phe Thr Val Ala
1               5                   10                  15

Trp Ser Leu Leu Ala Glu Arg Val Ser Trp Val Arg Asp Ala Glu
                20                  25                  30

Asp Ala His Arg Leu Gln Pro Phe Val Thr Glu Arg Thr Leu Gly
                35                  40                  45

Lys Val Gln Arg Trp Ser Gly Val His Thr Gln Thr Gly Gly Arg
                50                  55                  60

Ala Gly Gly Gly Gln Phe Cys Cys Ala Trp Leu Asp Ser Lys Arg
                65                  70                  75

Val Leu Ala Ser Pro Gly Trp Gly Ala Ala Asn Ser Ile Lys Asn
                80                  85                  90

Gln Arg Val Trp Ala Pro Ala Thr Glu Ser Ser Ala Gln Leu Leu
                95                  100                 105

Cys Cys Trp Pro Val Gly Val Ala Arg Gly Gly Ala Leu Cys Gln
                110                 115                 120

<210> 99
<211> 1073
<212> DNA
<213> Homo Sapien

<400> 99
aatttttcac cagagtaaac ttgagaaacc aactggacct tgagtattgt 50

acattttgcc tcgtggaccc aaaggtagca atctgaaaca tgaggagtac 100

gattctactg ttttgtcttc taggatcaac tcggtcatta ccacagctca 150

aacctgcttt gggactccct cccacaaaac tggctccgga tcagggaaca 200

ctaccaaacc aacagcagtc aaatcaggtc tttccttctt taagtctgat 250

accattaaca cagatgctca cactggggcc agatctgcat ctgttaaatc 300

ctgctgcagg aatgacacct ggtacccaga cccacccatt gaccctggga 350

gggttgaatg tacaacagca actgcaccca catgtgttac caatttttgt 400

cacacaactt ggagcccagg gcactatcct aagctcagag gaattgccac 450

aaatcttcac gagcctcatc atccattcct tgttcccggg aggcatcctg 500

cccaccagtc aggcaggggc taatccagat gtccaggatg gaagccttcc 550

agcaggagga gcaggtgtaa atcctgccac ccagggaacc ccagcaggcc 600

gcctcccaac tcccagtggc acagatgacg actttgcagt gaccacccct 650

gcaggcatcc aaaggagcac acatgccatc gaggaagcca ccacagaatc 700

agcaaatgga attcagtaag ctgtttcaaa ttttttcaac taagctgcct 750

cgaatttggt gatacatgtg aatctttatc attgattata ttatggaata 800

gattgagaca cattggatag tcttagaaga aattaattct taatttacct 850

gaaaatattc ttgaaatttc agaaaatatg ttctatgtag agaatcccaa 900

cttttaaaaa caataattca atggataaat ctgtctttga aatataacat 950

tatgctgcct ggatgatatg catattaaaa catatttgga aaactggaaa 1000

aaaaaaaaaa aaaaaaaaaa aaaaaaaaaa aaaaaaaaaa aaaaaaaaaa 1050

aaaaaaaaaa aaaaaaaaaa aaa 1073

<210> 100
<211> 209
<212> PRT
<213> Homo Sapien

<400> 100
Met Arg Ser Thr Ile Leu Leu Phe Cys Leu Leu Gly Ser Thr Arg
1               5                   10                  15

Ser Leu Pro Gln Leu Lys Pro Ala Leu Gly Leu Pro Pro Thr Lys
                20                  25                  30

Leu Ala Pro Asp Gln Gly Thr Leu Pro Asn Gln Gln Gln Ser Asn
                35                  40                  45

Gln Val Phe Pro Ser Leu Ser Leu Ile Pro Leu Thr Gln Met Leu
                50                  55                  60

Thr Leu Gly Pro Asp Leu His Leu Leu Asn Pro Ala Ala Gly Met
                65                  70                  75

Thr Pro Gly Thr Gln Thr His Pro Leu Thr Leu Gly Gly Leu Asn
                80                  85                  90

Val Gln Gln Gln Leu His Pro His Val Leu Pro Ile Phe Val Thr

95                  100                 105

Gln Leu Gly Ala Gln Gly Thr Ile Leu Ser Ser Glu Glu Leu Pro
                110                 115                 120

Gln Ile Phe Thr Ser Leu Ile Ile His Ser Leu Phe Pro Gly Gly
                125                 130                 135

Ile Leu Pro Thr Ser Gln Ala Gly Ala Asn Pro Asp Val Gln Asp
                140                 145                 150

Gly Ser Leu Pro Ala Gly Gly Ala Gly Val Asn Pro Ala Thr Gln
                155                 160                 165

Gly Thr Pro Ala Gly Arg Leu Pro Thr Pro Ser Gly Thr Asp Asp
                170                 175                 180

Asp Phe Ala Val Thr Thr Pro Ala Gly Ile Gln Arg Ser Thr His
                185                 190                 195

Ala Ile Glu Glu Ala Thr Thr Glu Ser Ala Asn Gly Ile Gln
                200                 205

<210> 101
<211> 1514
<212> DNA
<213> Homo Sapien

<400> 101
ggggtctccc tcagggccgg gaggcacagc ggtccctgct tgctgaaggg 50

ctggatgtac gcatccgcag gttcccgcgg acttgggggc gcccgctgag 100

ccccggcgcc cgcagaagac ttgtgtttgc ctcctgcagc ctcaacccgg 150

agggcagcga gggcctacca ccatgatcac tggtgtgttc agcatgcgct 200

tgtggacccc agtgggcgtc ctgacctcgc tggcgtactg cctgcaccag 250

cggcgggtgg ccctggccga gctgcaggag gccgatggcc agtgtccggt 300

cgaccgcagc ctgctgaagt tgaaaatggt gcaggtcgtg tttcgacacg 350

gggctcggag tcctctcaag ccgctcccgc tggaggagca ggtagagtgg 400

aacccccagc tattagaggt cccaccccaa actcagtttg attacacagt 450

caccaatcta gctggtggtc cgaaaccata ttctccttac gactctcaat 500

accatgagac caccctgaag gggggcatgt ttgctgggca gctgaccaag 550

gtgggcatgc agcaaatgtt tgccttggga gagagactga ggaagaacta 600

tgtggaagac attccctttc tttcaccaac cttcaaccca caggaggtct 650

ttattcgttc cactaacatt tttcggaatc tggagtccac ccgttgtttg 700

ctggctgggc ttttccagtg tcagaaagaa ggacccatca tcatccacac 750

tgatgaagca gattcagaag tcttgtatcc caactaccaa agctgctgga 800

gcctgaggca gagaaccaga ggccggaggc agactgcctc tttacagcca 850

ggaatctcag aggatttgaa aaaggtgaag gacaggatgg gcattgacag 900

tagtgataaa gtggacttct tcatcctcct ggacaacgtg gctgccgagc 950

aggcacacaa cctcccaagc tgccccatgc tgaagagatt tgcacggatg 1000

atcgaacaga gagctgtgga cacatccttg tacatactgc ccaaggaaga 1050

cagggaaagt cttcagatgg cagtaggccc attcctccac atcctagaga 1100

gcaacctgct gaaagccatg gactctgcca ctgcccccga caagatcaga 1150

aagctgtatc tctatgcggc tcatgatgtg accttcatac cgctcttaat 1200

gaccctgggg atttttgacc acaaatggcc accgtttgct gttgacctga 1250

ccatggaact ttaccagcac ctggaatcta aggagtggtt tgtgcagctc 1300

tattaccacg ggaaggagca ggtgccgaga ggttgccctg atgggctctg 1350

cccgctggac atgttcttga atgccatgtc agtttatacc ttaagcccag 1400

aaaaatacca tgcactctgc tctcaaactc aggtgatgga agttggaaat 1450

gaagagtaac tgatttataa aagcaggatg tgttgatttt aaaataaagt 1500

gcctttatac aatg 1514

<210> 102
<211> 428
<212> PRT
<213> Homo Sapien

<400> 102

<210> 103
<211> 2555
<212> DNA
<213> Homo Sapien

<400> 103
ggggcgggtg gacgcggact cgaacgcagt tgcttcggga cccaggaccc 50

cctcgggccc gacccgccag gaaagactga ggccgcggcc tgccccgccc 100

ggctccctgc gccgccgccg cctcccggga cagaagatgt gctccagggt 150

ccctctgctg ctgccgctgc tcctgctact ggccctgggg cctggggtgc 200

agggctgccc atccggctgc cagtgcagcc agccacagac agtcttctgc 250

actgcccgcc aggggaccac ggtgccccga gacgtgccac ccgacacggt 300

ggggctgtac gtctttgaga acggcatcac catgctcgac gcaagcagct 350

ttgccggcct gccgggcctg cagctcctgg acctgtcaca gaaccagatc 400
gccagcctgc gcctgccccg cctgctgctg ctggacctca gccacaacag 450
cctcctggcc ctggagcccg gcatcctgga cactgccaac gtggaggcgc 500
tgcggctggc tggtctgggg ctgcagcagc tggacgaggg gctcttcagc 550
cgcttgcgca acctccacga cctggatgtg tccgacaacc agctggagcg 600
agtgccacct gtgatccgag gcctccgggg cctgacgcgc ctgcggctgg 650
ccggcaacac ccgcattgcc cagctgcggc ccgaggacct ggccggcctg 700
gctgccctgc aggagctgga tgtgagcaac ctaagcctgc aggccctgcc 750
tggcgacctc tcgggcctct tcccccgcct gcggctgctg gcagctgccc 800
gcaacccctt caactgcgtg tgccccctga gctggtttgg cccctgggtg 850
cgcgagagcc acgtcacact ggccagccct gaggagacgc gctgccactt 900
cccgcccaag aacgctggcc ggctgctcct ggagcttgac tacgccgact 950
ttggctgccc agccaccacc accacagcca cagtgcccac cacgaggccc 1000
gtggtgcggg agcccacagc cttgtcttct agcttggctc ctacctggct 1050
tagccccaca gcgccggcca ctgaggcccc cagcccgccc tccactgccc 1100
caccgactgt agggcctgtc ccccagcccc aggactgccc accgtccacc 1150
tgcctcaatg ggggcacatg ccacctgggg acacggcacc acctggcgtg 1200
cttgtgcccc gaaggcttca cgggcctgta ctgtgagagc cagatggggc 1250
aggggacacg gcccagccct acaccagtca cgccgaggcc accacggtcc 1300
ctgaccctgg gcatcgagcc ggtgagcccc acctccctgc gcgtggggct 1350
gcagcgctac ctccagggga gctccgtgca gctcaggagc ctccgtctca 1400
cctatcgcaa cctatcgggc cctgataagc ggctggtgac gctgcgactg 1450
cctgcctcgc tcgctgagta cacggtcacc cagctgcggc ccaacgccac 1500
ttactccgtc tgtgtcatgc ctttggggcc cgggcgggtg ccggagggcg 1550
aggaggcctg cggggaggcc catacacccc cagccgtcca ctccaaccac 1600
gccccagtca cccaggcccg cgagggcaac ctgccgctcc tcattgcgcc 1650
cgccctggcc gcggtgctcc tggccgcgct ggctgcggtg ggggcagcct 1700
actgtgtgcg gcgggggcgg gccatggcag cagcggctca ggacaaaggg 1750
caggtggggc caggggctgg gcccctggaa ctggagggag tgaaggtccc 1800
cttggagcca ggcccgaagg caacagaggg cggtggagag gccctgccca 1850
gcgggtctga gtgtgaggtg ccactcatgg gcttcccagg gcctggcctc 1900
cagtcacccc tccacgcaaa gccctacatc taagccagag agagacaggg 1950
cagctggggc cgggctctca gccagtgaga tggccagccc cctcctgctg 2000
ccacaccacg taagttctca gtcccaacct cggggatgtg tgcagacagg 2050

gctgtgtgac cacagctggg ccctgttccc tctggacctc ggtctcctca 2100

tctgtgagat gctgtggccc agctgacgag ccctaacgtc cccagaaccg 2150

agtgcctatg aggacagtgt ccgccctgcc ctccgcaacg tgcagtccct 2200

gggcacggcg ggccctgcca tgtgctggta acgcatgcct gggccctgct 2250

gggctctccc actccaggcg gaccctgggg gccagtgaag gaagctcccg 2300

gaaagagcag agggagagcg ggtaggcggc tgtgtgactc tagtcttggc 2350

cccaggaagc gaaggaacaa aagaaactgg aaaggaagat gctttaggaa 2400

catgttttgc ttttttaaaa tatatatata tttataagag atcctttccc 2450

atttattctg ggaagatgtt tttcaaactc agagacaagg actttggttt 2500

ttgtaagaca aacgatgata tgaaggcctt ttgtaagaaa aaataaaaaa 2550

aaaaa 2555

<210> 104
<211> 598
<212> PRT
<213> Homo Sapien

<400> 104

<210> 105
<211> 647
<212> DNA
<213> Homo Sapien

<400> 105
cccacgcgtc cgaaggcaga caaaggttca tttgtaaaga agctccttcc 50

agcacctcct ctcttctcct tttgcccaaa ctcacccagt gagtgtgagc 100

atttaagaag catcctctgc caagaccaaa aggaaagaag aaaaagggcc 150

aaaagccaaa atgaaactga tggtacttgt tttcaccatt gggctaactt 200

tgctgctagg agttcaagcc atgcctgcaa atcgcctctc ttgctacaga 250

aagatactaa aagatcacaa ctgtcacaac cttccggaag gagtagctga 300

cctgacacag attgatgtca atgtccagga tcatttctgg gatgggaagg 350

gatgtgagat gatctgttac tgcaacttca gcgaattgct ctgctgccca 400

aaagacgttt tctttggacc aaagatctct ttcgtgattc cttgcaacaa 450

tcaatgagaa tcttcatgta ttctggagaa caccattcct gatttcccac 500

aaactgcact acatcagtat aactgcattt ctagtttcta tatagtgcaa 550

tagagcatag attctataaa ttcttacttg tctaagacaa gtaaatctgt 600

gttaaacaag tagtaataaa agttaattca atctaaaaaa aaaaaaa 647

<210> 106
<211> 98
<212> PRT
<213> Homo Sapien

<400> 106
Met Lys Leu Met Val Leu Val Phe Thr Ile Gly Leu Thr Leu Leu
  1               5                  10                  15

Leu Gly Val Gln Ala Met Pro Ala Asn Arg Leu Ser Cys Tyr Arg
                 20                  25                  30

Lys Ile Leu Lys Asp His Asn Cys His Asn Leu Pro Glu Gly Val
                 35                  40                  45

Ala Asp Leu Thr Gln Ile Asp Val Asn Val Gln Asp His Phe Trp
                 50                  55                  60

Asp Gly Lys Gly Cys Glu Met Ile Cys Tyr Cys Asn Phe Ser Glu
                 65                  70                  75

Leu Leu Cys Cys Pro Lys Asp Val Phe Phe Gly Pro Lys Ile Ser
                 80                  85                  90

Phe Val Ile Pro Cys Asn Asn Gln
                95

<210> 107
<211> 972
<212> DNA
<213> Homo Sapien

<400> 107
agtgactgca gccttcctag atcccctcca ctcggtttct ctctttgcag 50

gagcaccggc agcaccagtg tgtgagggga gcaggcagcg gtcctagcca 100

gttccttgat cctgccagac cacccagccc ccggcacaga gctgctccac 150

aggcaccatg aggatcatgc tgctattcac agccatcctg gccttcagcc 200

tagctcagag ctttggggct gtctgtaagg agccacagga ggaggtggtt 250

cctggcgggg gccgcagcaa gagggatcca gatctctacc agctgctcca 300

gagactcttc aaaagccact catctctgga gggattgctc aaagccctga 350

gccaggctag cacagatcct aaggaatcaa catctcccga gaaacgtgac 400

atgcatgact tctttgtggg acttatgggc aagaggagcg tccagccaga 450

gggaaagaca ggacctttct taccttcagt gagggttcct cggccccttc 500

atcccaatca gcttggatcc acaggaaagt cttccctggg aacagaggag 550

cagagacctt tataagactc tcctacggat gtgaatcaag agaacgtccc 600

cagctttggc atcctcaagt atcccccgag agcagaatag gtactccact 650

tccggactcc tggactgcat taggaagacc tctttccctg tcccaatccc 700

caggtgcgca cgctcctgtt accctttctc ttccctgttc ttgtaacatt 750

cttgtgcttt gactccttct ccatcttttc tacctgaccc tggtgtggaa 800

actgcatagt gaatatcccc aaccccaatg ggcattgact gtagaatacc 850

ctagagttcc tgtagtgtcc tacattaaaa atataatgtc tctctctatt 900

cctcaacaat aaaggatttt tgcatatgaa aaaaaaaaaa aaaaaaaaaa 950

aaaaaaaaaa aaaaaaaaaa aa 972

<210> 108
<211> 135
<212> PRT
<213> Homo Sapien

<400> 108
Met Arg Ile Met Leu Leu Phe Thr Ala Ile Leu Ala Phe Ser Leu
  1               5                  10                  15

Ala Gln Ser Phe Gly Ala Val Cys Lys Glu Pro Gln Glu Glu Val
                 20                  25                  30

Val Pro Gly Gly Gly Arg Ser Lys Arg Asp Pro Asp Leu Tyr Gln
                 35                  40                  45

Leu Leu Gln Arg Leu Phe Lys Ser His Ser Ser Leu Glu Gly Leu
                 50                  55                  60

Leu Lys Ala Leu Ser Gln Ala Ser Thr Asp Pro Lys Glu Ser Thr

                65                  70                  75

Ser Pro Glu Lys Arg Asp Met His Asp Phe Phe Val Gly Leu Met
                80                  85                  90

Gly Lys Arg Ser Val Gln Pro Glu Gly Lys Thr Gly Pro Phe Leu
                95                  100                 105

Pro Ser Val Arg Val Pro Arg Pro Leu His Pro Asn Gln Leu Gly
                110                 115                 120

Ser Thr Gly Lys Ser Ser Leu Gly Thr Glu Glu Gln Arg Pro Leu
                125                 130                 135

<210> 109
<211> 1485
<212> DNA
<213> Homo Sapien

<400> 109
gcggccacac gcagctagcc ggagcccgga ccaggcgcct gtgcctcctc 50

ctcgtccctc gccgcgtccg cgaagcctgg agccggcggg agccccgcgc 100

tcgccatgtc gggcgagctc agcaacaggt tccaaggagg gaaggcgttc 150

ggcttgctca aagcccggca ggagaggagg ctggccgaga tcaaccggga 200

gtttctgtgt gaccagaagt acagtgatga agagaacctt ccagaaaagc 250

tcacagcctt caaagagaag tacatggagt ttgacctgaa caatgaaggc 300

gagattgacc tgatgtcttt aaagaggatg atggagaagc ttggtgtccc 350

caagacccac ctggagatga agaagatgat ctcagaggtg acaggagggg 400

tcagtgacac tatatcctac cgagactttg tgaacatgat gctggggaaa 450

cggtcggctg tcctcaagtt agtcatgatg tttgaaggaa aagccaacga 500

gagcagcccc aagccagttg gcccccctcc agagagagac attgctagcc 550

tgccctgagg accccgcctg gactccccag ccttcccacc ccatacctcc 600

ctcccgatct tgctgccctt cttgacacac tgtgatctct ctctctctca 650

tttgtttggt cattgagggt ttgtttgtgt tttcatcaat gtctttgtaa 700

agcacaaatt atctgcctta aaggggctct gggtcgggga atcctgagcc 750

ttgggtcccc tccctctctt cttccctcct tccccgctcc ctgtgcagaa 800

gggctgatat caaaccaaaa actagagggg gcagggccag ggcagggagg 850

cttccagcct gtgttcccct cacttggagg aaccagcact ctccatcctt 900

tcagaaagtc tccaagccaa gttcaggctc actgacctgg ctctgacgag 950

gaccccaggc cactctgaga agaccttgga gtagggacaa ggctgcaggg 1000

cctctttcgg gtttccttgg acagtgccat ggttccagtg ctctggtgtc 1050

acccaggaca cagccactcg gggccccgct gccccagctg atccccactc 1100

attccacacc tcttctcatc ctcagtgatg tgaaggtggg aaggaaagga 1150

gcttggcatt gggagccctt caagaaggta ccagaaggaa ccctccagtc 1200

ctgctctctg gccacacctg tgcaggcagc tgagaggcag cgtgcagccc 1250

tactgtccct tactggggca gcagagggct tcggaggcag aagtgaggcc 1300

tggggtttgg ggggaaaggt cagctcagtg ctgttccacc ttttagggag 1350

gatactgagg ggaccaggat gggagaatga ggagtaaaat gctcacggca 1400

aagtcagcag cactggtaag ccaagactga gaaatacaag gttgcttgtc 1450

tgaccccaat ctgcttgaaa aaaaaaaaaa aaaaa 1485

<210> 110
<211> 150
<212> PRT
<213> Homo Sapien

<400> 110
Met Ser Gly Glu Leu Ser Asn Arg Phe Gln Gly Gly Lys Ala Phe
1               5                   10                  15

Gly Leu Leu Lys Ala Arg Gln Glu Arg Arg Leu Ala Glu Ile Asn
                20                  25                  30

Arg Glu Phe Leu Cys Asp Gln Lys Tyr Ser Asp Glu Glu Asn Leu
                35                  40                  45

Pro Glu Lys Leu Thr Ala Phe Lys Glu Lys Tyr Met Glu Phe Asp
                50                  55                  60

Leu Asn Asn Glu Gly Glu Ile Asp Leu Met Ser Leu Lys Arg Met
                65                  70                  75

Met Glu Lys Leu Gly Val Pro Lys Thr His Leu Glu Met Lys Lys
                80                  85                  90

Met Ile Ser Glu Val Thr Gly Gly Val Ser Asp Thr Ile Ser Tyr
                95                  100                 105

Arg Asp Phe Val Asn Met Met Leu Gly Lys Arg Ser Ala Val Leu
                110                 115                 120

Lys Leu Val Met Met Phe Glu Gly Lys Ala Asn Glu Ser Ser Pro
                125                 130                 135

Lys Pro Val Gly Pro Pro Pro Glu Arg Asp Ile Ala Ser Leu Pro
                140                 145                 150

<210> 111
<211> 537
<212> DNA
<213> Homo Sapien

<400> 111
taaaacagct acaatattcc agggccagtc acttgccatt tctcataaca 50

gcgtcagaga gaaagaactg actgaaacgt ttgagatgaa gaaagttctc 100

ctcctgatca cagccatctt ggcagtggct gttggtttcc cagtctctca 150

agaccaggaa cgagaaaaaa gaagtatcag tgacagcgat gaattagctt 200

cagggttttt tgtgttccct tacccatatc catttcgccc acttccacca 250

attccatttc caagatttcc atggtttaga cgtaattttc ctattccaat 300

acctgaatct gcccctacaa ctccccttcc tagcgaaaag taaacaagaa 350

ggataagtca cgataaacct ggtcacctga aattgaaatt gagccacttc 400

cttgaagaat caaaattcct gttaataaaa gaaaaacaaa tgtaattgaa 450

atagcacaca gcattctcta gtcaatatct ttagtgatct tctttaataa 500

acatgaaagc aaagattttg gtttcttaat ttccaca 537

<210> 112
<211> 85
<212> PRT
<213> Homo Sapien

<400> 112
Met Lys Lys Val Leu Leu Leu Ile Thr Ala Ile Leu Ala Val Ala
1               5                   10                  15

Val Gly Phe Pro Val Ser Gln Asp Gln Glu Arg Glu Lys Arg Ser
                20                  25                  30

Ile Ser Asp Ser Asp Glu Leu Ala Ser Gly Phe Phe Val Phe Pro
                35                  40                  45

Tyr Pro Tyr Pro Phe Arg Pro Leu Pro Pro Ile Pro Phe Pro Arg
                50                  55                  60

Phe Pro Trp Phe Arg Arg Asn Phe Pro Ile Pro Ile Pro Glu Ser
                65                  70                  75

Ala Pro Thr Thr Pro Leu Pro Ser Glu Lys
                80                  85

<210> 113
<211> 1686
<212> DNA
<213> Homo Sapien

<400> 113
ctcctcttaa catacttgca gctaaaacta aatattgctg cttggggacc 50

tccttctagc cttaaatttc agctcatcac cttcacctgc cttggtcatg 100

gctctgctat tctccttgat ccttgccatt tgcaccagac ctggattcct 150

agcgtctcca tctggagtgc ggctggtggg gggcctccac cgctgtgaag 200

ggcgggtgga ggtggaacag aaaggccagt ggggcaccgt gtgtgatgac 250

ggctgggaca ttaaggacgt ggctgtgttg tgccgggagc tgggctgtgg 300

agctgccagc ggaaccccta gtggtatttt gtatgagcca ccagcagaaa 350

aagagcaaaa ggtcctcatc caatcagtca gttgcacagg aacagaagat 400

acattggctc agtgtgagca agaagaagtt tatgattgtt cacatgatga 450

agatgctggg gcatcgtgtg agaacccaga gagctctttc tccccagtcc 500

cagagggtgt caggctggct gacggccctg ggcattgcaa gggacgcgtg 550

gaagtgaagc accagaacca gtggtatacc gtgtgccaga caggctggag 600

cctccgggcc gcaaaggtgg tgtgccggca gctgggatgt gggagggctg 650

tactgactca aaaacgctgc aacaagcatg cctatggccg aaaacccatc 700

tggctgagcc agatgtcatg ctcaggacga gaagcaaccc ttcaggattg 750

cccttctggg ccttggggga agaacacctg caaccatgat gaagacacgt 800

gggtcgaatg tgaagatccc tttgacttga gactagtagg aggagacaac 850

ctctgctctg ggcgactgga ggtgctgcac aagggcgtat ggggctctgt 900

ctgtgatgac aactggggag aaaaggagga ccaggtggta tgcaagcaac 950

tgggctgtgg gaagtccctc tctccctcct tcagagaccg gaaatgctat 1000

ggccctgggg ttggccgcat ctggctggat aatgttcgtt gctcagggga 1050

ggagcagtcc ctggagcagt gccagcacag attttggggg tttcacgact 1100

gcacccacca ggaagatgtg gctgtcatct gctcagtgta ggtgggcatc 1150

atctaatctg ttgagtgcct gaatagaaga aaaacacaga agaagggagc 1200

atttactgtc tacatgactg catgggatga acactgatct tcttctgccc 1250

ttggactggg acttatactt ggtgcccctg attctcaggc cttcagagtt 1300

ggatcagaac ttacaacatc aggtctagtt ctcaggccat cagacatagt 1350

ttggaactac atcaccacct ttcctatgtc tccacattgc acacagcaga 1400

ttcccagcct ccataattgt gtgtatcaac tacttaaata cattctcaca 1450

cacacacaca cacacacaca cacacacaca cacacataca ccatttgtcc 1500

tgtttctctg aagaactctg acaaaataca gattttggta ctgaaagaga 1550

ttctagagga acggaatttt aaggataaat tttctgaatt ggttatgggg 1600

tttctgaaat tggctctata atctaattag atataaaatt ctggtaactt 1650

tatttacaat aataaagata gcactatgtg ttcaaa 1686

<210> 114
<211> 347
<212> PRT
<213> Homo Sapien

<400> 114
Met Ala Leu Leu Phe Ser Leu Ile Leu Ala Ile Cys Thr Arg Pro
1               5                   10                  15

Gly Phe Leu Ala Ser Pro Ser Gly Val Arg Leu Val Gly Gly Leu
                20                  25                  30

His Arg Cys Glu Gly Arg Val Glu Val Glu Gln Lys Gly Gln Trp
                35                  40                  45

Gly Thr Val Cys Asp Asp Gly Trp Asp Ile Lys Asp Val Ala Val
                50                  55                  60

Leu Cys Arg Glu Leu Gly Cys Gly Ala Ala Ser Gly Thr Pro Ser
                65                  70                  75

Gly Ile Leu Tyr Glu Pro Pro Ala Glu Lys Glu Gln Lys Val Leu
                80                  85                  90

Ile Gln Ser Val Ser Cys Thr Gly Thr Glu Asp Thr Leu Ala Gln
                95                  100                 105

Cys Glu Gln Glu Glu Val Tyr Asp Cys Ser His Asp Glu Asp Ala
                110                 115                 120

Gly Ala Ser Cys Glu Asn Pro Glu Ser Ser Phe Ser Pro Val Pro
                125                 130                 135

Glu Gly Val Arg Leu Ala Asp Gly Pro Gly His Cys Lys Gly Arg
                140                 145                 150

Val Glu Val Lys His Gln Asn Gln Trp Tyr Thr Val Cys Gln Thr
                155                 160                 165

Gly Trp Ser Leu Arg Ala Ala Lys Val Val Cys Arg Gln Leu Gly
                170                 175                 180

Cys Gly Arg Ala Val Leu Thr Gln Lys Arg Cys Asn Lys His Ala
                185                 190                 195

Tyr Gly Arg Lys Pro Ile Trp Leu Ser Gln Met Ser Cys Ser Gly
                200                 205                 210

Arg Glu Ala Thr Leu Gln Asp Cys Pro Ser Gly Pro Trp Gly Lys
                215                 220                 225

Asn Thr Cys Asn His Asp Glu Asp Thr Trp Val Glu Cys Glu Asp
                230                 235                 240

Pro Phe Asp Leu Arg Leu Val Gly Gly Asp Asn Leu Cys Ser Gly
                245                 250                 255

Arg Leu Glu Val Leu His Lys Gly Val Trp Gly Ser Val Cys Asp
                260                 265                 270

Asp Asn Trp Gly Glu Lys Glu Asp Gln Val Val Cys Lys Gln Leu
                275                 280                 285

Gly Cys Gly Lys Ser Leu Ser Pro Ser Phe Arg Asp Arg Lys Cys
                290                 295                 300

Tyr Gly Pro Gly Val Gly Arg Ile Trp Leu Asp Asn Val Arg Cys
                305                 310                 315

Ser Gly Glu Glu Gln Ser Leu Glu Gln Cys Gln His Arg Phe Trp
                320                 325                 330

Gly Phe His Asp Cys Thr His Gln Glu Asp Val Ala Val Ile Cys
                335                 340                 345

Ser Val

<210> 115
<211> 728
<212> DNA
<213> Homo Sapien

<400> 115
catttccaac aagagcactg gccaagtcag cttcttctga gagagtctct 50

agaagacatg atgctacact cagctttggg tctctgcctc ttactcgtca 100

cagtttcttc caaccttgcc attgcaataa aaaaggaaaa gaggcctcct 150

cagacactct caagaggatg gggagatgac atcacttggg tacaaactta 200

tgaagaaggt ctcttttatg ctcaaaaaag taagaagcca ttaatggtta 250

ttcatcacct ggaggattgt caatactctc aagcactaaa gaaagtattt 300

gcccaaaatg aagaaataca agaaatggct cagaataagt tcatcatgct 350

aaaccttatg catgaaacca ctgataagaa tttatcacct gatgggcaat 400

atgtgcctag aatcatgttt gtagaccctt ctttaacagt tagagctgac 450

atagctggaa gatactctaa cagattgtac acatatgagc ctcgggattt 500

acccctattg atagaaaaca tgaagaaagc attaagactt attcagtcag 550

agctataaga gatgatggaa aaaagccttc acttcaaaga agtcaaattt 600

catgaagaaa acctctggca cattgacaaa tactaaatgt gcaagtatat 650

agattttgta atattactat ttagtttttt taatgtgttt gcaatagtct 700

tattaaaata aatgtttttt aaatctga 728

<210> 116
<211> 166
<212> PRT
<213> Homo Sapien

<400> 116
Met Met Leu His Ser Ala Leu Gly Leu Cys Leu Leu Leu Val Thr
1               5                   10                  15

Val Ser Ser Asn Leu Ala Ile Ala Ile Lys Lys Glu Lys Arg Pro
                20                  25                  30

Pro Gln Thr Leu Ser Arg Gly Trp Gly Asp Asp Ile Thr Trp Val
                35                  40                  45

Gln Thr Tyr Glu Glu Gly Leu Phe Tyr Ala Gln Lys Ser Lys Lys
                50                  55                  60

Pro Leu Met Val Ile His His Leu Glu Asp Cys Gln Tyr Ser Gln
                65                  70                  75

Ala Leu Lys Lys Val Phe Ala Gln Asn Glu Glu Ile Gln Glu Met
                80                  85                  90

Ala Gln Asn Lys Phe Ile Met Leu Asn Leu Met His Glu Thr Thr
                95                  100                 105

Asp Lys Asn Leu Ser Pro Asp Gly Gln Tyr Val Pro Arg Ile Met
                110                 115                 120

Phe Val Asp Pro Ser Leu Thr Val Arg Ala Asp Ile Ala Gly Arg
                125                 130                 135

Tyr Ser Asn Arg Leu Tyr Thr Tyr Glu Pro Arg Asp Leu Pro Leu
                140                 145                 150

Leu Ile Glu Asn Met Lys Lys Ala Leu Arg Leu Ile Gln Ser Glu
                155                 160                 165

Leu

<210> 117
<211> 2395
<212> DNA
<213> Homo Sapien

<400> 117
cctggagccg gaagcgcggc tgcagcaggg cgaggctcca ggtggggtcg 50

gttccgcatc cagcctagcg tgtccacgat gcggctgggc tccgggactt 100

tcgctacctg ttgcgtagcg atcgaggtgc tagggatcgc ggtcttcctt 150
cggggattct tcccggctcc cgttcgttcc tctgccagag cggaacacgg 200
agcggagccc ccagcgcccg aaccctcggc tggagccagt tctaactgga 250
ccacgctgcc accacctctc ttcagtaaag ttgttattgt tctgatagat 300
gccttgagag atgattttgt gtttgggtca aagggtgtga aatttatgcc 350
ctacacaact taccttgtgg aaaaaggagc atctcacagt tttgtggctg 400
aagcaaagcc acctacagtt actatgcctc gaatcaaggc attgatgacg 450
gggagccttc ctggctttgt cgacgtcatc aggaacctca attctcctgc 500
actgctggaa gacagtgtga taagacaagc aaaagcagct ggaaaaagaa 550
tagtctttta tggagatgaa acctgggtta aattattccc aaagcatttt 600
gtggaatatg atggaacaac ctcatttttc gtgtcagatt acacagaggt 650
ggataataat gtcacgaggc atttggataa agtattaaaa agaggagatt 700
gggacatatt aatcctccac tacctggggc tggaccacat tggccacatt 750
tcagggccca acagccccct gattgggcag aagctgagcg agatggacag 800
cgtgctgatg aagatccaca cctcactgca gtcgaaggag agagagacgc 850
ctttacccaa tttgctggtt ctttgtggtg accatggcat gtctgaaaca 900
ggaagtcacg gggcctcctc caccgaggag gtgaatacac ctctgatttt 950
aatcagttct gcgtttgaaa ggaaacccgg tgatatccga catccaaagc 1000
acgtccaata gacggatgtg gctgcgacac tggcgatagc acttggctta 1050
ccgattccaa aagacagtgt agggagcctc ctattcccag ttgtggaagg 1100
aagaccaatg agagagcagt tgagattttt acatttgaat acagtgcagc 1150
ttagtaaact gttgcaagag aatgtgccgt catatgaaaa agatcctggg 1200
tttgagcagt ttaaaatgtc agaaagattg catgggaact ggatcagact 1250
gtacttggag gaaaagcatt cagaagtcct attcaacctg ggctccaagg 1300
ttctcaggca gtacctggat gctctgaaga cgctgagctt gtccctgagt 1350
gcacaagtgg cccagttctc accctgctcc tgctcagcgt cccacaggca 1400
ctgcacagaa aggctgagct ggaagtccca ctgtcatctc ctgggttttc 1450
tctgctcttt tatttggtga tcctggttct ttcggccgtt cacgtcattg 1500
tgtgcacctc agctgaaagt tcgtgctact tctgtggcct ctcgtggctg 1550
gcggcaggct gcctttcgtt taccagactc tggttgaaca cctggtgtgt 1600
gccaagtgct ggcagtgccc tggacagggg gcctcaggga aggacgtgga 1650
gcagccttat cccaggcctc tgggtgtccc gacacaggtg ttcacatctg 1700
tgctgtcagg tcagatgcct cagttcttgg aaagctaggt tcctgcgact 1750
gttaccaagg tgattgtaaa gagctggcgg tcacagagga acaagccccc 1800

cagctgaggg ggtgtgtgaa tcggacagcc tcccagcaga ggtgtgggag 1850

ctgcagctga gggaagaaga gacaatcggc ctggacactc aggagggtca 1900

aaaggagact tggtcgcacc actcatcctg ccacccccag aatgcatcct 1950

gcctcatcag gtccagattt ctttccaagg cggacgtttt ctgttggaat 2000

tcttagtcct tggcctcgga caccttcatt cgttagctgg ggagtggtgg 2050

tgaggcagtg aagaagaggc ggatggtcac actcagatcc acagagccca 2100

ggatcaaggg acccactgca gtggcagcag gactgttggg cccccacccc 2150

aaccctgcac agccctcatc ccctcttggc ttgagccgtc agaggccctg 2200

tgctgagtgt ctgaccgaga cactcacagc tttgtcatca gggcacaggc 2250

ttcctcggag ccaggatgat ctgtgccacg cttgcacctc gggcccatct 2300

gggctcatgc tctctctcct gctattgaat tagtacctag ctgcacacag 2350

tatgtagtta ccaaaagaat aaacggcaat aattgagaaa aaaaa 2395

<210> 118
<211> 310
<212> PRT
<213> Homo Sapien

<400> 118
Met Arg Leu Gly Ser Gly Thr Phe Ala Thr Cys Cys Val Ala Ile
1               5                   10                  15

Glu Val Leu Gly Ile Ala Val Phe Leu Arg Gly Phe Phe Pro Ala
                20                  25                  30

Pro Val Arg Ser Ser Ala Arg Ala Glu His Gly Ala Glu Pro Pro
                35                  40                  45

Ala Pro Glu Pro Ser Ala Gly Ala Ser Ser Asn Trp Thr Thr Leu
                50                  55                  60

Pro Pro Pro Leu Phe Ser Lys Val Val Ile Val Leu Ile Asp Ala
                65                  70                  75

Leu Arg Asp Asp Phe Val Phe Gly Ser Lys Gly Val Lys Phe Met
                80                  85                  90

Pro Tyr Thr Thr Tyr Leu Val Glu Lys Gly Ala Ser His Ser Phe
                95                  100                 105

Val Ala Glu Ala Lys Pro Pro Thr Val Thr Met Pro Arg Ile Lys
                110                 115                 120

Ala Leu Met Thr Gly Ser Leu Pro Gly Phe Val Asp Val Ile Arg
                125                 130                 135

Asn Leu Asn Ser Pro Ala Leu Leu Glu Asp Ser Val Ile Arg Gln
                140                 145                 150

Ala Lys Ala Ala Gly Lys Arg Ile Val Phe Tyr Gly Asp Glu Thr
                155                 160                 165

Trp Val Lys Leu Phe Pro Lys His Phe Val Glu Tyr Asp Gly Thr
                170                 175                 180

Thr Ser Phe Phe Val Ser Asp Tyr Thr Glu Val Asp Asn Asn Val
                185                 190                 195

Thr Arg His Leu Asp Lys Val Leu Lys Arg Gly Asp Trp Asp Ile
                200                 205                 210

Leu Ile Leu His Tyr Leu Gly Leu Asp His Ile Gly His Ile Ser
                215                 220                 225

Gly Pro Asn Ser Pro Leu Ile Gly Gln Lys Leu Ser Glu Met Asp
                230                 235                 240

Ser Val Leu Met Lys Ile His Thr Ser Leu Gln Ser Lys Glu Arg
                245                 250                 255

Glu Thr Pro Leu Pro Asn Leu Leu Val Leu Cys Gly Asp His Gly
                260                 265                 270

Met Ser Glu Thr Gly Ser His Gly Ala Ser Ser Thr Glu Glu Val
                275                 280                 285

Asn Thr Pro Leu Ile Leu Ile Ser Ser Ala Phe Glu Arg Lys Pro
                290                 295                 300

Gly Asp Ile Arg His Pro Lys His Val Gln
                305                 310

<210> 119
<211> 1333
<212> DNA
<213> Homo Sapien

<400> 119
gcccacgcgt ccgatggcgt tcacgttcgc ggccttctgc tacatgctgg 50

cgctgctgct cactgccgcg ctcatcttct tcgccatttg gcacattata 100

gcatttgatg agctgaagac tgattacaag aatcctatag accagtgtaa 150

taccctgaat ccccttgtac tcccagagta cctcatccac gctttcttct 200

gtgtcatgtt tctttgtgca gcagagtggc ttacactggg tctcaatatg 250

cccctcttgg catatcatat ttggaggtat atgagtagac cagtgatgag 300

tggcccagga ctctatgacc ctacaaccat catgaatgca gatattctag 350

catattgtca gaaggaagga tggtgcaaat tagcttttta tcttctagca 400

tttttttact acctatatgg catgatctat gttttggtga gctcttagaa 450

caacacacag aagaattggt ccagttaagt gcatgcaaaa agccaccaaa 500

tgaaggattt ctatccagca agatcctgtc caagagtagc ctgtggaatc 550

tgatcagtta ctttaaaaaa tgactcctta ttttttaaat gtttccacat 600

ttttgcttgt ggaaagactg ttttcatatg ttatactcag ataaagattt 650

taaatggtat tacgtataaa ttaatataaa atgattacct ctggtgttga 700

caggtttgaa cttgcacttc ttaaggaaca gccataatcc tctgaatgat 750

gcattaatta ctgactgtcc tagtacattg gaagcttttg tttataggaa 800

cttgtagggc tcattttggt ttcattgaaa cagtatctaa ttataaatta 850

gctgtagata tcaggtgctt ctgatgaagt gaaaatgtat atctgactag 900

tgggaaactt catgggtttc ctcatctgtc atgtcgatga ttatatatgg 950

atacatttac aaaaataaaa agcgggaatt ttcccttcgc ttgaatatta 1000

tccctgtata ttgcatgaat gagagatttc ccatatttcc atcagagtaa 1050

taaatatact tgctttaatt cttaagcata agtaaacatg atataaaaat 1100

atatgctgaa ttacttgtga agaatgcatt taaagctatt ttaaatgtgt 1150

ttttatttgt aagacattac ttattaagaa attggttatt atgcttactg 1200

ttctaatctg gtggtaaagg tattcttaag aatttgcagg tactacagat 1250

tttcaaaact gaatgagaga aaattgtata accatcctgc tgttccttta 1300

gtgcaataca ataaaactct gaaattaaga ctc 1333

<210> 120
<211> 144
<212> PRT
<213> Homo Sapien

<400> 120
Met Ala Phe Thr Phe Ala Ala Phe Cys Tyr Met Leu Ala Leu Leu
1               5                   10                  15

Leu Thr Ala Ala Leu Ile Phe Phe Ala Ile Trp His Ile Ile Ala
                20                  25                  30

Phe Asp Glu Leu Lys Thr Asp Tyr Lys Asn Pro Ile Asp Gln Cys
                35                  40                  45

Asn Thr Leu Asn Pro Leu Val Leu Pro Glu Tyr Leu Ile His Ala
                50                  55                  60

Phe Phe Cys Val Met Phe Leu Cys Ala Ala Glu Trp Leu Thr Leu
                65                  70                  75

Gly Leu Asn Met Pro Leu Leu Ala Tyr His Ile Trp Arg Tyr Met
                80                  85                  90

Ser Arg Pro Val Met Ser Gly Pro Gly Leu Tyr Asp Pro Thr Thr
                95                  100                 105

Ile Met Asn Ala Asp Ile Leu Ala Tyr Cys Gln Lys Glu Gly Trp
                110                 115                 120

Cys Lys Leu Ala Phe Tyr Leu Leu Ala Phe Phe Tyr Tyr Leu Tyr
                125                 130                 135

Gly Met Ile Tyr Val Leu Val Ser Ser
                140

<210> 121
<211> 1838
<212> DNA
<213> Homo Sapien

<400> 121
cggacgcgtg ggcggacgcg tgggcggccc acggcgcccg cgggctgggg 50

cggtcgcttc ttccttctcc gtggcctacg agggtcccca gcctgggtaa 100

agatggcccc atggcccccg aagggcctag tcccagctgt gctctggggc 150

ctcagcctct tcctcaacct cccaggacct atctggctcc agccctctcc 200

acctccccag tcttctcccc cgcctcagcc ccatccgtgt catacctgcc 250

ggggactggt tgacagcttt aacaagggcc tggagagaac catccgggac 300
aactttggag gtggaaacac tgcctgggag gaagagaatt tgtccaaata 350
caaagacagt gagacccgcc tggtagaggt gctggagggt gtgtgcagca 400
agtcagactt cgagtgccac cgcctgctgg agctgagtga ggagctggtg 450
gagagctggt ggtttcacaa gcagcaggag gccccggacc tcttccagtg 500
gctgtgctca gattccctga agctctgctg ccccgcaggc accttcgggc 550
cctcctgcct tccctgtcct gggggaacag agaggccctg cggtggctac 600
gggcagtgtg aaggagaagg gacacgaggg ggcagcgggc actgtgactg 650
ccaagccggc tacgggggtg aggcctgtgg ccagtgtggc cttggctact 700
ttgaggcaga acgcaacgcc agccatctgg tatgttcggc ttgttttggc 750
ccctgtgccc gatgctcagg acctgaggaa tcaaactgtt tgcaatgcaa 800
gaagggctgg gccctgcatc acctcaagtg tgtagacatt gatgagtgtg 850
gcacagaggg agccaactgt ggagctgacc aattctgcgt gaacactgag 900
ggctcctatg agtgccgaga ctgtgccaag gcctgcctag gctgcatggg 950
ggcagggcca ggtcgctgta agaagtgtag ccctggctat cagcaggtgg 1000
gctccaagtg tctcgatgtg gatgagtgtg agacagaggt gtgtccggga 1050
gagaacaagc agtgtgaaaa caccgagggc ggttatcgct gcatctgtgc 1100
cgagggctac aagcagatgg aaggcatctg tgtgaaggag cagatcccag 1150
agtcagcagg cttcttctca gagatgacag aagacgagtt ggtggtgctg 1200
cagcagatgt tctttggcat catcatctgt gcactggcca cgctggctgc 1250
taagggcgac ttggtgttca ccgccatctt cattggggct gtggcggcca 1300
tgactggcta ctggttgtca gagcgcagtg accgtgtgct ggagggcttc 1350
atcaagggca gataatcgcg gccaccacct gtaggacctc ctcccaccca 1400
cgctgccccc agagcttggg ctgccctcct gctggacact caggacagct 1450
tggtttattt ttgagagtgg ggtaagcacc cctacctgcc ttacagagca 1500
gcccaggtac ccaggcccgg gcagacaagg cccctggggt aaaaagtagc 1550
cctgaaggtg gataccatga gctcttcacc tggcggggac tggcaggctt 1600
cacaatgtgt gaatttcaaa agtttttcct taatggtggc tgctagagct 1650
ttggcccctg cttaggatta ggtggtcctc acaggggtgg ggccatcaca 1700
gctccctcct gccagctgca tgctgccagt tcctgttctg tgttcaccac 1750
atccccacac cccattgcca cttatttatt catctcagga aataaagaaa 1800
ggtcttggaa agttaaaaaa aaaaaaaaaa aaaaaaaa 1838

<210> 122
<211> 420
<212> PRT

<213> Homo Sapien

<400> 122
Met Ala Pro Trp Pro Pro Lys Gly Leu Val Pro Ala Val Leu Trp
1               5                   10                  15

Gly Leu Ser Leu Phe Leu Asn Leu Pro Gly Pro Ile Trp Leu Gln
                20                  25                  30

Pro Ser Pro Pro Pro Gln Ser Ser Pro Pro Pro Gln Pro His Pro
                35                  40                  45

Cys His Thr Cys Arg Gly Leu Val Asp Ser Phe Asn Lys Gly Leu
                50                  55                  60

Glu Arg Thr Ile Arg Asp Asn Phe Gly Gly Gly Asn Thr Ala Trp
                65                  70                  75

Glu Glu Glu Asn Leu Ser Lys Tyr Lys Asp Ser Glu Thr Arg Leu
                80                  85                  90

Val Glu Val Leu Glu Gly Val Cys Ser Lys Ser Asp Phe Glu Cys
                95                  100                 105

His Arg Leu Leu Glu Leu Ser Glu Glu Leu Val Glu Ser Trp Trp
                110                 115                 120

Phe His Lys Gln Gln Glu Ala Pro Asp Leu Phe Gln Trp Leu Cys
                125                 130                 135

Ser Asp Ser Leu Lys Leu Cys Cys Pro Ala Gly Thr Phe Gly Pro
                140                 145                 150

Ser Cys Leu Pro Cys Pro Gly Gly Thr Glu Arg Pro Cys Gly Gly
                155                 160                 165

Tyr Gly Gln Cys Glu Gly Glu Gly Thr Arg Gly Gly Ser Gly His
                170                 175                 180

Cys Asp Cys Gln Ala Gly Tyr Gly Gly Glu Ala Cys Gly Gln Cys
                185                 190                 195

Gly Leu Gly Tyr Phe Glu Ala Glu Arg Asn Ala Ser His Leu Val
                200                 205                 210

Cys Ser Ala Cys Phe Gly Pro Cys Ala Arg Cys Ser Gly Pro Glu
                215                 220                 225

Glu Ser Asn Cys Leu Gln Cys Lys Lys Gly Trp Ala Leu His His
                230                 235                 240

Leu Lys Cys Val Asp Ile Asp Glu Cys Gly Thr Glu Gly Ala Asn
                245                 250                 255

Cys Gly Ala Asp Gln Phe Cys Val Asn Thr Glu Gly Ser Tyr Glu
                260                 265                 270

Cys Arg Asp Cys Ala Lys Ala Cys Leu Gly Cys Met Gly Ala Gly
                275                 280                 285

Pro Gly Arg Cys Lys Lys Cys Ser Pro Gly Tyr Gln Gln Val Gly
                290                 295                 300

Ser Lys Cys Leu Asp Val Asp Glu Cys Glu Thr Glu Val Cys Pro
                305                 310                 315

Gly Glu Asn Lys Gln Cys Glu Asn Thr Glu Gly Gly Tyr Arg Cys
                320                 325                 330

Ile Cys Ala Glu Gly Tyr Lys Gln Met Glu Gly Ile Cys Val Lys
                335                 340                 345

Glu Gln Ile Pro Glu Ser Ala Gly Phe Phe Ser Glu Met Thr Glu
                350                 355                 360

Asp Glu Leu Val Val Leu Gln Gln Met Phe Phe Gly Ile Ile Ile
                365                 370                 375

Cys Ala Leu Ala Thr Leu Ala Ala Lys Gly Asp Leu Val Phe Thr
                380                 385                 390

Ala Ile Phe Ile Gly Ala Val Ala Ala Met Thr Gly Tyr Trp Leu
                395                 400                 405

Ser Glu Arg Ser Asp Arg Val Leu Glu Gly Phe Ile Lys Gly Arg
                410                 415                 420

<210> 123
<211> 3401
<212> DNA
<213> Homo Sapien

<400> 123
gcaagccaag gcgctgtttg agaaggtgaa gaagttccgg acccatgtgg 50

aggaggggga cattgtgtac cgcctctaca tgcggcagac catcatcaag 100

gtgatcaagt tcatcctcat catctgctac accgtctact acgtgcacaa 150

catcaagttc gacgtggact gcaccgtgga cattgagagc ctgacgggct 200

accgcaccta ccgctgtgcc caccccctgg ccacactctt caagatcctg 250

gcgtccttct acatcagcct agtcatcttc tacggcctca tctgcatgta 300

cacactgtgg tggatgctac ggcgctccct caagaagtac tcgtttgagt 350

cgatccgtga ggagagcagc tacagcgaca tccccgacgt caagaacgac 400

ttcgccttca tgctgcacct cattgaccaa tacgacccgc tctactccaa 450

gcgcttcgcc gtcttcctgt cggaggtgag tgagaacaag ctgcggcagc 500

tgaacctcaa caacgagtgg acgctggaca agctccggca gcggctcacc 550

aagaacgcgc aggacaagct ggagctgcac ctgttcatgc tcagtggcat 600

ccctgacact gtgtttgacc tggtggagct ggaggtcctc aagctggagc 650

tgatccccga cgtgaccatc ccgcccagca ttgcccagct cacgggcctc 700

aaggagctgt ggctctacca cacagcggcc aagattgaag cgcctgcgct 750

ggccttcctg cgcgagaacc tgcgggcgct gcacatcaag ttcaccgaca 800

tcaaggagat cccgctgtgg atctatagcc tgaagacact ggaggagctg 850

cacctgacgg gcaacctgag cgcggagaac aaccgctaca tcgtcatcga 900

cgggctgcgg gagctcaaac gcctcaaggt gctgcggctc aagagcaacc 950

taagcaagct gccacaggtg gtcacagatg tgggcgtgca cctgcagaag 1000

ctgtccatca acaatgaggg caccaagctc atcgtcctca acagcctcaa 1050

gaagatggcg aacctgactg agctggagct gatccgctgc gacctggagc 1100

gcatccccca ctccatcttc agcctccaca acctgcagga gattgacctc 1150
aaggacaaca acctcaagac catcgaggag atcatcagct tccagcacct 1200
gcaccgcctc acctgcctta agctgtggta caaccacatc gcctacatcc 1250
ccatccagat cggcaacctc accaacctgg agcgcctcta cctgaaccgc 1300
aacaagatcg agaagatccc cacccagctc ttctactgcc gcaagctgcg 1350
ctacctggac ctcagccaca acaacctgac cttcctccct gccgacatcg 1400
gcctcctgca gaacctccag aacctagcca tcacggccaa ccggatcgag 1450
acgctccctc cggagctctt ccagtgccgg aagctgcggg ccctgcacct 1500
gggcaacaac gtgctgcagt cactgccctc cagggtgggc gagctgacca 1550
acctgacgca gatcgagctg cggggcaacc ggctggagtg cctgcctgtg 1600
gagctgggcg agtgcccact gctcaagcgc agcggcttgg tggtggagga 1650
ggacctgttc aacacactgc cacccgaggt gaaggagcgg ctgtggaggg 1700
ctgacaagga gcaggcctga gcgaggccgg cccagcacag caagcagcag 1750
gaccgctgcc cagtcctcag gcccggaggg gcaggcctag cttctcccag 1800
aactcccgga cagccaggac agcctcgcgg ctgggcagga gcctggggcc 1850
gcttgtgagt caggccagag cgagaggaca gtatctgtgg ggctggcccc 1900
ttttctccct ctgagactca cgtcccccag ggcaagtgct tgtggaggag 1950
agcaagtctc aagagcgcag tatttggata atcagggtct cctccctgga 2000
ggccagctct gccccagggg ctgagctgcc accagaggtc ctgggaccct 2050
cactttagtt cttggtattt atttttctcc atctcccacc tccttcatcc 2100
agataactta tacattccca agaaagttca gcccagatgg aaggtgttca 2150
gggaaaggtg ggctgccttt tccccttgtc cttatttagc gatgccgccg 2200
ggcatttaac acccacctgg acttcagcag agtggtccgg ggcgaaccag 2250
ccatgggacg gtcacccagc agtgccgggc tgggctctgc ggtgcggtcc 2300
acgggagagc aggcctccag ctggaaaggc caggcctgga gcttgcctct 2350
tcagtttttg tggcagtttt agttttttgt tttttttttt tttaatcaaa 2400
aaacaatttt ttttaaaaaa aagctttgaa aatggatggt ttgggtatta 2450
aaaagaaaaa aaaaacttaa aaaaaaaaag acactaacgg ccagtgagtt 2500
ggagtctcag ggcagggtgg cagtttccct tgagcaaagc agccagacgt 2550
tgaactgtgt ttcctttccc tgggcgcagg gtgcagggtg tcttccggat 2600
ctggtgtgac cttggtccag gagttctatt tgttcctggg gagggaggtt 2650
tttttgtttg ttttttgggt ttttttggtg tcttgttttc tttctcctcc 2700
atgtgtcttg gcaggcactc atttctgtgg ctgtcggcca gagggaatgt 2750
tctggagctg ccaaggaggg aggagactcg ggttggctaa tccccggatg 2800

aacggtgctc cattcgcacc tcccctcctc gtgcctgccc tgcctctcca 2850

cgcacagtgt taaggagcca agaggagcca cttcgcccag actttgtttc 2900

cccacctcct gcggcatggg tgtgtccagt gccaccgctg gcctccgctg 2950

cttccatcag ccctgtcgcc acctggtcct tcatgaagag cagacactta 3000

gaggctggtc gggaatgggg aggtcgcccc tgggagggca ggcgttggtt 3050

ccaagccggt tcccgtccct ggcgcctgga gtgcacacag cccagtcggc 3100

acctggtggc tggaagccaa cctgctttag atcactcggg tccccacctt 3150

agaagggtcc ccgccttaga tcaatcacgt ggacactaag gcacgtttta 3200

gagtctcttg tcttaatgat tatgtccatc cgtctgtccg tccatttgtg 3250

ttttctgcgt cgtgtcattg gatataatcc tcagaaataa tgcacactag 3300

cctctgacaa ccatgaagca aaaatccgtt acatgtgggt ctgaacttgt 3350

agactcggtc acagtatcaa ataaaatcta taacagaaaa aaaaaaaaaa 3400

a 3401

<210> 124
<211> 546
<212> PRT
<213> Homo Sapien

<400> 124
Met Arg Gln Thr Ile Ile Lys Val Ile Lys Phe Ile Leu Ile Ile
1               5                   10                  15

Cys Tyr Thr Val Tyr Tyr Val His Asn Ile Lys Phe Asp Val Asp
                20                  25                  30

Cys Thr Val Asp Ile Glu Ser Leu Thr Gly Tyr Arg Thr Tyr Arg
                35                  40                  45

Cys Ala His Pro Leu Ala Thr Leu Phe Lys Ile Leu Ala Ser Phe
                50                  55                  60

Tyr Ile Ser Leu Val Ile Phe Tyr Gly Leu Ile Cys Met Tyr Thr
                65                  70                  75

Leu Trp Trp Met Leu Arg Arg Ser Leu Lys Lys Tyr Ser Phe Glu
                80                  85                  90

Ser Ile Arg Glu Glu Ser Ser Tyr Ser Asp Ile Pro Asp Val Lys
                95                  100                 105

Asn Asp Phe Ala Phe Met Leu His Leu Ile Asp Gln Tyr Asp Pro
                110                 115                 120

Leu Tyr Ser Lys Arg Phe Ala Val Phe Leu Ser Glu Val Ser Glu
                125                 130                 135

Asn Lys Leu Arg Gln Leu Asn Leu Asn Asn Glu Trp Thr Leu Asp
                140                 145                 150

Lys Leu Arg Gln Arg Leu Thr Lys Asn Ala Gln Asp Lys Leu Glu
                155                 160                 165

Leu His Leu Phe Met Leu Ser Gly Ile Pro Asp Thr Val Phe Asp
                170                 175                 180

Leu Val Glu Leu Glu Val Leu Lys Leu Glu Leu Ile Pro Asp Val
                185                 190                 195

Thr Ile Pro Pro Ser Ile Ala Gln Leu Thr Gly Leu Lys Glu Leu
                200                 205                 210

Trp Leu Tyr His Thr Ala Ala Lys Ile Glu Ala Pro Ala Leu Ala
                215                 220                 225

Phe Leu Arg Glu Asn Leu Arg Ala Leu His Ile Lys Phe Thr Asp
                230                 235                 240

Ile Lys Glu Ile Pro Leu Trp Ile Tyr Ser Leu Lys Thr Leu Glu
                245                 250                 255

Glu Leu His Leu Thr Gly Asn Leu Ser Ala Glu Asn Asn Arg Tyr
                260                 265                 270

Ile Val Ile Asp Gly Leu Arg Glu Leu Lys Arg Leu Lys Val Leu
                275                 280                 285

Arg Leu Lys Ser Asn Leu Ser Lys Leu Pro Gln Val Val Thr Asp
                290                 295                 300

Val Gly Val His Leu Gln Lys Leu Ser Ile Asn Asn Glu Gly Thr
                305                 310                 315

Lys Leu Ile Val Leu Asn Ser Leu Lys Lys Met Ala Asn Leu Thr
                320                 325                 330

Glu Leu Glu Leu Ile Arg Cys Asp Leu Glu Arg Ile Pro His Ser
                335                 340                 345

Ile Phe Ser Leu His Asn Leu Gln Glu Ile Asp Leu Lys Asp Asn
                350                 355                 360

Asn Leu Lys Thr Ile Glu Glu Ile Ile Ser Phe Gln His Leu His
                365                 370                 375

Arg Leu Thr Cys Leu Lys Leu Trp Tyr Asn His Ile Ala Tyr Ile
                380                 385                 390

Pro Ile Gln Ile Gly Asn Leu Thr Asn Leu Glu Arg Leu Tyr Leu
                395                 400                 405

Asn Arg Asn Lys Ile Glu Lys Ile Pro Thr Gln Leu Phe Tyr Cys
                410                 415                 420

Arg Lys Leu Arg Tyr Leu Asp Leu Ser His Asn Asn Leu Thr Phe
                425                 430                 435

Leu Pro Ala Asp Ile Gly Leu Leu Gln Asn Leu Gln Asn Leu Ala
                440                 445                 450

Ile Thr Ala Asn Arg Ile Glu Thr Leu Pro Pro Glu Leu Phe Gln
                455                 460                 465

Cys Arg Lys Leu Arg Ala Leu His Leu Gly Asn Asn Val Leu Gln
                470                 475                 480

Ser Leu Pro Ser Arg Val Gly Glu Leu Thr Asn Leu Thr Gln Ile
                485                 490                 495

Glu Leu Arg Gly Asn Arg Leu Glu Cys Leu Pro Val Glu Leu Gly
                500                 505                 510

Glu Cys Pro Leu Leu Lys Arg Ser Gly Leu Val Val Glu Glu Asp

                515                 520                 525

Leu Phe Asn Thr Leu Pro Pro Glu Val Lys Glu Arg Leu Trp Arg
                530                 535                 540

Ala Asp Lys Glu Gln Ala
                545

<210> 125
<211> 1679
<212> DNA
<213> Homo Sapien

<400> 125
gttgtgtcct tcagcaaaac agtggattta aatctccttg cacaagcttg 50

agagcaacac aatctatcag gaaagaaaga aagaaaaaaa ccgaacctga 100

caaaaaagaa gaaaaagaag aagaaaaaaa atcatgaaaa ccatccagcc 150

aaaaatgcac aattctatct cttgggcaat cttcacgggg ctggctgctc 200

tgtgtctctt ccaaggagtg cccgtgcgca gcggagatgc caccttcccc 250

aaagctatgg acaacgtgac ggtccggcag ggggagagcg ccaccctcag 300

gtgcactatt gacaaccggg tcacccgggt ggcctggcta aaccgcagca 350

ccatcctcta tgctgggaat gacaagtggt gcctggatcc tcgcgtggtc 400

cttctgagca acacccaaac gcagtacagc atcgagatcc agaacgtgga 450

tgtgtatgac gagggccctt acacctgctc ggtgcagaca gacaaccacc 500

caaagacctc tagggtccac ctcattgtgc aagtatctcc caaaattgta 550

gagatttctt cagatatctc cattaatgaa gggaacaata ttagcctcac 600

ctgcatagca actggtagac cagagcctac ggttacttgg agacacatct 650

ctcccaaagc ggttggcttt gtgagtgaag acgaatactt ggaaattcag 700

ggcatcaccc gggagcagtc aggggactac gagtgcagtg cctccaatga 750

cgtggccgcg cccgtggtac ggagagtaaa ggtcaccgtg aactatccac 800

catacatttc agaagccaag ggtacaggtg tccccgtggg acaaaagggg 850

acactgcagt gtgaagcctc agcagtcccc tcagcagaat tccagtggta 900

caaggatgac aaaagactga ttgaaggaaa gaaaggggtg aaagtggaaa 950

acagaccttt cctctcaaaa ctcatcttct tcaatgtctc tgaacatgac 1000

tatgggaact acacttgcgt ggcctccaac aagctgggcc acaccaatgc 1050

cagcatcatg ctatttggtc caggcgccgt cagcgaggtg agcaacggca 1100

cgtcgaggag ggcaggctgc gtctggctgc tgcctcttct ggtcttgcac 1150

ctgcttctca aattttgatg tgagtgccac ttccccaccc gggaaaggct 1200

gccgccacca ccaccaccaa cacaacagca atggcaacac cgacagcaac 1250

caatcagata tatacaaatg aaattagaag aaacacagcc tcatgggaca 1300

gaaatttgag ggaggggaac aaagaatact ttgggggaaa aagagtttta 1350

aaaaagaaat tgaaaattgc cttgcagata tttaggtaca atggagtttt 1400

cttttcccaa acgggaagaa cacagcacac ccggcttgga cccactgcaa 1450

gctgcatcgt gcaacctctt tggtgccagt gtgggcaagg gctcagcctc 1500

tctgcccaca gagtgccccc acgtggaaca ttctggagct ggccatccca 1550

aattcaatca gtccatagag acgaacagaa tgagaccttc cggcccaagc 1600

gtggcgctgc gggcactttg gtagactgtg ccaccacggc gtgtgttgtg 1650

aaacgtgaaa taaaaagagc aaaaaaaaa 1679

<210> 126
<211> 344
<212> PRT
<213> Homo Sapien

<400> 126
Met Lys Thr Ile Gln Pro Lys Met His Asn Ser Ile Ser Trp Ala
1               5                   10                  15

Ile Phe Thr Gly Leu Ala Ala Leu Cys Leu Phe Gln Gly Val Pro
                20                  25                  30

Val Arg Ser Gly Asp Ala Thr Phe Pro Lys Ala Met Asp Asn Val
                35                  40                  45

Thr Val Arg Gln Gly Glu Ser Ala Thr Leu Arg Cys Thr Ile Asp
                50                  55                  60

Asn Arg Val Thr Arg Val Ala Trp Leu Asn Arg Ser Thr Ile Leu
                65                  70                  75

Tyr Ala Gly Asn Asp Lys Trp Cys Leu Asp Pro Arg Val Val Leu
                80                  85                  90

Leu Ser Asn Thr Gln Thr Gln Tyr Ser Ile Glu Ile Gln Asn Val
                95                  100                 105

Asp Val Tyr Asp Glu Gly Pro Tyr Thr Cys Ser Val Gln Thr Asp
                110                 115                 120

Asn His Pro Lys Thr Ser Arg Val His Leu Ile Val Gln Val Ser
                125                 130                 135

Pro Lys Ile Val Glu Ile Ser Ser Asp Ile Ser Ile Asn Glu Gly
                140                 145                 150

Asn Asn Ile Ser Leu Thr Cys Ile Ala Thr Gly Arg Pro Glu Pro
                155                 160                 165

Thr Val Thr Trp Arg His Ile Ser Pro Lys Ala Val Gly Phe Val
                170                 175                 180

Ser Glu Asp Glu Tyr Leu Glu Ile Gln Gly Ile Thr Arg Glu Gln
                185                 190                 195

Ser Gly Asp Tyr Glu Cys Ser Ala Ser Asn Asp Val Ala Ala Pro
                200                 205                 210

Val Val Arg Arg Val Lys Val Thr Val Asn Tyr Pro Pro Tyr Ile
                215                 220                 225

Ser Glu Ala Lys Gly Thr Gly Val Pro Val Gly Gln Lys Gly Thr
                230                 235                 240

Leu Gln Cys Glu Ala Ser Ala Val Pro Ser Ala Glu Phe Gln Trp
                245                 250                 255

Tyr Lys Asp Asp Lys Arg Leu Ile Glu Gly Lys Lys Gly Val Lys
                260                 265                 270

Val Glu Asn Arg Pro Phe Leu Ser Lys Leu Ile Phe Phe Asn Val
                275                 280                 285

Ser Glu His Asp Tyr Gly Asn Tyr Thr Cys Val Ala Ser Asn Lys
                290                 295                 300

Leu Gly His Thr Asn Ala Ser Ile Met Leu Phe Gly Pro Gly Ala
                305                 310                 315

Val Ser Glu Val Ser Asn Gly Thr Ser Arg Arg Ala Gly Cys Val
                320                 325                 330

Trp Leu Leu Pro Leu Leu Val Leu His Leu Leu Leu Lys Phe
                335                 340

<210> 127
<211> 2236
<212> DNA
<213> Homo Sapien

<400> 127
ggcgccggtg caccgggcgg gctgagcgcc tcctgcggcc cggcctgcgc 50

gccccggccc gccgcgccgc ccacgcccca accccggccc gcgcccccta 100

gcccccgccc gggcccgcgc ccgcgcccgc gcccaggtga gcgctccgcc 150

cgccgcgagg ccccgccccg gcccgccccc gccccgcccc ggccggcggg 200

ggaaccgggc ggattcctcg cgcgtcaaac cacctgatcc cataaaacat 250

tcatcctccc ggcggcccgc gctgcgagcg ccccgccagt ccgcgccgcc 300

gccgccctcg ccctgtgcgc cctgcgcgcc ctgcgcaccc gcggcccgag 350

cccagccaga gccgggcgga gcggagcgcg ccgagcctcg tcccgcggcc 400

gggccggggc cgggccgtag cggcggcgcc tggatgcgga cccggccgcg 450

gggagacggg cgcccgcccc gaaacgactt tcagtccccg acgcgccccg 500

cccaacccct acgatgaaga gggcgtccgc tggagggagc cggctgctgg 550

catgggtgct gtggctgcag gcctggcagg tggcagcccc atgcccaggt 600

gcctgcgtat gctacaatga gcccaaggtg acgacaagct gcccccagca 650

gggcctgcag gctgtgcccg tgggcatccc tgctgccagc cagcgcatct 700

tcctgcacgg caaccgcatc tcgcatgtgc cagctgccag cttccgtgcc 750

tgccgcaacc tcaccatcct gtggctgcac tcgaatgtgc tggcccgaat 800

tgatgcggct gccttcactg gcctggccct cctggagcag ctggacctca 850

gcgataatgc acagctccgg tctgtggacc ctgccacatt ccacggcctg 900

ggccgcctac acacgctgca cctggaccgc tgcggcctgc aggagctggg 950

cccggggctg ttccgcggcc tggctgccct gcagtacctc tacctgcagg 1000

acaacgcgct gcaggcactg cctgatgaca ccttccgcga cctgggcaac 1050

ctcacacacc tcttcctgca cggcaaccgc atctccagcg tgcccgagcg 1100

cgccttccgt gggctgcaca gcctcgaccg tctcctactg caccagaacc 1150

gcgtggccca tgtgcacccg catgccttcc gtgaccttgg ccgcctcatg 1200

acactctatc tgtttgccaa caatctatca gcgctgccca ctgaggccct 1250

ggcccccctg cgtgccctgc agtacctgag gctcaacgac aacccctggg 1300

tgtgtgactg ccgggcacgc ccactctggg cctggctgca gaagttccgc 1350

ggctcctcct ccgaggtgcc ctgcagcctc ccgcaacgcc tggctggccg 1400

tgacctcaaa cgcctagctg ccaatgacct gcagggctgc gctgtggcca 1450

ccggccctta ccatcccatc tggaccggca gggccaccga tgaggagccg 1500

ctggggcttc ccaagtgctg ccagccagat gccgctgaca aggcctcagt 1550

actggagcct ggaagaccag cttcggcagg caatgcgctg aagggacgcg 1600

tgccgcccgg tgacagcccg ccgggcaacg gctctggccc acggcacatc 1650

aatgactcac cctttgggac tctgcctggc tctgctgagc ccccgctcac 1700

tgcagtgcgg cccgagggct ccgagccacc agggttcccc acctcgggcc 1750

ctcgccggag gccaggctgt tcacgcaaga accgcacccg cagccactgc 1800

cgtctgggcc aggcaggcag cgggggtggc gggactggtg actcagaagg 1850

ctcaggtgcc ctacccagcc tcacctgcag cctcaccccc ctgggcctgg 1900

cgctggtgct gtggacagtg cttgggccct gctgaccccc agcggacaca 1950

agagcgtgct cagcagccag gtgtgtgtac atacggggtc tctctccacg 2000

ccgccaagcc agccgggcgg ccgacccgtg gggcaggcca ggccaggtcc 2050

tccctgatgg acgcctgccg cccgccaccc ccatctccac cccatcatgt 2100

ttacagggtt cggcggcagc gtttgttcca gaacgccgcc tcccacccag 2150

atcgcggtat atagagatat gcattttatt ttacttgtgt aaaaatatcg 2200

gacgacgtgg aataaagagc tcttttctta aaaaaa 2236

<210> 128
<211> 473
<212> PRT
<213> Homo Sapien

<400> 128
Met Lys Arg Ala Ser Ala Gly Gly Ser Arg Leu Leu Ala Trp Val
1               5                   10                  15

Leu Trp Leu Gln Ala Trp Gln Val Ala Ala Pro Cys Pro Gly Ala
                20                  25                  30

Cys Val Cys Tyr Asn Glu Pro Lys Val Thr Thr Ser Cys Pro Gln
                35                  40                  45

Gln Gly Leu Gln Ala Val Pro Val Gly Ile Pro Ala Ala Ser Gln
                50                  55                  60

Arg Ile Phe Leu His Gly Asn Arg Ile Ser His Val Pro Ala Ala

65                  70                  75

Ser Phe Arg Ala Cys Arg Asn Leu Thr Ile Leu Trp Leu His Ser
                80                  85                  90

Asn Val Leu Ala Arg Ile Asp Ala Ala Ala Phe Thr Gly Leu Ala
                95                  100                 105

Leu Leu Glu Gln Leu Asp Leu Ser Asp Asn Ala Gln Leu Arg Ser
                110                 115                 120

Val Asp Pro Ala Thr Phe His Gly Leu Gly Arg Leu His Thr Leu
                125                 130                 135

His Leu Asp Arg Cys Gly Leu Gln Glu Leu Gly Pro Gly Leu Phe
                140                 145                 150

Arg Gly Leu Ala Ala Leu Gln Tyr Leu Tyr Leu Gln Asp Asn Ala
                155                 160                 165

Leu Gln Ala Leu Pro Asp Asp Thr Phe Arg Asp Leu Gly Asn Leu
                170                 175                 180

Thr His Leu Phe Leu His Gly Asn Arg Ile Ser Ser Val Pro Glu
                185                 190                 195

Arg Ala Phe Arg Gly Leu His Ser Leu Asp Arg Leu Leu Leu His
                200                 205                 210

Gln Asn Arg Val Ala His Val His Pro His Ala Phe Arg Asp Leu
                215                 220                 225

Gly Arg Leu Met Thr Leu Tyr Leu Phe Ala Asn Asn Leu Ser Ala
                230                 235                 240

Leu Pro Thr Glu Ala Leu Ala Pro Leu Arg Ala Leu Gln Tyr Leu
                245                 250                 255

Arg Leu Asn Asp Asn Pro Trp Val Cys Asp Cys Arg Ala Arg Pro
                260                 265                 270

Leu Trp Ala Trp Leu Gln Lys Phe Arg Gly Ser Ser Ser Glu Val
                275                 280                 285

Pro Cys Ser Leu Pro Gln Arg Leu Ala Gly Arg Asp Leu Lys Arg
                290                 295                 300

Leu Ala Ala Asn Asp Leu Gln Gly Cys Ala Val Ala Thr Gly Pro
                305                 310                 315

Tyr His Pro Ile Trp Thr Gly Arg Ala Thr Asp Glu Glu Pro Leu
                320                 325                 330

Gly Leu Pro Lys Cys Cys Gln Pro Asp Ala Ala Asp Lys Ala Ser
                335                 340                 345

Val Leu Glu Pro Gly Arg Pro Ala Ser Ala Gly Asn Ala Leu Lys
                350                 355                 360

Gly Arg Val Pro Pro Gly Asp Ser Pro Pro Gly Asn Gly Ser Gly
                365                 370                 375

Pro Arg His Ile Asn Asp Ser Pro Phe Gly Thr Leu Pro Gly Ser
                380                 385                 390

Ala Glu Pro Pro Leu Thr Ala Val Arg Pro Glu Gly Ser Glu Pro
                395                 400                 405

Pro Gly Phe Pro Thr Ser Gly Pro Arg Arg Arg Pro Gly Cys Ser
                410                 415                 420

Arg Lys Asn Arg Thr Arg Ser His Cys Arg Leu Gly Gln Ala Gly
                425                 430                 435

Ser Gly Gly Gly Gly Thr Gly Asp Ser Glu Gly Ser Gly Ala Leu
                440                 445                 450

Pro Ser Leu Thr Cys Ser Leu Thr Pro Leu Gly Leu Ala Leu Val
                455                 460                 465

Leu Trp Thr Val Leu Gly Pro Cys
                470

<210> 129
<211> 2458
<212> DNA
<213> Homo Sapien

<400> 129
gcgccgggag cccatctgcc cccaggggca cggggcgcgg ggccggctcc 50

cgcccggcac atggctgcag ccacctcgcg cgcaccccga ggcgccgcgc 100

ccagctcgcc cgaggtccgt cggaggcgcc cggccgcccc ggagccaagc 150

agcaactgag cggggaagcg cccgcgtccg gggatcggga tgtccctcct 200

ccttctcctc ttgctagttt cctactatgt tggaaccttg gggactcaca 250

ctgagatcaa gagagtggca gaggaaaagg tcactttgcc ctgccaccat 300

caactggggc ttccagaaaa agacactctg gatattgaat ggctgctcac 350

cgataatgaa gggaaccaaa aagtggtgat cacttactcc agtcgtcatg 400

tctacaataa cttgactgag gaacagaagg gccgagtggc ctttgcttcc 450

aatttcctgg caggagatgc ctccttgcag attgaacctc tgaagcccag 500

tgatgagggc cggtacacct gtaaggttaa gaattcaggg cgctacgtgt 550

ggagccatgt catcttaaaa gtcttagtga gaccatccaa gcccaagtgt 600

gagttggaag gagagctgac agaaggaagt gacctgactt tgcagtgtga 650

gtcatcctct ggcacagagc ccattgtgta ttactggcag cgaatccgag 700

agaaagaggg agaggatgaa cgtctgcctc ccaaatctag gattgactac 750

aaccaccctg gacgagttct gctgcagaat cttaccatgt cctactctgg 800

actgtaccag tgcacagcag gcaacgaagc tgggaaggaa agctgtgtgg 850

tgcgagtaac tgtacagtat gtacaaagca tcggcatggt tgcaggagca 900

gtgacaggca tagtggctgg agccctgctg attttcctct tggtgtggct 950

gctaatccga aggaaagaca aagaaagata tgaggaagaa gagagaccta 1000

atgaaattcg agaagatgct gaagctccaa aagcccgtct tgtgaaaccc 1050

agctcctctt cctcaggctc tcggagctca cgctctggtt cttcctccac 1100

tcgctccaca gcaaatagtg cctcacgcag ccagcggaca ctgtcaactg 1150

acgcagcacc ccagccaggg ctggccaccc aggcatacag cctagtgggg 1200

ccagaggtga gaggttctga accaaagaaa gtccaccatg ctaatctgac 1250

caaagcagaa accacaccca gcatgatccc cagccagagc agagccttcc 1300

aaacggtctg aattacaatg gacttgactc ccacgctttc ctaggagtca 1350

gggtctttgg actcttctcg tcattggagc tcaagtcacc agccacacaa 1400

ccagatgaga ggtcatctaa gtagcagtga gcattgcacg gaacagattc 1450

agatgagcat tttccttata caataccaaa caagcaaaag gatgtaagct 1500

gattcatctg taaaaaggca tcttattgtg cctttagacc agagtaaggg 1550

aaagcaggag tccaaatcta tttgttgacc aggacctgtg gtgagaaggt 1600

tggggaaagg tgaggtgaat atacctaaaa cttttaatgt gggatatttt 1650

gtatcagtgc tttgattcac aattttcaag aggaaatggg atgctgtttg 1700

taaattttct atgcatttct gcaaacttat tggattatta gttattcaga 1750

cagtcaagca gaacccacag ccttattaca cctgtctaca ccatgtactg 1800

agctaaccac ttctaagaaa ctccaaaaaa ggaaacatgt gtcttctatt 1850

ctgacttaac ttcatttgtc ataaggtttg gatattaatt tcaaggggag 1900

ttgaaatagt gggagatgga gaagagtgaa tgagtttctc ccactctata 1950

ctaatctcac tatttgtatt gagcccaaaa taactatgaa aggagacaaa 2000

aatttgtgac aaaggattgt gaagagcttt ccatcttcat gatgttatga 2050

ggattgttga caaacattag aaatatataa tggagcaatt gtggatttcc 2100

cctcaaatca gatgcctcta aggactttcc tgctagatat ttctggaagg 2150

agaaaataca acatgtcatt tatcaacgtc cttagaaaga attcttctag 2200

agaaaaaggg atctaggaat gctgaaagat tacccaacat accattatag 2250

tctcttcttt ctgagaaaat gtgaaaccag aattgcaaga ctgggtggac 2300

tagaaaggga gattagatca gttttctctt aatatgtcaa ggaaggtagc 2350

cgggcatggt gccaggcacc tgtaggaaaa tccagcaggt ggaggttgca 2400

gtgagccgag attatgccat tgcactccag cctgggtgac agagcgggac 2450

tccgtctc 2458

<210> 130
<211> 373
<212> PRT
<213> Homo Sapien

<400> 130
Met Ser Leu Leu Leu Leu Leu Leu Leu Val Ser Tyr Tyr Val Gly
1               5                   10                  15

Thr Leu Gly Thr His Thr Glu Ile Lys Arg Val Ala Glu Glu Lys
                20                  25                  30

Val Thr Leu Pro Cys His His Gln Leu Gly Leu Pro Glu Lys Asp
                35                  40                  45

Thr Leu Asp Ile Glu Trp Leu Leu Thr Asp Asn Glu Gly Asn Gln
                50                  55                  60

Lys Val Val Ile Thr Tyr Ser Ser Arg His Val Tyr Asn Asn Leu
                65                  70                  75

Thr Glu Glu Gln Lys Gly Arg Val Ala Phe Ala Ser Asn Phe Leu
                80                  85                  90

Ala Gly Asp Ala Ser Leu Gln Ile Glu Pro Leu Lys Pro Ser Asp
                95                 100                 105

Glu Gly Arg Tyr Thr Cys Lys Val Lys Asn Ser Gly Arg Tyr Val
               110                 115                 120

Trp Ser His Val Ile Leu Lys Val Leu Val Arg Pro Ser Lys Pro
               125                 130                 135

Lys Cys Glu Leu Glu Gly Glu Leu Thr Glu Gly Ser Asp Leu Thr
               140                 145                 150

Leu Gln Cys Glu Ser Ser Ser Gly Thr Glu Pro Ile Val Tyr Tyr
               155                 160                 165

Trp Gln Arg Ile Arg Glu Lys Glu Gly Glu Asp Glu Arg Leu Pro
               170                 175                 180

Pro Lys Ser Arg Ile Asp Tyr Asn His Pro Gly Arg Val Leu Leu
               185                 190                 195

Gln Asn Leu Thr Met Ser Tyr Ser Gly Leu Tyr Gln Cys Thr Ala
               200                 205                 210

Gly Asn Glu Ala Gly Lys Glu Ser Cys Val Val Arg Val Thr Val
               215                 220                 225

Gln Tyr Val Gln Ser Ile Gly Met Val Ala Gly Ala Val Thr Gly
               230                 235                 240

Ile Val Ala Gly Ala Leu Leu Ile Phe Leu Leu Val Trp Leu Leu
               245                 250                 255

Ile Arg Arg Lys Asp Lys Glu Arg Tyr Glu Glu Glu Glu Arg Pro
               260                 265                 270

Asn Glu Ile Arg Glu Asp Ala Glu Ala Pro Lys Ala Arg Leu Val
               275                 280                 285

Lys Pro Ser Ser Ser Ser Ser Gly Ser Arg Ser Ser Arg Ser Gly
               290                 295                 300

Ser Ser Ser Thr Arg Ser Thr Ala Asn Ser Ala Ser Arg Ser Gln
               305                 310                 315

Arg Thr Leu Ser Thr Asp Ala Ala Pro Gln Pro Gly Leu Ala Thr
               320                 325                 330

Gln Ala Tyr Ser Leu Val Gly Pro Glu Val Arg Gly Ser Glu Pro
               335                 340                 345

Lys Lys Val His His Ala Asn Leu Thr Lys Ala Glu Thr Thr Pro
               350                 355                 360

Ser Met Ile Pro Ser Gln Ser Arg Ala Phe Gln Thr Val
               365                 370

<210> 131
<211> 2738

<212> DNA
<213> Homo Sapien

<400> 131
ggaagtccac ggggagcttg gatgccaaag ggaggacggc tgggtcctct 50

ggagaggact actcactggc atatttctga ggtatctgta gaataaccac 100

agcctcagat actggggact ttacagtccc acagaaccgt cctcccagga 150

agctgaatcc agcaagaaca atggaggcca gcgggaagct catttgcaga 200

caaaggcaag tccttttttc ctttctcctt ttgggcttat ctctggcggg 250

cgcggcggaa cctagaagct attctgtggt ggaggaaact gagggcagct 300

cctttgtcac caatttagca aaggacctgg gtctggagca gagggaattc 350

tccaggcggg gggttagggt tgtttccaga gggaacaaac tacatttgca 400

gctcaatcag gagaccgcgg atttgttgct aaatgagaaa ttggaccgtg 450

aggatctgtg cggtcacaca gagccctgtg tgctacgttt ccaagtgttg 500

ctagagagtc ccttcgagtt ttttcaagct gagctgcaag taatagacat 550

aaacgaccac tctccagtat ttctggacaa acaaatgttg gtgaaagtat 600

cagagagcag tcctcctggg actacgtttc ctctgaagaa tgccgaagac 650

ttagatgtag gccaaaacaa tattgagaac tatataatca gccccaactc 700

ctattttcgg gtcctcaccc gcaaacgcag tgatggcagg aaatacccag 750

agctggtgct ggacaaagcg ctggaccgag aggaagaagc tgagctcagg 800

ttaacactca cagcactgga tggtggctct ccgcccagat ctggcactgc 850

tcaggtctac atcgaagtcc tggatgtcaa cgataatgcc cctgaatttg 900

agcagccttt ctatagagtg cagatctctg aggacagtcc ggtaggcttc 950

ctggttgtga aggtctctgc cacggatgta gacacaggag tcaacggaga 1000

gatttcctat tcacttttcc aagcttcaga agagattggc aaaaccttta 1050

agatcaatcc cttgacagga gaaattgaac taaaaaaaca actcgatttc 1100

gaaaaacttc agtcctatga agtcaatatt gaggcaagag atgctggaac 1150

cttttctgga aaatgcaccg ttctgattca agtgatagat gtgaacgacc 1200

atgccccaga agttaccatg tctgcattta ccagcccaat acctgagaac 1250

gcgcctgaaa ctgtggttgc acttttcagt gtttcagatc ttgattcagg 1300

agaaaatggg aaaattagtt gctccattca ggaggatcta cccttcctcc 1350

tgaaatccgc ggaaaacttt tacaccctac taacggagag accactagac 1400

agagaaagca gagcggaata caacatcact atcactgtca ctgacttggg 1450

gacccctatg ctgataacac agctcaatat gaccgtgctg atcgccgatg 1500

tcaatgacaa cgctcccgcc ttcacccaaa cctcctacac cctgttcgtc 1550

cgcgagaaca acagccccgc cctgcacatc cgcagcgtca gcgctacaga 1600

cagagactca ggcaccaacg cccaggtcac ctactcgctg ctgccgcccc 1650

aggacccgca cctgcccctc acatccctgg tctccatcaa cgcggacaac 1700

ggccacctgt tcgccctcag gtctctggac tacgaggccc tgcaggggtt 1750

ccagttccgc gtgggcgctt cagaccacgg ctccccggcg ctgagcagcg 1800

aggcgctggt gcgcgtggtg gtgctggacg ccaacgacaa ctcgcccttc 1850

gtgctgtacc cgctgcagaa cggctccgcg ccctgcaccg agctggtgcc 1900

ccgggcggcc gagccgggct acctggtgac caaggtggtg gcggtggacg 1950

gcgactcggg ccagaacgcc tggctgtcgt accagctgct caaggccacg 2000

gagctcggtc tgttcggcgt gtgggcgcac aatggcgagg tgcgcaccgc 2050

caggctgctg agcgagcgcg acgcggccaa gcacaggctg gtggtgctgg 2100

tcaaggacaa tggcgagcct ccgcgctcgg ccaccgccac gctgcacgtg 2150

ctcctggtgg acggcttctc ccagccctac ctgcctctcc cggaggcggc 2200

cccgacccag gcccaggccg acttgctcac cgtctacctg gtggtggcgt 2250

tggcctcggt gtcttcgctc ttcctctttt cggtgctcct gttcgtggcg 2300

gtgcggctgt gtaggaggag cagggcggcc tcggtgggtc gctgcttggt 2350

gcccgagggc ccccttccag ggcatcttgt ggacatgagc ggcaccagga 2400

ccctatccca gagctaccag tatgaggtgt gtctggcagg aggctcaggg 2450

accaatgagt tcaagttcct gaagccgatt atccccaact tccctcccca 2500

gtgccctggg aaagaaatac aaggaaattc taccttcccc aataactttg 2550

ggttcaatat tcagtgacca tagttgactt ttacattcca taggtatttt 2600

attttgtggc atttccatgc caatgtttat ttcccccaat ttgtgtgtat 2650

gtaatattgt acggatttac tcttgatttt tctcatgttc tttctccctt 2700

tgttttaaag tgaacattta cctttattcc tggttctt 2738

<210> 132
<211> 798
<212> PRT
<213> Homo Sapien

<400> 132
Met Glu Ala Ser Gly Lys Leu Ile Cys Arg Gln Arg Gln Val Leu
1               5                   10                  15

Phe Ser Phe Leu Leu Leu Gly Leu Ser Leu Ala Gly Ala Ala Glu
                20                  25                  30

Pro Arg Ser Tyr Ser Val Val Glu Glu Thr Glu Gly Ser Ser Phe
                35                  40                  45

Val Thr Asn Leu Ala Lys Asp Leu Gly Leu Glu Gln Arg Glu Phe
                50                  55                  60

Ser Arg Arg Gly Val Arg Val Val Ser Arg Gly Asn Lys Leu His
                65                  70                  75

Leu Gln Leu Asn Gln Glu Thr Ala Asp Leu Leu Leu Asn Glu Lys

80                  85                  90

Leu Asp Arg Glu Asp Leu Cys Gly His Thr Glu Pro Cys Val Leu
                95                  100                 105

Arg Phe Gln Val Leu Leu Glu Ser Pro Phe Glu Phe Phe Gln Ala
                110                 115                 120

Glu Leu Gln Val Ile Asp Ile Asn Asp His Ser Pro Val Phe Leu
                125                 130                 135

Asp Lys Gln Met Leu Val Lys Val Ser Glu Ser Ser Pro Pro Gly
                140                 145                 150

Thr Thr Phe Pro Leu Lys Asn Ala Glu Asp Leu Asp Val Gly Gln
                155                 160                 165

Asn Asn Ile Glu Asn Tyr Ile Ile Ser Pro Asn Ser Tyr Phe Arg
                170                 175                 180

Val Leu Thr Arg Lys Arg Ser Asp Gly Arg Lys Tyr Pro Glu Leu
                185                 190                 195

Val Leu Asp Lys Ala Leu Asp Arg Glu Glu Glu Ala Glu Leu Arg
                200                 205                 210

Leu Thr Leu Thr Ala Leu Asp Gly Gly Ser Pro Pro Arg Ser Gly
                215                 220                 225

Thr Ala Gln Val Tyr Ile Glu Val Leu Asp Val Asn Asp Asn Ala
                230                 235                 240

Pro Glu Phe Glu Gln Pro Phe Tyr Arg Val Gln Ile Ser Glu Asp
                245                 250                 255

Ser Pro Val Gly Phe Leu Val Val Lys Val Ser Ala Thr Asp Val
                260                 265                 270

Asp Thr Gly Val Asn Gly Glu Ile Ser Tyr Ser Leu Phe Gln Ala
                275                 280                 285

Ser Glu Glu Ile Gly Lys Thr Phe Lys Ile Asn Pro Leu Thr Gly
                290                 295                 300

Glu Ile Glu Leu Lys Lys Gln Leu Asp Phe Glu Lys Leu Gln Ser
                305                 310                 315

Tyr Glu Val Asn Ile Glu Ala Arg Asp Ala Gly Thr Phe Ser Gly
                320                 325                 330

Lys Cys Thr Val Leu Ile Gln Val Ile Asp Val Asn Asp His Ala
                335                 340                 345

Pro Glu Val Thr Met Ser Ala Phe Thr Ser Pro Ile Pro Glu Asn
                350                 355                 360

Ala Pro Glu Thr Val Val Ala Leu Phe Ser Val Ser Asp Leu Asp
                365                 370                 375

Ser Gly Glu Asn Gly Lys Ile Ser Cys Ser Ile Gln Glu Asp Leu
                380                 385                 390

Pro Phe Leu Leu Lys Ser Ala Glu Asn Phe Tyr Thr Leu Leu Thr
                395                 400                 405

Glu Arg Pro Leu Asp Arg Glu Ser Arg Ala Glu Tyr Asn Ile Thr
                410                 415                 420

Ile Thr Val Thr Asp Leu Gly Thr Pro Met Leu Ile Thr Gln Leu
                425                 430                 435

Asn Met Thr Val Leu Ile Ala Asp Val Asn Asp Asn Ala Pro Ala
                440                 445                 450

Phe Thr Gln Thr Ser Tyr Thr Leu Phe Val Arg Glu Asn Asn Ser
                455                 460                 465

Pro Ala Leu His Ile Arg Ser Val Ser Ala Thr Asp Arg Asp Ser
                470                 475                 480

Gly Thr Asn Ala Gln Val Thr Tyr Ser Leu Leu Pro Pro Gln Asp
                485                 490                 495

Pro His Leu Pro Leu Thr Ser Leu Val Ser Ile Asn Ala Asp Asn
                500                 505                 510

Gly His Leu Phe Ala Leu Arg Ser Leu Asp Tyr Glu Ala Leu Gln
                515                 520                 525

Gly Phe Gln Phe Arg Val Gly Ala Ser Asp His Gly Ser Pro Ala
                530                 535                 540

Leu Ser Ser Glu Ala Leu Val Arg Val Val Val Leu Asp Ala Asn
                545                 550                 555

Asp Asn Ser Pro Phe Val Leu Tyr Pro Leu Gln Asn Gly Ser Ala
                560                 565                 570

Pro Cys Thr Glu Leu Val Pro Arg Ala Ala Glu Pro Gly Tyr Leu
                575                 580                 585

Val Thr Lys Val Val Ala Val Asp Gly Asp Ser Gly Gln Asn Ala
                590                 595                 600

Trp Leu Ser Tyr Gln Leu Leu Lys Ala Thr Glu Leu Gly Leu Phe
                605                 610                 615

Gly Val Trp Ala His Asn Gly Glu Val Arg Thr Ala Arg Leu Leu
                620                 625                 630

Ser Glu Arg Asp Ala Ala Lys His Arg Leu Val Val Leu Val Lys
                635                 640                 645

Asp Asn Gly Glu Pro Pro Arg Ser Ala Thr Ala Thr Leu His Val
                650                 655                 660

Leu Leu Val Asp Gly Phe Ser Gln Pro Tyr Leu Pro Leu Pro Glu
                665                 670                 675

Ala Ala Pro Thr Gln Ala Gln Ala Asp Leu Leu Thr Val Tyr Leu
                680                 685                 690

Val Val Ala Leu Ala Ser Val Ser Ser Leu Phe Leu Phe Ser Val
                695                 700                 705

Leu Leu Phe Val Ala Val Arg Leu Cys Arg Arg Ser Arg Ala Ala
                710                 715                 720

Ser Val Gly Arg Cys Leu Val Pro Glu Gly Pro Leu Pro Gly His
                725                 730                 735

Leu Val Asp Met Ser Gly Thr Arg Thr Leu Ser Gln Ser Tyr Gln
                740                 745                 750

Tyr Glu Val Cys Leu Ala Gly Gly Ser Gly Thr Asn Glu Phe Lys
                755                 760                 765

Phe Leu Lys Pro Ile Ile Pro Asn Phe Pro Pro Gln Cys Pro Gly
                770                 775                 780

Lys Glu Ile Gln Gly Asn Ser Thr Phe Pro Asn Asn Phe Gly Phe
                785                 790                 795

Asn Ile Gln

<210> 133
<211> 3819
<212> DNA
<213> Homo Sapien

<400> 133
ggaaggggag gagcaggcca cacaggcaca ggccggtgag ggacctgccc 50

agacctggag ggtctcgctc tgtcacacag gctggagtgc agtggtgtga 100

tcttggctca tcgtaacctc cacctcccgg gttcaagtga ttctcatgcc 150

tcagcctccc gagtagctgg gattacaggt ggtgacttcc aagagtgact 200

ccgtcggagg aaaatgactc cccagtcgct gctgcagacg acactgttcc 250

tgctgagtct gctcttcctg gtccaaggtg cccacggcag gggccacagg 300

gaagactttc gcttctgcag ccagcggaac cagacacaca ggagcagcct 350

ccactacaaa cccacaccag acctgcgcat ctccatcgag aactccgaag 400

aggccctcac agtccatgcc cctttccctg cagcccaccc tgcttcccga 450

tccttccctg accccagggg cctctaccac ttctgcctct actggaaccg 500

acatgctggg agattacatc ttctctatgg caagcgtgac ttcttgctga 550

gtgacaaagc ctctagcctc ctctgcttcc agcaccagga ggagagcctg 600

gctcagggcc ccccgctgtt agccacttct gtcacctcct ggtggagccc 650

tcagaacatc agcctgccca gtgccgccag cttcaccttc tccttccaca 700

gtcctcccca cacggccgct cacaatgcct cggtggacat gtgcgagctc 750

aaaagggacc tccagctgct cagccagttc ctgaagcatc cccagaaggc 800

ctcaaggagg ccctcggctg cccccgccag ccagcagttg cagagcctgg 850

agtcgaaact gacctctgtg agattcatgg gggacatggt gtccttcgag 900

gaggaccgga tcaacgccac ggtgtggaag ctccagccca cagccggcct 950

ccaggacctg cacatccact cccggcagga ggaggagcag agcgagatca 1000

tggagtactc ggtgctgctg cctcgaacac tcttccagag gacgaaaggc 1050

cggagcgggg aggctgagaa gagactcctc ctggtggact tcagcagcca 1100

agccctgttc caggacaaga attccagcca agtcctgggt gagaaggtct 1150

tggggattgt ggtacagaac accaaagtag ccaacctcac ggagcccgtg 1200

gtgctcactt tccagcacca gctacagccg aagaatgtga ctctgcaatg 1250

tgtgttctgg gttgaagacc ccacattgag cagcccgggg cattggagca 1300

gtgctgggtg tgagaccgtc aggagagaaa cccaaacatc ctgcttctgc 1350

aaccacttga cctactttgc agtgctgatg gtctcctcgg tggaggtgga 1400

cgccgtgcac aagcactacc tgagcctcct ctcctacgtg ggctgtgtcg 1450

tctctgccct ggcctgcctt gtcaccattg ccgcctacct ctgctccagg 1500

gtgcccctgc cgtgcaggag gaaacctcgg gactacacca tcaaggtgca 1550

catgaacctg ctgctggccg tcttcctgct ggacacgagc ttcctgctca 1600

gcgagccggt ggccctgaca ggctctgagg ctggctgccg agccagtgcc 1650

atcttcctgc acttctccct gctcacctgc ctttcctgga tgggcctcga 1700

ggggtacaac ctctaccgac tcgtggtgga ggtctttggc acctatgtcc 1750

ctggctacct actcaagctg agcgccatgg gctggggctt ccccatcttt 1800

ctggtgacgc tggtggccct ggtggatgtg gacaactatg gccccatcat 1850

cttggctgtg cataggactc cagagggcgt catctaccct tccatgtgct 1900

ggatccggga ctccctggtc agctacatca ccaacctggg cctcttcagc 1950

ctggtgtttc tgttcaacat ggccatgcta gccaccatgg tggtgcagat 2000

cctgcggctg cgcccccaca cccaaaagtg gtcacatgtg ctgacactgc 2050

tgggcctcag cctggtcctt ggcctgccct gggccttgat cttcttctcc 2100

tttgcttctg gcaccttcca gcttgtcgtc ctctaccttt tcagcatcat 2150

cacctccttc caaggcttcc tcatcttcat ctggtactgg tccatgcggc 2200

tgcaggcccg ggggtggccc tcccctctga agagcaactc agacagcgcc 2250

aggctcccca tcagctcggg cagcacctcg tccagccgca tctaggcctc 2300

cagcccacct gcccatgtga tgaagcagag atgcggcctc gtcgcacact 2350

gcctgtggcc cccgagccag gcccagcccc aggccagtca gccgcagact 2400

ttggaaagcc caacgaccat ggagagatgg gccgttgcca tggtggacgg 2450

actcccgggc tgggcttttg aattggcctt ggggactact cggctctcac 2500

tcagctccca cgggactcag aagtgcgccg ccatgctgcc tagggtactg 2550

tccccacatc tgtcccaacc cagctggagg cctggtctct ccttacaacc 2600

cctgggccca gccctcattg ctgggggcca ggccttggat cttgagggtc 2650

tggcacatcc ttaatcctgt gcccctgcct gggacagaaa tgtggctcca 2700

gttgctctgt ctctcgtggt caccctgagg gcactctgca tcctctgtca 2750

ttttaacctc aggtggcacc cagggcgaat ggggcccagg gcagaccttc 2800

agggccagag ccctggcgga ggagaggccc tttgccagga gcacagcagc 2850

agctcgccta cctctgagcc caggccccct ccctccctca gccccccagt 2900

cctccctcca tcttccctgg ggttctcctc ctctcccagg gcctccttgc 2950

tccttcgttc acagctgggg gtccccgatt ccaatgctgt tttttgggga 3000

gtggtttcca ggagctgcct ggtgtctgct gtaaatgttt gtctactgca 3050

caagcctcgg cctgcccctg agccaggctc ggtaccgatg cgtgggctgg 3100

gctaggtccc tctgtccatc tgggcctttg tatgagctgc attgcccttg 3150

ctcaccctga ccaagcacac gcctcagagg ggccctcagc ctctcctgaa 3200

gccctcttgt ggcaagaact gtggaccatg ccagtcccgt ctggtttcca 3250

tcccaccact ccaaggactg agactgacct cctctggtga cactggccta 3300

gagcctgaca ctctcctaag aggttctctc caagccccca aatagctcca 3350

ggcgccctcg gccgcccatc atggttaatt ctgtccaaca aacacacacg 3400

ggtagattgc tggcctgttg taggtggtag ggacacagat gaccgacctg 3450

gtcactcctc ctgccaacat tcagtctggt atgtgaggcg tgcgtgaagc 3500

aagaactcct ggagctacag ggacagggag ccatcattcc tgcctgggaa 3550

tcctggaaga cttcctgcag gagtcagcgt tcaatcttga ccttgaagat 3600

gggaaggatg ttctttttac gtaccaattc ttttgtcttt tgatattaaa 3650

aagaagtaca tgttcattgt agagaatttg gaaactgtag aagagaatca 3700

agaagaaaaa taaaaatcag ctgttgtaat cgcctagcaa aaaaaaaaaa 3750

aaaaaaaaaa aaaaaaaaaa aaaaaaaaaa aaaaaaaaaa aaaaaaaaaa 3800

aaaaaaaaaa aaaaaaaaa 3819

<210> 134
<211> 693
<212> PRT
<213> Homo Sapien

<400> 134

<210> 135
<211> 1615
<212> DNA
<213> Homo Sapien

<400> 135
gcctagccag gccaagaatg caattgcccc ggtggtggga gctgggagac 50

ccctgtgctt ggacgggaca gggtcggggg acacgcagga tgagccccgc 100

gaccactggc acattcttgc tgacagtgta cagtattttc tccaaggtac 150

actccgatcg gaatgtatac ccatcagcag gtgtcctctt tgttcatgtt 200

ttggaaagag aatattttaa gggggaattt ccaccttacc caaaacctgg 250

cgagattagt aatgatccca taacatttaa tacaaattta atgggttacc 300

cagaccgacc tggatggctt cgatatatcc aaaggacacc atatagtgat 350

ggagtcctat atgggtcccc aacagctgaa aatgtgggga agccaacaat 400

cattgagata actgcctaca acaggcgcac ctttgagact gcaaggcata 450

atttgataat taatataatg tctgcagaag acttcccgtt gccatatcaa 500

gcagaattct tcattaagaa tatgaatgta gaagaaatgt tggccagtga 550

ggttcttgga gactttcttg gcgcagtgaa aaatgtgtgg cagccagagc 600

gcctgaacgc cataaacatc acatcggccc tagacagggg tggcagggtg 650

ccacttccca ttaatgacct gaaggagggc gtttatgtca tggttggtgc 700

agatgtcccg ttttcttctt gtttacgaga agttgaaaat ccacagaatc 750

aattgagatg tagtcaagaa atggagcctg taataacatg tgataaaaaa 800

tttcgtactc aattttacat tgactggtgc aaaatttcat tggttgataa 850

aacaaagcaa gtgtccacct atcaggaagt gattcgtgga gaggggattt 900

tacctgatgg tggagaatac aaaccccctt ctgattcttt gaaaagcaga 950

gactattaca cggatttcct aattacactg gctgtgccct cggcagtggc 1000

actggtcctt tttctaatac ttgcttatat catgtgctgc cgacgggaag 1050

gcgtggaaaa gagaaacatg caaacaccag acatccaact ggtccatcac 1100

agtgctattc agaaatctac caaggagctt cgagacatgt ccaagaatag 1150

agagatagca tggcccctgt caacgcttcc tgtgttccac cctgtgactg 1200

gggaaatcat acctccttta cacacagaca actatgatag cacaaacatg 1250

ccattgatgc aaacgcagca gaacttgcca catcagactc agattcccca 1300

acagcagact acaggtaaat ggtatccctg aagaaagaaa actgactgaa 1350

gcaatgaatt tataatcaga caatatagca gttacatcac atttcttttc 1400

tcttccaata atgcatgagc ttttctggca tatgttatgc atgttggcag 1450

tattaagtgt ataccaaata atacaacata actttcattt tactaatgta 1500

tttttttgta cttaaagcat ttttgacaat ttgtaaaaca ttgatgactt 1550

tatatttgtt acaataaaag ttgatcttta aaataaatat tattaatgaa 1600

gcctaaaaaa aaaaa 1615

<210> 136
<211> 437
<212> PRT
<213> Homo Sapien

<400> 136

<210> 137
<211> 1621
<212> DNA
<213> Homo Sapien

<400> 137
cagaagaggg ggctagctag ctgtctctgc ggaccaggga gacccccgcg 50

cccccccggt gtgaggcggc ctcacagggc cgggtgggct ggcgagccga 100

cgcggcggcg gaggaggctg tgaggagtgt gtggaacagg acccgggaca 150

gaggaaccat ggctccgcag aacctgagca ccttttgcct gttgctgcta 200

tacctcatcg gggcggtgat tgccggacga gatttctata agatcttggg 250

ggtgcctcga agtgcctcta taaaggatat taaaaaggcc tataggaaac 300

tagccctgca gcttcatccc gaccggaacc ctgatgatcc acaagcccag 350

gagaaattcc aggatctggg tgctgcttat gaggttctgt cagatagtga 400

gaaacggaaa cagtacgata cttatggtga agaaggatta aaagatggtc 450

atcagagctc ccatggagac attttttcac acttctttgg ggattttggt 500

ttcatgtttg gaggaacccc tcgtcagcaa gacagaaata ttccaagagg 550

aagtgatatt attgtagatc tagaagtcac tttggaagaa gtatatgcag 600

gaaattttgt ggaagtagtt agaaacaaac ctgtggcaag gcaggctcct 650

ggcaaacgga agtgcaattg tcggcaagag atgcggacca cccagctggg 700

ccctgggcgc ttccaaatga cccaggaggt ggtctgcgac gaatgcccta 750

atgtcaaact agtgaatgaa gaacgaacgc tggaagtaga aatagagcct 800

ggggtgagag acggcatgga gtaccccttt attggagaag gtgagcctca 850

cgtggatggg gagcctggag atttacggtt ccgaatcaaa gttgtcaagc 900

acccaatatt tgaaaggaga ggagatgatt tgtacacaaa tgtgacaatc 950

tcattagttg agtcactggt tggctttgag atggatatta ctcacttgga 1000

tggtcacaag gtacatattt cccgggataa gatcaccagg ccaggagcga 1050

agctatggaa gaaaggggaa gggctcccca actttgacaa caacaatatc 1100

aagggctctt tgataatcac ttttgatgtg gattttccaa aagaacagtt 1150

aacagaggaa gcgagagaag gtatcaaaca gctactgaaa caagggtcag 1200

tgcagaaggt atacaatgga ctgcaaggat attgagagtg aataaaattg 1250

gactttgttt aaaataagtg aataagcgat atttattatc tgcaaggttt 1300

ttttgtgtgt gtttttgttt ttattttcaa tatgcaagtt aggcttaatt 1350

tttttatcta atgatcatca tgaaatgaat aagagggctt aagaatttgt 1400

ccatttgcat tcggaaaaga atgaccagca aaaggtttac taatacctct 1450

ccctttgggg atttaatgtc tggtgctgcc gcctgagttt caagaattaa 1500

agctgcaaga ggactccagg agcaaaagaa acacaatata gagggttgga 1550

gttgttagca atttcattca aaatgccaac tggagaagtc tgtttttaaa 1600

tacattttgt tgttattttt a 1621

<210> 138
<211> 358
<212> PRT
<213> Homo Sapien

<400> 138

<210> 139
<211> 550
<212> DNA
<213> Homo Sapien

<400> 139
ccagtctgtc gccacctcac ttggtgtctg ctgtccccgc caggcaagcc 50

tggggtgaga gcacagagga gtgggccggg accatgcggg ggacgcggct 100

ggcgctcctg gcgctggtgc tggctgcctg cggagagctg gcgccggccc 150

tgcgctgcta cgtctgtccg gagcccacag gagtgtcgga ctgtgtcacc 200

atcgccacct gcaccaccaa cgaaaccatg tgcaagacca cactctactc 250

ccgggagata gtgtacccct tccaggggga ctccacggtg accaagtcct 300

gtgccagcaa gtgtaagccc tcggatgtgg atggcatcgg ccagaccctg 350

cccgtgtcct gctgcaatac tgagctgtgc aatgtagacg gggcgcccgc 400

tctgaacagc ctccactgcg gggccctcac gctcctccca ctcttgagcc 450

tccgactgta gagtccccgc ccacccccat ggccctatgc ggcccagccc 500

cgaatgcctt gaagaagtgc cccctgcacc aggaaaaaaa aaaaaaaaaa 550

<210> 140
<211> 125
<212> PRT
<213> Homo Sapien

<400> 140
Met Arg Gly Thr Arg Leu Ala Leu Leu Ala Leu Val Leu Ala Ala
  1               5                  10                  15

Cys Gly Glu Leu Ala Pro Ala Leu Arg Cys Tyr Val Cys Pro Glu
                 20                  25                  30

Pro Thr Gly Val Ser Asp Cys Val Thr Ile Ala Thr Cys Thr Thr
                 35                  40                  45

Asn Glu Thr Met Cys Lys Thr Thr Leu Tyr Ser Arg Glu Ile Val
                 50                  55                  60

Tyr Pro Phe Gln Gly Asp Ser Thr Val Thr Lys Ser Cys Ala Ser
                65                  70                  75

Lys Cys Lys Pro Ser Asp Val Asp Gly Ile Gly Gln Thr Leu Pro
                80                  85                  90

Val Ser Cys Cys Asn Thr Glu Leu Cys Asn Val Asp Gly Ala Pro
                95                  100                 105

Ala Leu Asn Ser Leu His Cys Gly Ala Leu Thr Leu Leu Pro Leu
                110                 115                 120

Leu Ser Leu Arg Leu
                125

<210> 141
<211> 1524
<212> DNA
<213> Homo Sapien

<400> 141
ggcgccgcgt aggcccggga ggccgggccg gccgggctgc gagcgcctgc 50

cccatgcgcc gccgcctctc cgcacgatgt tcccctcgcg gaggaaagcg 100

gcgcagctgc cctgggagga cggcaggtcc gggttgctct ccggcggcct 150

ccctcggaag tgttccgtct tccacctgtt cgtggcctgc ctctcgctgg 200

gcttcttctc cctactctgg ctgcagctca gctgctctgg ggacgtggcc 250

cgggcagtca ggggacaagg gcaggagacc tcgggccctc cccgtgcctg 300

ccccccagag ccgccccctg agcactggga agaagacgca tcctggggcc 350

cccaccgcct ggcagtgctg gtgcccttcc gcgaacgctt cgaggagctc 400

ctggtcttcg tgccccacat gcgccgcttc ctgagcagga agaagatccg 450

gcaccacatc tacgtgctca accaggtgga ccacttcagg ttcaaccggg 500

cagcgctcat caacgtgggc ttcctggaga gcagcaacag cacggactac 550

attgccatgc acgacgttga cctgctccct ctcaacgagg agctggacta 600

tggctttcct gaggctgggc ccttccacgt ggcctccccg gagctccacc 650

ctctctacca ctacaagacc tatgtcggcg gcatcctgct gctctccaag 700

cagcactacc ggctgtgcaa tgggatgtcc aaccgcttct ggggctgggg 750

ccgcgaggac gacgagttct accggcgcat taagggagct gggctccagc 800

ttttccgccc ctcgggaatc acaactgggt acaagacatt tcgccacctg 850

catgacccag cctggcggaa gagggaccag aagcgcatcg cagctcaaaa 900

acaggagcag ttcaaggtgg acagggaggg aggcctgaac actgtgaagt 950

accatgtggc ttcccgcact gccctgtctg tgggcggggc cccctgcact 1000

gtcctcaaca tcatgttgga ctgtgacaag accgccacac cctggtgcac 1050

attcagctga gctggatgga cagtgaggaa gcctgtacct acaggccata 1100

ttgctcaggc tcaggacaag gcctcaggtc gtgggcccag ctctgacagg 1150

atgtggagtg gccaggacca agacagcaag ctacgcaatt gcagccaccc 1200

ggccgccaag gcaggcttgg gctgggccag gacacgtggg gtgcctggga 1250

cgctgcttgc catgcacagt gatcagagag aggctggggt gtgtcctgtc 1300

cgggaccccc cctgccttcc tgctcaccct actctgacct ccttcacgtg 1350

cccaggcctg tgggtagtgg ggagggctga acaggacaac ctctcatcac 1400

cctactctga cctccttcac gtgcccaggc ctgtgggtag tggggagggc 1450

tgaacaggac aacctctcat cacccccaaa aaaaaaaaaa aaaaaaaaaa 1500

aaaaaaaaaa aaaaaaaaaa aaaa 1524

<210> 142
<211> 327
<212> PRT
<213> Homo Sapien

<400> 142

<210> 143
<211> 1621
<212> DNA
<213> Homo Sapien

<400> 143
gtgggattta tttgagtgca agatcgtttt ctcagtggtg gtggaagttg 50

cctcatcgca ggcagatgtt ggggctttgt ccgaacagct cccctctgcc 100

agcttctgta gataagggtt aaaaactaat atttatatga cagaagaaaa 150

agatgtcatt ccgtaaagta aacatcatca tcttggtcct ggctgttgct 200

ctcttcttac tggttttgca ccataacttc ctcagcttga gcagtttgtt 250

aaggaatgag gttacagatt caggaattgt agggcctcaa cctatagact 300

ttgtcccaaa tgctctccga catgcagtag atgggagaca agaggagatt 350

cctgtggtca tcgctgcatc tgaagacagg cttggggggg ccattgcagc 400

tataaacagc attcagcaca acactcgctc caatgtgatt ttctacattg 450

ttactctcaa caatacagca gaccatctcc ggtcctggct caacagtgat 500

tccctgaaaa gcatcagata caaaattgtc aattttgacc ctaaactttt 550

ggaaggaaaa gtaaaggagg atcctgacca gggggaatcc atgaaacctt 600

taacctttgc aaggttctac ttgccaattc tggttcccag cgcaaagaag 650

gccatataca tggatgatga tgtaattgtg caaggtgata ttcttgccct 700

ttacaataca gcactgaagc caggacatgc agctgcattt tcagaagatt 750

gtgattcagc ctctactaaa gttgtcatcc gtggagcagg aaaccagtac 800

aattacattg gctatcttga ctataaaaag gaaagaattc gtaagctttc 850

catgaaagcc agcacttgct catttaatcc tggagttttt gttgcaaacc 900

tgacggaatg gaaacgacag aatataacta accaactgga aaaatggatg 950

aaactcaatg tagaagaggg actgtatagc agaaccctgg ctggtagcat 1000

cacaacacct cctctgctta tcgtatttta tcaacagcac tctaccatcg 1050

atcctatgtg gaatgtccgc caccttggtt ccagtgctgg aaaacgatat 1100

tcacctcagt ttgtaaaggc tgccaagtta ctccattgga atggacattt 1150

gaagccatgg ggaaggactg cttcatatac tgatgtttgg gaaaaatggt 1200

atattccaga cccaacaggc aaattcaacc taatccgaag atataccgag 1250

atctcaaaca taaagtgaaa cagaatttga actgtaagca agcatttctc 1300

aggaagtcct ggaagatagc atgcatggga agtaacagtt gctaggcttc 1350

aatgcctatc ggtagcaagc catggaaaaa gatgtgtcag ctaggtaaag 1400

atgacaaact gccctgtctg gcagtcagct tcccagacag actatagact 1450

ataaatatgt ctccatctgc cttaccaagt gttttcttac tacaatgctg 1500

aatgactgga aagaagaact gatatggcta gttcagctag ctggtacaga 1550

taattcaaaa ctgctgttgg ttttaatttt gtaacctgtg gcctgatctg 1600

taaataaaac ttacattttt c 1621

<210> 144
<211> 371
<212> PRT
<213> Homo Sapien

<400> 144
Met Ser Phe Arg Lys Val Asn Ile Ile Ile Leu Val Leu Ala Val
1               5                   10                  15

Ala Leu Phe Leu Leu Val Leu His His Asn Phe Leu Ser Leu Ser
                20                  25                  30

Ser Leu Leu Arg Asn Glu Val Thr Asp Ser Gly Ile Val Gly Pro
                35                  40                  45

Gln Pro Ile Asp Phe Val Pro Asn Ala Leu Arg His Ala Val Asp
                50                  55                  60

Gly Arg Gln Glu Glu Ile Pro Val Val Ile Ala Ala Ser Glu Asp
                65                  70                  75

Arg Leu Gly Gly Ala Ile Ala Ala Ile Asn Ser Ile Gln His Asn
                80                  85                  90

Thr Arg Ser Asn Val Ile Phe Tyr Ile Val Thr Leu Asn Asn Thr
                95                  100                 105

Ala Asp His Leu Arg Ser Trp Leu Asn Ser Asp Ser Leu Lys Ser
                110                 115                 120

Ile Arg Tyr Lys Ile Val Asn Phe Asp Pro Lys Leu Leu Glu Gly
                125                 130                 135

Lys Val Lys Glu Asp Pro Asp Gln Gly Glu Ser Met Lys Pro Leu
                140                 145                 150

Thr Phe Ala Arg Phe Tyr Leu Pro Ile Leu Val Pro Ser Ala Lys
                155                 160                 165

Lys Ala Ile Tyr Met Asp Asp Asp Val Ile Val Gln Gly Asp Ile
                170                 175                 180

Leu Ala Leu Tyr Asn Thr Ala Leu Lys Pro Gly His Ala Ala Ala
                185                 190                 195

Phe Ser Glu Asp Cys Asp Ser Ala Ser Thr Lys Val Val Ile Arg
                200                 205                 210

Gly Ala Gly Asn Gln Tyr Asn Tyr Ile Gly Tyr Leu Asp Tyr Lys
                215                 220                 225

Lys Glu Arg Ile Arg Lys Leu Ser Met Lys Ala Ser Thr Cys Ser
                230                 235                 240

Phe Asn Pro Gly Val Phe Val Ala Asn Leu Thr Glu Trp Lys Arg
                245                 250                 255

Gln Asn Ile Thr Asn Gln Leu Glu Lys Trp Met Lys Leu Asn Val
                260                 265                 270

Glu Glu Gly Leu Tyr Ser Arg Thr Leu Ala Gly Ser Ile Thr Thr
                275                 280                 285

Pro Pro Leu Leu Ile Val Phe Tyr Gln Gln His Ser Thr Ile Asp
                290                 295                 300

Pro Met Trp Asn Val Arg His Leu Gly Ser Ser Ala Gly Lys Arg
                305                 310                 315

Tyr Ser Pro Gln Phe Val Lys Ala Ala Lys Leu Leu His Trp Asn
                320                 325                 330

Gly His Leu Lys Pro Trp Gly Arg Thr Ala Ser Tyr Thr Asp Val
                335                 340                 345

Trp Glu Lys Trp Tyr Ile Pro Asp Pro Thr Gly Lys Phe Asn Leu
                350                 355                 360

Ile Arg Arg Tyr Thr Glu Ile Ser Asn Ile Lys
                365                 370

<210> 145
<211> 415
<212> DNA
<213> Homo Sapien

<400> 145
aaacttgacg ccatgaagat cccggtcctt cctgccgtgg tgctcctctc 50

cctcctggtg ctccactctg cccagggagc caccctgggt ggtcctgagg 100

aagaaagcac cattgagaat tatgcgtcac gacccgaggc ctttaacacc 150

ccgttcctga acatcgacaa attgcgatct gcgtttaagg ctgatgagtt 200

cctgaactgg cacgccctct ttgagtctat caaaaggaaa cttcctttcc 250

tcaactggga tgcctttcct aagctgaaag gactgaggag cgcaactcct 300

gatgcccagt gaccatgacc tccactggaa gagggggcta gcgtgagcgc 350

tgattctcaa cctaccataa ctctttcctg cctcaggaac tccaataaaa 400

cattttccat ccaaa 415

<210> 146
<211> 99
<212> PRT
<213> Homo Sapien

<400> 146
Met Lys Ile Pro Val Leu Pro Ala Val Val Leu Leu Ser Leu Leu

1               5                   10                  15

Val Leu His Ser Ala Gln Gly Ala Thr Leu Gly Gly Pro Glu Glu
                20                  25                  30

Glu Ser Thr Ile Glu Asn Tyr Ala Ser Arg Pro Glu Ala Phe Asn
                35                  40                  45

Thr Pro Phe Leu Asn Ile Asp Lys Leu Arg Ser Ala Phe Lys Ala
                50                  55                  60

Asp Glu Phe Leu Asn Trp His Ala Leu Phe Glu Ser Ile Lys Arg
                65                  70                  75

Lys Leu Pro Phe Leu Asn Trp Asp Ala Phe Pro Lys Leu Lys Gly
                80                  85                  90

Leu Arg Ser Ala Thr Pro Asp Ala Gln
                95

<210> 147
<211> 745
<212> DNA
<213> Homo Sapien

<400> 147
cctctgtcca ctgctttcgt gaagacaaga tgaagttcac aattgtcttt 50

gctggacttc ttggagtctt tctagctcct gccctagcta actataatat 100

caacgtcaat gatgacaaca acaatgctgg aagtgggcag cagtcagtga 150

gtgtcaacaa tgaacacaat gtggccaatg ttgacaataa caacggatgg 200

gactcctgga attccatctg ggattatgga aatggctttg ctgcaaccag 250

actctttcaa aagaagacat gcattgtgca caaaatgaac aaggaagtca 300

tgccctccat tcaatccctt gatgcactgg tcaaggaaaa gaagcttcag 350

ggtaagggac caggaggacc acctcccaag ggcctgatgt actcagtcaa 400

cccaaacaaa gtcgatgacc tgagcaagtt cggaaaaaac attgcaaaca 450

tgtgtcgtgg gattccaaca tacatggctg aggagatgca agaggcaagc 500

ctgttttttt actcaggaac gtgctacacg accagtgtac tatggattgt 550

ggacatttcc ttctgtggag acacggtgga gaactaaaca attttttaaa 600

gccactatgg atttagtcat ctgaatatgc tgtgcagaaa aaatatgggc 650

tccagtggtt tttaccatgt cattctgaaa tttttctcta ctagttatgt 700

ttgatttctt taagtttcaa taaaatcatt tagcattgaa aaaaa 745

<210> 148
<211> 185
<212> PRT
<213> Homo Sapien

<400> 148
Met Lys Phe Thr Ile Val Phe Ala Gly Leu Leu Gly Val Phe Leu
  1               5                   10                  15

Ala Pro Ala Leu Ala Asn Tyr Asn Ile Asn Val Asn Asp Asp Asn
                20                  25                  30

Asn Asn Ala Gly Ser Gly Gln Gln Ser Val Ser Val Asn Asn Glu
                35                  40                  45

His Asn Val Ala Asn Val Asp Asn Asn Asn Gly Trp Asp Ser Trp
                50                  55                  60

Asn Ser Ile Trp Asp Tyr Gly Asn Gly Phe Ala Ala Thr Arg Leu
                65                  70                  75

Phe Gln Lys Lys Thr Cys Ile Val His Lys Met Asn Lys Glu Val
                80                  85                  90

Met Pro Ser Ile Gln Ser Leu Asp Ala Leu Val Lys Glu Lys Lys
                95                  100                 105

Leu Gln Gly Lys Gly Pro Gly Gly Pro Pro Pro Lys Gly Leu Met
                110                 115                 120

Tyr Ser Val Asn Pro Asn Lys Val Asp Asp Leu Ser Lys Phe Gly
                125                 130                 135

Lys Asn Ile Ala Asn Met Cys Arg Gly Ile Pro Thr Tyr Met Ala
                140                 145                 150

Glu Glu Met Gln Glu Ala Ser Leu Phe Phe Tyr Ser Gly Thr Cys
                155                 160                 165

Tyr Thr Thr Ser Val Leu Trp Ile Val Asp Ile Ser Phe Cys Gly
                170                 175                 180

Asp Thr Val Glu Asn
                185

<210> 149
<211> 992
<212> DNA
<213> Homo Sapien

<400> 149
   ggcacgagcc aggaactagg aggttctcac tgcccgagca gaggccctac 50
   acccaccgag gcatggggct ccctgggctg ttctgcttgg ccgtgctggc 100
   tgccagcagc ttctccaagg cacgggagga agaaattacc cctgtggtct 150
   ccattgccta caaagtcctg gaagttttcc ccaaaggccg ctgggtgctc 200
   ataacctgct gtgcacccca gccaccaccg cccatcacct attccctctg 250
   tggaaccaag aacatcaagg tggccaagaa ggtggtgaag acccacgagc 300
   cggcctcctt caacctcaac gtcacactca agtccagtcc agacctgctc 350
   acctacttct gccgggcgtc ctccacctca ggtgcccatg tggacagtgc 400
   caggctacag atgcactggg agctgtggtc caagccagtg tctgagctgc 450
   gggccaactt cactctgcag gacagagggg caggccccag ggtggagatg 500
   atctgccagg cgtcctcggg cagcccacct atcaccaaca gcctgatcgg 550
   gaaggatggg caggtccacc tgcagcagag accatgccac aggcagcctg 600
   ccaacttctc cttcctgccg agccagacat cggactggtt ctggtgccag 650
   gctgcaaaca acgccaatgt ccagcacagc gccctcacag tggtgccccc 700
   aggtggtgac cagaagatgg aggactggca gggtcccctg gagagcccca 750
   tccttgcctt gccgctctac aggagcaccc gccgtctgag tgaagaggag 800
   tttggggggt tcaggatagg gaatggggag gtcagaggac gcaaagcagc 850
   agccatgtag aatgaaccgt ccagagagcc aagcacggca gaggactgca 900
   ggccatcagc gtgcactgtt cgtatttgga gttcatgcaa aatgagtgtg 950
   ttttagctgc tcttgccaca aaaaaaaaaa aaaaaaaaaa aa 992

<210> 150
<211> 265
<212> PRT
<213> Homo Sapien

<400> 150

<210> 151
<211> 683
<212> DNA
<213> Homo Sapien

<400> 151
gcgtggggat gtctaggagc tcgaaggtgg tgctgggcct ctcggtgctg 50

ctgacggcgg ccacagtggc cggcgtacat gtgaagcagc agtgggacca 100

gcagaggctt cgtgacggag ttatcagaga cattgagagg caaattcgga 150

aaaaagaaaa cattcgtctt ttgggagaac agattatttt gactgagcaa 200

cttgaagcag aaagagagaa gatgttattg gcaaaaggat ctcaaaaatc 250

atgacttgaa tgtgaaatat ctgttggaca gacaacacga gtttgtgtgt 300

gtgtgttgat ggagagtagc ttagtagtat cttcatcttt ttttttggtc 350

actgtccttt taaacttgat caaataaagg acagtgggtc atataagtta 400

ctgctttcag ggtcccttat atctgaataa aggagtgtgg gcagacactt 450

tttggaagag tctgtctggg tgatcctggt agaagcccca ttagggtcac 500

tgtccagtgc ttagggttgt tactgagaag cactgccgag cttgtgagaa 550

ggaagggatg gatagtagca tccacctgag tagtctgatc agtcggcatg 600

atgacgaagc cacgagaaca tcgacctcag aaggactgga ggaaggtgaa 650

gtggagggag agacgctcct gatcgtcgaa tcc 683

<210> 152
<211> 81
<212> PRT
<213> Homo Sapien

<400> 152
Met Ser Arg Ser Ser Lys Val Val Leu Gly Leu Ser Val Leu Leu
1               5                   10                  15

Thr Ala Ala Thr Val Ala Gly Val His Val Lys Gln Gln Trp Asp
                20                  25                  30

Gln Gln Arg Leu Arg Asp Gly Val Ile Arg Asp Ile Glu Arg Gln
                35                  40                  45

Ile Arg Lys Lys Glu Asn Ile Arg Leu Leu Gly Glu Gln Ile Ile
                50                  55                  60

Leu Thr Glu Gln Leu Glu Ala Glu Arg Glu Lys Met Leu Leu Ala
                65                  70                  75

Lys Gly Ser Gln Lys Ser
                80

<210> 153
<211> 1869
<212> DNA
<213> Homo Sapien

<400> 153
aatgtgagag gggctgatgg aagctgatag gcaggactgg agtgttagca 50

ccagtactgg atgtgacagc aggcagagga gcacttagca gcttattcag 100
tgtccgattc tgattccggc aaggatccaa gcatggaatg ctgccgtcgg 150
gcaactcctg gcacactgct cctctttctg gctttcctgc tcctgagttc 200
caggaccgca cgctccgagg aggaccggga cggcctatgg gatgcctggg 250
gcccatggag tgaatgctca cgcacctgcg gggagggggc ctcctactct 300
ctgaggcgct gcctgagcag caagagctgt gaaggaagaa atatccgata 350
cagaacatgc agtaatgtgg actgcccacc agaagcaggt gatttccgag 400
ctcagcaatg ctcagctcat aatgatgtca agcaccatgg ccagttttat 450
gaatggcttc ctgtgtctaa tgaccctgac aacccatgtt cactcaagtg 500
ccaagccaaa ggaacaaccc tggttgttga actagcacct aaggtcttag 550
atggtacgcg ttgctataca gaatctttgg atatgtgcat cagtggttta 600
tgccaaattg ttggctgcga tcaccagctg ggaagcaccg tcaaggaaga 650
taactgtggg gtctgcaacg gagatgggtc cacctgccgg ctggtccgag 700
ggcagtataa atcccagctc tccgcaacca aatcggatga tactgtggtt 750
gcacttccct atggaagtag acatattcgc cttgtcttaa aaggtcctga 800
tcacttatat ctggaaacca aaaccctcca ggggactaaa ggtgaaaaca 850
gtctcagctc cacaggaact ttccttgtgg acaattctag tgtggacttc 900
cagaaatttc cagacaaaga gatactgaga atggctggac cactcacagc 950
agatttcatt gtcaagattc gtaactcggg ctccgctgac agtacagtcc 1000
agttcatctt ctatcaaccc atcatccacc gatggaggga gacggatttc 1050
tttccttgct cagcaacctg tggaggaggt tatcagctga catcggctga 1100
gtgctacgat ctgaggagca accgtgtggt tgctgaccaa tactgtcact 1150
attacccaga gaacatcaaa cccaaaccca agcttcagga gtgcaacttg 1200
gatccttgtc cagccagtga cggatacaag cagatcatgc cttatgacct 1250
ctaccatccc cttcctcggt gggaggccac cccatggacc gcgtgctcct 1300
cctcgtgtgg ggggggcatc cagagccggg cagtttcctg tgtggaggag 1350
gacatccagg ggcatgtcac ttcagtggaa gagtggaaat gcatgtacac 1400
ccctaagatg cccatcgcgc agccctgcaa catttttgac tgccctaaat 1450
ggctggcaca ggagtggtct ccgtgcacag tgacatgtgg ccagggcctc 1500
agataccgtg tggtcctctg catcgaccat cgaggaatgc acacaggagg 1550
ctgtagccca aaaacaaagc cccacataaa agaggaatgc atcgtaccca 1600
ctccctgcta taaacccaaa gagaaacttc cagtcgaggc caagttgcca 1650
tggttcaaac aagctcaaga gctagaagaa ggagctgctg tgtcagagga 1700
gccctcgtaa gttgtaaaag cacagactgt tctatatttg aaactgtttt 1750

gtttaaagaa agcagtgtct cactggttgt agctttcatg ggttctgaac 1800

taagtgtaat catctcacca aagctttttg gctctcaaat taaagattga 1850

ttagtttcaa aaaaaaaa 1869

<210> 154
<211> 525
<212> PRT
<213> Homo Sapien

<400> 154

<210> 155
<211> 1734
<212> DNA
<213> Homo Sapien

<400> 155
gtggactctg agaagcccag gcagttgagg acaggagaga gaaggctgca 50

gacccagagg gagggaggac agggagtcgg aaggaggagg acagaggagg 100

gcacagagac gcagagcaag ggcggcaagg aggagaccct ggtgggagga 150

agacactctg gagagagagg gggctgggca gagatgaagt tccaggggcc 200

cctggcctgc ctcctgctgg ccctctgcct gggcagtggg gaggctggcc 250

ccctgcagag cggagaggaa agcactggga caaatattgg ggaggccctt 300

ggacatggcc tgggagacgc cctgagcgaa ggggtgggaa aggccattgg 350
caaagaggcc ggaggggcag ctggctctaa agtcagtgag gcccttggcc 400
aagggaccag agaagcagtt ggcactggag tcaggcaggt tccaggcttt 450
ggcgcagcag atgctttggg caacagggtc ggggaagcag cccatgctct 500
gggaaacact gggcacgaga ttggcagaca ggcagaagat gtcattcgac 550
acggagcaga tgctgtccgc ggctcctggc agggggtgcc tggccacagt 600
ggtgcttggg aaacttctgg aggccatggc atctttggct ctcaaggtgg 650
ccttggaggc cagggccagg gcaatcctgg aggtctgggg actccgtggg 700
tccacggata ccccggaaac tcagcaggca gctttggaat gaatcctcag 750
ggagctccct ggggtcaagg aggcaatgga gggccaccaa actttgggac 800
caacactcag ggagctgtgg cccagcctgg ctatggttca gtgagagcca 850
gcaaccagaa tgaagggtgc acgaatcccc caccatctgg ctcaggtgga 900
ggctccagca actctggggg aggcagcggc tcacagtcgg gcagcagtgg 950
cagtggcagc aatggtgaca acaacaatgg cagcagcagt ggtggcagca 1000
gcagtggcag cagcagtggc agcagcagtg gcggcagcag tggcggcagc 1050
agtggtggca gcagtggcaa cagtggtggc agcagaggtg acagcggcag 1100
tgagtcctcc tggggatcca gcaccggctc ctcctccggc aaccacggtg 1150
ggagcggcgg aggaaatgga cataaacccg ggtgtgaaaa gccagggaat 1200
gaagcccgcg ggagcgggga atctgggatt cagggcttca gaggacaggg 1250
agtttccagc aacatgaggg aaataagcaa agagggcaat cgcctccttg 1300
gaggctctgg agacaattat cgggggcaag ggtcgagctg gggcagtgga 1350
ggaggtgacg ctgttggtgg agtcaatact gtgaactctg agacgtctcc 1400
tgggatgttt aactttgaca ctttctggaa gaattttaaa tccaagctgg 1450
gtttcatcaa ctgggatgcc ataaacaagg accagagaag ctctcgcatc 1500
ccgtgacctc cagacaagga gccaccagat tggatgggag cccccacact 1550
ccctccttaa aacaccaccc tctcatcact aatctcagcc cttgcccttg 1600
aaataaacct tagctgcccc acaaaaaaaa aaaaaaaaaa aaaaaaaaaa 1650
aaaaaaaaaa aaaaaaaaaa aaaaaaaaaa aaaaaaaaaa aaaaaaaaaa 1700
aaaaaaaaaa aaaaaaaaaa aaaaaaaaaa aaaa 1734

<210> 156
<211> 440
<212> PRT
<213> Homo Sapien

<400> 156
Met Lys Phe Gln Gly Pro Leu Ala Cys Leu Leu Leu Ala Leu Cys
1               5                   10                  15

Leu Gly Ser Gly Glu Ala Gly Pro Leu Gln Ser Gly Glu Glu Ser

20                  25                  30

Thr Gly Thr Asn Ile Gly Glu Ala Leu Gly His Gly Leu Gly Asp
                35                  40                  45

Ala Leu Ser Glu Gly Val Gly Lys Ala Ile Gly Lys Glu Ala Gly
                50                  55                  60

Gly Ala Ala Gly Ser Lys Val Ser Glu Ala Leu Gly Gln Gly Thr
                65                  70                  75

Arg Glu Ala Val Gly Thr Gly Val Arg Gln Val Pro Gly Phe Gly
                80                  85                  90

Ala Ala Asp Ala Leu Gly Asn Arg Val Gly Glu Ala Ala His Ala
                95                  100                 105

Leu Gly Asn Thr Gly His Glu Ile Gly Arg Gln Ala Glu Asp Val
                110                 115                 120

Ile Arg His Gly Ala Asp Ala Val Arg Gly Ser Trp Gln Gly Val
                125                 130                 135

Pro Gly His Ser Gly Ala Trp Glu Thr Ser Gly Gly His Gly Ile
                140                 145                 150

Phe Gly Ser Gln Gly Gly Leu Gly Gly Gln Gly Gln Gly Asn Pro
                155                 160                 165

Gly Gly Leu Gly Thr Pro Trp Val His Gly Tyr Pro Gly Asn Ser
                170                 175                 180

Ala Gly Ser Phe Gly Met Asn Pro Gln Gly Ala Pro Trp Gly Gln
                185                 190                 195

Gly Gly Asn Gly Gly Pro Pro Asn Phe Gly Thr Asn Thr Gln Gly
                200                 205                 210

Ala Val Ala Gln Pro Gly Tyr Gly Ser Val Arg Ala Ser Asn Gln
                215                 220                 225

Asn Glu Gly Cys Thr Asn Pro Pro Pro Ser Gly Ser Gly Gly Gly
                230                 235                 240

Ser Ser Asn Ser Gly Gly Gly Ser Gly Ser Gln Ser Gly Ser Ser
                245                 250                 255

Gly Ser Gly Ser Asn Gly Asp Asn Asn Asn Gly Ser Ser Ser Gly
                260                 265                 270

Gly Ser Ser Ser Gly Ser Ser Ser Gly Ser Ser Ser Gly Gly Ser
                275                 280                 285

Ser Gly Gly Ser Ser Gly Gly Ser Ser Gly Asn Ser Gly Gly Ser
                290                 295                 300

Arg Gly Asp Ser Gly Ser Glu Ser Ser Trp Gly Ser Ser Thr Gly
                305                 310                 315

Ser Ser Ser Gly Asn His Gly Gly Ser Gly Gly Gly Asn Gly His
                320                 325                 330

Lys Pro Gly Cys Glu Lys Pro Gly Asn Glu Ala Arg Gly Ser Gly
                335                 340                 345

Glu Ser Gly Ile Gln Gly Phe Arg Gly Gln Gly Val Ser Ser Asn
                350                 355                 360

Met Arg Glu Ile Ser Lys Glu Gly Asn Arg Leu Leu Gly Gly Ser
                365                 370                 375

Gly Asp Asn Tyr Arg Gly Gln Gly Ser Ser Trp Gly Ser Gly Gly
                380                 385                 390

Gly Asp Ala Val Gly Gly Val Asn Thr Val Asn Ser Glu Thr Ser
                395                 400                 405

Pro Gly Met Phe Asn Phe Asp Thr Phe Trp Lys Asn Phe Lys Ser
                410                 415                 420

Lys Leu Gly Phe Ile Asn Trp Asp Ala Ile Asn Lys Asp Gln Arg
                425                 430                 435

Ser Ser Arg Ile Pro
                440

<210> 157
<211> 4104
<212> DNA
<213> Homo Sapien

<400> 157
cccacgcgtc cgcccacgcg tccgcccacg cgtccgccca cgcgtccgcc 50

cacgcgtccg cccacgcgtc cgcccacgcg tccggtgcaa gctcgcgccg 100

cacactgcct ggtggaggga aggagcccgg gcgcctctcg ccgctccccg 150

cgccgccgtc cgcacctccc caccgcccgc cgcccgccgc ccgccgcccg 200

caaagcatga gtgagcccgc tctctgcagc tgcccggggc gcgaatggca 250

ggctgtttcc gcggagtaaa aggtggcgcc ggtcagtggt cgtttccaat 300

gacggacatt aaccagactg tcagatcctg gggagtcgcg agccccgagt 350

ttggagtttt ttccccccac aacgtcacag tccgaactgc agagggaaag 400

gaaggcggca ggaaggcgaa gctcgggctc cggcacgtag ttgggaaact 450

tgcgggtcct agaagtcgcc tccccgcctt gccggccgcc cttgcagccc 500

cgagccgagc agcaaagtga gacattgtgc gcctgccaga tccgccggcc 550

gcggaccggg gctgcctcgg aaacacagag gggtcttctc tcgccctgca 600

tataattagc ctgcacacaa agggagcagc tgaatggagg ttgtcactct 650

ctggaaaagg atttctgacc gagcgcttcc aatggacatt ctccagtctc 700

tctggaaaga ttctcgctaa tggatttcct gctgctcggt ctctgtctat 750

actggctgct gaggaggccc tcgggggtgg tcttgtgtct gctgggggcc 800

tgctttcaga tgctgcccgc cgcccccagc gggtgcccgc agctgtgccg 850

gtgcgagggg cggctgctgt actgcgaggc gctcaacctc accgaggcgc 900

cccacaacct gtccggcctg ctgggcttgt ccctgcgcta caacagcctc 950

tcggagctgc gcgccggcca gttcacgggg ttaatgcagc tcacgtggct 1000

ctatctggat cacaatcaca tctgctccgt gcagggggac gcctttcaga 1050

aactgcgccg agttaaggaa ctcacgctga gttccaacca gatcacccaa 1100

ctgcccaaca ccaccttccg gcccatgccc aacctgcgca gcgtggacct 1150
ctcgtacaac aagctgcagg cgctcgcgcc cgacctcttc cacgggctgc 1200
ggaagctcac cacgctgcat atgcgggcca acgccatcca gtttgtgccc 1250
gtgcgcatct tccaggactg ccgcagcctc aagtttctcg acatcggata 1300
caatcagctc aagagtctgg cgcgcaactc tttcgccggc ttgtttaagc 1350
tcaccgagct gcacctcgag cacaacgact tggtcaaggt gaacttcgcc 1400
cacttcccgc gcctcatctc cctgcactcg ctctgcctgc ggaggaacaa 1450
ggtggccatt gtggtcagct cgctggactg ggtttggaac ctggagaaaa 1500
tggacttgtc gggcaacgag atcgagtaca tggagcccca tgtgttcgag 1550
accgtgccgc acctgcagtc cctgcagctg gactccaacc gcctcaccta 1600
catcgagccc cggatcctca actcttggaa gtccctgaca agcatcaccc 1650
tggccgggaa cctgtgggat tgcgggcgca acgtgtgtgc cctagcctcg 1700
tggctcagca acttccaggg gcgctacgat ggcaacttgc agtgcgccag 1750
cccggagtac gcacagggcg aggacgtcct ggacgccgtg tacgccttcc 1800
acctgtgcga ggatggggcc gagcccacca gcggccacct gctctcggcc 1850
gtcaccaacc gcagtgatct ggggccccct gccagctcgg ccaccacgct 1900
cgcggacggc ggggaggggc agcacgacgg cacattcgag cctgccaccg 1950
tggctcttcc aggcggcgag cacgccgaga acgccgtgca gatccacaag 2000
gtggtcacgg gcaccatggc cctcatcttc tccttcctca tcgtggtcct 2050
ggtgctctac gtgtcctgga agtgtttccc agccagcctc aggcagctca 2100
gacagtgctt tgtcacgcag cgcaggaagc aaaagcagaa acagaccatg 2150
catcagatgg ctgccatgtc tgcccaggaa tactacgttg attacaaacc 2200
gaaccacatt gagggagccc tggtgatcat caacgagtat ggctcgtgta 2250
cctgccacca gcagcccgcg agggaatgcg aggtgtgatt gtcccagtgg 2300
ctctcaaccc atgcgctacc aaatacgcct gggcagccgg gacgggccgg 2350
cgggcaccag gctggggtct ccttgtctgt gctctgatat gctccttgac 2400
tgaaacttta aggggatctc tcccagagac ttgacatttt agctttattg 2450
tgtcttaaaa acaaaagcga attaaaacac aacaaaaaac cccaccccac 2500
aaccttcagg acagtctatc ttaaatttca tatgagaact ccttcctccc 2550
tttgaagatc tgtccatatt caggaatctg agagtgtaaa aaaggtggcc 2600
ataagacaga gagagaataa tcgtgctttg ttttatgcta ctcctcccac 2650
cctgcccatg attaaacatc atgtatgtag aagatcttaa gtccatacgc 2700
atttcatgaa gaaccattgg aaagaggaat ctgcaatctg ggagcttaag 2750
agcaaatgat gaccatagaa agctatgttc ttactttgtg tgtgtgtctg 2800

tatgtttctg cgttgtgtgt ctttgtaggc aagcaaacgt tgtctacaca 2850

aacgggaatt tagctcacat catttcatgc ccctgtgcct ctagctctgg 2900

agattggtgg ggggaggtgg ggggaaacgg caggaataag ggaaagtggt 2950

agttttaact aaggttttgt aacacttgaa atcttttctt tctcaaatta 3000

attatcttta agcttcaaga aacttgctct gacccctcta agcaaactac 3050

taagcattta aaagagaatc taatttttaa aggtgtagca cctttttttt 3100

tattcttccc acagagggtg ctaatctcat tatgctgtgc tatctgaaaa 3150

gaacttaagg ccacaattca cgtctcgtcc tgggcattgt gatggattga 3200

ccctccattt gcagtacctt cccagctgat taaagttcag cagtggtatt 3250

gaggtttttc gaatatttat atagaaaaaa agtcttttca catgacaaat 3300

gacactctca caccagtctt agccctagta gttttttagg ttggaccaga 3350

ggaagcaggt taaatgagac ctgtcctctg ctgcactcag aaaaaatagg 3400

cagtccctga tgctcagatc ttagccttga tattaatagt tgagaccacc 3450

tacccacaat gcagcctata ctcccaagac tacaaagtta ccatcgcaaa 3500

ggaaaggtta ttccagtaaa aggaaatagt tttctcaacc atttaaaaat 3550

attcttctga actcatcaaa gtagaagagc cccaaccttt ttctctctgc 3600

cttcaagaag gcagacattt ggtatgattt agcatcaaca acacatttat 3650

gagtatatgt aagtaatcag aggggcaaat gccacttgtt attcctccca 3700

agttttccaa gcaagtacac acagatctct ggtaggatta ggggccactt 3750

gtgtttccgg cttattttag tcgacttgtc agcaagtttg atgcctagtc 3800

tatctgacat ggcccagtag aacagggcat tgatggatca catgagatgg 3850

tagaaggaac atcatcacat acccctctca cagagaaaat tatcaaagaa 3900

ccagaaatta tatctgtttt ggagcaagag tgtcataatg tttcagggta 3950

gtcaaaataa acataaatta tctcctctag atgagtggcg atgttggctg 4000

atttgggtct gccattgaca gaatgtcaaa taaaaaggaa ttagctagaa 4050

tatgaccatt aaatgtgctt ctgaaatata ttttgagata ggtttagaat 4100

gtca 4104

<210> 158
<211> 522
<212> PRT
<213> Homo Sapien

<400> 158
Met Asp Phe Leu Leu Leu Gly Leu Cys Leu Tyr Trp Leu Leu Arg
1               5                   10                  15

Arg Pro Ser Gly Val Val Leu Cys Leu Leu Gly Ala Cys Phe Gln
                20                  25                  30

Met Leu Pro Ala Ala Pro Ser Gly Cys Pro Gln Leu Cys Arg Cys
                35                  40                  45

Glu Gly Arg Leu Leu Tyr Cys Glu Ala Leu Asn Leu Thr Glu Ala
                50                  55                  60

Pro His Asn Leu Ser Gly Leu Leu Gly Leu Ser Leu Arg Tyr Asn
                65                  70                  75

Ser Leu Ser Glu Leu Arg Ala Gly Gln Phe Thr Gly Leu Met Gln
                80                  85                  90

Leu Thr Trp Leu Tyr Leu Asp His Asn His Ile Cys Ser Val Gln
                95                  100                 105

Gly Asp Ala Phe Gln Lys Leu Arg Arg Val Lys Glu Leu Thr Leu
                110                 115                 120

Ser Ser Asn Gln Ile Thr Gln Leu Pro Asn Thr Thr Phe Arg Pro
                125                 130                 135

Met Pro Asn Leu Arg Ser Val Asp Leu Ser Tyr Asn Lys Leu Gln
                140                 145                 150

Ala Leu Ala Pro Asp Leu Phe His Gly Leu Arg Lys Leu Thr Thr
                155                 160                 165

Leu His Met Arg Ala Asn Ala Ile Gln Phe Val Pro Val Arg Ile
                170                 175                 180

Phe Gln Asp Cys Arg Ser Leu Lys Phe Leu Asp Ile Gly Tyr Asn
                185                 190                 195

Gln Leu Lys Ser Leu Ala Arg Asn Ser Phe Ala Gly Leu Phe Lys
                200                 205                 210

Leu Thr Glu Leu His Leu Glu His Asn Asp Leu Val Lys Val Asn
                215                 220                 225

Phe Ala His Phe Pro Arg Leu Ile Ser Leu His Ser Leu Cys Leu
                230                 235                 240

Arg Arg Asn Lys Val Ala Ile Val Val Ser Ser Leu Asp Trp Val
                245                 250                 255

Trp Asn Leu Glu Lys Met Asp Leu Ser Gly Asn Glu Ile Glu Tyr
                260                 265                 270

Met Glu Pro His Val Phe Glu Thr Val Pro His Leu Gln Ser Leu
                275                 280                 285

Gln Leu Asp Ser Asn Arg Leu Thr Tyr Ile Glu Pro Arg Ile Leu
                290                 295                 300

Asn Ser Trp Lys Ser Leu Thr Ser Ile Thr Leu Ala Gly Asn Leu
                305                 310                 315

Trp Asp Cys Gly Arg Asn Val Cys Ala Leu Ala Ser Trp Leu Ser
                320                 325                 330

Asn Phe Gln Gly Arg Tyr Asp Gly Asn Leu Gln Cys Ala Ser Pro
                335                 340                 345

Glu Tyr Ala Gln Gly Glu Asp Val Leu Asp Ala Val Tyr Ala Phe
                350                 355                 360

His Leu Cys Glu Asp Gly Ala Glu Pro Thr Ser Gly His Leu Leu
                365                 370                 375

Ser Ala Val Thr Asn Arg Ser Asp Leu Gly Pro Pro Ala Ser Ser

                380                 385                 390

Ala Thr Thr Leu Ala Asp Gly Gly Glu Gly Gln His Asp Gly Thr
                395                 400                 405

Phe Glu Pro Ala Thr Val Ala Leu Pro Gly Gly Glu His Ala Glu
                410                 415                 420

Asn Ala Val Gln Ile His Lys Val Val Thr Gly Thr Met Ala Leu
                425                 430                 435

Ile Phe Ser Phe Leu Ile Val Val Leu Val Leu Tyr Val Ser Trp
                440                 445                 450

Lys Cys Phe Pro Ala Ser Leu Arg Gln Leu Arg Gln Cys Phe Val
                455                 460                 465

Thr Gln Arg Arg Lys Gln Lys Gln Lys Gln Thr Met His Gln Met
                470                 475                 480

Ala Ala Met Ser Ala Gln Glu Tyr Tyr Val Asp Tyr Lys Pro Asn
                485                 490                 495

His Ile Glu Gly Ala Leu Val Ile Ile Asn Glu Tyr Gly Ser Cys
                500                 505                 510

Thr Cys His Gln Gln Pro Ala Arg Glu Cys Glu Val
                515                 520

<210> 159
<211> 1049
<212> DNA
<213> Homo Sapien

<400> 159
   cagagaggag gctttgggaa ttgtccagca gaaacagaga agtctgaggt 50
   ggtgtcaaga caaaagatgc ttcagctttg gaaacttgtt ctcctgtgcg 100
   gcgtgctcac tgggacctca gagtctcttc ttgacaatct tggcaatgac 150
   ctaagcaatg tcgtggataa gctggaacct gttcttcacg agggacttga 200
   gacagttgac aatactctta aaggcatcct tgagaaactg aaggtcgacc 250
   taggagtgct tcagaaatcc agtgcttggc aactggccaa gcagaaggcc 300
   caggaagctg agaaattgct gaacaatgtc atttctaagc tgcttccaac 350
   taacacggac atttttgggt tgaaaatcag caactccctc atcctggatg 400
   tcaaagctga accgatcgat gatggcaaag gccttaacct gagcttccct 450
   gtcaccgcga atgtcactgt ggccgggccc atcattggcc agattatcaa 500
   cctgaaagcc tccttggacc tcctgaccgc agtcacaatt gaaactgatc 550
   cccagacaca ccagcctgtt gccgtcctgg gagaatgcgc cagtgaccca 600
   accagcatct cactttcctt gctggacaaa cacagccaaa tcatcaacaa 650
   gttcgtgaat agcgtgatca acacgctgaa aagcactgta tcctccctgc 700
   tgcagaagga gatatgtcca ctgatccgca tcttcatcca ctccctggat 750
   gtgaatgtca ttcagcaggt cgtcgataat cctcagcaca aaacccagct 800
   gcaaaccctc atctgaagag gacgaatgag gaggaccact gtggtgcatg 850
   ctgattggtt cccagtggct tgccccaccc ccttatagca tctccctcca 900
   ggaagctgct gccaccacct aaccagcgtg aaagcctgag tcccaccaga 950
   aggaccttcc cagatacccc ttctcctcac agtcagaaca gcagcctcta 1000
   cacatgttgt cctgcccctg gcaataaagg cccatttctg cacccttaa 1049

<210> 160
<211> 249
<212> PRT
<213> Homo Sapien

<400> 160
Met Leu Gln Leu Trp Lys Leu Val Leu Leu Cys Gly Val Leu Thr
1               5                   10                  15

Gly Thr Ser Glu Ser Leu Leu Asp Asn Leu Gly Asn Asp Leu Ser
                20                  25                  30

Asn Val Val Asp Lys Leu Glu Pro Val Leu His Glu Gly Leu Glu
                35                  40                  45

Thr Val Asp Asn Thr Leu Lys Gly Ile Leu Glu Lys Leu Lys Val
                50                  55                  60

Asp Leu Gly Val Leu Gln Lys Ser Ser Ala Trp Gln Leu Ala Lys
                65                  70                  75

Gln Lys Ala Gln Glu Ala Glu Lys Leu Leu Asn Asn Val Ile Ser
                80                  85                  90

Lys Leu Leu Pro Thr Asn Thr Asp Ile Phe Gly Leu Lys Ile Ser
                95                  100                 105

Asn Ser Leu Ile Leu Asp Val Lys Ala Glu Pro Ile Asp Asp Gly
                110                 115                 120

Lys Gly Leu Asn Leu Ser Phe Pro Val Thr Ala Asn Val Thr Val
                125                 130                 135

Ala Gly Pro Ile Ile Gly Gln Ile Ile Asn Leu Lys Ala Ser Leu
                140                 145                 150

Asp Leu Leu Thr Ala Val Thr Ile Glu Thr Asp Pro Gln Thr His
                155                 160                 165

Gln Pro Val Ala Val Leu Gly Glu Cys Ala Ser Asp Pro Thr Ser
                170                 175                 180

Ile Ser Leu Ser Leu Leu Asp Lys His Ser Gln Ile Ile Asn Lys
                185                 190                 195

Phe Val Asn Ser Val Ile Asn Thr Leu Lys Ser Thr Val Ser Ser
                200                 205                 210

Leu Leu Gln Lys Glu Ile Cys Pro Leu Ile Arg Ile Phe Ile His
                215                 220                 225

Ser Leu Asp Val Asn Val Ile Gln Gln Val Val Asp Asn Pro Gln
                230                 235                 240

His Lys Thr Gln Leu Gln Thr Leu Ile
                245

<210> 161
<211> 1587

<212> DNA
<213> Homo Sapien

<400> 161
cagccacaga cgggtcatga gcgcggtatt actgctggcc ctcctggggt 50

tcatcctccc actgccagga gtgcaggcgc tgctctgcca gtttgggaca 100

gttcagcatg tgtggaaggt gtccgaccta ccccggcaat ggacccctaa 150

gaacaccagc tgcgacagcg gcttggggtg ccaggacacg ttgatgctca 200

ttgagagcgg accccaagtg agcctggtgc tctccaaggg ctgcacggag 250

gccaaggacc aggagccccg cgtcactgag caccggatgg gccccggcct 300

ctccctgatc tcctacacct tcgtgtgccg ccaggaggac ttctgcaaca 350

acctcgttaa ctccctcccg ctttgggccc cacagccccc agcagaccca 400

ggatccttga ggtgcccagt ctgcttgtct atggaaggct gtctggaggg 450

gacaacagaa gagatctgcc ccaaggggac cacacactgt tatgatggcc 500

tcctcaggct caggggagga ggcatcttct ccaatctgag agtccaggga 550

tgcatgcccc agccaggttg caacctgctc aatgggacac aggaaattgg 600

gcccgtgggt atgactgaga actgcaatag gaaagatttt ctgacctgtc 650

atcgggggac caccattatg acacacggaa acttggctca agaacccact 700

gattggacca catcgaatac cgagatgtgc gaggtggggc aggtgtgtca 750

ggagacgctg ctgctcatag atgtaggact cacatcaacc ctggtgggga 800

caaaaggctg cagcactgtt ggggctcaaa attcccagaa gaccaccatc 850

cactcagccc ctcctggggt gcttgtggcc tcctataccc acttctgctc 900

ctcggacctg tgcaatagtg ccagcagcag cagcgttctg ctgaactccc 950

tccctcctca agctgcccct gtcccaggag accggcagtg tcctacctgt 1000

gtgcagcccc ttggaacctg ttcaagtggc tccccccgaa tgacctgccc 1050

caggggcgcc actcattgtt atgatgggta cattcatctc tcaggaggtg 1100

ggctgtccac caaaatgagc attcagggct gcgtggccca accttccagc 1150

ttcttgttga accacaccag acaaatcggg atcttctctg cgcgtgagaa 1200

gcgtgatgtg cagcctcctg cctctcagca tgagggaggt ggggctgagg 1250

gcctggagtc tctcacttgg ggggtggggc tggcactggc cccagcgctg 1300

tggtggggag tggtttgccc ttcctgctaa ctctattacc cccacgattc 1350

ttcaccgctg ctgaccaccc acactcaacc tccctctgac ctcataacct 1400

aatggccttg gacaccagat tctttcccat tctgtccatg aatcatcttc 1450

cccacacaca atcattcata tctactcacc taacagcaac actggggaga 1500

gcctggagca tccggacttg ccctatggga gaggggacgc tggaggagtg 1550

gctgcatgta tctgataata cagaccctgt cctttca 1587

<210> 162
<211> 437
<212> PRT
<213> Homo Sapien

<400> 162
Met Ser Ala Val Leu Leu Leu Ala Leu Leu Gly Phe Ile Leu Pro
1               5                   10                  15

Leu Pro Gly Val Gln Ala Leu Leu Cys Gln Phe Gly Thr Val Gln
                20                  25                  30

His Val Trp Lys Val Ser Asp Leu Pro Arg Gln Trp Thr Pro Lys
                35                  40                  45

Asn Thr Ser Cys Asp Ser Gly Leu Gly Cys Gln Asp Thr Leu Met
                50                  55                  60

Leu Ile Glu Ser Gly Pro Gln Val Ser Leu Val Leu Ser Lys Gly
                65                  70                  75

Cys Thr Glu Ala Lys Asp Gln Glu Pro Arg Val Thr Glu His Arg
                80                  85                  90

Met Gly Pro Gly Leu Ser Leu Ile Ser Tyr Thr Phe Val Cys Arg
                95                  100                 105

Gln Glu Asp Phe Cys Asn Asn Leu Val Asn Ser Leu Pro Leu Trp
                110                 115                 120

Ala Pro Gln Pro Pro Ala Asp Pro Gly Ser Leu Arg Cys Pro Val
                125                 130                 135

Cys Leu Ser Met Glu Gly Cys Leu Glu Gly Thr Thr Glu Glu Ile
                140                 145                 150

Cys Pro Lys Gly Thr Thr His Cys Tyr Asp Gly Leu Leu Arg Leu
                155                 160                 165

Arg Gly Gly Gly Ile Phe Ser Asn Leu Arg Val Gln Gly Cys Met
                170                 175                 180

Pro Gln Pro Gly Cys Asn Leu Leu Asn Gly Thr Gln Glu Ile Gly
                185                 190                 195

Pro Val Gly Met Thr Glu Asn Cys Asn Arg Lys Asp Phe Leu Thr
                200                 205                 210

Cys His Arg Gly Thr Thr Ile Met Thr His Gly Asn Leu Ala Gln
                215                 220                 225

Glu Pro Thr Asp Trp Thr Thr Ser Asn Thr Glu Met Cys Glu Val
                230                 235                 240

Gly Gln Val Cys Gln Glu Thr Leu Leu Leu Ile Asp Val Gly Leu
                245                 250                 255

Thr Ser Thr Leu Val Gly Thr Lys Gly Cys Ser Thr Val Gly Ala
                260                 265                 270

Gln Asn Ser Gln Lys Thr Thr Ile His Ser Ala Pro Pro Gly Val
                275                 280                 285

Leu Val Ala Ser Tyr Thr His Phe Cys Ser Ser Asp Leu Cys Asn
                290                 295                 300

Ser Ala Ser Ser Ser Ser Val Leu Leu Asn Ser Leu Pro Pro Gln
                305                 310                 315

Ala Ala Pro Val Pro Gly Asp Arg Gln Cys Pro Thr Cys Val Gln
                320                 325                 330

Pro Leu Gly Thr Cys Ser Ser Gly Ser Pro Arg Met Thr Cys Pro
                335                 340                 345

Arg Gly Ala Thr His Cys Tyr Asp Gly Tyr Ile His Leu Ser Gly
                350                 355                 360

Gly Gly Leu Ser Thr Lys Met Ser Ile Gln Gly Cys Val Ala Gln
                365                 370                 375

Pro Ser Ser Phe Leu Leu Asn His Thr Arg Gln Ile Gly Ile Phe
                380                 385                 390

Ser Ala Arg Glu Lys Arg Asp Val Gln Pro Pro Ala Ser Gln His
                395                 400                 405

Glu Gly Gly Gly Ala Glu Gly Leu Glu Ser Leu Thr Trp Gly Val
                410                 415                 420

Gly Leu Ala Leu Ala Pro Ala Leu Trp Trp Gly Val Val Cys Pro
                425                 430                 435

Ser Cys

<210> 163
<211> 2475
<212> DNA
<213> Homo Sapien

<400> 163
gaggatttgc cacagcagcg gatagagcag gagagcacca ccggagccct 50

tgagacatcc ttgagaagag ccacagcata agagactgcc ctgcttggtg 100

ttttgcagga tgatggtggc ccttcgagga gcttctgcat tgctggttct 150

gttccttgca gcttttctgc ccccgccgca gtgtacccag gacccagcca 200

tggtgcatta catctaccag cgctttcgag tcttggagca agggctggaa 250

aaatgtaccc aagcaacgag ggcatacatt caagaattcc aagagttctc 300

aaaaaatata tctgtcatgc tgggaagatg tcagacctac acaagtgagt 350

acaagagtgc agtgggtaac ttggcactga gagttgaacg tgcccaacgg 400

gagattgact acatacaata ccttcgagag gctgacgagt gcatcgtatc 450

agaggacaag acactggcag aaatgttgct ccaagaagct gaagaagaga 500

aaaagatccg gactctgctg aatgcaagct gtgacaacat gctgatgggc 550

ataaagtctt tgaaaatagt gaagaagatg atggacacac atggctcttg 600

gatgaaagat gctgtctata actctccaaa ggtgtactta ttaattggat 650

ccagaaacaa cactgtttgg gaatttgcaa acatacgggc attcatggag 700

gataacacca agccagctcc ccggaagcaa atcctaacac tttcctggca 750

gggaacaggc caagtgatct acaaaggttt tctatttttt cataaccaag 800

caacttctaa tgagataatc aaatataacc tgcagaagag gactgtggaa 850

gatcgaatgc tgctcccagg aggggtaggc cgagcattgg tttaccagca 900

ctccccctca acttacattg acctggctgt ggatgagcat gggctctggg 950

ccatccactc tgggccaggc acccatagcc atttggttct cacaaagatt 1000

gagccgggca cactgggagt ggagcattca tgggataccc catgcagaag 1050

ccaggatgct gaagcctcat tcctcttgtg tggggttctc tatgtggtct 1100

acagtactgg gggccagggc cctcatcgca tcacctgcat ctatgatcca 1150

ctgggcacta tcagtgagga ggacttgccc aacttgttct tccccaagag 1200

accaagaagt cactccatga tccattacaa ccccagagat aagcagctct 1250

atgcctggaa tgaaggaaac cagatcattt acaaactcca gacaaagaga 1300

aagctgcctc tgaagtaatg cattacagct gtgagaaaga gcactgtggc 1350

tttggcagct gttctacagg acagtgaggc tatagcccct tcacaatata 1400

gtatccctct aatcacacac aggaagagtg tgtagaagtg gaaatacgta 1450

tgcctccttt cccaaatgtc actgccttag gtatcttcca agagcttaga 1500

tgagagcata tcatcaggaa agtttcaaca atgtccatta ctcccccaaa 1550

cctcctggct ctcaaggatg accacattct gatacagcct acttcaagcc 1600

ttttgtttta ctgctcccca gcatttactg taactctgcc atcttccctc 1650

ccacaattag agttgtatgc cagcccctaa tattcaccac tggcttttct 1700

ctcccctggc ctttgctgaa gctcttccct ctttttcaaa tgtctattga 1750

tattctccca ttttcactgc ccaactaaaa tactattaat atttctttct 1800

tttcttttct tttttttgag acaaggtctc actatgttgc ccaggctggt 1850

ctcaaactcc agagctcaag agatcctcct gcctcagcct cctaagtacc 1900

tgggattaca ggcatgtgcc accacacctg gcttaaaata ctatttctta 1950

ttgaggttta acctctattt cccctagccc tgtccttcca ctaagcttgg 2000

tagatgtaat aataaagtga aaatattaac atttgaatat cgctttccag 2050

gtgtggagtg tttgcacatc attgaattct cgtttcacct ttgtgaaaca 2100

tgcacaagtc tttacagctg tcattctaga gtttaggtga gtaacacaat 2150

tacaaagtga aagatacagc tagaaaatac tacaaatccc atagtttttc 2200

cattgcccaa ggaagcatca aatacgtatg tttgttcacc tactcttata 2250

gtcaatgcgt tcatcgtttc agcctaaaaa taatagtctg tccctttagc 2300

cagttttcat gtctgcacaa gacctttcaa taggcctttc aaatgataat 2350

tcctccagaa aaccagtcta agggtgagga ccccaactct agcctcctct 2400

tgtcttgctg tcctctgttt ctctctttct gctttaaatt caataaaagt 2450

gacactgagc aaaaaaaaaa aaaaa 2475

<210> 164
<211> 402

<212> PRT
<213> Homo Sapien

<400> 164
Met Met Val Ala Leu Arg Gly Ala Ser Ala Leu Leu Val Leu Phe
1               5                   10                  15

Leu Ala Ala Phe Leu Pro Pro Pro Gln Cys Thr Gln Asp Pro Ala
                20                  25                  30

Met Val His Tyr Ile Tyr Gln Arg Phe Arg Val Leu Glu Gln Gly
                35                  40                  45

Leu Glu Lys Cys Thr Gln Ala Thr Arg Ala Tyr Ile Gln Glu Phe
                50                  55                  60

Gln Glu Phe Ser Lys Asn Ile Ser Val Met Leu Gly Arg Cys Gln
                65                  70                  75

Thr Tyr Thr Ser Glu Tyr Lys Ser Ala Val Gly Asn Leu Ala Leu
                80                  85                  90

Arg Val Glu Arg Ala Gln Arg Glu Ile Asp Tyr Ile Gln Tyr Leu
                95                  100                 105

Arg Glu Ala Asp Glu Cys Ile Val Ser Glu Asp Lys Thr Leu Ala
                110                 115                 120

Glu Met Leu Leu Gln Glu Ala Glu Glu Glu Lys Lys Ile Arg Thr
                125                 130                 135

Leu Leu Asn Ala Ser Cys Asp Asn Met Leu Met Gly Ile Lys Ser
                140                 145                 150

Leu Lys Ile Val Lys Lys Met Met Asp Thr His Gly Ser Trp Met
                155                 160                 165

Lys Asp Ala Val Tyr Asn Ser Pro Lys Val Tyr Leu Leu Ile Gly
                170                 175                 180

Ser Arg Asn Asn Thr Val Trp Glu Phe Ala Asn Ile Arg Ala Phe
                185                 190                 195

Met Glu Asp Asn Thr Lys Pro Ala Pro Arg Lys Gln Ile Leu Thr
                200                 205                 210

Leu Ser Trp Gln Gly Thr Gly Gln Val Ile Tyr Lys Gly Phe Leu
                215                 220                 225

Phe Phe His Asn Gln Ala Thr Ser Asn Glu Ile Ile Lys Tyr Asn
                230                 235                 240

Leu Gln Lys Arg Thr Val Glu Asp Arg Met Leu Leu Pro Gly Gly
                245                 250                 255

Val Gly Arg Ala Leu Val Tyr Gln His Ser Pro Ser Thr Tyr Ile
                260                 265                 270

Asp Leu Ala Val Asp Glu His Gly Leu Trp Ala Ile His Ser Gly
                275                 280                 285

Pro Gly Thr His Ser His Leu Val Leu Thr Lys Ile Glu Pro Gly
                290                 295                 300

Thr Leu Gly Val Glu His Ser Trp Asp Thr Pro Cys Arg Ser Gln
                305                 310                 315

Asp Ala Glu Ala Ser Phe Leu Leu Cys Gly Val Leu Tyr Val Val

                320                 325                 330

Tyr Ser Thr Gly Gly Gln Gly Pro His Arg Ile Thr Cys Ile Tyr
                335                 340                 345

Asp Pro Leu Gly Thr Ile Ser Glu Glu Asp Leu Pro Asn Leu Phe
                350                 355                 360

Phe Pro Lys Arg Pro Arg Ser His Ser Met Ile His Tyr Asn Pro
                365                 370                 375

Arg Asp Lys Gln Leu Tyr Ala Trp Asn Glu Gly Asn Gln Ile Ile
                380                 385                 390

Tyr Lys Leu Gln Thr Lys Arg Lys Leu Pro Leu Lys
                395                 400

<210> 165
<211> 1415
<212> DNA
<213> Homo Sapien

<400> 165
tggcctcccc agcttgccag gcacaaggct gagcgggagg aagcgagagg 50

catctaagca ggcagtgttt tgccttcacc ccaagtgacc atgagaggtg 100

ccacgcgagt ctcaatcatg ctcctcctag taactgtgtc tgactgtgct 150

gtgatcacag gggcctgtga gcgggatgtc cagtgtgggg caggcacctg 200

ctgtgccatc agcctgtggc ttcgagggct gcggatgtgc accccgctgg 250

ggcgggaagg cgaggagtgc caccccggca gccacaaggt ccccttcttc 300

aggaaacgca agcaccacac ctgtccttgc ttgcccaacc tgctgtgctc 350

caggttcccg gacggcaggt accgctgctc catggacttg aagaacatca 400

atttttaggc gcttgcctgg tctcaggata cccaccatcc ttttcctgag 450

cacagcctgg atttttattt ctgccatgaa acccagctcc catgactctc 500

ccagtcccta cactgactac cctgatctct cttgtctagt acgcacatat 550

gcacacaggc agacatacct cccatcatga catggtcccc aggctggcct 600

gaggatgtca cagcttgagg ctgtggtgtg aaaggtggcc agcctggttc 650

tcttccctgc tcaggctgcc agagaggtgg taaatggcag aaaggacatt 700

ccccctcccc tccccaggtg acctgctctc tttcctgggc cctgcccctc 750

tccccacatg tatccctcgg tctgaattag acattcctgg gcacaggctc 800

ttgggtgcat tgctcagagt cccaggtcct ggcctgaccc tcaggccctt 850

cacgtgaggt ctgtgaggac caatttgtgg gtagttcatc ttccctcgat 900

tggttaactc cttagtttca gaccacagac tcaagattgg ctcttcccag 950

agggcagcag acagtcaccc caaggcaggt gtagggagcc cagggaggcc 1000

aatcagcccc ctgaagactc tggtcccagt cagcctgtgg cttgtggcct 1050

gtgacctgtg accttctgcc agaattgtca tgcctctgag gccccctctt 1100

accacacttt accagttaac cactgaagcc cccaattccc acagcttttc 1150

cattaaaatg caaatggtgg tggttcaatc taatctgata ttgacatatt 1200

agaaggcaat tagggtgttt ccttaaacaa ctcctttcca aggatcagcc 1250

ctgagagcag gttggtgact ttgaggaggg cagtcctctg tccagattgg 1300

ggtgggagca agggacaggg agcagggcag gggctgaaag gggcactgat 1350

tcagaccagg gaggcaacta cacaccaaca tgctggcttt agaataaaag 1400

caccaactga aaaaa 1415

<210> 166
<211> 105
<212> PRT
<213> Homo Sapien

<400> 166
Met Arg Gly Ala Thr Arg Val Ser Ile Met Leu Leu Leu Val Thr
1               5                   10                  15

Val Ser Asp Cys Ala Val Ile Thr Gly Ala Cys Glu Arg Asp Val
                20                  25                  30

Gln Cys Gly Ala Gly Thr Cys Cys Ala Ile Ser Leu Trp Leu Arg
                35                  40                  45

Gly Leu Arg Met Cys Thr Pro Leu Gly Arg Glu Gly Glu Glu Cys
                50                  55                  60

His Pro Gly Ser His Lys Val Pro Phe Phe Arg Lys Arg Lys His
                65                  70                  75

His Thr Cys Pro Cys Leu Pro Asn Leu Leu Cys Ser Arg Phe Pro
                80                  85                  90

Asp Gly Arg Tyr Arg Cys Ser Met Asp Leu Lys Asn Ile Asn Phe
                95                  100                 105

<210> 167
<211> 1371
<212> DNA
<213> Homo Sapien

<400> 167
aactcaaact cctctctctg ggaaaacgcg gtgcttgctc ctcccggagt 50

ggccttggca gggtgttgga gccctcggtc tgccccgtcc ggtctctggg 100

gccaaggctg ggtttccctc atgtatggca agagctctac tcgtgcggtg 150

cttcttctcc ttggcataca gctcacagct ctttggccta tagcagctgt 200

ggaaatttat acctcccggg tgctggaggc tgttaatggg acagatgctc 250

ggttaaaatg cactttctcc agctttgccc ctgtgggtga tgctctaaca 300

gtgacctgga attttcgtcc tctagacggg ggacctgagc agtttgtatt 350

ctactaccac atagatccct tccaacccat gagtgggcgg tttaaggacc 400

gggtgtcttg ggatgggaat cctgagcggt acgatgcctc catccttctc 450

tggaaactgc agttcgacga caatgggaca tacacctgcc aggtgaagaa 500

cccacctgat gttgatgggg tgatagggga gatccggctc agcgtcgtgc 550

acactgtacg cttctctgag atccacttcc tggctctggc cattggctct 600

gcctgtgcac tgatgatcat aatagtaatt gtagtggtcc tcttccagca 650

ttaccggaaa aagcgatggg ccgaaagagc tcataaagtg gtggagataa 700

aatcaaaaga agaggaaagg ctcaaccaag agaaaaaggt ctctgtttat 750

ttagaagaca cagactaaca attttagatg gaagctgaga tgatttccaa 800

gaacaagaac cctagtattt cttgaagtta atggaaactt ttctttggct 850

tttccagttg tgacccgttt tccaaccagt tctgcagcat attagattct 900

agacaagcaa cacccctctg gagccagcac agtgctcctc catatcacca 950

gtcatacaca gcctcattat taaggtctta tttaatttca gagtgtaaat 1000

tttttcaagt gctcattagg ttttataaac aagaagctac atttttgccc 1050

ttaagacact acttacagtg ttatgacttg tatacacata tattggtatc 1100

aaaggggata aaagccaatt tgtctgttac atttcctttc acgtatttct 1150

tttagcagca cttctgctac taaagttaat gtgtttactc tctttccttc 1200

ccacattctc aattaaaagg tgagctaagc ctcctcggtg tttctgatta 1250

acagtaaatc ctaaattcaa actgttaaat gacattttta tttttatgtc 1300

tctccttaac tatgagacac atcttgtttt actgaatttc tttcaatatt 1350

ccaggtgata gatttttgtc g 1371

<210> 168
<211> 215
<212> PRT
<213> Homo Sapien

<400> 168
Met Tyr Gly Lys Ser Ser Thr Arg Ala Val Leu Leu Leu Leu Gly
1               5                   10                  15

Ile Gln Leu Thr Ala Leu Trp Pro Ile Ala Ala Val Glu Ile Tyr
                20                  25                  30

Thr Ser Arg Val Leu Glu Ala Val Asn Gly Thr Asp Ala Arg Leu
                35                  40                  45

Lys Cys Thr Phe Ser Ser Phe Ala Pro Val Gly Asp Ala Leu Thr
                50                  55                  60

Val Thr Trp Asn Phe Arg Pro Leu Asp Gly Gly Pro Glu Gln Phe
                65                  70                  75

Val Phe Tyr Tyr His Ile Asp Pro Phe Gln Pro Met Ser Gly Arg
                80                  85                  90

Phe Lys Asp Arg Val Ser Trp Asp Gly Asn Pro Glu Arg Tyr Asp
                95                  100                 105

Ala Ser Ile Leu Leu Trp Lys Leu Gln Phe Asp Asp Asn Gly Thr
                110                 115                 120

Tyr Thr Cys Gln Val Lys Asn Pro Pro Asp Val Asp Gly Val Ile
                125                 130                 135

Gly Glu Ile Arg Leu Ser Val Val His Thr Val Arg Phe Ser Glu

                140                 145                 150

Ile His Phe Leu Ala Leu Ala Ile Gly Ser Ala Cys Ala Leu Met
                155                 160                 165

Ile Ile Ile Val Ile Val Val Val Leu Phe Gln His Tyr Arg Lys
                170                 175                 180

Lys Arg Trp Ala Glu Arg Ala His Lys Val Val Glu Ile Lys Ser
                185                 190                 195

Lys Glu Glu Glu Arg Leu Asn Gln Glu Lys Lys Val Ser Val Tyr
                200                 205                 210

Leu Glu Asp Thr Asp
                215

<210> 169
<211> 2213
<212> DNA
<213> Homo Sapien

<400> 169
gagcgaacat ggcagcgcgt tggcggtttt ggtgtgtctc tgtgaccatg 50

gtggtggcgc tgctcatcgt ttgcgacgtt ccctcagcct ctgcccaaag 100

aaagaaggag atggtgttat ctgaaaaggt tagtcagctg atggaatgga 150

ctaacaaaag acctgtaata agaatgaatg gagacaagtt ccgtcgcctt 200

gtgaaagccc caccgagaaa ttactccgtt atcgtcatgt tcactgctct 250

ccaactgcat agacagtgtg tcgtttgcaa gcaagctgat gaagaattcc 300

agatcctggc aaactcctgg cgatactcca gtgcattcac caacaggata 350

ttttttgcca tggtggattt tgatgaaggc tctgatgtat ttcagatgct 400

aaacatgaat tcagctccaa ctttcatcaa ctttcctgca aaagggaaac 450

ccaaacgggg tgatacatat gagttacagg tgcggggttt ttcagctgag 500

cagattgccc ggtggatcgc cgacagaact gatgtcaata ttagagtgat 550

tagaccccca aattatgctg gtccccttat gttgggattg cttttggctg 600

ttattggtgg acttgtgtat cttcgaagaa gtaatatgga atttctcttt 650

aataaaactg gatgggcttt tgcagctttg tgttttgtgc ttgctatgac 700

atctggtcaa atgtggaacc atataagagg accaccatat gcccataaga 750

atccccacac gggacatgtg aattatatcc atggaagcag tcaagcccag 800

tttgtagctg aaacacacat tgttcttctg tttaatggtg gagttacctt 850

aggaatggtg cttttatgtg aagctgctac ctctgacatg gatattggaa 900

agcgaaagat aatgtgtgtg gctggtattg gacttgttgt attattcttc 950

agttggatgc tctctatttt tagatctaaa tatcatggct acccatacag 1000

ctttctgatg agttaaaaag gtcccagaga tatatagaca ctggagtact 1050

ggaaattgaa aaacgaaaat cgtgtgtgtt tgaaaagaag aatgcaactt 1100

gtatattttg tattacctct ttttttcaag tgatttaaat agttaatcat 1150

ttaaccaaag aagatgtgta gtgccttaac aagcaatcct ctgtcaaaat 1200

ctgaggtatt tgaaaataat tatcctctta accttctctt cccagtgaac 1250

tttatggaac atttaattta gtacaattaa gtatattata aaaattgtaa 1300

aactactact ttgttttagt tagaacaaag ctcaaaacta ctttagttaa 1350

cttggtcatc tgattttata ttgccttatc caaagatggg gaaagtaagt 1400

cctgaccagg tgttcccaca tatgcctgtt acagataact acattaggaa 1450

ttcattctta gcttcttcat ctttgtgtgg atgtgtatac tttacgcatc 1500

tttccttttg agtagagaaa ttatgtgtgt catgtggtct tctgaaaatg 1550

gaacaccatt cttcagagca cacgtctagc cctcagcaag acagttgttt 1600

ctcctcctcc ttgcatattt cctactgcgc tccagcctga gtgatagagt 1650

gagactctgt ctcaaaaaaa agtatctcta aatacaggat tataatttct 1700

gcttgagtat ggtgttaact accttgtatt tagaaagatt tcagattcat 1750

tccatctcct tagttttctt ttaaggtgac ccatctgtga taaaaatata 1800

gcttagtgct aaaatcagtg taacttatac atggcctaaa atgtttctac 1850

aaattagagt ttgtcactta ttccatttgt acctaagaga aaaataggct 1900

cagttagaaa aggactccct ggccaggcgc agtgacttac gcctgtaatc 1950

tcagcacttt gggaggccaa ggcaggcaga tcacgaggtc aggagttcga 2000

gaccatcctg gccaacatgg tgaaaccccg tctctactaa aaatataaaa 2050

attagctggg tgtggtggca ggagcctgta atcccagcta cacaggaggc 2100

tgaggcacga gaatcacttg aactcaggag atggaggttt cagtgagccg 2150

agatcacgcc actgcactcc agcctggcaa cagagcgaga ctccatctca 2200

aaaaaaaaaa aaa 2213

<210> 170
<211> 335
<212> PRT
<213> Homo Sapien

<400> 170
Met Ala Ala Arg Trp Arg Phe Trp Cys Val Ser Val Thr Met Val
1               5                   10                  15

Val Ala Leu Leu Ile Val Cys Asp Val Pro Ser Ala Ser Ala Gln
                20                  25                  30

Arg Lys Lys Glu Met Val Leu Ser Glu Lys Val Ser Gln Leu Met
                35                  40                  45

Glu Trp Thr Asn Lys Arg Pro Val Ile Arg Met Asn Gly Asp Lys
                50                  55                  60

Phe Arg Arg Leu Val Lys Ala Pro Pro Arg Asn Tyr Ser Val Ile
                65                  70                  75

Val Met Phe Thr Ala Leu Gln Leu His Arg Gln Cys Val Val Cys
                80                  85                  90

Lys Gln Ala Asp Glu Glu Phe Gln Ile Leu Ala Asn Ser Trp Arg
                95                  100                 105

Tyr Ser Ser Ala Phe Thr Asn Arg Ile Phe Phe Ala Met Val Asp
                110                 115                 120

Phe Asp Glu Gly Ser Asp Val Phe Gln Met Leu Asn Met Asn Ser
                125                 130                 135

Ala Pro Thr Phe Ile Asn Phe Pro Ala Lys Gly Lys Pro Lys Arg
                140                 145                 150

Gly Asp Thr Tyr Glu Leu Gln Val Arg Gly Phe Ser Ala Glu Gln
                155                 160                 165

Ile Ala Arg Trp Ile Ala Asp Arg Thr Asp Val Asn Ile Arg Val
                170                 175                 180

Ile Arg Pro Pro Asn Tyr Ala Gly Pro Leu Met Leu Gly Leu Leu
                185                 190                 195

Leu Ala Val Ile Gly Gly Leu Val Tyr Leu Arg Arg Ser Asn Met
                200                 205                 210

Glu Phe Leu Phe Asn Lys Thr Gly Trp Ala Phe Ala Ala Leu Cys
                215                 220                 225

Phe Val Leu Ala Met Thr Ser Gly Gln Met Trp Asn His Ile Arg
                230                 235                 240

Gly Pro Pro Tyr Ala His Lys Asn Pro His Thr Gly His Val Asn
                245                 250                 255

Tyr Ile His Gly Ser Ser Gln Ala Gln Phe Val Ala Glu Thr His
                260                 265                 270

Ile Val Leu Leu Phe Asn Gly Gly Val Thr Leu Gly Met Val Leu
                275                 280                 285

Leu Cys Glu Ala Ala Thr Ser Asp Met Asp Ile Gly Lys Arg Lys
                290                 295                 300

Ile Met Cys Val Ala Gly Ile Gly Leu Val Val Leu Phe Phe Ser
                305                 310                 315

Trp Met Leu Ser Ile Phe Arg Ser Lys Tyr His Gly Tyr Pro Tyr
                320                 325                 330

Ser Phe Leu Met Ser
                335

<210> 171
<211> 771
<212> DNA
<213> Homo Sapien

<400> 171
ctccactgca accacccaga gccatggctc cccgaggctg catcgtagct 50

gtctttgcca ttttctgcat ctccaggctc ctctgctcac acggagcccc 100

agtggccccc atgactcctt acctgatgct gtgccagcca cacaagagat 150

gtggggacaa gttctacgac cccctgcagc actgttgcta tgatgatgcc 200

gtcgtgccct tggccaggac ccagacgtgt ggaaactgca ccttcagagt 250

ctgctttgag cagtgctgcc cctggacctt catggtgaag ctgataaacc 300

agaactgcga ctcagcccgg acctcggatg acaggctttg tcgcagtgtc 350

agctaatgga acatcagggg aacgatgact cctggattct ccttcctggg 400

tgggcctgga gaaagaggct ggtgttacct gagatctggg atgctgagtg 450

gctgtttggg ggccagagaa acacacactc aactgcccac ttcattctgt 500

gacctgtctg aggcccaccc tgcagctgcc ctgaggaggc ccacaggtcc 550

ccttctagaa ttctggacag catgagatgc gtgtgctgat gggggcccag 600

ggactctgaa ccctcctgat gacccctatg gccaacatca acccggcacc 650

accccaaggc tggctgggga acccttcacc cttctgtgag attttccatc 700

atctcaagtt ctcttctatc caggagcaaa gcacaggatc ataataaatt 750

tatgtacttt ataaatgaaa a 771

<210> 172
<211> 110
<212> PRT
<213> Homo Sapien

<400> 172
Met Ala Pro Arg Gly Cys Ile Val Ala Val Phe Ala Ile Phe Cys
1               5                   10                  15

Ile Ser Arg Leu Leu Cys Ser His Gly Ala Pro Val Ala Pro Met
                20                  25                  30

Thr Pro Tyr Leu Met Leu Cys Gln Pro His Lys Arg Cys Gly Asp
                35                  40                  45

Lys Phe Tyr Asp Pro Leu Gln His Cys Cys Tyr Asp Asp Ala Val
                50                  55                  60

Val Pro Leu Ala Arg Thr Gln Thr Cys Gly Asn Cys Thr Phe Arg
                65                  70                  75

Val Cys Phe Glu Gln Cys Cys Pro Trp Thr Phe Met Val Lys Leu
                80                  85                  90

Ile Asn Gln Asn Cys Asp Ser Ala Arg Thr Ser Asp Asp Arg Leu
                95                  100                 105

Cys Arg Ser Val Ser
                110

<210> 173
<211> 2044
<212> DNA
<213> Homo Sapien

<400> 173
gggggcgggt gcctggagca cggcgctggg gccgcccgca gcgctcactc 50

gctcgcactc agtcgcggga ggcttccccg cgccggccgc gtcccgcccg 100

ctccccggca ccagaagttc ctctgcgcgt ccgacggcga catgggcgtc 150

cccacggccc tggaggccgg cagctggcgc tggggatccc tgctcttcgc 200

tctcttcctg gctgcgtccc taggtccggt ggcagccttc aaggtcgcca 250

cgccgtattc cctgtatgtc tgtcccgagg ggcagaacgt caccctcacc 300
tgcaggctct tgggccctgt ggacaaaggg cacgatgtga ccttctacaa 350
gacgtggtac cgcagctcga ggggcgaggt gcagacctgc tcagagcgcc 400
ggcccatccg caacctcacg ttccaggacc ttcacctgca ccatggaggc 450
caccaggctg ccaacaccag ccacgacctg gctcagcgcc acgggctgga 500
gtcggcctcc gaccaccatg gcaacttctc catcaccatg cgcaacctga 550
ccctgctgga tagcggcctc tactgctgcc tggtggtgga gatcaggcac 600
caccactcgg agcacagggt ccatggtgcc atggagctgc aggtgcagac 650
aggcaaagat gcaccatcca actgtgtggt gtacccatcc tcctcccagg 700
atagtgaaaa catcacggct gcagccctgg ctacgggtgc ctgcatcgta 750
ggaatcctct gcctccccct catcctgctc ctggtctaca agcaaaggca 800
ggcagcctcc aaccgccgtg cccaggagct ggtgcggatg gacagcaaca 850
ttcaagggat tgaaaacccc ggctttgaag cctcaccacc tgcccagggg 900
atacccgagg ccaaagtcag gcaccccctg tcctatgtgg cccagcggca 950
gccttctgag tctgggcggc atctgctttc ggagcccagc acccccctgt 1000
ctcctccagg ccccggagac gtcttcttcc catccctgga ccctgtccct 1050
gactctccaa actttgaggt catctagccc agctggggga cagtgggctg 1100
ttgtggctgg gtctggggca ggtgcatttg agccagggct ggctctgtga 1150
gtggcctcct tggcctcggc cctggttccc tccctcctgc tctgggctca 1200
gatactgtga catcccagaa gcccagcccc tcaacccctc tggatgctac 1250
atggggatgc tggacggctc agcccctgtt ccaaggattt tggggtgctg 1300
agattctccc ctagagacct gaaattcacc agctacagat gccaaatgac 1350
ttacatctta agaagtctca gaacgtccag cccttcagca gctctcgttc 1400
tgagacatga gccttgggat gtggcagcat cagtgggaca agatggacac 1450
tgggccaccc tcccaggcac cagacacagg gcacggtgga gagacttctc 1500
ccccgtggcc gccttggctc ccccgttttg cccgaggctg ctcttctgtc 1550
agacttcctc tttgtaccac agtggctctg gggccaggcc tgcctgccca 1600
ctggccatcg ccaccttccc cagctgcctc ctaccagcag tttctctgaa 1650
gatctgtcaa caggttaagt caatctgggg cttccactgc ctgcattcca 1700
gtccccagag cttggtggtc ccgaaacggg aagtacatat tggggcatgg 1750
tggcctccgt gagcaaatgg tgtcttgggc aatctgaggc caggacagat 1800
gttgccccac ccactggaga tggtgctgag ggaggtgggt ggggccttct 1850
gggaaggtga gtggagaggg gcacctgccc cccgccctcc ccatccccta 1900
ctcccactgc tcagcgcggg ccattgcaag ggtgccacac aatgtcttgt 1950

ccaccctggg acacttctga gtatgaagcg ggatgctatt aaaaactaca 2000

tggggaaaaa aaaaaaaaaa aaaaaaaaaa aaaaaaaaaa aaga 2044

<210> 174
<211> 311
<212> PRT
<213> Homo Sapien

<400> 174
Met Gly Val Pro Thr Ala Leu Glu Ala Gly Ser Trp Arg Trp Gly
1               5                   10                  15

Ser Leu Leu Phe Ala Leu Phe Leu Ala Ala Ser Leu Gly Pro Val
                20                  25                  30

Ala Ala Phe Lys Val Ala Thr Pro Tyr Ser Leu Tyr Val Cys Pro
                35                  40                  45

Glu Gly Gln Asn Val Thr Leu Thr Cys Arg Leu Leu Gly Pro Val
                50                  55                  60

Asp Lys Gly His Asp Val Thr Phe Tyr Lys Thr Trp Tyr Arg Ser
                65                  70                  75

Ser Arg Gly Glu Val Gln Thr Cys Ser Glu Arg Arg Pro Ile Arg
                80                  85                  90

Asn Leu Thr Phe Gln Asp Leu His Leu His His Gly Gly His Gln
                95                  100                 105

Ala Ala Asn Thr Ser His Asp Leu Ala Gln Arg His Gly Leu Glu
                110                 115                 120

Ser Ala Ser Asp His His Gly Asn Phe Ser Ile Thr Met Arg Asn
                125                 130                 135

Leu Thr Leu Leu Asp Ser Gly Leu Tyr Cys Cys Leu Val Val Glu
                140                 145                 150

Ile Arg His His His Ser Glu His Arg Val His Gly Ala Met Glu
                155                 160                 165

Leu Gln Val Gln Thr Gly Lys Asp Ala Pro Ser Asn Cys Val Val
                170                 175                 180

Tyr Pro Ser Ser Ser Gln Asp Ser Glu Asn Ile Thr Ala Ala Ala
                185                 190                 195

Leu Ala Thr Gly Ala Cys Ile Val Gly Ile Leu Cys Leu Pro Leu
                200                 205                 210

Ile Leu Leu Leu Val Tyr Lys Gln Arg Gln Ala Ala Ser Asn Arg
                215                 220                 225

Arg Ala Gln Glu Leu Val Arg Met Asp Ser Asn Ile Gln Gly Ile
                230                 235                 240

Glu Asn Pro Gly Phe Glu Ala Ser Pro Pro Ala Gln Gly Ile Pro
                245                 250                 255

Glu Ala Lys Val Arg His Pro Leu Ser Tyr Val Ala Gln Arg Gln
                260                 265                 270

Pro Ser Glu Ser Gly Arg His Leu Leu Ser Glu Pro Ser Thr Pro
                275                 280                 285

Leu Ser Pro Pro Gly Pro Gly Asp Val Phe Phe Pro Ser Leu Asp

                290                 295                 300

Pro Val Pro Asp Ser Pro Asn Phe Glu Val Ile
                305                 310

<210> 175
<211> 693
<212> DNA
<213> Homo Sapien

<400> 175
ctagcctgcg ccaaggggta gtgagaccgc gcggcaacag cttgcggctg 50

cggggagctc ccgtgggcgc tccgctggct gtgcaggcgg ccatggattc 100

cttgcggaaa atgctgatct cagtcgcaat gctgggcgca ggggctggcg 150

tgggctacgc gctcctcgtt atcgtgaccc cgggagagcg gcggaagcag 200

gaaatgctaa aggagatgcc actgcaggac ccaaggagca gggaggaggc 250

ggccaggacc cagcagctat tgctggccac tctgcaggag gcagcgacca 300

cgcaggagaa cgtggcctgg aggaagaact ggatggttgg cggcgaaggc 350

ggcgccagcg ggaggtcacc gtgagaccgg acttgcctcc gtgggcgccg 400

gaccttggct tgggcgcagg aatccgaggc agcctttctc cttcgtgggc 450

ccagcggaga gtccggaccg agataccatg ccaggactct ccggggtcct 500

gtgagctgcc gtcgggtgag cacgtttccc ccaaaccctg gactgactgc 550

tttaaggtcc gcaaggcggg ccagggccga gacgcgagtc ggatgtggtg 600

aactgaaaga accaataaaa tcatgttcct ccaaaaaaaa aaaaaaaaaa 650

aaaaaaaaaa aaaaaaaaaa aaaaaaaaaa aaaaaaaaaa aaa 693

<210> 176
<211> 93
<212> PRT
<213> Homo Sapien

<400> 176

<210> 177
<211> 1700

<212> DNA
<213> Homo Sapien

<400> 177
gccaggcagg tgggcctcag gaggtgcctc caggcggcca gtgggcctga 50

ggccccagca agggctaggg tccatctcca gtcccaggac acagcagcgg 100

ccaccatggc cacgcctggg ctccagcagc atcagagcag cccctgtggt 150

tggcagcaaa gttcagcttg gctgggcccg ctgtgagggg cttcgcgcta 200

cgccctgcgg tgtcccgagg gctgaggtct cctcatcttc tccctagcag 250

tggatgagca acccaacggg ggcccgggga ggggaactgg ccccgaggga 300

gaggaacccc aaagccacat ctgtagccag gatgagcagt gtgaatccag 350

gcagccccca ggaccgggga ggcacaggtg gcccccacca cccggaggag 400

cagctcctgc ccctgtccgg gggatgactg attctcctcc gccaggccac 450

ccagaggaga aggccacccc gcctggaggc acaggccatg aggggctctc 500

aggaggtgct gctgatgtgg cttctggtgt tggcagtggg cggcacagag 550

cacgcctacc ggcccggccg tagggtgtgt gctgtccggg ctcacgggga 600

ccctgtctcc gagtcgttcg tgcagcgtgt gtaccagccc ttcctcacca 650

cctgcgacgg gcaccgggcc tgcagcacct accgaaccat ctataggacc 700

gcctaccgcc gcagccctgg gctggcccct gccaggcctc gctacgcgtg 750

ctgccccggc tggaagagga ccagcgggct tcctggggcc tgtggagcag 800

caatatgcca gccgccatgc cggaacggag ggagctgtgt ccagcctggc 850

cgctgccgct gccctgcagg atggcggggt gacacttgcc agtcagatgt 900

ggatgaatgc agtgctagga ggggcggctg tccccagcgc tgcatcaaca 950

ccgccggcag ttactggtgc cagtgttggg aggggcacag cctgtctgca 1000

gacggtacac tctgtgtgcc caagggaggg ccccccaggg tggcccccaa 1050

cccgacagga gtggacagtg caatgaagga agaagtgcag aggctgcagt 1100

ccagggtgga cctgctggag gagaagctgc agctggtgct ggccccactg 1150

cacagcctgg cctcgcaggc actggagcat gggctcccgg accccggcag 1200

cctcctggtg cactccttcc agcagctcgg ccgcatcgac tccctgagcg 1250

agcagatttc cttcctggag gagcagctgg ggtcctgctc ctgcaagaaa 1300

gactcgtgac tgcccagcgc tccaggctgg actgagcccc tcacgccgcc 1350

ctgcagcccc catgcccctg cccaacatgc tgggggtcca gaagccacct 1400

cggggtgact gagcggaagg ccaggcaggg ccttcctcct cttcctcctc 1450

cccttcctcg ggaggctccc cagaccctgg catgggatgg gctgggatct 1500

tctctgtgaa tccacccctg gctaccccca ccctggctac cccaacggca 1550

tcccaaggcc aggtggaccc tcagctgagg gaaggtacga gctccctgct 1600

ggagcctggg acccatggca caggccaggc agcccggagg ctgggtgggg 1650

cctcagtggg ggctgctgcc tgacccccag cacaataaaa atgaaacgtg 1700

<210> 178
<211> 273
<212> PRT
<213> Homo Sapien

<400> 178

<210> 179

<211> 2052
<212> DNA
<213> Homo Sapien

<400> 179
gacagctgtg tctcgatgga gtagactctc agaacagcgc agtttgccct 50

ccgctcacgc agagcctctc cgtggcttcc gcaccttgag cattaggcca 100

gttctcctct tctctctaat ccatccgtca cctctcctgt catccgtttc 150

catgccgtga ggtccattca cagaacacat ccatggctct catgctcagt 200

ttggttctga gtctcctcaa gctgggatca gggcagtggc aggtgtttgg 250

gccagacaag cctgtccagg ccttggtggg ggaggacgca gcattctcct 300

gtttcctgtc tcctaagacc aatgcagagg ccatggaagt gcggttcttc 350

aggggccagt tctctagcgt ggtccacctc tacagggacg ggaaggacca 400

gccatttatg cagatgccac agtatcaagg caggacaaaa ctggtgaagg 450

attctattgc ggaggggcgc atctctctga ggctggaaaa cattactgtg 500

ttggatgctg gcctctatgg gtgcaggatt agttcccagt cttactacca 550

gaaggccatc tgggagctac aggtgtcagc actgggctca gttcctctca 600

tttccatcac gggatatgtt gatagagaca tccagctact ctgtcagtcc 650

tcgggctggt tcccccggcc cacagcgaag tggaaaggtc cacaaggaca 700

ggatttgtcc acagactcca ggacaaacag agacatgcat ggcctgtttg 750

atgtggagat ctctctgacc gtccaagaga acgccgggag catatcctgt 800

tccatgcggc atgctcatct gagccgagag gtggaatcca gggtacagat 850

aggagatacc tttttcgagc ctatatcgtg gcacctggct accaaagtac 900

tgggaatact ctgctgtggc ctattttttg gcattgttgg actgaagatt 950

ttcttctcca aattccagtg gaaaatccag gcggaactgg actggagaag 1000

aaagcacgga caggcagaat tgagagacgc ccggaaacac gcagtggagg 1050

tgactctgga tccagagacg gctcacccga agctctgcgt ttctgatctg 1100

aaaactgtaa cccatagaaa agctccccag gaggtgcctc actctgagaa 1150

gagatttaca aggaagagtg tggtggcttc tcagagtttc caagcaggga 1200

aacattactg ggaggtggac ggaggacaca ataaaaggtg gcgcgtggga 1250

gtgtgccggg atgatgtgga caggaggaag gagtacgtga ctttgtctcc 1300

cgatcatggg tactgggtcc tcagactgaa tggagaacat ttgtatttca 1350

cattaaatcc ccgttttatc agcgtcttcc ccaggacccc acctacaaaa 1400

ataggggtct tcctggacta tgagtgtggg accatctcct tcttcaacat 1450

aaatgaccag tcccttattt ataccctgac atgtcggttt gaaggcttat 1500

tgaggcccta cattgagtat ccgtcctata atgagcaaaa tggaactccc 1550

atagtcatct gcccagtcac ccaggaatca gagaaagagg cctcttggca 1600

aagggcctct gcaatcccag agacaagcaa cagtgagtcc tcctcacagg 1650

caaccacgcc cttcctcccc aggggtgaaa tgtaggatga atcacatccc 1700

acattcttct ttagggatat taaggtctct ctcccagatc caaagtcccg 1750

cagcagccgg ccaaggtggc ttccagatga agggggactg gcctgtccac 1800

atgggagtca ggtgtcatgg ctgccctgag ctgggaggga agaaggctga 1850

cattacattt agtttgctct cactccatct ggctaagtga tcttgaaata 1900

ccacctctca ggtgaagaac cgtcaggaat tcccatctca caggctgtgg 1950

tgtagattaa gtagacaagg aatgtgaata atgcttagat cttattgatg 2000

acagagtgta tcctaatggt ttgttcatta tattacactt tcagtaaaaa 2050

aa 2052

<210> 180
<211> 500
<212> PRT
<213> Homo Sapien

<400> 180
Met Ala Leu Met Leu Ser Leu Val Leu Ser Leu Leu Lys Leu Gly
1               5                   10                  15

Ser Gly Gln Trp Gln Val Phe Gly Pro Asp Lys Pro Val Gln Ala
                20                  25                  30

Leu Val Gly Glu Asp Ala Ala Phe Ser Cys Phe Leu Ser Pro Lys
                35                  40                  45

Thr Asn Ala Glu Ala Met Glu Val Arg Phe Phe Arg Gly Gln Phe
                50                  55                  60

Ser Ser Val Val His Leu Tyr Arg Asp Gly Lys Asp Gln Pro Phe
                65                  70                  75

Met Gln Met Pro Gln Tyr Gln Gly Arg Thr Lys Leu Val Lys Asp
                80                  85                  90

Ser Ile Ala Glu Gly Arg Ile Ser Leu Arg Leu Glu Asn Ile Thr
                95                  100                 105

Val Leu Asp Ala Gly Leu Tyr Gly Cys Arg Ile Ser Ser Gln Ser
                110                 115                 120

Tyr Tyr Gln Lys Ala Ile Trp Glu Leu Gln Val Ser Ala Leu Gly
                125                 130                 135

Ser Val Pro Leu Ile Ser Ile Thr Gly Tyr Val Asp Arg Asp Ile
                140                 145                 150

Gln Leu Leu Cys Gln Ser Ser Gly Trp Phe Pro Arg Pro Thr Ala
                155                 160                 165

Lys Trp Lys Gly Pro Gln Gly Gln Asp Leu Ser Thr Asp Ser Arg
                170                 175                 180

Thr Asn Arg Asp Met His Gly Leu Phe Asp Val Glu Ile Ser Leu
                185                 190                 195

Thr Val Gln Glu Asn Ala Gly Ser Ile Ser Cys Ser Met Arg His
                200                 205                 210

Ala His Leu Ser Arg Glu Val Glu Ser Arg Val Gln Ile Gly Asp
                215                 220                 225

Thr Phe Phe Glu Pro Ile Ser Trp His Leu Ala Thr Lys Val Leu
                230                 235                 240

Gly Ile Leu Cys Cys Gly Leu Phe Phe Gly Ile Val Gly Leu Lys
                245                 250                 255

Ile Phe Phe Ser Lys Phe Gln Trp Lys Ile Gln Ala Glu Leu Asp
                260                 265                 270

Trp Arg Arg Lys His Gly Gln Ala Glu Leu Arg Asp Ala Arg Lys
                275                 280                 285

His Ala Val Glu Val Thr Leu Asp Pro Glu Thr Ala His Pro Lys
                290                 295                 300

Leu Cys Val Ser Asp Leu Lys Thr Val Thr His Arg Lys Ala Pro
                305                 310                 315

Gln Glu Val Pro His Ser Glu Lys Arg Phe Thr Arg Lys Ser Val
                320                 325                 330

Val Ala Ser Gln Ser Phe Gln Ala Gly Lys His Tyr Trp Glu Val
                335                 340                 345

Asp Gly Gly His Asn Lys Arg Trp Arg Val Gly Val Cys Arg Asp
                350                 355                 360

Asp Val Asp Arg Arg Lys Glu Tyr Val Thr Leu Ser Pro Asp His
                365                 370                 375

Gly Tyr Trp Val Leu Arg Leu Asn Gly Glu His Leu Tyr Phe Thr
                380                 385                 390

Leu Asn Pro Arg Phe Ile Ser Val Phe Pro Arg Thr Pro Pro Thr
                395                 400                 405

Lys Ile Gly Val Phe Leu Asp Tyr Glu Cys Gly Thr Ile Ser Phe
                410                 415                 420

Phe Asn Ile Asn Asp Gln Ser Leu Ile Tyr Thr Leu Thr Cys Arg
                425                 430                 435

Phe Glu Gly Leu Leu Arg Pro Tyr Ile Glu Tyr Pro Ser Tyr Asn
                440                 445                 450

Glu Gln Asn Gly Thr Pro Ile Val Ile Cys Pro Val Thr Gln Glu
                455                 460                 465

Ser Glu Lys Glu Ala Ser Trp Gln Arg Ala Ser Ala Ile Pro Glu
                470                 475                 480

Thr Ser Asn Ser Glu Ser Ser Ser Gln Ala Thr Thr Pro Phe Leu
                485                 490                 495

Pro Arg Gly Glu Met
                500

<210> 181
<211> 2294
<212> DNA
<213> Homo Sapien

<400> 181
gcgatggtgc gcccggtggc ggtggcggcg gcggttgcgg aggcttcctt 50

ggtcggattg caacgaggag aagatgactg accaaccgac tggctgaatg 100
aatgaatggc ggagccgagc gcgccatgag gagcctgccg agcctgggcg 150
gcctcgccct gttgtgctgc gccgccgccg ccgccgccgt cgcctcagcc 200
gcctcggcgg ggaatgtcac cggtggcggc ggggccgcgg ggcaggtgga 250
cgcgtcgccg ggccccgggt tgcggggcga gcccagccac cccttcccta 300
gggcgacggc tcccacggcc caggccccga ggaccgggcc cccgcgcgcc 350
accgtccacc gacccctggc tgcgacttct ccagcccagt ccccggagac 400
cacccctctt tgggcgactg ctggaccctc ttccaccacc tttcaggcgc 450
cgctcggccc ctcgccgacc acccctccgg cggcggaacg cacttcgacc 500
acctctcagg cgccgaccag acccgcgccg accacccttt cgacgaccac 550
tggcccggcg ccgaccaccc ctgtagcgac caccgtaccg gcgcccacga 600
ctccccggac cccgaccccc gatctcccca gcagcagcaa cagcagcgtc 650
ctccccaccc cacctgccac cgaggccccc tcttcgcctc ctccagagta 700
tgtatgtaac tgctctgtgg ttggaagcct gaatgtgaat cgctgcaacc 750
agaccacagg gcagtgtgag tgtcggccag gttatcaggg gcttcactgt 800
gaaacctgca aagagggctt ttacctaaat tacacttctg ggctctgtca 850
gccatgtgac tgtagtccac atggagctct cagcataccg tgcaacaggt 900
aagcaacaga gggtggaact gaagtttatt ttattttagc aagggaaaaa 950
aaaaggctgc tactctcaag gaccatactg gtttaaacaa aggaggatga 1000
gggtcataga tttacaaaat attttatata cttttattct cttactttat 1050
atgttatatt taatgtcagg atttaaaaac atctaattta ctgatttagt 1100
tcttcaaaag cactagagtc gccaattttt ctctgggata atttctgtaa 1150
atttcatggg aaaaaattat tgaagaataa atctgctttc tggaagggct 1200
ttcaggcatg aaacctgcta ggaggtttag aaatgttctt atgtttatta 1250
atataccatt ggagtttgag gaaatttgtt gtttggttta tttttctctc 1300
taatcaaaat tctacatttg tttctttgga catctaaagc ttaacctggg 1350
ggtaccctaa tttatttaac tagtggtaag tagactggtt ttactctatt 1400
taccagtaca tttttgagac caaaagtaga ttaagcagga attatcttta 1450
aactattatg ttatttggag gtaatttaat ctagtggaat aatgtactgt 1500
tatctaagca tttgccttgt actgcactga aagtaattat tctttgacct 1550
tatgtgaggc acttggcttt ttgtggaccc caagtcaaaa aactgaagag 1600
acagtattaa ataatgaaaa aaataatgac aggttatact cagtgtaacc 1650
tgggtataac ccaagatctg ctgccactta cgagctgtgt tccttgggca 1700
agtaatttcc tttcactgag cttgtttctt ctcaaggttg ttgtgaagat 1750

taaatgagtt gatatatata aaatgcctag cacatgtcac tcaataaatt 1800

ctggtttgtt ttaatttcaa aggaatatta tggactgaaa tgagagaaca 1850

tgttttaaga acttttagct ccttgacaaa gaagtgcttt atactttagc 1900

actaaatatt ttaaatgctt tataaatgat attatactgt tatggaatat 1950

tgtatcatat tgtagtttat taaaaatgta gaagaggctg ggcgcggtgg 2000

ctcacgcctg taatcctagc actttgggag gccaaggcgg gtggatcact 2050

tgaggccagg agttctagat gagcctggcc agcacagtga aaccccgtct 2100

ctactaaaaa tacaaacaaa ttagctgggc gtggtggcac acacctgtag 2150

tcccagctac tcgggaggct gaggcaggag aatcggttga acccgggagg 2200

tggaggttgc agtgagctga gatcgcgcca ctgcactcca gcctggtgag 2250

agagggagac tctgtcttaa aaaaaaaaaa aaaaaaaaaa aaaa 2294

<210> 182
<211> 258
<212> PRT
<213> Homo Sapien

<400> 182
Met Arg Ser Leu Pro Ser Leu Gly Gly Leu Ala Leu Leu Cys Cys
1               5                   10                  15

Ala Ala Ala Ala Ala Ala Val Ala Ser Ala Ala Ser Ala Gly Asn
                20                  25                  30

Val Thr Gly Gly Gly Gly Ala Ala Gly Gln Val Asp Ala Ser Pro
                35                  40                  45

Gly Pro Gly Leu Arg Gly Glu Pro Ser His Pro Phe Pro Arg Ala
                50                  55                  60

Thr Ala Pro Thr Ala Gln Ala Pro Arg Thr Gly Pro Pro Arg Ala
                65                  70                  75

Thr Val His Arg Pro Leu Ala Ala Thr Ser Pro Ala Gln Ser Pro
                80                  85                  90

Glu Thr Thr Pro Leu Trp Ala Thr Ala Gly Pro Ser Ser Thr Thr
                95                  100                 105

Phe Gln Ala Pro Leu Gly Pro Ser Pro Thr Thr Pro Pro Ala Ala
                110                 115                 120

Glu Arg Thr Ser Thr Thr Ser Gln Ala Pro Thr Arg Pro Ala Pro
                125                 130                 135

Thr Thr Leu Ser Thr Thr Thr Gly Pro Ala Pro Thr Thr Pro Val
                140                 145                 150

Ala Thr Thr Val Pro Ala Pro Thr Thr Pro Arg Thr Pro Thr Pro
                155                 160                 165

Asp Leu Pro Ser Ser Ser Asn Ser Ser Val Leu Pro Thr Pro Pro
                170                 175                 180

Ala Thr Glu Ala Pro Ser Ser Pro Pro Pro Glu Tyr Val Cys Asn
                185                 190                 195

Cys Ser Val Val Gly Ser Leu Asn Val Asn Arg Cys Asn Gln Thr
                200                 205                 210

Thr Gly Gln Cys Glu Cys Arg Pro Gly Tyr Gln Gly Leu His Cys
                215                 220                 225

Glu Thr Cys Lys Glu Gly Phe Tyr Leu Asn Tyr Thr Ser Gly Leu
                230                 235                 240

Cys Gln Pro Cys Asp Cys Ser Pro His Gly Ala Leu Ser Ile Pro
                245                 250                 255

Cys Asn Arg

<210> 183
<211> 689
<212> DNA
<213> Homo Sapien

<400> 183
tgcggcgcag tgtagacctg ggaggatggg cggcctgctg ctggctgctt 50

ttctggcttt ggtctcggtg cccagggccc aggccgtgtg gttgggaaga 100

ctggaccctg agcagcttct tgggccctgg tacgtgcttg cggtggcctc 150

ccgggaaaag ggctttgcca tggagaagga catgaagaac gtcgtggggg 200

tggtggtgac cctcactcca gaaaacaacc tgcggacgct gtcctctcag 250

cacgggctgg gagggtgtga ccagagtgtc atggacctga taaagcgaaa 300

ctccggatgg gtgtttgaga atccctcaat aggcgtgctg gagctctggg 350

tgctggccac caacttcaga gactatgcca tcatcttcac tcagctggag 400

ttcggggacg agcccttcaa caccgtggag ctgtacagtc tgacggagac 450

agccagccag gaggccatgg ggctcttcac caagtggagc aggagcctgg 500

gcttcctgtc acagtagcag gcccagctgc agaaggacct cacctgtgct 550

cacaagatcc ttctgtgagt gctgcgtccc cagtagggat ggcgcccaca 600

gggtcctgtg acctcggcca gtgtccaccc acctcgctca gcggctcccg 650

gggcccagca ccagctcaga ataaagcgat tccacagca 689

<210> 184
<211> 163
<212> PRT
<213> Homo Sapien

<400> 184
Met Gly Gly Leu Leu Leu Ala Ala Phe Leu Ala Leu Val Ser Val
1               5                   10                  15

Pro Arg Ala Gln Ala Val Trp Leu Gly Arg Leu Asp Pro Glu Gln
                20                  25                  30

Leu Leu Gly Pro Trp Tyr Val Leu Ala Val Ala Ser Arg Glu Lys
                35                  40                  45

Gly Phe Ala Met Glu Lys Asp Met Lys Asn Val Val Gly Val Val
                50                  55                  60

Val Thr Leu Thr Pro Glu Asn Asn Leu Arg Thr Leu Ser Ser Gln

65                  70                  75

His Gly Leu Gly Gly Cys Asp Gln Ser Val Met Asp Leu Ile Lys
                80                  85                  90

Arg Asn Ser Gly Trp Val Phe Glu Asn Pro Ser Ile Gly Val Leu
                95                  100                 105

Glu Leu Trp Val Leu Ala Thr Asn Phe Arg Asp Tyr Ala Ile Ile
                110                 115                 120

Phe Thr Gln Leu Glu Phe Gly Asp Glu Pro Phe Asn Thr Val Glu
                125                 130                 135

Leu Tyr Ser Leu Thr Glu Thr Ala Ser Gln Glu Ala Met Gly Leu
                140                 145                 150

Phe Thr Lys Trp Ser Arg Ser Leu Gly Phe Leu Ser Gln
                155                 160

<210> 185
<211> 1204
<212> DNA
<213> Homo Sapien

<400> 185
gttccgcaga tgcagaggtt gaggtggctg cgggactgga agtcatcggg 50

cagaggtctc acagcagcca aggaacctgg ggcccgctcc tcccccctcc 100

aggccatgag gattctgcag ttaatcctgc ttgctctggc aacagggctt 150

gtagggggag agaccaggat catcaagggg ttcgagtgca agcctcactc 200

ccagccctgg caggcagccc tgttcgagaa gacgcggcta ctctgtgggg 250

cgacgctcat cgcccccaga tggctcctga cagcagccca ctgcctcaag 300

ccccgctaca tagttcacct ggggcagcac aacctccaga aggaggaggg 350

ctgtgagcag acccggacag ccactgagtc cttcccccac cccggcttca 400

acaacagcct ccccaacaaa gaccaccgca atgacatcat gctggtgaag 450

atggcatcgc cagtctccat cacctgggct gtgcgacccc tcaccctctc 500

ctcacgctgt gtcactgctg gcaccagctg cctcatttcc ggctggggca 550

gcacgtccag cccccagtta cgcctgcctc acaccttgcg atgcgccaac 600

atcaccatca ttgagcacca gaagtgtgag aacgcctacc ccggcaacat 650

cacagacacc atggtgtgtg ccagcgtgca ggaaggggc aaggactcct 700

gccagggtga ctccgggggc cctctggtct gtaaccagtc tcttcaaggc 750

attatctcct ggggccagga tccgtgtgcg atcacccgaa agcctggtgt 800

ctacacgaaa gtctgcaaat atgtggactg gatccaggag acgatgaaga 850

acaattagac tggacccacc caccacagcc catcaccctc catttccact 900

tggtgtttgg ttcctgttca ctctgttaat aagaaaccct aagccaagac 950

cctctacgaa cattctttgg gcctcctgga ctacaggaga tgctgtcact 1000

taataatcaa cctggggttc gaaatcagtg agacctggat tcaaattctg 1050

ccttgaaata ttgtgactct gggaatgaca acacctggtt tgttctctgt 1100

tgtatcccca gccccaaaga cagctcctgg ccatatatca aggtttcaat 1150

aaatatttgc taaatgaaaa aaaaaaaaaa aaaaaaaaaa aaaaaaaaaa 1200

aaaa 1204

<210> 186
<211> 250
<212> PRT
<213> Homo Sapien

<400> 186
Met Arg Ile Leu Gln Leu Ile Leu Leu Ala Leu Ala Thr Gly Leu
1               5                   10                  15

Val Gly Gly Glu Thr Arg Ile Ile Lys Gly Phe Glu Cys Lys Pro
                20                  25                  30

His Ser Gln Pro Trp Gln Ala Ala Leu Phe Glu Lys Thr Arg Leu
                35                  40                  45

Leu Cys Gly Ala Thr Leu Ile Ala Pro Arg Trp Leu Leu Thr Ala
                50                  55                  60

Ala His Cys Leu Lys Pro Arg Tyr Ile Val His Leu Gly Gln His
                65                  70                  75

Asn Leu Gln Lys Glu Glu Gly Cys Glu Gln Thr Arg Thr Ala Thr
                80                  85                  90

Glu Ser Phe Pro His Pro Gly Phe Asn Asn Ser Leu Pro Asn Lys
                95                  100                 105

Asp His Arg Asn Asp Ile Met Leu Val Lys Met Ala Ser Pro Val
                110                 115                 120

Ser Ile Thr Trp Ala Val Arg Pro Leu Thr Leu Ser Ser Arg Cys
                125                 130                 135

Val Thr Ala Gly Thr Ser Cys Leu Ile Ser Gly Trp Gly Ser Thr
                140                 145                 150

Ser Ser Pro Gln Leu Arg Leu Pro His Thr Leu Arg Cys Ala Asn
                155                 160                 165

Ile Thr Ile Ile Glu His Gln Lys Cys Glu Asn Ala Tyr Pro Gly
                170                 175                 180

Asn Ile Thr Asp Thr Met Val Cys Ala Ser Val Gln Glu Gly Gly
                185                 190                 195

Lys Asp Ser Cys Gln Gly Asp Ser Gly Gly Pro Leu Val Cys Asn
                200                 205                 210

Gln Ser Leu Gln Gly Ile Ile Ser Trp Gly Gln Asp Pro Cys Ala
                215                 220                 225

Ile Thr Arg Lys Pro Gly Val Tyr Thr Lys Val Cys Lys Tyr Val
                230                 235                 240

Asp Trp Ile Gln Glu Thr Met Lys Asn Asn
                245                 250

<210> 187
<211> 2848
<212> DNA

<213> Homo Sapien

<400> 187
gctcaagtgc cctgccttgc cccacccagc ccagcctggc cagagccccc 50

tggagaagga gctctcttct tgcttggcag ctggaccaag ggagccagtc 100

ttgggcgctg gagggcctgt cctgaccatg gtccctgcct ggctgtggct 150

gctttgtgtc tccgtccccc aggctctccc caaggcccag cctgcagagc 200

tgtctgtgga agttccagaa aactatggtg gaaatttccc tttatacctg 250

accaagttgc cgctgccccg tgagggggct gaaggccaga tcgtgctgtc 300

aggggactca ggcaaggcaa ctgagggccc atttgctatg gatccagatt 350

ctggcttcct gctggtgacc agggccctgg accgagagga gcaggcagag 400

taccagctac aggtcaccct ggagatgcag gatggacatg tcttgtgggg 450

tccacagcct gtgcttgtgc acgtgaagga tgagaatgac caggtgcccc 500

atttctctca agccatctac agagctcggc tgagccgggg taccaggcct 550

ggcatcccct tcctcttcct tgaggcttca gaccgggatg agccaggcac 600

agccaactcg gatcttcgat tccacatcct gagccaggct ccagcccagc 650

cttccccaga catgttccag ctggagcctc ggctgggggc tctggccctc 700

agccccaagg ggagcaccag ccttgaccac gccctggaga ggacctacca 750

gctgttggta caggtcaagg acatgggtga ccaggcctca ggccaccagg 800

ccactgccac cgtggaagtc tccatcatag agagcacctg ggtgtcccta 850

gagcctatcc acctggcaga gaatctcaaa gtcctatacc cgcaccacat 900

ggcccaggta cactggagtg ggggtgatgt gcactatcac ctggagagcc 950

atcccccggg accctttgaa gtgaatgcag agggaaacct ctacgtgacc 1000

agagagctgg acagagaagc ccaggctgag tacctgctcc aggtgcgggc 1050

tcagaattcc catggcgagg actatgcggc ccctctggag ctgcacgtgc 1100

tggtgatgga tgagaatgac aacgtgccta tctgccctcc ccgtgacccc 1150

acagtcagca tccctgagct cagtccacca ggtactgaag tgactagact 1200

gtcagcagag gatgcagatg cccccggctc ccccaattcc cacgttgtgt 1250

atcagctcct gagccctgag cctgaggatg gggtagaggg gagagccttc 1300

caggtggacc ccacttcagg cagtgtgacg ctgggggtgc tcccactccg 1350

agcaggccag aacatcctgc ttctggtgct ggccatggac ctggcaggcg 1400

cagagggtgg cttcagcagc acgtgtgaag tcgaagtcgc agtcacagat 1450

atcaatgatc acgcccctga gttcatcact tcccagattg ggcctataag 1500

cctccctgag gatgtggagc ccgggactct ggtggccatg ctaacagcca 1550

ttgatgctga cctcgagccc gccttccgcc tcatggattt tgccattgag 1600

aggggagaca cagaagggac ttttggcctg gattgggagc cagactctgg 1650

gcatgttaga ctcagactct gcaagaacct cagttatgag gcagctccaa 1700

gtcatgaggt ggtggtggtg gtgcagagtg tggcgaagct ggtggggcca 1750

ggcccaggcc ctggagccac cgccacggtg actgtgctag tggagagagt 1800

gatgccaccc cccaagttgg accaggagag ctacgaggcc agtgtcccca 1850

tcagtgcccc agccggctct ttcctgctga ccatccagcc ctccgacccc 1900

atcagccgaa ccctcaggtt ctccctagtc aatgactcag agggctggct 1950

ctgcattgag aaattctccg gggaggtgca caccgcccag tccctgcagg 2000

gcgcccagcc tggggacacc tacacggtgc ttgtggaggc ccaggataca 2050

gccctgactc ttgcccctgt gccctcccaa tacctctgca caccccgcca 2100

agaccatggc ttgatcgtga gtggacccag caaggacccc gatctggcca 2150

gtgggcacgg tccctacagc ttcacccttg gtcccaaccc cacggtgcaa 2200

cgggattggc gcctccagac tctcaatggt tcccatgcct acctcacctt 2250

ggccctgcat tgggtggagc cacgtgaaca cataatcccc gtggtggtca 2300

gccacaatgc ccagatgtgg cagctcctgg ttcgagtgat cgtgtgtcgc 2350

tgcaacgtgg aggggcagtg catgcgcaag gtgggccgca tgaagggcat 2400

gcccacgaag ctgtcggcag tgggcatcct tgtaggcacc ctggtagcaa 2450

taggaatctt cctcatcctc attttcaccc actggaccat gtcaaggaag 2500

aaggacccgg atcaaccagc agacagcgtg cccctgaagg cgactgtctg 2550

aatggcccag gcagctctag ctgggagctt ggcctctggc tccatctgag 2600

tcccctggga gagagcccag cacccaagat ccagcagggg acaggacaga 2650

gtagaagccc ctccatctgc cctggggtgg aggcaccatc accatcacca 2700

ggcatgtctg cagagcctgg acaccaactt tatggactgc ccatgggagt 2750

gctccaaatg tcagggtgtt tgcccaataa taaagcccca gagaactggg 2800

ctgggcccta tgggaaaaaa aaaaaaaaaa aaaaaaaaaa aaaaaaag 2848

<210> 188
<211> 807
<212> PRT
<213> Homo Sapien

<400> 188
Met Val Pro Ala Trp Leu Trp Leu Leu Cys Val Ser Val Pro Gln
1               5                   10                  15

Ala Leu Pro Lys Ala Gln Pro Ala Glu Leu Ser Val Glu Val Pro
                20                  25                  30

Glu Asn Tyr Gly Gly Asn Phe Pro Leu Tyr Leu Thr Lys Leu Pro
                35                  40                  45

Leu Pro Arg Glu Gly Ala Glu Gly Gln Ile Val Leu Ser Gly Asp
                50                  55                  60

Ser Gly Lys Ala Thr Glu Gly Pro Phe Ala Met Asp Pro Asp Ser

65                  70                  75

Gly Phe Leu Leu Val Thr Arg Ala Leu Asp Arg Glu Glu Gln Ala
                80                  85                  90

Glu Tyr Gln Leu Gln Val Thr Leu Glu Met Gln Asp Gly His Val
                95                  100                 105

Leu Trp Gly Pro Gln Pro Val Leu Val His Val Lys Asp Glu Asn
                110                 115                 120

Asp Gln Val Pro His Phe Ser Gln Ala Ile Tyr Arg Ala Arg Leu
                125                 130                 135

Ser Arg Gly Thr Arg Pro Gly Ile Pro Phe Leu Phe Leu Glu Ala
                140                 145                 150

Ser Asp Arg Asp Glu Pro Gly Thr Ala Asn Ser Asp Leu Arg Phe
                155                 160                 165

His Ile Leu Ser Gln Ala Pro Ala Gln Pro Ser Pro Asp Met Phe
                170                 175                 180

Gln Leu Glu Pro Arg Leu Gly Ala Leu Ala Leu Ser Pro Lys Gly
                185                 190                 195

Ser Thr Ser Leu Asp His Ala Leu Glu Arg Thr Tyr Gln Leu Leu
                200                 205                 210

Val Gln Val Lys Asp Met Gly Asp Gln Ala Ser Gly His Gln Ala
                215                 220                 225

Thr Ala Thr Val Glu Val Ser Ile Ile Glu Ser Thr Trp Val Ser
                230                 235                 240

Leu Glu Pro Ile His Leu Ala Glu Asn Leu Lys Val Leu Tyr Pro
                245                 250                 255

His His Met Ala Gln Val His Trp Ser Gly Gly Asp Val His Tyr
                260                 265                 270

His Leu Glu Ser His Pro Pro Gly Pro Phe Glu Val Asn Ala Glu
                275                 280                 285

Gly Asn Leu Tyr Val Thr Arg Glu Leu Asp Arg Glu Ala Gln Ala
                290                 295                 300

Glu Tyr Leu Leu Gln Val Arg Ala Gln Asn Ser His Gly Glu Asp
                305                 310                 315

Tyr Ala Ala Pro Leu Glu Leu His Val Leu Val Met Asp Glu Asn
                320                 325                 330

Asp Asn Val Pro Ile Cys Pro Pro Arg Asp Pro Thr Val Ser Ile
                335                 340                 345

Pro Glu Leu Ser Pro Pro Gly Thr Glu Val Thr Arg Leu Ser Ala
                350                 355                 360

Glu Asp Ala Asp Ala Pro Gly Ser Pro Asn Ser His Val Val Tyr
                365                 370                 375

Gln Leu Leu Ser Pro Glu Pro Glu Asp Gly Val Glu Gly Arg Ala
                380                 385                 390

Phe Gln Val Asp Pro Thr Ser Gly Ser Val Thr Leu Gly Val Leu
                395                 400                 405

Pro Leu Arg Ala Gly Gln Asn Ile Leu Leu Leu Val Leu Ala Met
                410                 415                 420

Asp Leu Ala Gly Ala Glu Gly Gly Phe Ser Ser Thr Cys Glu Val
                425                 430                 435

Glu Val Ala Val Thr Asp Ile Asn Asp His Ala Pro Glu Phe Ile
                440                 445                 450

Thr Ser Gln Ile Gly Pro Ile Ser Leu Pro Glu Asp Val Glu Pro
                455                 460                 465

Gly Thr Leu Val Ala Met Leu Thr Ala Ile Asp Ala Asp Leu Glu
                470                 475                 480

Pro Ala Phe Arg Leu Met Asp Phe Ala Ile Glu Arg Gly Asp Thr
                485                 490                 495

Glu Gly Thr Phe Gly Leu Asp Trp Glu Pro Asp Ser Gly His Val
                500                 505                 510

Arg Leu Arg Leu Cys Lys Asn Leu Ser Tyr Glu Ala Ala Pro Ser
                515                 520                 525

His Glu Val Val Val Val Val Gln Ser Val Ala Lys Leu Val Gly
                530                 535                 540

Pro Gly Pro Gly Pro Gly Ala Thr Ala Thr Val Thr Val Leu Val
                545                 550                 555

Glu Arg Val Met Pro Pro Pro Lys Leu Asp Gln Glu Ser Tyr Glu
                560                 565                 570

Ala Ser Val Pro Ile Ser Ala Pro Ala Gly Ser Phe Leu Leu Thr
                575                 580                 585

Ile Gln Pro Ser Asp Pro Ile Ser Arg Thr Leu Arg Phe Ser Leu
                590                 595                 600

Val Asn Asp Ser Glu Gly Trp Leu Cys Ile Glu Lys Phe Ser Gly
                605                 610                 615

Glu Val His Thr Ala Gln Ser Leu Gln Gly Ala Gln Pro Gly Asp
                620                 625                 630

Thr Tyr Thr Val Leu Val Glu Ala Gln Asp Thr Ala Leu Thr Leu
                635                 640                 645

Ala Pro Val Pro Ser Gln Tyr Leu Cys Thr Pro Arg Gln Asp His
                650                 655                 660

Gly Leu Ile Val Ser Gly Pro Ser Lys Asp Pro Asp Leu Ala Ser
                665                 670                 675

Gly His Gly Pro Tyr Ser Phe Thr Leu Gly Pro Asn Pro Thr Val
                680                 685                 690

Gln Arg Asp Trp Arg Leu Gln Thr Leu Asn Gly Ser His Ala Tyr
                695                 700                 705

Leu Thr Leu Ala Leu His Trp Val Glu Pro Arg Glu His Ile Ile
                710                 715                 720

Pro Val Val Val Ser His Asn Ala Gln Met Trp Gln Leu Leu Val
                725                 730                 735

Arg Val Ile Val Cys Arg Cys Asn Val Glu Gly Gln Cys Met Arg
                740                 745                 750

Lys Val Gly Arg Met Lys Gly Met Pro Thr Lys Leu Ser Ala Val
                755                 760                 765

Gly Ile Leu Val Gly Thr Leu Val Ala Ile Gly Ile Phe Leu Ile
                770                 775                 780

Leu Ile Phe Thr His Trp Thr Met Ser Arg Lys Lys Asp Pro Asp
                785                 790                 795

Gln Pro Ala Asp Ser Val Pro Leu Lys Ala Thr Val
                800                 805

<210> 189
<211> 2668
<212> DNA
<213> Homo Sapien

<400> 189
gactttgctt gaatgtttac attttctgct cgctgtccta catatcacaa 50

tatagtgttc acgttttgtt aaaactttgg ggtgtcagga gttgagcttg 100

ctcagcaagc cagcatggct aggatgagct ttgttatagc agcttgccaa 150

ttggtgctgg gcctactaat gacttcatta accgagtctt ccatacagaa 200

tagtgagtgt ccacaacttt gcgtatgtga aattcgtccc tggtttaccc 250

cacagtcaac ttacagagaa gccaccactg ttgattgcaa tgacctccgc 300

ttaacaagga ttcccagtaa cctctctagt gacacacaag tgcttctctt 350

acagagcaat aacatcgcga agactgtgga tgagctgcag cagcttttca 400

acttgactga actagatttc tcccaaaaca actttactaa cattaaggag 450

gtcgggctgg caaacctaac ccagctcaca acgctgcatt tggaggaaaa 500

tcagattacc gagatgactg attactgtct acaagacctc agcaaccttc 550

aagaactcta catcaaccac aaccaaatta gcactatttc tgctcatgct 600

tttgcaggct taaaaaatct attaaggctc cacctgaact ccaacaaatt 650

gaaagttatt gatagtcgct ggtttgattc tacacccaac ctggaaattc 700

tcatgatcgg agaaaaccct gtgattggaa ttctggatat gaacttcaaa 750

cccctcgcaa atttgagaag cttagttttg gcaggaatgt atctcactga 800

tattcctgga aatgctttgg tgggtctgga tagccttgag agcctgtctt 850

tttatgataa caaactggtt aaagtccctc aacttgccct gcaaaaagtt 900

ccaaatttga aattcttaga cctcaacaaa aaccccattc acaaaatcca 950

agaaggggac ttcaaaaata tgcttcggtt aaaagaactg ggaatcaaca 1000

atatgggcga gctcgtttct gtcgaccgct atgccctgga taacttgcct 1050

gaactcacaa agctggaagc caccaataac cctaaactct cttacatcca 1100

ccgcttggct ttccgaagtg tccctgctct ggaaagcttg atgctgaaca 1150

acaatgcctt gaatgccatt taccaaaaga cagtcgaatc cctccccaat 1200

ctgcgtgaga tcagtatcca tagcaatccc ctcaggtgtg actgtgtgat 1250

ccactggatt aactccaaca aaaccaacat ccgcttcatg gagcccctgt 1300

ccatgttctg tgccatgccg cccgaatata aagggcacca ggtgaaggaa 1350

gttttaatcc aggattcgag tgaacagtgc ctcccaatga tatctcacga 1400

cagcttccca aatcgtttaa acgtggatat cggcacgacg gttttcctag 1450

actgtcgagc catggctgag ccagaacctg aaatttactg ggtcactccc 1500

attggaaata agataactgt ggaaaccctt tcagataaat acaagctaag 1550

tagcgaaggt accttggaaa tatctaacat acaaattgaa gactcaggaa 1600

gatacacatg tgttgcccag aatgtccaag gggcagacac tcgggtggca 1650

acaattaagg ttaacgggac ccttctggat ggtacccagg tgctaaaaat 1700

atacgtcaag cagacagaat cccattccat cttagtgtcc tggaaagtta 1750

attccaatgt catgacgtca aacttaaaat ggtcgtctgc caccatgaag 1800

attgataacc ctcacataac atatactgcc agggtcccag tcgatgtcca 1850

tgaatacaac ctaacgcatc tgcagccttc cacagattat gaagtgtgtc 1900

tcacagtgtc caatattcat cagcagactc aaaagtcatg cgtaaatgtc 1950

acaaccaaaa atgccgcctt cgcagtggac atctctgatc aagaaaccag 2000

tacagccctt gctgcagtaa tggggtctat gtttgccgtc attagccttg 2050

cgtccattgc tgtgtacttt gccaaaagat ttaagagaaa aaactaccac 2100

cactcattaa aaaagtatat gcaaaaaacc tcttcaatcc cactaaatga 2150

gctgtaccca ccactcatta acctctggga aggtgacagc gagaaagaca 2200

aagatggttc tgcagacacc aagccaaccc aggtcgacac atccagaagc 2250

tattacatgt ggtaactcag aggatatttt gcttctggta gtaaggagca 2300

caaagacgtt tttgctttat tctgcaaaag tgaacaagtt gaagactttt 2350

gtatttttga ctttgctagt ttgtggcaga gtggagagga cgggtggata 2400

tttcaaattt ttttagtata gcgtatcgca agggtttgac acggctgcca 2450

gcgactctag gcttccagtc tgtgtttggt ttttattctt atcattatta 2500

tgattgttat tatattatta ttttatttta gttgttgtgc taaactcaat 2550

aatgctgttc taactacagt gctcaataaa atgattaatg acaggaaaaa 2600

aaaaaaaaaa aaaaaaaaaa aaaaaaaaaa aaaaaaaaaa aaaaaaaaaa 2650

aaaaaaaaaa aaaaaaaa 2668

<210> 190
<211> 716
<212> PRT
<213> Homo Sapien

<400> 190
Met Ala Arg Met Ser Phe Val Ile Ala Ala Cys Gln Leu Val Leu
1               5                   10                  15

Gly Leu Leu Met Thr Ser Leu Thr Glu Ser Ser Ile Gln Asn Ser
                20                  25                  30

Glu Cys Pro Gln Leu Cys Val Cys Glu Ile Arg Pro Trp Phe Thr
                35                  40                  45

Pro Gln Ser Thr Tyr Arg Glu Ala Thr Thr Val Asp Cys Asn Asp
                50                  55                  60

Leu Arg Leu Thr Arg Ile Pro Ser Asn Leu Ser Ser Asp Thr Gln
                65                  70                  75

Val Leu Leu Leu Gln Ser Asn Asn Ile Ala Lys Thr Val Asp Glu
                80                  85                  90

Leu Gln Gln Leu Phe Asn Leu Thr Glu Leu Asp Phe Ser Gln Asn
                95                  100                 105

Asn Phe Thr Asn Ile Lys Glu Val Gly Leu Ala Asn Leu Thr Gln
                110                 115                 120

Leu Thr Thr Leu His Leu Glu Glu Asn Gln Ile Thr Glu Met Thr
                125                 130                 135

Asp Tyr Cys Leu Gln Asp Leu Ser Asn Leu Gln Glu Leu Tyr Ile
                140                 145                 150

Asn His Asn Gln Ile Ser Thr Ile Ser Ala His Ala Phe Ala Gly
                155                 160                 165

Leu Lys Asn Leu Leu Arg Leu His Leu Asn Ser Asn Lys Leu Lys
                170                 175                 180

Val Ile Asp Ser Arg Trp Phe Asp Ser Thr Pro Asn Leu Glu Ile
                185                 190                 195

Leu Met Ile Gly Glu Asn Pro Val Ile Gly Ile Leu Asp Met Asn
                200                 205                 210

Phe Lys Pro Leu Ala Asn Leu Arg Ser Leu Val Leu Ala Gly Met
                215                 220                 225

Tyr Leu Thr Asp Ile Pro Gly Asn Ala Leu Val Gly Leu Asp Ser
                230                 235                 240

Leu Glu Ser Leu Ser Phe Tyr Asp Asn Lys Leu Val Lys Val Pro
                245                 250                 255

Gln Leu Ala Leu Gln Lys Val Pro Asn Leu Lys Phe Leu Asp Leu
                260                 265                 270

Asn Lys Asn Pro Ile His Lys Ile Gln Glu Gly Asp Phe Lys Asn
                275                 280                 285

Met Leu Arg Leu Lys Glu Leu Gly Ile Asn Asn Met Gly Glu Leu
                290                 295                 300

Val Ser Val Asp Arg Tyr Ala Leu Asp Asn Leu Pro Glu Leu Thr
                305                 310                 315

Lys Leu Glu Ala Thr Asn Asn Pro Lys Leu Ser Tyr Ile His Arg
                320                 325                 330

Leu Ala Phe Arg Ser Val Pro Ala Leu Glu Ser Leu Met Leu Asn
                335                 340                 345

Asn Asn Ala Leu Asn Ala Ile Tyr Gln Lys Thr Val Glu Ser Leu
                350                 355                 360

Pro Asn Leu Arg Glu Ile Ser Ile His Ser Asn Pro Leu Arg Cys
                365                 370                 375

Asp Cys Val Ile His Trp Ile Asn Ser Asn Lys Thr Asn Ile Arg
                380                 385                 390

Phe Met Glu Pro Leu Ser Met Phe Cys Ala Met Pro Pro Glu Tyr
                395                 400                 405

Lys Gly His Gln Val Lys Glu Val Leu Ile Gln Asp Ser Ser Glu
                410                 415                 420

Gln Cys Leu Pro Met Ile Ser His Asp Ser Phe Pro Asn Arg Leu
                425                 430                 435

Asn Val Asp Ile Gly Thr Thr Val Phe Leu Asp Cys Arg Ala Met
                440                 445                 450

Ala Glu Pro Glu Pro Glu Ile Tyr Trp Val Thr Pro Ile Gly Asn
                455                 460                 465

Lys Ile Thr Val Glu Thr Leu Ser Asp Lys Tyr Lys Leu Ser Ser
                470                 475                 480

Glu Gly Thr Leu Glu Ile Ser Asn Ile Gln Ile Glu Asp Ser Gly
                485                 490                 495

Arg Tyr Thr Cys Val Ala Gln Asn Val Gln Gly Ala Asp Thr Arg
                500                 505                 510

Val Ala Thr Ile Lys Val Asn Gly Thr Leu Leu Asp Gly Thr Gln
                515                 520                 525

Val Leu Lys Ile Tyr Val Lys Gln Thr Glu Ser His Ser Ile Leu
                530                 535                 540

Val Ser Trp Lys Val Asn Ser Asn Val Met Thr Ser Asn Leu Lys
                545                 550                 555

Trp Ser Ser Ala Thr Met Lys Ile Asp Asn Pro His Ile Thr Tyr
                560                 565                 570

Thr Ala Arg Val Pro Val Asp Val His Glu Tyr Asn Leu Thr His
                575                 580                 585

Leu Gln Pro Ser Thr Asp Tyr Glu Val Cys Leu Thr Val Ser Asn
                590                 595                 600

Ile His Gln Gln Thr Gln Lys Ser Cys Val Asn Val Thr Thr Lys
                605                 610                 615

Asn Ala Ala Phe Ala Val Asp Ile Ser Asp Gln Glu Thr Ser Thr
                620                 625                 630

Ala Leu Ala Ala Val Met Gly Ser Met Phe Ala Val Ile Ser Leu
                635                 640                 645

Ala Ser Ile Ala Val Tyr Phe Ala Lys Arg Phe Lys Arg Lys Asn
                650                 655                 660

Tyr His His Ser Leu Lys Lys Tyr Met Gln Lys Thr Ser Ser Ile
                665                 670                 675

Pro Leu Asn Glu Leu Tyr Pro Pro Leu Ile Asn Leu Trp Glu Gly
                680                 685                 690

Asp Ser Glu Lys Asp Lys Asp Gly Ser Ala Asp Thr Lys Pro Thr

695                    700                    705

Gln Val Asp Thr Ser Arg Ser Tyr Tyr Met Trp
                710                 715

<210> 191
<211> 957
<212> DNA
<213> Homo Sapien

<400> 191
gggagagagg ataaatagca gcgtggcttc cctggctcct ctctgcatcc 50

ttcccgacct tcccagcaat atgcatcttg cacgtctggt cggctcctgc 100

tccctccttc tgctactggg ggccctgtct ggatgggcgg ccagcgatga 150

ccccattgag aaggtcattg aagggatcaa ccgagggctg agcaatgcag 200

agagagaggt gggcaaggcc ctggatggca tcaacagtgg aatcacgcat 250

gccggaaggg aagtggagaa ggttttcaac ggacttagca acatggggag 300

ccacaccggc aaggagttgg acaaaggcgt ccaggggctc aaccacggca 350

tggacaaggt tgcccatgag atcaaccatg gtattggaca agcaggaaag 400

gaagcagaga agcttggcca tggggtcaac aacgctgctg gacaggccgg 450

gaaggaagca gacaaagcgg tccaagggtt ccacactggg gtccaccagg 500

ctgggaagga agcagagaaa cttggccaag gggtcaacca tgctgctgac 550

caggctggaa aggaagtgga gaagcttggc caaggtgccc accatgctgc 600

tggccaggcc gggaaggagc tgcagaatgc tcataatggg gtcaaccaag 650

ccagcaagga ggccaaccag ctgctgaatg gcaaccatca aagcggatct 700

tccagccatc aaggaggggc cacaaccacg ccgttagcct ctggggcctc 750

agtcaacacg cctttcatca accttcccgc cctgtggagg agcgtcgcca 800

acatcatgcc ctaaactggc atccggcctt gctgggagaa taatgtcgcc 850

gttgtcacat cagctgacat gacctggagg ggttgggggt gggggacagg 900

tttctgaaat ccctgaaggg ggttgtactg ggatttgtga ataaacttga 950

tacacca 957

<210> 192
<211> 247
<212> PRT
<213> Homo Sapien

<400> 192
Met His Leu Ala Arg Leu Val Gly Ser Cys Ser Leu Leu Leu Leu
  1               5                  10                  15

Leu Gly Ala Leu Ser Gly Trp Ala Ala Ser Asp Asp Pro Ile Glu
                 20                  25                  30

Lys Val Ile Glu Gly Ile Asn Arg Gly Leu Ser Asn Ala Glu Arg
                 35                  40                  45

Glu Val Gly Lys Ala Leu Asp Gly Ile Asn Ser Gly Ile Thr His
                 50                  55                  60

Ala Gly Arg Glu Val Glu Lys Val Phe Asn Gly Leu Ser Asn Met
                65                  70                  75

Gly Ser His Thr Gly Lys Glu Leu Asp Lys Gly Val Gln Gly Leu
                80                  85                  90

Asn His Gly Met Asp Lys Val Ala His Glu Ile Asn His Gly Ile
                95                  100                 105

Gly Gln Ala Gly Lys Glu Ala Glu Lys Leu Gly His Gly Val Asn
                110                 115                 120

Asn Ala Ala Gly Gln Ala Gly Lys Glu Ala Asp Lys Ala Val Gln
                125                 130                 135

Gly Phe His Thr Gly Val His Gln Ala Gly Lys Glu Ala Glu Lys
                140                 145                 150

Leu Gly Gln Gly Val Asn His Ala Ala Asp Gln Ala Gly Lys Glu
                155                 160                 165

Val Glu Lys Leu Gly Gln Gly Ala His His Ala Ala Gly Gln Ala
                170                 175                 180

Gly Lys Glu Leu Gln Asn Ala His Asn Gly Val Asn Gln Ala Ser
                185                 190                 195

Lys Glu Ala Asn Gln Leu Leu Asn Gly Asn His Gln Ser Gly Ser
                200                 205                 210

Ser Ser His Gln Gly Gly Ala Thr Thr Thr Pro Leu Ala Ser Gly
                215                 220                 225

Ala Ser Val Asn Thr Pro Phe Ile Asn Leu Pro Ala Leu Trp Arg
                230                 235                 240

Ser Val Ala Asn Ile Met Pro
                245

<210> 193
<211> 904
<212> DNA
<213> Homo Sapien

<400> 193
gaagtagagg tgttgtgctg agcggcgctc ggcgaactgt gtggaccgtc 50

tgctgggact ccggccctgc gtccgctcag ccccgtggcc ccgcgcacct 100

actgccatgg agacgcggcc tcgtctcggg gccacctgtt tgctgggctt 150

cagtttcctg ctcctcgtca tctcttctga tggacataat gggcttggaa 200

agggttttgg agatcatatt cattggagga cactggaaga tgggaagaaa 250

gaagcagctg ccagtggact gcccctgatg gtgattattc ataaatcctg 300

gtgtggagct tgcaaagctc taaagcccaa atttgcagaa tctacggaaa 350

tttcagaact ctcccataat tttgttatgg taaatcttga ggatgaagag 400

gaacccaaag atgaagattt cagccctgac gggggttata ttccacgaat 450

cctttttctg gatcccagtg gcaaggtgca tcctgaaatc atcaatgaga 500

atggaaaccc cagctacaag tatttttatg tcagtgccga gcaagttgtt 550

caggggatga aggaagctca ggaaaggctg acgggtgatg ccttcagaaa 600

gaaacatctt gaagatgaat tgtaacatga atgtgcccct tctttcatca 650

gagttagtgt tctggaagga aagcagcagg gaagggaata ttgaggaatc 700

atctagaaca attaagccga ccaggaaacc tcattcctac ctacactgga 750

aggagcgctc tcactgtgga agagttctgc taacagaagc tggtctgcat 800

gtttgtggat ccagcggaga gtggcagact ttcttctcct tttccctctc 850

acctaaatgt caacttgtca ttgaatgtaa agaatgaaac cttctgacac 900

aaaa 904

<210> 194
<211> 172
<212> PRT
<213> Homo Sapien

<400> 194
Met Glu Thr Arg Pro Arg Leu Gly Ala Thr Cys Leu Leu Gly Phe
1               5                   10                  15

Ser Phe Leu Leu Leu Val Ile Ser Ser Asp Gly His Asn Gly Leu
                20                  25                  30

Gly Lys Gly Phe Gly Asp His Ile His Trp Arg Thr Leu Glu Asp
                35                  40                  45

Gly Lys Lys Glu Ala Ala Ala Ser Gly Leu Pro Leu Met Val Ile
                50                  55                  60

Ile His Lys Ser Trp Cys Gly Ala Cys Lys Ala Leu Lys Pro Lys
                65                  70                  75

Phe Ala Glu Ser Thr Glu Ile Ser Glu Leu Ser His Asn Phe Val
                80                  85                  90

Met Val Asn Leu Glu Asp Glu Glu Glu Pro Lys Asp Glu Asp Phe
                95                  100                 105

Ser Pro Asp Gly Gly Tyr Ile Pro Arg Ile Leu Phe Leu Asp Pro
                110                 115                 120

Ser Gly Lys Val His Pro Glu Ile Ile Asn Glu Asn Gly Asn Pro
                125                 130                 135

Ser Tyr Lys Tyr Phe Tyr Val Ser Ala Glu Gln Val Val Gln Gly
                140                 145                 150

Met Lys Glu Ala Gln Glu Arg Leu Thr Gly Asp Ala Phe Arg Lys
                155                 160                 165

Lys His Leu Glu Asp Glu Leu
                170

<210> 195
<211> 1630
<212> DNA
<213> Homo Sapien

<400> 195
cggctcgagt gcagctgtgg ggagatttca gtgcattgcc tcccctgggt 50

gctcttcatc ttggatttga aagttgagag cagcatgttt tgcccactga 100

aactcatcct gctgccagtg ttactggatt attccttggg cctgaatgac 150

ttgaatgttt ccccgcctga gctaacagtc catgtgggtg attcagctct 200

gatgggatgt gttttccaga gcacagaaga caaatgtata ttcaagatag 250

actggactct gtcaccagga gagcacgcca aggacgaata tgtgctatac 300

tattactcca atctcagtgt gcctattggg cgcttccaga accgcgtaca 350

cttgatgggg gacatcttat gcaatgatgg ctctctcctg ctccaagatg 400

tgcaagaggc tgaccaggga acctatatct gtgaaatccg cctcaaaggg 450

gagagccagg tgttcaagaa ggcggtggta ctgcatgtgc ttccagagga 500

gcccaaagag ctcatggtcc atgtgggtgg attgattcag atgggatgtg 550

ttttccagag cacagaagtg aaacacgtga ccaaggtaga atggatattt 600

tcaggacggc gcgcaaagga ggagattgta tttcgttact accacaaact 650

caggatgtct gtggagtact cccagagctg gggccacttc cagaatcgtg 700

tgaacctggt gggggacatt ttccgcaatg acggttccat catgcttcaa 750

ggagtgaggg agtcagatgg aggaaactac acctgcagta tccacctagg 800

gaacctggtg ttcaagaaaa ccattgtgct gcatgtcagc ccggaagagc 850

ctcgaacact ggtgaccccg gcagccctga ggcctctggt cttgggtggt 900

aatcagttgg tgatcattgt gggaattgtc tgtgccacaa tcctgctgct 950

ccctgttctg atattgatcg tgaagaagac ctgtggaaat aagagttcag 1000

tgaattctac agtcttggtg aagaacacga agaagactaa tccagagata 1050

aaagaaaaac cctgccattt tgaaagatgt gaaggggaga aacacattta 1100

ctccccaata attgtacggg aggtgatcga ggaagaagaa ccaagtgaaa 1150

aatcagaggc cacctacatg accatgcacc cagtttggcc ttctctgagg 1200

tcagatcgga acaactcact tgaaaaaaag tcaggtgggg gaatgccaaa 1250

aacacagcaa gccttttgag aagaatggag agtcccttca tctcagcagc 1300

ggtggagact ctctcctgtg tgtgtcctgg gccactctac cagtgatttc 1350

agactcccgc tctcccagct gtcctcctgt ctcattgttt ggtcaataca 1400

ctgaagatgg agaatttgga gcctggcaga gagactggac agctctggag 1450

gaacaggcct gctgagggga ggggagcatg gacttggcct ctggagtggg 1500

acactggccc tgggaaccag gctgagctga gtggcctcaa accccccgtt 1550

ggatcagacc ctcctgtggg cagggttctt agtggatgag ttactgggaa 1600

gaatcagaga taaaaaccaa cccaaatcaa 1630

<210> 196
<211> 394
<212> PRT
<213> Homo Sapien

<400> 196

Met Phe Cys Pro Leu Lys Leu Ile Leu Leu Pro Val Leu Leu Asp
1               5                   10                  15

Tyr Ser Leu Gly Leu Asn Asp Leu Asn Val Ser Pro Pro Glu Leu
                20                  25                  30

Thr Val His Val Gly Asp Ser Ala Leu Met Gly Cys Val Phe Gln
                35                  40                  45

Ser Thr Glu Asp Lys Cys Ile Phe Lys Ile Asp Trp Thr Leu Ser
                50                  55                  60

Pro Gly Glu His Ala Lys Asp Glu Tyr Val Leu Tyr Tyr Tyr Ser
                65                  70                  75

Asn Leu Ser Val Pro Ile Gly Arg Phe Gln Asn Arg Val His Leu
                80                  85                  90

Met Gly Asp Ile Leu Cys Asn Asp Gly Ser Leu Leu Leu Gln Asp
                95                  100                 105

Val Gln Glu Ala Asp Gln Gly Thr Tyr Ile Cys Glu Ile Arg Leu
                110                 115                 120

Lys Gly Glu Ser Gln Val Phe Lys Lys Ala Val Val Leu His Val
                125                 130                 135

Leu Pro Glu Glu Pro Lys Glu Leu Met Val His Val Gly Gly Leu
                140                 145                 150

Ile Gln Met Gly Cys Val Phe Gln Ser Thr Glu Val Lys His Val
                155                 160                 165

Thr Lys Val Glu Trp Ile Phe Ser Gly Arg Arg Ala Lys Glu Glu
                170                 175                 180

Ile Val Phe Arg Tyr Tyr His Lys Leu Arg Met Ser Val Glu Tyr
                185                 190                 195

Ser Gln Ser Trp Gly His Phe Gln Asn Arg Val Asn Leu Val Gly
                200                 205                 210

Asp Ile Phe Arg Asn Asp Gly Ser Ile Met Leu Gln Gly Val Arg
                215                 220                 225

Glu Ser Asp Gly Gly Asn Tyr Thr Cys Ser Ile His Leu Gly Asn
                230                 235                 240

Leu Val Phe Lys Lys Thr Ile Val Leu His Val Ser Pro Glu Glu
                245                 250                 255

Pro Arg Thr Leu Val Thr Pro Ala Ala Leu Arg Pro Leu Val Leu
                260                 265                 270

Gly Gly Asn Gln Leu Val Ile Ile Val Gly Ile Val Cys Ala Thr
                275                 280                 285

Ile Leu Leu Leu Pro Val Leu Ile Leu Ile Val Lys Lys Thr Cys
                290                 295                 300

Gly Asn Lys Ser Ser Val Asn Ser Thr Val Leu Val Lys Asn Thr
                305                 310                 315

Lys Lys Thr Asn Pro Glu Ile Lys Glu Lys Pro Cys His Phe Glu
                320                 325                 330

Arg Cys Glu Gly Glu Lys His Ile Tyr Ser Pro Ile Ile Val Arg
                335                 340                 345

Glu Val Ile Glu Glu Glu Glu Pro Ser Glu Lys Ser Glu Ala Thr
                350                 355                 360

Tyr Met Thr Met His Pro Val Trp Pro Ser Leu Arg Ser Asp Arg
                365                 370                 375

Asn Asn Ser Leu Glu Lys Lys Ser Gly Gly Gly Met Pro Lys Thr
                380                 385                 390

Gln Gln Ala Phe

<210> 197
<211> 783
<212> DNA
<213> Homo Sapien

<400> 197
cgccatggcc gggctatccc gcgggtccgc gcgcgcactg ctcgccgccc 50

tgctggcgtc gacgctgttg gcgctgctcg tgtcgcccgc gcggggtcgc 100

ggcggccggg accacgggga ctgggacgag gcctcccggc tgccgccgct 150

accaccccgc gaggacgcgg cgcgcgtggc ccgcttcgtg acgcacgtct 200

ccgactgggg cgctctggcc accatctcca cgctggaggc ggtgcgcggc 250

cggcccttcg ccgacgtcct ctcgctcagc gacgggcccc cgggcgcggg 300

cagcggcgtg ccctatttct acctgagccc gctgcagctc tccgtgagca 350

acctgcagga gaatccatat gctacactga ccatgacttt ggcacagacc 400

aacttctgca agaaacatgg atttgatcca caaagtcccc tttgtgttca 450

cataatgctg tcaggaactg tgaccaaggt gaatgaaaca gaaatggata 500

ttgcaaagca ttcgttattc attcgacacc ctgagatgaa aacctggcct 550

tccagccata attggttctt tgctaagttg aatataacca atatctgggt 600

cctggactac tttggtggac caaaaatcgt gacaccagaa gaatattata 650

atgtcacagt tcagtgaagc agactgtggt gaatttagca acacttatga 700

agtttcttaa agtggctcat acacacttaa aaggcttaat gtttctctgg 750

aaagcgtccc agaatattag ccagttttct gtc 783

<210> 198
<211> 220
<212> PRT
<213> Homo Sapien

<400> 198
Met Ala Gly Leu Ser Arg Gly Ser Ala Arg Ala Leu Leu Ala Ala
  1               5                  10                  15

Leu Leu Ala Ser Thr Leu Leu Ala Leu Leu Val Ser Pro Ala Arg
                 20                  25                  30

Gly Arg Gly Gly Arg Asp His Gly Asp Trp Asp Glu Ala Ser Arg
                 35                  40                  45

Leu Pro Pro Leu Pro Pro Arg Glu Asp Ala Ala Arg Val Ala Arg
                 50                  55                  60

Phe Val Thr His Val Ser Asp Trp Gly Ala Leu Ala Thr Ile Ser
                65                  70                  75

Thr Leu Glu Ala Val Arg Gly Arg Pro Phe Ala Asp Val Leu Ser
                80                  85                  90

Leu Ser Asp Gly Pro Pro Gly Ala Gly Ser Gly Val Pro Tyr Phe
                95                  100                 105

Tyr Leu Ser Pro Leu Gln Leu Ser Val Ser Asn Leu Gln Glu Asn
                110                 115                 120

Pro Tyr Ala Thr Leu Thr Met Thr Leu Ala Gln Thr Asn Phe Cys
                125                 130                 135

Lys Lys His Gly Phe Asp Pro Gln Ser Pro Leu Cys Val His Ile
                140                 145                 150

Met Leu Ser Gly Thr Val Thr Lys Val Asn Glu Thr Glu Met Asp
                155                 160                 165

Ile Ala Lys His Ser Leu Phe Ile Arg His Pro Glu Met Lys Thr
                170                 175                 180

Trp Pro Ser Ser His Asn Trp Phe Phe Ala Lys Leu Asn Ile Thr
                185                 190                 195

Asn Ile Trp Val Leu Asp Tyr Phe Gly Gly Pro Lys Ile Val Thr
                200                 205                 210

Pro Glu Glu Tyr Tyr Asn Val Thr Val Gln
                215                 220

<210> 199
<211> 1315
<212> DNA
<213> Homo Sapien

<400> 199
tcgccatggc ctctgccgga atgcagatcc tgggagtcgt cctgacactg 50

ctgggctggg tgaatggcct ggtctcctgt gccctgccca tgtggaaggt 100

gaccgctttc atcggcaaca gcatcgtggt ggcccaggtg gtgtgggagg 150

gcctgtggat gtcctgcgtg gtgcagagca ccggccagat gcagtgcaag 200

gtgtacgact cactgctggc gctgccacag gacctgcagg ctgcacgtgc 250

cctctgtgtc atcgccctcc ttgtggccct gttcggcttg ctggtctacc 300

ttgctggggc caagtgtacc acctgtgtgg aggagaagga ttccaaggcc 350

cgcctggtgc tcacctctgg gattgtcttt gtcatctcag gggtcctgac 400

gctaatcccc gtgtgctgga cggcgcatgc catcatccgg gacttctata 450

accccctggt ggctgaggcc caaaagcggg agctgggggc ctccctctac 500

ttgggctggg cggcctcagg ccttttgttg ctgggtgggg ggttgctgtg 550

ctgcacttgc ccctcggggg ggtcccaggg ccccagccat tacatggccc 600

gctactcaac atctgcccct gccatctctc gggggccctc tgagtaccct 650

accaagaatt acgtctgacg tggaggggaa tgggggctcc gctggcgcta 700

gagccatcca gaagtggcag tgcccaacag ctttgggatg ggttcgtacc 750

ttttgtttct gcctcctgct atttttcttt tgactgagga tatttaaaat 800

tcatttgaaa actgagccaa ggtgttgact cagactctca cttaggctct 850

gctgtttctc acccttggat gatggagcca aagaggggat gctttgagat 900

tctggatctt gacatgccca tcttagaagc cagtcaagct atggaactaa 950

tgcggaggct gcttgctgtg ctggctttgc aacaagacag actgtcccca 1000

agagttcctg ctgctgctgg gggctgggct tccctagatg tcactggaca 1050

gctgcccccc atcctactca ggtctctgga gctcctctct tcacccctgg 1100

aaaaacaaat catctgttaa caaaggactg cccacctccg gaacttctga 1150

cctctgtttc ctccgtcctg ataagacgtc caccccccag ggccaggtcc 1200

cagctatgta gacccccgcc cccacctcca acactgcacc cttctgccct 1250

gcccccctcg tctcaccccc tttacactca catttttatc aaataaagca 1300

tgttttgtta gtgca 1315

<210> 200
<211> 220
<212> PRT
<213> Homo Sapien

<400> 200
Met Ala Ser Ala Gly Met Gln Ile Leu Gly Val Val Leu Thr Leu
1               5                   10                  15

Leu Gly Trp Val Asn Gly Leu Val Ser Cys Ala Leu Pro Met Trp
                20                  25                  30

Lys Val Thr Ala Phe Ile Gly Asn Ser Ile Val Val Ala Gln Val
                35                  40                  45

Val Trp Glu Gly Leu Trp Met Ser Cys Val Val Gln Ser Thr Gly
                50                  55                  60

Gln Met Gln Cys Lys Val Tyr Asp Ser Leu Leu Ala Leu Pro Gln
                65                  70                  75

Asp Leu Gln Ala Ala Arg Ala Leu Cys Val Ile Ala Leu Leu Val
                80                  85                  90

Ala Leu Phe Gly Leu Leu Val Tyr Leu Ala Gly Ala Lys Cys Thr
                95                  100                 105

Thr Cys Val Glu Glu Lys Asp Ser Lys Ala Arg Leu Val Leu Thr
                110                 115                 120

Ser Gly Ile Val Phe Val Ile Ser Gly Val Leu Thr Leu Ile Pro
                125                 130                 135

Val Cys Trp Thr Ala His Ala Ile Ile Arg Asp Phe Tyr Asn Pro
                140                 145                 150

Leu Val Ala Glu Ala Gln Lys Arg Glu Leu Gly Ala Ser Leu Tyr
                155                 160                 165

Leu Gly Trp Ala Ala Ser Gly Leu Leu Leu Leu Gly Gly Gly Leu
                170                 175                 180

Leu Cys Cys Thr Cys Pro Ser Gly Gly Ser Gln Gly Pro Ser His
                185                 190                 195

Tyr Met Ala Arg Tyr Ser Thr Ser Ala Pro Ala Ile Ser Arg Gly
                200                 205                 210

Pro Ser Glu Tyr Pro Thr Lys Asn Tyr Val
                215                 220

<210> 201
<211> 535
<212> DNA
<213> Homo Sapien

<400> 201
agtgacaatc tcagagcagc ttctacacca cagccatttc cagcatgaag 50

atcactgggg gtctccttct gctctgtaca gtggtctatt tctgtagcag 100

ctcagaagct gctagtctgt ctccaaaaaa agtggactgc agcatttaca 150

agaagtatcc agtggtggcc atcccctgcc ccatcacata cctaccagtt 200

tgtggttctg actacatcac ctatgggaat gaatgtcact tgtgtaccga 250

gagcttgaaa agtaatggaa gagttcagtt tcttcacgat ggaagttgct 300

aaattctcca tggacataga gagaaaggaa tgatattctc atcatcatct 350

tcatcatccc aggctctgac tgagtttctt tcagttttac tgatgttctg 400

ggtgggggac agagccagat tcagagtaat cttgactgaa tggagaaagt 450

ttctgtgcta cccctacaaa cccatgcctc actgacagac cagcattttt 500

tttttaacac gtcaataaaa aaataatctc ccaga 535

<210> 202
<211> 85
<212> PRT
<213> Homo Sapien

<400> 202
Met Lys Ile Thr Gly Gly Leu Leu Leu Leu Cys Thr Val Val Tyr
1               5                   10                  15

Phe Cys Ser Ser Ser Glu Ala Ala Ser Leu Ser Pro Lys Lys Val
                20                  25                  30

Asp Cys Ser Ile Tyr Lys Lys Tyr Pro Val Val Ala Ile Pro Cys
                35                  40                  45

Pro Ile Thr Tyr Leu Pro Val Cys Gly Ser Asp Tyr Ile Thr Tyr
                50                  55                  60

Gly Asn Glu Cys His Leu Cys Thr Glu Ser Leu Lys Ser Asn Gly
                65                  70                  75

Arg Val Gln Phe Leu His Asp Gly Ser Cys
                80                  85

<210> 203
<211> 2387
<212> DNA
<213> Homo Sapien

<400> 203
   cgacgatgct acgcgcgccc ggctgcctcc tccggacctc cgtagcgcct 50
   gccgcggccc tggctgcggc gctgctctcg tcgcttgcgc gctgctctct 100
   tctagagccg agggacccgg tggcctcgtc gctcagcccc tatttcggca 150
   ccaagactcg ctacgaggat gtcaaccccg tgctattgtc gggccccgag 200
   gctccgtggc gggaccctga gctgctggag gggacctgca ccccggtgca 250
   gctggtcgcc ctcattcgcc acggcacccg ctaccccacg gtcaaacaga 300
   tccgcaagct gaggcagctg cacgggttgc tgcaggcccg cgggtccagg 350
   gatggcgggg ctagtagtac cggcagccgc gacctgggtg cagcgctggc 400
   cgactggcct ttgtggtacg cggactggat ggacgggcag ctagtagaga 450
   agggacggca ggatatgcga cagctggcgc tgcgtctggc ctcgctcttc 500
   ccggcccttt tcagccgtga gaactacggc cgcctgcggc tcatcaccag 550
   ttccaagcac cgctgcatgg atagcagcgc cgccttcctg caggggctgt 600
   ggcagcacta ccaccctggc ttgccgccgc cggacgtcgc agatatggag 650
   tttggacctc caacagttaa tgataaacta atgagatttt ttgatcactg 700
   tgagaagttt ttaactgaag tagaaaaaaa tgctacagct ctttatcacg 750
   tggaagcctt caaaactgga ccagaaatgc agaacatttt aaaaaaagtt 800
   gcagctactt tgcaagtgcc agtaaatgat ttaaatgcag atttaattca 850
   agtagccttt ttcacctgtt catttgacct ggcaattaaa ggtgttaaat 900
   ctccttggtg tgatgttttt gacatagatg atgcaaaggt attagaatat 950
   ttaaatgatc tgaaacaata ttggaaaaga ggatatgggt atactattaa 1000
   cagtcgatcc agctgcacct tgtttcagga tatctttcag cacttggaca 1050
   aagcagttga acagaaacaa aggtctcagc caatttcttc tccagtcatc 1100
   ctccagtttg gtcatgcaga gactcttctt ccactgcttt ctctcatggg 1150
   ctacttcaaa gacaaggaac ccctaacagc gtacaattac aaaaaacaaa 1200
   tgcatcggaa gttccgaagt ggtctcattg taccttatgc ctcgaacctg 1250
   atatttgtgc tttaccactg tgaaaatgct aagactccta aagaacaatt 1300
   ccgagtgcag atgttattaa atgaaaaggt gttacctttg gcttactcac 1350
   aagaaactgt ttcattttat gaagatctga agaaccacta caaggacatc 1400
   cttcagagtt gtcaaaccag tgaagaatgt gaattagcaa gggctaacag 1450
   tacatctgat gaactatgag taactgaaga acatttttaa ttctttagga 1500
   atctgcaatg agtgattaca tgcttgtaat aggtaggcaa ttccttgatt 1550
   acaggaagct tttatattac ttgagtattt ctgtcttttc acagaaaaac 1600
   attgggtttc tctctgggtt tggacatgaa atgtaagaaa agatttttca 1650
   ctggagcagc tctcttaagg agaaacaaat ctatttagag aaacagctgg 1700
   ccctgcaaat gtttacagaa atgaaattct tcctacttat ataagaaatc 1750
   tcacactgag atagaattgt gatttcataa taacacttga aaagtgctgg 1800
   agtaacaaaa tatctcagtt ggaccatcct taacttgatt gaactgtcta 1850
   ggaactttac agattgttct gcagttctct cttcttttcc tcaggtagga 1900
   cagctctagc attttcttaa tcaggaatat tgtggtaagc tgggagtatc 1950
   actctggaag aaagtaacat ctccagatga gaatttgaaa caagaaacag 2000
   agtgttgtaa aaggacacct tcactgaagc aagtcggaaa gtacaatgaa 2050
   aataaatatt tttggtattt atttatgaaa tatttgaaca ttttttcaat 2100
   aattcctttt tacttctagg aagtctcaaa agaccatctt aaattattat 2150
   atgtttggac aattagcaac aagtcagata gttagaatcg aagtttttca 2200
   aatccattgc ttagctaact ttttcattct gtcacttggc ttcgattttt 2250
   atattttcct attatatgaa atgtatcttt tggttgtttg atttttcttt 2300
   ctttctttgt aaatagttct gagttctgtc aaatgccgtg aaagtatttg 2350
   ctataataaa gaaaattctt gtgactttaa aaaaaaa 2387

<210> 204
<211> 487
<212> PRT
<213> Homo Sapien

<400> 204

<210> 205
<211> 1098
<212> DNA
<213> Homo Sapien

<400> 205
   gcgacgcgcg gcggggcggc gagaggaaac gcggcgccgg gccgggcccg 50
   gccctggaga tggtccccgg cgccgcgggc tggtgttgtc tcgtgctctg 100
   gctccccgcg tgcgtcgcgg cccacggctt ccgtatccat gattatttgt 150
   actttcaagt gctgagtcct ggggacattc gatacatctt cacagccaca 200
   cctgccaagg actttggtgg tatctttcac acaaggtatg agcagattca 250
   ccttgtcccc gctgaacctc cagaggcctg cggggaactc agcaacggtt 300
   tcttcatcca ggaccagatt gctctggtgg agaggggggg ctgctccttc 350
   ctctccaaga ctcgggtggt ccaggagcac ggcgggcggg cggtgatcat 400
   ctctgacaac gcagttgaca atgacagctt ctacgtggag atgatccagg 450
   acagtaccca gcgcacagct gacatccccg ccctcttcct gctcggccga 500
   gacggctaca tgatccgccg ctctctggaa cagcatgggc tgccatgggc 550
   catcatttcc atcccagtca atgtcaccag catccccacc tttgagctgc 600
   tgcaaccgcc ctggaccttc tggtagaaga gtttgtccca cattccagcc 650
   ataagtgact ctgagctggg aaggggaaac ccaggaattt tgctacttgg 700
   aatttggaga tagcatctgg ggacaagtgg agccaggtag aggaaaaggg 750
   tttgggcgtt gctaggctga aagggaagcc acaccactgg ccttcccttc 800
   cccagggccc ccaagggtgt ctcatgctac aagaagaggc aagagacagg 850
   ccccagggct tctggctaga acccgaaaca aaaggagctg aaggcaggtg 900
   gcctgagagc catctgtgac ctgtcacact cacctggctc cagcctcccc 950
   tacccagggt ctctgcacag tgaccttcac agcagttgtt ggagtggttt 1000
   aaagagctgg tgtttgggga ctcaataaac cctcactgac tttttagcaa 1050
   taaagcttct catcagggtt gcaaaaaaaa aaaaaaaaaa aaaaaaaa 1098

<210> 206
<211> 188
<212> PRT
<213> Homo Sapien

<400> 206
Met Val Pro Gly Ala Ala Gly Trp Cys Cys Leu Val Leu Trp Leu
1               5                   10                  15

Pro Ala Cys Val Ala Ala His Gly Phe Arg Ile His Asp Tyr Leu
                20                  25                  30

Tyr Phe Gln Val Leu Ser Pro Gly Asp Ile Arg Tyr Ile Phe Thr
                35                  40                  45

Ala Thr Pro Ala Lys Asp Phe Gly Gly Ile Phe His Thr Arg Tyr
                50                  55                  60

Glu Gln Ile His Leu Val Pro Ala Glu Pro Pro Glu Ala Cys Gly
                65                  70                  75

Glu Leu Ser Asn Gly Phe Phe Ile Gln Asp Gln Ile Ala Leu Val

                80                  85                  90

Glu Arg Gly Gly Cys Ser Phe Leu Ser Lys Thr Arg Val Val Gln
                95                  100                 105

Glu His Gly Gly Arg Ala Val Ile Ile Ser Asp Asn Ala Val Asp
                110                 115                 120

Asn Asp Ser Phe Tyr Val Glu Met Ile Gln Asp Ser Thr Gln Arg
                125                 130                 135

Thr Ala Asp Ile Pro Ala Leu Phe Leu Leu Gly Arg Asp Gly Tyr
                140                 145                 150

Met Ile Arg Arg Ser Leu Glu Gln His Gly Leu Pro Trp Ala Ile
                155                 160                 165

Ile Ser Ile Pro Val Asn Val Thr Ser Ile Pro Thr Phe Glu Leu
                170                 175                 180

Leu Gln Pro Pro Trp Thr Phe Trp
                185

<210> 207
<211> 764
<212> DNA
<213> Homo Sapien

<400> 207
ctcgcttctt ccttctggat gggggcccag ggggcccagg agagtataaa 50

ggcgatgtgg agggtgcccg gcacaaccag acgcccagtc acaggcgaga 100

gccctgggat gcaccggcca gaggccatgc tgctgctgct cacgcttgcc 150

ctcctggggg gccccacctg ggcagggaag atgtatggcc ctggaggagg 200

caagtatttc agcaccactg aagactacga ccatgaaatc acagggctgc 250

gggtgtctgt aggtcttctc ctggtgaaaa gtgtccaggt gaaacttgga 300

gactcctggg acgtgaaact gggagcctta ggtgggaata cccaggaagt 350

caccctgcag ccaggcgaat acatcacaaa agtctttgtc gccttccaag 400

ctttcctccg gggtatggtc atgtacacca gcaaggaccg ctatttctat 450

tttgggaagc ttgatggcca gatctcctct gcctacccca gccaagaggg 500

gcaggtgctg gtgggcatct atggccagta tcaactcctt ggcatcaaga 550

gcattggctt tgaatggaat tatccactag aggagccgac cactgagcca 600

ccagttaatc tcacatactc agcaaactca cccgtgggtc gctagggtgg 650

ggtatggggc catccgagct gaggccatct gtgtggtggt ggctgatggt 700

actggagtaa ctgagtcggg acgctgaatc tgaatccacc aataaataaa 750

gcttctgcag aaaa 764

<210> 208
<211> 178
<212> PRT
<213> Homo Sapien

<400> 208
Met His Arg Pro Glu Ala Met Leu Leu Leu Leu Thr Leu Ala Leu

1               5                   10                  15

Leu Gly Gly Pro Thr Trp Ala Gly Lys Met Tyr Gly Pro Gly Gly
                20                  25                  30

Gly Lys Tyr Phe Ser Thr Thr Glu Asp Tyr Asp His Glu Ile Thr
                35                  40                  45

Gly Leu Arg Val Ser Val Gly Leu Leu Leu Val Lys Ser Val Gln
                50                  55                  60

Val Lys Leu Gly Asp Ser Trp Asp Val Lys Leu Gly Ala Leu Gly
                65                  70                  75

Gly Asn Thr Gln Glu Val Thr Leu Gln Pro Gly Glu Tyr Ile Thr
                80                  85                  90

Lys Val Phe Val Ala Phe Gln Ala Phe Leu Arg Gly Met Val Met
                95                  100                 105

Tyr Thr Ser Lys Asp Arg Tyr Phe Tyr Phe Gly Lys Leu Asp Gly
                110                 115                 120

Gln Ile Ser Ser Ala Tyr Pro Ser Gln Glu Gly Gln Val Leu Val
                125                 130                 135

Gly Ile Tyr Gly Gln Tyr Gln Leu Leu Gly Ile Lys Ser Ile Gly
                140                 145                 150

Phe Glu Trp Asn Tyr Pro Leu Glu Glu Pro Thr Thr Glu Pro Pro
                155                 160                 165

Val Asn Leu Thr Tyr Ser Ala Asn Ser Pro Val Gly Arg
                170                 175

<210> 209
<211> 3824
<212> DNA
<213> Homo Sapien

<400> 209
ggagaatgga gagagcagtg agagtggagt ccggggtcct ggtcggggtg 50

gtctgtctgc tcctggcatg ccctgccaca gccactgggc ccgaagttgc 100

tcagcctgaa gtagacacca ccctgggtcg tgtgcgaggc cggcaggtgg 150

gcgtgaaggg cacagaccgc cttgtgaatg tctttctggg cattccattt 200

gcccagccgc cactgggccc tgaccggttc tcagccccac acccagcaca 250

gccctgggag ggtgtgcggg atgccagcac tgcgccccca atgtgcctac 300

aagacgtgga gagcatgaac agcagcagat ttgtcctcaa cggaaaacag 350

cagatcttct ccgtttcaga ggactgcctg gtcctcaacg tctatagccc 400

agctgaggtc cccgcagggt ccggtaggcc ggtcatggta tgggtccatg 450

gaggcgctct gataactggc gctgccacct cctacgatgg atcagctctg 500

gctgcctatg gggatgtggt cgtggttaca gtccagtacc gccttggggt 550

ccttggcttc ttcagcactg gagatgagca tgcacctggc aaccagggct 600

tcctagatgt ggtagctgct ttgcgctggg tgcaagaaaa catcgccccc 650

ttcgggggtg acctcaactg tgtcactgtc tttggtggat ctgccggtgg 700

gagcatcatc tctggcctgg tcctgtcccc agtggctgca gggctgttcc 750
acagagccat cacacagagt ggggtcatca ccaccccagg gatcatcgac 800
tctcaccctt ggcccctagc tcagaaaatc gcaaacacct tggcctgcag 850
ctccagctcc ccggctgaga tggtgcagtg ccttcagcag aaagaaggag 900
aagagctggt ccttagcaag aagctgaaaa atactatcta tcctctcacc 950
gttgatggca ctgtcttccc caaaagcccc aaggaactcc tgaaggagaa 1000
gcccttccac tctgtgccct tcctcatggg tgtcaacaac catgagttca 1050
gctggctcat ccccaggggc tggggtctcc tggatacaat ggagcagatg 1100
agccgggagg acatgctggc catctcaaca cccgtcttga ccagtctgga 1150
tgtgccccct gagatgatgc ccaccgtcat agatgaatac ctaggaagca 1200
actcggacgc acaagccaaa tgccaggcgt tccaggaatt catgggtgac 1250
gtattcatca atgttcccac cgtcagtttt tcaagatacc ttcgagattc 1300
tggaagccct gtctttttct atgagttcca gcatcgaccc agttcttttg 1350
cgaagatcaa acctgcctgg gtgaaggctg atcatggggc cgagggtgct 1400
tttgtgttcg gaggtccctt cctcatggac gagagctccc gcctggcctt 1450
tccagaggcc acagaggagg agaagcagct aagcctcacc atgatggccc 1500
agtggaccca ctttgcccgg acaggggacc ccaatagcaa ggctctgcct 1550
ccttggcccc aattcaacca ggcggaacaa tatctggaga tcaacccagt 1600
gccacgggcc ggacagaagt tcagggaggc ctggatgcag ttctggtcag 1650
agacgctccc cagcaagata caacagtggc accagaagca gaagaacagg 1700
aaggcccagg aggacctctg aggccaggcc tgaaccttct tggctggggc 1750
aaaccactct tcaagtggtg gcagagtccc agcacggcag cccgcctctc 1800
cccctgctga gactttaatc tccaccagcc cttaaagtgt cggccgctct 1850
gtgactggag ttatgctctt ttgaaatgtc acaaggccgc ctcccacctc 1900
tggggcattg tacaagttct tccctctccc tgaagtgcct ttcctgcttt 1950
cttcgtggta ggttctagca cattcctcta gcttcctgga ggactcactc 2000
cccaggaagc cttccctgcc ttctctgggc tgtgcggccc cgagtctgcg 2050
tccattagag cacagtccac ccgaggctag caccgtgtct gtgtctgtct 2100
ccccctcaga ggagctctct caaaatgggg attagcctaa ccccactctg 2150
tcacccacac caggatcggg tgggacctgg agctaggggg tgtttgctga 2200
gtgagtgagt gaaacacaga atatgggaat ggcagctgct gaacttgaac 2250
ccagagcctt caggtgccaa agccatactc aggcccccac cgacattgtc 2300
caccctggcc agaagggtgc atgccaatgg cagagacctg ggatgggaga 2350
agtcctgggg cgccagggga tccagcctag agcagacctt agcccctgac 2400

taaggcctca gactagggcg ggaggggtct cctcctctct gctgcccagt 2450
cctggcccct gcacaagaca acagaatcca tcagggccat gagtgtcacc 2500
cagacctgac cctcaccaat tccagcccct gaccctcagg acgctggatg 2550
ccagctccca gccccagtgc cgggtcctcc ctcccttcct ggcttgggga 2600
gaccagtttc tggggagctt ccaagagcac ccaccaagac acagcaggac 2650
aggccagggg agggcatctg gaccagggca tccgtcgggc tattgtcaca 2700
gagaaaagaa gagacccacc cactcgggct gcaaaaggtg aaaagcacca 2750
agaggttttc agatggaagt gagaggtgac agtgtgctgg cagccctcac 2800
agccctcgct tgctctccct gccgcctctg cctgggctcc cactttggca 2850
gcacttgagg agcccttcaa cccgccgctg cactgtagga gcccctttct 2900
gggctggcca aggccggagc cagctccctc agcttgcggg gaggtgcgga 2950
gggagagggg cgggcaggaa ccggggctgc gcgcagcgct tgcgggccag 3000
agtgagttcc gggtgggcgt gggctcggcg gggccccact cagagcagct 3050
ggccggcccc aggcagtgag ggccttagca cctgggccag cagctgctgt 3100
gctcgatttc tcgctgggcc ttagctgcct ccccgcgggg cagggctcgg 3150
gacctgcagc cctccatgcc tgaccctccc cccacccccc gtgggctcct 3200
gtgcggccgg agcctcccca aggagcgccg ccccctgctc cacagcgccc 3250
agtcccatcg accacccaag ggctgaggag tgcgggtgca cagcgcggga 3300
ctggcaggca gctccacctg ctgccccagt gctggatcca ctgggtgaag 3350
ccagctgggc tcctgagtct ggtggggact tggagaacct ttatgtctag 3400
ctaagggatt gtaaatacac cgatgggcac tctgtatcta gctcaaggtt 3450
tgtaaacaca ccaatcagca ccctgtgtct agctcagtgt ttgtgaatgc 3500
accaatccac actctgtatc tggctactct ggtggggact tggagaacct 3550
ttgtgtccac actctgtatc tagctaatct agtggggatg tggagaacct 3600
ttgtgtctag ctcaggatcg gtaaacgcac caatcagcac cctgtcaaaa 3650
cagaccactt gactctctgt aaaatggacc aatcagcagg atgtgggtgg 3700
ggcgagacaa gagaataaaa gcaggctgcc tgagccagca gtgacaaccc 3750
ccctcgggtc ccctcccacg ccgtggaagc tttgttcttt cgctctttgc 3800
aataaatctt gctactgccc aaaa 3824

<210> 210
<211> 571
<212> PRT
<213> Homo Sapien

<400> 210
Met Glu Arg Ala Val Arg Val Glu Ser Gly Val Leu Val Gly Val
1               5                   10                  15

Val Cys Leu Leu Leu Ala Cys Pro Ala Thr Ala Thr Gly Pro Glu
                20                  25                  30

Val Ala Gln Pro Glu Val Asp Thr Thr Leu Gly Arg Val Arg Gly
                35                  40                  45

Arg Gln Val Gly Val Lys Gly Thr Asp Arg Leu Val Asn Val Phe
                50                  55                  60

Leu Gly Ile Pro Phe Ala Gln Pro Pro Leu Gly Pro Asp Arg Phe
                65                  70                  75

Ser Ala Pro His Pro Ala Gln Pro Trp Glu Gly Val Arg Asp Ala
                80                  85                  90

Ser Thr Ala Pro Pro Met Cys Leu Gln Asp Val Glu Ser Met Asn
                95                  100                 105

Ser Ser Arg Phe Val Leu Asn Gly Lys Gln Gln Ile Phe Ser Val
                110                 115                 120

Ser Glu Asp Cys Leu Val Leu Asn Val Tyr Ser Pro Ala Glu Val
                125                 130                 135

Pro Ala Gly Ser Gly Arg Pro Val Met Val Trp Val His Gly Gly
                140                 145                 150

Ala Leu Ile Thr Gly Ala Ala Thr Ser Tyr Asp Gly Ser Ala Leu
                155                 160                 165

Ala Ala Tyr Gly Asp Val Val Val Val Thr Val Gln Tyr Arg Leu
                170                 175                 180

Gly Val Leu Gly Phe Phe Ser Thr Gly Asp Glu His Ala Pro Gly
                185                 190                 195

Asn Gln Gly Phe Leu Asp Val Val Ala Ala Leu Arg Trp Val Gln
                200                 205                 210

Glu Asn Ile Ala Pro Phe Gly Gly Asp Leu Asn Cys Val Thr Val
                215                 220                 225

Phe Gly Gly Ser Ala Gly Gly Ser Ile Ile Ser Gly Leu Val Leu
                230                 235                 240

Ser Pro Val Ala Ala Gly Leu Phe His Arg Ala Ile Thr Gln Ser
                245                 250                 255

Gly Val Ile Thr Thr Pro Gly Ile Ile Asp Ser His Pro Trp Pro
                260                 265                 270

Leu Ala Gln Lys Ile Ala Asn Thr Leu Ala Cys Ser Ser Ser Ser
                275                 280                 285

Pro Ala Glu Met Val Gln Cys Leu Gln Gln Lys Glu Gly Glu Glu
                290                 295                 300

Leu Val Leu Ser Lys Lys Leu Lys Asn Thr Ile Tyr Pro Leu Thr
                305                 310                 315

Val Asp Gly Thr Val Phe Pro Lys Ser Pro Lys Glu Leu Leu Lys
                320                 325                 330

Glu Lys Pro Phe His Ser Val Pro Phe Leu Met Gly Val Asn Asn
                335                 340                 345

His Glu Phe Ser Trp Leu Ile Pro Arg Gly Trp Gly Leu Leu Asp
                350                 355                 360

Thr Met Glu Gln Met Ser Arg Glu Asp Met Leu Ala Ile Ser Thr
                365                 370                 375

Pro Val Leu Thr Ser Leu Asp Val Pro Pro Glu Met Met Pro Thr
                380                 385                 390

Val Ile Asp Glu Tyr Leu Gly Ser Asn Ser Asp Ala Gln Ala Lys
                395                 400                 405

Cys Gln Ala Phe Gln Glu Phe Met Gly Asp Val Phe Ile Asn Val
                410                 415                 420

Pro Thr Val Ser Phe Ser Arg Tyr Leu Arg Asp Ser Gly Ser Pro
                425                 430                 435

Val Phe Phe Tyr Glu Phe Gln His Arg Pro Ser Ser Phe Ala Lys
                440                 445                 450

Ile Lys Pro Ala Trp Val Lys Ala Asp His Gly Ala Glu Gly Ala
                455                 460                 465

Phe Val Phe Gly Gly Pro Phe Leu Met Asp Glu Ser Ser Arg Leu
                470                 475                 480

Ala Phe Pro Glu Ala Thr Glu Glu Glu Lys Gln Leu Ser Leu Thr
                485                 490                 495

Met Met Ala Gln Trp Thr His Phe Ala Arg Thr Gly Asp Pro Asn
                500                 505                 510

Ser Lys Ala Leu Pro Pro Trp Pro Gln Phe Asn Gln Ala Glu Gln
                515                 520                 525

Tyr Leu Glu Ile Asn Pro Val Pro Arg Ala Gly Gln Lys Phe Arg
                530                 535                 540

Glu Ala Trp Met Gln Phe Trp Ser Glu Thr Leu Pro Ser Lys Ile
                545                 550                 555

Gln Gln Trp His Gln Lys Gln Lys Asn Arg Lys Ala Gln Glu Asp
                560                 565                 570

Leu

<210> 211
<211> 1177
<212> DNA
<213> Homo Sapien

<400> 211
aacttctaca tgggcctcct gctgctggtg ctcttcctca gcctcctgcc 50

ggtggcctac accatcatgt ccctcccacc ctcctttgac tgcgggccgt 100

tcaggtgcag agtctcagtt gcccgggagc acctcccctc ccgaggcagt 150

ctgctcagag ggcctcggcc cagaattcca gttctggttt catgccagcc 200

tgtaaaaggc catggaactt tgggtgaatc accgatgcca tttaagaggg 250

ttttctgcca ggatggaaat gttaggtcgt tctgtgtctg cgctgttcat 300

ttcagtagcc accagccacc tgtggccgtt gagtgcttga aatgaggaac 350

tgagaaaatt aatttctcat gtatttttct catttattta ttaattttta 400

actgatagtt gtacatattt gggggtacat gtgatatttg gatacatgta 450

tacaatatat aatgatcaaa tcagggtaac tgggatatcc atcacatcaa 500

acatttattt tttattcttt ttagacagag tctcactctg tcacccaggc 550

tggagtgcag tggtgccatc tcagcttact gcaacctctg cctgccaggt 600

tcaagcgatt ctcatgcctc cacctcccaa gtagctggga ctacaggcat 650

gcaccacaat gcccaactaa tttttgtatt tttagtagag acggggtttt 700

gccatgttgc ccaggctggc cttgaactcc tggcctcaaa caatccactt 750

gcctcggcct cccaaagtgt tatgattaca ggcgtgagcc accgtgcctg 800

gcctaaacat ttatcttttc tttgtgttgg gaactttgaa attatacaat 850

gaattattgt taactgtcat ctccctgctg tgctatggaa cactgggact 900

tcttccctct atctaactgt atatttgtac cagttaacca accgtacttc 950

atccccactc ctctctatcc ttcccaacct ctgatcacct cattctactc 1000

tctacctcca tgagatccac ttttttagct cccacatgtg agtaagaaaa 1050

tgcaatattt gtctttctgt gcctggctta tttcacttaa cataatgact 1100

tcctgttcca tccatgttgc tgcaaatgac aggatttcgt tcttaatttc 1150

aattaaaata accacacatg gcaaaaa 1177

<210> 212
<211> 111
<212> PRT
<213> Homo Sapien

<400> 212
Met Gly Leu Leu Leu Leu Val Leu Phe Leu Ser Leu Leu Pro Val
1               5                   10                  15

Ala Tyr Thr Ile Met Ser Leu Pro Pro Ser Phe Asp Cys Gly Pro
                20                  25                  30

Phe Arg Cys Arg Val Ser Val Ala Arg Glu His Leu Pro Ser Arg
                35                  40                  45

Gly Ser Leu Leu Arg Gly Pro Arg Pro Arg Ile Pro Val Leu Val
                50                  55                  60

Ser Cys Gln Pro Val Lys Gly His Gly Thr Leu Gly Glu Ser Pro
                65                  70                  75

Met Pro Phe Lys Arg Val Phe Cys Gln Asp Gly Asn Val Arg Ser
                80                  85                  90

Phe Cys Val Cys Ala Val His Phe Ser Ser His Gln Pro Pro Val
                95                  100                 105

Ala Val Glu Cys Leu Lys
                110

<210> 213
<211> 2159
<212> DNA
<213> Homo Sapien

<400> 213

agggcccgcg ggtggagaga gcgacgcccg aggggatggc ggcagcgtcc 50
cggagcgcct ctggctgggc gctactgctg ctggtggcac tttggcagca 100
gcgcgcggcc ggctccggcg tcttccagct gcagctgcag gagttcatca 150
acgagcgcgg cgtactggcc agtgggcggc cttgcgagcc cggctgccgg 200
actttcttcc gcgtctgcct taagcacttc caggcggtcg tctcgcccgg 250
accctgcacc ttcgggaccg tctccacgcc ggtattgggc accaactcct 300
tcgctgtccg ggacgacagt agcggcgggg ggcgcaaccc tctccaactg 350
cccttcaatt tcacctggcc gggtaccttc tcgctcatca tcgaagcttg 400
gcacgcgcca ggagacgacc tgcggccaga ggccttgcca ccagatgcac 450
tcatcagcaa gatcgccatc cagggctccc tagctgtggg tcagaactgg 500
ttattggatg agcaaaccag caccctcaca aggctgcgct actcttaccg 550
ggtcatctgc agtgacaact actatggaga caactgctcc cgcctgtgca 600
agaagcgcaa tgaccacttc ggccactatg tgtgccagcc agatggcaac 650
ttgtcctgcc tgcccggttg gactggggaa tattgccaac agcctatctg 700
tctttcgggc tgtcatgaac agaatggcta ctgcagcaag ccagcagagt 750
gcctctgccg cccaggctgg cagggccggc tgtgtaacga atgcatcccc 800
cacaatggct gtcgccacgg cacctgcagc actccctggc aatgtacttg 850
tgatgagggc tggggaggcc tgttttgtga ccaagatctc aactactgca 900
cccaccactc cccatgcaag aatggggcaa cgtgctccaa cagtgggcag 950
cgaagctaca cctgcacctg tcgcccaggc tacactggtg tggactgtga 1000
gctggagctc agcgagtgtg acagcaaccc ctgtcgcaat ggaggcagct 1050
gtaaggacca ggaggatggc taccactgcc tgtgtcctcc gggctactat 1100
ggcctgcact gtgaacacag caccttgagc tgcgccgact ccccctgctt 1150
caatgggggc tcctgccggg agcgcaacca gggggccaac tatgcttgtg 1200
aatgtccccc caacttcacc ggctccaact gcgagaagaa agtggacagg 1250
tgcaccagca acccctgtgc caacggggga cagtgcctga accgaggtcc 1300
aagccgcatg tgccgctgcc gtcctggatt cacgggcacc tactgtgaac 1350
tccacgtcag cgactgtgcc cgtaaccctt gcgcccacgg tggcacttgc 1400
catgacctgg agaatgggct catgtgcacc tgccctgccg gcttctctgg 1450
ccgacgctgt gaggtgcgga catccatcga tgcctgtgcc tcgagtccct 1500
gcttcaacag ggccacctgc tacaccgacc tctccacaga cacctttgtg 1550
tgcaactgcc cttatggctt tgtgggcagc cgctgcgagt tccccgtggg 1600
cttgccgccc agcttcccct gggtggccgt ctcgctgggt gtggggctgg 1650
cagtgctgct ggtactgctg ggcatggtgg cagtggctgt gcggcagctg 1700

cggcttcgac ggccggacga cggcagcagg gaagccatga acaacttgtc 1750

ggacttccag aaggacaacc tgattcctgc cgcccagctt aaaaacacaa 1800

accagaagaa ggagctggaa gtggactgtg gcctggacaa gtccaactgt 1850

ggcaaacagc aaaaccacac attggactat aatctggccc cagggcccct 1900

ggggcggggg accatgccag gaaagtttcc ccacagtgac aagagcttag 1950

gagagaaggc gccactgcgg ttacacagtg aaaagccaga gtgtcggata 2000

tcagcgatat gctcccccag ggactccatg taccagtctg tgtgtttgat 2050

atcagaggag aggaatgaat gtgtcattgc cacggaggta taaggcagga 2100

gcctacctgg acatccctgc tcagccccgc ggctggacct tccttctgca 2150

ttgtttaca 2159

<210> 214
<211> 685
<212> PRT
<213> Homo Sapien

<400> 214

<210> 215
<211> 3060
<212> DNA
<213> Homo Sapien

<400> 215
cgcgaggcgc ggggagcctg ggaccaggag cgagagccgc ctacctgcag 50

ccgccgccca cggcacggca gccaccatgg cgctcctgct gtgcttcgtg 100

ctcctgtgcg gagtagtgga tttcgccaga agtttgagta tcactactcc 150

tgaagagatg attgaaaaag ccaaagggga aactgcctat ctgccatgca 200

aatttacgct tagtcccgaa gaccagggac cgctggacat cgagtggctg 250

atatcaccag ctgataatca gaaggtggat caagtgatta ttttatattc 300

tggagacaaa atttatgatg actactatcc agatctgaaa ggccgagtac 350

attttacgag taatgatctc aaatctggtg atgcatcaat aaatgtaacg 400

aatttacaac tgtcagatat tggcacatat cagtgcaaag tgaaaaaagc 450

tcctggtgtt gcaaataaga agattcatct ggtagttctt gttaagcctt 500

caggtgcgag atgttacgtt gatggatctg aagaaattgg aagtgacttt 550

aagataaaat gtgaaccaaa agaaggttca cttccattac agtatgagtg 600

gcaaaaattg tctgactcac agaaaatgcc cacttcatgg ttagcagaaa 650

tgacttcatc tgttatatct gtaaaaaatg cctcttctga gtactctggg 700

acatacagct gtacagtcag aaacagagtg ggctctgatc agtgcctgtt 750

gcgtctaaac gttgtccctc cttcaaataa agctggacta attgcaggag 800

ccattatagg aactttgctt gctctagcgc tcattggtct tatcatcttt 850

tgctgtcgta aaaagcgcag agaagaaaaa tatgaaaagg aagttcatca 900
cgatatcagg gaagatgtgc cacctccaaa gagccgtacg tccactgcca 950
gaagctacat cggcagtaat cattcatccc tggggtccat gtctccttcc 1000
aacatggaag gatattccaa gactcagtat aaccaagtac caagtgaaga 1050
ctttgaacgc actcctcaga gtccgactct cccacctgct aagttcaagt 1100
acccttacaa gactgatgga attacagttg tataaatatg gactactgaa 1150
gaatctgaag tattgtatta tttgacttta ttttaggcct ctagtaaaga 1200
cttaaatgtt ttttaaaaaa agcacaaggc acagagatta gagcagctgt 1250
aagaacacat ctactttatg caatggcatt agacatgtaa gtcagatgtc 1300
atgtcaaaat tagtacgagc caaattcttt gttaaaaaac cctatgtata 1350
gtgacactga tagttaaaag atgttttatt atattttcaa taactaccac 1400
taacaaattt ttaacttttc atatgcatat tctgatatgt ggtcttttag 1450
gaaaagtatg gttaatagtt gatttttcaa aggaaatttt aaaattctta 1500
cgttctgttt aatgtttttg ctatttagtt aaatacattg aagggaaata 1550
cccgttcttt tcccctttta tgcacacaac agaaacacgc gttgtcatgc 1600
ctcaaactat tttttatttg caactacatg atttcacaca attctcttaa 1650
acaacgacat aaaatagatt tccttgtata taaataactt acatacgctc 1700
cataaagtaa attctcaaag gtgctagaac aaatcgtcca cttctacagt 1750
gttctcgtat ccaacagagt tgatgcacaa tatataaata ctcaagtcca 1800
atattaaaaa cttaggcact tgactaactt taataaaatt tctcaaacta 1850
tatcaatatc taaagtgcat atatttttta agaaagatta ttctcaataa 1900
cttctataaa aataagtttg atggtttggc ccatctaact tcactactat 1950
tagtaagaac ttttaacttt taatgtgtag taaggtttat tctacctttt 2000
tctcaacatg acaccaacac aatcaaaaac gaagttagtg aggtgctaac 2050
atgtgaggat taatccagtg attccggtca caatgcattc caggaggagg 2100
tacccatgtc actggaattg ggcgatatgg tttatttttt cttccctgat 2150
ttggataacc aaatggaaca ggaggaggat agtgattctg atggccattc 2200
cctcgataca ttcctggctt ttttctgggc aaagggtgcc acattggaag 2250
aggtggaaat ataagttctg aaatctgtag ggaagagaac acattaagtt 2300
aattcaaagg aaaaaatcat catctatgtt ccagatttct cattaaagac 2350
aaagttaccc acaacactga gatcacatct aagtgacact cctattgtca 2400
ggtctaaata cattaaaaac ctcatgtgta ataggcgtat aatgtataac 2450
aggtgaccaa tgttttctga atgcataaag aaatgaataa actcaaacac 2500
agtacttcct aaacaacttc aaccaaaaaa gaccaaaaca tggaacgaat 2550

ggaagcttgt aaggacatgc ttgttttagt ccagtggttt ccacagctgg 2600

ctaagccagg agtcacttgg aggcttttaa atacaaaaca ttggagctgg 2650

aggccattat ccttagcaaa ctaatgcaga aacagaaaat caactaccgc 2700

atgttctcac ttataagtgg gaggtaatga taagaactta tgaacacaaa 2750

gaaggaaaca atagacattg gagtctattt gagaggggag ggtgggagaa 2800

ggaaaaggag cagaaaagat aactattgag tactgccttc acacctgggt 2850

gatgaaataa tatgtacaac aaatccctgt gacacatgtt tacctatgga 2900

acaaaccttc atgtgtatcc ctaaacctaa aataaaagtt aaaaaaaaaa 2950

aaaraaaaaa aaaaaaaaaa aaaaaaaaaa aaaaaaaaaa aaaaaaaaaa 3000

aaaaaaaaaa aaaaaaaaaa aaaaaaaaaa aaaaaaaaaa aaaaaaaaaa 3050

aaaaaaaaaa 3060

<210> 216
<211> 352
<212> PRT
<213> Homo Sapien

<400> 216
Met Ala Leu Leu Leu Cys Phe Val Leu Leu Cys Gly Val Val Asp
1               5                   10                  15

Phe Ala Arg Ser Leu Ser Ile Thr Thr Pro Glu Glu Met Ile Glu
                20                  25                  30

Lys Ala Lys Gly Glu Thr Ala Tyr Leu Pro Cys Lys Phe Thr Leu
                35                  40                  45

Ser Pro Glu Asp Gln Gly Pro Leu Asp Ile Glu Trp Leu Ile Ser
                50                  55                  60

Pro Ala Asp Asn Gln Lys Val Asp Gln Val Ile Ile Leu Tyr Ser
                65                  70                  75

Gly Asp Lys Ile Tyr Asp Asp Tyr Tyr Pro Asp Leu Lys Gly Arg
                80                  85                  90

Val His Phe Thr Ser Asn Asp Leu Lys Ser Gly Asp Ala Ser Ile
                95                  100                 105

Asn Val Thr Asn Leu Gln Leu Ser Asp Ile Gly Thr Tyr Gln Cys
                110                 115                 120

Lys Val Lys Lys Ala Pro Gly Val Ala Asn Lys Lys Ile His Leu
                125                 130                 135

Val Val Leu Val Lys Pro Ser Gly Ala Arg Cys Tyr Val Asp Gly
                140                 145                 150

Ser Glu Glu Ile Gly Ser Asp Phe Lys Ile Lys Cys Glu Pro Lys
                155                 160                 165

Glu Gly Ser Leu Pro Leu Gln Tyr Glu Trp Gln Lys Leu Ser Asp
                170                 175                 180

Ser Gln Lys Met Pro Thr Ser Trp Leu Ala Glu Met Thr Ser Ser
                185                 190                 195

Val Ile Ser Val Lys Asn Ala Ser Ser Glu Tyr Ser Gly Thr Tyr

                200                 205                 210

Ser Cys Thr Val Arg Asn Arg Val Gly Ser Asp Gln Cys Leu Leu
                215                 220                 225

Arg Leu Asn Val Val Pro Pro Ser Asn Lys Ala Gly Leu Ile Ala
                230                 235                 240

Gly Ala Ile Ile Gly Thr Leu Leu Ala Leu Ala Leu Ile Gly Leu
                245                 250                 255

Ile Ile Phe Cys Cys Arg Lys Lys Arg Arg Glu Glu Lys Tyr Glu
                260                 265                 270

Lys Glu Val His His Asp Ile Arg Glu Asp Val Pro Pro Pro Lys
                275                 280                 285

Ser Arg Thr Ser Thr Ala Arg Ser Tyr Ile Gly Ser Asn His Ser
                290                 295                 300

Ser Leu Gly Ser Met Ser Pro Ser Asn Met Glu Gly Tyr Ser Lys
                305                 310                 315

Thr Gln Tyr Asn Gln Val Pro Ser Glu Asp Phe Glu Arg Thr Pro
                320                 325                 330

Gln Ser Pro Thr Leu Pro Pro Ala Lys Phe Lys Tyr Pro Tyr Lys
                335                 340                 345

Thr Asp Gly Ile Thr Val Val
                350

<210> 217
<211> 1571
<212> DNA
<213> Homo Sapien

<400> 217
gatggcgcag ccacagcttc tgtgagattc gatttctccc cagttcccct 50

gtgggtctga ggggaccaga agggtgagct acgttggctt tctggaaggg 100

gaggctatat gcgtcaattc cccaaaacaa gttttgacat ttcccctgaa 150

atgtcattct ctatctattc actgcaagtg cctgctgttc caggccttac 200

ctgctgggca ctaacggcgg agccaggatg gggacagaat aaaggagcca 250

cgacctgtgc caccaactcg cactcagact ctgaactcag acctgaaatc 300

ttctcttcac gggaggcttg gcagtttttc ttactcctgt ggtctccaga 350

tttcaggcct aagatgaaag cctctagtct tgccttcagc cttctctctg 400

ctgcgtttta tctcctatgg actccttcca ctggactgaa gacactcaat 450

ttgggaagct gtgtgatcgc cacaaacctt caggaaatac gaaatggatt 500

ttctgagata cggggcagtg tgcaagccaa agatggaaac attgacatca 550

gaatcttaag gaggactgag tctttgcaag acacaaagcc tgcgaatcga 600

tgctgcctcc tgcgccattt gctaagactc tatctggaca gggtatttaa 650

aaactaccag acccctgacc attatactct ccggaagatc agcagcctcg 700

ccaattcctt tcttaccatc aagaaggacc tccggctctc tcatgcccac 750

atgacatgcc attgtgggga ggaagcaatg aagaaataca gccagattct 800

gagtcacttt gaaaagctgg aacctcaggc agcagttgtg aaggctttgg 850

gggaactaga cattcttctg caatggatgg aggagacaga ataggaggaa 900

agtgatgctg ctgctaagaa tattcgaggt caagagctcc agtcttcaat 950

acctgcagag gaggcatgac cccaaaccac catctcttta ctgtactagt 1000

cttgtgctgg tcacagtgta tcttatttat gcattacttg cttccttgca 1050

tgattgtctt tatgcatccc caatcttaat tgagaccata cttgtataag 1100

atttttgtaa tatctttctg ctattggata tatttattag ttaatatatt 1150

tatttatttt ttgctattta atgtatttat ttttttactt ggacatgaaa 1200

ctttaaaaaa attcacagat tatatttata acctgactag agcaggtgat 1250

gtatttttat acagtaaaaa aaaaaaacct tgtaaattct agaagagtgg 1300

ctaggggggt tattcatttg tattcaacta aggacatatt tactcatgct 1350

gatgctctgt gagatatttg aaattgaacc aatgactact taggatgggt 1400

tgtggaataa gttttgatgt ggaattgcac atctacctta caattactga 1450

ccatccccag tagactcccc agtcccataa ttgtgtatct tccagccagg 1500

aatcctacac ggccagcatg tatttctaca aataaagttt tctttgcata 1550

ccaaaaaaaa aaaaaaaaaa a 1571

<210> 218
<211> 176
<212> PRT
<213> Homo Sapien

<400> 218

<210> 219
<211> 2870
<212> DNA
<213> Homo Sapien

<400> 219
cgcggagccc tgcgctggga ggtgcacggt gtgcacgctg gactggaccc 50

ccatgcaacc ccgcgccctg cgccttaacc aggactgctc cgcgcgcccc 100

tgagcctcgg gctccggccc ggacctgcag cctcccaggt ggctgggaag 150

aactctccaa caataaatac atttgataag aaagatggct ttaaaagtgc 200

tactagaaca agagaaaacg tttttcactc ttttagtatt actaggctat 250

ttgtcatgta aagtgacttg tgaatcagga gactgtagac agcaagaatt 300

cagggatcgg tctggaaact gtgttccctg caaccagtgt gggccaggca 350

tggagttgtc taaggaatgt ggcttcggct atggggagga tgcacagtgt 400

gtgacgtgcc ggctgcacag gttcaaggag gactggggct tccagaaatg 450

caagccctgt ctggactgcg cagtggtgaa ccgctttcag aaggcaaatt 500

gttcagccac cagtgatgcc atctgcgggg actgcttgcc aggattttat 550

aggaagacga aacttgtcgg ctttcaagac atggagtgtg tgccttgtgg 600

agaccctcct cctccttacg aaccgcactg tgccagcaag gtcaacctcg 650

tgaagatcgc gtccacggcc tccagcccac gggacacggc gctggctgcc 700

gttatctgca gcgctctggc caccgtcctg ctggccctgc tcatcctctg 750

tgtcatctat tgtaagagac agtttatgga gaagaaaccc agctggtctc 800

tgcggtcgca ggacattcag tacaacggct ctgagctgtc gtgttttgac 850

agacctcagc tccacgaata tgcccacaga gcctgctgcc agtgccgccg 900

tgactcagtg cagacctgcg ggccggtgcg cttgctccca tccatgtgct 950

gtgaggaggc ctgcagcccc aacccggcga ctcttggttg tggggtgcat 1000

tctgcagcca gtcttcaggc aagaaacgca ggcccagccg gggagatggt 1050

gccgactttc ttcggatccc tcacgcagtc catctgtggc gagttttcag 1100

atgcctggcc tctgatgcag aatcccatgg gtggtgacaa catctctttt 1150

tgtgactctt atcctgaact cactggagaa gacattcatt ctctcaatcc 1200

agaacttgaa agctcaacgt ctttggattc aaatagcagt caagatttgg 1250

ttggtggggc tgttccagtc cagtctcatt ctgaaaactt tacagcagct 1300

actgatttat ctagatataa caacacactg gtagaatcag catcaactca 1350

ggatgcacta actatgagaa gccagctaga tcaggagagt ggcgctgtca 1400
tccacccagc cactcagacg tccctccagg aagcttaaag aacctgcttc 1450
tttctgcagt agaagcgtgt gctggaaccc aaagagtact cctttgttag 1500
gcttatggac tgagcagtct ggaccttgca tggcttctgg ggcaaaaata 1550
aatctgaacc aaactgacgg catttgaagc ctttcagcca gttgcttctg 1600
agccagacca gctgtaagct gaaacctcaa tgaataacaa gaaaagactc 1650
caggccgact catgatactc tgcatctttc ctacatgaga agcttctctg 1700
ccacaaaagt gacttcaaag actgatgggt tgagctggca gcctatgaga 1750
ttgtggacat ataacaagaa acagaaatgc cctcatgctt attttcatgg 1800
tgattgtggt tttacaagac tgaagaccca gagtatactt tttctttcca 1850
gaaataattt cataccgcct atgaaatatc agataaatta ccttagcttt 1900
tatgtagaat gggttcaaaa gtgagtgttt ctatttgaga aggacacttt 1950
ttcatcatct aaactgattc gcataggtgg ttagaatggc cctcatattg 2000
cctgcctaaa tcttgggttt attagatgaa gtttactgaa tcagaggaat 2050
cagacagagg aggatagctc tttccagaat ccacacttct gacctcagcc 2100
tcggtctcat gaacacccgc tgatctcagg agaacacctg ggctagggaa 2150
tgtggtcgag aaagggcagc ccattgccca gaattaacac atattgtaga 2200
gacttgtatg caaaggttgg catatttata tgaaaattag ttgctataga 2250
aacatttgtt gcatctgtcc ctctgcctga gcttagaagg ttatagaaaa 2300
agggtattta taaacataaa tgacctttta cttgcattgt atcttatact 2350
aaaggcttta gaaattacaa catatcaggt tcccctacta ctgaagtagc 2400
cttccgtgag aacacaccac atgttaggac tagaagaaaa tgcacaattt 2450
gtaggggttt ggatgaagca gctgtaactg ccctagtgta gtttgaccag 2500
gacattgtcg tgctccttcc aattgtgtaa gattagttag cacatcatct 2550
cctactttag ccatccggtg ttggatttaa gaggacggtg cttctttcta 2600
ttaaagtgct ccatccccta ccatctacac attagcattg tctctagagc 2650
taagacagaa attaaccccg ttcagtcaca aagcagggaa tggttcattt 2700
actcttaatc tttatgccct ggagaagacc tacttgaaca gggcatattt 2750
tttagacttc tgaacatcag tatgttcgag ggtactatga tattttggtt 2800
tggaattgcc ctgcccaagt cactgtcttt taacttttaa actgaatatt 2850
aaaatgtatc tgtctttcct 2870

<210> 220
<211> 417
<212> PRT
<213> Homo Sapien

<400> 220

Met Ala Leu Lys Val Leu Leu Glu Gln Glu Lys Thr Phe Phe Thr
1               5                   10                  15

Leu Leu Val Leu Leu Gly Tyr Leu Ser Cys Lys Val Thr Cys Glu
                20                  25                  30

Ser Gly Asp Cys Arg Gln Gln Glu Phe Arg Asp Arg Ser Gly Asn
                35                  40                  45

Cys Val Pro Cys Asn Gln Cys Gly Pro Gly Met Glu Leu Ser Lys
                50                  55                  60

Glu Cys Gly Phe Gly Tyr Gly Glu Asp Ala Gln Cys Val Thr Cys
                65                  70                  75

Arg Leu His Arg Phe Lys Glu Asp Trp Gly Phe Gln Lys Cys Lys
                80                  85                  90

Pro Cys Leu Asp Cys Ala Val Val Asn Arg Phe Gln Lys Ala Asn
                95                  100                 105

Cys Ser Ala Thr Ser Asp Ala Ile Cys Gly Asp Cys Leu Pro Gly
                110                 115                 120

Phe Tyr Arg Lys Thr Lys Leu Val Gly Phe Gln Asp Met Glu Cys
                125                 130                 135

Val Pro Cys Gly Asp Pro Pro Pro Pro Tyr Glu Pro His Cys Ala
                140                 145                 150

Ser Lys Val Asn Leu Val Lys Ile Ala Ser Thr Ala Ser Ser Pro
                155                 160                 165

Arg Asp Thr Ala Leu Ala Ala Val Ile Cys Ser Ala Leu Ala Thr
                170                 175                 180

Val Leu Leu Ala Leu Leu Ile Leu Cys Val Ile Tyr Cys Lys Arg
                185                 190                 195

Gln Phe Met Glu Lys Lys Pro Ser Trp Ser Leu Arg Ser Gln Asp
                200                 205                 210

Ile Gln Tyr Asn Gly Ser Glu Leu Ser Cys Phe Asp Arg Pro Gln
                215                 220                 225

Leu His Glu Tyr Ala His Arg Ala Cys Cys Gln Cys Arg Arg Asp
                230                 235                 240

Ser Val Gln Thr Cys Gly Pro Val Arg Leu Leu Pro Ser Met Cys
                245                 250                 255

Cys Glu Glu Ala Cys Ser Pro Asn Pro Ala Thr Leu Gly Cys Gly
                260                 265                 270

Val His Ser Ala Ala Ser Leu Gln Ala Arg Asn Ala Gly Pro Ala
                275                 280                 285

Gly Glu Met Val Pro Thr Phe Phe Gly Ser Leu Thr Gln Ser Ile
                290                 295                 300

Cys Gly Glu Phe Ser Asp Ala Trp Pro Leu Met Gln Asn Pro Met
                305                 310                 315

Gly Gly Asp Asn Ile Ser Phe Cys Asp Ser Tyr Pro Glu Leu Thr
                320                 325                 330

Gly Glu Asp Ile His Ser Leu Asn Pro Glu Leu Glu Ser Ser Thr
                335                 340                 345

Ser Leu Asp Ser Asn Ser Ser Gln Asp Leu Val Gly Gly Ala Val
                350                 355                 360

Pro Val Gln Ser His Ser Glu Asn Phe Thr Ala Ala Thr Asp Leu
                365                 370                 375

Ser Arg Tyr Asn Asn Thr Leu Val Glu Ser Ala Ser Thr Gln Asp
                380                 385                 390

Ala Leu Thr Met Arg Ser Gln Leu Asp Gln Glu Ser Gly Ala Val
                395                 400                 405

Ile His Pro Ala Thr Gln Thr Ser Leu Gln Glu Ala
                410                 415

<210> 221
<211> 961
<212> DNA
<213> Homo Sapien

<400> 221
ctagagagta tagggcagaa ggatggcaga tgagtgactc cacatccaga 50

gctgcctccc tttaatccag gatcctgtcc ttcctgtcct gtaggagtgc 100

ctgttgccag tgtggggtga gacaagtttg tcccacaggg ctgtctgagc 150

agataagatt aagggctggg tctgtgctca attaactcct gtgggcacgg 200

gggctgggaa gagcaaagtc agcggtgcct acagtcagca ccatgctggg 250

cctgccgtgg aagggaggtc tgtcctgggc gctgctgctg cttctcttag 300

gctcccagat cctgctgatc tatgcctggc atttccacga gcaaagggac 350

tgtgatgaac acaatgtcat ggctcgttac ctccctgcca cagtggagtt 400

tgctgtccac acattcaacc aacagagcaa ggactactat gcctacagac 450

tggggcacat cttgaattcc tggaaggagc aggtggagtc caagactgta 500

ttctcaatgg agctactgct ggggagaact aggtgtggga aatttgaaga 550

cgacattgac aactgccatt tccaagaaag cacagagctg aacaatactt 600

tcacctgctt cttcaccatc agcaccaggc cctggatgac tcagttcagc 650

ctcctgaaca agacctgctt ggagggattc cactgagtga aacccactca 700

caggcttgtc catgtgctgc tcccacattc cgtggacatc agcactactc 750

tcctgaggac tcttcagtgg ctgagcagct ttggacttgt ttgttatcct 800

attttgcatg tgtttgagat ctcagatcag tgttttagaa aatccacaca 850

tcttgagcct aatcatgtag tgtagatcat taaacatcag cattttaaga 900

aaaaaaaaaa aaaaaaaaaa aaaaaaaaaa aaaaaaaaaa aaaaaaaaaa 950

aaaaaaaaaa a 961

<210> 222
<211> 147
<212> PRT
<213> Homo Sapien

<400> 222

Met Leu Gly Leu Pro Trp Lys Gly Gly Leu Ser Trp Ala Leu Leu
1               5                   10                  15

Leu Leu Leu Leu Gly Ser Gln Ile Leu Leu Ile Tyr Ala Trp His
                20                  25                  30

Phe His Glu Gln Arg Asp Cys Asp Glu His Asn Val Met Ala Arg
                35                  40                  45

Tyr Leu Pro Ala Thr Val Glu Phe Ala Val His Thr Phe Asn Gln
                50                  55                  60

Gln Ser Lys Asp Tyr Tyr Ala Tyr Arg Leu Gly His Ile Leu Asn
                65                  70                  75

Ser Trp Lys Glu Gln Val Glu Ser Lys Thr Val Phe Ser Met Glu
                80                  85                  90

Leu Leu Leu Gly Arg Thr Arg Cys Gly Lys Phe Glu Asp Asp Ile
                95                  100                 105

Asp Asn Cys His Phe Gln Glu Ser Thr Glu Leu Asn Asn Thr Phe
                110                 115                 120

Thr Cys Phe Phe Thr Ile Ser Thr Arg Pro Trp Met Thr Gln Phe
                125                 130                 135

Ser Leu Leu Asn Lys Thr Cys Leu Glu Gly Phe His
                140                 145

<210> 223
<211> 846
<212> DNA
<213> Homo Sapien

<400> 223
aatcggctga ttctgcatct ggaaactgcc ttcatcttga aagaaaagct 50

ccaggtccct tctccagcca cccagcccca agatggtgat gctgctgctg 100

ctgctttccg cactggctgg cctcttcggt gcggcagagg gacaagcatt 150

tcatcttggg aagtgcccca atcctccggt gcaggagaat tttgacgtga 200

ataagtatct cggaagatgg tacgaaattg agaagatccc aacaaccttt 250

gagaatggac gctgcatcca ggccaactac tcactaatgg aaaacggaaa 300

gatcaaagtg ttaaaccagg agttgagagc tgatggaact gtgaatcaaa 350

tcgaaggtga agccacccca gttaacctca cagagcctgc caagctggaa 400

gttaagtttt cctggtttat gccatcggca ccgtactgga tcctggccac 450

cgactatgag aactatgccc tcgtgtattc ctgtacctgc atcatccaac 500

tttttcacgt ggattttgct tggatcttgg caagaaaccc taatctccct 550

ccagaaacag tggactctct aaaaaatatc ctgacttcta ataacattga 600

tgtcaagaaa atgacggtca cagaccaggt gaactgcccc aagctctcgt 650

aaccaggttc tacagggagg ctgcacccac tccatgttac ttctgcttcg 700

ctttccccta ccccaccccc cccccataaa gacaaaccaa tcaaccacga 750

caaaggaagt tgacctgaac atgtaaccat gccctaccct gttaccttgc 800

tagctgcaaa ataaacttgt tgctgacctg ctgtgctcgc aaaaaa 846

<210> 224
<211> 189
<212> PRT
<213> Homo Sapien

<400> 224
Met Val Met Leu Leu Leu Leu Leu Ser Ala Leu Ala Gly Leu Phe
1               5                   10                  15

Gly Ala Ala Glu Gly Gln Ala Phe His Leu Gly Lys Cys Pro Asn
                20                  25                  30

Pro Pro Val Gln Glu Asn Phe Asp Val Asn Lys Tyr Leu Gly Arg
                35                  40                  45

Trp Tyr Glu Ile Glu Lys Ile Pro Thr Thr Phe Glu Asn Gly Arg
                50                  55                  60

Cys Ile Gln Ala Asn Tyr Ser Leu Met Glu Asn Gly Lys Ile Lys
                65                  70                  75

Val Leu Asn Gln Glu Leu Arg Ala Asp Gly Thr Val Asn Gln Ile
                80                  85                  90

Glu Gly Glu Ala Thr Pro Val Asn Leu Thr Glu Pro Ala Lys Leu
                95                  100                 105

Glu Val Lys Phe Ser Trp Phe Met Pro Ser Ala Pro Tyr Trp Ile
                110                 115                 120

Leu Ala Thr Asp Tyr Glu Asn Tyr Ala Leu Val Tyr Ser Cys Thr
                125                 130                 135

Cys Ile Ile Gln Leu Phe His Val Asp Phe Ala Trp Ile Leu Ala
                140                 145                 150

Arg Asn Pro Asn Leu Pro Pro Glu Thr Val Asp Ser Leu Lys Asn
                155                 160                 165

Ile Leu Thr Ser Asn Asn Ile Asp Val Lys Lys Met Thr Val Thr
                170                 175                 180

Asp Gln Val Asn Cys Pro Lys Leu Ser
                185

<210> 225
<211> 1088
<212> DNA
<213> Homo Sapien

<400> 225
gggtgattga actaaacctt cgccgcaccg agtttgcagt acggccgtca 50

cccgcaccgc tgcctgcttg cggttggaga aatcaaggcc ctaccgggcc 100

tccgtagtca cctctctata gtgggcgtgg ccgaggccgg ggtgaccctg 150

ccggagcctc cgctgccagc gacatgttca aggtaattca gaggtccgtg 200

gggccagcca gcctgagctt gctcaccttc aaagtctatg cagcaccaaa 250

aaaggactca cctcccaaaa attccgtgaa ggttgatgag ctttcactct 300

actcagttcc tgagggtcaa tcgaagtatg tggaggaggc aaggagccag 350

cttgaagaaa gcatctcaca gctccgacac tattgcgagc catacacaac 400

ctggtgtcag gaaacgtact cccaaactaa gcccaagatg caaagtttgg 450

ttcaatgggg gttagacagc tatgactatc tccaaaatgc acctcctgga 500

ttttttccga gacttggtgt tattggtttt gctggcctta ttggactcct 550

tttggctaga ggttcaaaaa taaagaagct agtgtatccg cctggtttca 600

tgggattagc tgcctccctc tattatccac aacaagccat cgtgtttgcc 650

caggtcagtg gggagagatt atatgactgg ggtttacgag gatatatagt 700

catagaagat ttgtggaagg agaactttca aaagccagga aatgtgaaga 750

attcacctgg aactaagtag aaaactccat gctctgccat cttaatcagt 800

tataggtaaa cattggaaac tccatagaat aaatcagtat ttctacagaa 850

aaatggcata gaagtcagta ttgaatgtat taaattggct ttcttcttca 900

ggaaaaacta gaccagacct ctgttatctt ctgtgaaatc atcctacaag 950

caaactaacc tggaatccct tcacctagag ataatgtaca agccttagaa 1000

ctcctcattc tcatgttgct atttatgtac ctaattaaaa cccaagttta 1050

aaaaaaaaaa aaaaaaaaaa aaaaaaaaaa aaaaaaaa 1088

<210> 226
<211> 198
<212> PRT
<213> Homo Sapien

<400> 226

<210> 227
<211> 2164
<212> DNA
<213> Homo Sapien

<400> 227
caccggaggg cacgcagctg acggagctgc gctgcgttcg cctcgtttgc 50

ctcgcgccct ccactggagc tgttcgcgcc tcccggctcc caccgcagcc 100

cacccggcag aggagtcgct accagcgccc agtgcgctct gtcagtccgc 150

aaactccttg ccgcccgccc cgggctgggc accaaatacc aggctaccat 200

ggtctacaag actctcttcg ctctttgcat cttaactgca ggatggaggg 250

tacagagtct gcctacatca gctcctttgt ctgtttctct tccgacaaac 300

attgtaccac cgaccaccat ctggactagc tctccacaaa acactgatgc 350

agacactgcc tccccatcca acggcactca caacaactcg gtgctcccag 400

ttacagcatc agccccaaca tctctgcttc ctaagaacat ttccatagag 450

tccagagaag aggagatcac cagcccaggt tcgaattggg aaggcacaaa 500

cacagacccc tcaccttctg ggttctcgtc aacaagcggt ggagtccact 550

taacaaccac gttggaggaa cacagctcgg gcactcctga agcaggcgtg 600

gcagctacac tgtcgcagtc cgctgctgag cctcccacac tcatctcccc 650

tcaagctcca gcctcatcac cctcatccct atcaacctca ccacctgagg 700

tcttttctgc ctccgttact accaaccata gctccactgt gaccagcacc 750

caacccactg gagctccaac tgcaccagag tccccgacag aggagtccag 800

ctctgaccac acacccactt cacatgccac agctgagcca gtgccccagg 850

agaaaacacc cccaacaact gtgtcaggca aagtgatgtg tgagctcata 900

gacatggaga ccaccaccac ctttcccagg gtgatcatgc aggaagtaga 950

acatgcatta agttcaggca gcatcgccgc cattaccgtg acagtcattg 1000

ccgtggtgct gctggtgttt ggagttgcag cctacctaaa aatcaggcat 1050

tcctcctatg gaagactttt ggacgaccat gactacgggt cctggggaaa 1100

ctacaacaac cctctgtacg atgactccta acaatggaat atggcctggg 1150

atgaggatta actgttcttt atttataagt gcttatccag tagaattaat 1200

aagtacctga tgcgcattga acgacaatct taagccctgt tttgttggta 1250

tggttgtttt tgttttcctc cctctcctct ggctgctaca acttcccctt 1300

tctggtacaa gaagaaccat tctttaaagg tgagtggagg ctgatttgca 1350

gctgaagtgg gccagccttg caccagccag gccagaccac catggtgaag 1400

gcttctttcc ccactgcagg acccactttg agaaggatcg aggaggagga 1450

tttgggttgt tttgttaggg gttactttca ggggaacatt tcatttgtgt 1500

tatttcttaa acttctattt aggaaattac attaagtatt aatgagggga 1550

aaggaaatga gctctacgag gatttcacct tgcatgggag agagcagggt 1600

tttctcagat tcctttttaa tctctattta tctggttgtt tctgacagga 1650

tgctgcctgc ttggctctac gagctggaaa gcagcttctt agctgcctaa 1700

ttaatgaaag atgaaaatag gaagtgccct ggagggggcc agcaggtcac 1750

ggggcagaat ctctcaggtt gctgtgggat ctcagtgtgc ccctacctgt 1800

tctcccctcc aggccacctg tctctgtaaa ggatgtctgc tctgttcaaa 1850

aggcagctgg gatcccagcc cacaagtgat cagcagagtt gcatttccaa 1900

agaaaaaggc tatgagatga gctgagttat agagagaaag ggagaggcat 1950

gtacggtgtg gggaagtgga agagaagctg gcgggggaga aggaggctaa 2000

cctgcactga gtacttcatt aggacaagtg agaatcagct attgataatg 2050

gccagagata tccacagctt ggaggagccc agagactgtt tgctttatac 2100

ccacacagca actggtccac tgctttactg tctgttggat aatggctgta 2150

aaatgtttaa aaac 2164

<210> 228
<211> 310
<212> PRT
<213> Homo Sapien

<400> 228
Met Val Tyr Lys Thr Leu Phe Ala Leu Cys Ile Leu Thr Ala Gly
1               5                   10                  15

Trp Arg Val Gln Ser Leu Pro Thr Ser Ala Pro Leu Ser Val Ser
                20                  25                  30

Leu Pro Thr Asn Ile Val Pro Pro Thr Thr Ile Trp Thr Ser Ser
                35                  40                  45

Pro Gln Asn Thr Asp Ala Asp Thr Ala Ser Pro Ser Asn Gly Thr
                50                  55                  60

His Asn Asn Ser Val Leu Pro Val Thr Ala Ser Ala Pro Thr Ser
                65                  70                  75

Leu Leu Pro Lys Asn Ile Ser Ile Glu Ser Arg Glu Glu Glu Ile
                80                  85                  90

Thr Ser Pro Gly Ser Asn Trp Glu Gly Thr Asn Thr Asp Pro Ser
                95                  100                 105

Pro Ser Gly Phe Ser Ser Thr Ser Gly Gly Val His Leu Thr Thr
                110                 115                 120

Thr Leu Glu Glu His Ser Ser Gly Thr Pro Glu Ala Gly Val Ala
                125                 130                 135

Ala Thr Leu Ser Gln Ser Ala Ala Glu Pro Pro Thr Leu Ile Ser
                140                 145                 150

Pro Gln Ala Pro Ala Ser Ser Pro Ser Ser Leu Ser Thr Ser Pro
                155                 160                 165

Pro Glu Val Phe Ser Ala Ser Val Thr Thr Asn His Ser Ser Thr
                170                 175                 180

Val Thr Ser Thr Gln Pro Thr Gly Ala Pro Thr Ala Pro Glu Ser
                185                 190                 195

Pro Thr Glu Glu Ser Ser Ser Asp His Thr Pro Thr Ser His Ala
                200                 205                 210

Thr Ala Glu Pro Val Pro Gln Glu Lys Thr Pro Pro Thr Thr Val
                215                 220                 225

Ser Gly Lys Val Met Cys Glu Leu Ile Asp Met Glu Thr Thr Thr
                230                 235                 240

Thr Phe Pro Arg Val Ile Met Gln Glu Val Glu His Ala Leu Ser
                245                 250                 255

Ser Gly Ser Ile Ala Ala Ile Thr Val Thr Val Ile Ala Val Val
                260                 265                 270

Leu Leu Val Phe Gly Val Ala Ala Tyr Leu Lys Ile Arg His Ser
                275                 280                 285

Ser Tyr Gly Arg Leu Leu Asp Asp His Asp Tyr Gly Ser Trp Gly
                290                 295                 300

Asn Tyr Asn Asn Pro Leu Tyr Asp Asp Ser
                305                 310

<210> 229
<211> 633
<212> DNA
<213> Homo Sapien

<400> 229
ctcctgcact aggctctcag ccagggatga tgcgctgctg ccgccgccgc 50

tgctgctgcc ggcaaccacc ccatgccctg aggccgttgc tgttgctgcc 100

cctcgtcctt ttacctcccc tggcagcagc tgcagcgggc ccaaaccgat 150

gtgacaccat ataccagggc ttcgccgagt gtctcatccg cttgggggac 200

agcatgggcc gcggaggcga gctggagacc atctgcaggt cttggaatga 250

cttccatgcc tgtgcctctc aggtcctgtc aggctgtccg gaggaggcag 300

ctgcagtgtg ggaatcacta cagcaagaag ctcgccaggc cccccgtccg 350

aataacttgc acactctgtg cggtgccccg gtgcatgttc gggagcgcgg 400

cacaggctcc gaaaccaacc aggagacgct gcgggctaca gcgcctgcac 450

tccccatggc ccctgcgccc ccactgctgg cggctgctct ggctctggcc 500

tacctcctga ggcctctggc ctagcttgtt gggttgggta gcagcgcccg 550

tacctccagc cctgctctgg cggtggttgt ccaggctctg cagagcgcag 600

cagggctttt cattaaaggt atttatattt gta 633

<210> 230
<211> 165
<212> PRT
<213> Homo Sapien

<400> 230
Met Met Arg Cys Cys Arg Arg Arg Cys Cys Cys Arg Gln Pro Pro
1               5                   10                  15

His Ala Leu Arg Pro Leu Leu Leu Leu Pro Leu Val Leu Leu Pro
                20                  25                  30

Pro Leu Ala Ala Ala Ala Ala Gly Pro Asn Arg Cys Asp Thr Ile
                35                  40                  45

Tyr Gln Gly Phe Ala Glu Cys Leu Ile Arg Leu Gly Asp Ser Met
                50                  55                  60

Gly Arg Gly Gly Glu Leu Glu Thr Ile Cys Arg Ser Trp Asn Asp
                65                  70                  75

Phe His Ala Cys Ala Ser Gln Val Leu Ser Gly Cys Pro Glu Glu
                80                  85                  90

Ala Ala Ala Val Trp Glu Ser Leu Gln Gln Glu Ala Arg Gln Ala
                95                  100                 105

Pro Arg Pro Asn Asn Leu His Thr Leu Cys Gly Ala Pro Val His
                110                 115                 120

Val Arg Glu Arg Gly Thr Gly Ser Glu Thr Asn Gln Glu Thr Leu
                125                 130                 135

Arg Ala Thr Ala Pro Ala Leu Pro Met Ala Pro Ala Pro Pro Leu
                140                 145                 150

Leu Ala Ala Ala Leu Ala Leu Ala Tyr Leu Leu Arg Pro Leu Ala
                155                 160                 165

<210> 231
<211> 890
<212> DNA
<213> Homo Sapien

<400> 231
aagtacttgt gtccgggtgg tggactggat tagctgcgga gccctggaag 50

ctgcctgtcc ttctccctgt gcttaaccag aggtgcccat gggttggaca 100

atgaggctgg tcacagcagc actgttactg ggtctcatga tggtggtcac 150

tggagacgag gatgagaaca gcccgtgtgc ccatgaggcc ctcttggacg 200

aggacaccct cttttgccag ggccttgaag ttttctaccc agagttgggg 250

aacattggct gcaaggttgt tcctgattgt aacaactaca gacagaagat 300

cacctcctgg atggagccga tagtcaagtt cccgggggcc gtggacggcg 350

caacctatat cctggtgatg gtggatccag atgcccctag cagagcagaa 400

cccagacaga gattctggag acattggctg gtaacagata tcaagggcgc 450

cgacctgaag aaagggaaga ttcagggcca ggagttatca gcctaccagg 500

ctccctcccc accggcacac agtggcttcc atcgctacca gttctttgtc 550

tatcttcagg aaggaaaagt catctctctc cttcccaagg aaaacaaaac 600

tcgaggctct tggaaaatgg acagatttct gaaccgcttc cacctgggcg 650

aacctgaagc aagcacccag ttcatgaccc agaactacca ggactcacca 700

accctccagg ctcccagagg aagggccagc gagcccaagc acaaaaccag 750

gcagagatag ctgcctgcta gatagccggc tttgccatcc gggcatgtgg 800

ccacactgct caccaccgac gatgtgggta tggaaccccc tctggataca 850

gaaccccttc ttttccaaat taaaaaaaaa aatcatcaaa 890

<210> 232
<211> 223
<212> PRT
<213> Homo Sapien

<400> 232

<210> 233
<211> 924
<212> DNA
<213> Homo Sapien

<400> 233
aaggagcagc ccgcaagcac caagtgagag gcatgaagtt acagtgtgtt 50

tccctttggc tcctgggtac aatactgata ttgtgctcag tagacaacca 100

cggtctcagg agatgtctga tttccacaga catgcaccat atagaagaga 150

gtttccaaga aatcaaaaga gccatccaag ctaaggacac cttcccaaat 200

gtcactatcc tgtccacatt ggagactctg cagatcatta agcccttaga 250

tgtgtgctgc gtgaccaaga acctcctggc gttctacgtg gacagggtgt 300

tcaaggatca tcaggagcca aaccccaaaa tcttgagaaa aatcagcagc 350

attgccaact ctttcctcta catgcagaaa actctgcggc aatgtcagga 400

acagaggcag tgtcactgca ggcaggaagc caccaatgcc accagagtca 450

tccatgacaa ctatgatcag ctggaggtcc acgctgctgc cattaaatcc 500

ctgggagagc tcgacgtctt tctagcctgg attaataaga atcatgaagt 550

aatgttctca gcttgatgac aaggaacctg tatagtgatc cagggatgaa 600

caccccctgt gcggtttact gtgggagaca gcccaccttg aaggggaagg 650

agatggggaa ggccccttgc agctgaaagt cccactggct ggcctcaggc 700

tgtcttattc cgcttgaaaa taggcaaaaa gtctactgtg gtatttgtaa 750

taaactctat ctgctgaaag ggcctgcagg ccatcctggg agtaaagggc 800

tgccttccca tctaatttat tgtaaagtca tatagtccat gtctgtgatg 850

tgagccaagt gatatcctgt agtacacatt gtactgagtg gtttttctga 900

ataaattcca tattttacct atga 924

<210> 234
<211> 177
<212> PRT
<213> Homo Sapien

<400> 234
Met Lys Leu Gln Cys Val Ser Leu Trp Leu Leu Gly Thr Ile Leu
1               5                   10                  15

Ile Leu Cys Ser Val Asp Asn His Gly Leu Arg Arg Cys Leu Ile
                20                  25                  30

Ser Thr Asp Met His His Ile Glu Glu Ser Phe Gln Glu Ile Lys
                35                  40                  45

Arg Ala Ile Gln Ala Lys Asp Thr Phe Pro Asn Val Thr Ile Leu
                50                  55                  60

Ser Thr Leu Glu Thr Leu Gln Ile Ile Lys Pro Leu Asp Val Cys
                65                  70                  75

Cys Val Thr Lys Asn Leu Leu Ala Phe Tyr Val Asp Arg Val Phe
                80                  85                  90

Lys Asp His Gln Glu Pro Asn Pro Lys Ile Leu Arg Lys Ile Ser
                95                  100                 105

Ser Ile Ala Asn Ser Phe Leu Tyr Met Gln Lys Thr Leu Arg Gln

                110                 115                 120

Cys Gln Glu Gln Arg Gln Cys His Cys Arg Gln Glu Ala Thr Asn
                125                 130                 135

Ala Thr Arg Val Ile His Asp Asn Tyr Asp Gln Leu Glu Val His
                140                 145                 150

Ala Ala Ala Ile Lys Ser Leu Gly Glu Leu Asp Val Phe Leu Ala
                155                 160                 165

Trp Ile Asn Lys Asn His Glu Val Met Phe Ser Ala
                170                 175

<210> 235
<211> 1161
<212> DNA
<213> Homo Sapien

<220>
<221> unsure
<222> 1149
<223> unknown base

<400> 235
gcccgggcgg ctgcccttgg gtgctccctt ccctgcccga cacccagacc 50

gaccttgacc gcccacctgg caggagcagg acaggacggc cggacgcggc 100

catggccgag ctcccggggc cctttctctg cggggccctg ctaggcttcc 150

tgtgcctgag tgggctggcc gtggaggtga aggtacccac agagccgctg 200

agcacgcccc tggggaagac agccgagctg acctgcacct acagcacgtc 250

ggtgggagac agcttcgccc tggagtggag ctttgtgcag cctgggaaac 300

ccatctctga gtcccatcca atcctgtact tcaccaatgg ccatctgtat 350

ccaactggtt ctaagtcaaa gcgggtcagc ctgcttcaga acccccccac 400

agtgggggtg gccacactga aactgactga cgtccacccc tcagatactg 450

gaacctacct ctgccaagtc aacaacccac cagatttcta caccaatggg 500

ttggggctaa tcaaccttac tgtgctggtt ccccccagta atcccttatg 550

cagtcagagt ggacaaacct ctgtgggagg ctctactgca ctgagatgca 600

gctcttccga gggggctcct aagccagtgt acaactgggt gcgtcttgga 650

acttttccta caccttctcc tggcagcatg gttcaagatg aggtgtctgg 700

ccagctcatt ctcaccaacc tctccctgac ctcctcgggc acctaccgct 750

gtgtggccac caaccagatg ggcagtgcat cctgtgagct gaccctctct 800

gtgaccgaac cctcccaagg ccgagtggcc ggagctctga ttggggtgct 850

cctgggcgtg ctgttgctgt cagttgctgc gttctgcctg gtcaggttcc 900

agaaagagag ggggaagaag cccaaggaga catatggggg tagtgacctt 950

cgggaggatg ccatcgctcc tgggatctct gagcacactt gtatgagggc 1000

tgattctagc aaggggttcc tggaaagacc ctcgtctgcc agcaccgtga 1050

cgaccaccaa gtccaagctc cctatggtcg tgtgacttct cccgatccct 1100

gagggcggtg agggggaata tcaataatta aagtctgtgg gtacccttna 1150

aaaaaaaaaa a 1161

<210> 236
<211> 327
<212> PRT
<213> Homo Sapien

<400> 236
Met Ala Glu Leu Pro Gly Pro Phe Leu Cys Gly Ala Leu Leu Gly
1               5                   10                  15

Phe Leu Cys Leu Ser Gly Leu Ala Val Glu Val Lys Val Pro Thr
                20                  25                  30

Glu Pro Leu Ser Thr Pro Leu Gly Lys Thr Ala Glu Leu Thr Cys
                35                  40                  45

Thr Tyr Ser Thr Ser Val Gly Asp Ser Phe Ala Leu Glu Trp Ser
                50                  55                  60

Phe Val Gln Pro Gly Lys Pro Ile Ser Glu Ser His Pro Ile Leu
                65                  70                  75

Tyr Phe Thr Asn Gly His Leu Tyr Pro Thr Gly Ser Lys Ser Lys
                80                  85                  90

Arg Val Ser Leu Leu Gln Asn Pro Pro Thr Val Gly Val Ala Thr
                95                  100                 105

Leu Lys Leu Thr Asp Val His Pro Ser Asp Thr Gly Thr Tyr Leu
                110                 115                 120

Cys Gln Val Asn Asn Pro Pro Asp Phe Tyr Thr Asn Gly Leu Gly
                125                 130                 135

Leu Ile Asn Leu Thr Val Leu Val Pro Pro Ser Asn Pro Leu Cys
                140                 145                 150

Ser Gln Ser Gly Gln Thr Ser Val Gly Gly Ser Thr Ala Leu Arg
                155                 160                 165

Cys Ser Ser Ser Glu Gly Ala Pro Lys Pro Val Tyr Asn Trp Val
                170                 175                 180

Arg Leu Gly Thr Phe Pro Thr Pro Ser Pro Gly Ser Met Val Gln
                185                 190                 195

Asp Glu Val Ser Gly Gln Leu Ile Leu Thr Asn Leu Ser Leu Thr
                200                 205                 210

Ser Ser Gly Thr Tyr Arg Cys Val Ala Thr Asn Gln Met Gly Ser
                215                 220                 225

Ala Ser Cys Glu Leu Thr Leu Ser Val Thr Glu Pro Ser Gln Gly
                230                 235                 240

Arg Val Ala Gly Ala Leu Ile Gly Val Leu Leu Gly Val Leu Leu
                245                 250                 255

Leu Ser Val Ala Ala Phe Cys Leu Val Arg Phe Gln Lys Glu Arg
                260                 265                 270

Gly Lys Lys Pro Lys Glu Thr Tyr Gly Gly Ser Asp Leu Arg Glu
                275                 280                 285

Asp Ala Ile Ala Pro Gly Ile Ser Glu His Thr Cys Met Arg Ala
                290                 295                 300

Asp Ser Ser Lys Gly Phe Leu Glu Arg Pro Ser Ser Ala Ser Thr
                305                 310                 315

Val Thr Thr Thr Lys Ser Lys Leu Pro Met Val Val
                320                 325

<210> 237
<211> 983
<212> DNA
<213> Homo Sapien

<400> 237
ggatgcagca gagaggagca gctggaagcc gtggctgcgc tctcttccct 50

ctgctgggcg tcctgttctt ccagggtgtt tatatcgtct tttccttgga 100

gattcgtgca gatgcccatg tccgaggtta tgttggagaa aagatcaagt 150

tgaaatgcac tttcaagtca acttcagatg tcactgacaa gcttactata 200

gactggacat atcgccctcc cagcagcagc cacacagtat caatatttca 250

ttatcagtct ttccagtacc caaccacagc aggcacattt cgggatcgga 300

tttcctgggt tggaaatgta tacaaagggg atgcatctat aagtataagc 350

aaccctacca taaaggacaa tgggacattc agctgtgctg tgaagaatcc 400

cccagatgtg caccataata ttcccatgac agagctaaca gtcacagaaa 450

ggggttttgg caccatgctt tcctctgtgg cccttctttc catccttgtc 500

tttgtgccct cagccgtggt ggttgctctg ctgctggtga gaatggggag 550

gaaggctgct gggctgaaga agaggagcag gtctggctat aagaagtcat 600

ctattgaggt ttccgatgac actgatcagg aggaggaaga ggcgtgtatg 650

gcgaggcttt gtgtccgttg cgctgagtgc ctggattcag actatgaaga 700

gacatattga tgaaagtctg tatgacacaa gaagagtcac ctaaagacag 750

gaaacatccc attccactgg cagctaaagc ctgtcagaga aagtggagct 800

ggcctggacc atagcgatgg acaatcctgg agatcatcag taaagacttt 850

aggaaccact tatttattga ataaatgttc ttgttgtatt tataaactgt 900

tcaggaagtc tcataagaga ctcatgactt cccctttcaa tgaattatgc 950

tgtaattgaa tgaagaaatt cttttcctga gca 983

<210> 238
<211> 235
<212> PRT
<213> Homo Sapien

<400> 238
Met Gln Gln Arg Gly Ala Ala Gly Ser Arg Gly Cys Ala Leu Phe
  1               5                  10                  15

Pro Leu Leu Gly Val Leu Phe Phe Gln Gly Val Tyr Ile Val Phe
                 20                  25                  30

Ser Leu Glu Ile Arg Ala Asp Ala His Val Arg Gly Tyr Val Gly

35                  40                  45

Glu Lys Ile Lys Leu Lys Cys Thr Phe Lys Ser Thr Ser Asp Val
                50                  55                  60

Thr Asp Lys Leu Thr Ile Asp Trp Thr Tyr Arg Pro Pro Ser Ser
                65                  70                  75

Ser His Thr Val Ser Ile Phe His Tyr Gln Ser Phe Gln Tyr Pro
                80                  85                  90

Thr Thr Ala Gly Thr Phe Arg Asp Arg Ile Ser Trp Val Gly Asn
                95                  100                 105

Val Tyr Lys Gly Asp Ala Ser Ile Ser Ile Ser Asn Pro Thr Ile
                110                 115                 120

Lys Asp Asn Gly Thr Phe Ser Cys Ala Val Lys Asn Pro Pro Asp
                125                 130                 135

Val His His Asn Ile Pro Met Thr Glu Leu Thr Val Thr Glu Arg
                140                 145                 150

Gly Phe Gly Thr Met Leu Ser Ser Val Ala Leu Leu Ser Ile Leu
                155                 160                 165

Val Phe Val Pro Ser Ala Val Val Val Ala Leu Leu Leu Val Arg
                170                 175                 180

Met Gly Arg Lys Ala Ala Gly Leu Lys Lys Arg Ser Arg Ser Gly
                185                 190                 195

Tyr Lys Lys Ser Ser Ile Glu Val Ser Asp Asp Thr Asp Gln Glu
                200                 205                 210

Glu Glu Glu Ala Cys Met Ala Arg Leu Cys Val Arg Cys Ala Glu
                215                 220                 225

Cys Leu Asp Ser Asp Tyr Glu Glu Thr Tyr
                230                 235

<210> 239
<211> 2024
<212> DNA
<213> Homo Sapien

<400> 239
caggcgggcc cccgcgcggc agggccctgg acccgcgcgg ctcccgggga 50

tggtgagcaa ggcgctgctg cgcctcgtgt ctgccgtcaa ccgcaggagg 100

atgaagctgc tgctgggcat cgccttgctg gcctacgtcg cctctgtttg 150

gggcaacttc gttaatatga ggtctatcca ggaaaatggt gaactaaaaa 200

ttgaaagcaa gattgaagag atggttgaac cactaagaga gaaaatcaga 250

gatttagaaa aaagctttac ccagaaatac ccaccagtaa agtttttatc 300

agaaaaggat cggaaaagaa ttttgataac aggaggcgca gggttcgtgg 350

gctcccatct aactgacaaa ctcatgatgg acggccacga ggtgaccgtg 400

gtggacaatt tcttcacggg caggaagaga aacgtggagc actggatcgg 450

acatgagaac ttcgagttga ttaaccacga cgtggtggag cccctctaca 500

tcgaggttga ccagatatac catctggcat ctccagcctc ccctccaaac 550

tacatgtata atcctatcaa gacattaaag accaatacga ttgggacatt 600

aaacatgttg gggctggcaa aacgagtcgg tgcccgtctg ctcctggcct 650

ccacatcgga ggtgtatgga gatcctgaag tccaccctca aagtgaggat 700

tactggggcc acgtgaatcc aataggacct cgggcctgct acgatgaagg 750

caaacgtgtt gcagagacca tgtgctatgc ctacatgaag caggaaggcg 800

tggaagtgcg agtggccaga atcttcaaca cctttgggcc acgcatgcac 850

atgaacgatg ggcgagtagt cagcaacttc atcctgcagg cgctccaggg 900

ggagccactc acggtatacg gatccgggtc tcagacaagg gcgttccagt 950

acgtcagcga tctagtgaat ggcctcgtgg ctctcatgaa cagcaacgtc 1000

agcagcccgg tcaacctggg gaacccagaa gaacacacaa tcctagaatt 1050

tgctcagtta attaaaaacc ttgttggtag cggaagtgaa attcagtttc 1100

tctccgaagc ccaggatgac ccacagaaaa gaaaaccaga catcaaaaaa 1150

gcaaagctga tgctggggtg ggagcccgtg gtcccgctgg aggaaggttt 1200

aaacaaagca attcactact tccgtaaaga actcgagtac caggcaaata 1250

atcagtacat ccccaaacca aagcctgcca gaataaagaa aggacggact 1300

cgccacagct gaactcctca cttttaggac acaagactac cattgtacac 1350

ttgatgggat gtatttttgg cttttttttg ttgtcgttta aagaaagact 1400

ttaacaggtg tcatgaagaa caaactggaa tttcattctg aagcttgctt 1450

taatgaaatg gatgtgccta aaagctcccc tcaaaaaact gcagattttg 1500

ccttgcactt tttgaatctc tctttttatg taaaatagcg tagatgcatc 1550

tctgcgtatt ttcaagtttt tttatcttgc tgtgagagca tatgttgtga 1600

ctgtcgttga cagttttatt tactggtttc tttgtgaagc tgaaaaggaa 1650

cattaagcgg gacaaaaaat gccgatttta tttataaaag tgggtactta 1700

ataaatgagt cgttatacta tgcataaaga aaaatcctag cagtattgtc 1750

aggtggtggt gcgccggcat tgattttagg gcagataaaa gaattctgtg 1800

tgagagcttt atgtttctct tttaattcag agtttttcca aggtctactt 1850

ttgagttgca aacttgactt tgaaatattc ctgttggtca tgatcaagga 1900

tatttgaaat cactactgtg ttttgctgcg tatctggggc gggggcaggt 1950

tggggggcac aaagttaaca tattcttggt taaccatggt taaatatgct 2000

attttaataa aatattgaaa ctca 2024

<210> 240
<211> 420
<212> PRT
<213> Homo Sapien

<400> 240
Met Val Ser Lys Ala Leu Leu Arg Leu Val Ser Ala Val Asn Arg

1                   5                   10                  15

Arg Arg Met Lys Leu Leu Leu Gly Ile Ala Leu Leu Ala Tyr Val
                20                  25                  30

Ala Ser Val Trp Gly Asn Phe Val Asn Met Arg Ser Ile Gln Glu
                35                  40                  45

Asn Gly Glu Leu Lys Ile Glu Ser Lys Ile Glu Glu Met Val Glu
                50                  55                  60

Pro Leu Arg Glu Lys Ile Arg Asp Leu Glu Lys Ser Phe Thr Gln
                65                  70                  75

Lys Tyr Pro Pro Val Lys Phe Leu Ser Glu Lys Asp Arg Lys Arg
                80                  85                  90

Ile Leu Ile Thr Gly Gly Ala Gly Phe Val Gly Ser His Leu Thr
                95                  100                 105

Asp Lys Leu Met Met Asp Gly His Glu Val Thr Val Val Asp Asn
                110                 115                 120

Phe Phe Thr Gly Arg Lys Arg Asn Val Glu His Trp Ile Gly His
                125                 130                 135

Glu Asn Phe Glu Leu Ile Asn His Asp Val Val Glu Pro Leu Tyr
                140                 145                 150

Ile Glu Val Asp Gln Ile Tyr His Leu Ala Ser Pro Ala Ser Pro
                155                 160                 165

Pro Asn Tyr Met Tyr Asn Pro Ile Lys Thr Leu Lys Thr Asn Thr
                170                 175                 180

Ile Gly Thr Leu Asn Met Leu Gly Leu Ala Lys Arg Val Gly Ala
                185                 190                 195

Arg Leu Leu Leu Ala Ser Thr Ser Glu Val Tyr Gly Asp Pro Glu
                200                 205                 210

Val His Pro Gln Ser Glu Asp Tyr Trp Gly His Val Asn Pro Ile
                215                 220                 225

Gly Pro Arg Ala Cys Tyr Asp Glu Gly Lys Arg Val Ala Glu Thr
                230                 235                 240

Met Cys Tyr Ala Tyr Met Lys Gln Glu Gly Val Glu Val Arg Val
                245                 250                 255

Ala Arg Ile Phe Asn Thr Phe Gly Pro Arg Met His Met Asn Asp
                260                 265                 270

Gly Arg Val Val Ser Asn Phe Ile Leu Gln Ala Leu Gln Gly Glu
                275                 280                 285

Pro Leu Thr Val Tyr Gly Ser Gly Ser Gln Thr Arg Ala Phe Gln
                290                 295                 300

Tyr Val Ser Asp Leu Val Asn Gly Leu Val Ala Leu Met Asn Ser
                305                 310                 315

Asn Val Ser Ser Pro Val Asn Leu Gly Asn Pro Glu Glu His Thr
                320                 325                 330

Ile Leu Glu Phe Ala Gln Leu Ile Lys Asn Leu Val Gly Ser Gly
                335                 340                 345

Ser Glu Ile Gln Phe Leu Ser Glu Ala Gln Asp Asp Pro Gln Lys
                350                 355                 360

Arg Lys Pro Asp Ile Lys Lys Ala Lys Leu Met Leu Gly Trp Glu
                365                 370                 375

Pro Val Val Pro Leu Glu Glu Gly Leu Asn Lys Ala Ile His Tyr
                380                 385                 390

Phe Arg Lys Glu Leu Glu Tyr Gln Ala Asn Asn Gln Tyr Ile Pro
                395                 400                 405

Lys Pro Lys Pro Ala Arg Ile Lys Lys Gly Arg Thr Arg His Ser
                410                 415                 420

<210> 241
<211> 2025
<212> DNA
<213> Homo Sapien

<400> 241
gcccggtgga gaattaggtg ctgctgggag ctcctgcctc ccacaggatt 50

ccagctgcag ggagcctcag ggactctggg ccgcacggag ttgggggcat 100

tccccagaga gcgtcgccat ggtctgcagg gagcagttat caaagaatca 150

ggtcaagtgg gtgtttgccg gcattacctg tgtgtctgtg gtggtcattg 200

ccgcaatagt ccttgccatc accctgcggc ggccaggctg tgagctggag 250

gcctgcagcc ctgatgccga catgctggac tacctgctga gcctgggcca 300

gatcagccgg cgagatgcct tggaggtcac ctggtaccac gcagccaaca 350

gcaagaaagc catgacagct gccctgaaca gcaacatcac agtcctggag 400

gctgacgtca atgtagaagg gctcggcaca gccaatgaga caggagttcc 450

catcatggca caccccccca ctatctacag tgacaacaca ctggagcagt 500

ggctggacgc tgtgctgggc tcttcccaaa agggcatcaa actggacttc 550

aagaacatca aggcagtggg cccctccctg gacctcctgc ggcagctgac 600

agaggaaggc aaagtccggc ggcccatatg gatcaacgct gacatcttaa 650

agggccccaa catgctcatc tcaactgagg tcaatgccac acagttcctg 700

gccctggtcc aggagaagta tcccaaggct accctatctc caggctggac 750

caccttctac atgtccacgt ccccaaacag gacgtacacc caagccatgg 800

tggagaagat gcacgagctg gtgggaggag tgccccagag ggtcaccttc 850

cctgtacggt cttccatggt gcgggctgcc tggccccact tcagctggct 900

gctgagccaa tctgagaggt acagcctgac gctgtggcag gctgcctcgg 950

accccatgtc ggtggaagat ctgctctacg tccgggataa cactgctgtc 1000

caccaagtct actatgacat ctttgagcct ctcctgtcac agttcaagca 1050

gctggccttg aatgccacac ggaaaccaat gtactacacg ggaggcagcc 1100

tgatccctct tctccagctg cctggggatg acggtctgaa tgtggagtgg 1150

ctggttcctg acgtccaggg cagcggtaaa acagcaacaa tgaccctccc 1200

agacacagaa ggcatgatcc tgctgaacac tggcctcgag ggaactgtgg 1250

ctgaaaaccc cgtgcccatt gttcatactc caagtggcaa catcctgacg 1300

ctggagtcct gcctgcagca gctggccaca catcccggac actggggcat 1350

ccatttgcaa atagtggagc ccgcagccct ccggccatcc ctggccttgc 1400

tggcacgcct ctccagcctt ggcctcttgc attggcctgt gtgggttggg 1450

gccaaaatct cccacgggag tttttcggtc cccggccatg tggctggcag 1500

agagctgctt acagctgtgg ctgaggtctt cccccacgtg actgtggcac 1550

caggctggcc tgaggaggtg ctgggcagtg gctacaggga acagctgctc 1600

acagatatgc tagagttgtg ccaggggctc tggcaacctg tgtccttcca 1650

gatgcaggcc atgctgctgg gccacagcac agctggagcc ataggcaggc 1700

tgctggcatc ctcccccggg gccaccgtca cagtggagca caacccagct 1750

gggggcgact atgcctctgt gaggacagca ttgctggcag ctagggctgt 1800

ggacaggacc cgagtctact acaggctacc ccagggctac cacaaggact 1850

tgctggctca tgttggtaga aactgagcac ccaggggtgg tgggccagcg 1900

gacctcaggg cggaggcttc ccacggggag gcaggaagaa ataaaggtct 1950

ttggctttct ccaggcaaaa aaaaaaaaaa aaaaaaaaaa aaaaaaaaaa 2000

aaaaaaaaaa aaaaaaaaaa aaaag 2025

<210> 242
<211> 585
<212> PRT
<213> Homo Sapien

<400> 242

<210> 243
<211> 1152
<212> DNA
<213> Homo Sapien

<400> 243
cttcagaaca ggttctcctt ccccagtcac cagttgctcg agttagaatt 50

gtctgcaatg gccgccctgc agaaatctgt gagctctttc cttatgggga 100

ccctggccac cagctgcctc cttctcttgg ccctcttggt acagggagga 150

gcagctgcgc ccatcagctc ccactgcagg cttgacaagt ccaacttcca 200

gcagccctat atcaccaacc gcaccttcat gctggctaag gaggctagct 250

tggctgataa caacacagac gttcgtctca ttggggagaa actgttccac 300

ggagtcagta tgagtgagcg ctgctatctg atgaagcagg tgctgaactt 350

cacccttgaa gaagtgctgt tccctcaatc tgataggttc cagccttata 400

tgcaggaggt ggtgcccttc ctggccaggc tcagcaacag gctaagcaca 450

tgtcatattg aaggtgatga cctgcatatc cagaggaatg tgcaaaagct 500

gaaggacaca gtgaaaaagc ttggagagag tggagagatc aaagcaattg 550

gagaactgga tttgctgttt atgtctctga gaaatgcctg catttgacca 600

gagcaaagct gaaaaatgaa taactaaccc cctttccctg ctagaaataa 650

caattagatg ccccaaagcg atttttttta accaaaagga agatgggaag 700

ccaaactcca tcatgatggg tggattccaa atgaacccct gcgttagtta 750

caaaggaaac caatgccact tttgtttata agaccagaag gtagactttc 800

taagcataga tatttattga taacatttca ttgtaactgg tgttctatac 850

acagaaaaca atttattttt taaataattg tctttttcca taaaaaagat 900

tactttccat tcctttaggg gaaaaaaccc ctaaatagct tcatgtttcc 950

ataatcagta ctttatattt ataaatgtat ttattattat tataagactg 1000

cattttattt atatcatttt attaatatgg atttatttat agaaacatca 1050

ttcgatattg ctacttgagt gtaaggctaa tattgatatt tatgacaata 1100

attatagagc tataacatgt ttatttgacc tcaataaaca cttggatatc 1150

cc 1152

<210> 244
<211> 179
<212> PRT
<213> Homo Sapien

<400> 244
Met Ala Ala Leu Gln Lys Ser Val Ser Ser Phe Leu Met Gly Thr
1               5                   10                  15

Leu Ala Thr Ser Cys Leu Leu Leu Leu Ala Leu Leu Val Gln Gly
                20                  25                  30

Gly Ala Ala Ala Pro Ile Ser Ser His Cys Arg Leu Asp Lys Ser
                35                  40                  45

Asn Phe Gln Gln Pro Tyr Ile Thr Asn Arg Thr Phe Met Leu Ala
                50                  55                  60

Lys Glu Ala Ser Leu Ala Asp Asn Asn Thr Asp Val Arg Leu Ile
                65                  70                  75

Gly Glu Lys Leu Phe His Gly Val Ser Met Ser Glu Arg Cys Tyr
                80                  85                  90

Leu Met Lys Gln Val Leu Asn Phe Thr Leu Glu Glu Val Leu Phe
                95                  100                 105

Pro Gln Ser Asp Arg Phe Gln Pro Tyr Met Gln Glu Val Val Pro
                110                 115                 120

Phe Leu Ala Arg Leu Ser Asn Arg Leu Ser Thr Cys His Ile Glu
                125                 130                 135

Gly Asp Asp Leu His Ile Gln Arg Asn Val Gln Lys Leu Lys Asp
                140                 145                 150

Thr Val Lys Lys Leu Gly Glu Ser Gly Glu Ile Lys Ala Ile Gly
                155                 160                 165

Glu Leu Asp Leu Leu Phe Met Ser Leu Arg Asn Ala Cys Ile
                170                 175

<210> 245
<211> 43
<212> DNA
<213> Artificial Sequence

<220>
<223> Synthetic Oligonucleotide Probe

<400> 245
tgtaaaacga cggccagtta aatagacctg caattattaa tct 43

<210> 246
<211> 41
<212> DNA
<213> Artificial Sequence

<220>
<223> Synthetic Oligonucleotide Probe

<400> 246
caggaaacag ctatgaccac ctgcacacct gcaaatccat t 41